# EUROPEAN PATENT APPLICATION

(11) **EP 2 204 444 A1**
(43) Date of publication of application: **07.07.2010**
(21) Application number: 10000366.4
(22) Date of filing: 16.10.2003
(51) Int. Cl.: C12N 9/02, C12N 15/53, C12N 15/82, A01H 5/00

(54) **Cloning of cytochrome P450 genes from Nicotiana**

(30) Priority: 16.10.2002 US 418933 P; 08.07.2003 US 485368 P; 18.09.2003 US 503989 P
(62) Divisional of application: 03809043.7
(71) Applicant: U.S. Smokeless Tobacco Company, Richmond, VA 23230 (US)
(72) Inventor: XU, Dongmei, Glen Allen Virginia 23059 (US)
(74) Representative: Wichmann, Hendrik

(57) **Abstract**

The present invention relates to p450 enzymes and nucleic acid sequences encoding p450 enzymes in Nicotiana, and methods of using those enzymes and nucleic acid sequences to alter plant phenotypes.

## Description

The present invention relates to nucleic acid sequences encoding cytochrome p450 enzymes (hereinafter referred to as p450 and p450 enzymes) in *Nicotiana* plants and methods for using those nucleic acid sequences to alter plant phenotypes.

### BACKGROUND

Cytochrome p450s catalyze enzymatic reactions for a diverse range of chemically dissimilar substrates that include the oxidative, peroxidative and reductive metabolism of endogenous and xenobiotic substrates. In plants, p450s participate in biochemical pathways that include the synthesis of plant products such as phenylpropanoids, alkaloids, terpenoids, lipids, cyanogenic glycosides, and glucosinolates (Chappel, Annu. Rev. Plant Physiol. Plant Mol. Biol. 198, 49:311-343). Cytochrome p450s, also known as p450 heme-thiolate proteins, usually act as terminal oxidases in multicomponent electron transfer chains, called p450-containing monooxygenase systems. Specific reactions catalyzed include demethylation, hydroxylation, epoxidation, N-oxidation, sulfooxidation, N-, S-, and O-dealkylations, desulfation, deamination, and reduction of azo, nitro, and N-oxide groups.

The diverse role of *Nicotiana* plant p450 enzymes has been implicated in effecting a variety of plant metabolites such as phenylpropanoids, alkaloids, terpenoids, lipids, cyanogenic glycosides, glucosinolates and a host of other chemical entities. During recent years, it is becoming apparent that some p450 enzymes can impact the composition of plant metabolites in plants. For example, it has been long desired to improve the flavor and aroma of certain plants by altering its profile of selected fatty acids through breeding; however very little is known about mechanisms involved in controlling the levels of these leaf constituents. The down regulation of p450 enzymes associated with the modification of fatty acids may facilitate accumulation of desired fatty acids that provide more preferred *leaf* phenotypic qualities. The function of p450 enzymes and their broadening roles in plant constituents is still being discovered. For instance, a special class of p450 enzymes was found to catalyze the breakdown of fatty acid into volatile C6- and C9-aldehydes and -alcohols that are major contributors of "fresh green" odor of fruits and vegetables. The level of other novel targeted p450s may be altered to enhance the qualities of leaf constituents by modifying lipid composition and related break down metabolites in Nicotiana leaf. Several of these constituents in leaf are affected by senescence that stimulates the maturation of leaf quality properties. Still other reports have shown that p450s enzymes are play a functional role in altering fatty acids that are involved in plant-pathogen interactions and disease resistance.

In other instances, p450 enzymes have been suggested to be involved in alkaloid biosynthesis. Nornicotine is a minor alkaloid found in *Nicotiana tabaceum*. It has been postulated that it is produced by the p450 mediated demethylation of nicotine followed by acylation and nitrosation at the N position thereby producing a series of N-acylnonicotines and N-nitrosonornicotines. N-demethylation, catalyzed by a putative p450 demethylase, is thought to be a primary source of nornicotine biosyntheses in Nicotiana. While the enzyme is believed to be microsomal, thus far a nicotine demethylase enzyme has not been successfully purified, nor have the genes involved been isolated.

Furthermore, it is hypothesized but not proven that the activity of p450 enzymes is genetically controlled and also strongly influenced by environment factors. For example, the demethylation of nicotine in *Nicotiana* is thought to increase substantially when the plants reach a mature stage. Furthermore, it is hypothesized yet not proven that the demethylase gene contains a transposable element that can inhibit translation of RNA when present.

The large multiplicity of p450 enzyme forms, their differing structure and function have made their research on *Nicotiana* p450 enzymes very difficult before the enclosed invention. In addition, cloning of p450 enzymes has been hampered at least in part because these membrane-localized proteins are typically present in low abundance and often unstable to purification. Hence, a need exists for the identification of p450 enzymes in plants and the nucleic acid sequences associated with those p450 enzymes. In particular, only a few cytochrome p450 proteins have been reported in Nicotiana. The inventions described herein entail the discovery of a substantial number of cytochrome p450 fragments that correspond to several groups of p450 species based on their sequence identity.

### SUMMARY

The present invention is directed to plant p450 enzymes. The present invention is further directed to plant p450 enzymes from *Nicotiana*. The present invention is also directed to p450 enzymes in plants whose expression is induced by ethylene and/or plant senescence. The present invention is yet further directed to nucleic acid sequences in plants having enzymatic activities, for example, being categorized as oxygenase, demethylase and the like, or other and the use of those sequences to reduce or silence the expression or over-expression of these enzymes. The invention also relates to p450 enzymes found in plants containing higher nornicotine levels than plants exhibiting lower nornicotine levels.

In one aspect, the invention is directed to nucleic acid sequences as set forth in SEQ. ID. Nos. 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 95, 97, 99, 101, 103, 105, 107, 109, 111, 113, 115, 117, 119, 121, 123, 125, 127, 129, 131, 133, 135, 137, 139, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295 and 297.

In a second related aspect, those fragments containing greater than 75% identity in nucleic acid sequence were placed into groups dependent upon their identity in a region corresponding to the first nucleic acid following the cytochrome p450 motif GXRXCX(G/A) to the stop codon. The representative nucleic acid groups and respective species are shown in Table I.

In a third aspect, the invention is directed to amino acid sequences as set forth in SEQ. ID. Nos. 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 96, 98, 100, 102, 104, 106, 108, 110, 112, 114, 116, 118, 120, 122, 124, 126, 128, 130, 132, 134, 136, 138, 140, 144, 146, 148, 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296 and 298.

In a fourth related aspect, those fragments containing greater than 71% identity in amino acid sequence were placed into groups dependent upon their identity to each other in a region corresponding to the first amino acid following the cytochrome p450 motif GXRXCX(G/A) to the stop codon. The representative amino acid groups and respective species are shown in Table II.

In a fifth aspect, the invention is directed to amino acid sequences of full length genes as set forth in SEQ. ID. Nos. 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296 and 298.

In a sixth related aspect, those full length genes containing 85% or greater identity in amino acid sequence were placed into groups dependent upon the identity to each other. The representative amino acid groups and respective species are shown in Table III.

In a seventh aspect, the invention is directed to amino acid sequences of the fragments set forth in SEQ. ID. Nos. 299-357.

In the eighth related aspect, those fragments containing 90% or greater identity in amino acid sequence were placed into groups dependent upon their identity to each other in a region corresponding to the first cytochrome p450 domain, UXXRXXZ, to the third cytochrome domain, GXRXO, where U is E or K, X is any amino acid and' Z is R, T, S or M. The representative amino acid groups respective species shown in Table IV.

In a ninth related aspect, the reduction or elimination or over-expression of p450 enzymes in Nicotiana plants may be accomplished transiently using RNA viral systems.

Resulting transformed or infected plants are assessed for phenotypic changes including, but not limited to, analysis of endogenous p450 RNA transcripts, p450 expressed peptides, and concentrations of plant metabolites using techniques commonly available to one having ordinary skill in the art.

In a tenth important aspect, the present invention is also directed to generation of trangenic Nicotiana lines that have altered p450 enzyme activity levels. In accordance with the invention, these transgenic lines include nucleic acid sequences that are effective for reducing or silencing or increasing the expression of certain enzyme thus resulting in phenotypic effects within Nicotiana. Such nucleic acid sequences include SEQ. ID. Nos. 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 95, 97, 99, 101, 103, 105, 107, 109, 111, 113, 115, 117, 119, 121, 123, 125, 127, 129, 131, 133, 135, 137, 139, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295 and 297.

In a very important eleventh aspect of the invention, plant cultivars including nucleic acids of the present invention in a down regulation capacity using either full length genes or fragments thereof or in an over-expression capacity using full length genes will have altered metabolite profiles relative to control plants.

In a twelfth aspect of the invention, plant cultivars including nucleic acid of the present invention using either full length genes or fragments thereof in modifying the biosynthesis or breakdown of metabolites derived from the plant or external to the plants, will have use in tolerating certain exogenous chemicals or plant pests. Such nucleic acid sequences include SEQ ID. Nos. 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, .77, 79, 81, 83, 85, 87, 89, 91, 95, 97, 99, 101, 103, 105, 107, 109, 111, 113, 115, 117, 119, 121, 123, 125, 127, 129, 131, 133, 135, 137, 139, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295 and 297.

In a thirteenth aspect, the present invention is directed to the screening of plants, more preferably Nicotiana, that contain genes that have substantial nucleic acid identity to the taught nucleic acid sequence. The use of the invention would be advantageous to identify and select plants that contain a nucleic acid sequence with exact or substantial identity where such plants are part of a breeding program for traditional or transgenic varieties, a mutagenesis program, or naturally occurring diverse plant populations. The screening of ants for substantial nucleic acid identity may be accomplished by evaluating plant nucleic acid materials using a nucleic acid probe in conjunction with nucleic acid detection protocols including, but not limited to, nucleic acid hybridization and PCR analysis. The nucleic acid probe may consist of the taught nucleic acid sequence or fragment thereof corresponding to SEQ ID 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 95, 97, 99, 101, 103, 105, 107, 109, 111, 113, 115, 117, 119, 121, 123, 125, 127, 129, 131, 133, 135, 137, 139, 143, 145, 147, 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295 and 297.

In a fourteenth aspect, the present invention is directed to the identification of plant genes, more preferably Nicotiana, that share substantial amino acid identity corresponding to the taught nucleic acid sequence. The identification of plant genes including both cDNA and genomic clones, those cDNAs and genomic clones, more preferably from Nicotiana may be accomplished by screening plant cDNA libraries using a nucleic acid probe in conjunction with nucleic acid detection protocols including, but not limited to, nucleic acid hybridization and PCR analysis. The nucleic acid probe may be comprised of nucleic acid sequence or fragment thereof corresponding to SEQ ID 1, 3, 5, 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 95, 97, 99, 101, 103, 105, 107, 109, 111, 113, 115, 117, 119, 121, 123, 125, 127, 129, 131, 133, 135, 137, 139, 143, 145 and 147.

In an alterative fifteenth aspect, cDNA expression libraries that express peptides may be screened using antibodies directed to part or all of the taught amino acid sequence. Such amino acid sequences include SEQ ID 2, 4, 8, 9, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 96, 98, 100, 102, 104, 106, 108, 110, 112, 114, 116, 118, 120, 122, 124, 126, 128, 130, 132, 134, 136, 138, 140, 144, 146, 148.

In a sixteenth important aspect, the present invention is also directed to generation of transgenic Nicotiana lines that have over-expression of p450 enzyme activity levels. In accordance with the invention, these transgenic lines include all nucleic acid sequences encoding the amino acid sequences of full length genes that are effective for increasing the expression of certain enzyme thus resulting in phenotypic effects within Nicotiana. Such amino acid sequences include SEQ. ID. 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296 and 298.

### BRIEF DESCRIPTION OF DRAWINGS

Figure 1 shows nucleic acid SEQ. ID. No.:1 and amino acid SEQ. ID. No.:2.
Figure 2 shows nucleic acid SEQ. ID. No.:3 and amino acid SEQ. ID. No.:4.
Figure 3 shows nucleic acid SEQ. ID. No.:5 and amino acid SEQ. ID. No.:6.
Figure 4 shows nucleic acid SEQ. ID. No.:7 and amino acid SEQ. ID. No.:8.
Figure 5 shows nucleic acid SEQ. ID. No.:9 and amino acid SEQ. ID. No.:10.

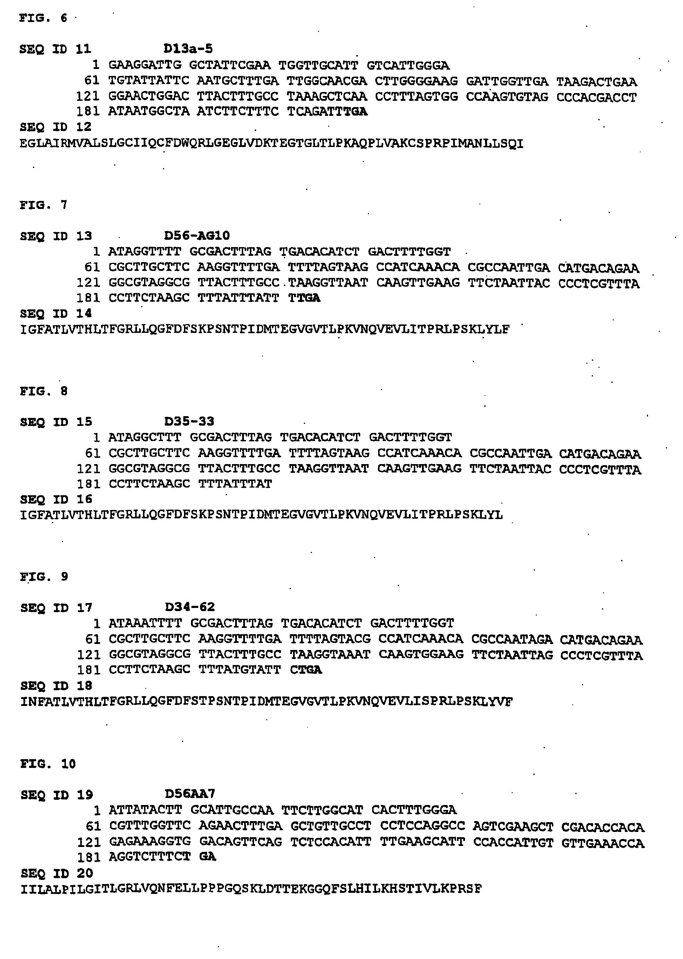

Figure 6 shows nucleic acid SEQ. ID. No.:11 and amino acid SEQ. ID. No.:12.
Figure 7 shows nucleic acid SEQ. ID. No.:13 and amino acid SEQ. ID. No.:14.
Figure 8 shows nucleic acid SEQ. ID. No.:15 and amino acid SEQ. ID. No.:16.
Figure 9 shows nucleic acid SEQ. ID. No.:17 and amino acid SEQ. ID. No.:18.
Figure 10 shows nucleic acid SEQ. ID. No.:19 and amino acid SEQ. ID. No.:20.

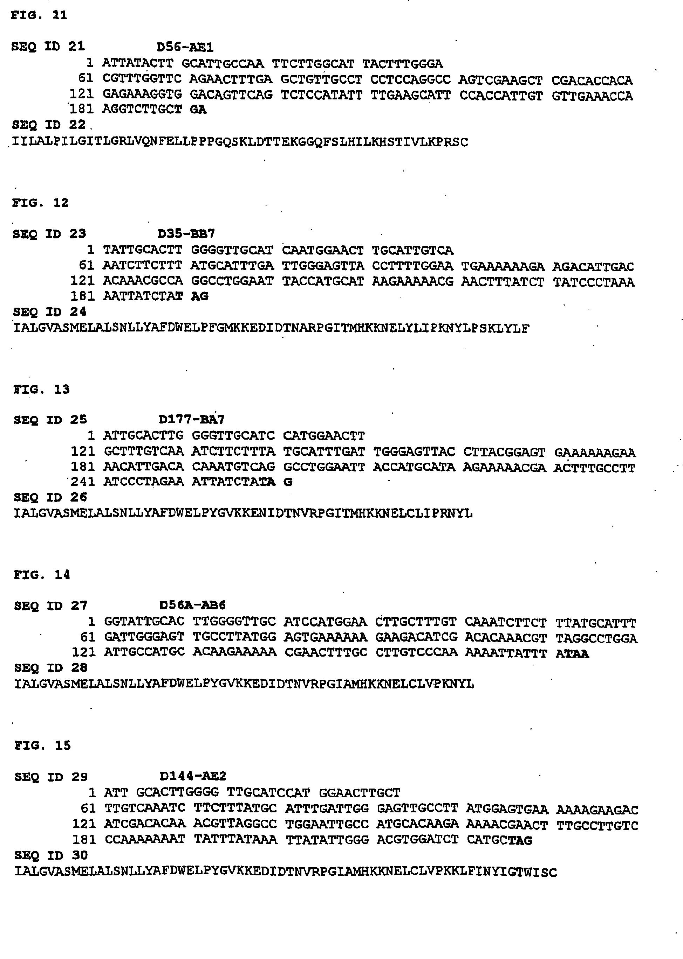

Figure 11 shows nucleic acid SEQ. ID. No.:21 and amino acid SEQ. ID. No.:22.
Figure 12 shows nucleic acid SEQ. ID. No.:23 and amino acid SEQ. ID. No.:24.
Figure 13 shows nucleic acid SEQ. ID. No.:25 and amino acid SEQ. ID. No.:26.
Figure 14 shows nucleic acid SEQ. ID. No.:27 and amino acid SEQ. ID. No.:28.
Figure 15 shows nucleic acid SEQ. ID. No.:29 and amino acid SEQ. ID. No.:30.

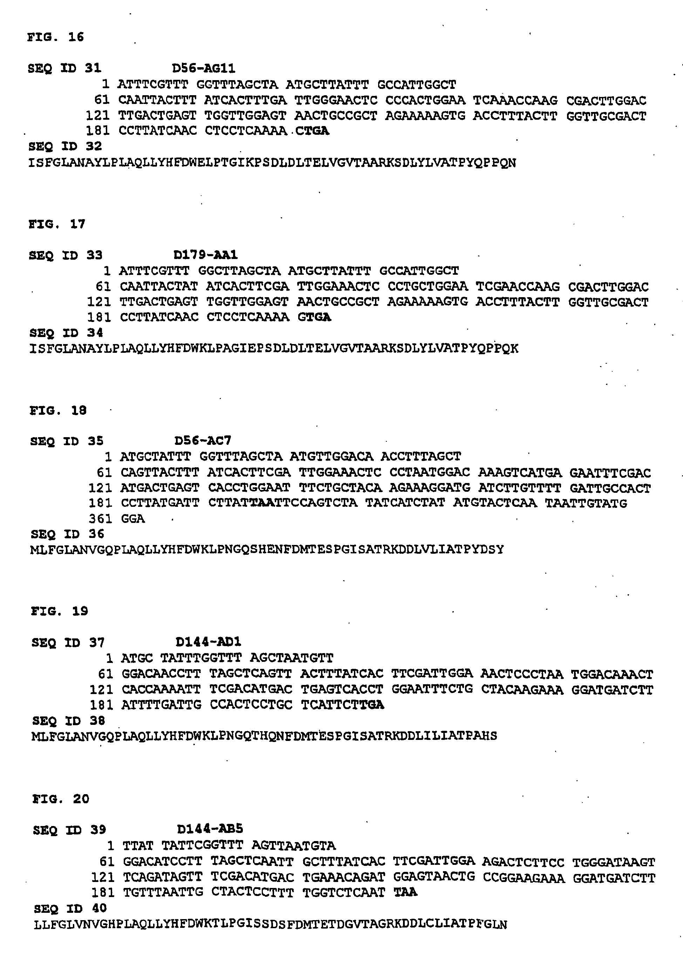

Figure 16 shows nucleic acid SEQ. ID. No.:31 and amino acid SEQ. ID. No.:32.
Figure 17 shows nucleic acid SEQ. ID. No.:33 and amino acid SEQ. ID. No.:34.
Figure 18 shows nucleic acid SEQ. ID. No.:35 and amino acid SEQ. ID. No.:36.
Figure 19 shows nucleic acid SEQ. ID. No.:37 and amino acid SEQ. ID. No.:38.
Figure 20 shows nucleic acid SEQ. ID. No.:39 and amino acid SEQ. ID. No.:40.

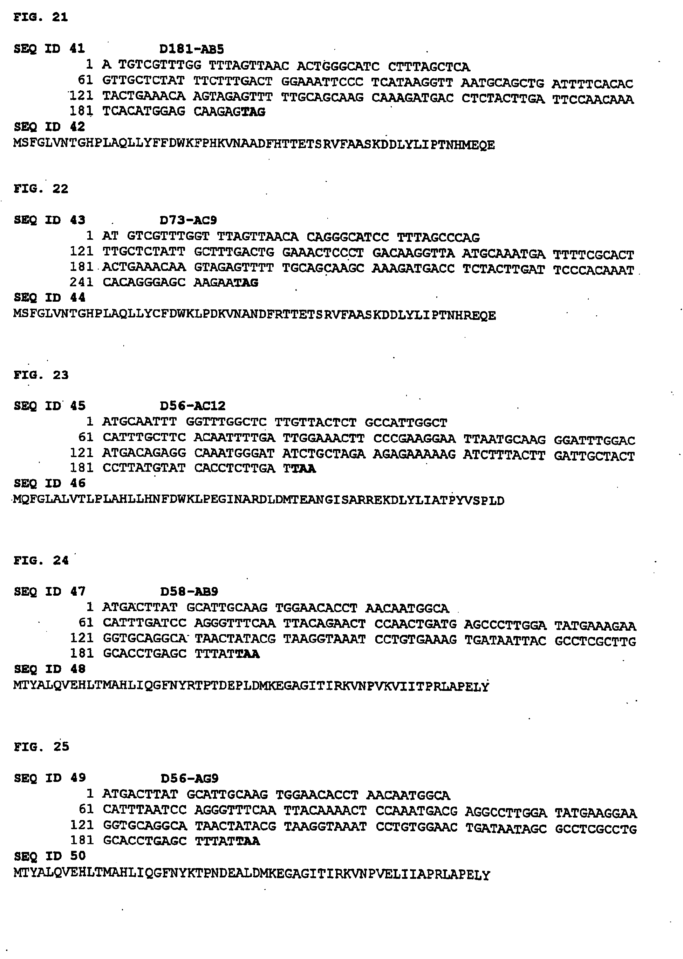

Figure 21 shows nucleic acid SEQ. ID. No.:41 and amino acid SEQ. ID. No.:42.
Figure 22 shows nucleic acid SEQ. ID. No.:43 and amino acid SEQ. ID. No.:44.
Figure 23 shows nucleic acid SEQ. ID. No.:45 and amino acid SEQ. ID. No.:46.
Figure 24 shows nucleic acid SEQ. ID. No.:47 and amino acid SEQ. ID. No.:48.
Figure 25 shows nucleic acid SEQ. ID. No.:49 and amino acid SEQ. ID. No.:50.

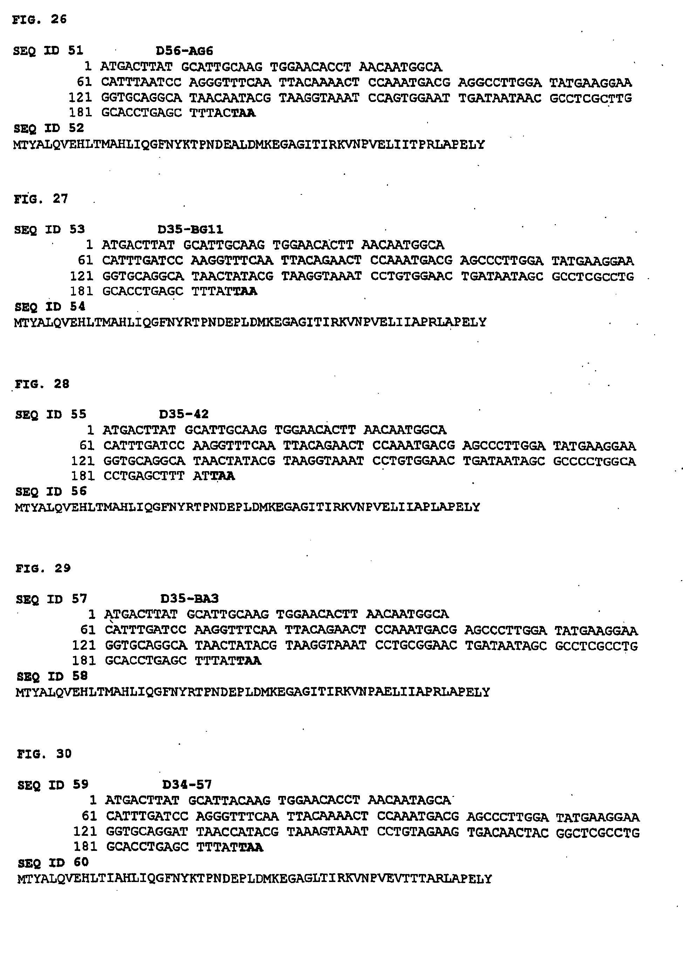

Figure 26 shows nucleic acid SEQ. ID. No.:51 and amino acid SEQ. ID. No.:52.
Figure 27 shows nucleic acid SEQ. ID. No.:53 and amino acid SEQ. ID. No.:54.
Figure 28 shows nucleic acid SEQ. ID. No.:55 and amino acid SEQ. ID. No.:56.
Figure 29 shows nucleic acid SEQ. ID. No.:57 and amino acid SEQ. ID. No.:58.
Figure 30 shows nucleic acid SEQ. ID. No.:59 and amino acid SEQ. ID. No.:60.

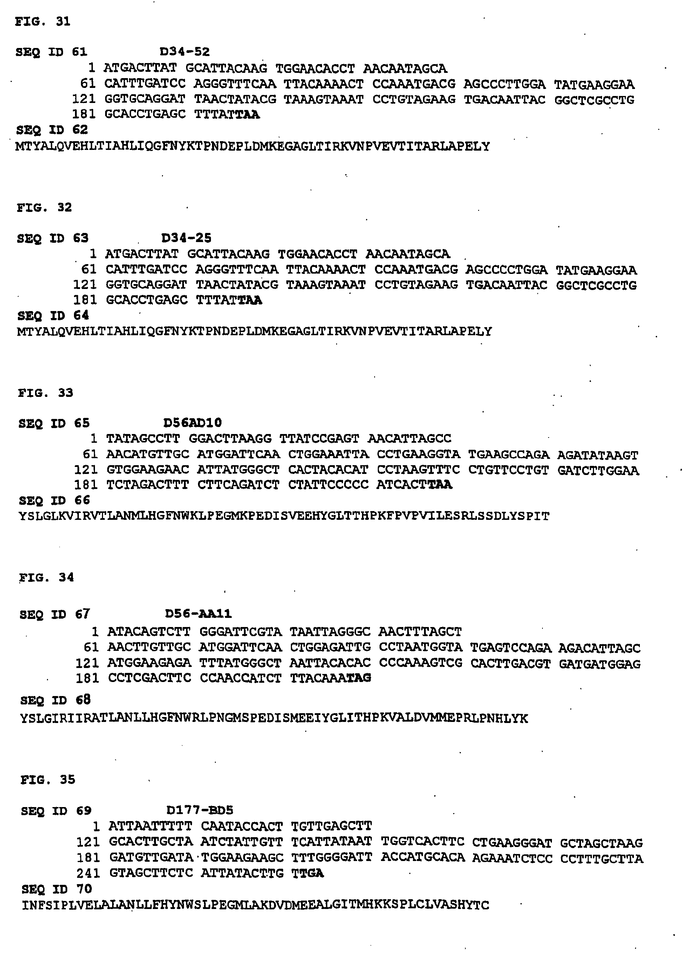

Figure 31 shows nucleic acid SEQ. ID. No.:61 and amino acid SEQ. ID. No.:62.
Figure 32 shows nucleic acid SEQ. ID. No.:63 and amino acid SEQ. ID. No.:64.
Figure 33 shows nucleic acid SEQ. ID. No.:65 and amino acid SEQ. ID. No.:66.
Figure 34 shows nucleic acid SEQ. ID. No.:67 and amino acid SEQ. ID. No.:68.
Figure 35 shows nucleic acid SEQ. ID. No.:69 and amino acid SEQ. ID. No.:70.

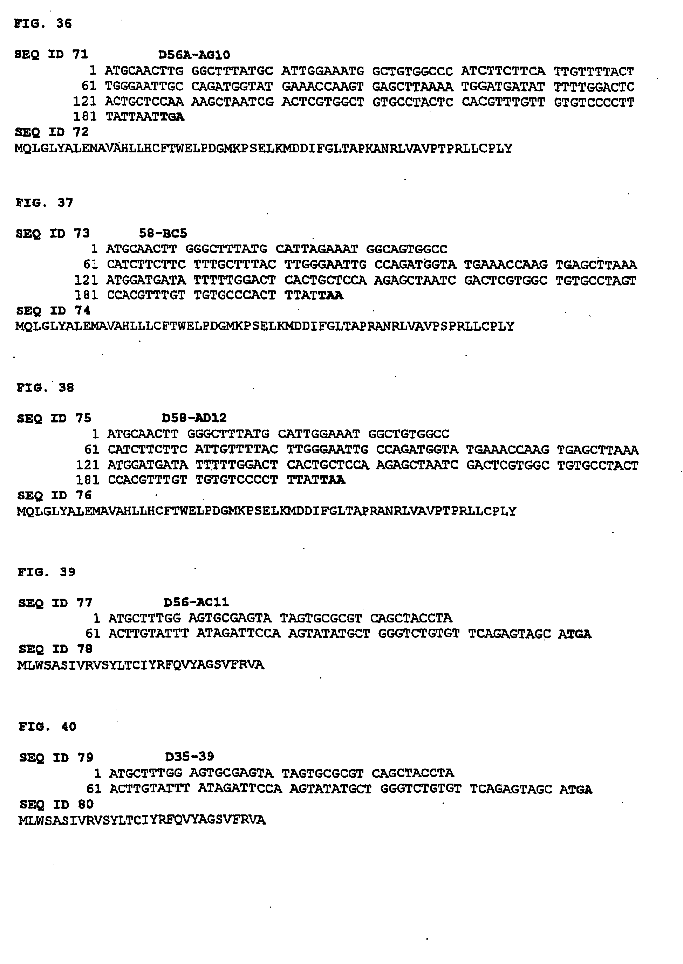

Figure 36 shows nucleic acid SEQ. ID. No.:71 and amino acid SEQ. ID. No.:72.
Figure 37 shows nucleic acid SEQ. ID. No.:73 and amino acid SEQ. ID. No.:74.
Figure 38 shows nucleic acid SEQ. ID. No.:75 and amino acid SEQ. ID. No.:76.
Figure 39 shows nucleic acid SEQ. ID. No.:77 and amino acid SEQ. ID. No.:78.
Figure 40 shows nucleic acid SEQ. ID. No.:79 and amino acid SEQ. ID. No.:80.

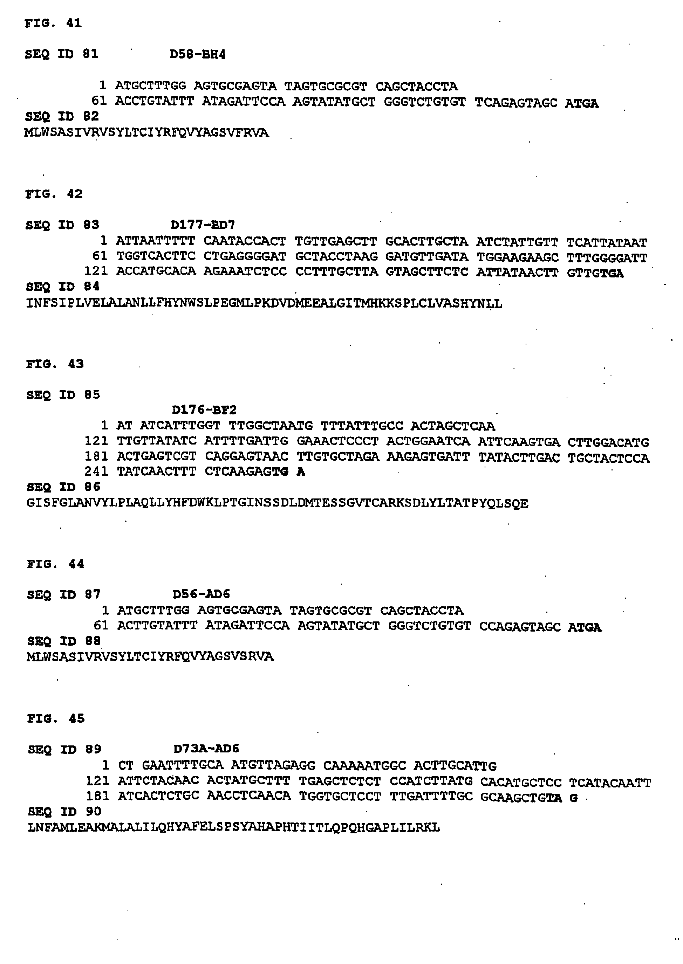

Figure 41 shows nucleic acid SEQ. ID. No.:81 and amino acid SEQ. ID. No.:82.
Figure 42 shows nucleic acid SEQ. ID. No.:83 and amino acid SEQ. ID. No.:84.
Figure 43 shows nucleic acid SEQ. ID. No.:85 and amino acid SEQ. ID. No.:86.
Figure 44 shows nucleic acid SEQ. ID. No.:87 and amino acid SEQ. ID. No.:88.
Figure 45 shows nucleic acid SEQ. ID. No.:89 and amino acid SEQ. ID. No.:90.

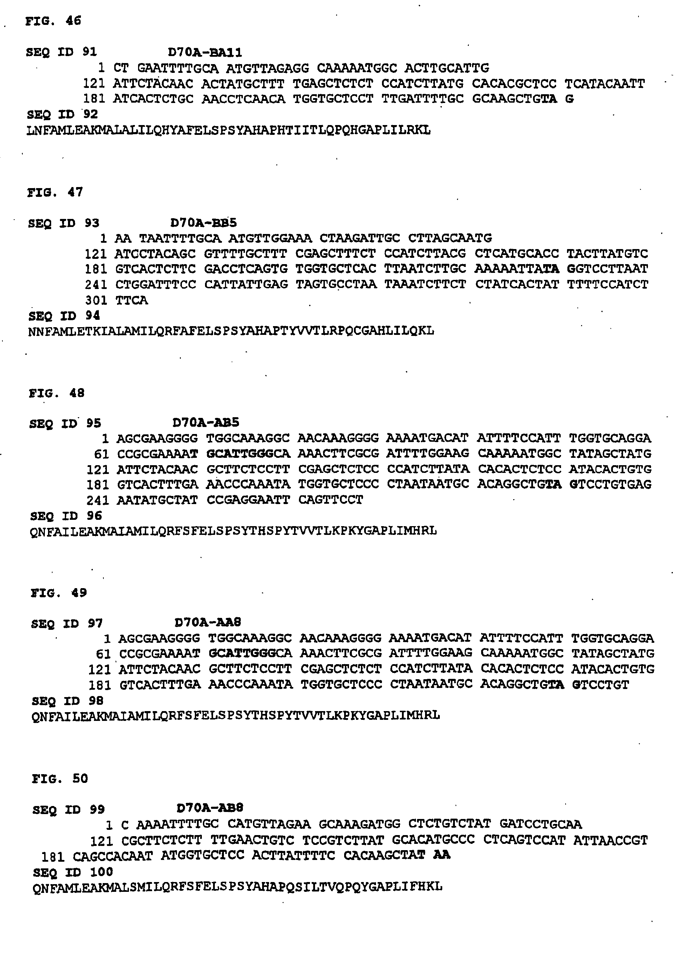

Figure 46 shows nucleic acid SEQ. ID. No.:91 and amino acid SEQ. ID. No.:92.
Figure 48 shows nucleic acid SEQ. ID. No.:95 and amino acid SEQ. ID. No.:96.
Figure 49 shows nucleic acid SEQ. ID. No.:97 and amino acid SEQ. ID. No.:98.
Figure 50 shows nucleic acid SEQ. ID. No.:99 and amino acid SEQ. ID. No.:100.

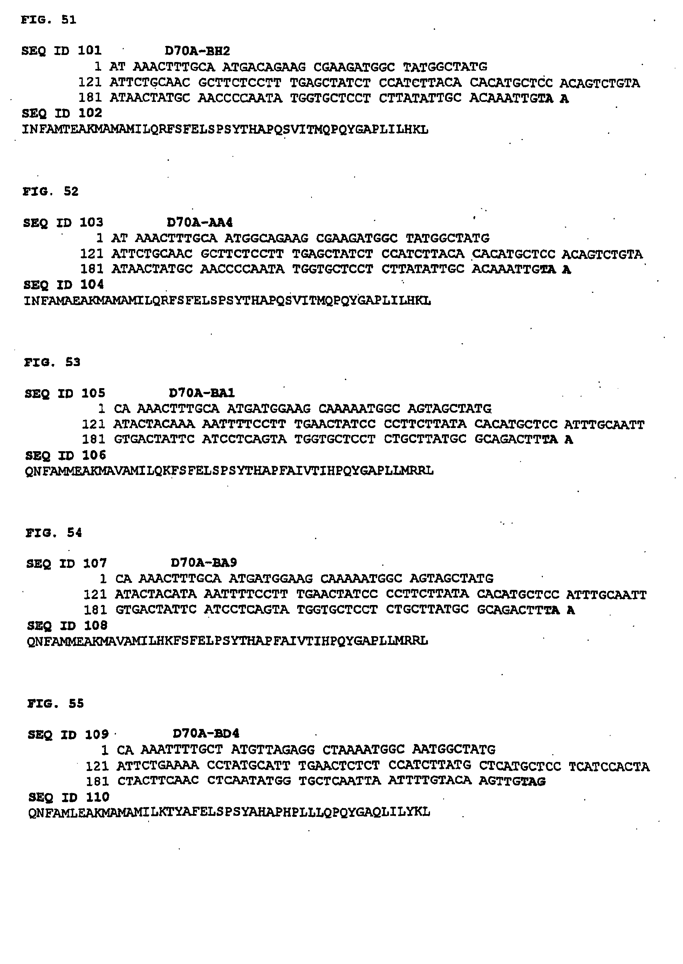

Figure 51 shows nucleic acid SEQ. ID. No.:101 and amino acid SEQ. ID. No.:102.
Figure 52 shows nucleic acid SEQ. ID. No.:103 and amino acid SEQ. ID. No.:104.
Figure 53 shows nucleic acid SEQ. ID. No.:105 and amino acid SEQ. ID. No.:106.
Figure 54 shows nucleic acid SEQ. ID. No.:107 and amino acid SEQ. ID. No.:108.
Figure 55 shows nucleic acid SEQ. ID. No.:109 and amino acid SEQ. ID. No.:110.

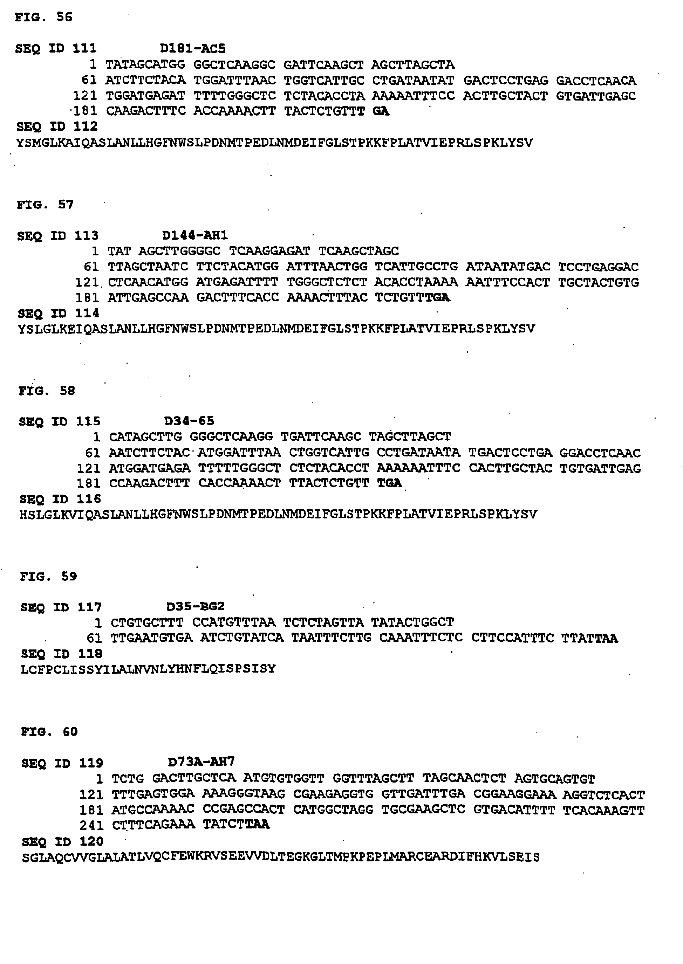

Figure 56 shows nucleic acid SEQ. ID. No.:111 and amino acid SEQ. ID. No.:112.
Figure 57 shows nucleic acid SEQ. ID. No.:113 and amino acid SEQ. ID. No.:114.
Figure 58 shows nucleic acid SEQ. ID. No.:115 and amino acid SEQ. ID. No.:116.
Figure 59 shows nucleic acid SEQ. ID. No.:117 and amino acid SEQ. ID. No.:118.
Figure 60 shows nucleic acid SEQ. ID. No.:119 and amino acid SEQ. ID. No.:120.

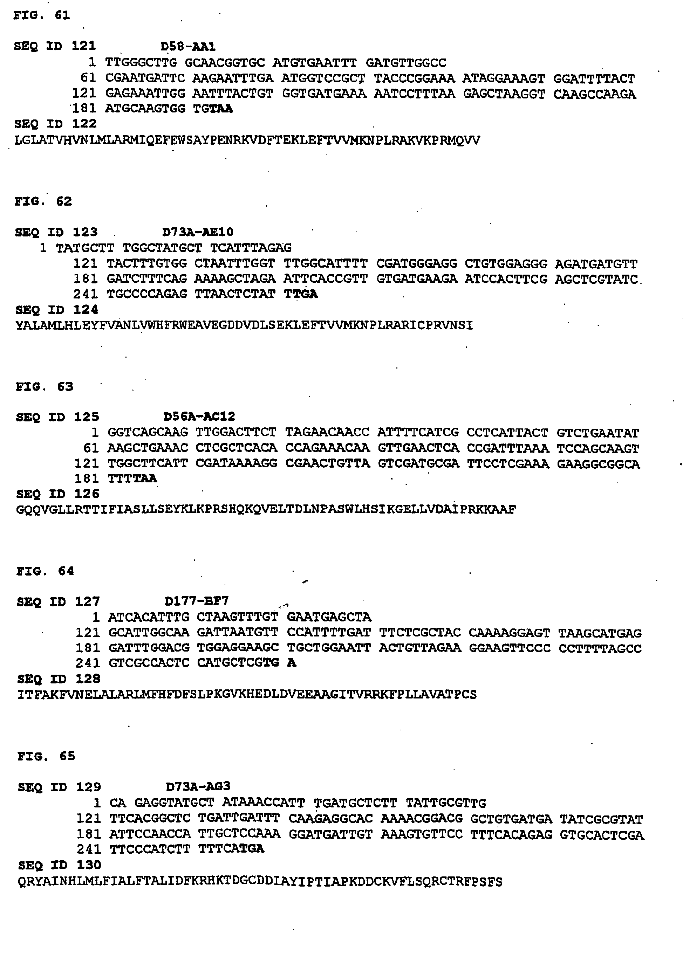

Figure 61 shows nucleic acid SEQ. ID. No.:121 and amino acid SEQ. ID. No.:122.
Figure 62 shows nucleic acid SEQ. ID. No.:123 and amino acid SEQ. ID. No.:124.
Figure 63 shows nucleic acid SEQ. ID. No.:125 and amino acid SEQ. ID. No.:126.
Figure 64 shows nucleic acid SEQ. ID. No.:127 and amino acid SEQ. ID. No.:128.
Figure 65 shows nucleic acid SEQ. ID. No.:129 and amino acid SEQ. ID. No.:130.

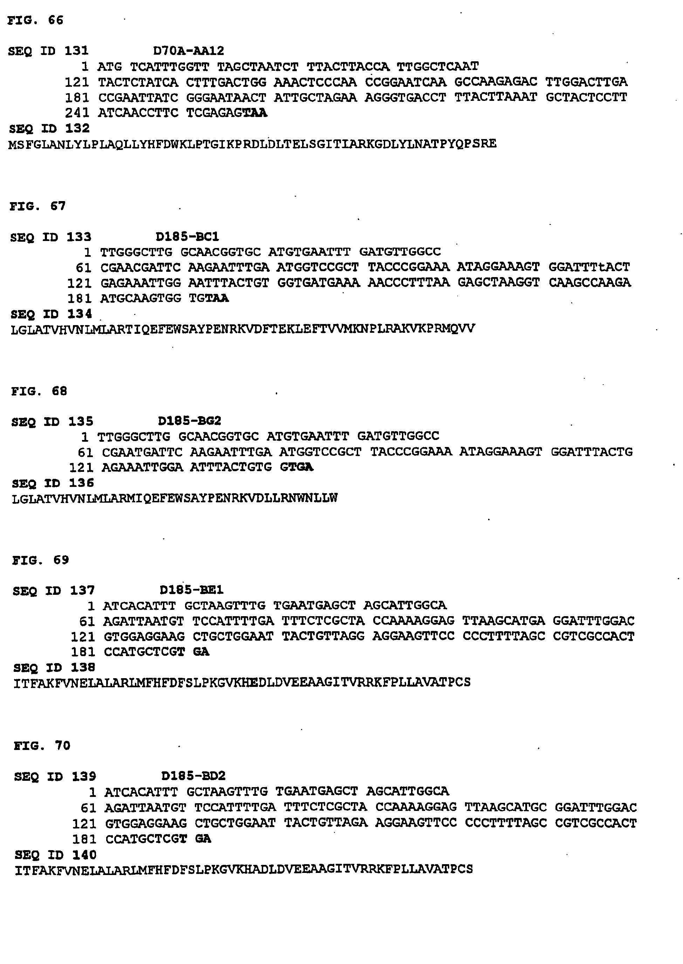

Figure 66 shows nucleic acid SEQ. ID. No.:131 and amino acid SEQ. ID. No.:132.
Figure 67 shows nucleic acid SEQ. ID. No.:133 and amino acid SEQ. ID. No.:134.
Figure 68 shows nucleic acid SEQ. ID. No.:135 and amino acid SEQ. ID. No.:136.
Figure 69 shows nucleic acid SEQ. ID. No.:137 and amino acid SEQ. ID. No.:138.
Figure 70 shows nucleic acid SEQ. ID. No.:139 and amino acid SEQ. ID. No.:140.

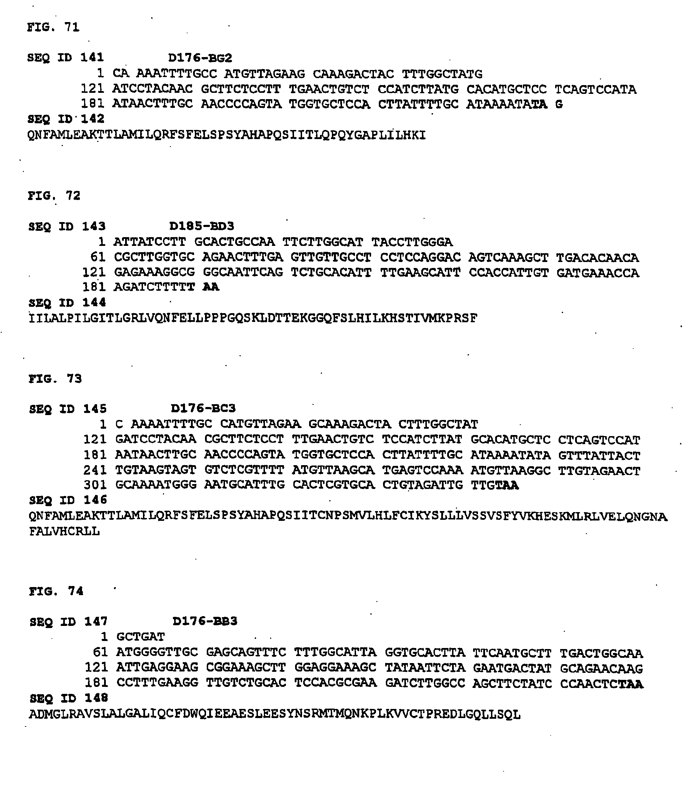

Figure 72 shows nucleic acid SEQ. ID. No.:143 and amino acid SEQ. ID. No.:144.
Figure 73 shows nucleic acid SEQ. ID. No.:145 and amino acid SEQ. ID. No.:146.
Figure 74 shows nucleic acid SEQ. ID. No.:147 and amino acid SEQ. ID. No.:148.
Figure 75 shows nucleic acid SEQ. ID No.: 149 and amino acid SEQ. ID. No.: 150.
Figure 76 shows nucleic acid SEQ. ID No.: 151 and amino acid SEQ. ID. No.: 152.
Figure 77 shows nucleic acid SEQ. ID No.: 153 and amino acid SEQ. ID. No.: 154.

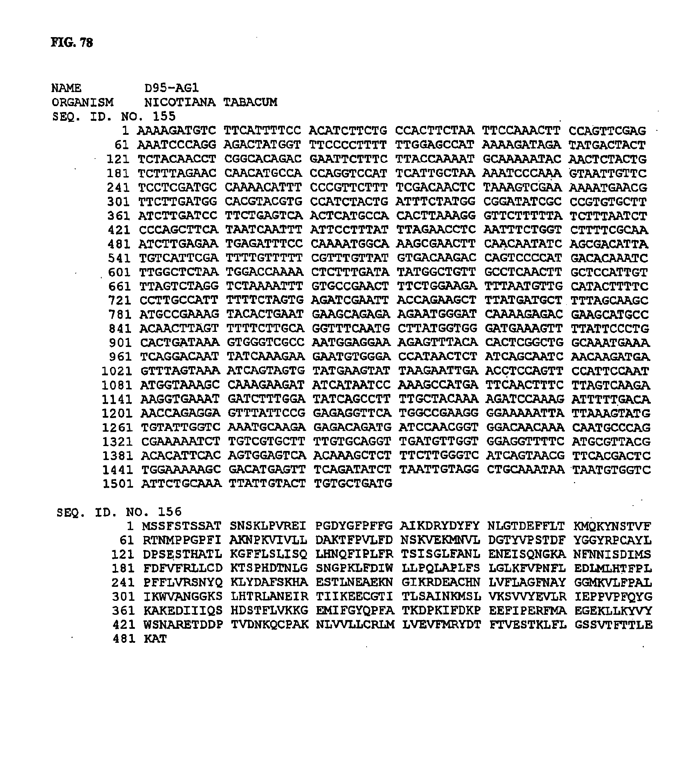

Figure 78 shows nucleic acid SEQ. ID No.: 155 and amino acid SEQ. ID. No.: 156.

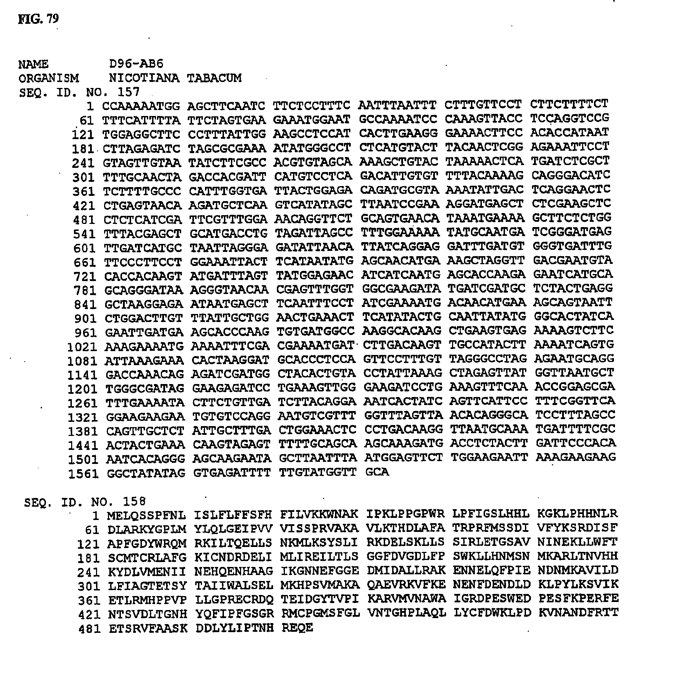

Figure 79 shows nucleic acid SEQ. ID No.: 157 and amino acid SEQ. ID. No.: 158.

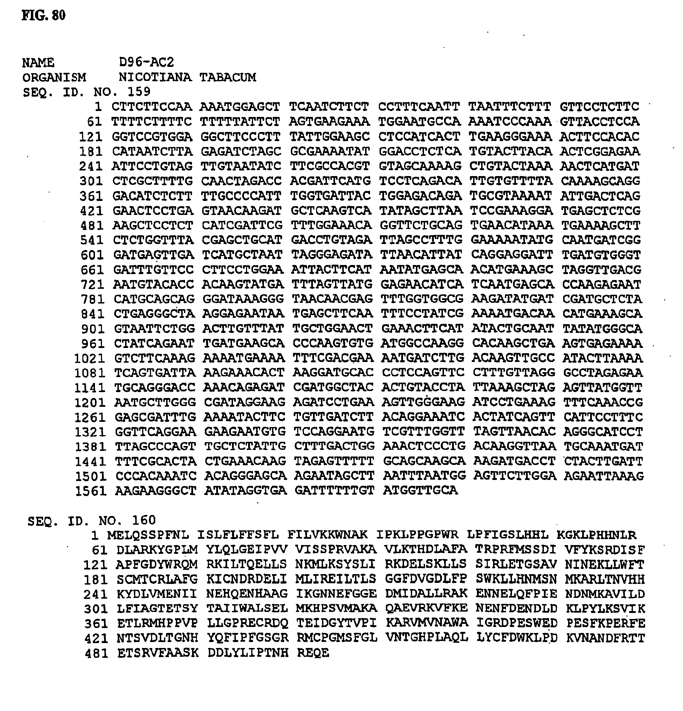

Figure 80 shows nucleic acid SEQ. ID No.: 159 and amino acid SEQ. ID. No.: 160.

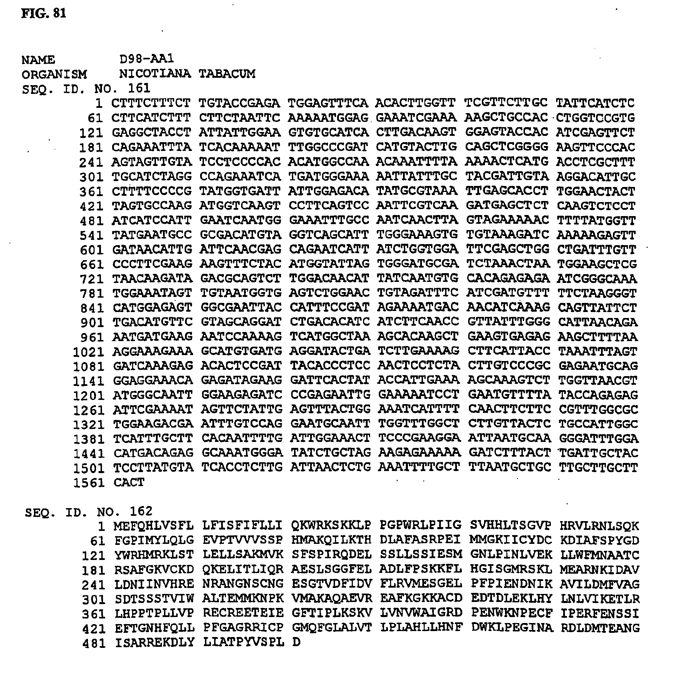

Figure 81 shows nucleic acid SEQ. ID No.: 161 and amino acid SEQ. ID. No.: 162.

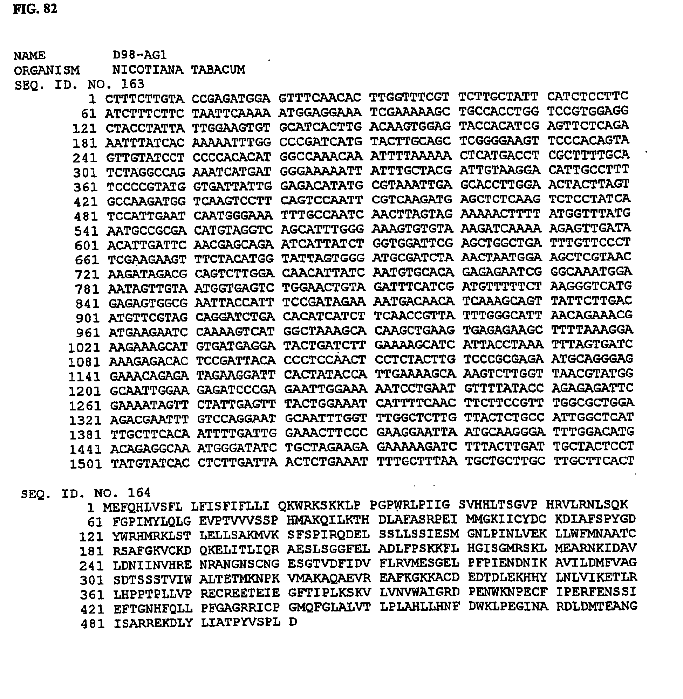

Figure 82 shows nucleic acid SEQ. ID No.: 163 and amino acid SEQ. ID. No.: 164.

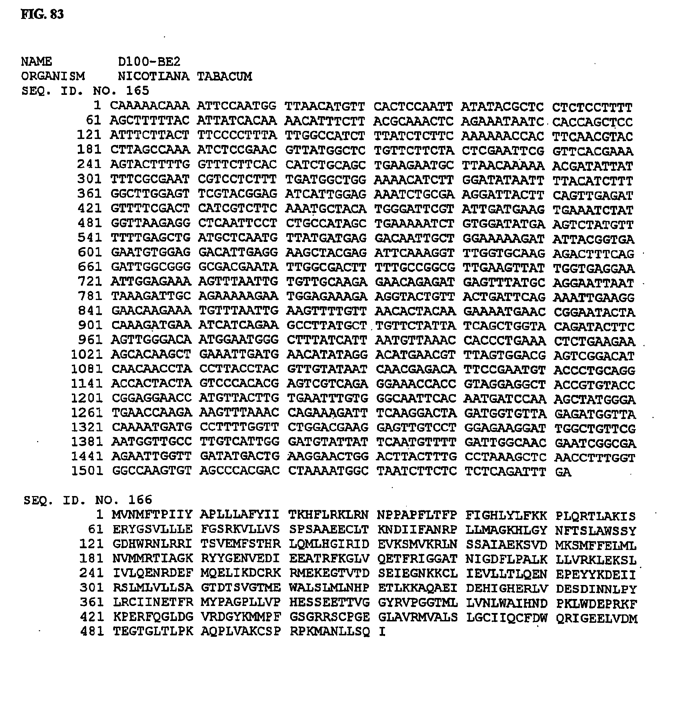

Figure 83 shows nucleic acid SEQ. ID No.: 165 and amino acid SEQ. ID. No.: 166.

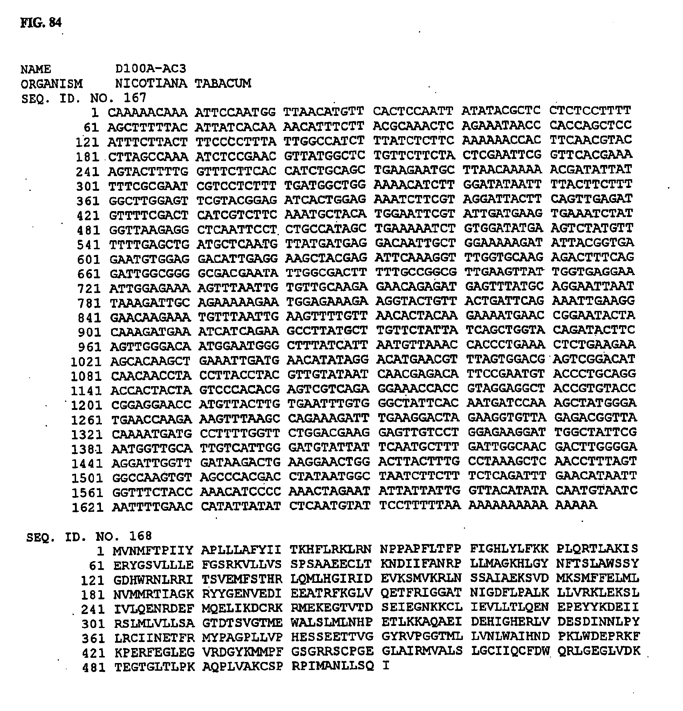

Figure 84 shows nucleic acid SEQ. ID No.: 167 and amino acid SEQ. ID. No.: 168.

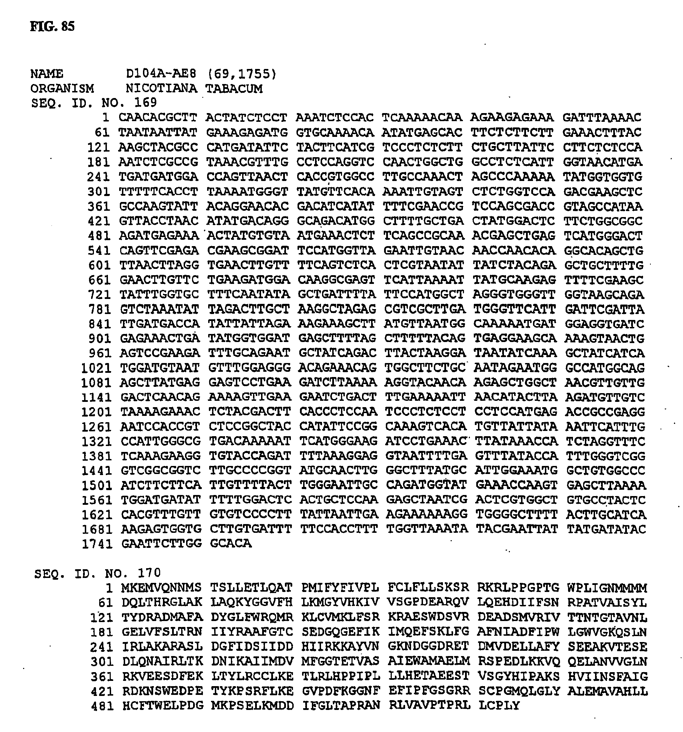

Figure 85 shows nucleic acid SEQ. ID No.: 169 and amino acid SEQ. ID. No.: 170.

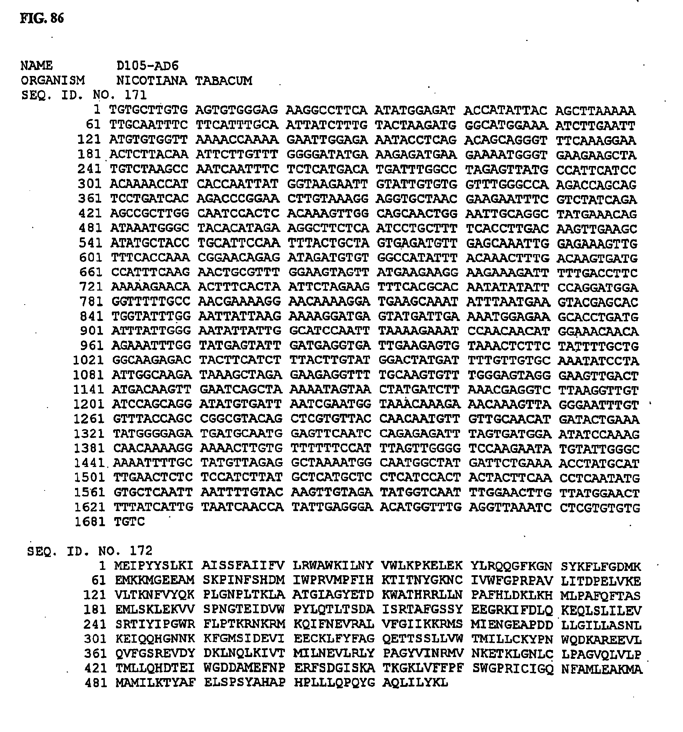

Figure 86 shows nucleic acid SEQ. ID No.: 171 and amino acid SEQ. ID. No.: 172.

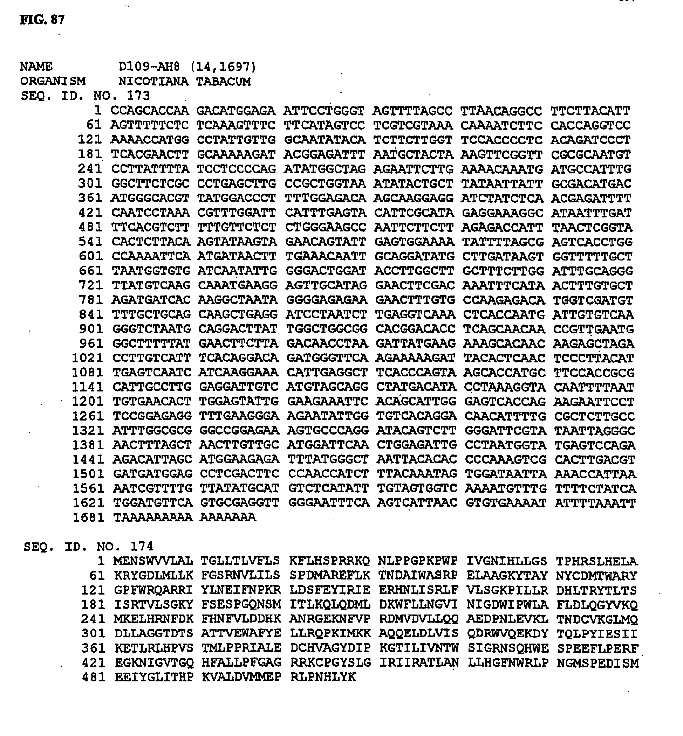

Figure 87 shows nucleic acid SEQ. ID No.: 173 and amino acid SEQ. ID. No.: 174.

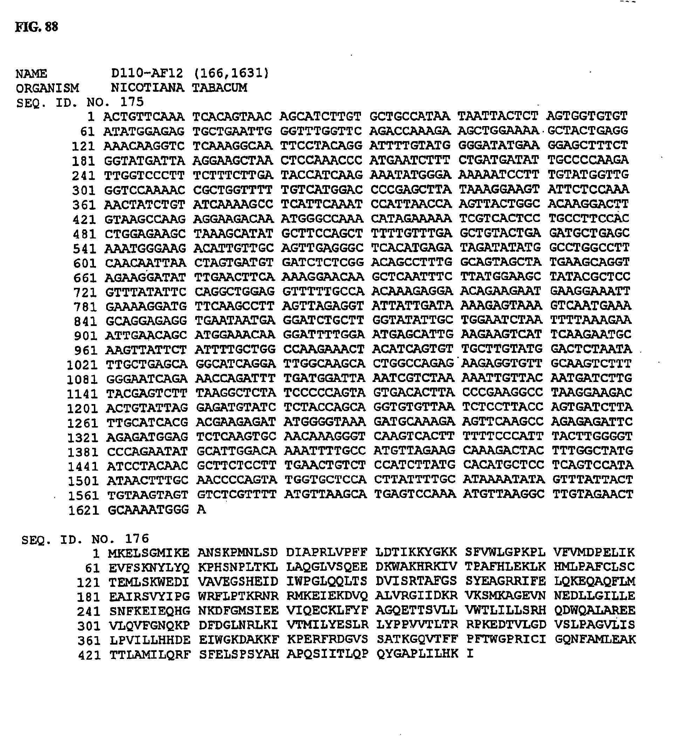

Figure 88 shows nucleic acid SEQ. ID No.: 175 and amino acid SEQ. ID. No.: 176.

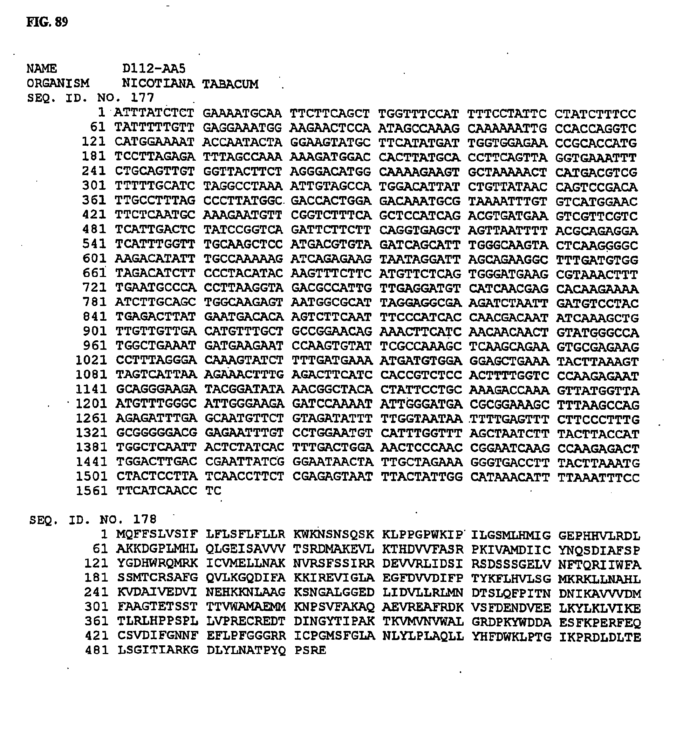

Figure 89 shows nucleic acid SEQ. ID No.: 177 and amino acid SEQ. ID. No.: 178.

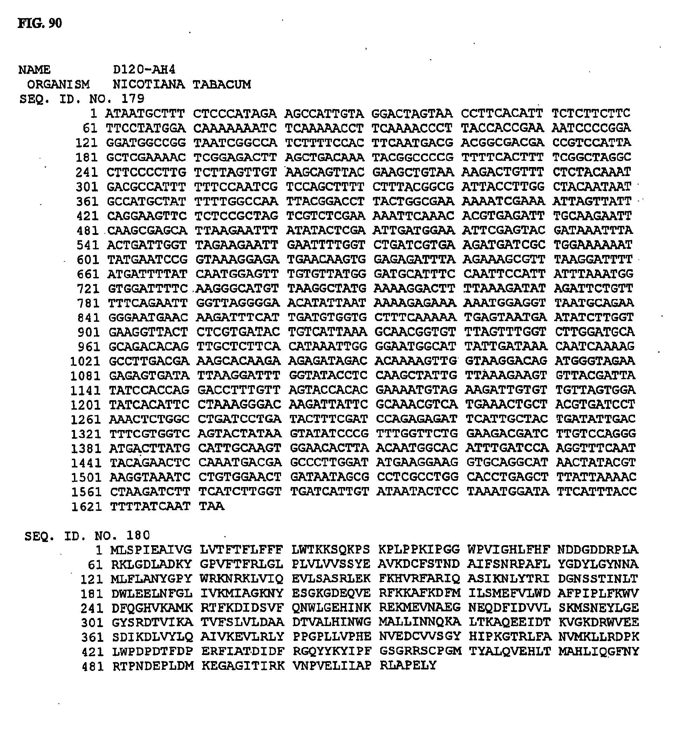

Figure 90 shows nucleic acid SEQ. ID No.: 179 and amino acid SEQ. ID. No.: 180.

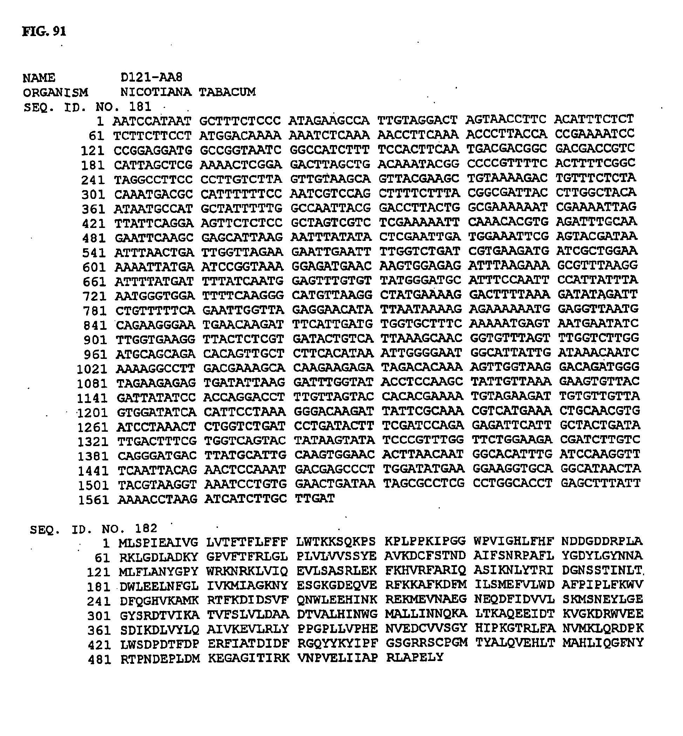

Figure 91 shows nucleic acid SEQ. ID No.: 181 and amino acid SEQ. ID. No.: 182.

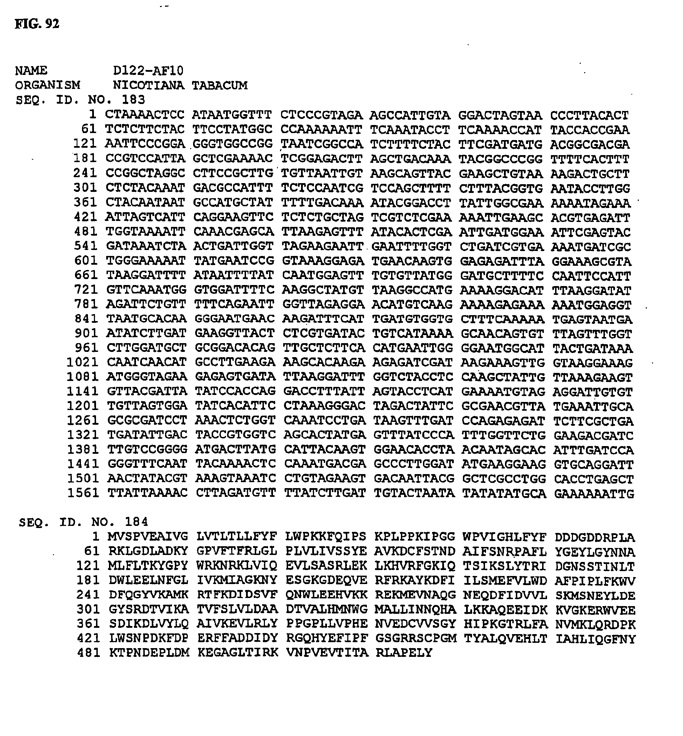

Figure 92 shows nucleic acid SEQ. ID No.: 183 and amino acid SEQ. ID. No.: 184.

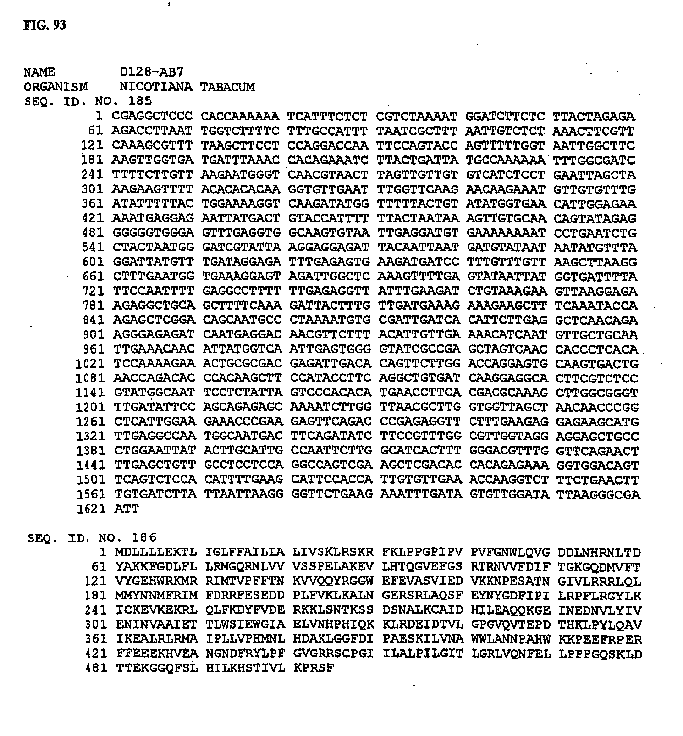

Figure 93 shows nucleic acid SEQ. ID No.: 185 and amino acid SEQ. ID. No.: 186.

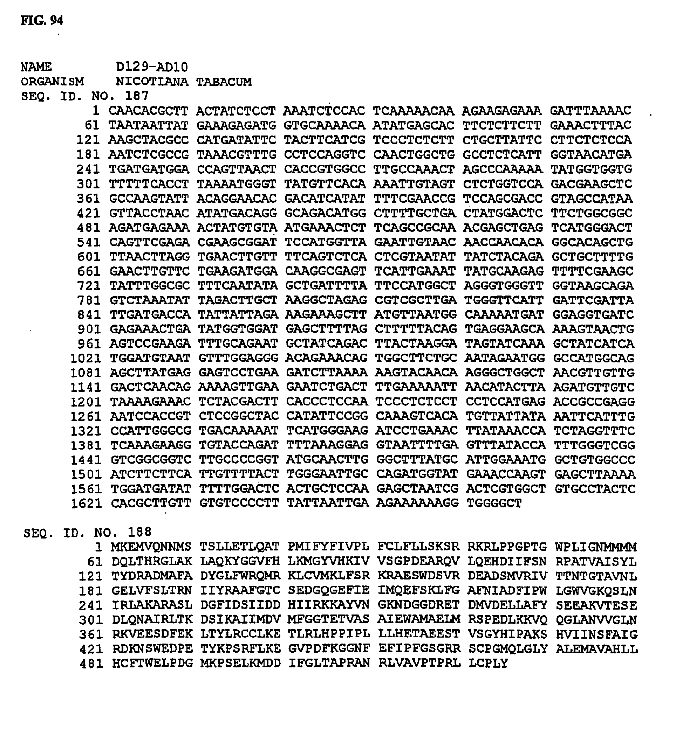

Figure 94 shows nucleic acid SEQ. ID No.: 187 and amino acid SEQ. ID. No.: 188.

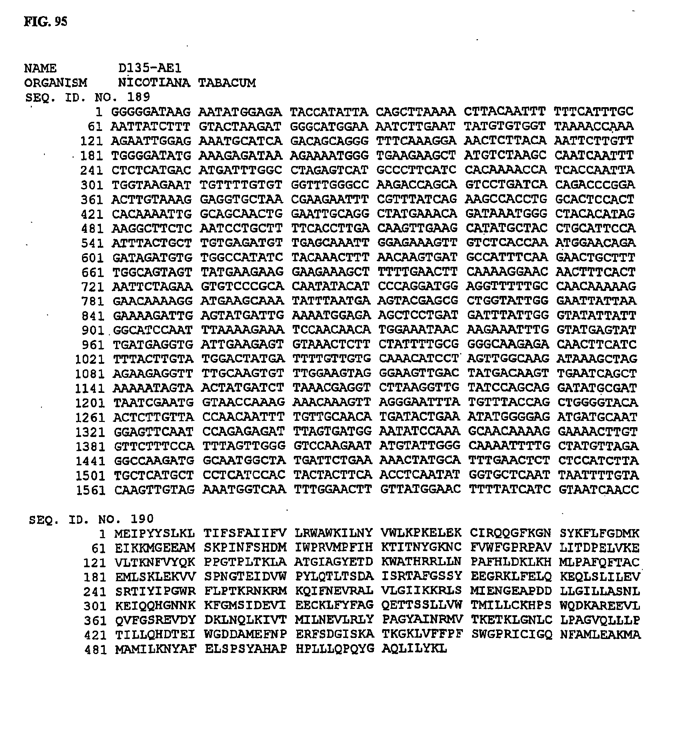

Figure 95 shows nucleic acid SEQ. ID No.: 189 and amino acid SEQ. ID. No.: 190.

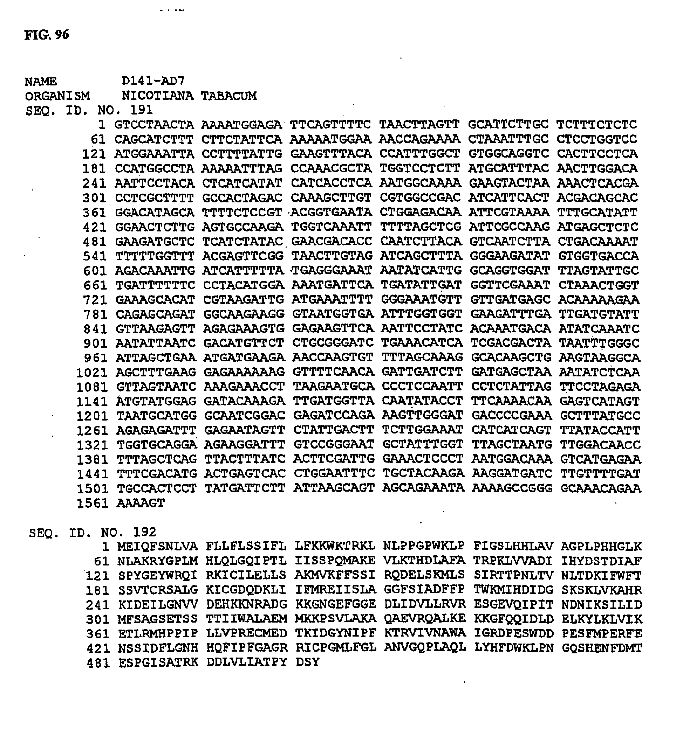

Figure 96 shows nucleic acid SEQ. ID No.: 191 and amino acid SEQ. ID. No.: 192.

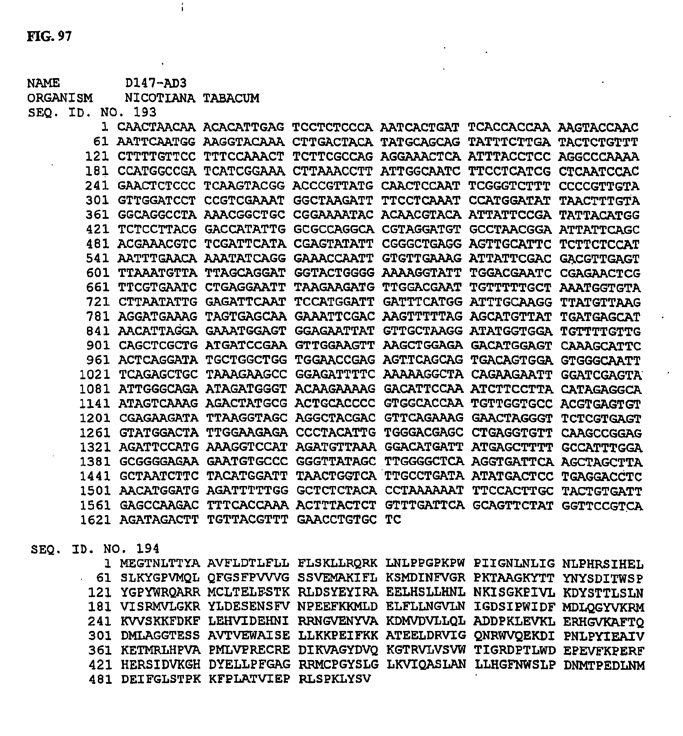

Figure 97 shows nucleic acid SEQ. ID No.: 193 and amino acid SEQ. ID. No.: 194.

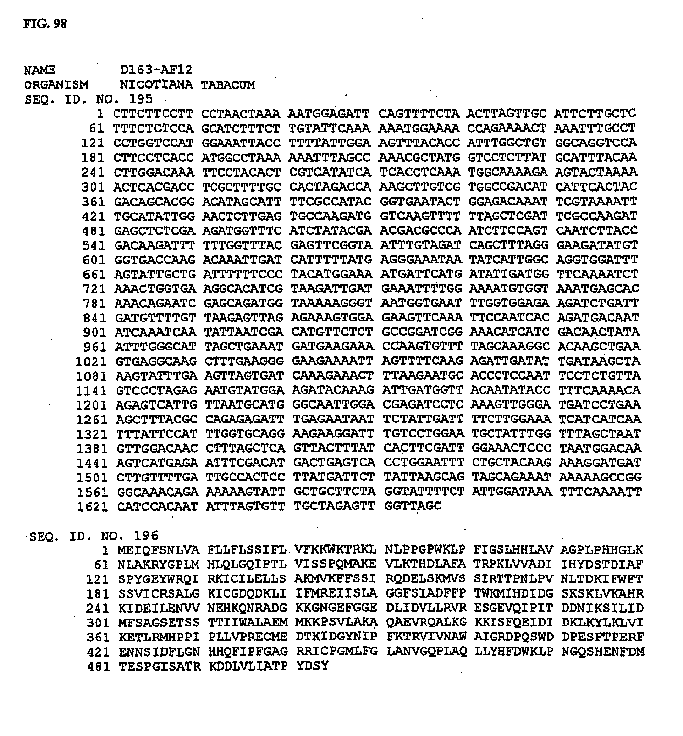

Figure 98 shows nucleic acid SEQ. ID No.: 195 and amino acid SEQ. ID. No.: 196.

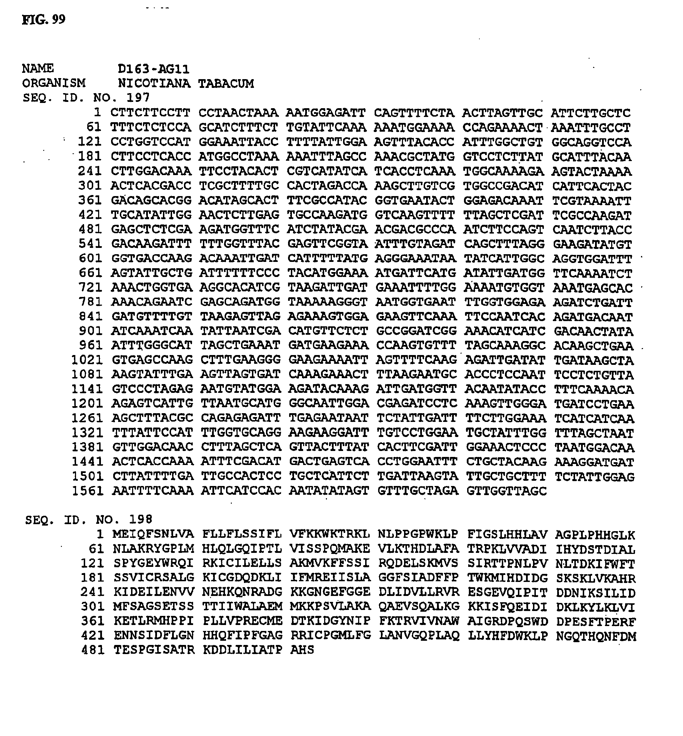

Figure 99 shows nucleic acid SEQ. ID No.: 197 and amino acid SEQ. ID. No.: 198.

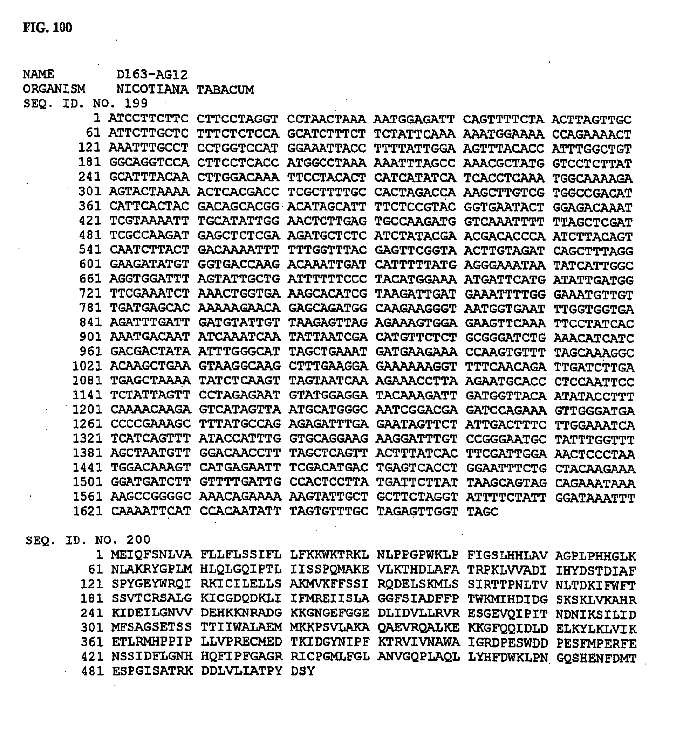

Figure 100 shows nucleic acid SEQ. ID No.: 199 and amino acid SEQ. ID. No.: 200.

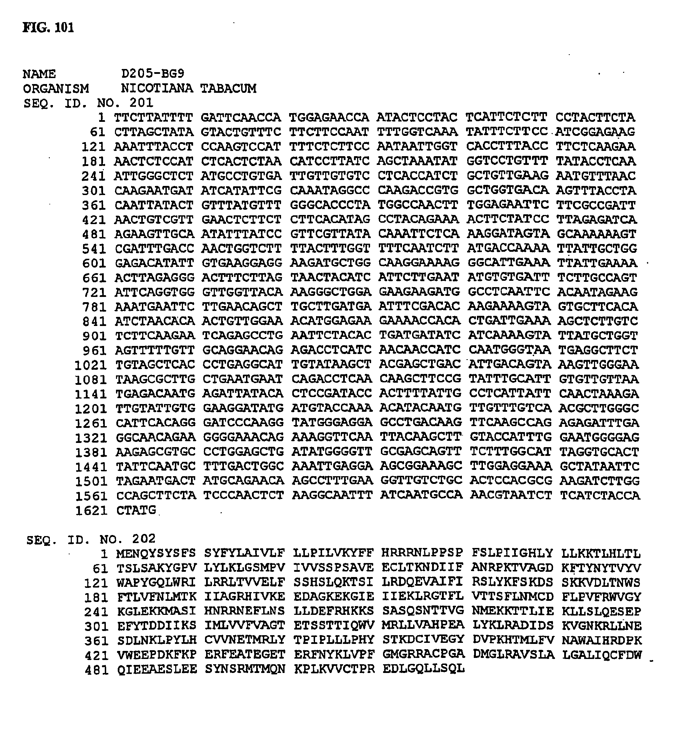

Figure 101 shows nucleic acid SEQ. ID No.: 201 and amino acid SEQ. ID. No.: 202.

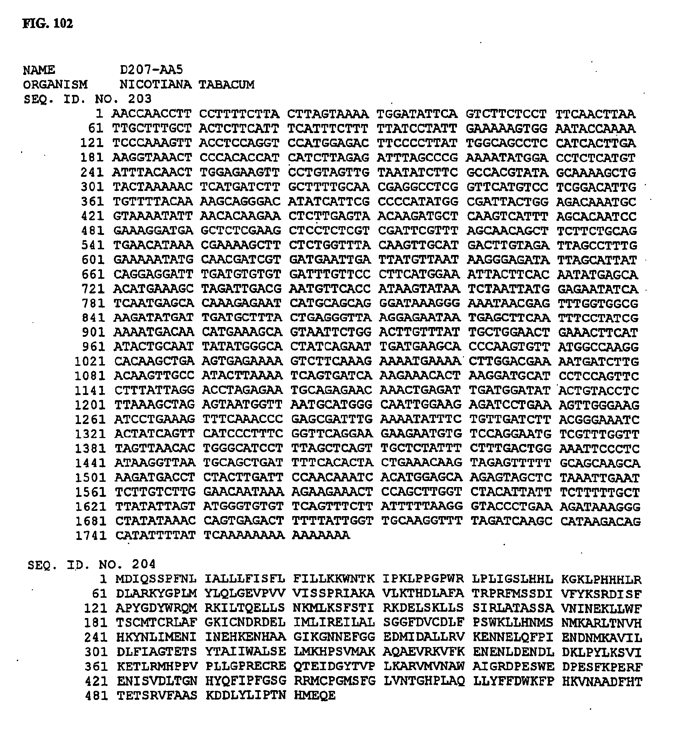

Figure 102 shows nucleic acid SEQ. ID No.: 203 and amino acid SEQ. ID. No.: 204.

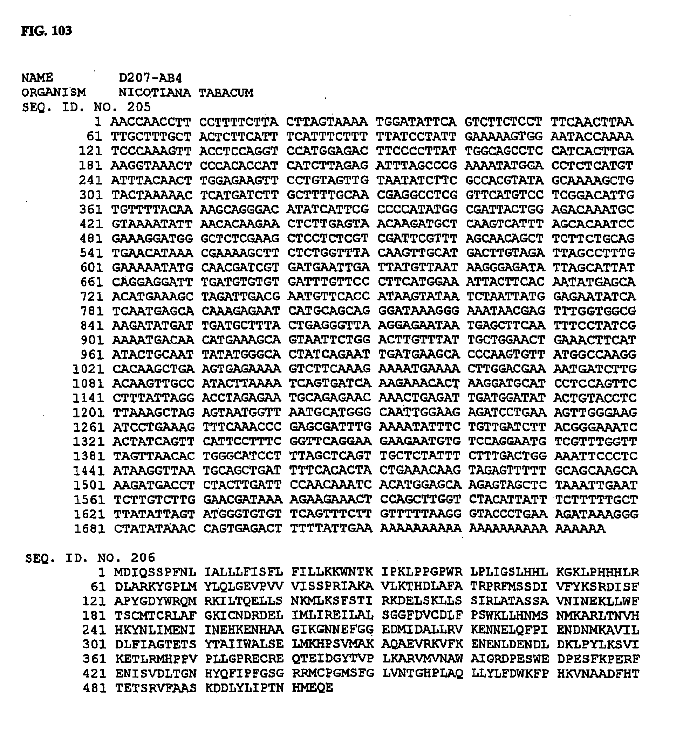

Figure 103 shows nucleic acid SEQ. ID No.: 205 and amino acid SEQ. ID. No.: 206.

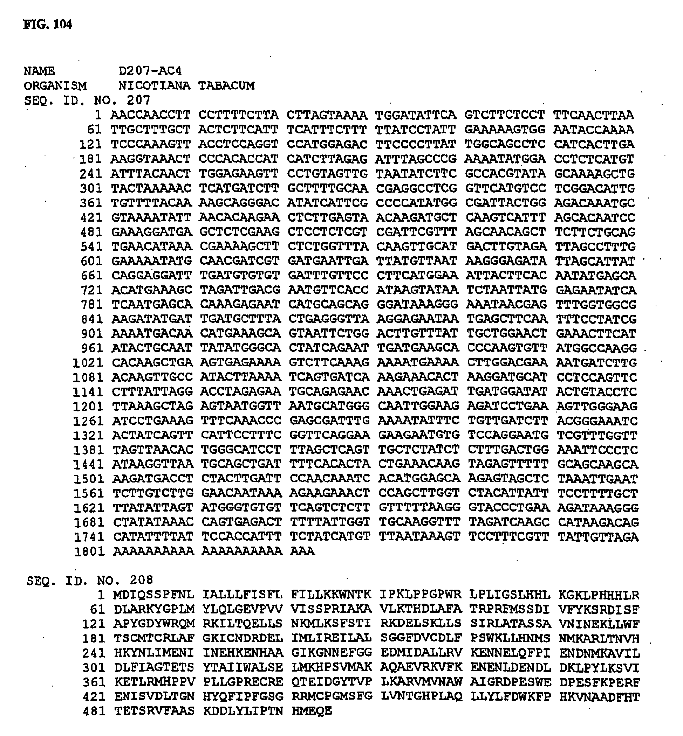

Figure 104 shows nucleic acid SEQ. ID No.: 207 and amino acid SEQ. ID. No.: 208.

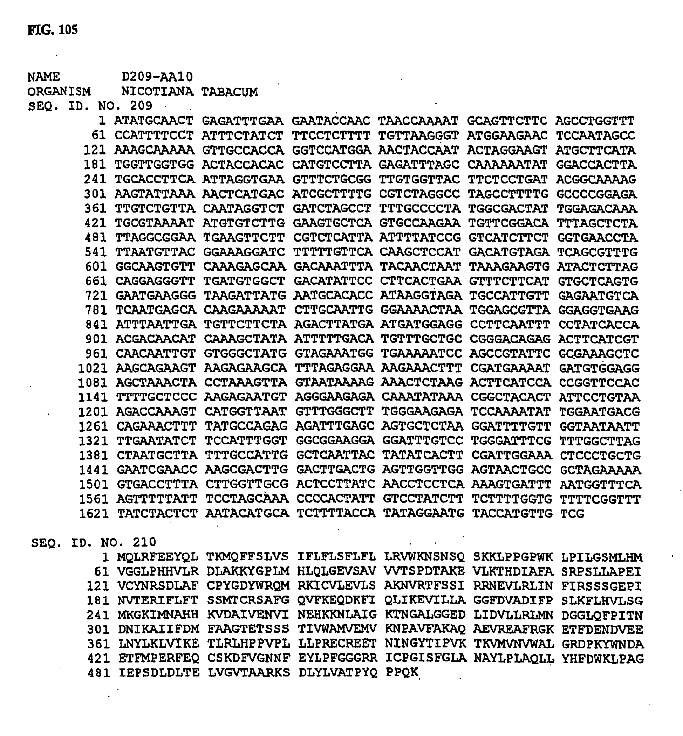

Figure 105 shows nucleic acid SEQ. ID No.: 209 and amino acid SEQ. ID. No.: 210.

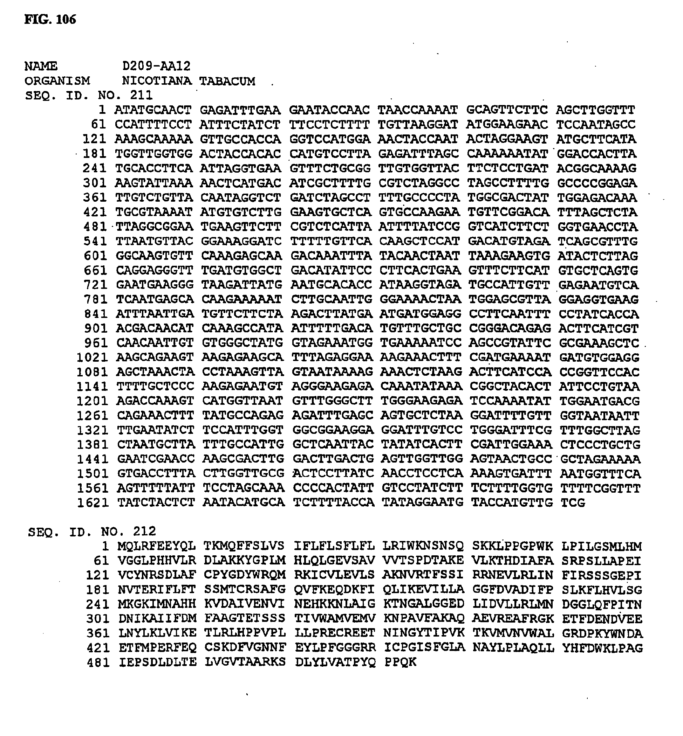

Figure 106 shows nucleic acid SEQ. ID No.: 211 and amino acid SEQ. ID. No.: 212.

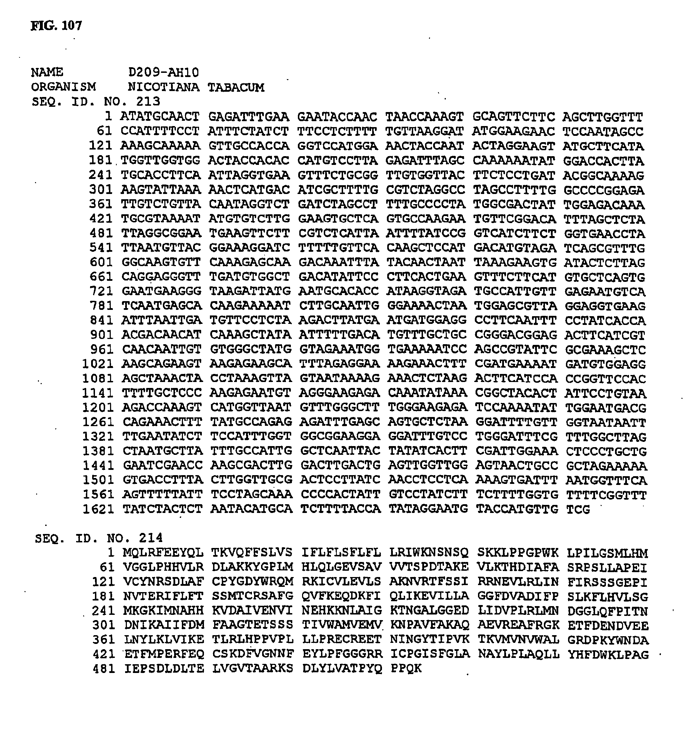

Figure 107 shows nucleic acid SEQ. ID No.: 213 and amino acid SEQ. ID. No.: 214.

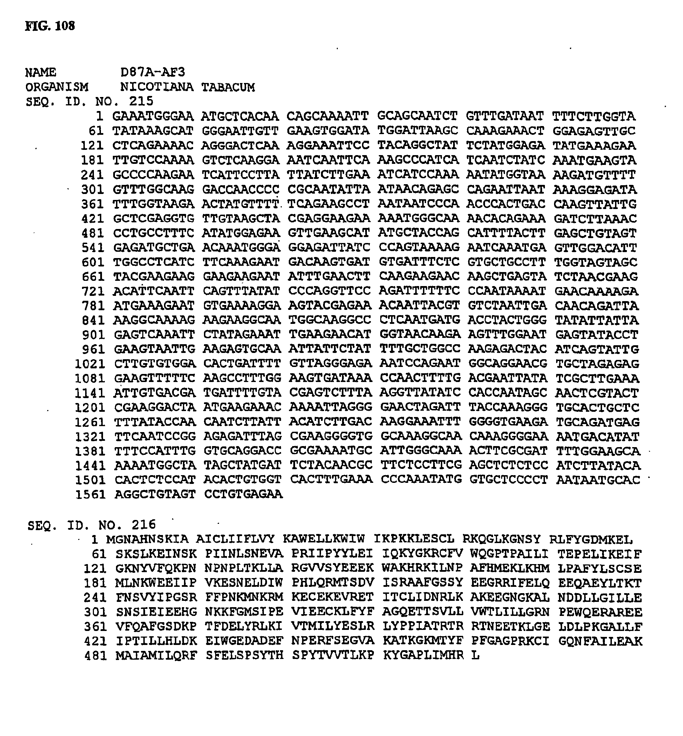

Figure 108 shows nucleic acid SEQ. ID No.: 215 and amino acid SEQ. ID. No.: 216.

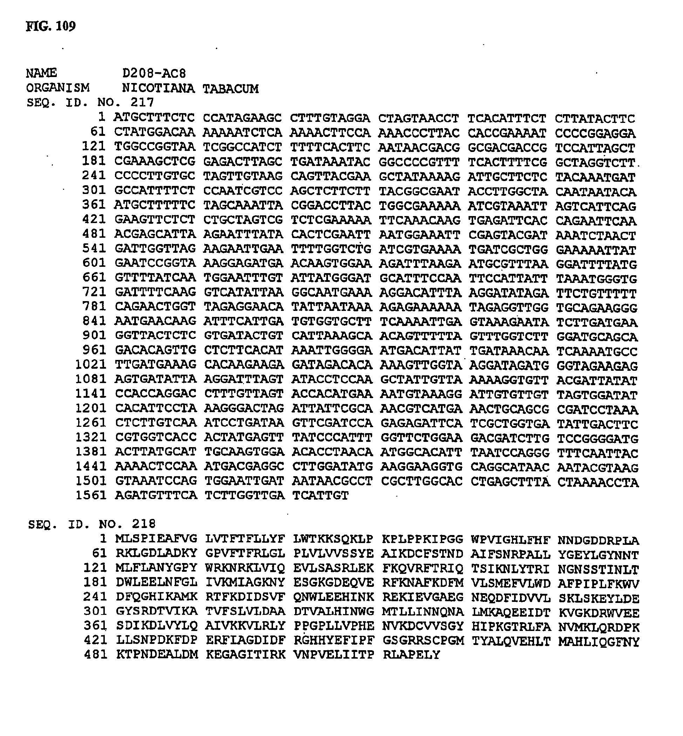

Figure 109 shows nucleic acid SEQ. ID No.: 217 and amino acid SEQ. ID. No.: 218.

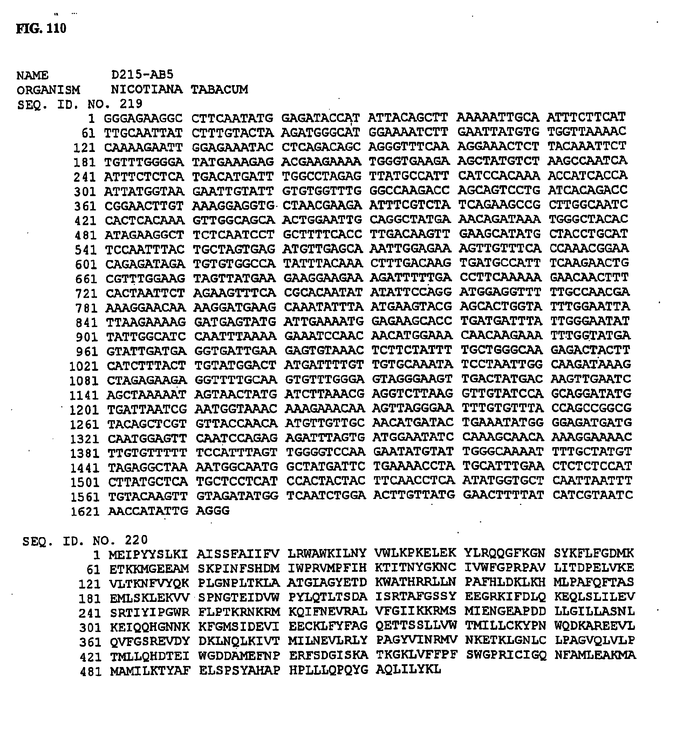

Figure 110 shows nucleic acid SEQ. ID No.: 219 and amino acid SEQ. ID. No.: 220.

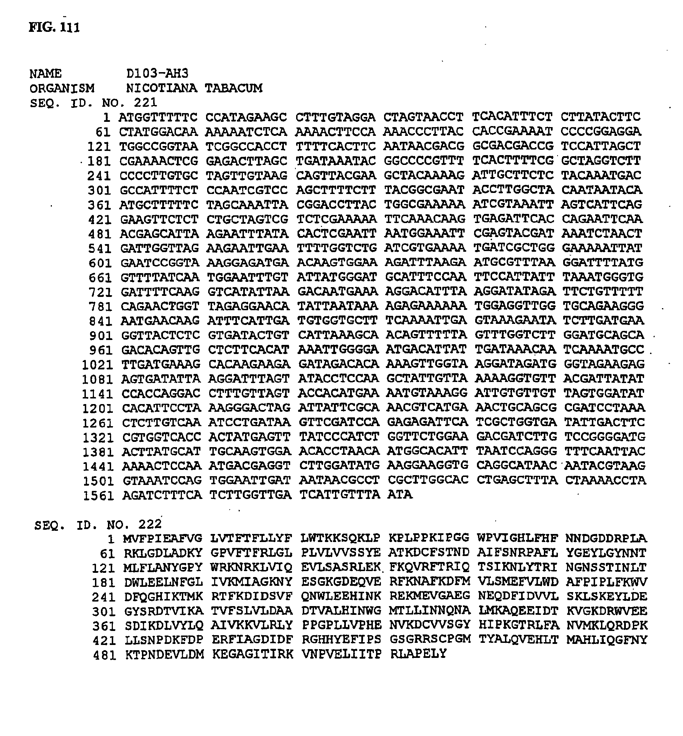

Figure 111 shows nucleic acid SEQ. ID No.: 221 and amino acid SEQ. ID. No.: 222.

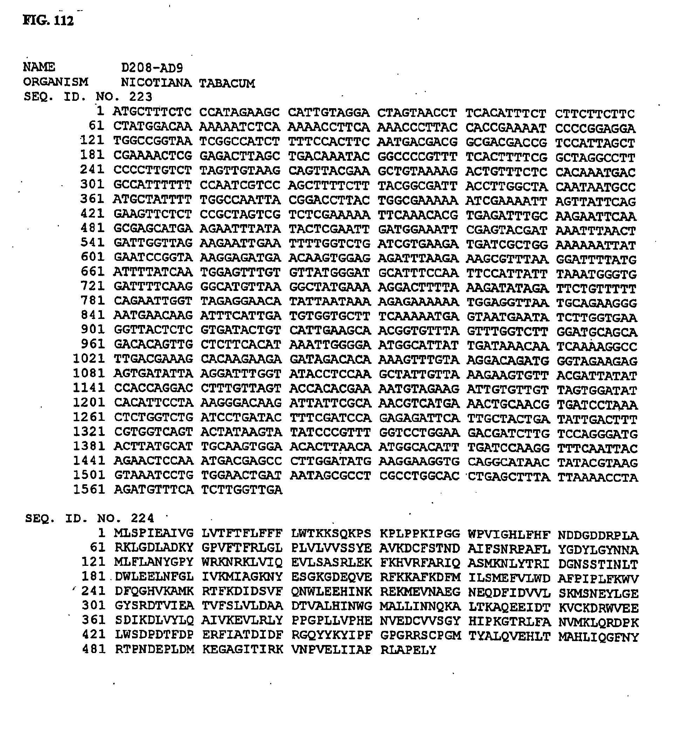

Figure 112 shows nucleic acid SEQ. ID No.: 223 and amino acid SEQ. ID. No.: 224.

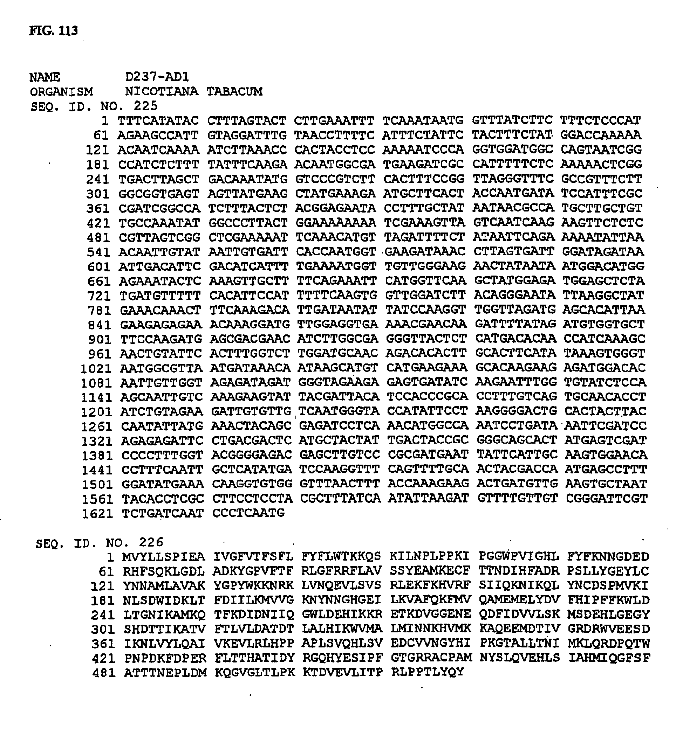

Figure 113 shows nucleic acid SEQ. ID No.: 225 and amino acid SEQ. ID. No.: 226.

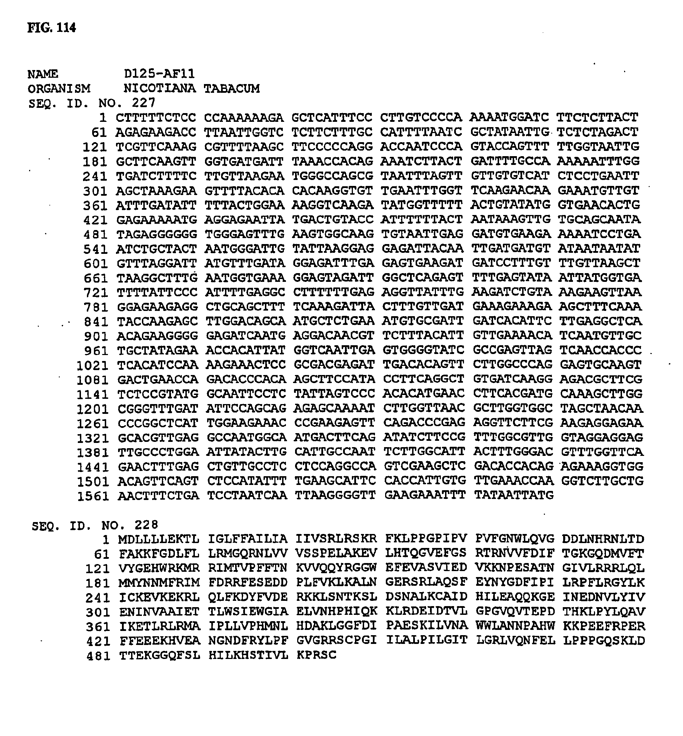

Figure 114 shows nucleic acid SEQ. ID No.: 227 and amino acid SEQ. ID. No.: 228.

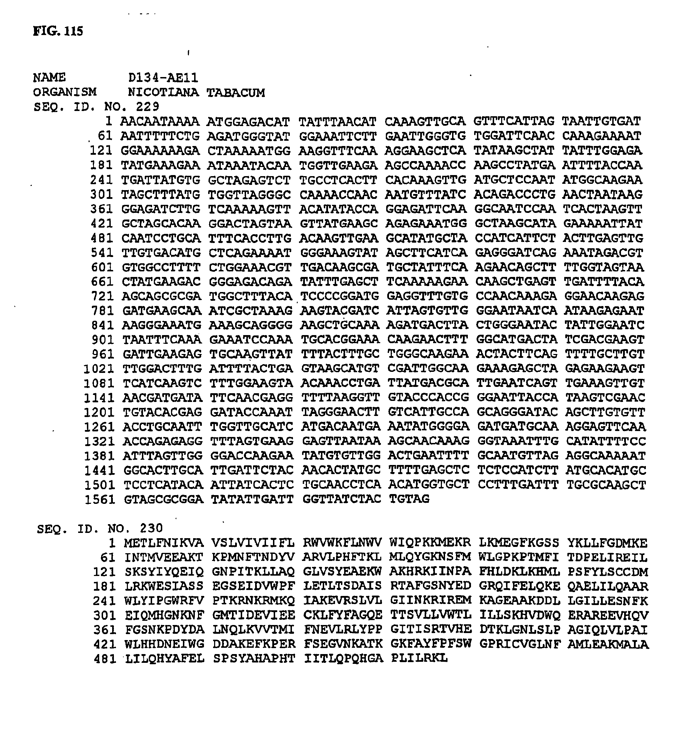

Figure 115 shows nucleic acid SEQ. ID No.: 229 and amino acid SEQ. ID. No.: 230.

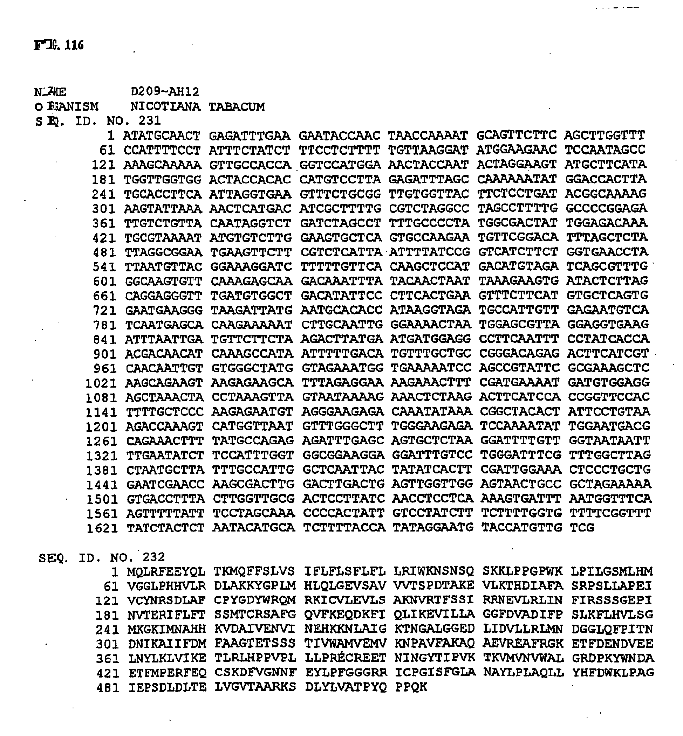

Figure 116 shows nucleic acid SEQ. ID No.: 231 and amino acid SEQ. ID. No.: 232.

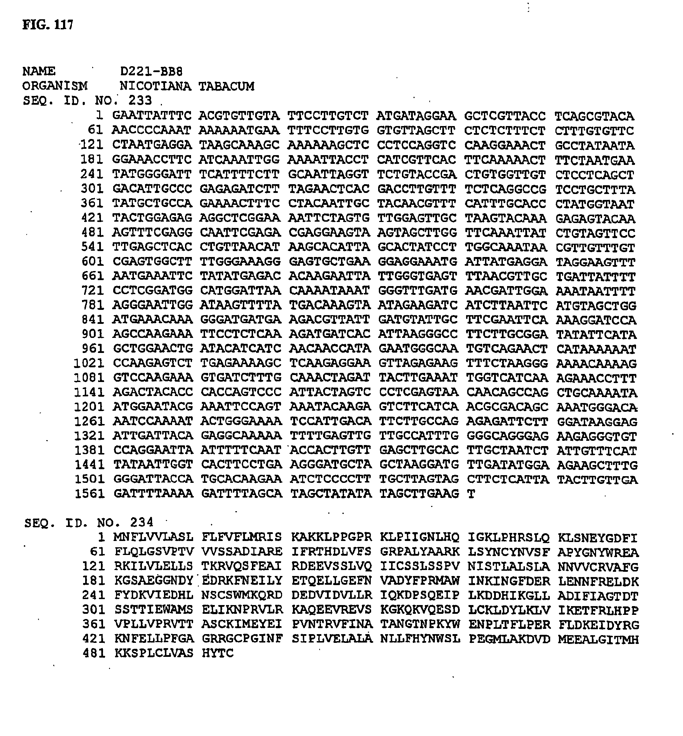

Figure 117 shows nucleic acid SEQ. ID No.: 233 and amino acid SEQ. ID. No.: 234.

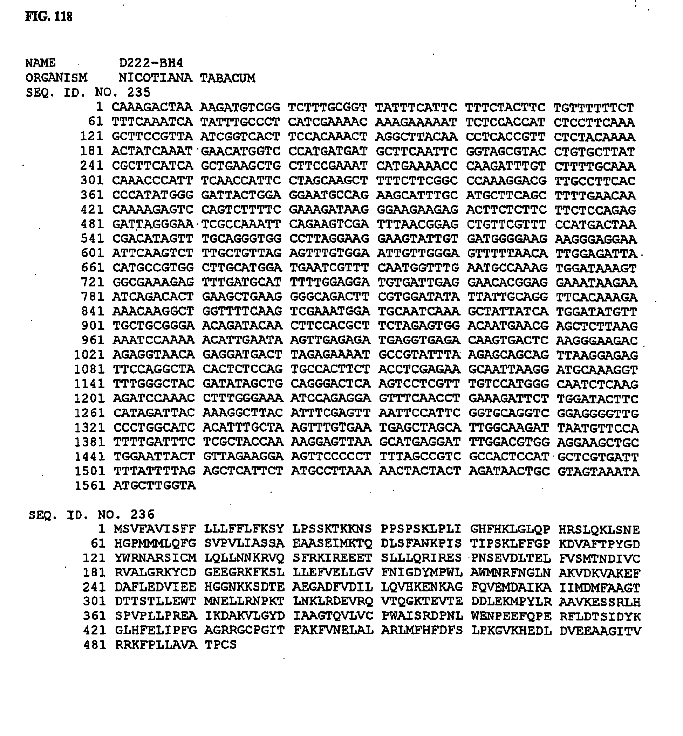

Figure 118 shows nucleic acid SEQ. ID No.: 235 and amino acid SEQ. ID. No.: 236.

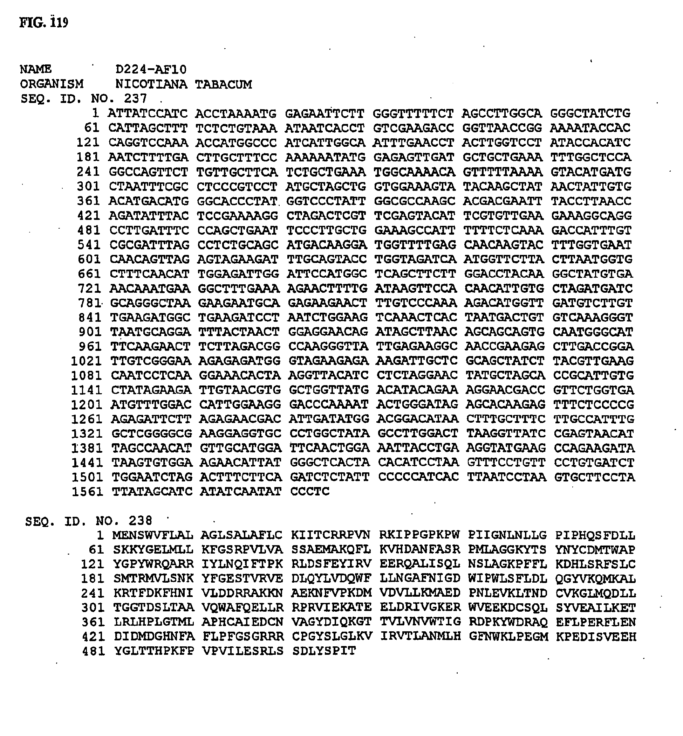

Figure 119 shows nucleic acid SEQ. ID No.: 237 and amino acid SEQ. ID. No.: 238.

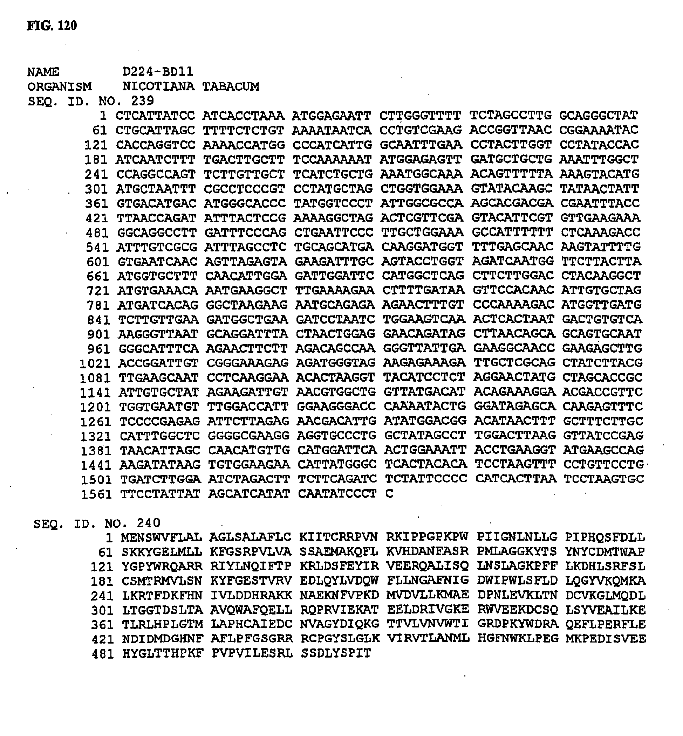

Figure 120 shows nucleic acid SEQ. ID No.: 239 and amino acid SEQ. ID. No.: 240.

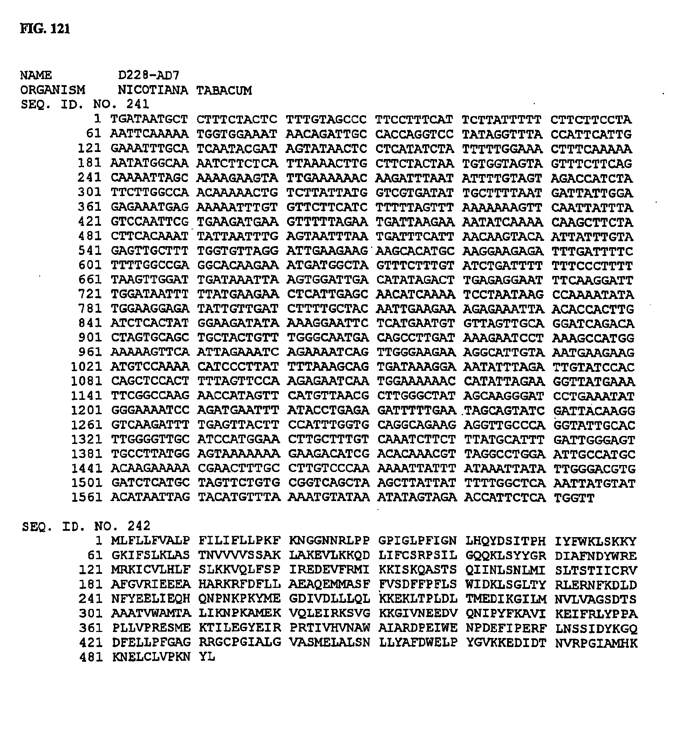

Figure 121 shows nucleic acid SEQ. ID No.: 241 and amino acid SEQ. ID. No.: 242.

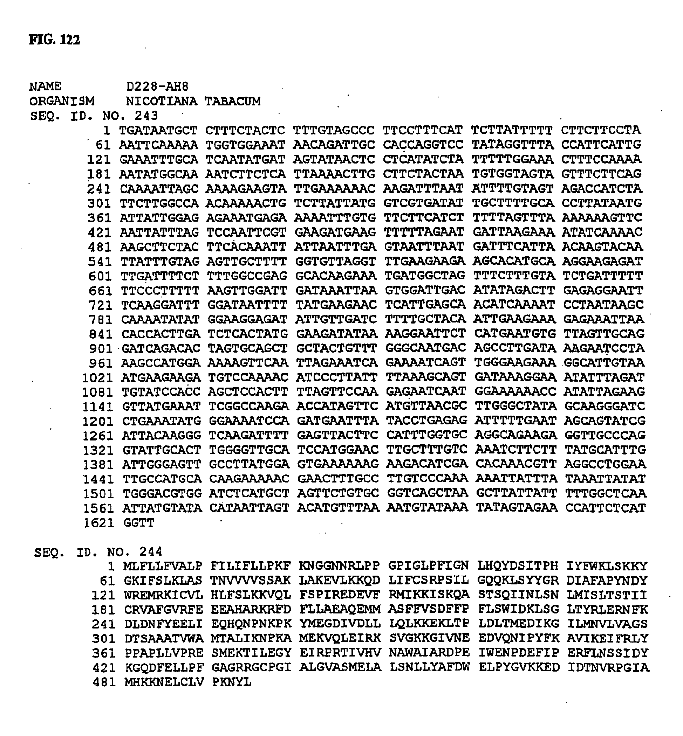

Figure 122 shows nucleic acid SEQ. ID No.: 243 and amino acid SEQ. ID. No.: 244.

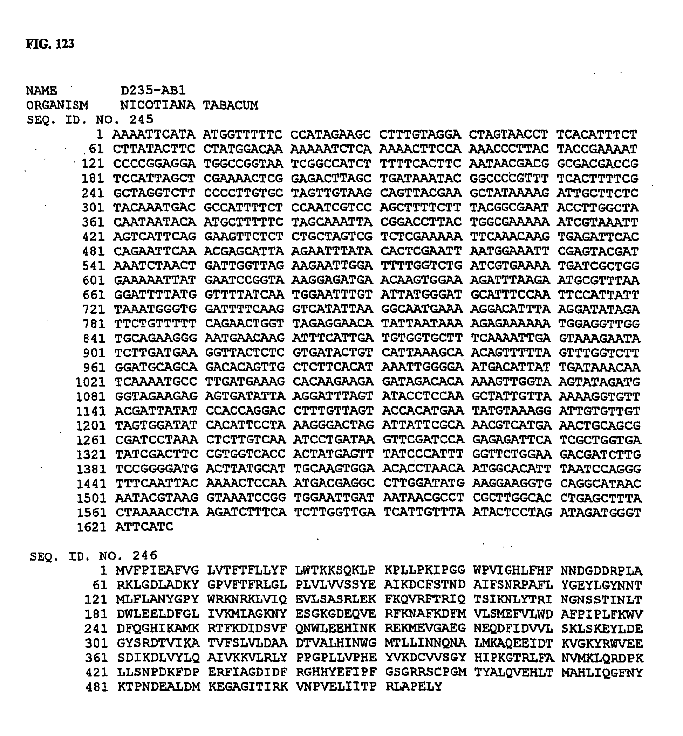

Figure 123 shows nucleic acid SEQ. ID No.: 245 and amino acid SEQ. ID. No.: 246.

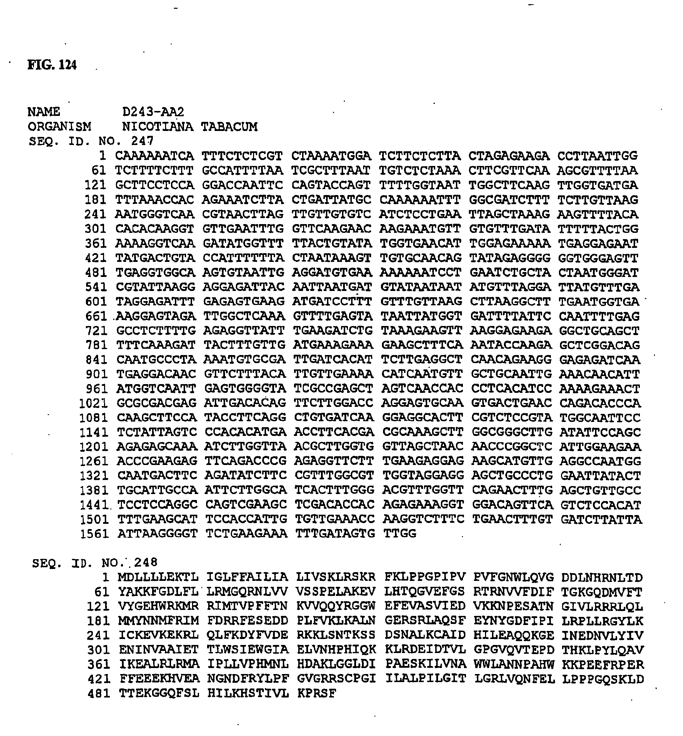

Figure 124 shows nucleic acid SEQ. ID No.: 247 and amino acid SEQ. ID. No.: 248.

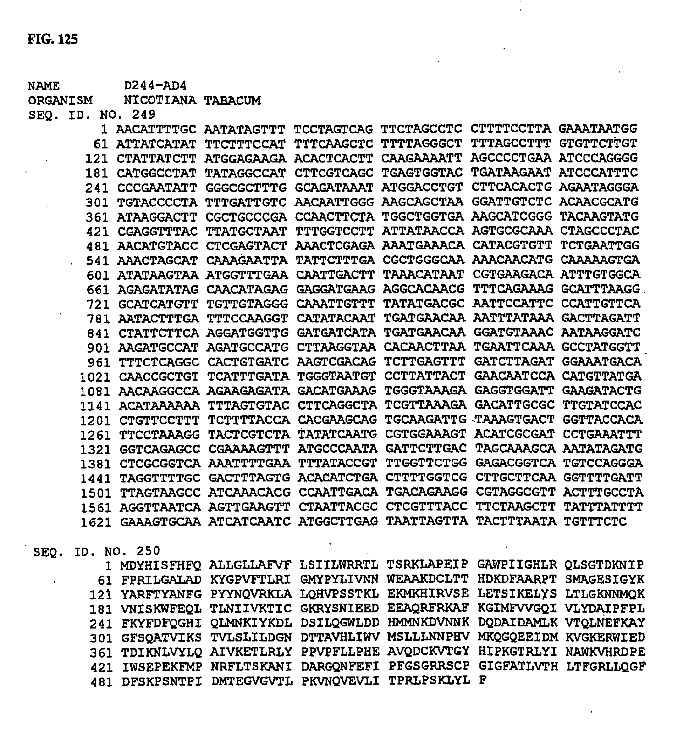

Figure 125 shows nucleic acid SEQ. ID No.: 249 and amino acid SEQ. ID. No.: 250.

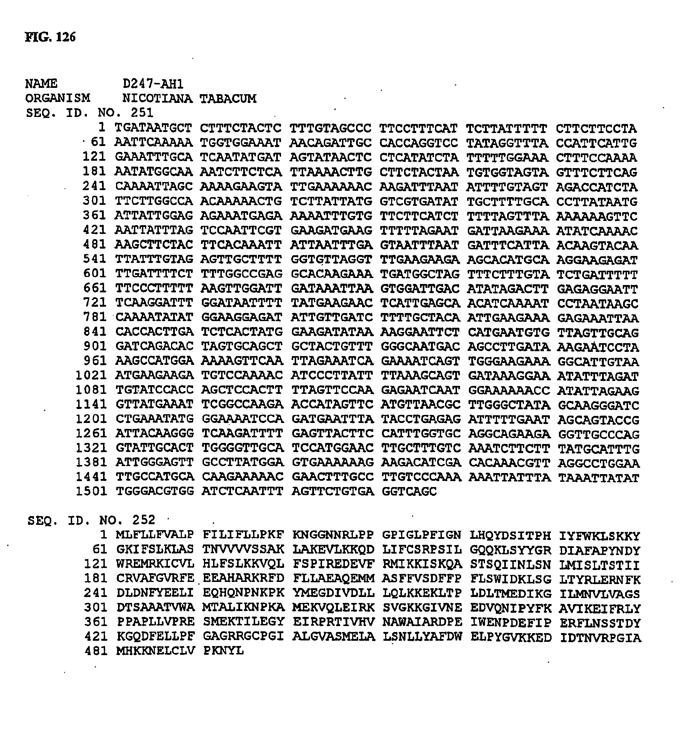

Figure 126 shows nucleic acid SEQ. ID No.: 251 and amino acid SEQ. ID. No.: 252.

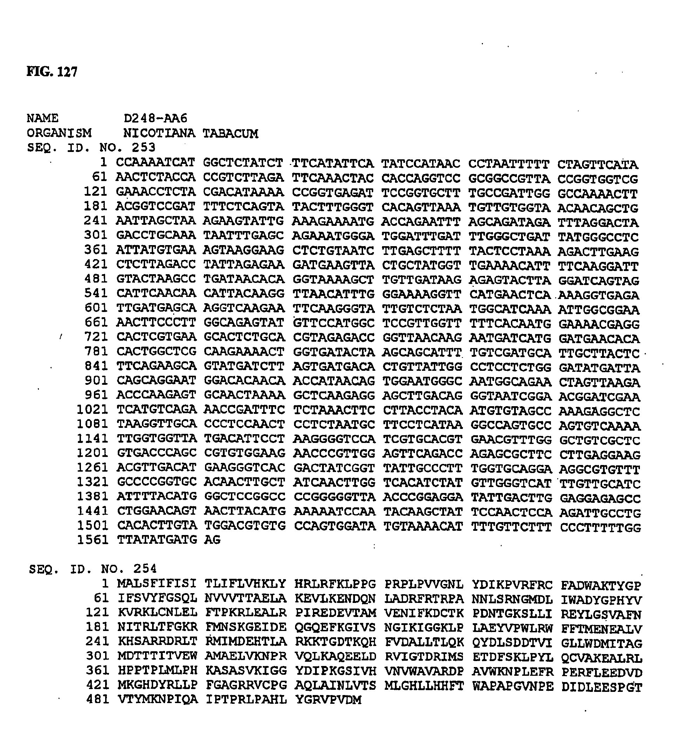

Figure 127 shows nucleic acid SEQ. ID No.: 253 and amino acid SEQ. ID. No.: 254.

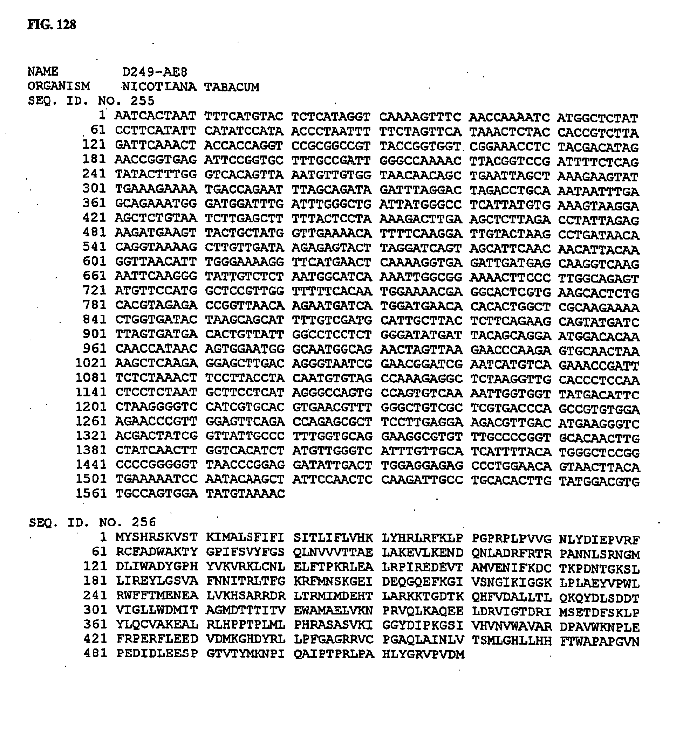

Figure 128 shows nucleic acid SEQ. ID No.: 255 and amino acid SEQ. ID. No.: 256.

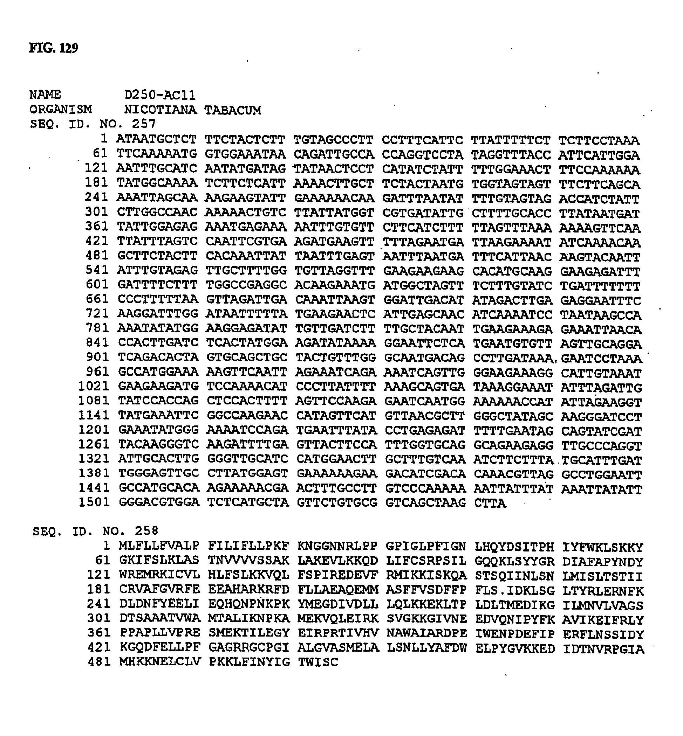

Figure 129 shows nucleic acid SEQ. ID No.: 257 and amino acid SEQ. ID. No.: 258.

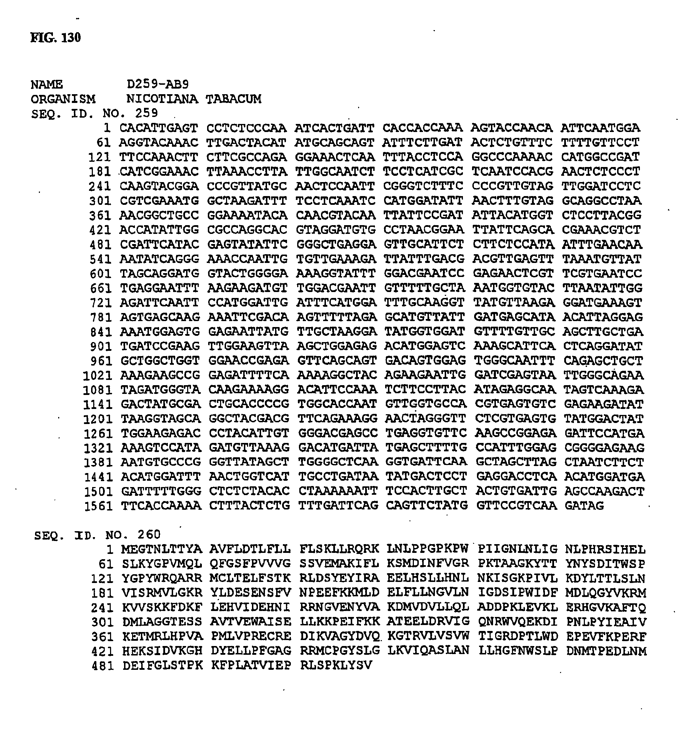

Figure 130 shows nucleic acid SEQ. ID No.: 259 and amino acid SEQ. ID. No.: 260.

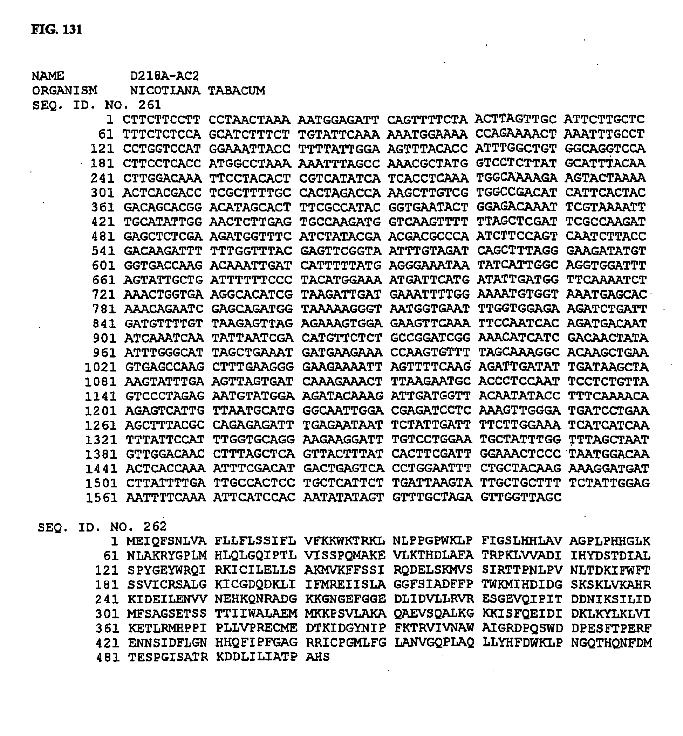

Figure 131 shows nucleic acid SEQ. ID No.: 261 and amino acid SEQ. ID. No.: 262.

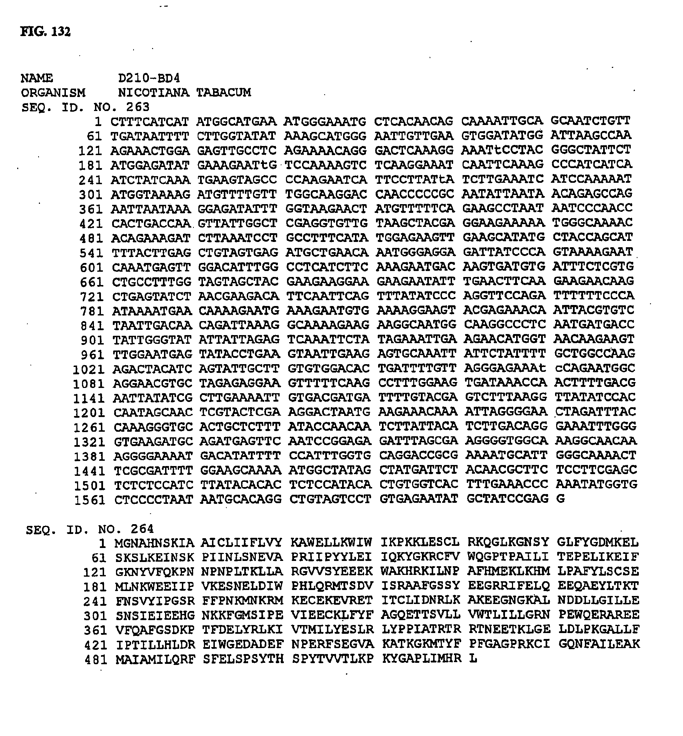

Figure 132 shows nucleic acid SEQ. ID No.: 263 and amino acid SEQ. ID. No.: 264.

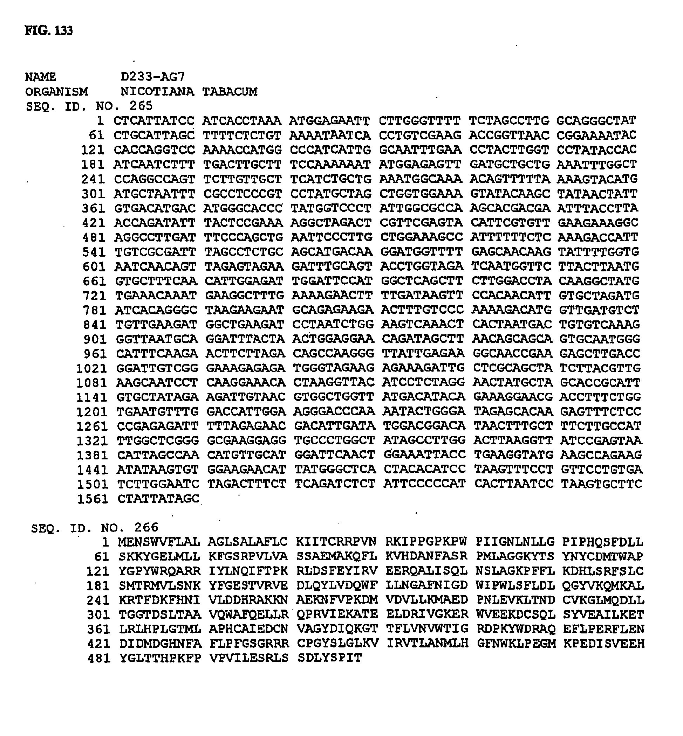

Figure 133 shows nucleic acid SEQ. ID No.: 265 and amino acid SEQ. ID. No.: 266.

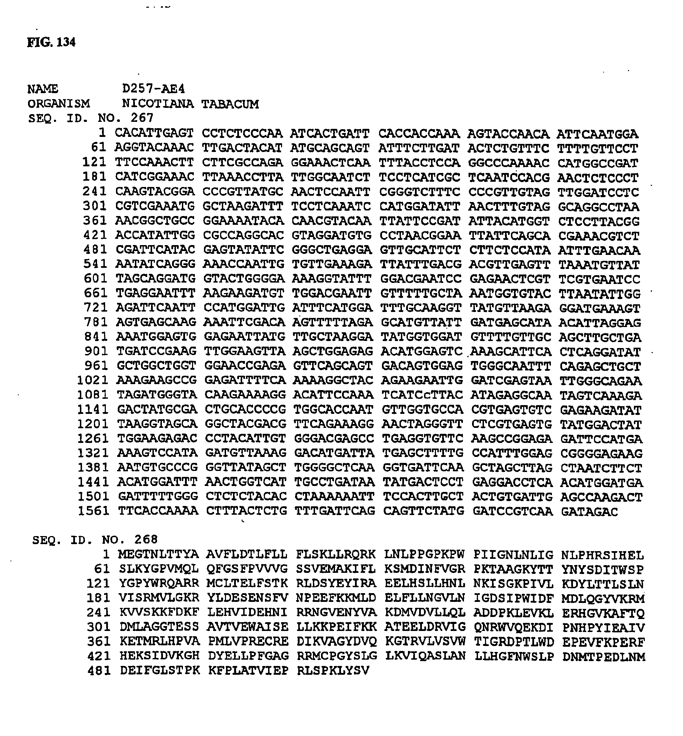

Figure 134 shows nucleic acid SEQ. ID No.: 267 and amino acid SEQ. ID. No.: 268.

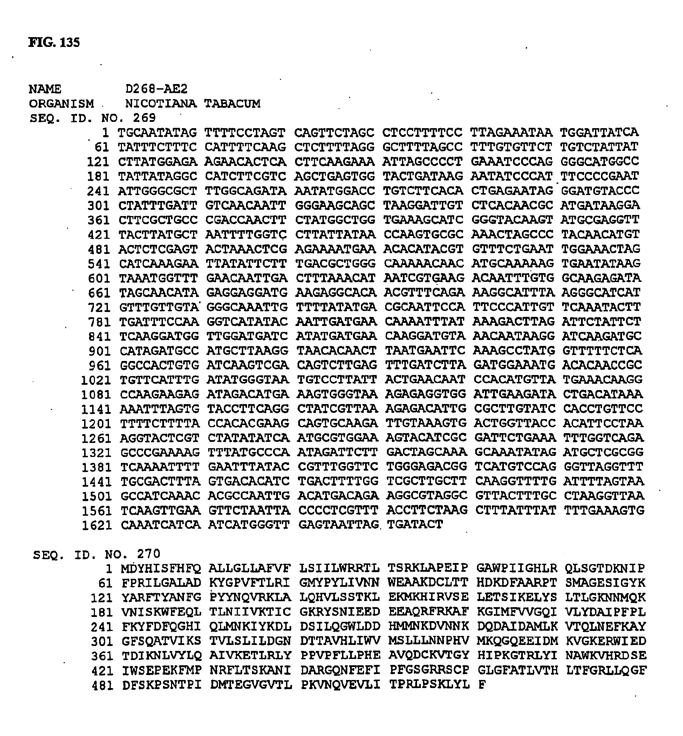

Figure 135 shows nucleic acid SEQ. ID No.: 269 and amino acid SEQ. ID. No.: 270.

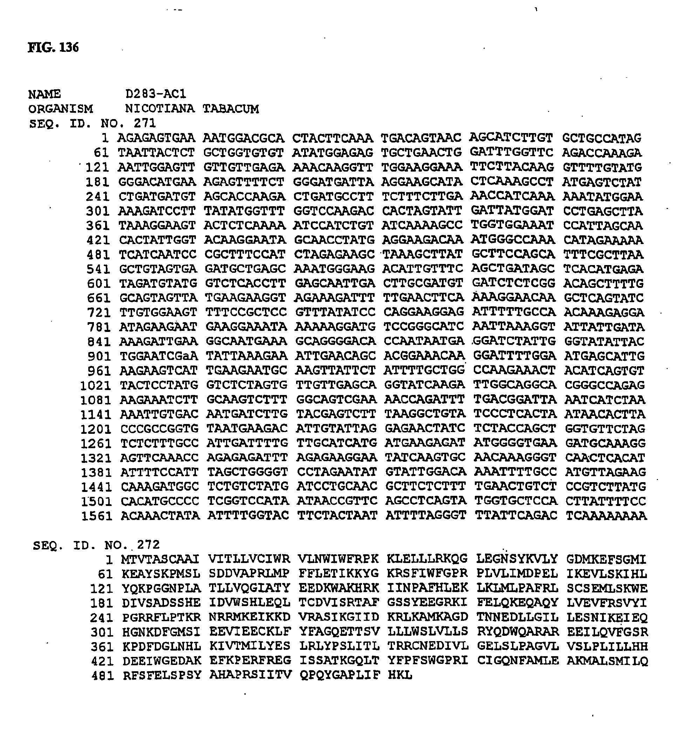

Figure 136 shows nucleic acid SEQ. ID No.: 271 and amino acid SEQ. ID. No.: 272.

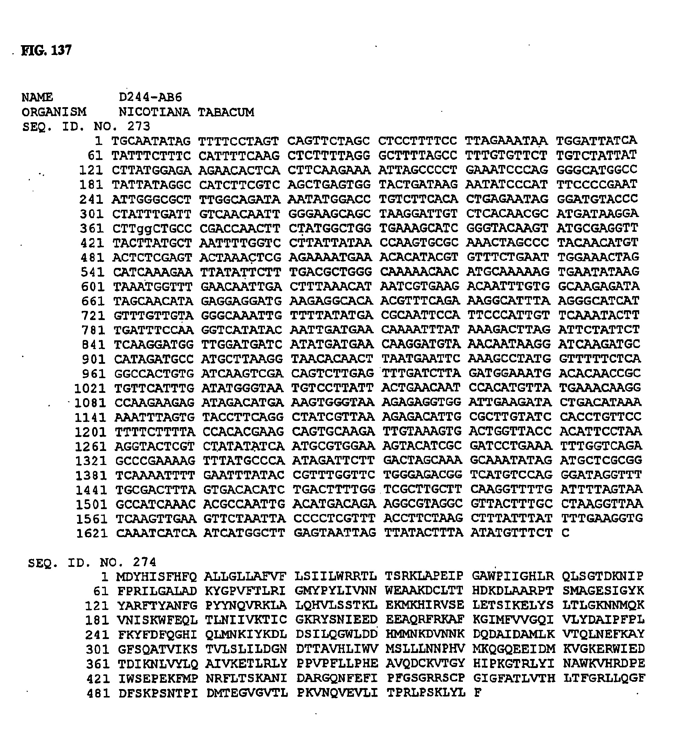

Figure 137 shows nucleic acid SEQ. ID No.: 273 and amino acid SEQ. ID. No.: 274.

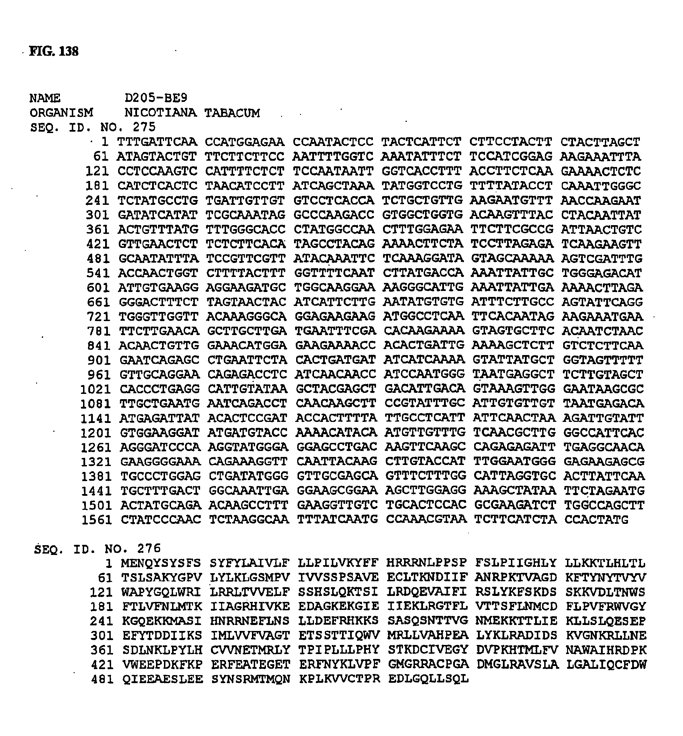

Figure 138 shows nucleic acid SEQ. ID No.: 275 and amino acid SEQ. ID. No.: 276.

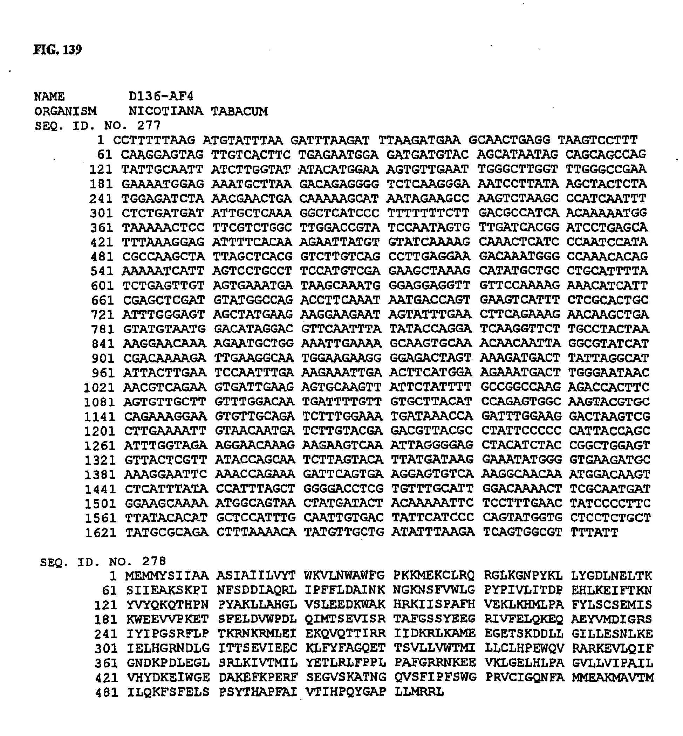

Figure 139 shows nucleic acid SEQ. ID No.: 277 and amino acid SEQ. ID. No.: 278.

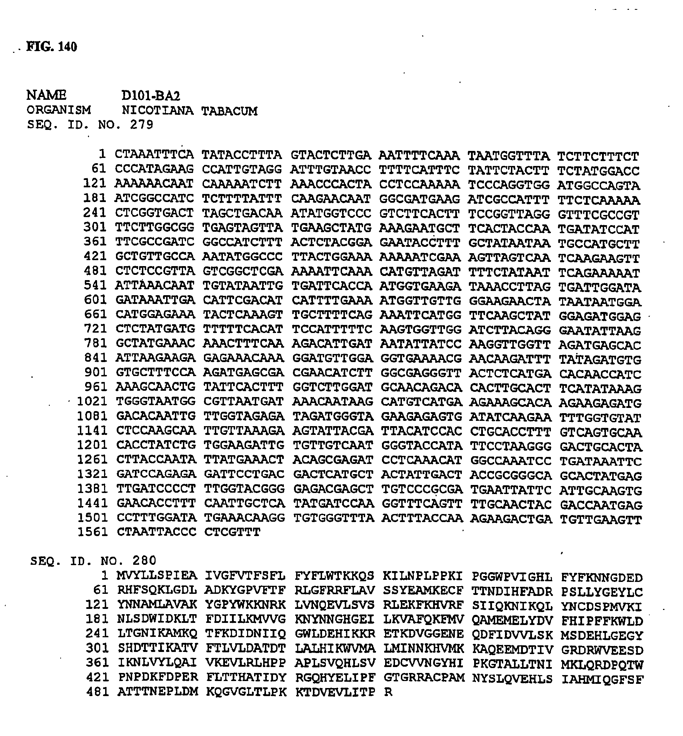

Figure 140 shows nucleic acid SEQ. ID No.: 279 and amino acid SEQ. ID. No.: 280.

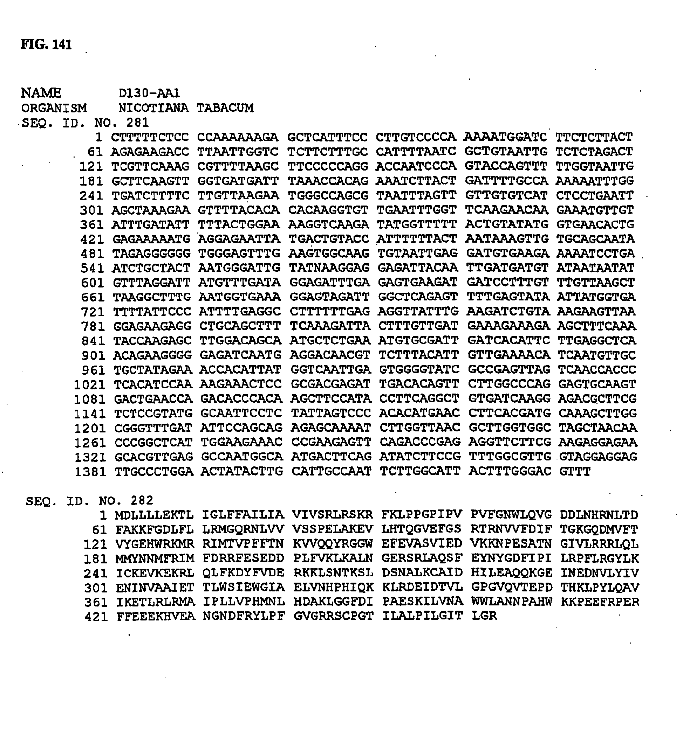

Figure 141 shows nucleic acid SEQ. ID No.: 281 and amino acid SEQ. ID. No.: 282.

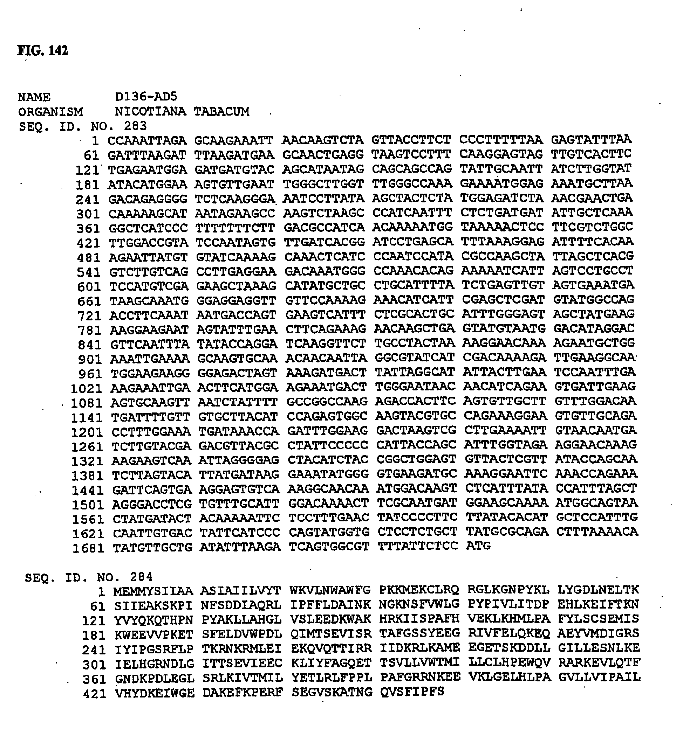

Figure 142 shows nucleic acid SEQ. ID No.: 283 and amino acid SEQ. ID. No.: 284.

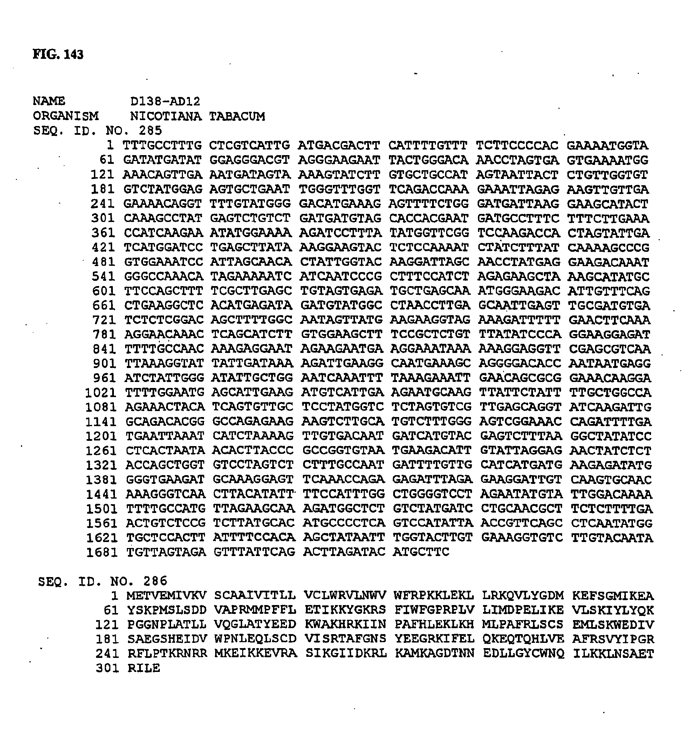

Figure 143 shows nucleic acid SEQ. ID No.: 285 and amino acid SEQ. ID. No.: 286.

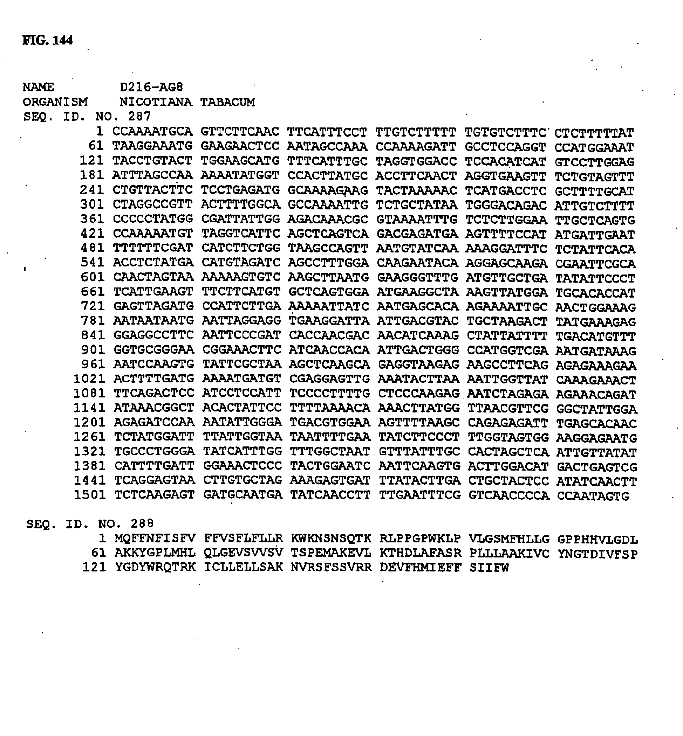

Figure 144 shows nucleic acid SEQ. ID No.: 287 and amino acid SEQ. ID. No.: 288.

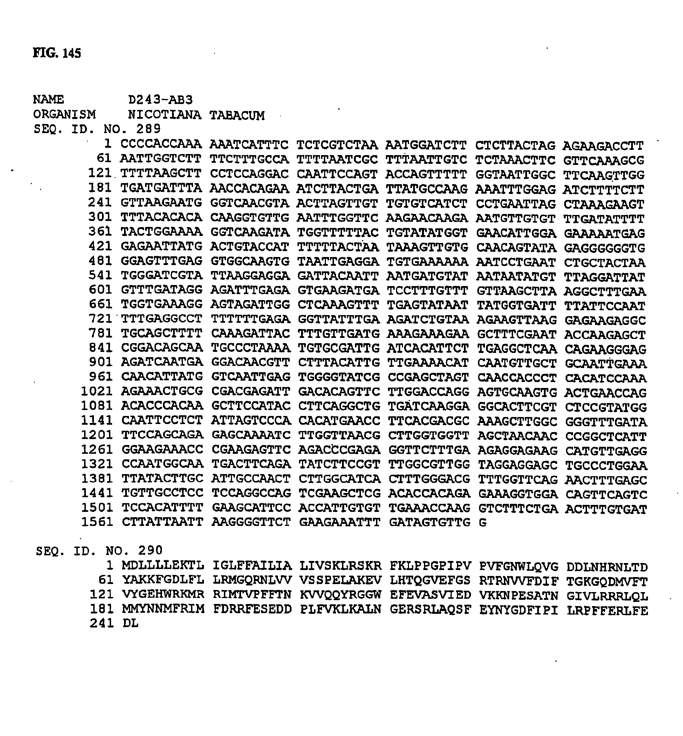

Figure 145 shows nucleic acid SEQ. ID No.: 289 and amino acid SEQ. ID. No.: 290.

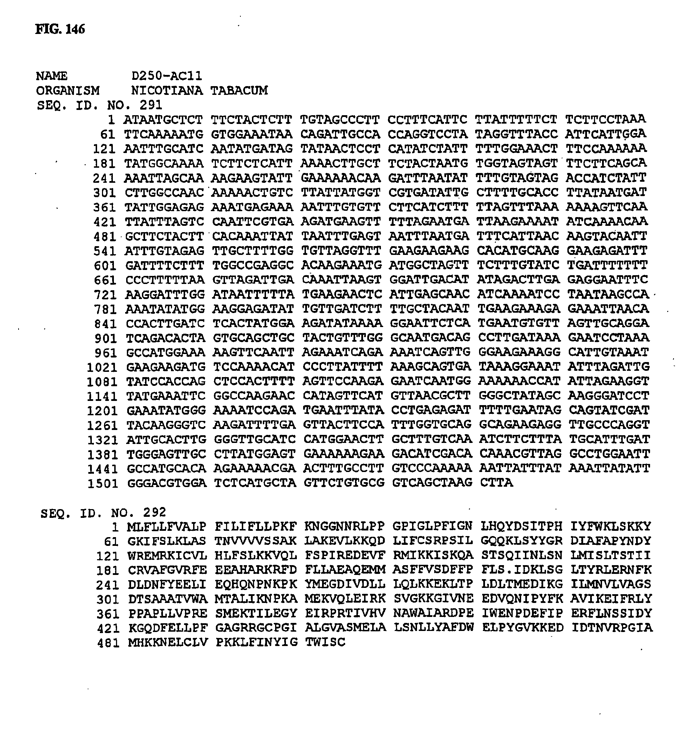

Figure 146 shows nucleic acid SEQ. ID No.: 291 and amino acid SEQ. ID. No.: 292.

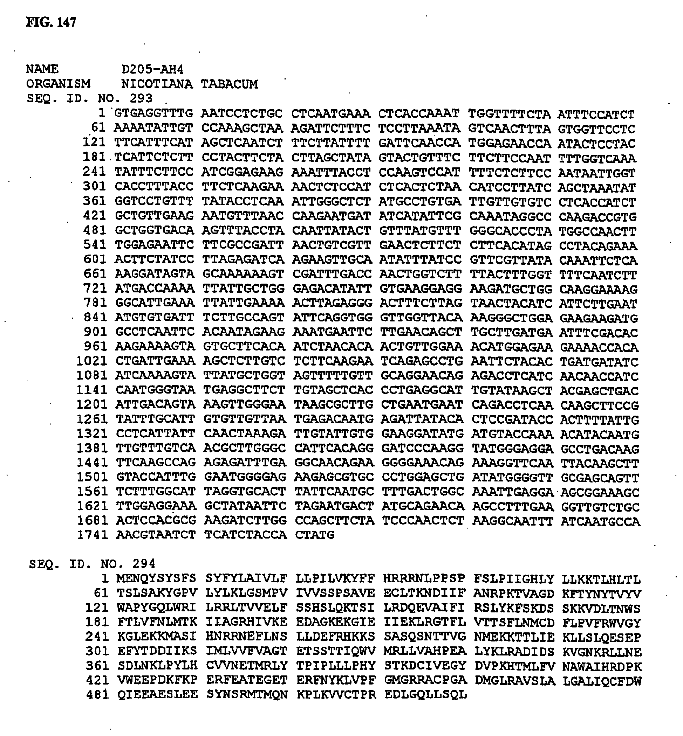

Figure 147 shows nucleic acid SEQ. ID No.: 293 and amino acid SEQ. ID. No.: 294.

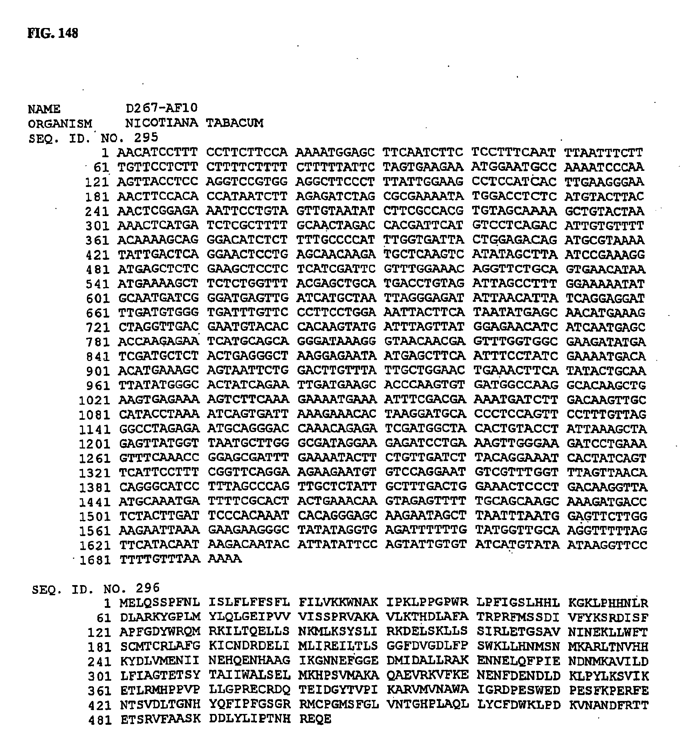

Figure 148 shows nucleic acid SEQ. ID No.: 295 and amino acid SEQ. ID. No.: 296.

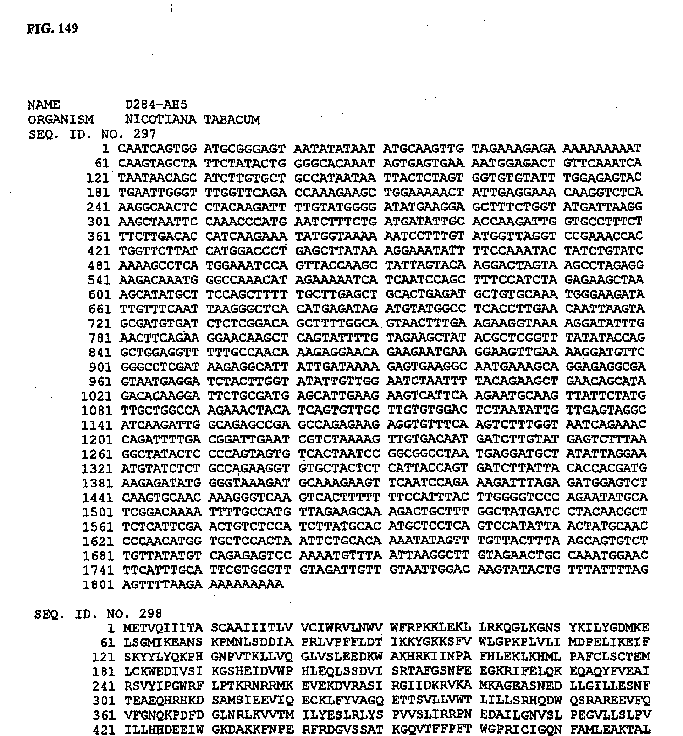

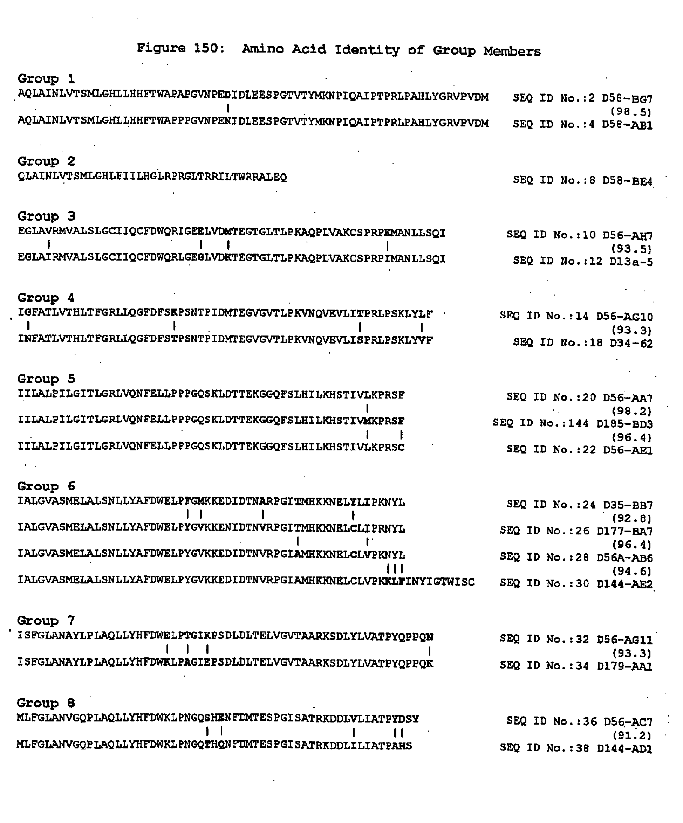

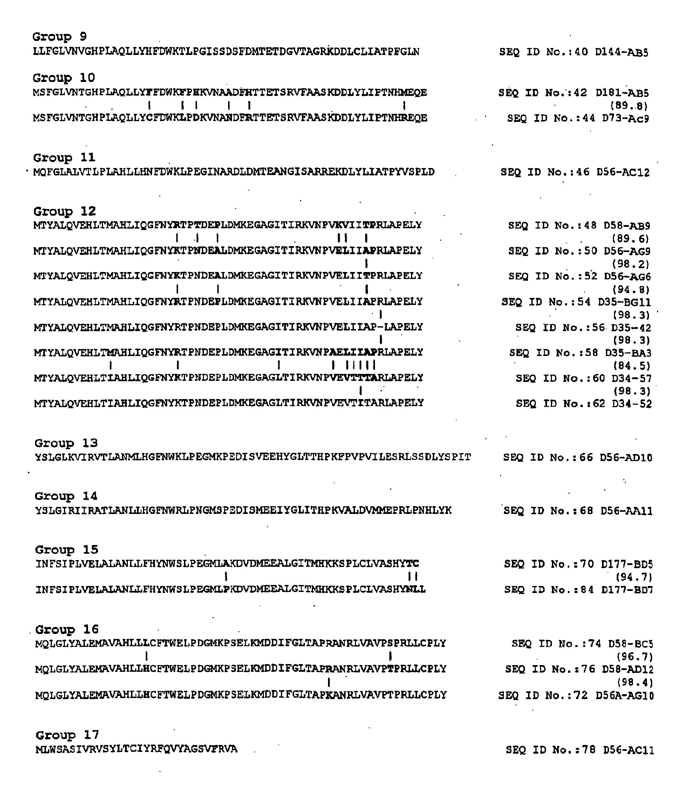

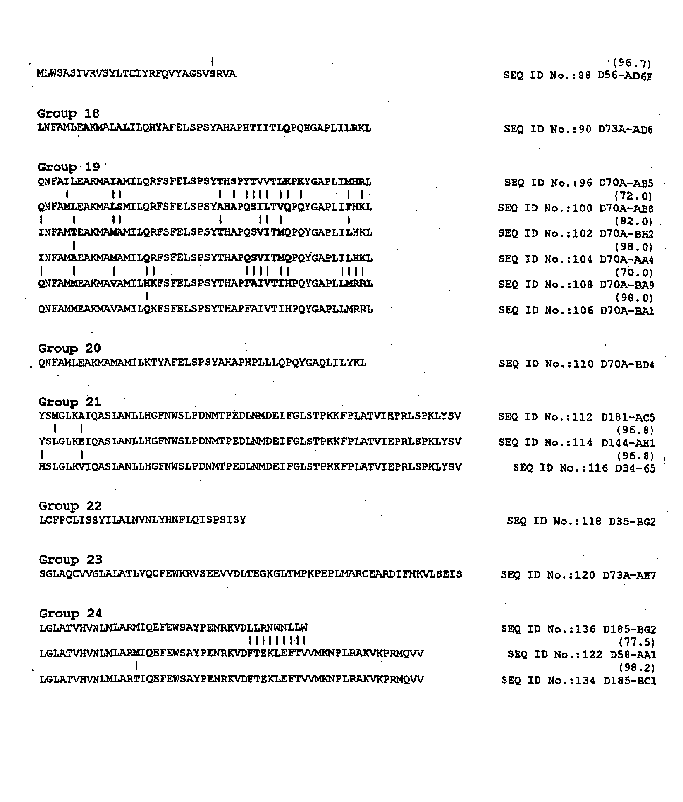

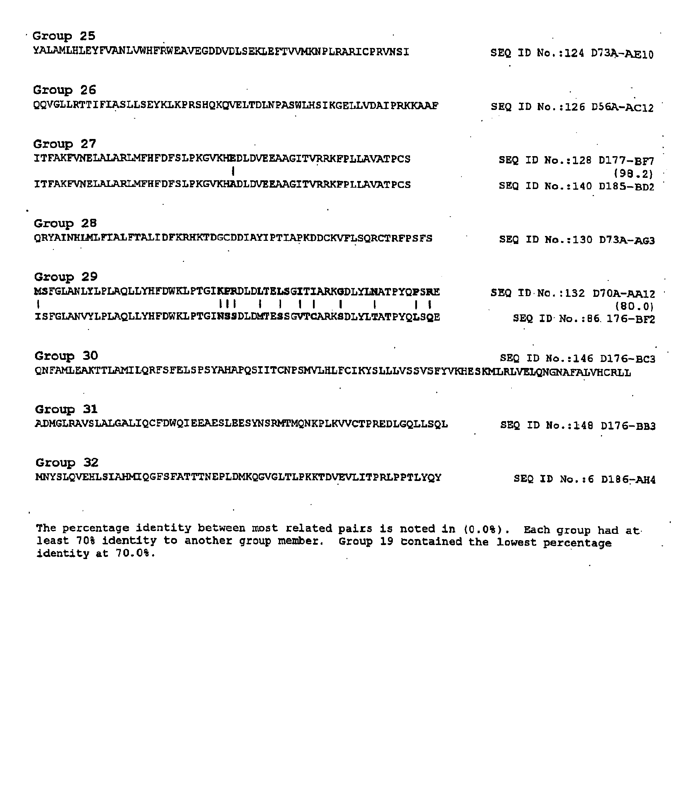

Figure 149 shows nucleic acid SEQ. ID No.: 297 and amino acid SEQ. ID. No.: 298.

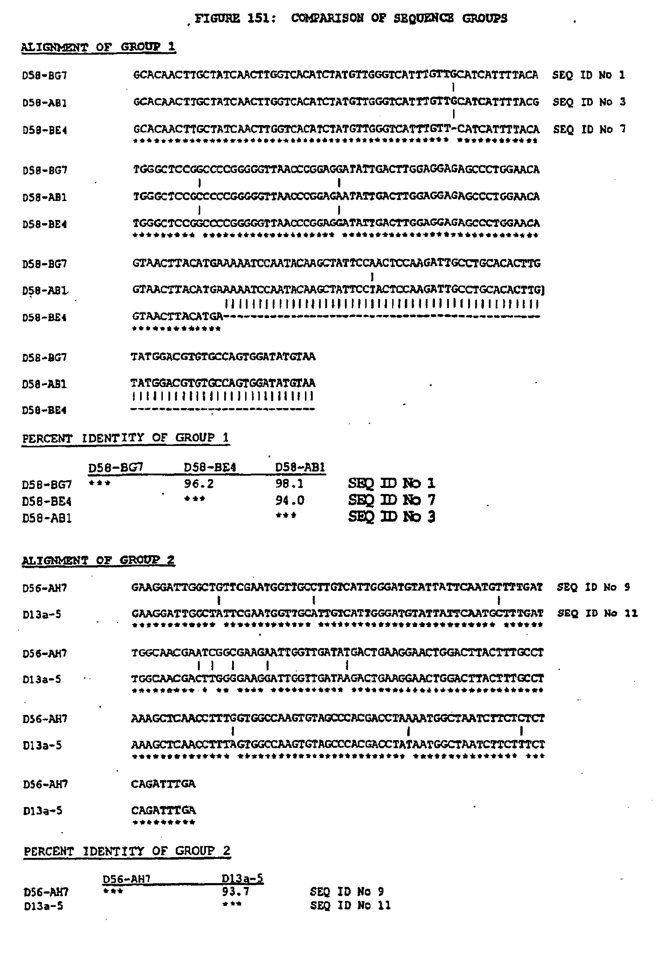

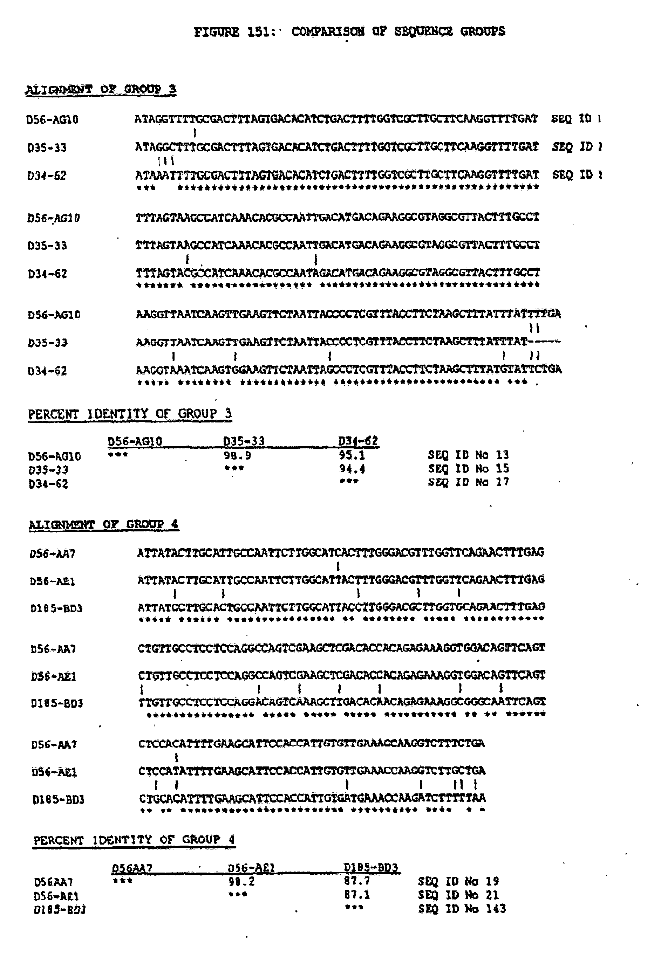

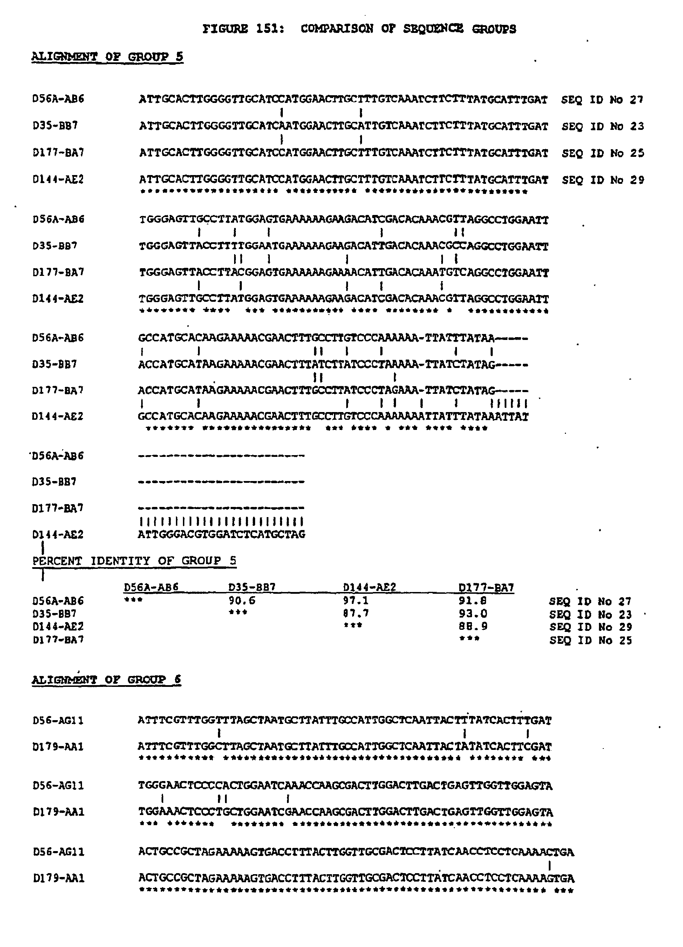

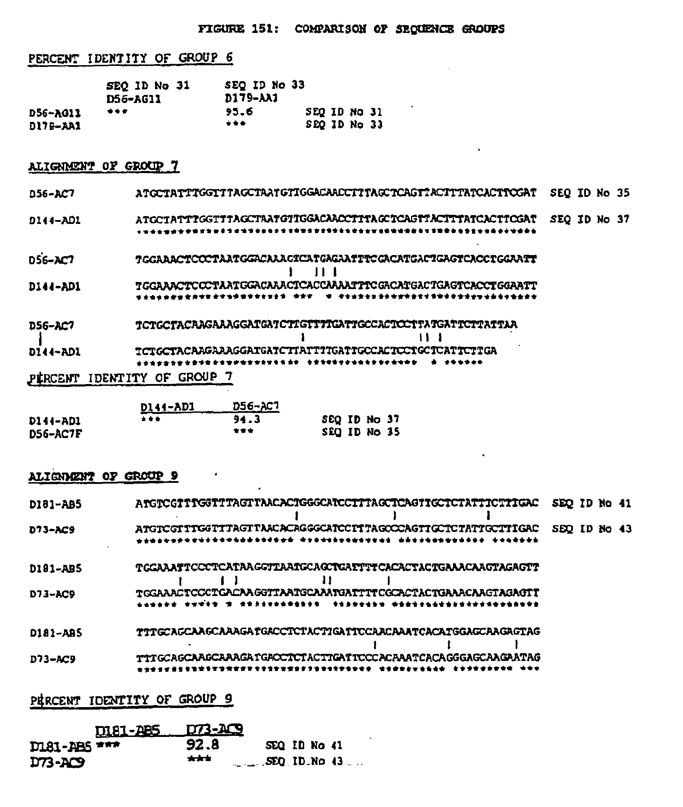

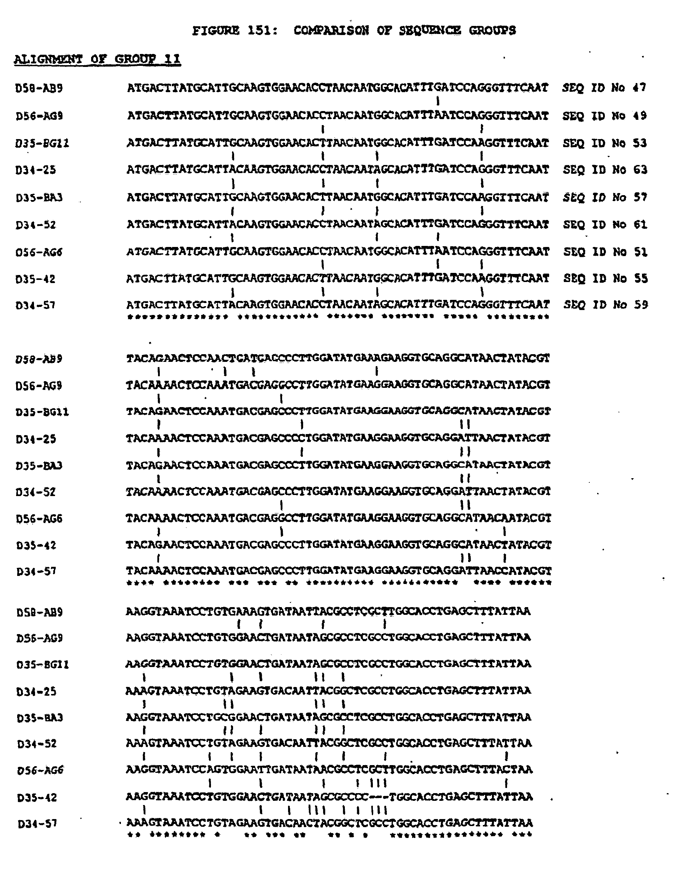

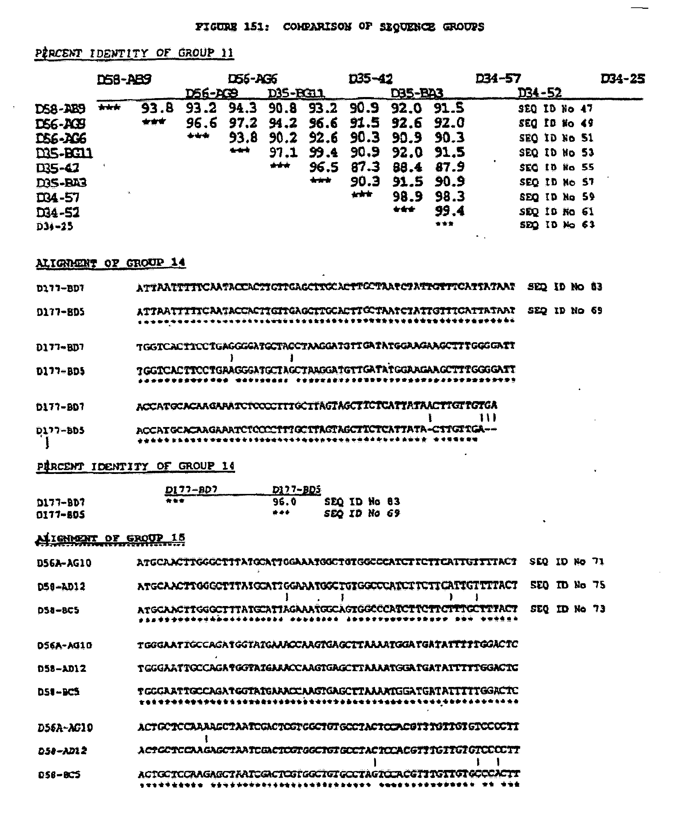

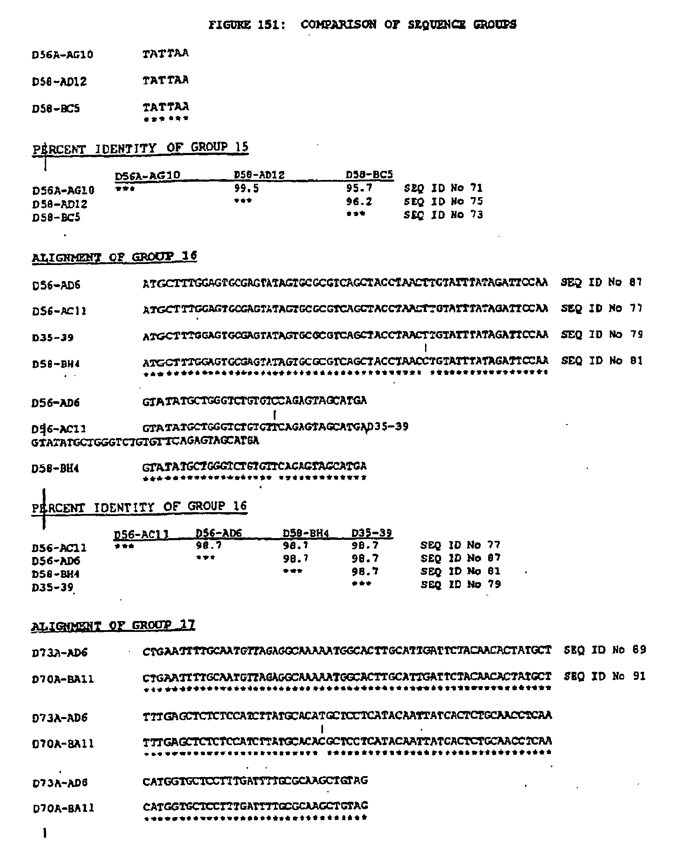

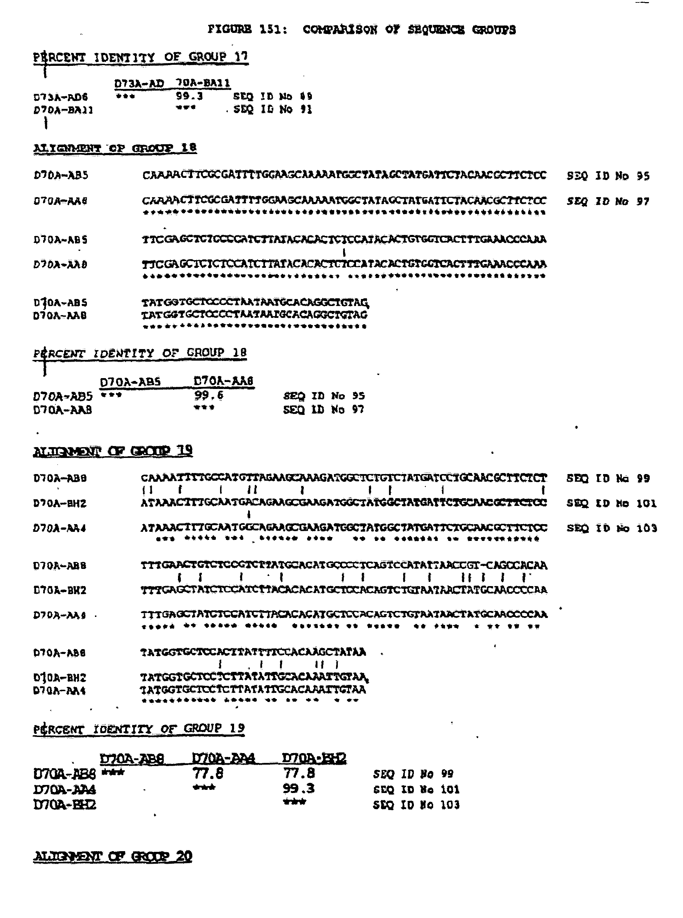

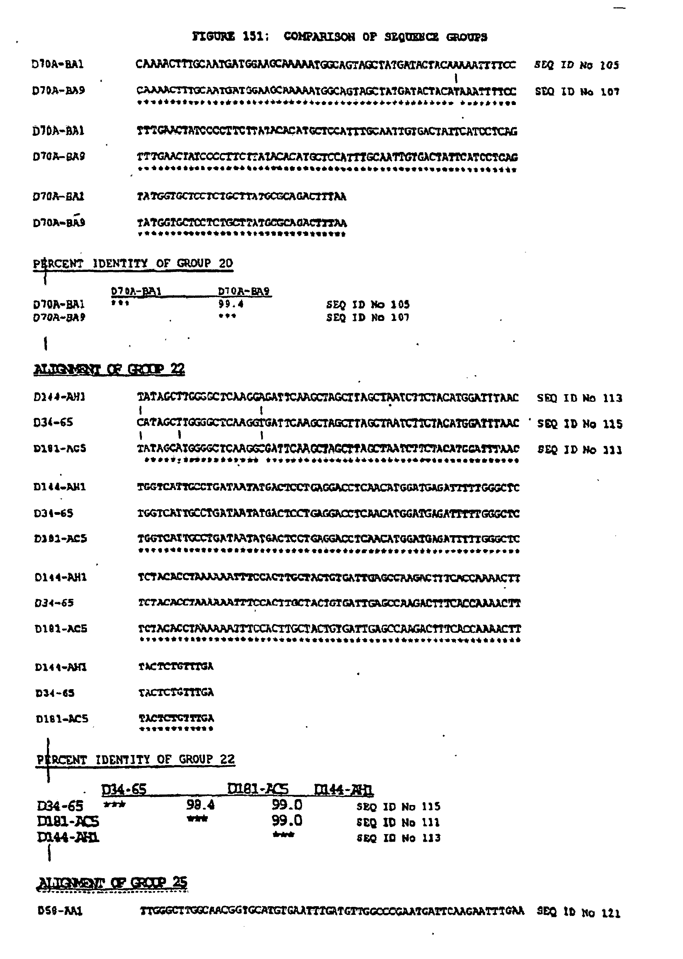

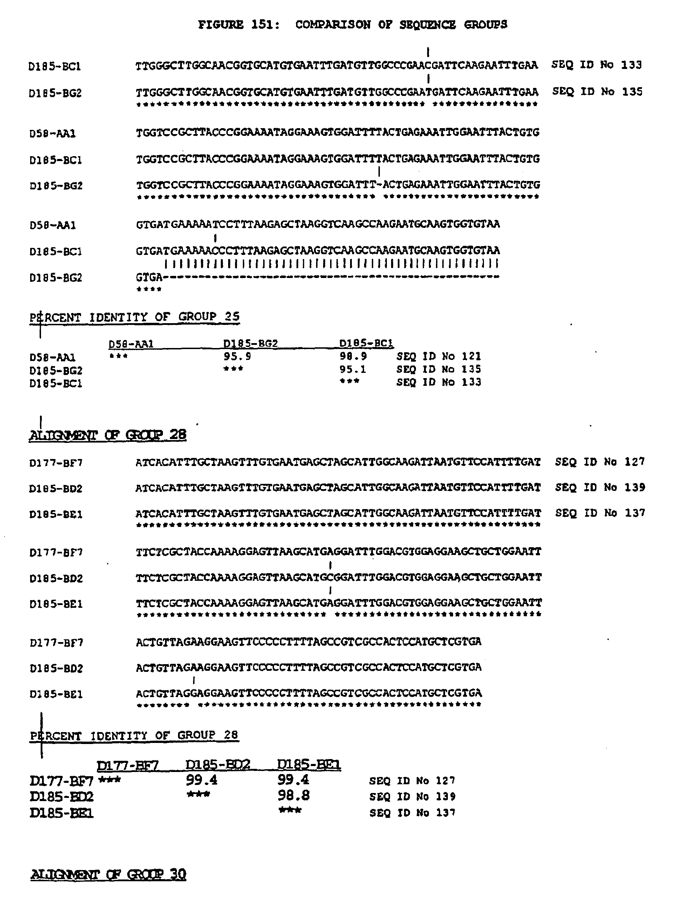

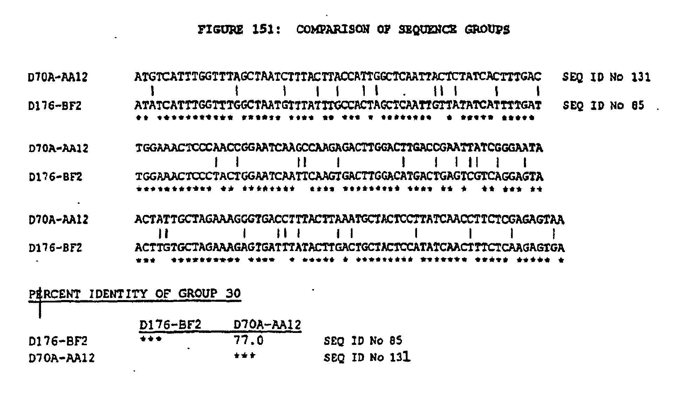

Figure 151 shows a comparison of Sequence Groups.

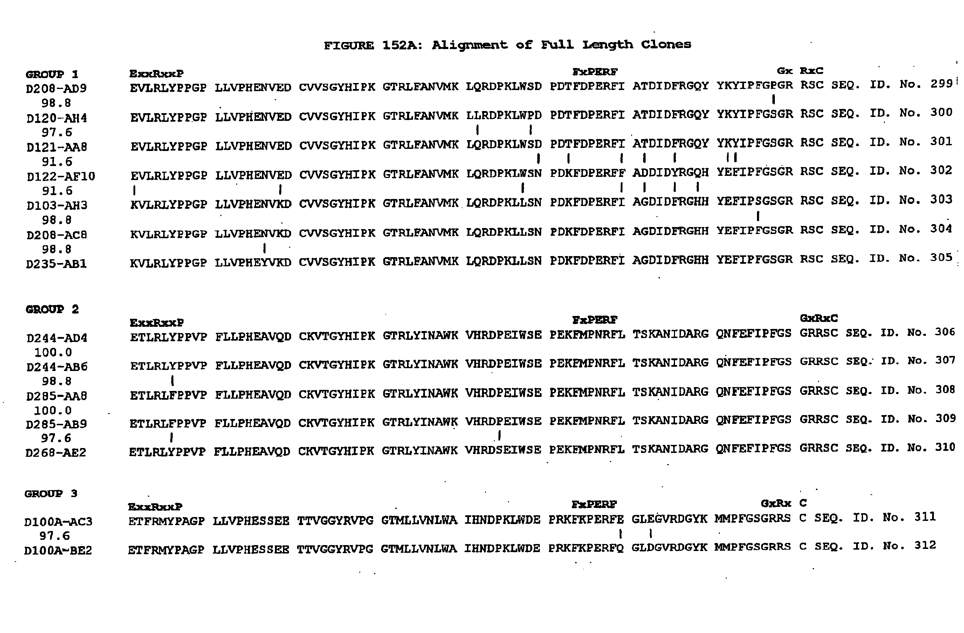

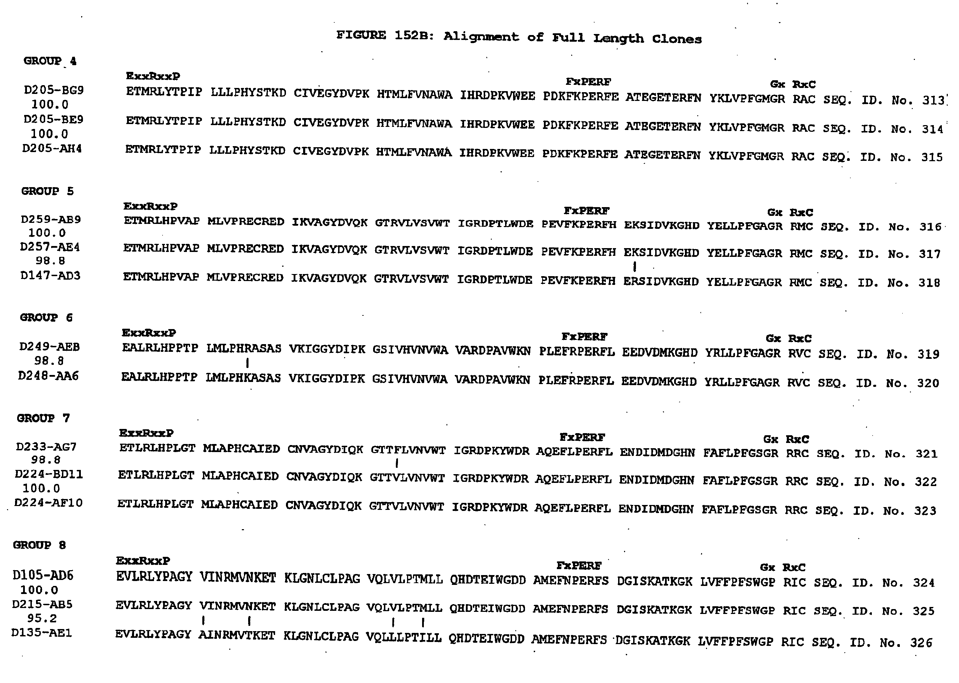

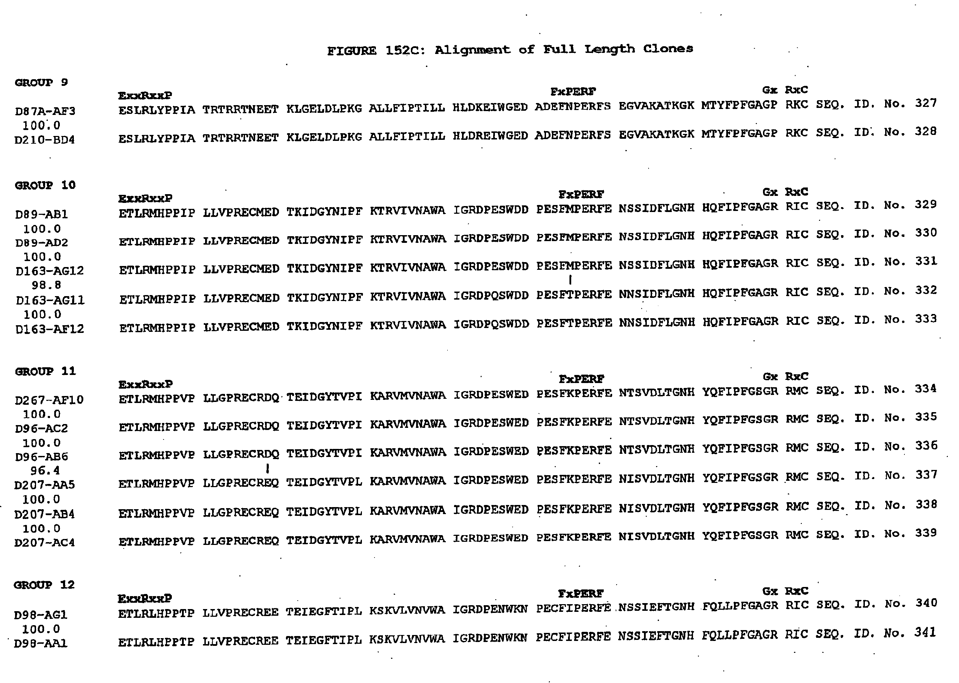

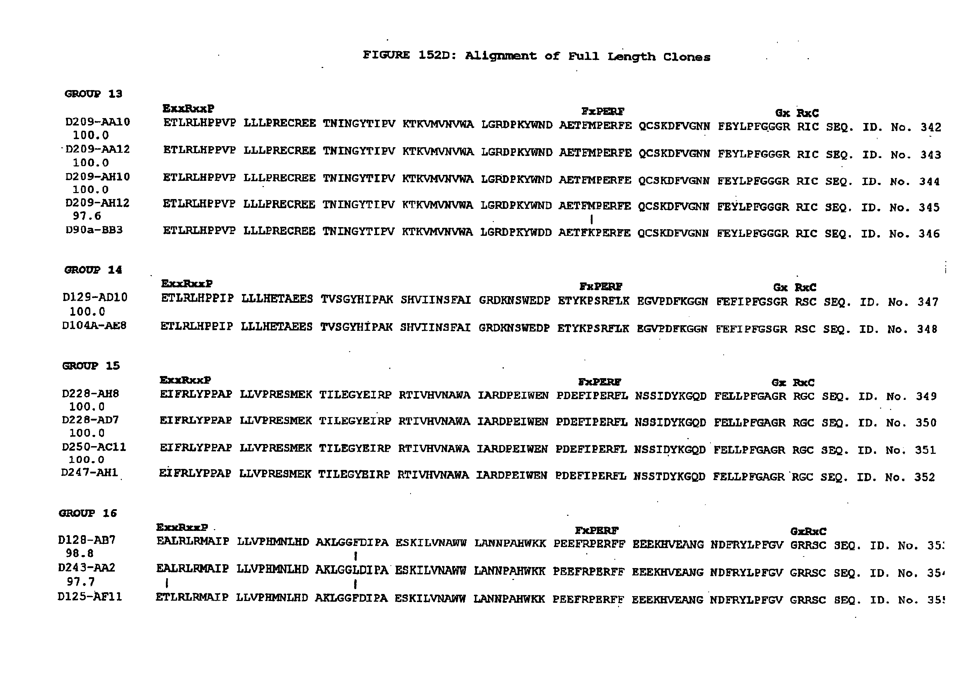

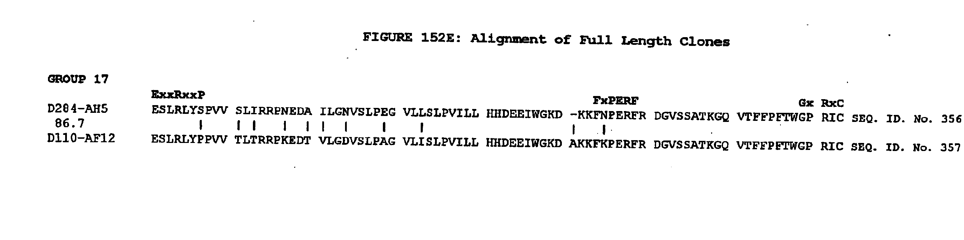

Figure 152 illustrates alignment of full length clones.
Figure 153 shows a procedure used for cloning of cytochrome p450 cDNA fragments by PCR

### DETAILED DESCRIPTION

### DEFINITIONS

Unless defined otherwise, all technical and scientific terms used herein have the same meaning as commonly understood by one of ordinary skill in the art to which this invention belongs. Singleton et al. (1994) Dictionary of Microbiology and Molecular Biology, second edition, John Wiley and Sons (New York) provides one of skill with a general dictionary of many of the terms used in this invention. All patents and publications referred to herein are incorporated by reference herein. For purposes of the present invention, the following terms are defined below.

"Enzymatic activity" is meant to include demethylation, hydroxylation, epoxidation, N-oxidation, sulfooxidation, N-, S-, and O dealkylations, desulfation, deamination, and reduction of azo, nitro, and N-oxide groups. The term "nucleic acid" refers to a deoxyribonucleotide or ribonucleotide polymer in either single- or double-stranded form, or sense or anti-sense, and unless otherwise limited, encompasses known analogues of natural nucleotides that hybridize to nucleic acids in a manner similar to naturally occurring nucleotides. Unless otherwise indicated, a particular nucleic acid sequence includes the complementary sequence thereof.

The terms "operably linked", "in operable combination", and "in operable order" refer to functional linkage between a nucleic acid expression control sequence (such as a promoter, signal sequence, or array of transcription factor binding sites) and a second nucleic acid sequence, wherein the expression control sequence affects transcription and/or translation of the nucleic acid corresponding to the second sequence.

The term "recombinant" when used with reference to a cell indicates that the cell replicates a heterologous nucleic acid, expresses said nucleic acid or expresses a peptide, heterologous peptide, or protein encoded by a heterologous nucleic acid. Recombinant cells can express genes or gene fragments in either the sense or antisense form that are not found within the native (non-recombinant) form of the cell. Recombinant cells can also express genes that are found in the native form of the cell, but wherein the genes are modified and re-introduced into the cell by artificial means.

A "structural gene" is that portion of a gene comprising a DNA segment encoding a protein, polypeptide or a portion thereof, and excluding the 5' sequence which drives the initiation of transcription. The structural gene may alternatively encode a nontranslatable product. The structural gene may be one which is normally found in the cell or one which is not normally found in the cell or cellular location wherein it is introduced, in which case it is termed a "heterologous gene". A heterologous gene may be derived in whole or in part from any source known to the art, including a bacterial genome or episome, eukaryotic, nuclear or plasmid DNA, cDNA, viral DNA or chemically synthesized DNA. A structural gene may contain one or more modifications that could effect biological activity or its characteristics, the biological activity or the chemical structure of the expression product, the rate of expression or the manner of expression control. Such modifications include, but are not limited to, mutations, insertions, deletions and substitutions of one or more nucleotides. The structural gene may constitute an uninterrupted coding sequence or it may include one or more introns, bounded by the appropriate splice junctions. The structural gene may be translatable or non-translatable, including in an anti-sense orientation. The structural gene may be a composite of segments derived from a plurality of sources and from a plurality of gene sequences (naturally occurring or synthetic, where synthetic refers to DNA that is chemically synthesized).

"Derived from" is used to mean taken, obtained, received, traced, replicated or descended from a source (chemical and/or biological). A derivative may be produced by chemical or biological manipulation (including, but not limited to, substitution, addition, insertion, deletion, extraction, isolation, mutation and replication) of the original source.

"Chemically synthesized", as related to a sequence of DNA, means that portions of the component nucleotides were assembled in vitro. Manual chemical synthesis of DNA may be accomplished using well established procedures (Caruthers, Methodology of DNA and RNA Sequencing, (1983), Weissman (ed.), Praeger Publishers, New York, Chapter 1); automated chemical synthesis can be performed using one of a number of commercially available machines.

Optimal alignment of sequences for comparison may be conducted by the local homology algorithm of Smith and Waterman, Adv. Appl. Math. 2:482 (1981), by the homology alignment algorithm of Needleman and Wunsch, J. Mol. Biol. 48:443 (1970), by the search for similarity method of Pearson and Lipman Proc. Natl. Acad. Sci. (U.S.A.) 85: 2444 (1988), by computerized implementations of these algorithms (GAP, BESTFIT, FASTA, and TFASTA in the Wisconsin Genetics Software Package, Genetics Computer Group, 575 Science Dr., Madison, Wis.), or by inspection.

The NCBI Basic Local Alignment Search Tool (BLAST) (Altschul et al., 1990) is available from several sources, including the National Center for Biological Information (NCBI, Bethesda, Md.) and on the Internet, for use in connection with the sequence analysis programs blastp, blastn, blastx, tblastn and tblastx. It can be accessed at htp://www.ncbi.nlm.nih.gov/BLAST/. A description of how to determine sequence identity using this program is available at http://www.ncbi.nlm.nih.gov/BLAST/blast help.html.

The terms "substantial amino acid identity" or "substantial amino acid sequence identity" as applied to amino acid sequences and as used herein denote a characteristic of a polypeptide, wherein the peptide comprises a sequence that has at least 70 percent sequence identity, preferably 80 percent amino acid sequence identity, more preferably 90 percent amino acid sequence identity, and most preferably at least 99 to 100 percent sequence identity as compared to a reference group over region corresponding to the first amino acid following the cytochrome p450 motif GXRXCX(G/A) to the stop codon of the translated peptide.

The terms "substantial nucleic acid identity" or "substantial nucleic acid sequence identity" as applied to nucleic acid sequences and as used herein denote a characteristic of a polynucleotide sequence, wherein the polynucleotide comprises a sequence that has at least 75 percent sequence identity, preferably 81 percent amino acid sequence identity, more preferably at least 91 percent sequence identity, and most preferably at least 99 to 100 percent sequence identity as compared to a reference group over region corresponding to the first nucleic acid following the cytochrome p450 motif GXRXCX(G/A) to the stop codon of the translated peptide.

Another indication that nucleotide sequences are substantially identical is if two molecules hybridize to each other under stringent conditions. Stringent conditions are sequence-dependent and will be different in different circumstances. Generally, stringent conditions are selected to be about 5°C to about 20°C, usually about 10°C to about 15°C, lower than the thermal melting point (Tm) for the specific sequence at a defined ionic strength and pH. The Tm is the temperature (under defined ionic strength and pH) at which 50% of the target sequence hybridizes to a matched probe. Typically, stringent conditions will be those in which the salt concentration is about 0.02 molar at pH 7 and the temperature is at least about 60°C. For instance in a standard Southern hybridization procedure, stringent conditions will include an initial wash in 6xSSC at 42 °C followed by one or more additional washes in 0.2xSSC at a temperature of at least about 55°C, typically about 60°C and often about 65°C.

Nucleotide sequences are also substantially identical for purposes of this invention when the polypeptides and/or proteins which they encode are substantially identical. Thus, where one nucleic acid sequence encodes essentially the same polypeptide as a second nucleic acid sequence, the two nucleic acid sequences are substantially identical, even if they would not hybridize under stringent conditions due to degeneracy permitted by the genetic code (see, Darnell et al. (1990) Molecular Cell Biology, Second Edition Scientific American Books W. H. Freeman and Company New York for an explanation of codon degeneracy and the genetic code). Protein purity or homogeneity can be indicated by a number of means well known in the art, such as polyacrylamide gel electrophoresis of a protein sample, followed by visualization upon staining. For certain purposes high resolution may be needed and HPLC or a similar means for purification may be utilized.

As used herein, the term "vector" is used in reference to nucleic acid molecules that transfer DNA segment(s) into a cell. A vector may act to replicate DNA and may reproduce independently in a host cell. The term "vehicle" is sometimes used interchangeably with "vector." The term "expression vector" as used herein refers to a recombinant DNA molecule containing a desired coding sequence and appropriate nucleic acid sequences necessary for the expression of the operably linked coding sequence in a particular host organism. Nucleic acid sequences necessary for expression in prokaryotes usually include a promoter, an operator (optional), and a ribosome binding site, often along with other sequences. Eucaryotic cells are known to utilize promoters, enhancers, and termination and polyadenylation signals.

For the purpose of regenerating complete genetically engineered plants with roots, a nucleic acid may be inserted into plant cells, for example, by any technique such as in vivo inoculation or by any of the known in vitro tissue culture techniques to produce transformed plant cells that can be regenerated into complete plants. Thus, for example, the insertion into plant cells may be by in vitro inoculation by pathogenic or non-pathogenic *A. tumefaciens*. Other such tissue culture techniques may also be employed.

"Plant tissue" includes differentiated and undifferentiated tissues of plants, including, but not limited to, roots, shoots, leaves, pollen, seeds, tumor tissue and various forms of cells in culture, such as single cells, protoplasts, embryos and callus tissue. The plant tissue may be *in planta* or in organ, tissue or cell culture.

"Plant cell" as used herein includes plant cells *in planta* and plant cells and protoplasts in culture.

"cDNA" or "complementary DNA" generally refers to a single stranded DNA molecule with a nucleotide sequence that is complementary to an RNA molecule. cDNA is formed by the action of the enzyme reverse transcriptase on an RNA template.

### STRATEGIES FOR OBTAINING NUCLEIC ACID SEQUENCES

In accordance with the present invention, RNA was extracted from Nicotiana tissue of converter and non-converter Nicotiana lines. The extracted RNA was then used to create cDNA. Nucleic acid sequences of the present invention were then generated using two strategies.

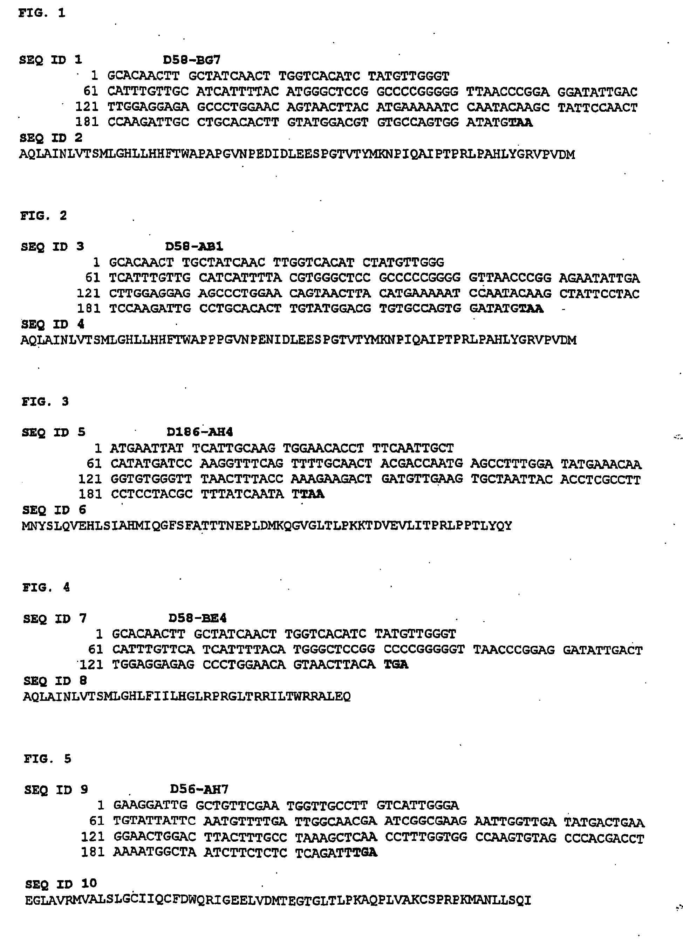

In the first strategy, the poly A enriched RNA was extracted from plant tissue and cDNA was made by reverse transcription PCR. The single strand cDNA was then used to create p450 specific PCR populations using degenerate primers plus a oligo d(T) reverse primer. The primer design was based on the highly conserved motifs of p450. Examples of specific degenerate primers are set forth in Figure 1. Sequence fragments from plasmids containing appropriate size inserts were further analyzed. These size inserts typically ranged from about 300 to about 800 nucleotides depending on which primers were used.

In a second strategy, a cDNA library was initially constructed. The cDNA in the plasmids was used to create p450 specific PCR populations using degenerate primers plus T7 primer on plasmid as reverse primer. As in the first strategy, sequence fragments from plasmids containing appropriate size inserts were further analyzed.

Nicotiana plant lines known to produce high levels of nornicotine (converter) and plant lines having undetectable levels of nornicotine may be used as starting materials.

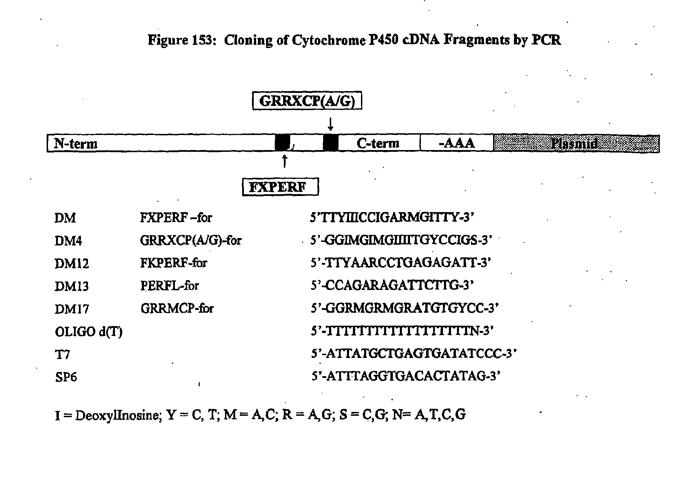

Leaves can then be removed from plants and treated with ethylene to activate p450 enzymatic activities defined herein. Total RNA is extracted using techniques known in the art. cDNA fragments can then be generated using PCR (RT-PCR) with the oligo d(T) primer as described in Figure 153. The cDNA library can then be constructed more fully described in examples herein.

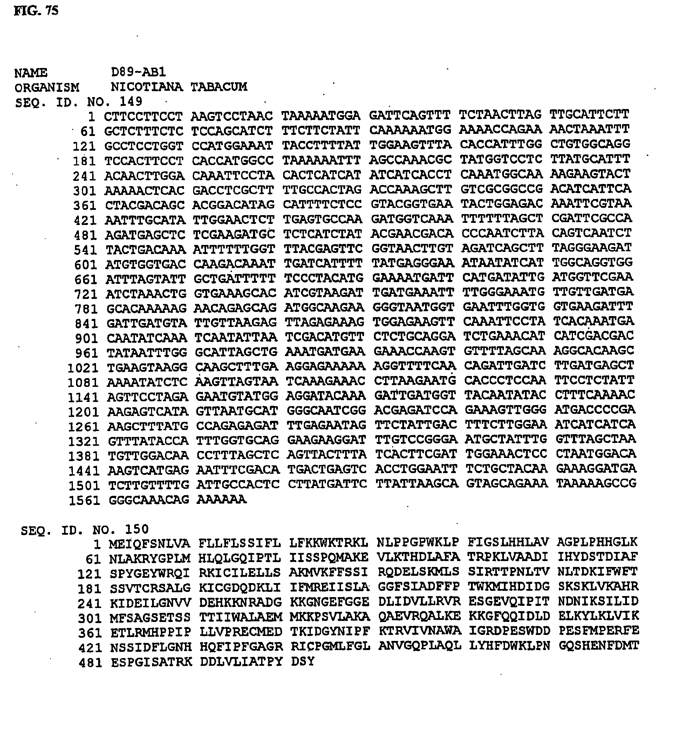

The conserved region of p450 type enzymes can be used as a template for degenerate primers (Figure 75). Using degenerate primers, p450 specific bands can be amplified by PCR. Bands indicative for p450 like enzymes can be identified by DNA sequencing. PCR fragments can be characterized using BLAST search, alignment or other tools to identify appropriate candidates.

Sequence information from identified fragments can be used to develop PCR primers. These primers in combination of plasmid primers in cDNA library were used to clone full length p450 genes. Large-scale Southern reverse analysis was conducted to examine the differential expression for all fragment clones obtained and in some cases full length clones. In this aspect of the invention, these large-scale reverse Southern assays can be conducted using labeled total cDNA's from different tissues as a probe to hybridize with cloned DNA fragments in order to screen all cloned inserts.

Nonradioactive and radioactive (P³²) Northern blotting assays were also used to characterize clones p450 fragments and full length clones.

Peptide specific antibodies were made against several full-length clones by deriving their amino acid sequence and selecting peptide regions that were antigenic and unique relative to other clones. Rabbit antibodies were made to synthetic peptides conjugated to a carrier protein. Western blotting analyses or other immunological methods were performed on plant tissue using these antibodies.

Nucleic acid sequences identified as described above can be examined by using virus induced gene silencing technology (VIGS, Baulcombe, Current Opinions in Plant Biology, 1999, 2:109-113).

Peptide specific antibodies were made for several full-length clones by deriving their amino acid sequence and selecting peptide regions that were potentially antigenic and were unique relative to other clones. Rabbit antibodies were made to synthetic petides conjugated to a carrier protein. Western blotting analyses were perfomed using these antibodies.

In another aspect of the invention, interfering RNA technology (RNAi) is used to further characterize cytochrome p450 enzymatic activities in Nicotiana plants of the present invention. The following references which describe this technology are incorporated by reference herein, Smith et al., Nature, 2000, 407:319-320; Fire et al., Nature, 1998, 391:306- 311; Waterhouse et al., PNAS, 1998, 95:13959-13964; Stalberg et al., Plant Molecular Biology, 1993, 23:671- 683; Baulcombe, Current Opinions in Plant Biology, 1999, 2:109-113; and Brigneti et al., EMBO Journal, 1998, 17(22):6739-6746. Plants may be transformed using RNAi techniques, antisense techniques, or a variety of other methods described.

Several techniques exist for introducing foreign genetic material into plant cells, and for obtaining plants that stably maintain and express the introduced gene. Such techniques include acceleration of genetic material coated onto microparticles directly into cells (US Patents 4,945,050 to Cornell and 5,141,131 to DowElanco). Plants may be transformed using Agrobacterium technology, see US Patent 5,177,010 to University of Toledo, 5,104,310 to Texas A&M, European Patent Application 0131624B1, European Patent Applications 120516, 159418B1, European Patent Applications 120516, 159418B1 and 176, 112 to Schilperoot, US Patents 5,149,645, 5,469,976, 5,464,763 and 4,940,838 and 4,693,976 to Schilperoot, European Patent Applications 116718, 290799, 320500 all to MaxPlanck, European Patent Applications 604662 and 627752 to Japan Nicotiana, European Patent Applications 0267159, and 0292435 and US Patent 5,231,019 all to Ciba Geigy, US Patents 5,463,174 and 4,762,785 both to Calgene, and US Patents 5, 004, 863 and 5, 159, 135 both to Agracetus. Other transformation technology includes whiskers technology, see U.S. Patents 5,302,523 and 5,464,765 both to Zeneca. Electroporation technology has also been used to transform plants, see WO 87/06614 to Boyce Thompson Institute, 5,472,869 and 5,384,253 both to Dekalb, WO9209696 and WO9321335 both to PGS. All of these transformation patents and publications are incorporated by reference. In addition to numerous technologies for transforming plants, the type of tissue which is contacted with the foreign genes may vary as well. Such tissue would include but would not be limited to embryogenic tissue, callus tissue type I and II, hypocotyl, meristem, and the like. Almost all plant tissues may be transformed during dedifferentiation using appropriate techniques within the skill of an artisan.

Foreign genetic material introduced into a plant may include a selectable marker. The preference for a particular marker is at the discretion of the artisan, but any of the following selectable markers may be used along with any other gene not listed herein which could function as a selectable marker. Such selectable markers include but are not limited to aminoglycoside phosphotransferase gene of transposon Tn5 (Aph II) which encodes resistance to the antibiotics kanamycin, neomycin and G418, as well as those genes which code for resistance or tolerance to glyphosate; hygromycin; methotrexate; phosphinothricin (bar); imidazolinones, sulfonylureas and triazolopyrimidine herbicides, such as chlorosulfuron; bromoxynil, dalapon and the like.

In addition to a selectable marker, it may be desirous to use a reporter gene. In some instances a reporter gene may be used without a selectable marker. Reporter genes are genes which are typically not present or expressed in the recipient organism or tissue. The reporter gene typically encodes for a protein which provide for some phenotypic change or enzymatic property. Examples of such genes are provided in K. Weising et al. Ann. Rev. Genetics, 22, 421 (1988), which is incorporated herein by reference. Preferred reporter genes include without limitation glucuronidase (GUS) gene and GFP genes.

Once introduced into the plant tissue, the expression of the structural gene may be assayed by any means known to the art, and expression may be measured as mRNA transcribed, protein synthesized, or the amount of gene silencing that occurs (see U.S. Patent No. 5,583,021 which is hereby incorporated by reference). Techniques are known for the in vitro culture of plant tissue, and in a number of cases, for regeneration into whole plants (EP Appln No. 88810309.0). Procedures for transferring the introduced expression complex to commercially useful cultivars are known to those skilled in the art.

Once plant cells expressing the desired level of p450 enzyme are obtained, plant tissues and whole plants can be regenerated therefrom using methods and techniques well-known in the art. The regenerated plants are then reproduced by conventional means and the introduced genes can be transferred to other strains and cultivars by conventional plant breeding techniques.

The following examples illustrate methods for carrying out the invention and should be understood to be illustrative of, but not limiting upon, the scope of the invention which is defined in the appended claims.

### EXAMPLES

### EXAMPLE I: DEVELOPMENT OF PLANT TISSUE AND ETHYLENE TREATMENT

### Plant Growth

Plants were seeded in pots and grown in a greenhouse for 4 weeks. The 4 week old seedlings were transplanted into individual pots and grown in the greenhouse for 2 months. The plants were watered 2 times a day with water containing 150ppm NPK fertilizer during growth. The expanded green leaves were detached from plants to do the ethylene treatment described below.

### Cell Line 78379

Tobacco line 78379, which is a burley tobacco line released by the University of Kentucky was used as a source of plant material. One hundred plants were cultured as standard in the art of growing tobacco and transplanted and tagged with a distinctive number (1-100). Fertilization and field management were conducted as recommended.

Three quarters of the 100 plants converted between 20 and 100% of the nicotine to nornicotine. One quarter of the 100 plants converted less than 5% of the nicotine to nornicotine. Plant number 87 had the least conversion (2%) while plant number 21 had 100% conversion. Plants converting less than 3% were classified as non-converters. Self-pollinated seed of plant number 87 and plant number 21, as well as crossed (21 x 87 and 87 x 21) seeds were made to study genetic and phenotypic differences. Plants from selfed 21 were converters, and 99% of selfs from 87 were non-converters. The other 1% of the plants from 87 showed low conversion (5-15%). Plants from reciprocal crosses were all converters.

### Cell Line 4407

Nicotiana line 4407, which is a burley line was used as a source of plant material. Uniform and representative plants (100) were selected and tagged. Of the 100 plants 97 were non-converters and three were converters. Plant number 56 had the least amount of conversion (1.2%) and plant number 58 had the highest level of conversion (96%). Self-pollenated seeds and crossed seeds were made with these two plants.

Plants from selfed-58 segregated with 3:1 converter to non-converter ratio. Plants 58-33 and 58-25, were identified as homozygous converter and nonconverter plant lines, respectively. The stable conversion of 58-33 was confirmed by analysis of its progenies of next generation.

### Cell Line PBLB01

PBLB01 is a burley line developed by ProfiGen, Inc. and was used as a source of plant material. The converter plant was selected from foundation seeds of PBLB01.

### Ethylene Treatment Procedures

Green leaves were detached from 2-3 month greenhouse grown plants and sprayed with 0.3% ethylene solution (Prep brand Ethephon (Rhone-Poulenc)). Each sprayed leaf was hung in a curing rack equipped with humidifier and covered with plastic. During the treatment, the sample leaves were periodically sprayed with the ethylene solution. Approximately 24-48 hour post ethylene treatment, leaves were collected for RNA extraction. Another sub-sample was taken for metabolic constituent analysis to determine the concentration of leaf metabolites and more specific constituents of interest such as a variety of alkaloids.

As an example, alkaloids analysis could be performed as follows. Samples (0.1 g) were shaken at 150 rpm with 0.5 ml 2N NaOH, and a 5 ml extraction solution which contained quinoline as an internal standard and methyl t-butyl ether. Samples were analyzed on a HP 6890 GC equipped with a FID detector. A temperature of 250°C was used for the detector and injector. An HP column (30m-0.32nm-1·m) consisting of fused silica crosslinked with 5% phenol and 95% methyl silicon was used at a temperature gradient of 110-185 °C at 10°C per minute. The column was operated at 100°C with a flow rate of 1.7cm³min⁻¹ with a split ratio of 40:1 with a 2·l injection volume using helium as the carrier gas.

### EXAMPLE 2: RNA ISOLATION

For RNA extractions, middle leaves from 2 month old greenhouse grown plants were treated with ethylene as described. The 0 and 24-48 hours samples were used for RNA extraction. In some cases, leaf samples under the senescence process were taken from the plants 10 days post flower-head removal. These samples were also used for extraction. Total RNA was isolated using Rneasy Plant Mini Kit® (Qiagen, Inc., Valencia, California) following manufacturer's protocol.

The tissue sample was ground under liquid nitrogen to a fine powder using a DEPC treated mortar and pestle. Approximately 100 milligrams of ground tissue were transferred to a sterile 1.5 ml eppendorf tube. This sample tube was placed in liquid nitrogen until all samples were collected. Then, 450µ-1 of Buffer RLT as provided in the kit (with the addition of Mercaptoethanol) was added to each individual tube. The sample was vortexed vigorously and incubated at 56° C for 3 minutes. The lysate was then, applied to the QIAshredder^{™} spin column sitting in a 2-ml collection tube, and centrifuged for 2 minutes at maximum speed. The flow through was collected and 0.5 volume of ethanol was added to the cleared lysate. The sample is mixed well and transferred to an Rneasy® mini spin column sitting in a 2 ml collection tube. The sample was centrifuged for 1 minute at 10,000rpm. Next, 700µl of buffer RW1 was pipetted onto the Rneasy® column and centrifuged for 1 minute at 10,000rpm. Buffer RPE was pipetted onto the Rneasy® column in a new collection tube and centrifuged for 1 minute at 10,000 rpm. Buffer RPE was again, added to the Rneasy® spin column and centrifuged for 2 minutes at maximum speed to dry the membrane. To eliminate any ethanol carry over, the membrane was placed in a separate collection tube and centrifuged for an additional 1 minute at maximum speed. The Rneasy® column was transferred into a new 1.5 ml collection tube, and 40 µl of Rnase-free water was pipetted directly onto the Rneasy® membrane. This final elute tube was centrifuged for 1 minute at 10,000rpm. Quality and quantity of total RNA was analyzed by denatured formaldehyde gel and spectrophotometer.

Poly(A)RNA was isolated using Oligotex^{™} poly A+ RNA purification kit (Qiagen Inc.) following manufacture's protocol. About 200 µg total RNA in 250 µl maximum volume was used. A volume of 250µl of Buffer OBB and 15 µl of Oligotex^{™} suspension was added to the 250 µl of total RNA. The contents were mixed thoroughly by pipetting and incubated for 3 minutes at 70°C on a heating block. The sample was then, placed at room temperature for approximately 20 minutes. The oligotex:mRNA complex was pelleted by centrifugation for 2 minutes at maximum speed. All but 50 µl of the supernatant was removed from the microcentrifuge tube. The sample was treated further by OBB buffer. The oligotex:mRNA pellet was resuspended in 400 µl of Buffer OW2 by vortexing. This mix was transferred onto a small spin column placed in a new tube and centrifuged for 1 minute at maximum speed. The spin column was transferred to a new tube and an additional 400 µl of Buffer OW2 was added to the column. The tube was then centrifuged for 1 minute at maximum speed. The spin column was transferred to a final 1.5ml microcentrifuge tube. The sample was eluted with 60 ul of hot (70°C) Buffer OEB. Poly A product was analyzed by denatured formaldehyde gels and spectrophotometric analysis.

### EXAMPLE 3: REVERSE TRANSCRIPTION-PCR

First strand cDNA was produced using SuperScript reverse transcriptase following manufacturer's protocol (Invitrogen, Carlsbad, California). The poly A+ enriched RNA/oligo dT primer mix consisted of less than 5 µg of total RNA, 1 µl of 10mM dNTP mix, 1 µl of Oligo d(T)₁₂₋₁₈ (0.5µg/µl), and up to 10 µl of DEPC-treated water. Each sample was incubated at 65°C for 5 minutes, then placed on ice for at least 1 minute. A reaction mixture was prepared by adding each of the following components in order: 2 µl 10X RT buffer, 4 µl of 25 mM MgCl2, 2µl of 0.1 M DTT, and 1 µl of RNase OUT Recombinant RNase Inhibitor. An addition of 9 µl of reaction mixture was pipetted to each RNA/primer mixture and gently mixed. It was incubated at 42°C for 2 minutes and 1 µl of Super Script II^{™} RT was added to each tube. The tube was incubated for 50 minutes at 42°C. The reaction was terminated at 70° C for 15 minutes and chilled on ice. The sample was collected by centrifugation and 1 µl of RNase H was added to each tube and incubated for 20 minutes at 37°C. The second PCR was carried out with 200 pmoles of forward primer (degenerate primers as in Figure 75, SEQ.ID Nos. 149-156) and 100 pmoles reverse primer (mix of 18nt oligo d(T) followed by 1 random base).

Reaction conditions were 94°C for 2 minutes and then performed 40 cycles of PCR at 94°C for 1 minute, 45° to 60°C for 2 minutes, 72°C for 3 minutes with a 72°C extension for an extra 10 min.

Ten microliters of the amplified sample were analyzed by electrophoresis using a 1% agarose gel. The correct size fragments were purified from agarose gel.

### EXAMPLE 4: GENERATION OF PCR FRAGMENT POPULATIONS

PCR fragments from Example 3 were ligated into a pGEM-T® Easy Vector (Promega, Madison, Wisconsin) following manufacturer's instructions. The ligated product was transformed into JM109 competent cells and plated on LB media plates for blue/white selection. Colonies were selected and grown in a 96 well plate with 1.2 ml of LB media overnight at 37°C. Frozen stock was generated for all selected colonies. Plasmid DNA from plates were purified using Beckman's Biomeck 2000 miniprep robotics with Wizard SV Miniprep® kit (Promega). Plasmid DNA was eluted with 100µl water and stored in a 96 well plate. Plasmids were digested by EcoR1 and were analyzed using 1% agarose gel to confirm the DNA quantity and size of inserts. The plasmids containing a 400-600 bp insert were sequenced using an CEQ 2000 sequencer (Beckman, Fullerton, California). The sequences were aligned with GenBank database by BLAST search. The p450 related fragments were identified and further analyzed. Alternatively, p450 fragments were isolated from substraction libraries. These fragments were also analyzed as described above.

### EXAMPLE 5: CONSTRUCTION OF CDNA LIBRARY

A cDNA library was constructed by preparing total RNA from ethylene treated leaves as follows. First, total RNA was extracted from ethylene treated leaves of tobacco line 58-33 using a modified acid phenol and chloroform extraction protocol. Protocol was modified to use one gram of tissue that was ground and subsequently vortexed in 5 ml of extraction buffer (100 mM Tris-HCl, pH 8.5; 200 mM NaCl; 10mM EDTA; 0.5% SDS) to which 5 ml phenol (pH5.5) and 5 ml chloroform was added. The extracted sample was centrifuged and the supernatant was saved. This extraction step was repeated 2-3 more times until the supernatant appeared clear. Approximately 5 ml of chloroform was added to remove trace amounts of phenol. RNA was precipitated from the combined supernatant fractions by adding a 3-fold volume of ETOH and 1/10 volume of 3M NaOAc (pH5.2) and storing at -20°C for 1 hour. After transferring to a Corex glass container the RNA fraction was centrifuged at 9,000 RPM for 45 minutes at 4°C. The pellet was washed with 70% ethanol and spun for 5 minutes at 9,000 RPM at 4°C. After drying the pellet, the pelleted RNA was dissolved in 0.5 ml RNase free water. The pelleted RNA was dissolved in 0.5 ml RNase free water. The quality and quantity of total RNA was analyzed by denatured formaldehyde gel and spectrophotometer, respectively.

The resultant total RNA was isolated for poly A+ RNA using an Oligo(dT) cellulose protocol (Invitrogen) and Microcentrifuge spin columns (Invitrogen) by the following protocol. Approximately twenty mg of total RNA was subjected to twice purification to obtain high quality poly A+ RNA. Poly A+ RNA product was analyzed by performing denatured formaldehyde gel and subsequent RT-PCR of known full-length genes to ensure high quality of mRNA.

Next, poly A+ RNA was used as template to produce a cDNA library employing cDNA synthesis kit, ZAP-cDNA® synthesis kit, and ZAP-cDNA® Gigapack® III gold cloning kit (Stratagene, La Jolla, California). The method involved following the manufacture's protocol as specified. Approximately 8 µg of poly A+ RNA was used to construct cDNA library. Analysis of the primary library revealed about 2.5 x 10⁶ - 1x 10⁷ pfu. A quality background test of the library was completed by complementation assays using IPTG and X-gal, where recombinant plaques was expressed at more than 100-fold above the background reaction.

A more quantitative analysis of the library by random PCR showed that average size of insert cDNA was approximately 1.2 kb. The method used a two-step PCR method as followed. For the first step, reverse primers were designed based on the preliminary sequence information obtained from p450 fragments. The designed reverse primers and T3 (forward) primers were used amplify corresponding genes from the cDNA library. PCR reactions were subjected to agarose electrophoresis and the corresponding bands of high molecular weight were excised, purified, cloned and sequenced. In the second step, new primers designed from 5'UTR or the start coding region of p450 as the forward primers together with the reverse primers (designed from 3'UTR of p450) were used in the subsequent PCR to obtain full-length p450 clones.

The p450 fragments were generated by PCR amplification from the constructed cDNA library as described in Example 3 with the exception of the reverse primer. The T7 primer located on the plasmid downstream of cDNA inserts (see Figure 75) was used as a reverse primer. PCR fragments were isolated, cloned and sequenced as described in Example 4.

Full-length p450 genes were isolated by PCR method from constructed cDNA library. Gene specific reverse primers (designed from the downstream sequence of p450 fragments) and a forward primer (T3 on library plasmid) were used to clone the full length genes. PCR fragments were isolated, cloned and sequenced. If necessary, second step PCR was applied. In the second step, new forward primers designed from 5'UTR of cloned p450s together with the reverse primers designed from 3'UTR of p450 clones were used in the subsequent PCR reactions to obtain full-length p450 clones. The clones were subsequently sequenced.

### EXAMPLE 6: CHARACTERIZATION OF CLONED FRAGMENTS - REVERSE SOUTHERN BLOTTING ANALYSIS

Nonradioactive large scale reverse southern blotting assays were performed on all p450 clones identified in above examples to detect the differential expression. It was observed that the level of expression among different p450 clusters was very different. Further real time detection was conducted on those with high expression.

Nonradioactive Southern blotting procedures were conducted as follows.
1) Total RNA was extracted from ethylene treated and nontreated converter (58-33) and nonconverter (58-25) leaves using the Qiagen Rnaeasy kit as described in Example 2.
2) Probe was produced by biotin-tail labeling a single strand cDNA derived from poly A+ enriched RNA generated in above step. This labeled single strand cDNA was generated by RT-PCR of the converter and nonconverter total RNA (Invitrogen) as described in Example 3 with the exception of using biotinalyted oligo dT as a primer (Promega). These were used as a probe to hybridize with cloned DNA.
3) Plasmid DNA was digested with restriction enzyme EcoR1 and run on agarose gels. Gels were simultaneously dried and transferred to two nylon membranes (Biodyne B®). One membrane was hybridized with converter probe and the other with nonconverter probe. Membranes were UV-crosslinked (auto crosslink setting, 254 nm, Stratagene, Stratalinker) before hybridization.
   Alternatively, the inserts were PCR amplified from each plasmid using the sequences located on both arms of p-GEM plasmid, T3 and SP6, as primers. The PCR products were analyzed by running on a 96 well Ready-to-run agarose gels. The confirmed inserts were dotted on two nylon membranes. One membrane was hybridized with converter probe and the other with nonconverter probe.
4) The membranes were hybridized and washed following manufacture's instruction with the modification of washing stringency (Enzo MaxSence™ kit, Enzo Diagnostics, Inc, Farmingdale, NY). The membranes were prehybridized with hybridization buffer (2x SSC buffered formamide, containing detergent and hybridization enhancers) at 42°C for 30 min and hybridized with 10µl denatured probe overnight at 42°C. The membranes then were washed in 1X hybridization wash buffer 1 time at room temperature for 10 min and 4 times at 68°C for 15 min. The membranes were ready for the detection.
5) The washed membranes were detected by alkaline phosphatase labeling followed by NBT/BCIP colometric detection as described in manufacture's detection procedure (Enzo Diagnostics, Inc.). The membranes were blocked for one hour at room temperature with 1x blocking solution, washed 3 times with 1X detection reagents for 10 min, washed 2 times with 1x predevelopment reaction buffer for 5 min and then developed the blots in developing solution for 30-45 min until the dots appear. All reagents were provided by manufacture (Enzo Diagnostics, Inc). In Addition, large scale reverse Southern assay was also performed using KPL southern hybridization and detection kit™ following manfacturer's instruction(KPL, Gaithersburg, Maryland).

### EXAMPLE 7: CHARACTERIZATION OF CLONES - NORTHERN BLOT ANALYSIS

Alternative to Southern Blot analysis, some membranes were hybridized and detected as described in the example of Northern blotting assays. Northern Hybridization was used to detect mRNA differentially expressed in Nicotiana as follows.

A random priming method was used to prepare probes from cloned p450 (Megaprime™ DNA Labelling Systems, Amersham Biosciences).

The following components were mixed: 25ng denatured DNA template; 4ul of each unlabeled dTTP, dGTP and dCTP; 5ul of reaction buffer; P³²-labelled dATP and 2ul of Klenow I; and H₂O, to bring the reaction to 50µl. The mixture was incubated in 37°C for 1-4 hours, then stopped with 2µl of 0.5 M EDTA. The probe was denatured by incubating at 95°C for 5 minutes before use.

RNA samples were prepared from ethylene treated and non-treated fresh leaves of several pairs of tobacco lines. In some cases poly A+ enriched RNA was used. Approximately 15µg total RNA or 1.8µg mRNA (methods of RNA and mRNA extraction as described in Example 5) were brought to equal volume with DEPC H₂O (5-10 µl). The same volume of loading buffer (1 x MOPS; 18.5 % Formaldehyde; 50 % Formamide; 4 % Ficoll400; Bromophenolblue) and 0.5 µl EtBr (0.5 µg/µl) were added. The samples were subsequently denatured in preparation for separation of the RNA by electrophoresis.

Samples were subjected to electrophoresis on a formaldehyde gel (1 % Agarose, 1 x MOPS, 0.6 M Formaldehyde) with 1XMOP buffer (0.4 M Morpholinopropanesulfonic acid; 0.1 M Na-acetate-3 x H2O; 10 mM EDTA; adjust to pH 7.2 with NaOH). RNA was transferred to a Hybond-N+ membrane (Nylon, Amersham Pharmacia Biotech) by capillary method in 10 X SSC buffer (1.5 M NaCl; 0.15 M Na-citrate) for 24 hours. Membranes with RNA samples were UV-crosslinked (auto crosslink setting, 254 nm, Stratagene, Stratalinker) before hybridization.

The membrane was prehybridized for 1-4 hours at 42°C with 5-10 ml prehybridization buffer (5 x SSC; 50 % Formamide; 5 x Denhardt's-solution; 1 % SDS; 100µg/ml heat-denatured sheared non- homologous DNA). Old prehybridization buffer was discarded, and new prehybridization buffer and probe were added. The hybridization was carried out over night at 42°C. The membrane was washed for 15 minutes with 2 x SSC at room temperature, followed by a wash with 2 x SSC.

A major focus of the invention was the discovery of novels genes that may be induced as a result of ethylene treatment or play a key role in tobacco leaf quality and constituents. As illustrated in the table below, Northern blots and reverse Southern Blot were useful in determining which genes were induced by ethylene treatment relative to non-induced plants. Interestingly, not all fragments were affected similarly in the converter and nonconverter. The cytochrome p450 fragments of interest were partially sequenced to determine their structural relatedness. This information was used to subsequently isolate and characterize full length gene clones of interest.

| Fragments | Induced mRNA Expression Ethylene Treatment |
|---|---|
| | Converter |
| D56-AC7 (SEQ ID No: 35) | + |
| D56-AG11 (SEQ ID No: 31) | + |
| D56-AC12 (SEQ ID No: 45) | + |
| D70A-AB5 (SEQ ID No: 95) | + |
| D73-AC9 (SEQ ID No: 43) | + |
| D70A-AA12 (SEQ ID No: 131) | + |
| D73A-AG3 (SEQ ID No: 129) | + |
| D34-52 (SEQ ID No: 61) | + |
| D56-AG6 (SEQ ID No: 51) | + |

Northern analysis was performed using full length clones on tobacco tissue obtained from converter and nonconverter burley lines that were induced by ethylene treatment. The purpose was to identify those full length clones that showed elevated expression in ethylene induced converter lines relative to ethylene induced converter lines relative to ethylene induced nonconverter burley lines. By so doing, the functionality relationship of full length clones may be determined by comparing biochemical differences in leaf constituents between converter and nonconverter lines. As shown in table below, six clones showed significantly higher expression, as denoted by ++ and +++, in converter ethylene treated tissue than that of nonconverter treated tissue, denoted by +. All of these clones showed little or no expression in converter and nonconverter lines that were not ethylene treated.

| **Full Length** **Clones** | **Converter** | **Nonconverter** |
|---|---|---|
| D101-BA2 | ++ | + |
| D207-AA5 | ++ | + |
| D208-AC8 | +++ | + |
| D237-AD1 | ++ | + |
| D89-AB1 | ++ | + |
| D90A-BB3 | ++ | + |

### EXAMPLE 8: IMMUNODETECTION OF p450S ENCODED BY THE CLONED GENES

Peptide regions corresponding to 20-22 amino acids in length from three p450 clones were selected for 1) having lower or no homology to other clones and 2) having good hydrophilicity and antigenicity. The amino acid sequences of the peptide regions selected from the respective p450 clones are listed below. The synthesized peptides were conjugated with KHL and then injected into rabbits. Antisera were collected 2 and 4 weeks after the 4^{th} injection (Alpha Diagnostic Intl. Inc. San Antonio, TX).
D234-AD1 DIDGSKSKLVKAHRKIDEILG
D90a-BB3 RDAFREKETFDENDVEELNY
D89-AB1 FKNNGDEDRHFSQKLGDLADKY

Antisera were examined for crossreactivity to target proteins from tobacco plant tissue by Western Blot analysis. Crude protein extracts were obtained from ethylene treated (0 to 40 hours) middle leaves of converter and nonconverter lines. Protein concentrations of the extracts were determined using RC DC Protein Assay Kit (BIO-RAD) following the manufacturer's protocol.

Two micrograms of protein were loaded onto each lane and the proteins separated on 10% - 20% gradient gels using the Laemmli SDS-PAGE system. The proteins were transferred from gels to PROTRAN® Nitrocellulose Transfer Membranes (Schleicher & Schuell) with the Trans-Blot® Semi-Dry cell (BIO-RAD). Target p450 proteins were detected and visualized with the ECL Advance™ Western Blotting Detection Kit (Amersham Biosciences). Primary antibodies against the synthetic-KLH conjugates were made in rabbits. Secondary antibody against rabbit IgG, coupled with peroxidase, was purchased from Sigma. Both primary and secondary antibodies were used at 1:1000 dilutions. Antibodies showed strong reactivity to a single band on the Western Blots indicating that the antisera were monospecific to the target peptide of interest. Antisera were also crossreactive with synthetic peptides conjuated to KLH.

### EXAMPLE 9: NUCLEIC ACID IDENTITY AND STRUCTURE RELATEDNESS OF ISOLATED NUCLEIC ACID FRAGMENTS

Over 100 cloned p450 fragments were sequenced in conjunction with Northern blot analysis to determine their structural relatedness. The approach used utilized forward primers based either of two common p450 motifs located near the carboxyl-terminus of the p450 genes. The forward primers corresponded to cytochrome p450 motifs FXPERF or GRRXCP(A/G) as denoted in Figure 1. The reverse primers used standard primers from either the plasmid, SP6 or T7 located on both arms of pGEM™ plasmid, or a poly A tail.
The protocol used is described below.

Spectrophotometry was used to estimate the concentration of starting double stranded DNA following the manufacturer's protocol (Beckman Coulter). The template was diluted with water to the appropriate concentration, denatured by heating at 95° C for 2 minutes, and subsequently placed on ice. The sequencing reaction was prepared on ice using 0.5 to 10µl of denatured DNA template, 2 µl of 1.6 pmole of the forward primer, 8 µl of DTCS Quick Start Master Mix and the total volume brought to 20 µl with water. The thermocycling program consisted of 30 cycles of the follow cycle: 96° C for 20 seconds, 50° C for 20 seconds, and 60° C for 4 minutes followed by holding at 4° C.

The sequence was stopped by adding 5 µl of stop buffer (equal volume of 3M NaOAc and 100mM EDTA and 1 µl of 20 mg/ml glycogen). The sample was precipitated with 60 µl of cold 95% ethanol and centrifuged at 6000g for 6 minutes. Ethanol was discarded. The pellet was 2 washes with 200 µl of cold 70% ethanol. After the pellet was dry, 40 µl of SLS solution was added and the pellet was resuspended. A layer of mineral oil was over laid. The sample was then, placed on the CEQ 8000 Automated Sequencer for further analysis.

In order to verify nucleic acid sequences, nucleic acid sequence was re-sequenced in both directions using forward primers to the FXPERF or GRRXCP(A/G) region of the p450 gene or reverse primers to either the plasmid or poly A tail. All sequencing was performed at least twice in both directions.

The nucleic acid sequences of cytochrome p450 fragments were compared to each other from the coding region corresponding to the first nucleic acid after the region encoding the GRRXCP(A/G) motif through to the stop codon. This region was selected as an indicator of genetic diversity among p450 proteins. A large number of genetically distinct p450 genes, in excess of 70 genes, were observed, similar to that of other plant species. Upon comparison of nucleic acid sequences, it was found that the genes could be placed into distinct sequences groups based on their sequence identity. It was found that the best unique grouping of p450 members was determined to be those sequences with 75% nucleic acid identity or greater (shown in Table I). Reducing the percentage identity resulted in significantly larger groups. A preferred grouping was observed for those sequences with 81% nucleic acid identity or greater, a more preferred grouping 91% nucleic acid identity or greater, and a most preferred grouping for those sequences 99% nucleic acid identity of greater. Most of the groups contained at least two members and frequently three or more members. Others were not repeatedly discovered suggesting that approach taken was able to isolated both low and high expressing mRNA in the tissue used.

Based on 75% nucleic acid identity or greater, two cytochrome p450 groups were found to contain nucleic acid sequence identity to previously tobacco cytochrome genes that genetically distinct from that within the group. Group 23, showed nucleic acid identity, within the parameters used for Table I, to prior GenBank sequences of GI:1171579 (CAA64635) and GI:14423327 (or AAK62346) by Czernic et al and Ralston et al, respectively. GI:1171579 had nucleic acid identity to Group 23 members ranging 96.9% to 99.5% identity to members of Group 23 while GI:14423327 ranged 95.4% to 96.9% identity to this group. The members of Group 31 had nucleic acid identity ranging from 76.7% to 97.8% identity to the GenBank reported sequence of GI:14423319 (AAK62342) by Ralston et al. None of the other p450 identity groups of Table 1 contained parameter identity, as used in Table 1, to Nicotiana p450s genes reported by Ralston et al, Czernic et al., Wang et al or LaRosa and Smigocki.

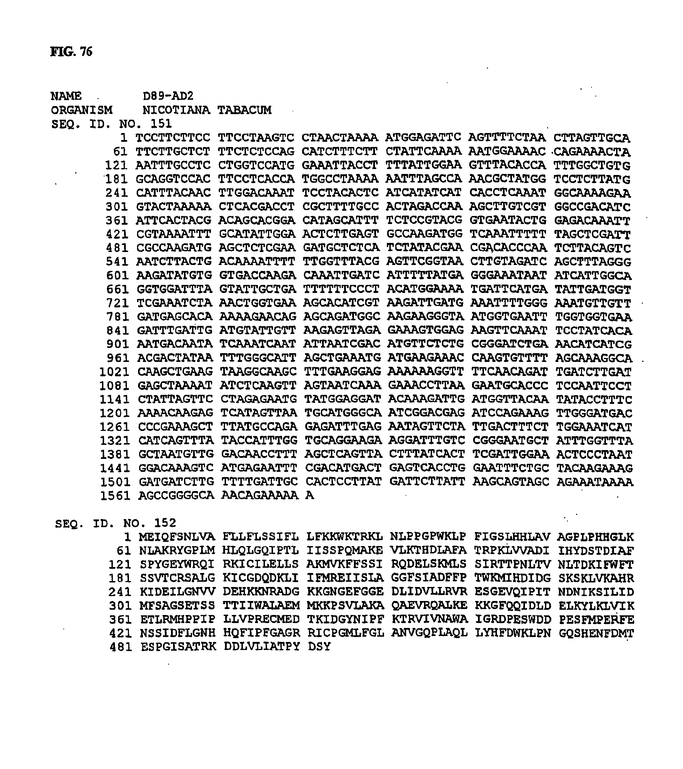

As shown in Figure 76, consensus sequence with appropriate nucleic acid degenerate probes could be derived for group to preferentially identify and isolate additional members of each group from Nicotiana plants.

**Table I: Nicotiana p450 Nucleic Acid Sequence Identity Groups**

| GROUP | FRAGMENTS |
|---|---|
| 1 | D58-BG7 (SEQ ID No.:1), D58-AB1 (SEQ ID No.:3); D58-BE4 (SEQ ID No.:7) |
| 2 | D56-AH7 (SEQ ID No.:9); D13a-5 (SEQ ID No.:11) |
| 3 | D56-AG10 (SEQ ID No.:13); D35-33 (SEQ ID No.:15); D34-62 (SEQ ID No.:17) |
| 4 | D56-AA7 (SEQ ID No.:19); D56-AE1 (SEQ ID No.:21); 185-BD3 (SEQ ID No.:143) |
| 5 | D35-BB7 (SEQ ID No.:23); D177-BA7 (SEQ ID No.:25); D56A-AB6 (SEQ ID No.:27); D144-AE2 (SEQ ID No.:29) |
| 6 | D56-AG11 (SEQ ID No.:31); D179-AA1 (SEQ ID No.:33) |
| 7 | D56-AC7 (SEQ ID No.:35); D144-AD1 (SEQ ID No.:37) |
| 8 | D144-AB5 (SEQ ID No.:39) |
| 9 | D181-AB5 (SEQ ID No.:41); D73-Ac9 (SEQ ID No.:43) |
| 10 | D56-AC12 (SEQ ID No.:45) |
| 11 | D58-AB9 (SEQ ID No.:47); D56-AG9 (SEQ ID No.:49); D56-AG6 (SEQ ID No.:51); D35-BG11 (SEQ ID No.:53); D35-42 (SEQ ID No.:55); D35-BA3 (SEQ ID No.:57); D34-57 (SEQ ID No.:59); D34-52 (SEQ ID No.:61); D34-25 (SEQ ID No.:63) |
| 12 | D56-AD10 (SEQ ID No.:65) |
| 13 | 56-AA11 (SEQ ID No.:67) |
| 14 | D177-BD5 (SEQ ID No.:69); D177-BD7 (SEQ ID No.:83) |
| 15 | D56A-AG10 (SEQ ID No.:71); D58-BC5 (SEQ ID No.:73); D58-AD12 (SEQ ID No.:75) |
| 16 | D56-AC11 (SEQ ID No.:77); D35-39 (SEQ ID No.:79); D58-BH4 (SEQ ID No.:81); D56-AD6 (SEQ ID No.:87) |
| 17 | D73A-AD6 (SEQ ID No.:89); D70A-BA11 (SEQ ID No.:91) |
| 18 | D70A-AB5 (SEQ ID No.:95); D70A-AA8 (SEQ ID No.:97) |
| 19 | D70A-AB8 (SEQ ID No.:99); D70A-BH2 (SEQ ID No.:101); D70A-AA4 (SEQ ID No.:103) |
| 20 | D70A-BA1 (SEQ ID No.:105); D70A-BA9 (SEQ ID No.:107) |
| 21 | D70A-BD4 (SEQ ID No.:109) |
| 22 | D181-AC5 (SEQ ID No.:111); D144-AH1 (SEQ ID No.:113); D34-65 (SEQ ID No.:115) |
| 23 | D35-BG2 (SEQ ID No.:117) |
| 24 | D73A-AH7 (SEQ ID No.:119) |
| 25 | D58-AA1 (SEQ ID No.:121); D185-BC1 (SEQ ID No.:133); D185-BG2 (SEQ ID No.:135) |
| 26 | D73-AE10 (SEQ ID No.:123) |
| 27 | D56-AC12 (SEQ ID No.:125) |
| 28 | D177-BF7 (SEQ ID No.:127); D185-BE1 (SEQ ID No.:137); D185-BD2 (SEQ ID No.:139) |
| 29 | D73A-AG3 (SEQ ID No.:129) |
| 30 | D70A-AA12 (SEQ ID No.:131); D176-BF2 (SEQ ID No.:85) |
| 31 | D176-BC3 (SEQ ID No.:145) |
| 32 | D176-BB3 (SEQ ID No.: 147) |
| 33 | D186-AH4 (SEQ ID No.:5) |

### EXAMPLE 10: RELATED AMINO ACID SEQUENCE IDENTITY OF ISOLATED NUCLEIC ACID FRAGMENTS

The amino acid sequences of nucleic acid sequences obtained for cytochrome p450 fragments from Example 8 were deduced. The deduced region corresponded to the amino acid immediately after the GXRXCP(A/G) sequence motif to the end of the carboxyl-terminus, or stop codon. Upon comparison of sequence identity of the fragments, a unique grouping was observed for those sequences with 70%'amino acid identity or greater. A preferred grouping was observed for those sequences with 80% amino acid identity or greater, more preferred with 90% amino acid identity or greater, and a most preferred grouping for those sequences. 99% amino acid identity of greater. The groups and corresponding amino acid sequences of group members are shown in Figure 2. Several of the unique nucleic acid sequences were found to have complete amino acid identity to other fragments and therefore only one member with the identical amino acid was reported.

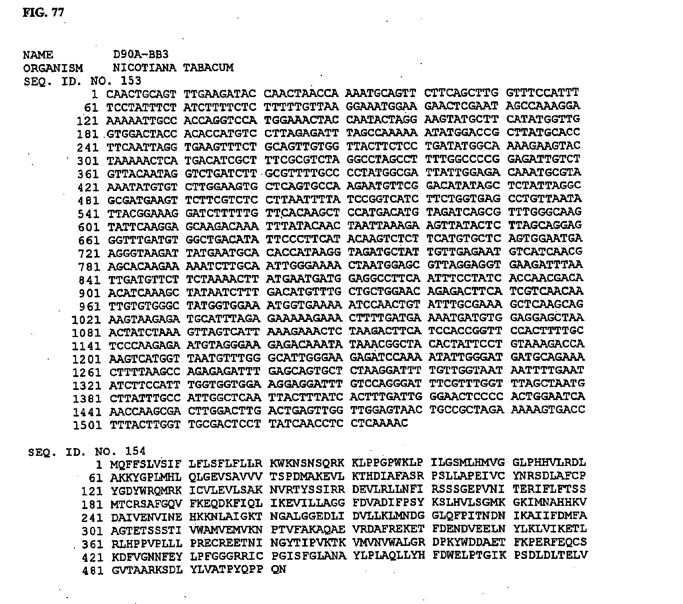

The amino acid identity for Group 19 of Table II corresponded to three distinct groups based on their nucleic acid sequences. The amino acid sequences of each group member and their identity is shown in Figure. 77. The amino acid differences are appropriated marked.

At least one member of each amino acid identity group was selected for gene cloning and functional studies using plants. In addition, group members that are differentially affected by ethylene treatment or other biological differences as assessed by Northern and Southern analysis were selected for gene cloning and functional studies. To assist in gene cloning, expression studies and whole plant evaluations, peptide specific antibodies will be prepared on sequence identity and differential sequence.

**Table II: Nicotiana p450 Amino Acid Sequence Identity Groups**

| GROUP | FRAGMENTS |
|---|---|
| 1 | D58-BG7 (SEQ ID No.:2), D58-AB1 (SEQ ID No.:4) |
| 2 | D58-BE4 (SEQ ID No.:8) |
| 3 | D56-AH7 (SEQ ID No.:10); D13a-5 (SEQ ID No.:12) |
| 4 | D56-AG10 (SEQ ID No.:14); D34-62 (SEQ ID No.:18) |
| 5 | D56-AA7 (SEQ ID No.:20); D56-AE1 (SEQ ID No.:22); 185-BD3 (SEQ ID No.:144) |
| 6 | D35-BB7 (SEQ ID No.:24); D177-BA7 (SEQ ID No.:26); D56A-AB6 (SEQ ID No.:28); D144-AE2 (SEQ ID No.:30) |
| 7 | D56-AG11 (SEQ ID No.:32); D179-AA1 (SEQ ID No.:34) |
| 8 | D56-AC7 (SEQ ID No.:36); D144-AD1 (SEQ ID No.:38) |
| 9 | D144-AB5 (SEQ ID No.:40) |
| 10 | D181-AB5 (SEQ ID No.:42); D73-Ac9 (SEQ ID No.:44) |
| 11 | D56-AC12 (SEQ ID No.:46) |
| 12 | D58-AB9 (SEQ ID No.:48); D56-AG9 (SEQ ID No.:50); D56-AG6 (SEQ ID No.:52); D35-BG11 (SEQ ID No.:54); D35-42 (SEQ ID No.:56); D35-BA3 (SEQ ID No.:58); D34-57 (SEQ ID No.:60); D34-52 (SEQ ID No.:62) |
| 13 | D56AD10 (SEQ ID No.:66) |
| 14 | 56-AA11 (SEQ ID No.:68) |
| 15 | D177-BD5 (SEQ ID No.:70); D177-BD7 (SEQ ID No.:84) |
| 16 | D56A-AG10 (SEQ ID No.:72); D58-BC5 (SEQ ID No.:74); D58-AD12 (SEQ ID No.:76) |
| 17 | D56-AC11 (SEQ ID No.:78); D56-AD6 (SEQ ID No.:88) |
| 18 | D73A-AD6 (SEQ ID No.90:) |
| 19 | D70A-AB5 (SEQ ID No.:96); D70A-AB8 (SEQ ID No.:100); D70A-BH2 (SEQ ID No.:102); D70A-AA4 (SEQ ID No.:104); D70A-BA1 (SEQ ID No.:106); D70A-BA9 (SEQ ID No.:108) |
| 20 | D70A-BD4 (SEQ ID No.:110) |
| 21 | D181-AC5 (SEQ ID No.:112); D144-AH1 (SEQ ID No.:114); D34-65 (SEQ ID No.:116) |
| 22 | D35-BG2 (SEQ ID No.:118) |
| 23 | D73A-AH7 (SEQ ID No.:120) |
| 24 | D58-AA1 (SEQ ID No.:122); D185-BC1 (SEQ ID No.:134); D185-BG2 (SEQ ID No.:136) |
| 25 | D73-AE10 (SEQ ID No.:124) |
| 26 | D56-AC12 (SEQ ID No.:126) |
| 27 | D177-BF7 (SEQ ID No.:128); 185-BD2 (SEQ ID No.:140) |
| 28 | D73A-AG3 (SEQ ID No.:130) |
| 29 | D70A-AA12 (SEQ ID No.:132); D176-BF2 (SEQ ID No.:86) |
| 30 | D176-BC3 (SEQ ID No.:146) |
| 31 | D176-BB3 (SEQ ID No.:148) |
| 32 | D186-AH4 (SEQ ID No.:6) |

### EXAMPLE 11: RELATED AMINO ACID SEQUENCE IDENTITY OF FULL LENGTH CLONES

The nucleic acid sequence of full length Nicotiana genes cloned in Example 5 were deduced for their entire amino acid sequence. Cytochrome p450 genes were identified by the presence of three conserved p450 domain motifs, which corresponded to UXXRXXZ, PXRFXF or GXRXC at the carboxyl-terminus where U is E or K, X is any amino acid and Z is P, T, S or M. It was also noted that two of the clones appeared nearly complete but lacked the appropriate stop codon, D130-AA1 and D101-BA2, however but both contained all three p450 cytochrome domains. All p450 genes were characterized for amino acid identity using a BLAST program comparing their full length sequences to each other and to known tobacco genes. The program used the NCBI special BLAST tool (Align two sequences (b12seq), http://www.ncbi.nlm.nih.gov/blast/b12seq/b12.html). Two sequences were aligned under BLASTN without filter for nucleic acid sequences and BLASTP for amino acid sequences. Based on their percentage amino acid identity, each sequence was grouped into identity groups where the grouping contained members that shared at least 85% identity with another member. A preferred grouping was observed for those sequences with 90% amino acid identity or greater, a more preferred grouping had 95% amino acid identity or greater, and a most preferred grouping had those sequences 99% amino acid identity or greater. Using these criteria, 25 unique groups were identified and are depicted in Table III

Within the parameters used for Table III for amino acid identity, three groups were found to contain greater than 85% or greater identity to known tobacco genes. Members of Group 5 had up to 96% amino acid identity for full length sequences to prior GenBank sequences of GI:14423327 (or AAK62346) by Ralston et al. Group 23 had up to 93% amino acid identity to GI:14423328 (or AAK62347) by Ralston et al. and Group 24 had 92% identity to GI:14423318 (or AAK62343) by Ralston et al.

**Table III: Amino Acid Sequence Identity Groups of Full Length Nicotiana p450 Genes**

| | |
|---|---|
| 1 | D208-AD9 (SEQ. ID. No. 224); D120-AH4 (SEQ. ID. No. 180); D121-AA8 (SEQ. ID. No. 182), D122-AF10 (SEQ. ID. No. 184); D103-AH3 (SEQ. ID. No. 222); D208-AC8 (SEQ. ID. No. 218); D-235-ABI (SEQ. ID. No. 246) |
| 2 | D244-AD4 (SEQ. ID. No. 250); D244-AB6 (SEQ. ID. No. 274) ; D285-AA8; D285-AB9; D268-AE2 (SEQ. ID. No. 270) |
| 3 | D100A-AC3 (SEQ. ID. No. 168); D100A-BE2 |
| 4 | D205-BE9 (SEQ. ID. No. 276); D205-BG9 (SEQ. ID. No. 202); D205-AH4 (SEQ. ID. No. 294) |
| 5 | D259-AB9 (SEQ. ID. No. 260) ; D257-AE4 (SEQ. ID. No. 268); D147-AD3 (SEQ. ID. No. 194) |
| 6 | D249-AE8 (SEQ. ID. No. 256); D-248-AA6 (SEQ. ID. No. 254) |
| 7 | D233-AG7 (SEQ. ID. No. 266; D224-BD11 (SEQ. ID. No. 240); DAF10 |
| 8 | D105-AD6 (SEQ. ID. No. 172); D215-AB5 (SEQ. ID. No. 220); D135-AE1 (SEQ. ID. No. 190) |
| 9 | D87A-AF3 (SEQ. ID. No. 216), D210-BD4 (SEQ. ID. No. 262) |
| 10 | D89-AB1 (SEQ. ID. No. 150); D89-AD2 (SEQ. ID. No. 152); 163-AG11 (SEQ. ID. No. 198); 163-AF12 (SEQ. ID. No. 196) |
| 11 | D267-AF10 (SEQ. ID. No. 296); D96-AC2 (SEQ. ID. No. 160); D96-AB6 (SEQ. ID. No. 158); D207-AA5 (SEQ. ID. No. 204); D207-AB4 (SEQ. ID. No. 206); D207-AC4 (SEQ. ID. No. 208) |
| 12 | D98-AG1 (SEQ. ID. No. 164); D98-AA1 (SEQ. ID. No. 162) |
| 13 | D209-AA12 (SEQ. ID. No. 212); D209-AA11; D209-AH10 (SEQ. ID. No. 214); D209-AH12 (SEQ. ID. No. 232); D90a-BB3 (SEQ. ID. No. 154) |
| 14 | D129-AD10 (SEQ. ID. No. 188); D104A-AE8 (SEQ. ID. No. 170) |
| 15 | D228-AH8 (SEQ. ID. No. 244); D228-AD7 (SEQ. ID. No. 241), D250-AC11 (SEQ. ID. No. 258); D247-AH1 (SEQ. ID. No. 252) |
| 16 | D128-AB7 (SEQ. ID. No. 186) ; D243-AA2 (SEQ. ID. No. 248); D125-AF11 (SEQ. ID. No. 228) |
| 17 | D284-AH5 (SEQ. ID. No. 298); D110-AF12 (SEQ. ID. No. 176) |
| 18 | D221-BB8 (SEQ. ID. No. 234) |
| 19 | D222-BH4 (SEQ. ID. No. 236) |
| 20 | D134-AE11 (SEQ. ID. No. 230) |
| 21 | D109-AH8 (SEQ. ID. No. 174) |
| 22 | D136-AF4 (SEQ. ID. No. 278) |
| 23 | D237-AD1 (SEQ. ID. No. 226) |
| 24 | D112-AA5 (SEQ. ID. No. 178) |
| 25 | D283-AC1 (SEQ. ID. No. 272) |

The full length genes were further grouped based on the highly conversed amino acid homology between UXXRXXZ p450 domain and GXRXC p450 domain near the end the carboxyl-terminus. As shown in Figure 3, individual clones were aligned for their sequence homology between the conserved domains relative to each other and placed in distinct identity groups. In several cases, although the nucleic acid sequence of the clone was unique, the amino acid sequence for the region was identical. The preferred grouping was observed for those sequences with 90% amino acid identity or greater, a more preferred group had 95% amino acid identity or greater, and a most preferred grouping had those sequences 99% amino acid identity of greater. The final grouping was similar to that based on the percent identity for the entire amino acid sequence of the clones except for Group 17 (of Table III) which was divided into two distinct groups.

Within the parameters used for amino acid identity in Table IV, three groups were found to contain 90% or greater identity to known tobacco genes. Members of Group 5 had up to 93.4% amino acid identity for full length sequences to prior GenBank sequences of GI:14423326 (AAK62346) by Ralston et al. Group 23 had up to 91.8% amino acid identity to GI:14423328 (or AAK62347) by Ralston et al. and Group 24 had 98.8% identity to GI:14423318 (or AAK62342) by Ralston et al.

**Table IV: Amino Acid Sequence Identity Groups of Regions between Conserved Domains of Nicotiana p450 Genes**

| | |
|---|---|
| 1 1 | D208-AD9 (SEQ. ID. No. 224); D120-AH4 (SEQ. ID. No. 180); D121-AA8 (SEQ. ID. No. 182), D122-AF10 (SEQ. ID. No. 184); D103-AH3 (SEQ. ID. No. 222); D208-AC8 (SEQ. ID. No. 218); D-235-ABI (SEQ. ID. No. 246) |
| 2 | D244-AD4 (SEQ. ID. No. 250); D244-AB6 (SEQ. ID. No. 274) ; D285-AA8; D285-AB9; D268-AE2 (SEQ. ID. No. 270) |
| 3 | D100A-AC3 (SEQ. ID. No. 168); D100A-BE2 |
| 4 | D205-BE9 (SEQ. ID. No. 276); D205-BG9 (SEQ. ID. No. 202); D205-AH4 (SEQ. ID. No. 294) |
| 5 | D259-AB9 (SEQ. ID. No. 260) ; D257-AE4 (SEQ. ID. No. 268); D147-AD3 (SEQ. ID. No. 194) |
| 6 | D249-AE8 (SEQ. ID. No. 256); D-248-AA6 (SEQ. ID. No. 254) |
| 7 | D233-AG7 (SEQ. ID. No. 266; D224-BD11 (SEQ. ID. No. 240); DAF10 |
| 8 | D105-AD6 (SEQ. ID. No. 172); D215-AB5 (SEQ. ID. No. 220); D135-AE1 (SEQ. ID. No. 190) |
| 9 | D87A-AF3 (SEQ. ID. No. 216), D210-BD4 (SEQ. ID. No. 262) |
| 10 | D89-AB1 (SEQ. ID. No. 150); D89-AD2 (SEQ. ID. No. 152); 163-AG11 (SEQ. ID. No. 198); 163-AF12 (SEQ. ID. No. 196) |
| 11 | D267-AF10 (SEQ. ID. No. 296); D96-AC2 (SEQ. ID. No. 160); D96-AB6 (SEQ. ID. No. 158); D207-AA5 (SEQ. ID. No. 204); D207-AB4 (SEQ. ID. No. 206); D207-AC4 (SEQ. ID. No. 208) |
| 12 | D98-AG1 (SEQ. ID. No. 164); D98-AA1 (SEQ. ID. No. 162) |
| 13 | D209-AA12 (SEQ. ID. No. 212); D209-AA11; D209-AH10 (SEQ. ID. No. 214); D209-AH12 (SEQ. ID. No. 232); D90a-BB3 (SEQ. ID. No. 154) |
| 14 | D129-AD10 (SEQ. ID. No. 188); D104A-AE8 (SEQ. ID. No. 170) |
| 15 | D228-AH8 (SEQ. ID. No. 244); D228-AD7 (SEQ. ID. No. 241), D250-AC11 (SEQ. ID. No. 258); D247-AH1 (SEQ. ID. No. 252) |
| 16 | D128-AB7 (SEQ. ID. No. 186) ; D243-AA2 (SEQ. ID. No. 248); D125-AF11 (SEQ. ID. No. 228) |
| 17 | D284-AH5 (SEQ. ID. No. 298); D110-AF12 (SEQ. ID. No. 176) |
| 18 | D221-BB8 (SEQ. ID. No. 234) |
| 19 | D222-BH4 (SEQ. ID. No. 236) |
| 20 | D134-AE11 (SEQ. ID. No. 230) |
| 21 | D109-AH8 (SEQ. ID. No. 174) |
| 22 | D136-AF4 (SEQ. ID. No. 278) |
| 23 | D237-AD1 (SEQ. ID. No. 226) |
| 24 | D112-AA5 (SEQ. ID. No. 178) |
| 25 | D283-AC1 (SEQ. ID. No. 272) |
| 26 | D110-AF12 (SEQ. ID. No. 176) |

### EXAMPLE 12: NICOTIANA CYTOCHROME P450 CLONES LACKING ONE OR MORE OF THE TOBACCO CYTOCHROME P450 SPECIFIC DOMAINS

Four clones had high nucleic acid homology, ranging 90% to 99%'nucleic acid homology, to other tobacco cytochrome genes reported in Table III. The four clones included D136-AD5, D138-AD12, D243-AB3 and D250-AC11. However, due to a nucleotide frameshift these genes did not contain one or more of three C-terminus cytochrome p450 domains and were excluded from identity groups presented in Table III or Table IV.

The amino acid identity of one clone, D95-AG1, did not contain the third domain, GXRXC, used to group p450 tobacco genes in Table III or Table IV. The nucleic acid homology of this clone had low homology to other tobacco cytochrome genes. This clone represents a novel and different group of cytochrome p450 genes in Nicotiana.

### EXAMPLE 13: USE OF NICOTIANA CYTOCHROME P450 FRAGMENTS AND CLONES IN ALTERED REGULARTION OF TOBACCO PROPERTIES

The use of tobacco p450 nucleic acid fragments or whole genes are useful in identifying and selecting those plants that have altered tobacco phenotypes or tobacco constituents and, more importantly, altered metabolites. Transgenic tobacco plants are generated by a variety of transformation systems that incorporate nucleic acid fragments or full length genes, selected from those reported herein, in orientations for either down-regulation, for example anti-sense orientation, or over-expression for example, sense orienation. For over-expression to full length genes, any nucleic acid sequence that encodes the entire or a functional part or amino acide sequence of the full-length genes described in this invention are desired that are effective for increasing the expression of a certain enzyme and thus resulting in phenotypic effect within Nicotiana. Nicotiana lines that are homozygous lines are obtained through a series of backcrossing and assessed for phenotypic changes including, but not limited to, analysis of endogenous p450 RNA, transcripts, p450 expressed peptides and concentrations of plant metabolites using techniques commonly avaiable to one having ordinary skill in the art.

The changes exhibited in the tobacco plans provide information on the functional role of the selected gene of interest or are of a utility as a preffered Nicotiana plant species.

Numerous modifications and variations in practice of the invention are expected to occur to those skilled in the art upon consideration of the foregoing detailed description of the invention. Consequently, such modifications and variations are intended to be included within the scope of the following claims.

The following pages 73 to 86 contain preferred embodiments. Accordingly, the term "claim" as used therein refers to such a "preferred embodiment".
1. An isolated nucleic acid molecule from Nicotiana, wherein said nucleic acid molecule comprises a nucleic acid sequence selected from the group consisting of SEQ. ID. No.:149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295 and 297.
2. An isolated nucleic acid molecule from Nicotiana wherein said nucleic acid molecule compres a nucleic acid sequence selected from the group consiting of SEQ. ID. No. 299 through SEQ. ID. No. 357.
3. An isolated protein from Nicotiana, wherein said protein comprises an amino acid sequence selected from the group consisting of SEQ. ID. No.:. 150, 152, 154, 156, 158, 160, 162, 164, 166, 168, 170, 172, 174, 176, 178, 180, 182, 184, 186, 188, 190, 192, 194, 196, 198, 200, 202, 204, 206, 208, 210, 212, 214, 216, 218, 220, 222, 224, 226, 228, 230, 232, 234, 236, 238, 240, 242, 244, 246, 248, 250, 252, 254, 256, 258, 260, 262, 264, 266, 268, 270, 272, 274, 276, 278, 280, 282, 284, 286, 288, 290, 292, 294, 296 and 298.
4. An isolated protein from Nicotiana, wherein said protein comprises an amino acid sequence comprising at least 85% amino acid identity to SEQ. ID. No. 180 or SEQ. ID. No. 182, SEQ. ID. No. 184 or SEQ. ID. No. 224.
5. An isolated protein from Nicotiana, wherein said protein comprises an amino acid sequence comprising at least 85% amino acid identity to SEQ. ID. No. 218 or SEQ. ID no. 246.
6. An isolated protein from Nicotiana, wherein said protein comprises an amino acid sequence comprising at least 85% amino acid identity to SEQ. ID. No. 168.
7. An isolated protein from Nicotiana, wherein said protein comprises an amino acid sequence comprising at least 85% amino acid identity to SEQ. ID. No. 202, 204 or SEQ. ID. No. 276.
8. An isolated protein from Nicotiana, wherein said protein comprises an amino acid sequence comprising at least 85% amino acid identity to SEQ. ID. No. 20, SEQ. ID. No. 260, or SEQ. ID. No. 268.
9. An isolated protein from Nicotiana, wherein said protein comprises an amino acid sequence comprising at least 85% amino acid identity to SEQ. ID. No. 256 and SEQ. ID. No. 254.
10. An isolated protein from Nicotiana, wherein said wherein said protein comprises an amino acid sequence comprising at least 85%'amino acid identity to SEQ. ID. No. 266 or SEQ. ID. No. 240.
11. An isolated protein from Nicotiana, wherein said protein comprises an amino acid sequence comprising at least 85% amino acid identity to SEQ. ID. No. 172, SEQ. ID. No. 190 or SEQ. ID. No. 220.
12. An isolated protein from Nicotiana, wherein said protein comprises an amino acid sequence comprising at least 85% amino acid identity to SEQ. ID. No. 216 or SEQ. ID. No. 262.
13. An isolated protein from Nicotiana, wherein said protein comprises an amino acid sequence comprising at least 85% amino acid identity to SEQ. ID. No. 50, SEQ. ID. No. 152, SEQ. ID. No. 196 or SEQ. ID No. 198.
14. An isolated protein from Nicotiana, wherein said protein comprises an amino acid sequence comprising at least 85% amino acid identity to SEQ. ID. No. 296, SEQ. ID. No. 160, SEQ. ID. No. 158, SEQ. ID. No. 204 SEQ. ID. No. 206 and SEQ. ID. No. 208.
15. An isolated protein from Nicotiana, wherein said protein comprises an amino acid sequence comprising at least 85% amino acid identity to SEQ. ID. No. 162 or SEQ. ID. No. 164.
16. An isolated protein from Nicotiana, wherein said protein comprises an amino acid sequence comprising at least 85% amino acid identity to SEQ. ID. No. 212, 214 238 or 254.
17. An isolated protein from Nicotiana, wherein said protein comprises an amino acid sequence comprising at least 85% amino acid identity to SEQ. ID. No. 188 or 170.
18. An isolated protein from Nicotiana, wherein said protein comprises an amino acid sequence comprising at least 85% amino acid identity to SEQ. ID. No.214, 241, 258 or 252.
19. An isolated protein from Nicotiana, wherein said protein comprises an amino acid sequence comprising at least 85% amino acid identity to SEQ. ID. No. 186, SEQ. ID. No. 248, or SEQ. ID. No. 228.
20. An isolated protein from Nicotiana, wherein said protein comprises an amino acid sequence comprising at least 85% amino acid identity to SEQ. ID. No. 298 or SEQ. ID. No. 176.
21. An isolated protein from Nicotiana, wherein said protein comprises an amino acid sequence comprising at least 85% amino acid identity to SEQ. ID. No. 234.
22. An isolated protein from Nicotiana, wherein said protein comprises an amino acid sequence comprising at least 85% amino acid identity to SEQ. ID. No. 236.
23. An isolated protein from Nicotiana, wherein said protein comprises an amino acid sequence comprising at least 85% amino acid identity to SEQ. ID. No. 230.
24. An isolated protein from Nicotiana, wherein said protein comprises an amino acid sequence comprising at least 85% amino acid identity to SEQ. ID. No.174.
25. An isolated protein from Nicotiana, wherein said protein comprises an amino acid sequence comprising at least 85% amino acid identity to SEQ. ID. No. 174.
26. An isolated protein from Nicotiana, wherein said protein comprises an amino acid sequence comprising at least 85% amino acid identity to SEQ. ID. No. 226.
27. An isolated protein from Nicotiana, wherein said protein comprises an amino acid sequence comprising at least 85% amino acid identity to SEQ. ID. No. 178.
28. An isolated protein from Nicotiana, wherein said protein comprises an amino acid sequence comprising at least 85% amino acid identity to SEQ. ID. No. 272.
29. An isolated protein from Nicotiana, wherein said protein comprises an amino acid sequence comprising at least 90% amino acid identity to SEQ. ID. No. 180 or SEQ. ID. No. 182, SEQ. ID. No. 184 or SEQ. ID. No. 224.
30. An isolated protein from Nicotiana, wherein said protein has at least 90% homology to an amino acid sequence comprising SEQ. ID. No. 218 or SEQ. ID no. 246.
31. An isolated protein from Nicotiana, wherein said protein comprises an amino acid sequence comprising at least 90% amino acid identity to SEQ. ID. No.168
32. An isolated protein from Nicotiana, wherein said protein comprises an amino acid sequence comprising at least 90% amino acid identity to SEQ. ID. No. 202, 204 or SEQ. ID. No. 276.
33. An isolated protein from Nicotiana, wherein said protein comprises an amino acid sequence comprising at least 90% amino acid identity to SEQ. ID. No. 20, SEQ. ID. No. 260, or SEQ. ID. No. 268.
34. An isolated protein from Nicotiana, wherein said protein comprises an amino acid sequence comprising at least 90% amino acid identity to SEQ. ID. No. 256 and SEQ. ID. No. 254.
35. An isolated protein from Nicotiana, wherein said wherein said protein comprises an amino acid sequence comprising at least 85% amino acid identity to SEQ. ID. No. 266 or SEQ. ID. No. 240.
36. An isolated protein from Nicotiana, wherein said protein comprises an amino acid sequence comprising at least 90% amino acid identity to SEQ. ID. No. 172, SEQ. ID. No. 190 or SEQ. ID. No. 220.
37. An isolated protein from Nicotiana, wherein said protein comprises an amino acid sequence comprising at least 90% amino acid identity to SEQ. ID. No. 216 or SEQ. ID. No. 262.
38. An isolated protein from Nicotiana, wherein said protein comprises an amino acid sequence comprising at least 90% amino acid identity to SEQ. ID. No. 50, SEQ. ID. No. 152, SEQ. ID. No. 196 or SEQ. ID No. 198.
39. An isolated protein from Nicotiana, wherein said protein comprises an amino acid sequence comprising at least 90% amino acid identity to SEQ. ID. No. 296, SEQ. ID. No. 160, SEQ. ID. No. 158, SEQ. ID. No. 204 SEQ. ID. No. 206 and SEQ. ID. No. 208.
40. An isolated protein from Nicotiana, wherein said protein comprises an amino acid sequence comprising at least 90% amino acid identity to SEQ. ID. No. 162 or SEQ. ID. No. 164.
41. An isolated protein from Nicotiana, wherein said protein comprises an amino acid sequence comprising at least 90% amino acid identity to SEQ. ID. No. 212, 214 238 or 254.
42. An isolated protein from Nicotiana, wherein said protein comprises an amino acid sequence comprising at least 90% amino acid identity to SEQ. ID. No. 188 or 170.
43. An isolated protein from Nicotiana, wherein said protein comprises an amino acid sequence comprising at least 90% amino acid identity to SEQ. ID. No.214, 241, 258 or 252.
44. An isolated protein from Nicotiana, wherein said protein comprises an amino acid sequence comprising at least 90% amino acid identity to SEQ. ID. No. 186, SEQ. ID. No. 248, or SEQ. ID. No. 228.
45. An isolated protein from Nicotiana, wherein said protein comprises an amino acid sequence comprising at least 90% amino acid identity to SEQ. ID. No. 298 or SEQ. ID. No. 176.
46. An isolated protein from Nicotiana, wherein said protein comprises an amino acid sequence comprising at least 90% amino acid identity to SEQ. ID. No. 234.
47. An isolated protein from Nicotiana, wherein said protein comprises an amino acid sequence comprising at least 90% amino acid identity to SEQ. ID. No. 236.
48. An isolated protein from Nicotiana, wherein said protein comprises an amino acid sequence comprising at least 90% amino acid identity to SEQ. ID. No. 230.
49. An isolated protein from Nicotiana, wherein said protein comprises an amino acid sequence comprising at least 90% amino acid identity to SEQ. ID. No.174.
50. An isolated protein from Nicotiana, wherein said protein comprises an amino acid sequence comprising at least 90% amino acid identity to SEQ. ID. No. 174.
51. An isolated protein from Nicotiana, wherein said protein comprises an amino acid sequence comprising at least 90% amino acid identity to SEQ. ID. No. 226.
52. An isolated protein from Nicotiana, wherein said protein comprises an amino acid sequence comprising at least 90% amino acid identity to SEQ. ID. No. 178.
53. An isolated protein from Nicotiana, wherein said protein comprises an amino acid sequence comprising at least 90% amino acid identity to SEQ. ID. No. 272.
54. A transgenic plant, wherein said transgenic plant comprises the nucleic acid molecule of Claim 1 or 2.
55. The transgenic plant of Claim 54, wherein said plant is a tobacco plant.
56. A method of producing a transgenic plant, wherein said method comprises the steps of:
   (i) operably linking said nucleic acid molecule of any one of the Claims 1 or 2 with a promoter functional in said plant to create a plant transformational vector;
   (ii) transforming said plant with said plant transformational vector of step;
   (iii) selecting a plant cell transformed with said transformation vector; and
   (iv) regenerating a transformation plant from said transformed plant cell.
57. The method of Claim 56, wherein said nucleic acid molecule is in an antisense orientation.
58. The method of Claim 56, wherein said nucleic acid molecule is in a sense orientation.
59. The method of Claim 56, wherein said nucleic acid molecule is in a RNA interference orientation.
60. The method of Claim 56, wherein said nucleic acid molecule is expressed as a double stranded RNA molecule.
61. The method of Claim 56, wherein said double stranded RNA molecule is about 15 to 25 nucleotides in length.
62. The method of Claim 56, wherein said transgenic plant is a tobacco plant.
63. A method of selecting a plant containing a nucleic acid molecule, wherein said plant is analyzed for the presence of nucleic acid sequence selected from the group consisting of 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295 and 297.
64. The method of selecting a plant of Claim 63, wherein said plant is analyzed by DNA hybridization.
65. The method of selecting a plant of Claim 64, wherein said DNA hybridization is Southern blot analysis.
66. The method of selecting a plant of Claim 65, wherein said DNA hybridization is Northern blot analysis.
67. The method of selecting a plant of Claim 66, wherein said plant is analyzed by PCR detection.
68. The method of Claim 67, wherein said plant is a tobacco plant.
69. The method of Claim 85, wherein said DNA hybridization comprises a nucleic acid probe, said nucleic acid probe is a nucleic acid fragment comprising a nucleic acid sequence selected from the group consisting of 149, 151, 153, 155, 157, 159, 161, 163, 165, 167, 169, 171, 173, 175, 177, 179, 181, 183, 185, 187, 189, 191, 193, 195, 197, 199, 201, 203, 205, 207, 209, 211, 213, 215, 217, 219, 221, 223, 225, 227, 229, 231, 233, 235, 237, 239, 241, 243, 245, 247, 249, 251, 253, 255, 257, 259, 261, 263, 265, 267, 269, 271, 273, 275, 277, 279, 281, 283, 285, 287, 289, 291, 293, 295 and 297.
70. The method of selecting a plant of Claim 69, wherein said plant is a transgenic plant.
71. The method of selecting a plant of Claim 69, wherein said plant is selected from a mutagenesis population.
72. The method of selecting a plant of Claim 69, wherein said plant is selected from a breeding population.
73. The method of selecting a plant of Claim 69, wherein said plant is selected from a Nicotiana.

## Claims

1. An isolated nucleic acid encoding an amino acid sequence having at least 85% sequence identity to SEQ ID NO: 182.

2. The isolated nucleic acid of claim 1, wherein said nucleic acid encodes an amino acid sequence having at least 99% identity to SEQ ID NO: 182, in particular wherein said nucleic acid has 100% identity to SEQ ID NO: 181.

3. An isolated protein having at least 90% sequence identity to SEQ ID NO: 301.

4. An isolated protein having at least 85% identity to SEQ ID NO: 182, in particular wherein said protein has at least 90% amino acid identity to SEQ ID NO: 182.

5. A transgenic plant stably transformed with the nucleic acid of Claim 1 or 2.

6. The transgenic plant of Claim 5, wherein said plant is a tobacco plant.

7. A method of producing a transgenic plant, wherein said method comprises the Steps of:
i) operably linking the nucleic acid of Claim 1 or 2 with a promoter functional in said plant to create a plant transformational vector;
ii) transforming said plant with said plant transformational vector of step (i);
iii) selecting a plant cell transformed with said transformation vector; and
iv) regenerating a transformation plant from said plant cell of step (iii).

8. The method of Claim 7, wherein said nucleic acid is in an antisense orientation; or wherein said nucleic acid is in a sense orientation; or wherein said nucleic acid is in a RNA interference orientation; or wherein said nucleic acid is expressed as a double stranded RNA, in particular wherein said double stranded RNA molecule is about 15 to 25 nucleotides in length.

9. The method of Claim 7 or 8, wherein said transgenic plant is a tobacco plant.

10. The method of any one of Claims 7-9, wherein the method further comprises step v) selecting a transgenic plant having reduced or silenced expression of a p450 enzyme.

11. The method of Claim 10, wherein the p450 enzyme is a p450 demethylase, in particular a p450 nicotine demethylase.

12. A method of screening for plants containing a nucleic acid, comprising evaluating plant nucleic acid materials using a nucleic acid probe in conjunction with nucleic acid detection protocols for the presence of the nucleic acid of claim 1 or 2 and identifying a plant that contains said nucleic acid.

13. The method of Claim 12, wherein said nucleic acid detection protocol comprises nucleic acid hybridization, in particular wherein said nucleic acid detection protocol comprises PCR analysis.

14. The method of claim 12 or 13, wherein said plant is a tobacco plant, in particular wherein said plant is a transgenic plant or wherein said plant is part of a mutagenesis program or wherein said plant is part of a breeding program for traditional or transgenic varieties.

15. Use of a nucleic acid according to claim 1 or 2 for reducing or silencing the expression of a p450 enzyme in a plant, in particular in a tobacco plant.

16. The use of claim 16 or 17, wherein the nucleic acid is in an antisense orientation or in a sense orientation or expressed as a double stranded RNA.

17. The use of any one of claims 15-17, wherein the p450 enzyme is a p450 demethylase, in particular a p450 nicotine demethylase.

18. The use of any one of claims 15-17, wherein the p450 enzyme is a p450 enzyme found in a plant converting more than 3% nicotine to nornicotine.


SEQUENCE LISTING

<110>  U. S. Smokeless Tobacco Company

<120>  Cloning of Cytochrome P450 Genes From Nicotiana

<130>  07678/142EP4

<150>  PCT/US2003/032722
<151>  2003-10-16

<150>  US 60/503,989
<151>  2003-09-18

<150>  US 60/485,368
<151>  2003-07-08

<150>  US 60/418,933
<151>  2002-10-16

<160>  383

<170>  PatentIn version 3.3

<210>  1
<211>  207
<212>  DNA
<213>  Nicotiana tabacum

<400>  1
gcacaacttg ctatcaactt ggtcacatct atgttgggtc atttgttgca tcattttaca      60

tgggctccgg ccccgggggt taacccggag gatattgact tggaggagag ccctggaaca     120

gtaacttaca tgaaaaatcc aatacaagct attccaactc caagattgcc tgcacacttg     180

tatggacgtg tgccagtgga tatgtaa                                         207


<210>  2
<211>  68
<212>  PRT
<213>  Nicotiana tabacum

<400>  2

Ala Gln Leu Ala Ile Asn Leu Val Thr Ser Met Leu Gly His Leu Leu
1               5                   10                  15


His His Phe Thr Trp Ala Pro Ala Pro Gly Val Asn Pro Glu Asp Ile
            20                  25                  30


Asp Leu Glu Glu Ser Pro Gly Thr Val Thr Tyr Met Lys Asn Pro Ile
        35                  40                  45


Gln Ala Ile Pro Thr Pro Arg Leu Pro Ala His Leu Tyr Gly Arg Val
    50                  55                  60


Pro Val Asp Met
65


<210>  3
<211>  207

<212>  DNA
<213>  Nicotiana tabacum

<400>  3
gcacaacttg ctatcaactt ggtcacatct atgttgggtc atttgttgca tcattttacg      60

tgggctccgc ccccgggggt taacccggag aatattgact tggaggagag ccctggaaca     120

gtaacttaca tgaaaaatcc aatacaagct attcctactc caagattgcc tgcacacttg     180

tatggacgtg tgccagtgga tatgtaa                                         207


<210>  4
<211>  68
<212>  PRT
<213>  Nicotiana tabacum

<400>  4

Ala Gln Leu Ala Ile Asn Leu Val Thr Ser Met Leu Gly His Leu Leu
1               5                   10                  15


His His Phe Thr Trp Ala Pro Pro Pro Gly Val Asn Pro Glu Asn Ile
            20                  25                  30


Asp Leu Glu Glu Ser Pro Gly Thr Val Thr Tyr Met Lys Asn Pro Ile
        35                  40                  45


Gln Ala Ile Pro Thr Pro Arg Leu Pro Ala His Leu Tyr Gly Arg Val
    50                  55                  60


Pro Val Asp Met
65


<210>  5
<211>  183
<212>  DNA
<213>  Nicotiana tabacum

<400>  5
atgaattatt cattgcaagt ggaacacctt tcaattgctc atatgatcca aggtttcagt      60

tttgcaacta cgaccaatga gcctttggat atgaaacaag gtgtgggttt aactttacca     120

aagaagactg atgttgaagt gctaattaca cctcgccttc ctcctacgct ttatcaatat     180

taa                                                                   183


<210>  6
<211>  60
<212>  PRT
<213>  Nicotiana tabacum

<400>  6

Met Asn Tyr Ser Leu Gln Val Glu His Leu Ser Ile Ala His Met Ile
1               5                   10                  15


Gln Gly Phe Ser Phe Ala Thr Thr Thr Asn Glu Pro Leu Asp Met Lys
            20                  25                  30

Gln Gly Val Gly Leu Thr Leu Pro Lys Lys Thr Asp Val Glu Val Leu
        35                  40                  45

Ile Thr Pro Arg Leu Pro Pro Thr Leu Tyr Gln Tyr
    50                  55                  60

<210>  7
<211>  132
<212>  DNA
<213>  Nicotiana tabacum

<400>  7
gcacaacttg ctatcaactt ggtcacatct atgttgggtc atttgttcat cattttacat      60

gggctccggc cccgggggtt aacccggagg atattgactt ggaggagagc cctggaacag     120

taacttacat ga                                                         132

<210>  8
<211>  40
<212>  PRT
<213>  Nicotiana tabacum

<400>  8

Ala Gln Leu Ala Ile Asn Leu Val Thr Ser Met Leu Gly His Leu Phe
1               5                   10                  15

Ile Ile Leu His Gly Leu Arg Pro Arg Gly Leu Thr Arg Arg Ile Leu
            20                  25                  30

Thr Trp Arg Arg Ala Leu Glu Gln
        35                  40

<210>  9
<211>  189
<212>  DNA
<213>  Nicotiana tabacum

<400>  9
gaaggattgg ctgttcgaat ggttgccttg tcattgggat gtattattca atgttttgat      60

tggcaacgaa tcggcgaaga attggttgat atgactgaag gaactggact tactttgcct     120

aaagctcaac ctttggtggc caagtgtagc ccacgaccta aaatggctaa tcttctctct     180

cagatttga                                                             189

<210>  10
<211>  62
<212>  PRT
<213>  Nicotiana tabacum

<400>  10

Glu Gly Leu Ala Val Arg Met Val Ala Leu Ser Leu Gly Cys Ile Ile
1               5                   10                  15

Gln Cys Phe Asp Trp Gln Arg Ile Gly Glu Glu Leu Val Asp Met Thr
            20                  25                  30

Glu Gly Thr Gly Leu Thr Leu Pro Lys Ala Gln Pro Leu Val Ala Lys
        35                  40                  45

Cys Ser Pro Arg Pro Lys Met Ala Asn Leu Leu Ser Gln Ile
    50                  55                  60

<210>  11
<211>  189
<212>  DNA
<213>  Nicotiana tabacum

<400>  11
gaaggattgg ctattcgaat ggttgcattg tcattgggat gtattattca atgctttgat      60

tggcaacgac ttggggaagg attggttgat aagactgaag gaactggact tactttgcct     120

aaagctcaac ctttagtggc caagtgtagc ccacgaccta taatggctaa tcttctttct     180

cagatttga                                                             189

<210>  12
<211>  62
<212>  PRT
<213>  Nicotiana tabacum

<400>  12

Glu Gly Leu Ala Ile Arg Met Val Ala Leu Ser Leu Gly Cys Ile Ile
1               5                   10                  15

Gln Cys Phe Asp Trp Gln Arg Leu Gly Glu Gly Leu Val Asp Lys Thr
            20                  25                  30

Glu Gly Thr Gly Leu Thr Leu Pro Lys Ala Gln Pro Leu Val Ala Lys
        35                  40                  45

Cys Ser Pro Arg Pro Ile Met Ala Asn Leu Leu Ser Gln Ile
    50                  55                  60

<210>  13
<211>  183
<212>  DNA
<213>  Nicotiana tabacum

<400>  13
ataggttttg cgactttagt gacacatctg acttttggtc gcttgcttca aggttttgat      60

tttagtaagc catcaaacac gccaattgac atgacagaag gcgtaggcgt tactttgcct     120

aaggttaatc aagttgaagt tctaattacc cctcgtttac cttctaagct ttatttattt     180

tga                                                                   183

<210>  14
<211>  60
<212>  PRT

<213>  Nicotiana tabacum

<400>  14

Ile Gly Phe Ala Thr Leu Val Thr His Leu Thr Phe Gly Arg Leu Leu
1               5                   10                  15

Gln Gly Phe Asp Phe Ser Lys Pro Ser Asn Thr Pro Ile Asp Met Thr
            20                  25                  30

Glu Gly Val Gly Val Thr Leu Pro Lys Val Asn Gln Val Glu Val Leu
        35                  40                  45

Ile Thr Pro Arg Leu Pro Ser Lys Leu Tyr Leu Phe
    50                  55                  60

<210>  15
<211>  178
<212>  DNA
<213>  Nicotiana tabacum

<400>  15
ataggctttg cgactttagt gacacatctg acttttggtc gcttgcttca aggttttgat      60

tttagtaagc catcaaacac gccaattgac atgacagaag gcgtaggcgt tactttgcct     120

aaggttaatc aagttgaagt tctaattacc cctcgtttac cttctaagct ttatttat       178

<210>  16
<211>  59
<212>  PRT
<213>  Nicotiana tabacum

<400>  16

Ile Gly Phe Ala Thr Leu Val Thr His Leu Thr Phe Gly Arg Leu Leu
1               5                   10                  15

Gln Gly Phe Asp Phe Ser Lys Pro Ser Asn Thr Pro Ile Asp Met Thr
            20                  25                  30

Glu Gly Val Gly Val Thr Leu Pro Lys Val Asn Gln Val Glu Val Leu
        35                  40                  45

Ile Thr Pro Arg Leu Pro Ser Lys Leu Tyr Leu
    50                  55

<210>  17
<211>  183
<212>  DNA
<213>  Nicotiana tabacum

<400>  17
ataaattttg cgactttagt gacacatctg acttttggtc gcttgcttca aggttttgat      60

tttagtacgc catcaaacac gccaatagac atgacagaag gcgtaggcgt tactttgcct     120

aaggtaaatc aagtggaagt tctaattagc cctcgtttac cttctaagct ttatgtattc     180

tga                                                                 183

<210>  18
<211>  60
<212>  PRT
<213>  Nicotiana tabacum

<400>  18

Ile Asn Phe Ala Thr Leu Val Thr His Leu Thr Phe Gly Arg Leu Leu
1               5                   10                  15

Gln Gly Phe Asp Phe Ser Thr Pro Ser Asn Thr Pro Ile Asp Met Thr
            20                  25                  30

Glu Gly Val Gly Val Thr Leu Pro Lys Val Asn Gln Val Glu Val Leu
        35                  40                  45

Ile Ser Pro Arg Leu Pro Ser Lys Leu Tyr Val Phe
    50                  55                  60

<210>  19
<211>  171
<212>  DNA
<213>  Nicotiana tabacum

<400>  19
attatacttg cattgccaat tcttggcatc actttgggac gtttggttca gaactttgag      60

ctgttgcctc ctccaggcca gtcgaagctc gacaccacag agaaaggtgg acagttcagt     120

ctccacattt tgaagcattc caccattgtg ttgaaaccaa ggtctttctg a              171

<210>  20
<211>  56
<212>  PRT
<213>  Nicotiana tabacum

<400>  20

Ile Ile Leu Ala Leu Pro Ile Leu Gly Ile Thr Leu Gly Arg Leu Val
1               5                   10                  15

Gln Asn Phe Glu Leu Leu Pro Pro Pro Gly Gln Ser Lys Leu Asp Thr
            20                  25                  30

Thr Glu Lys Gly Gly Gln Phe Ser Leu His Ile Leu Lys His Ser Thr
        35                  40                  45

Ile Val Leu Lys Pro Arg Ser Phe
    50                  55

<210>  21
<211>  171
<212>  DNA
<213>  Nicotiana tabacum

<400>  21

attatacttg cattgccaat tcttggcatt actttgggac gtttggttca gaactttgag     60

ctgttgcctc ctccaggcca gtcgaagctc gacaccacag agaaaggtgg acagttcagt    120

ctccatattt tgaagcattc caccattgtg ttgaaaccaa ggtcttgctg a             171

<210>  22
<211>  56
<212>  PRT
<213>  Nicotiana tabacum

<400>  22

Ile Ile Leu Ala Leu Pro Ile Leu Gly Ile Thr Leu Gly Arg Leu Val
1               5                   10                  15

Gln Asn Phe Glu Leu Leu Pro Pro Pro Gly Gln Ser Lys Leu Asp Thr
            20                  25                  30

Thr Glu Lys Gly Gly Gln Phe Ser Leu His Ile Leu Lys His Ser Thr
        35                  40                  45

Ile Val Leu Lys Pro Arg Ser Cys
    50                  55

<210>  23
<211>  172
<212>  DNA
<213>  Nicotiana tabacum

<400>  23
tattgcactt ggggttgcat caatggaact tgcattgtca aatcttcttt atgcatttga     60

ttgggagtta ccttttggaa tgaaaaaaga agacattgac acaaacgcca ggcctggaat    120

taccatgcat aagaaaaacg aactttatct tatccctaaa aattatctat ag            172

<210>  24
<211>  63
<212>  PRT
<213>  Nicotiana tabacum

<400>  24

Ile Ala Leu Gly Val Ala Ser Met Glu Leu Ala Leu Ser Asn Leu Leu
1               5                   10                  15

Tyr Ala Phe Asp Trp Glu Leu Pro Phe Gly Met Lys Lys Glu Asp Ile
            20                  25                  30

Asp Thr Asn Ala Arg Pro Gly Ile Thr Met His Lys Lys Asn Glu Leu
        35                  40                  45

Tyr Leu Ile Pro Lys Asn Tyr Leu Pro Ser Lys Leu Tyr Leu Phe
    50                  55                  60

<210>  25
<211>  171

<212>  DNA
<213>  Nicotiana tabacum

<400>  25
attgcacttg gggttgcatc catggaactt gctttgtcaa atcttcttta tgcatttgat      60

tgggagttac cttacggagt gaaaaaagaa aacattgaca caaatgtcag gcctggaatt     120

accatgcata agaaaaacga actttgcctt atccctagaa attatctata g             171


<210>  26
<211>  56
<212>  PRT
<213>  Nicotiana tabacum

<400>  26

Ile Ala Leu Gly Val Ala Ser Met Glu Leu Ala Leu Ser Asn Leu Leu
1               5                   10                  15


Tyr Ala Phe Asp Trp Glu Leu Pro Tyr Gly Val Lys Lys Glu Asn Ile
            20                  25                  30


Asp Thr Asn Val Arg Pro Gly Ile Thr Met His Lys Lys Asn Glu Leu
        35                  40                  45


Cys Leu Ile Pro Arg Asn Tyr Leu
    50                  55


<210>  27
<211>  174
<212>  DNA
<213>  Nicotiana tabacum

<400>  27
ggtattgcac ttggggttgc atccatggaa cttgctttgt caaatcttct ttatgcattt      60

gattgggagt tgccttatgg agtgaaaaaa gaagacatcg acacaaacgt taggcctgga     120

attgccatgc acaagaaaaa cgaactttgc cttgtcccaa aaaattattt ataa           174


<210>  28
<211>  56
<212>  PRT
<213>  Nicotiana tabacum

<400>  28

Ile Ala Leu Gly Val Ala Ser Met Glu Leu Ala Leu Ser Asn Leu Leu
1               5                   10                  15


Tyr Ala Phe Asp Trp Glu Leu Pro Tyr Gly Val Lys Lys Glu Asp Ile
            20                  25                  30


Asp Thr Asn Val Arg Pro Gly Ile Ala Met His Lys Lys Asn Glu Leu
        35                  40                  45


Cys Leu Val Pro Lys Asn Tyr Leu
    50                  55

<210> 29
<211> 201
<212> DNA
<213> Nicotiana tabacum

<400> 29
attgcacttg gggttgcatc catggaactt gctttgtcaa atcttcttta tgcatttgat 60

tgggagttgc cttatggagt gaaaaaagaa gacatcgaca caaacgttag gcctggaatt 120

gccatgcaca agaaaaacga actttgcctt gtcccaaaaa aattatttat aaattatatt 180

gggacgtgga tctcatgcta g 201


<210> 30
<211> 66
<212> PRT
<213> Nicotiana tabacum

<400> 30

Ile Ala Leu Gly Val Ala Ser Met Glu Leu Ala Leu Ser Asn Leu Leu
1               5                   10                  15


Tyr Ala Phe Asp Trp Glu Leu Pro Tyr Gly Val Lys Lys Glu Asp Ile
            20                  25                  30


Asp Thr Asn Val Arg Pro Gly Ile Ala Met His Lys Lys Asn Glu Leu
        35                  40                  45


Cys Leu Val Pro Lys Lys Leu Phe Ile Asn Tyr Ile Gly Thr Trp Ile
    50                  55                  60


Ser Cys
65


<210> 31
<211> 183
<212> DNA
<213> Nicotiana tabacum

<400> 31
atttcgtttg gtttagctaa tgcttatttg ccattggctc aattacttta tcactttgat 60

tgggaactcc ccactggaat caaaccaagc gacttggact tgactgagtt ggttggagta 120

actgccgcta gaaaaagtga cctttacttg gttgcgactc cttatcaacc tcctcaaaac 180

tga 183


<210> 32
<211> 60
<212> PRT
<213> Nicotiana tabacum

<400> 32

Ile Ser Phe Gly Leu Ala Asn Ala Tyr Leu Pro Leu Ala Gln Leu Leu
1               5                   10                  15

Tyr His Phe Asp Trp Glu Leu Pro Thr Gly Ile Lys Pro Ser Asp Leu
            20                  25                  30

Asp Leu Thr Glu Leu Val Gly Val Thr Ala Ala Arg Lys Ser Asp Leu
        35                  40                  45

Tyr Leu Val Ala Thr Pro Tyr Gln Pro Pro Gln Asn
    50                  55                  60

<210>  33
<211>  183
<212>  DNA
<213>  Nicotiana tabacum

<400>  33
atttcgtttg gcttagctaa tgcttatttg ccattggctc aattactata tcacttcgat      60

tggaaactcc ctgctggaat cgaaccaagc gacttggact tgactgagtt ggttggagta     120

actgccgcta gaaaaagtga cctttacttg gttgcgactc cttatcaacc tcctcaaaag     180

tga                                                                   183

<210>  34
<211>  60
<212>  PRT
<213>  Nicotiana tabacum

<400>  34

Ile Ser Phe Gly Leu Ala Asn Ala Tyr Leu Pro Leu Ala Gln Leu Leu
1               5                   10                  15

Tyr His Phe Asp Trp Lys Leu Pro Ala Gly Ile Glu Pro Ser Asp Leu
            20                  25                  30

Asp Leu Thr Glu Leu Val Gly Val Thr Ala Ala Arg Lys Ser Asp Leu
        35                  40                  45

Tyr Leu Val Ala Thr Pro Tyr Gln Pro Pro Gln Lys
    50                  55                  60

<210>  35
<211>  220
<212>  DNA
<213>  Nicotiana tabacum

<400>  35
atgctatttg gtttagctaa tgttggacaa cctttagctc agttacttta tcacttcgat      60

tggaaactcc ctaatggaca aagtcatgag aatttcgaca tgactgagtc acctggaatt     120

tctgctacaa gaaaggatga tcttgttttg attgccactc cttatgattc ttattaattc     180

cagtctatat catctatatg tactcaataa ttgtatggga                           220

<210>  36

<211>  58
<212>  PRT
<213>  Nicotiana tabacum

<400>  36

Met Leu Phe Gly Leu Ala Asn Val Gly Gln Pro Leu Ala Gln Leu Leu
1               5                   10                  15

Tyr His Phe Asp Trp Lys Leu Pro Asn Gly Gln Ser His Glu Asn Phe
            20                  25                  30

Asp Met Thr Glu Ser Pro Gly Ile Ser Ala Thr Arg Lys Asp Asp Leu
        35                  40                  45

Val Leu Ile Ala Thr Pro Tyr Asp Ser Tyr
    50                  55

<210>  37
<211>  174
<212>  DNA
<213>  Nicotiana tabacum

<400>  37
atgctatttg gtttagctaa tgttggacaa cctttagctc agttacttta tcacttcgat      60

tggaaactcc ctaatggaca aactcaccaa aatttcgaca tgactgagtc acctggaatt     120

tctgctacaa gaaaggatga tcttattttg attgccactc ctgctcattc ttga           174

<210>  38
<211>  57
<212>  PRT
<213>  Nicotiana tabacum

<400>  38

Met Leu Phe Gly Leu Ala Asn Val Gly Gln Pro Leu Ala Gln Leu Leu
1               5                   10                  15

Tyr His Phe Asp Trp Lys Leu Pro Asn Gly Gln Thr His Gln Asn Phe
            20                  25                  30

Asp Met Thr Glu Ser Pro Gly Ile Ser Ala Thr Arg Lys Asp Asp Leu
        35                  40                  45

Ile Leu Ile Ala Thr Pro Ala His Ser
    50                  55

<210>  39
<211>  177
<212>  DNA
<213>  Nicotiana tabacum

<400>  39
ttattattcg gtttagttaa tgtaggacat cctttagctc aattgcttta tcacttcgat      60

tggaagactc ttcctgggat aagttcagat agtttcgaca tgactgaaac agatggagta     120

actgccggaa gaaaggatga tctttgttta attgctactc cttttggtct caattaa          177

<210>  40
<211>  58
<212>  PRT
<213>  Nicotiana tabacum

<400>  40

Leu Leu Phe Gly Leu Val Asn Val Gly His Pro Leu Ala Gln Leu Leu
1               5                   10                  15

Tyr His Phe Asp Trp Lys Thr Leu Pro Gly Ile Ser Ser Asp Ser Phe
            20                  25                  30

Asp Met Thr Glu Thr Asp Gly Val Thr Ala Gly Arg Lys Asp Asp Leu
        35                  40                  45

Cys Leu Ile Ala Thr Pro Phe Gly Leu Asn
    50                  55

<210>  41
<211>  180
<212>  DNA
<213>  Nicotiana tabacum

<400>  41
atgtcgtttg gtttagttaa cactgggcat cctttagctc agttgctcta tttctttgac      60

tggaaattcc ctcataaggt taatgcagct gattttcaca ctactgaaac aagtagagtt     120

tttgcagcaa gcaaagatga cctctacttg attccaacaa atcacatgga gcaagagtag     180

<210>  42
<211>  59
<212>  PRT
<213>  Nicotiana tabacum

<400>  42

Met Ser Phe Gly Leu Val Asn Thr Gly His Pro Leu Ala Gln Leu Leu
1               5                   10                  15

Tyr Phe Phe Asp Trp Lys Phe Pro His Lys Val Asn Ala Ala Asp Phe
            20                  25                  30

His Thr Thr Glu Thr Ser Arg Val Phe Ala Ala Ser Lys Asp Asp Leu
        35                  40                  45

Tyr Leu Ile Pro Thr Asn His Met Glu Gln Glu
    50                  55

<210>  43
<211>  180
<212>  DNA
<213>  Nicotiana tabacum

<400>  43

atgtcgtttg gtttagttaa cacagggcat cctttagccc agttgctcta ttgctttgac 60

tggaaactcc ctgacaaggt taatgcaaat gattttcgca ctactgaaac aagtagagtt 120

tttgcagcaa gcaaagatga cctctacttg attcccacaa atcacaggga gcaagaatag 180

<210> 44
<211> 59
<212> PRT
<213> Nicotiana tabacum

<400> 44

Met Ser Phe Gly Leu Val Asn Thr Gly His Pro Leu Ala Gln Leu Leu
1               5                   10                  15

Tyr Cys Phe Asp Trp Lys Leu Pro Asp Lys Val Asn Ala Asn Asp Phe
            20                  25                  30

Arg Thr Thr Glu Thr Ser Arg Val Phe Ala Ala Ser Lys Asp Asp Leu
        35                  40                  45

Tyr Leu Ile Pro Thr Asn His Arg Glu Gln Glu
    50                  55

<210> 45
<211> 183
<212> DNA
<213> Nicotiana tabacum

<400> 45
atgcaatttg gtttggctct tgttactctg ccattggctc atttgcttca caattttgat 60

tggaaacttc ccgaaggaat taatgcaagg gatttggaca tgacagaggc aaatgggata 120

tctgctagaa gagaaaaaga tctttacttg attgctactc cttatgtatc acctcttgat 180

taa 183

<210> 46
<211> 60
<212> PRT
<213> Nicotiana tabacum

<400> 46

Met Gln Phe Gly Leu Ala Leu Val Thr Leu Pro Leu Ala His Leu Leu
1               5                   10                  15

His Asn Phe Asp Trp Lys Leu Pro Glu Gly Ile Asn Ala Arg Asp Leu
            20                  25                  30

Asp Met Thr Glu Ala Asn Gly Ile Ser Ala Arg Arg Glu Lys Asp Leu
        35                  40                  45

Tyr Leu Ile Ala Thr Pro Tyr Val Ser Pro Leu Asp
    50                  55                  60

<210> 47
<211> 177
<212> DNA
<213> Nicotiana tabacum

<400> 47
atgacttatg cattgcaagt ggaacaccta acaatggcac atttgatcca gggtttcaat 60

tacagaactc caactgatga gcccttggat atgaaagaag gtgcaggcat aactatacgt 120

aaggtaaatc ctgtgaaagt gataattacg cctcgcttgg cacctgagct ttattaa 177


<210> 48
<211> 58
<212> PRT
<213> Nicotiana tabacum

<400> 48

Met Thr Tyr Ala Leu Gln Val Glu His Leu Thr Met Ala His Leu Ile
1               5                   10                  15


Gln Gly Phe Asn Tyr Arg Thr Pro Thr Asp Glu Pro Leu Asp Met Lys
            20                  25                  30


Glu Gly Ala Gly Ile Thr Ile Arg Lys Val Asn Pro Val Lys Val Ile
        35                  40                  45


Ile Thr Pro Arg Leu Ala Pro Glu Leu Tyr
    50                  55


<210> 49
<211> 177
<212> DNA
<213> Nicotiana tabacum

<400> 49
atgacttatg cattgcaagt ggaacaccta acaatggcac atttaatcca gggtttcaat 60

tacaaaactc caaatgacga ggccttggat atgaaggaag gtgcaggcat aactatacgt 120

aaggtaaatc ctgtggaact gataatagcg cctcgcctgg cacctgagct ttattaa 177


<210> 50
<211> 58
<212> PRT
<213> Nicotiana tabacum

<400> 50

Met Thr Tyr Ala Leu Gln Val Glu His Leu Thr Met Ala His Leu Ile
1               5                   10                  15


Gln Gly Phe Asn Tyr Lys Thr Pro Asn Asp Glu Ala Leu Asp Met Lys
            20                  25                  30


Glu Gly Ala Gly Ile Thr Ile Arg Lys Val Asn Pro Val Glu Leu Ile
        35                  40                  45

Ile Ala Pro Arg Leu Ala Pro Glu Leu Tyr
    50                  55

<210>  51
<211>  177
<212>  DNA
<213>  Nicotiana tabacum

<400>  51
atgacttatg cattgcaagt ggaacaccta acaatggcac atttaatcca gggtttcaat      60

tacaaaactc caaatgacga ggccttggat atgaaggaag gtgcaggcat aacaatacgt     120

aaggtaaatc cagtggaatt gataataacg cctcgcttgg cacctgagct ttactaa        177

<210>  52
<211>  58
<212>  PRT
<213>  Nicotiana tabacum

<400>  52

Met Thr Tyr Ala Leu Gln Val Glu His Leu Thr Met Ala His Leu Ile
1               5                   10                  15

Gln Gly Phe Asn Tyr Lys Thr Pro Asn Asp Glu Ala Leu Asp Met Lys
            20                  25                  30

Glu Gly Ala Gly Ile Thr Ile Arg Lys Val Asn Pro Val Glu Leu Ile
        35                  40                  45

Ile Thr Pro Arg Leu Ala Pro Glu Leu Tyr
    50                  55

<210>  53
<211>  177
<212>  DNA
<213>  Nicotiana tabacum

<400>  53
atgacttatg cattgcaagt ggaacactta acaatggcac atttgatcca aggtttcaat      60

tacagaactc caaatgacga gcccttggat atgaaggaag gtgcaggcat aactatacgt     120

aaggtaaatc ctgtggaact gataatagcg cctcgcctgg cacctgagct ttattaa        177

<210>  54
<211>  58
<212>  PRT
<213>  Nicotiana tabacum

<400>  54

Met Thr Tyr Ala Leu Gln Val Glu His Leu Thr Met Ala His Leu Ile
1               5                   10                  15

Gln Gly Phe Asn Tyr Arg Thr Pro Asn Asp Glu Pro Leu Asp Met Lys
            20                  25                  30

Glu Gly Ala Gly Ile Thr Ile Arg Lys Val Asn Pro Val Glu Leu Ile
        35                  40                  45

Ile Ala Pro Arg Leu Ala Pro Glu Leu Tyr
    50                  55

<210>  55
<211>  174
<212>  DNA
<213>  Nicotiana tabacum

<400>  55
atgacttatg cattgcaagt ggaacactta acaatggcac atttgatcca aggtttcaat      60

tacagaactc caaatgacga gcccttggat atgaaggaag gtgcaggcat aactatacgt     120

aaggtaaatc ctgtggaact gataatagcg cccctggcac ctgagcttta ttaa           174

<210>  56
<211>  57
<212>  PRT
<213>  Nicotiana tabacum

<400>  56

Met Thr Tyr Ala Leu Gln Val Glu His Leu Thr Met Ala His Leu Ile
1               5                   10                  15

Gln Gly Phe Asn Tyr Arg Thr Pro Asn Asp Glu Pro Leu Asp Met Lys
            20                  25                  30

Glu Gly Ala Gly Ile Thr Ile Arg Lys Val Asn Pro Val Glu Leu Ile
        35                  40                  45

Ile Ala Pro Leu Ala Pro Glu Leu Tyr
    50                  55

<210>  57
<211>  177
<212>  DNA
<213>  Nicotiana tabacum

<400>  57
atgacttatg cattgcaagt ggaacactta acaatggcac atttgatcca aggtttcaat      60

tacagaactc caaatgacga gcccttggat atgaaggaag gtgcaggcat aactatacgt     120

aaggtaaatc ctgcggaact gataatagcg cctcgcctgg cacctgagct ttattaa        177

<210>  58
<211>  58
<212>  PRT
<213>  Nicotiana tabacum

<400>  58

Met Thr Tyr Ala Leu Gln Val Glu His Leu Thr Met Ala His Leu Ile
1               5                   10                  15

Gln Gly Phe Asn Tyr Arg Thr Pro Asn Asp Glu Pro Leu Asp Met Lys
            20                  25                  30

Glu Gly Ala Gly Ile Thr Ile Arg Lys Val Asn Pro Ala Glu Leu Ile
        35                  40                  45

Ile Ala Pro Arg Leu Ala Pro Glu Leu Tyr
    50                  55

<210>  59
<211>  177
<212>  DNA
<213>  Nicotiana tabacum

<400>  59
atgacttatg cattacaagt ggaacaccta acaatagcac atttgatcca gggtttcaat      60

tacaaaactc caaatgacga gcccttggat atgaaggaag gtgcaggatt aaccatacgt     120

aaagtaaatc ctgtagaagt gacaactacg gctcgcctgg cacctgagct ttattaa        177

<210>  60
<211>  58
<212>  PRT
<213>  Nicotiana tabacum

<400>  60

Met Thr Tyr Ala Leu Gln Val Glu His Leu Thr Ile Ala His Leu Ile
1               5                   10                  15

Gln Gly Phe Asn Tyr Lys Thr Pro Asn Asp Glu Pro Leu Asp Met Lys
            20                  25                  30

Glu Gly Ala Gly Leu Thr Ile Arg Lys Val Asn Pro Val Glu Val Thr
        35                  40                  45

Thr Thr Ala Arg Leu Ala Pro Glu Leu Tyr
    50                  55

<210>  61
<211>  177
<212>  DNA
<213>  Nicotiana tabacum

<400>  61
atgacttatg cattacaagt ggaacaccta acaatagcac atttgatcca gggtttcaat      60

tacaaaactc caaatgacga gcccttggat atgaaggaag gtgcaggatt aactatacgt     120

aaagtaaatc ctgtagaagt gacaattacg gctcgcctgg cacctgagct ttattaa        177

<210>  62
<211>  58
<212>  PRT
<213>  Nicotiana tabacum

<400>  62

Met Thr Tyr Ala Leu Gln Val Glu His Leu Thr Ile Ala His Leu Ile
1               5                   10                  15

Gln Gly Phe Asn Tyr Lys Thr Pro Asn Asp Glu Pro Leu Asp Met Lys
            20                  25                  30

Glu Gly Ala Gly Leu Thr Ile Arg Lys Val Asn Pro Val Glu Val Thr
        35                  40                  45

Ile Thr Ala Arg Leu Ala Pro Glu Leu Tyr
    50                  55

<210>  63
<211>  177
<212>  DNA
<213>  Nicotiana tabacum

<400>  63
atgacttatg cattacaagt ggaacaccta acaatagcac atttgatcca gggtttcaat      60

tacaaaactc caaatgacga gcccctggat atgaaggaag gtgcaggatt aactatacgt     120

aaagtaaatc ctgtagaagt gacaattacg gctcgcctgg cacctgagct ttattaa        177

<210>  64
<211>  58
<212>  PRT
<213>  Nicotiana tabacum

<400>  64

Met Thr Tyr Ala Leu Gln Val Glu His Leu Thr Ile Ala His Leu Ile
1               5                   10                  15

Gln Gly Phe Asn Tyr Lys Thr Pro Asn Asp Glu Pro Leu Asp Met Lys
            20                  25                  30

Glu Gly Ala Gly Leu Thr Ile Arg Lys Val Asn Pro Val Glu Val Thr
        35                  40                  45

Ile Thr Ala Arg Leu Ala Pro Glu Leu Tyr
    50                  55

<210>  65
<211>  198
<212>  DNA
<213>  Nicotiana tabacum

<400>  65
tatagccttg gacttaaggt tatccgagta acattagcca acatgttgca tggattcaac      60

tggaaattac ctgaaggtat gaagccagaa gatataagtg tggaagaaca ttatgggctc     120

actacacatc ctaagtttcc tgttcctgtg atcttggaat ctagactttc ttcagatctc     180

tattccccca tcacttaa                                                   198

<210>  66

<211>  65
<212>  PRT
<213>  Nicotiana tabacum

<400>  66

Tyr Ser Leu Gly Leu Lys Val Ile Arg Val Thr Leu Ala Asn Met Leu
1               5                   10                  15

His Gly Phe Asn Trp Lys Leu Pro Glu Gly Met Lys Pro Glu Asp Ile
            20                  25                  30

Ser Val Glu Glu His Tyr Gly Leu Thr Thr His Pro Lys Phe Pro Val
        35                  40                  45

Pro Val Ile Leu Glu Ser Arg Leu Ser Ser Asp Leu Tyr Ser Pro Ile
    50                  55                  60

Thr
65

<210>  67
<211>  190
<212>  DNA
<213>  Nicotiana tabacum

<400>  67
atacagtctt gggattcgta taattagggc aactttagct aacttgttgc atggattcaa      60

ctggagattg cctaatggta tgagtccaga agacattagc atggaagaga tttatgggct     120

aattacacac cccaaagtcg cacttgacgt gatgatggag cctcgacttc ccaaccatct     180

ttacaaatag                                                            190

<210>  68
<211>  62
<212>  PRT
<213>  Nicotiana tabacum

<400>  68

Tyr Ser Leu Gly Ile Arg Ile Ile Arg Ala Thr Leu Ala Asn Leu Leu
1               5                   10                  15

His Gly Phe Asn Trp Arg Leu Pro Asn Gly Met Ser Pro Glu Asp Ile
            20                  25                  30

Ser Met Glu Glu Ile Tyr Gly Leu Ile Thr His Pro Lys Val Ala Leu
        35                  40                  45

Asp Val Met Met Glu Pro Arg Leu Pro Asn His Leu Tyr Lys
    50                  55                  60

<210>  69
<211>  174
<212>  DNA
<213>  Nicotiana tabacum

<400> 69
attaattttt caataccact tgttgagctt gcacttgcta atctattgtt tcattataat 60

tggtcacttc ctgaagggat gctagctaag gatgttgata tggaagaagc tttggggatt 120

accatgcaca agaaatctcc cctttgctta gtagcttctc attatacttg ttga 174

<210> 70
<211> 57
<212> PRT
<213> Nicotiana tabacum

<400> 70

Ile Asn Phe Ser Ile Pro Leu Val Glu Leu Ala Leu Ala Asn Leu Leu
1               5                   10                  15

Phe His Tyr Asn Trp Ser Leu Pro Glu Gly Met Leu Ala Lys Asp Val
            20                  25                  30

Asp Met Glu Glu Ala Leu Gly Ile Thr Met His Lys Lys Ser Pro Leu
        35                  40                  45

Cys Leu Val Ala Ser His Tyr Thr Cys
    50                  55

<210> 71
<211> 190
<212> DNA
<213> Nicotiana tabacum

<400> 71
atgcaacttg ggctttatgc attggaaatg gctgtggccc atcttcttca ttgttttact 60

tgggaattgc cagatggtat gaaaccaagt gagcttaaaa tggatgatat ttttggactc 120

actgctccaa aagctaatcg actcgtggct gtgcctactc cacgtttgtt gtgtcccctt 180

tattaattga 190

<210> 72
<211> 61
<212> PRT
<213> Nicotiana tabacum

<400> 72

Met Gln Leu Gly Leu Tyr Ala Leu Glu Met Ala Val Ala His Leu Leu
1               5                   10                  15

His Cys Phe Thr Trp Glu Leu Pro Asp Gly Met Lys Pro Ser Glu Leu
            20                  25                  30

Lys Met Asp Asp Ile Phe Gly Leu Thr Ala Pro Lys Ala Asn Arg Leu
        35                  40                  45

Val Ala Val Pro Thr Pro Arg Leu Leu Cys Pro Leu Tyr
    50                  55                  60

<210> 73
<211> 186
<212> DNA
<213> Nicotiana tabacum

<400> 73
atgcaacttg ggctttatgc attagaaatg gcagtggccc atcttcttct ttgctttact 60

tgggaattgc cagatggtat gaaaccaagt gagcttaaaa tggatgatat ttttggactc 120

actgctccaa gagctaatcg actcgtggct gtgcctagtc cacgtttgtt gtgcccactt 180

tattaa 186

<210> 74
<211> 61
<212> PRT
<213> Nicotiana tabacum

<400> 74

Met Gln Leu Gly Leu Tyr Ala Leu Glu Met Ala Val Ala His Leu Leu
1               5                   10                  15

Leu Cys Phe Thr Trp Glu Leu Pro Asp Gly Met Lys Pro Ser Glu Leu
            20                  25                  30

Lys Met Asp Asp Ile Phe Gly Leu Thr Ala Pro Arg Ala Asn Arg Leu
        35                  40                  45

Val Ala Val Pro Ser Pro Arg Leu Leu Cys Pro Leu Tyr
    50                  55                  60

<210> 75
<211> 186
<212> DNA
<213> Nicotiana tabacum

<400> 75
atgcaacttg ggctttatgc attggaaatg gctgtggccc atcttcttca ttgttttact 60

tgggaattgc cagatggtat gaaaccaagt gagcttaaaa tggatgatat ttttggactc 120

actgctccaa gagctaatcg actcgtggct gtgcctactc cacgtttgtt gtgtcccctt 180

tattaa 186

<210> 76
<211> 61
<212> PRT
<213> Nicotiana tabacum

<400> 76

Met Gln Leu Gly Leu Tyr Ala Leu Glu Met Ala Val Ala His Leu Leu
1               5                   10                  15

His Cys Phe Thr Trp Glu Leu Pro Asp Gly Met Lys Pro Ser Glu Leu
            20                  25                  30

Lys Met Asp Asp Ile Phe Gly Leu Thr Ala Pro Arg Ala Asn Arg Leu
        35                  40                  45

Val Ala Val Pro Thr Pro Arg Leu Leu Cys Pro Leu Tyr
    50                  55                  60

<210>  77
<211>  93
<212>  DNA
<213>  Nicotiana tabacum

<400>  77
atgctttgga gtgcgagtat agtgcgcgtc agctacctaa cttgtattta tagattccaa      60

gtatatgctg ggtctgtgtt cagagtagca tga                                   93

<210>  78
<211>  30
<212>  PRT
<213>  Nicotiana tabacum

<400>  78

Met Leu Trp Ser Ala Ser Ile Val Arg Val Ser Tyr Leu Thr Cys Ile
1               5                   10                  15

Tyr Arg Phe Gln Val Tyr Ala Gly Ser Val Phe Arg Val Ala
            20                  25                  30

<210>  79
<211>  93
<212>  DNA
<213>  Nicotiana tabacum

<400>  79
atgctttgga gtgcgagtat agtgcgcgtc agctacctaa cttgtattta tagattccaa      60

gtatatgctg ggtctgtgtt cagagtagca tga                                   93

<210>  80
<211>  30
<212>  PRT
<213>  Nicotiana tabacum

<400>  80

Met Leu Trp Ser Ala Ser Ile Val Arg Val Ser Tyr Leu Thr Cys Ile
1               5                   10                  15

Tyr Arg Phe Gln Val Tyr Ala Gly Ser Val Phe Arg Val Ala
            20                  25                  30

<210>  81
<211>  93
<212>  DNA
<213>  Nicotiana tabacum

<400>  81

atgctttgga gtgcgagtat agtgcgcgtc agctacctaa cctgtattta tagattccaa 60

gtatatgctg ggtctgtgtt cagagtagca tga 93

<210> 82
<211> 30
<212> PRT
<213> Nicotiana tabacum

<400> 82

Met Leu Trp Ser Ala Ser Ile Val Arg Val Ser Tyr Leu Thr Cys Ile
1               5                   10                  15

Tyr Arg Phe Gln Val Tyr Ala Gly Ser Val Phe Arg Val Ala
            20                  25                  30

<210> 83
<211> 177
<212> DNA
<213> Nicotiana tabacum

<400> 83
attaattttt caataccact tgttgagctt gcacttgcta atctattgtt tcattataat 60

tggtcacttc ctgaggggat gctacctaag gatgttgata tggaagaagc tttggggatt 120

accatgcaca agaaatctcc cctttgctta gtagcttctc attataactt gttgtga 177

<210> 84
<211> 58
<212> PRT
<213> Nicotiana tabacum

<400> 84

Ile Asn Phe Ser Ile Pro Leu Val Glu Leu Ala Leu Ala Asn Leu Leu
1               5                   10                  15

Phe His Tyr Asn Trp Ser Leu Pro Glu Gly Met Leu Pro Lys Asp Val
            20                  25                  30

Asp Met Glu Glu Ala Leu Gly Ile Thr Met His Lys Lys Ser Pro Leu
        35                  40                  45

Cys Leu Val Ala Ser His Tyr Asn Leu Leu
    50                  55

<210> 85
<211> 183
<212> DNA
<213> Nicotiana tabacum

<400> 85
atatcatttg gtttggctaa tgtttatttg ccactagctc aattgttata tcattttgat 60

tggaaactcc ctactggaat caattcaagt gacttggaca tgactgagtc gtcaggagta 120

acttgtgcta gaaagagtga tttatacttg actgctactc catatcaact ttctcaagag 180

tga                                                                183

<210>  86
<211>  61
<212>  PRT
<213>  Nicotiana tabacum

<400>  86

Gly Ile Ser Phe Gly Leu Ala Asn Val Tyr Leu Pro Leu Ala Gln Leu
1               5                   10                  15

Leu Tyr His Phe Asp Trp Lys Leu Pro Thr Gly Ile Asn Ser Ser Asp
            20                  25                  30

Leu Asp Met Thr Glu Ser Ser Gly Val Thr Cys Ala Arg Lys Ser Asp
        35                  40                  45

Leu Tyr Leu Thr Ala Thr Pro Tyr Gln Leu Ser Gln Glu
    50                  55                  60

<210>  87
<211>  93
<212>  DNA
<213>  Nicotiana tabacum

<400>  87
atgctttgga gtgcgagtat agtgcgcgtc agctacctaa cttgtattta tagattccaa      60

gtatatgctg ggtctgtgtc cagagtagca tga                                   93

<210>  88
<211>  30
<212>  PRT
<213>  Nicotiana tabacum

<400>  88

Met Leu Trp Ser Ala Ser Ile Val Arg Val Ser Tyr Leu Thr Cys Ile
1               5                   10                  15

Tyr Arg Phe Gln Val Tyr Ala Gly Ser Val Ser Arg Val Ala
            20                  25                  30

<210>  89
<211>  153
<212>  DNA
<213>  Nicotiana tabacum

<400>  89
ctgaattttg caatgttaga ggcaaaaatg gcacttgcat tgattctaca acactatgct      60

tttgagctct ctccatctta tgcacatgct cctcatacaa ttatcactct gcaacctcaa     120

catggtgctc ctttgatttt gcgcaagctg tag                                  153

<210>  90
<211>  50
<212>  PRT

<213>  Nicotiana tabacum

<400>  90

Leu Asn Phe Ala Met Leu Glu Ala Lys Met Ala Leu Ala Leu Ile Leu
1               5                   10                  15

Gln His Tyr Ala Phe Glu Leu Ser Pro Ser Tyr Ala His Ala Pro His
            20                  25                  30

Thr Ile Ile Thr Leu Gln Pro Gln His Gly Ala Pro Leu Ile Leu Arg
        35                  40                  45

Lys Leu
    50

<210>  91
<211>  153
<212>  DNA
<213>  Nicotiana tabacum

<400>  91
ctgaattttg caatgttaga ggcaaaaatg gcacttgcat tgattctaca acactatgct      60

tttgagctct ctccatctta tgcacacgct cctcatacaa ttatcactct gcaacctcaa     120

catggtgctc ctttgatttt gcgcaagctg tag                                  153

<210>  92
<211>  50
<212>  PRT
<213>  Nicotiana tabacum

<400>  92

Leu Asn Phe Ala Met Leu Glu Ala Lys Met Ala Leu Ala Leu Ile Leu
1               5                   10                  15

Gln His Tyr Ala Phe Glu Leu Ser Pro Ser Tyr Ala His Ala Pro His
            20                  25                  30

Thr Ile Ile Thr Leu Gln Pro Gln His Gly Ala Pro Leu Ile Leu Arg
        35                  40                  45

Lys Leu
    50

<210>  93
<211>  226
<212>  DNA
<213>  Nicotiana tabacum

<400>  93
aataattttg caatgttgga aactaagatt gccttagcaa tgatcctaca gcgttttgct      60

ttcgagcttt ctccatctta cgctcatgca cctacttatg tcgtcactct tcgacctcag     120

tgtggtgctc acttaatctt gcaaaaatta taggtcctta atctggattt cccattattg     180

agtagtgcct aataaatctt ctctatcact atttttccat ctttca 226

<210> 94
<211> 50
<212> PRT
<213> Nicotiana tabacum

<400> 94

Asn Asn Phe Ala Met Leu Glu Thr Lys Ile Ala Leu Ala Met Ile Leu
1               5                   10                  15

Gln Arg Phe Ala Phe Glu Leu Ser Pro Ser Tyr Ala His Ala Pro Thr
            20                  25                  30

Tyr Val Val Thr Leu Arg Pro Gln Cys Gly Ala His Leu Ile Leu Gln
        35                  40                  45

Lys Leu
    50

<210> 95
<211> 268
<212> DNA
<213> Nicotiana tabacum

<400> 95
agcgaagggg tggcaaaggc aacaaagggg aaaatgacat attttccatt tggtgcagga 60

ccgcgaaaat gcattgggca aaacttcgcg attttggaag caaaaatggc tatagctatg 120

attctacaac gcttctcctt cgagctctcc ccatcttata cacactctcc atacactgtg 180

gtcactttga aacccaaata tggtgctccc ctaataatgc acaggctgta gtcctgtgag 240

aatatgctat ccgaggaatt cagttcct 268

<210> 96
<211> 50
<212> PRT
<213> Nicotiana tabacum

<400> 96

Gln Asn Phe Ala Ile Leu Glu Ala Lys Met Ala Ile Ala Met Ile Leu
1               5                   10                  15

Gln Arg Phe Ser Phe Glu Leu Ser Pro Ser Tyr Thr His Ser Pro Tyr
            20                  25                  30

Thr Val Val Thr Leu Lys Pro Lys Tyr Gly Ala Pro Leu Ile Met His
        35                  40                  45

Arg Leu
    50

<210> 97
<211> 237

<212>  DNA
<213>  Nicotiana tabacum

<400>  97
agcgaagggg tggcaaaggc aacaaagggg aaaatgacat attttccatt tggtgcagga      60

ccgcgaaaat gcattgggca aaacttcgcg attttggaag caaaaatggc tatagctatg     120

attctacaac gcttctcctt cgagctctct ccatcttata cacactctcc atacactgtg     180

gtcactttga aacccaaata tggtgctccc ctaataatgc acaggctgta gtcctgt        237


<210>  98
<211>  50
<212>  PRT
<213>  Nicotiana tabacum

<400>  98

Gln Asn Phe Ala Ile Leu Glu Ala Lys Met Ala Ile Ala Met Ile Leu
1               5                   10                  15


Gln Arg Phe Ser Phe Glu Leu Ser Pro Ser Tyr Thr His Ser Pro Tyr
            20                  25                  30


Thr Val Val Thr Leu Lys Pro Lys Tyr Gly Ala Pro Leu Ile Met His
        35                  40                  45


Arg Leu
    50


<210>  99
<211>  152
<212>  DNA
<213>  Nicotiana tabacum

<400>  99
caaaattttg ccatgttaga agcaaagatg gctctgtcta tgatcctgca acgcttctct      60

tttgaactgt ctccgtctta tgcacatgcc cctcagtcca tattaaccgt cagccacaat     120

atggtgctcc acttattttc cacaagctat aa                                   152


<210>  100
<211>  50
<212>  PRT
<213>  Nicotiana tabacum

<400>  100

Gln Asn Phe Ala Met Leu Glu Ala Lys Met Ala Leu Ser Met Ile Leu
1               5                   10                  15


Gln Arg Phe Ser Phe Glu Leu Ser Pro Ser Tyr Ala His Ala Pro Gln
            20                  25                  30


Ser Ile Leu Thr Val Gln Pro Gln Tyr Gly Ala Pro Leu Ile Phe His
        35                  40                  45

Lys Leu
    50

<210>  101
<211>  153
<212>  DNA
<213>  Nicotiana tabacum

<400>  101
ataaactttg caatgacaga agcgaagatg gctatggcta tgattctgca acgcttctcc      60

tttgagctat ctccatctta cacacatgct ccacagtctg taataactat gcaaccccaa     120

tatggtgctc ctcttatatt gcacaaattg taa                                  153

<210>  102
<211>  50
<212>  PRT
<213>  Nicotiana tabacum

<400>  102

Ile Asn Phe Ala Met Thr Glu Ala Lys Met Ala Met Ala Met Ile Leu
1               5                   10                  15

Gln Arg Phe Ser Phe Glu Leu Ser Pro Ser Tyr Thr His Ala Pro Gln
            20                  25                  30

Ser Val Ile Thr Met Gln Pro Gln Tyr Gly Ala Pro Leu Ile Leu His
        35                  40                  45

Lys Leu
    50

<210>  103
<211>  153
<212>  DNA
<213>  Nicotiana tabacum

<400>  103
ataaactttg caatggcaga agcgaagatg gctatggcta tgattctgca acgcttctcc      60

tttgagctat ctccatctta cacacatgct ccacagtctg taataactat gcaaccccaa     120

tatggtgctc ctcttatatt gcacaaattg taa                                  153

<210>  104
<211>  50
<212>  PRT
<213>  Nicotiana tabacum

<400>  104

Ile Asn Phe Ala Met Ala Glu Ala Lys Met Ala Met Ala Met Ile Leu
1               5                   10                  15

Gln Arg Phe Ser Phe Glu Leu Ser Pro Ser Tyr Thr His Ala Pro Gln
            20                  25                  30

Ser Val Ile Thr Met Gln Pro Gln Tyr Gly Ala Pro Leu Ile Leu His
        35                  40                  45

Lys Leu
    50

<210>  105
<211>  153
<212>  DNA
<213>  Nicotiana tabacum

<400>  105
caaaactttg caatgatgga agcaaaaatg gcagtagcta tgatactaca aaaattttcc      60

tttgaactat ccccttctta tacacatgct ccatttgcaa ttgtgactat tcatcctcag     120

tatggtgctc ctctgcttat gcgcagactt taa                                  153

<210>  106
<211>  50
<212>  PRT
<213>  Nicotiana tabacum

<400>  106

Gln Asn Phe Ala Met Met Glu Ala Lys Met Ala Val Ala Met Ile Leu
1               5                   10                  15

Gln Lys Phe Ser Phe Glu Leu Ser Pro Ser Tyr Thr His Ala Pro Phe
            20                  25                  30

Ala Ile Val Thr Ile His Pro Gln Tyr Gly Ala Pro Leu Leu Met Arg
        35                  40                  45

Arg Leu
    50

<210>  107
<211>  153
<212>  DNA
<213>  Nicotiana tabacum

<400>  107
caaaactttg caatgatgga agcaaaaatg gcagtagcta tgatactaca taaattttcc      60

tttgaactat ccccttctta tacacatgct ccatttgcaa ttgtgactat tcatcctcag     120

tatggtgctc ctctgcttat gcgcagactt taa                                  153

<210>  108
<211>  49
<212>  PRT
<213>  Nicotiana tabacum

<400>  108

Gln Asn Phe Ala Met Met Glu Ala Lys Met Ala Val Ala Met Ile Leu
1               5                   10                  15

His Lys Phe Ser Phe Glu Leu Pro Ser Tyr Thr His Ala Pro Phe Ala
            20                  25                  30

Ile Val Thr Ile His Pro Gln Tyr Gly Ala Pro Leu Leu Met Arg Arg
        35                  40                  45

Leu

<210>  109
<211>  150
<212>  DNA
<213>  Nicotiana tabacum

<400>  109
caaaattttg ctatgttaga ggctaaaatg gcaatggcta tgattctgaa aacctatgca      60

tttgaactct ctccatctta tgctcatgct cctcatccac tactacttca acctcaatat     120

ggtgctcaat taattttgta caagttgtag                                      150

<210>  110
<211>  49
<212>  PRT
<213>  Nicotiana tabacum

<400>  110

Gln Asn Phe Ala Met Leu Glu Ala Lys Met Ala Met Ala Met Ile Leu
1               5                   10                  15

Lys Thr Tyr Ala Phe Glu Leu Ser Pro Ser Tyr Ala His Ala Pro His
            20                  25                  30

Pro Leu Leu Leu Gln Pro Gln Tyr Gly Ala Gln Leu Ile Leu Tyr Lys
        35                  40                  45

Leu

<210>  111
<211>  192
<212>  DNA
<213>  Nicotiana tabacum

<400>  111
tatagcatgg ggctcaaggc gattcaagct agcttagcta atcttctaca tggatttaac      60

tggtcattgc ctgataatat gactcctgag gacctcaaca tggatgagat ttttgggctc     120

tctacaccta aaaaatttcc acttgctact gtgattgagc caagactttc accaaaactt     180

tactctgttt ga                                                         192

<210>  112
<211>  63
<212>  PRT
<213>  Nicotiana tabacum

<400>  112

Tyr Ser Met Gly Leu Lys Ala Ile Gln Ala Ser Leu Ala Asn Leu Leu
1               5                   10                  15

His Gly Phe Asn Trp Ser Leu Pro Asp Asn Met Thr Pro Glu Asp Leu
            20                  25                  30

Asn Met Asp Glu Ile Phe Gly Leu Ser Thr Pro Lys Lys Phe Pro Leu
        35                  40                  45

Ala Thr Val Ile Glu Pro Arg Leu Ser Pro Lys Leu Tyr Ser Val
    50                  55                  60

<210>  113
<211>  192
<212>  DNA
<213>  Nicotiana tabacum

<400>  113
tatagcttgg ggctcaagga gattcaagct agcttagcta atcttctaca tggatttaac      60

tggtcattgc ctgataatat gactcctgag gacctcaaca tggatgagat ttttgggctc      120

tctacaccta aaaaatttcc acttgctact gtgattgagc caagactttc accaaaactt     180

tactctgttt ga                                                         192

<210>  114
<211>  63
<212>  PRT
<213>  Nicotiana tabacum

<400>  114

Tyr Ser Leu Gly Leu Lys Glu Ile Gln Ala Ser Leu Ala Asn Leu Leu
1               5                   10                  15

His Gly Phe Asn Trp Ser Leu Pro Asp Asn Met Thr Pro Glu Asp Leu
            20                  25                  30

Asn Met Asp Glu Ile Phe Gly Leu Ser Thr Pro Lys Lys Phe Pro Leu
        35                  40                  45

Ala Thr Val Ile Glu Pro Arg Leu Ser Pro Lys Leu Tyr Ser Val
    50                  55                  60

<210>  115
<211>  192
<212>  DNA
<213>  Nicotiana tabacum

<400>  115
catagcttgg ggctcaaggt gattcaagct agcttagcta atcttctaca tggatttaac      60

tggtcattgc ctgataatat gactcctgag gacctcaaca tggatgagat ttttgggctc      120

tctacaccta aaaaatttcc acttgctact gtgattgagc caagactttc accaaaactt     180

tactctgttt ga                                                          192

<210>  116
<211>  63
<212>  PRT
<213>  Nicotiana tabacum

<400>  116

His Ser Leu Gly Leu Lys Val Ile Gln Ala Ser Leu Ala Asn Leu Leu
1               5                   10                  15

His Gly Phe Asn Trp Ser Leu Pro Asp Asn Met Thr Pro Glu Asp Leu
            20                  25                  30

Asn Met Asp Glu Ile Phe Gly Leu Ser Thr Pro Lys Lys Phe Pro Leu
        35                  40                  45

Ala Thr Val Ile Glu Pro Arg Leu Ser Pro Lys Leu Tyr Ser Val
    50                  55                  60

<210>  117
<211>  96
<212>  DNA
<213>  Nicotiana tabacum

<400>  117
ctgtgctttc catgtttaat ctctagttat atactggctt tgaatgtgaa tctgtatcat      60

aatttcttgc aaatttctcc ttccatttct tattaa                                96

<210>  118
<211>  31
<212>  PRT
<213>  Nicotiana tabacum

<400>  118

Leu Cys Phe Pro Cys Leu Ile Ser Ser Tyr Ile Leu Ala Leu Asn Val
1               5                   10                  15

Asn Leu Tyr His Asn Phe Leu Gln Ile Ser Pro Ser Ile Ser Tyr
            20                  25                  30

<210>  119
<211>  192
<212>  DNA
<213>  Nicotiana tabacum

<400>  119
tctggacttg ctcaatgtgt ggttggttta gctttagcaa ctctagtgca gtgttttgag      60

tggaaaaggg taagcgaaga ggtggttgat ttgacggaag gaaaaggtct cactatgcca     120

aaacccgagc cactcatggc taggtgcgaa gctcgtgaca tttttcacaa agttctttca     180

gaaatatctt aa                                                         192

<210>  120

<211>  63
<212>  PRT
<213>  Nicotiana tabacum

<400>  120

Ser Gly Leu Ala Gln Cys Val Val Gly Leu Ala Leu Ala Thr Leu Val
1               5                   10                  15

Gln Cys Phe Glu Trp Lys Arg Val Ser Glu Glu Val Val Asp Leu Thr
            20                  25                  30

Glu Gly Lys Gly Leu Thr Met Pro Lys Pro Glu Pro Leu Met Ala Arg
        35                  40                  45

Cys Glu Ala Arg Asp Ile Phe His Lys Val Leu Ser Glu Ile Ser
    50                  55                  60

<210>  121
<211>  174
<212>  DNA
<213>  Nicotiana tabacum

<400>  121
ttgggcttgg caacggtgca tgtgaatttg atgttggccc gaatgattca agaatttgaa      60

tggtccgctt acccggaaaa taggaaagtg gattttactg agaaattgga atttactgtg     120

gtgatgaaaa atcctttaag agctaaggtc aagccaagaa tgcaagtggt gtaa           174

<210>  122
<211>  57
<212>  PRT
<213>  Nicotiana tabacum

<400>  122

Leu Gly Leu Ala Thr Val His Val Asn Leu Met Leu Ala Arg Met Ile
1               5                   10                  15

Gln Glu Phe Glu Trp Ser Ala Tyr Pro Glu Asn Arg Lys Val Asp Phe
            20                  25                  30

Thr Glu Lys Leu Glu Phe Thr Val Val Met Lys Asn Pro Leu Arg Ala
        35                  40                  45

Lys Val Lys Pro Arg Met Gln Val Val
    50                  55

<210>  123
<211>  171
<212>  DNA
<213>  Nicotiana tabacum

<400>  123
tatgctttgg ctatgcttca tttagagtac tttgtggcta atttggtttg gcattttcga      60

tgggaggctg tggagggaga tgatgttgat ctttcagaaa agctagaatt caccgttgtg     120

atgaagaatc cacttcgagc tcgtatctgc cccagagtta actctatttg a          171

<210>  124
<211>  56
<212>  PRT
<213>  Nicotiana tabacum

<400>  124

Tyr Ala Leu Ala Met Leu His Leu Glu Tyr Phe Val Ala Asn Leu Val
1               5                   10                  15

Trp His Phe Arg Trp Glu Ala Val Glu Gly Asp Asp Val Asp Leu Ser
            20                  25                  30

Glu Lys Leu Glu Phe Thr Val Val Met Lys Asn Pro Leu Arg Ala Arg
        35                  40                  45

Ile Cys Pro Arg Val Asn Ser Ile
    50                  55

<210>  125
<211>  186
<212>  DNA
<213>  Nicotiana tabacum

<400>  125
ggtcagcaag ttggacttct tagaacaacc attttcatcg cctcattact gtctgaatat      60

aagctgaaac ctcgctcaca ccagaaacaa gttgaactca ccgatttaaa tccagcaagt     120

tggcttcatt cgataaaagg cgaactgtta gtcgatgcga ttcctcgaaa gaaggcggca     180

ttttaa                                                                186

<210>  126
<211>  61
<212>  PRT
<213>  Nicotiana tabacum

<400>  126

Gly Gln Gln Val Gly Leu Leu Arg Thr Thr Ile Phe Ile Ala Ser Leu
1               5                   10                  15

Leu Ser Glu Tyr Lys Leu Lys Pro Arg Ser His Gln Lys Gln Val Glu
            20                  25                  30

Leu Thr Asp Leu Asn Pro Ala Ser Trp Leu His Ser Ile Lys Gly Glu
        35                  40                  45

Leu Leu Val Asp Ala Ile Pro Arg Lys Lys Ala Ala Phe
    50                  55                  60

<210>  127
<211>  171
<212>  DNA
<213>  Nicotiana tabacum

<400> 127
atcacatttg ctaagtttgt gaatgagcta gcattggcaa gattaatgtt ccattttgat 60

ttctcgctac caaaaggagt taagcatgag gatttggacg tggaggaagc tgctggaatt 120

actgttagaa ggaagttccc ccttttagcc gtcgccactc catgctcgtg a 171

<210> 128
<211> 56
<212> PRT
<213> Nicotiana tabacum

<400> 128

Ile Thr Phe Ala Lys Phe Val Asn Glu Leu Ala Leu Ala Arg Leu Met
1               5                   10                  15

Phe His Phe Asp Phe Ser Leu Pro Lys Gly Val Lys His Glu Asp Leu
            20                  25                  30

Asp Val Glu Glu Ala Ala Gly Ile Thr Val Arg Arg Lys Phe Pro Leu
        35                  40                  45

Leu Ala Val Ala Thr Pro Cys Ser
    50                  55

<210> 129
<211> 180
<212> DNA
<213> Nicotiana tabacum

<400> 129
cagaggtatg ctataaacca tttgatgctc tttattgcgt tgttcacggc tctgattgat 60

ttcaagaggc acaaaacgga cggctgtgat gatatcgcgt atattccaac cattgctcca 120

aaggatgatt gtaaagtgtt cctttcacag aggtgcactc gattcccatc tttttcatga 180

<210> 130
<211> 59
<212> PRT
<213> Nicotiana tabacum

<400> 130

Gln Arg Tyr Ala Ile Asn His Leu Met Leu Phe Ile Ala Leu Phe Thr
1               5                   10                  15

Ala Leu Ile Asp Phe Lys Arg His Lys Thr Asp Gly Cys Asp Asp Ile
            20                  25                  30

Ala Tyr Ile Pro Thr Ile Ala Pro Lys Asp Asp Cys Lys Val Phe Leu
        35                  40                  45

Ser Gln Arg Cys Thr Arg Phe Pro Ser Phe Ser
    50                  55

<210>  131
<211>  183
<212>  DNA
<213>  Nicotiana tabacum

<400>  131
atgtcatttg gtttagctaa tctttactta ccattggctc aattactcta tcactttgac      60

tggaaactcc caaccggaat caagccaaga gacttggact tgaccgaatt atcgggaata     120

actattgcta gaaagggtga cctttactta aatgctactc cttatcaacc ttctcgagag     180

taa                                                                   183

<210>  132
<211>  60
<212>  PRT
<213>  Nicotiana tabacum

<400>  132

Met Ser Phe Gly Leu Ala Asn Leu Tyr Leu Pro Leu Ala Gln Leu Leu
1               5                   10                  15

Tyr His Phe Asp Trp Lys Leu Pro Thr Gly Ile Lys Pro Arg Asp Leu
            20                  25                  30

Asp Leu Thr Glu Leu Ser Gly Ile Thr Ile Ala Arg Lys Gly Asp Leu
        35                  40                  45

Tyr Leu Asn Ala Thr Pro Tyr Gln Pro Ser Arg Glu
    50                  55                  60

<210>  133
<211>  174
<212>  DNA
<213>  Nicotiana tabacum

<400>  133
ttgggcttgg caacggtgca tgtgaatttg atgttggccc gaacgattca agaatttgaa      60

tggtccgctt acccggaaaa taggaaagtg gattttactg agaaattgga atttactgtg     120

gtgatgaaaa accctttaag agctaaggtc aagccaagaa tgcaagtggt gtaa           174

<210>  134
<211>  57
<212>  PRT
<213>  Nicotiana tabacum

<400>  134

Leu Gly Leu Ala Thr Val His Val Asn Leu Met Leu Ala Arg Thr Ile
1               5                   10                  15

Gln Glu Phe Glu Trp Ser Ala Tyr Pro Glu Asn Arg Lys Val Asp Phe
            20                  25                  30

Thr Glu Lys Leu Glu Phe Thr Val Val Met Lys Asn Pro Leu Arg Ala
        35                  40                  45

Lys Val Lys Pro Arg Met Gln Val Val
    50                  55

<210>  135
<211>  123
<212>  DNA
<213>  Nicotiana tabacum

<400>  135
ttgggcttgg caacggtgca tgtgaatttg atgttggccc gaatgattca agaatttgaa      60

tggtccgctt acccggaaaa taggaaagtg gatttactga gaaattggaa tttactgtgg     120

tga                                                                   123

<210>  136
<211>  40
<212>  PRT
<213>  Nicotiana tabacum

<400>  136

Leu Gly Leu Ala Thr Val His Val Asn Leu Met Leu Ala Arg Met Ile
1               5                   10                  15

Gln Glu Phe Glu Trp Ser Ala Tyr Pro Glu Asn Arg Lys Val Asp Leu
            20                  25                  30

Leu Arg Asn Trp Asn Leu Leu Trp
        35                  40

<210>  137
<211>  171
<212>  DNA
<213>  Nicotiana tabacum

<400>  137
atcacatttg ctaagtttgt gaatgagcta gcattggcaa gattaatgtt ccattttgat      60

ttctcgctac caaaaggagt taagcatgag gatttggacg tggaggaagc tgctggaatt     120

actgttagga ggaagttccc ccttttagcc gtcgccactc catgctcgtg a              171

<210>  138
<211>  56
<212>  PRT
<213>  Nicotiana tabacum

<400>  138

Ile Thr Phe Ala Lys Phe Val Asn Glu Leu Ala Leu Ala Arg Leu Met
1               5                   10                  15

Phe His Phe Asp Phe Ser Leu Pro Lys Gly Val Lys His Glu Asp Leu
            20                  25                  30

Asp Val Glu Glu Ala Ala Gly Ile Thr Val Arg Arg Lys Phe Pro Leu
        35                  40                  45

Leu Ala Val Ala Thr Pro Cys Ser
    50                  55

<210>  139
<211>  171
<212>  DNA
<213>  Nicotiana tabacum

<400>  139
atcacatttg ctaagtttgt gaatgagcta gcattggcaa gattaatgtt ccattttgat      60

ttctcgctac caaaaggagt taagcatgcg gatttggacg tggaggaagc tgctggaatt     120

actgttagaa ggaagttccc ccttttagcc gtcgccactc catgctcgtg a              171

<210>  140
<211>  56
<212>  PRT
<213>  Nicotiana tabacum

<400>  140

Ile Thr Phe Ala Lys Phe Val Asn Glu Leu Ala Leu Ala Arg Leu Met
1               5                   10                  15

Phe His Phe Asp Phe Ser Leu Pro Lys Gly Val Lys His Ala Asp Leu
            20                  25                  30

Asp Val Glu Glu Ala Ala Gly Ile Thr Val Arg Arg Lys Phe Pro Leu
        35                  40                  45

Leu Ala Val Ala Thr Pro Cys Ser
    50                  55

<210>  141
<211>  153
<212>  DNA
<213>  Nicotiana tabacum

<400>  141
caaaattttg ccatgttaga agcaaagact actttggcta tgatcctaca acgcttctcc      60

tttgaactgt ctccatctta tgcacatgct cctcagtcca taataacttt gcaaccccag     120

tatggtgctc cacttatttt gcataaaata tag                                  153

<210>  142
<211>  50
<212>  PRT
<213>  Nicotiana tabacum

<400>  142

Gln Asn Phe Ala Met Leu Glu Ala Lys Thr Thr Leu Ala Met Ile Leu
1               5                   10                  15

Gln Arg Phe Ser Phe Glu Leu Ser Pro Ser Tyr Ala His Ala Pro Gln
            20                  25                  30

Ser Ile Ile Thr Leu Gln Pro Gln Tyr Gly Ala Pro Leu Ile Leu His
        35                  40                  45

Lys Ile
    50

<210>  143
<211>  171
<212>  DNA
<213>  Nicotiana tabacum

<400>  143
attatccttg cactgccaat tcttggcatt accttgggac gcttggtgca gaactttgag      60

ttgttgcctc ctccaggaca gtcaaagctt gacacaacag agaaaggcgg gcaattcagt     120

ctgcacattt tgaagcattc caccattgtg atgaaaccaa gatcttttta a              171

<210>  144
<211>  56
<212>  PRT
<213>  Nicotiana tabacum

<400>  144

Ile Ile Leu Ala Leu Pro Ile Leu Gly Ile Thr Leu Gly Arg Leu Val
1               5                   10                  15

Gln Asn Phe Glu Leu Leu Pro Pro Pro Gly Gln Ser Lys Leu Asp Thr
            20                  25                  30

Thr Glu Lys Gly Gly Gln Phe Ser Leu His Ile Leu Lys His Ser Thr
        35                  40                  45

Ile Val Met Lys Pro Arg Ser Phe
    50                  55

<210>  145
<211>  267
<212>  DNA
<213>  Nicotiana tabacum

<400>  145
caaaattttg ccatgttaga agcaaagact actttggcta tgatcctaca acgcttctcc      60

tttgaactgt ctccatctta tgcacatgct cctcagtcca taataacttg caaccccagt     120

atggtgctcc acttattttg cataaaatat agtttattac ttgtaagtag tgtctcgttt     180

tatgttaagc atgagtccaa aatgttaagg cttgtagaac tgcaaaatgg gaatgcattt     240

gcactcgtgc actgtagatt gttgtaa                                         267

<210>  146
<211>  88
<212>  PRT
<213>  Nicotiana tabacum

<400> 146

Gln Asn Phe Ala Met Leu Glu Ala Lys Thr Thr Leu Ala Met Ile Leu
1               5                   10                  15

Gln Arg Phe Ser Phe Glu Leu Ser Pro Ser Tyr Ala His Ala Pro Gln
            20                  25                  30

Ser Ile Ile Thr Cys Asn Pro Ser Met Val Leu His Leu Phe Cys Ile
        35                  40                  45

Lys Tyr Ser Leu Leu Leu Val Ser Ser Val Ser Phe Tyr Val Lys His
    50                  55                  60

Glu Ser Lys Met Leu Arg Leu Val Glu Leu Gln Asn Gly Asn Ala Phe
65                  70                  75                  80

Ala Leu Val His Cys Arg Leu Leu
                85

<210>  147
<211>  186
<212>  DNA
<213>  Nicotiana tabacum

<400>  147
gctgatatgg ggttgcgagc agtttctttg gcattaggtg cacttattca atgctttgac      60

tggcaaattg aggaagcgga aagcttggag gaaagctata attctagaat gactatgcag     120

aacaagcctt tgaaggttgt ctgcactcca cgcgaagatc ttggccagct tctatcccaa     180

ctctaa                                                                186

<210>  148
<211>  61
<212>  PRT
<213>  Nicotiana tabacum

<400>  148

Ala Asp Met Gly Leu Arg Ala Val Ser Leu Ala Leu Gly Ala Leu Ile
1               5                   10                  15

Gln Cys Phe Asp Trp Gln Ile Glu Glu Ala Glu Ser Leu Glu Glu Ser
            20                  25                  30

Tyr Asn Ser Arg Met Thr Met Gln Asn Lys Pro Leu Lys Val Val Cys
        35                  40                  45

Thr Pro Arg Glu Asp Leu Gly Gln Leu Leu Ser Gln Leu
    50                  55                  60

<210>  149
<211>  1576
<212>  DNA
<213>  Nicotiana tabacum

<400> 149
cttccttcct aagtcctaac taaaaatgga gattcagttt tctaacttag ttgcattctt 60
gctctttctc tccagcatct ttcttctatt caaaaaatgg aaaaccagaa aactaaattt 120
gcctcctggt ccatggaaat taccttttat tggaagttta caccatttgg ctgtggcagg 180
tccacttcct caccatggcc taaaaaattt agccaaacgc tatggtcctc ttatgcattt 240
acaacttgga caaattccta cactcatcat atcatcacct caaatggcaa aagaagtact 300
aaaaactcac gacctcgctt ttgccactag accaaagctt gtcgcggccg acatcattca 360
ctacgacagc acggacatag cattttctcc gtacggtgaa tactggagac aaattcgtaa 420
aatttgcata ttggaactct tgagtgccaa gatggtcaaa ttttttagct cgattcgcca 480
agatgagctc tcgaagatgc tctcatctat acgaacgaca cccaatctta cagtcaatct 540
tactgacaaa atttttggtt ttacgagttc ggtaacttgt agatcagctt tagggaagat 600
atgtggtgac caagacaaat tgatcatttt tatgagggaa ataatatcat tggcaggtgg 660
atttagtatt gctgattttt tccctacatg gaaaatgatt catgatattg atggttcgaa 720
atctaaactg gtgaaagcac atcgtaagat tgatgaaatt ttgggaaatg ttgttgatga 780
gcacaaaaag aacagagcag atggcaagaa gggtaatggt gaatttggtg gtgaagattt 840
gattgatgta ttgttaagag ttagagaaag tggagaagtt caaattccta tcacaaatga 900
caatatcaaa tcaatattaa tcgacatgtt ctctgcagga tctgaaacat catcgacgac 960
tataatttgg gcattagctg aaatgatgaa gaaaccaagt gttttagcaa aggcacaagc 1020
tgaagtaagg caagctttga aggagaaaaa aggttttcaa cagattgatc ttgatgagct 1080
aaaatatctc aagttagtaa tcaaagaaac cttaagaatg caccctccaa ttcctctatt 1140
agttcctaga gaatgtatgg aggatacaaa gattgatggt tacaatatac ctttcaaaac 1200
aagagtcata gttaatgcat gggcaatcgg acgagatcca gaaagttggg atgaccccga 1260
aagctttatg ccagagagat ttgagaatag ttctattgac tttcttggaa atcatcatca 1320
gtttatacca tttggtgcag gaagaaggat ttgtccggga atgctatttg gtttagctaa 1380
tgttggacaa cctttagctc agttacttta tcacttcgat tggaaactcc ctaatggaca 1440
aagtcatgag aatttcgaca tgactgagtc acctggaatt tctgctacaa gaaaggatga 1500
tcttgttttg attgccactc cttatgattc ttattaagca gtagcagaaa taaaaagccg 1560
gggcaaacag aaaaaa 1576

<210> 150
<211> 503
<212> PRT
<213> Nicotiana tabacum

<400> 150

Met Glu Ile Gln Phe Ser Asn Leu Val Ala Phe Leu Leu Phe Leu Ser
1               5                   10                  15

Ser Ile Phe Leu Leu Phe Lys Lys Trp Lys Thr Arg Lys Leu Asn Leu
            20                  25                  30

Pro Pro Gly Pro Trp Lys Leu Pro Phe Ile Gly Ser Leu His His Leu
        35                  40                  45

Ala Val Ala Gly Pro Leu Pro His His Gly Leu Lys Asn Leu Ala Lys
    50                  55                  60

Arg Tyr Gly Pro Leu Met His Leu Gln Leu Gly Gln Ile Pro Thr Leu
65                  70                  75                  80

Ile Ile Ser Ser Pro Gln Met Ala Lys Glu Val Leu Lys Thr His Asp
                85                  90                  95

Leu Ala Phe Ala Thr Arg Pro Lys Leu Val Ala Ala Asp Ile Ile His
            100                 105                 110

Tyr Asp Ser Thr Asp Ile Ala Phe Ser Pro Tyr Gly Glu Tyr Trp Arg
        115                 120                 125

Gln Ile Arg Lys Ile Cys Ile Leu Glu Leu Leu Ser Ala Lys Met Val
    130                 135                 140

Lys Phe Phe Ser Ser Ile Arg Gln Asp Glu Leu Ser Lys Met Leu Ser
145                 150                 155                 160

Ser Ile Arg Thr Thr Pro Asn Leu Thr Val Asn Leu Thr Asp Lys Ile
                165                 170                 175

Phe Trp Phe Thr Ser Ser Val Thr Cys Arg Ser Ala Leu Gly Lys Ile
            180                 185                 190

Cys Gly Asp Gln Asp Lys Leu Ile Ile Phe Met Arg Glu Ile Ile Ser
        195                 200                 205

Leu Ala Gly Gly Phe Ser Ile Ala Asp Phe Phe Pro Thr Trp Lys Met
    210                 215                 220

Ile His Asp Ile Asp Gly Ser Lys Ser Lys Leu Val Lys Ala His Arg
225                 230                 235                 240

Lys Ile Asp Glu Ile Leu Gly Asn Val Val Asp Glu His Lys Lys Asn
                245                 250                 255

Arg Ala Asp Gly Lys Lys Gly Asn Gly Glu Phe Gly Gly Glu Asp Leu
            260                 265                 270

Ile Asp Val Leu Leu Arg Val Arg Glu Ser Gly Glu Val Gln Ile Pro
        275                 280                 285

Ile Thr Asn Asp Asn Ile Lys Ser Ile Leu Ile Asp Met Phe Ser Ala
    290                 295                 300

Gly Ser Glu Thr Ser Ser Thr Thr Ile Ile Trp Ala Leu Ala Glu Met
305                 310                 315                 320

Met Lys Lys Pro Ser Val Leu Ala Lys Ala Gln Ala Glu Val Arg Gln
                325                 330                 335

Ala Leu Lys Glu Lys Lys Gly Phe Gln Gln Ile Asp Leu Asp Glu Leu
            340                 345                 350

Lys Tyr Leu Lys Leu Val Ile Lys Glu Thr Leu Arg Met His Pro Pro
        355                 360                 365

Ile Pro Leu Leu Val Pro Arg Glu Cys Met Glu Asp Thr Lys Ile Asp
    370                 375                 380

Gly Tyr Asn Ile Pro Phe Lys Thr Arg Val Ile Val Asn Ala Trp Ala
385                 390                 395                 400

Ile Gly Arg Asp Pro Glu Ser Trp Asp Asp Pro Glu Ser Phe Met Pro
                405                 410                 415

Glu Arg Phe Glu Asn Ser Ser Ile Asp Phe Leu Gly Asn His His Gln
            420                 425                 430

Phe Ile Pro Phe Gly Ala Gly Arg Arg Ile Cys Pro Gly Met Leu Phe
        435                 440                 445

Gly Leu Ala Asn Val Gly Gln Pro Leu Ala Gln Leu Leu Tyr His Phe
    450                 455                 460

Asp Trp Lys Leu Pro Asn Gly Gln Ser His Glu Asn Phe Asp Met Thr
465                 470                 475                 480

Glu Ser Pro Gly Ile Ser Ala Thr Arg Lys Asp Asp Leu Val Leu Ile
                485                 490                 495

Ala Thr Pro Tyr Asp Ser Tyr
            500

<210> 151
<211> 1581
<212> DNA
<213> Nicotiana tabacum

<400> 151
tccttcttcc ttcctaagtc ctaactaaaa atggagattc agttttctaa cttagttgca 60

ttcttgctct ttctctccag catctttctt ctattcaaaa aatggaaaac cagaaaacta 120

aatttgcctc ctggtccatg gaaattacct tttattggaa gtttacacca tttggctgtg 180

gcaggtccac ttcctcacca tggcctaaaa aatttagcca aacgctatgg tcctcttatg 240
catttacaac ttggacaaat tcctacactc atcatatcat cacctcaaat ggcaaaagaa 300
gtactaaaaa ctcacgacct cgcttttgcc actagaccaa agcttgtcgt ggccgacatc 360
attcactacg acagcacgga catagcattt tctccgtacg gtgaatactg gagacaaatt 420
cgtaaaattt gcatattgga actcttgagt gccaagatgg tcaaattttt tagctcgatt 480
cgccaagatg agctctcgaa gatgctctca tctatacgaa cgacacccaa tcttacagtc 540
aatcttactg acaaaatttt ttggtttacg agttcggtaa cttgtagatc agctttaggg 600
aagatatgtg gtgaccaaga caaattgatc atttttatga gggaaataat atcattggca 660
ggtggattta gtattgctga tttttttcct acatggaaaa tgattcatga tattgatggt 720
tcgaaatcta aactggtgaa agcacatcgt aagattgatg aaattttggg aaatgttgtt 780
gatgagcaca aaaagaacag agcagatggc aagaagggta atggtgaatt tggtggtgaa 840
gatttgattg atgtattgtt aagagttaga gaaagtggag aagttcaaat tcctatcaca 900
aatgacaata tcaaatcaat attaatcgac atgttctctg cgggatctga aacatcatcg 960
acgactataa tttgggcatt agctgaaatg atgaagaaac caagtgtttt agcaaaggca 1020
caagctgaag taaggcaagc tttgaaggag aaaaaaggtt ttcaacagat tgatcttgat 1080
gagctaaaat atctcaagtt agtaatcaaa gaaaccttaa gaatgcaccc tccaattcct 1140
ctattagttc ctagagaatg tatggaggat acaaagattg atggttacaa tatacctttc 1200
aaaacaagag tcatagttaa tgcatgggca atcggacgag atccagaaag ttgggatgac 1260
cccgaaagct ttatgccaga gagatttgag aatagttcta ttgactttct tggaaatcat 1320
catcagttta taccatttgg tgcaggaaga aggatttgtc cgggaatgct atttggttta 1380
gctaatgttg gacaaccttt agctcagtta ctttatcact tcgattggaa actccctaat 1440
ggacaaagtc atgagaattt cgacatgact gagtcacctg gaatttctgc tacaagaaag 1500
gatgatcttg ttttgattgc cactccttat gattcttatt aagcagtagc agaaataaaa 1560
agccggggca aacagaaaaa a 1581

<210> 152
<211> 503
<212> PRT
<213> Nicotiana tabacum

<400> 152

Met Glu Ile Gln Phe Ser Asn Leu Val Ala Phe Leu Leu Phe Leu Ser
1               5                   10                  15

Ser Ile Phe Leu Leu Phe Lys Lys Trp Lys Thr Arg Lys Leu Asn Leu
            20                  25                  30

Pro Pro Gly Pro Trp Lys Leu Pro Phe Ile Gly Ser Leu His His Leu
        35                  40                  45

Ala Val Ala Gly Pro Leu Pro His His Gly Leu Lys Asn Leu Ala Lys
    50                  55                  60

Arg Tyr Gly Pro Leu Met His Leu Gln Leu Gly Gln Ile Pro Thr Leu
65                  70                  75                  80

Ile Ile Ser Ser Pro Gln Met Ala Lys Glu Val Leu Lys Thr His Asp
                85                  90                  95

Leu Ala Phe Ala Thr Arg Pro Lys Leu Val Val Ala Asp Ile Ile His
            100                 105                 110

Tyr Asp Ser Thr Asp Ile Ala Phe Ser Pro Tyr Gly Glu Tyr Trp Arg
        115                 120                 125

Gln Ile Arg Lys Ile Cys Ile Leu Glu Leu Leu Ser Ala Lys Met Val
    130                 135                 140

Lys Phe Phe Ser Ser Ile Arg Gln Asp Glu Leu Ser Lys Met Leu Ser
145                 150                 155                 160

Ser Ile Arg Thr Thr Pro Asn Leu Thr Val Asn Leu Thr Asp Lys Ile
                165                 170                 175

Phe Trp Phe Thr Ser Ser Val Thr Cys Arg Ser Ala Leu Gly Lys Ile
            180                 185                 190

Cys Gly Asp Gln Asp Lys Leu Ile Ile Phe Met Arg Glu Ile Ile Ser
        195                 200                 205

Leu Ala Gly Gly Phe Ser Ile Ala Asp Phe Phe Pro Thr Trp Lys Met
    210                 215                 220

Ile His Asp Ile Asp Gly Ser Lys Ser Lys Leu Val Lys Ala His Arg
225                 230                 235                 240

Lys Ile Asp Glu Ile Leu Gly Asn Val Val Asp Glu His Lys Lys Asn
                245                 250                 255

Arg Ala Asp Gly Lys Lys Gly Asn Gly Glu Phe Gly Gly Glu Asp Leu
            260                 265                 270

Ile Asp Val Leu Leu Arg Val Arg Glu Ser Gly Glu Val Gln Ile Pro
        275                 280                 285

Ile Thr Asn Asp Asn Ile Lys Ser Ile Leu Ile Asp Met Phe Ser Ala
    290                 295                 300

Gly Ser Glu Thr Ser Ser Thr Thr Ile Ile Trp Ala Leu Ala Glu Met
305                 310                 315                 320

Met Lys Lys Pro Ser Val Leu Ala Lys Ala Gln Ala Glu Val Arg Gln
                325                 330                 335

Ala Leu Lys Glu Lys Lys Gly Phe Gln Gln Ile Asp Leu Asp Glu Leu
            340                 345                 350

Lys Tyr Leu Lys Leu Val Ile Lys Glu Thr Leu Arg Met His Pro Pro
        355                 360                 365

Ile Pro Leu Leu Val Pro Arg Glu Cys Met Glu Asp Thr Lys Ile Asp
    370                 375                 380

Gly Tyr Asn Ile Pro Phe Lys Thr Arg Val Ile Val Asn Ala Trp Ala
385                 390                 395                 400

Ile Gly Arg Asp Pro Glu Ser Trp Asp Asp Pro Glu Ser Phe Met Pro
                405                 410                 415

Glu Arg Phe Glu Asn Ser Ser Ile Asp Phe Leu Gly Asn His His Gln
            420                 425                 430

Phe Ile Pro Phe Gly Ala Gly Arg Arg Ile Cys Pro Gly Met Leu Phe
        435                 440                 445

Gly Leu Ala Asn Val Gly Gln Pro Leu Ala Gln Leu Leu Tyr His Phe
    450                 455                 460

Asp Trp Lys Leu Pro Asn Gly Gln Ser His Glu Asn Phe Asp Met Thr
465                 470                 475                 480

Glu Ser Pro Gly Ile Ser Ala Thr Arg Lys Asp Asp Leu Val Leu Ile
                485                 490                 495

Ala Thr Pro Tyr Asp Ser Tyr
            500

<210>  153
<211>  1538
<212>  DNA
<213>  Nicotiana tabacum

<400>  153
caactgcagt ttgaagatac caactaacca aaatgcagtt cttcagcttg gtttccattt      60

tcctatttct atcttttctc tttttgttaa ggaaatggaa gaactcgaat agccaaagga     120

aaaaattgcc accaggtcca tggaaactac caatactagg aagtatgctt catatggttg     180

gtggactacc acaccatgtc cttagagatt tagccaaaaa atatggaccg cttatgcacc     240

ttcaattagg tgaagtttct gcagttgtgg ttacttctcc tgatatggca aaagaagtac     300

taaaaactca tgacatcgct ttcgcgtcta ggcctagcct tttggccccg gagattgtct     360

gttacaatag gtctgatctt gcgttttgcc cctatggcga ttattggaga caaatgcgta     420

aaatatgtgt cttggaagtg ctcagtgcca agaatgttcg gacatatagc tctattaggc 480
gcgatgaagt tcttcgtctc cttaatttta tccggtcatc ttctggtgag cctgttaata 540
ttacggaaag gatctttttg ttcacaagct ccatgacatg tagatcagcg tttgggcaag 600
tattcaagga gcaagacaaa tttatacaac taattaaaga agttatactc ttagcaggag 660
ggtttgatgt ggctgacata ttcccttcat acaagtctct tcatgtgctc agtggaatga 720
agggtaagat tatgaatgca caccataagg tagatgctat tgttgagaat gtcatcaacg 780
agcacaagaa aaatcttgca attgggaaaa ctaatggagc gttaggaggt gaagatttaa 840
ttgatgttct tctaaaactt atgaatgatg gaggccttca atttcctatc accaacgaca 900
acatcaaagc tataatcttt gacatgtttg ctgctggaac agagacttca tcgtcaacaa 960
ttgtgtgggc tatggtggaa atggtgaaaa atccaactgt atttgcgaaa gctcaagcag 1020
aagtaagaga tgcatttaga gaaaaagaaa cttttgatga aaatgatgtg gaggagctaa 1080
actatctaaa gttagtcatt aaagaaactc taagacttca tccaccggtt ccacttttgc 1140
tcccaagaga atgtagggaa gagacaaata taaacggcta cactattcct gtaaagacca 1200
aagtcatggt taatgtttgg gcattgggaa gagatccaaa atattgggat gatgcagaaa 1260
cttttaagcc agagagattt gagcagtgct ctaaggattt tgttggtaat aattttgaat 1320
atcttccatt tggtggtgga aggaggattt gtccagggat ttcgtttggt ttagctaatg 1380
cttatttgcc attggctcaa ttactttatc actttgattg ggaactcccc actggaatca 1440
aaccaagcga cttggacttg actgagttgg ttggagtaac tgccgctaga aaaagtgacc 1500
tttacttggt tgcgactcct tatcaacctc ctcaaaac 1538

<210> 154
<211> 502
<212> PRT
<213> Nicotiana tabacam

<400> 154

Met Gln Phe Phe Ser Leu Val Ser Ile Phe Leu Phe Leu Ser Phe Leu
1               5                   10                  15

Phe Leu Leu Arg Lys Trp Lys Asn Ser Asn Ser Gln Arg Lys Lys Leu
            20                  25                  30

Pro Pro Gly Pro Trp Lys Leu Pro Ile Leu Gly Ser Met Leu His Met
        35                  40                  45

Val Gly Gly Leu Pro His His Val Leu Arg Asp Leu Ala Lys Lys Tyr
    50                  55                  60

Gly Pro Leu Met His Leu Gln Leu Gly Glu Val Ser Ala Val Val Val
65                  70                  75                  80

Thr Ser Pro Asp Met Ala Lys Glu Val Leu Lys Thr His Asp Ile Ala
                85                  90                  95

Phe Ala Ser Arg Pro Ser Leu Leu Ala Pro Glu Ile Val Cys Tyr Asn
            100                 105                 110

Arg Ser Asp Leu Ala Phe Cys Pro Tyr Gly Asp Tyr Trp Arg Gln Met
        115                 120                 125

Arg Lys Ile Cys Val Leu Glu Val Leu Ser Ala Lys Asn Val Arg Thr
    130                 135                 140

Tyr Ser Ser Ile Arg Arg Asp Glu Val Leu Arg Leu Leu Asn Phe Ile
145                 150                 155                 160

Arg Ser Ser Ser Gly Glu Pro Val Asn Ile Thr Glu Arg Ile Phe Leu
                165                 170                 175

Phe Thr Ser Ser Met Thr Cys Arg Ser Ala Phe Gly Gln Val Phe Lys
            180                 185                 190

Glu Gln Asp Lys Phe Ile Gln Leu Ile Lys Glu Val Ile Leu Leu Ala
        195                 200                 205

Gly Gly Phe Asp Val Ala Asp Ile Phe Pro Ser Tyr Lys Ser Leu His
    210                 215                 220

Val Leu Ser Gly Met Lys Gly Lys Ile Met Asn Ala His His Lys Val
225                 230                 235                 240

Asp Ala Ile Val Glu Asn Val Ile Asn Glu His Lys Lys Asn Leu Ala
                245                 250                 255

Ile Gly Lys Thr Asn Gly Ala Leu Gly Gly Glu Asp Leu Ile Asp Val
            260                 265                 270

Leu Leu Lys Leu Met Asn Asp Gly Gly Leu Gln Phe Pro Ile Thr Asn
        275                 280                 285

Asp Asn Ile Lys Ala Ile Ile Phe Asp Met Phe Ala Ala Gly Thr Glu
    290                 295                 300

Thr Ser Ser Ser Thr Ile Val Trp Ala Met Val Glu Met Val Lys Asn
305                 310                 315                 320

Pro Thr Val Phe Ala Lys Ala Gln Ala Glu Val Arg Asp Ala Phe Arg
                325                 330                 335

Glu Lys Glu Thr Phe Asp Glu Asn Asp Val Glu Glu Leu Asn Tyr Leu
            340                 345                 350

Lys Leu Val Ile Lys Glu Thr Leu Arg Leu His Pro Pro Val Pro Leu
        355                 360                 365

Leu Leu Pro Arg Glu Cys Arg Glu Glu Thr Asn Ile Asn Gly Tyr Thr
    370                 375                 380

Ile Pro Val Lys Thr Lys Val Met Val Asn Val Trp Ala Leu Gly Arg
385                 390                 395                 400

Asp Pro Lys Tyr Trp Asp Asp Ala Glu Thr Phe Lys Pro Glu Arg Phe
                405                 410                 415

Glu Gln Cys Ser Lys Asp Phe Val Gly Asn Asn Phe Glu Tyr Leu Pro
            420                 425                 430

Phe Gly Gly Gly Arg Arg Ile Cys Pro Gly Ile Ser Phe Gly Leu Ala
        435                 440                 445

Asn Ala Tyr Leu Pro Leu Ala Gln Leu Leu Tyr His Phe Asp Trp Glu
    450                 455                 460

Leu Pro Thr Gly Ile Lys Pro Ser Asp Leu Asp Leu Thr Glu Leu Val
465                 470                 475                 480

Gly Val Thr Ala Ala Arg Lys Ser Asp Leu Tyr Leu Val Ala Thr Pro
                485                 490                 495

Tyr Gln Pro Pro Gln Asn
            500

<210> 155
<211> 1530
<212> DNA
<213> Nicotiana tabacum

<400> 155
aaaagatgtc ttcattttcc acatcttctg ccacttctaa ttccaaactt ccagttcgag 60

aaatcccagg agactatggt ttcccctttt ttggagccat aaaagataga tatgactact 120

tctacaacct cggcacagac gaattctttc ttaccaaaat gcaaaaatac aactctactg 180

tctttagaac caacatgcca ccaggtccat tcattgctaa aaatcccaaa gtaattgttc 240

tcctcgatgc caaaacattt cccgttcttt tcgacaactc taaagtcgaa aaaatgaacg 300

ttcttgatgg cacgtacgtg ccatctactg atttctatgg cggatatcgc ccgtgtgctt 360

atcttgatcc ttctgagtca actcatgcca cacttaaagg gttcttttta tctttaatct 420

cccagcttca taatcaattt attcctttat ttagaacctc aatttctggt cttttcgcaa 480

atcttgagaa tgagatttcc caaaatggca aagcgaactt caacaatatc agcgacatta 540

tgtcattcga ttttgttttt cgtttgttat gtgacaagac cagtccccat gacacaaatc 600

ttggctctaa tggaccaaaa ctctttgata tatggctgtt gcctcaactt gctccattgt 660

ttagtctagg tctaaaattt gtgccgaact ttctggaaga tttaatgttg catacttttc 720

ccttgccatt ttttctagtg agatcgaatt accagaagct ttatgatgct tttagcaagc 780
atgccgaaag tacactgaat gaagcagaga agaatgggat caaaagagac gaagcatgcc 840
acaacttagt ttttcttgca ggtttcaatg cttatggtgg gatgaaagtt ttattccctg 900
cactgataaa gtgggtcgcc aatggaggaa agagtttaca cactcggctg gcaaatgaaa 960
tcaggacaat tatcaaagaa gaatgtggga ccataactct atcagcaatc aacaagatga 1020
gtttagtaaa atcagtagtg tatgaagtat taagaattga acctccagtt ccattccaat 1080
atggtaaagc caaagaagat atcataatcc aaagccatga ttcaactttc ttagtcaaga 1140
aaggtgaaat gatctttgga tatcagcctt ttgctacaaa agatccaaag atttttgaca 1200
aaccagagga gtttattccg gagaggttca tggccgaagg ggaaaaatta ttaaagtatg 1260
tgtattggtc aaatgcaaga gagacagatg atccaacggt ggacaacaaa caatgcccag 1320
cgaaaaatct tgtcgtgctt ttgtgcaggt tgatgttggt ggaggttttc atgcgttacg 1380
acacattcac agtggagtca acaaagctct ttcttgggtc atcagtaacg ttcacgactc 1440
tggaaaaagc gacatgagtt tcagatatct taattgtagg ctgcaaataa taatgtggtc 1500
attctgcaaa ttattgtact tgtgctgatg 1530

<210> 156
<211> 483
<212> PRT
<213> Nicotiana tabacum

<400> 156

Met Ser Ser Phe Ser Thr Ser Ser Ala Thr Ser Asn Ser Lys Leu Pro
1               5                   10                  15

Val Arg Glu Ile Pro Gly Asp Tyr Gly Phe Pro Phe Phe Gly Ala Ile
            20                  25                  30

Lys Asp Arg Tyr Asp Tyr Phe Tyr Asn Leu Gly Thr Asp Glu Phe Phe
        35                  40                  45

Leu Thr Lys Met Gln Lys Tyr Asn Ser Thr Val Phe Arg Thr Asn Met
    50                  55                  60

Pro Pro Gly Pro Phe Ile Ala Lys Asn Pro Lys Val Ile Val Leu Leu
65                  70                  75                  80

Asp Ala Lys Thr Phe Pro Val Leu Phe Asp Asn Ser Lys Val Glu Lys
                85                  90                  95

Met Asn Val Leu Asp Gly Thr Tyr Val Pro Ser Thr Asp Phe Tyr Gly
            100                 105                 110

Gly Tyr Arg Pro Cys Ala Tyr Leu Asp Pro Ser Glu Ser Thr His Ala
        115                 120                 125

Thr Leu Lys Gly Phe Phe Leu Ser Leu Ile Ser Gln Leu His Asn Gln
    130                 135                 140

Phe Ile Pro Leu Phe Arg Thr Ser Ile Ser Gly Leu Phe Ala Asn Leu
145                 150                 155                 160

Glu Asn Glu Ile Ser Gln Asn Gly Lys Ala Asn Phe Asn Asn Ile Ser
                165                 170                 175

Asp Ile Met Ser Phe Asp Phe Val Phe Arg Leu Leu Cys Asp Lys Thr
            180                 185                 190

Ser Pro His Asp Thr Asn Leu Gly Ser Asn Gly Pro Lys Leu Phe Asp
        195                 200                 205

Ile Trp Leu Leu Pro Gln Leu Ala Pro Leu Phe Ser Leu Gly Leu Lys
    210                 215                 220

Phe Val Pro Asn Phe Leu Glu Asp Leu Met Leu His Thr Phe Pro Leu
225                 230                 235                 240

Pro Phe Phe Leu Val Arg Ser Asn Tyr Gln Lys Leu Tyr Asp Ala Phe
                245                 250                 255

Ser Lys His Ala Glu Ser Thr Leu Asn Glu Ala Glu Lys Asn Gly Ile
            260                 265                 270

Lys Arg Asp Glu Ala Cys His Asn Leu Val Phe Leu Ala Gly Phe Asn
        275                 280                 285

Ala Tyr Gly Gly Met Lys Val Leu Phe Pro Ala Leu Ile Lys Trp Val
    290                 295                 300

Ala Asn Gly Gly Lys Ser Leu His Thr Arg Leu Ala Asn Glu Ile Arg
305                 310                 315                 320

Thr Ile Ile Lys Glu Glu Cys Gly Thr Ile Thr Leu Ser Ala Ile Asn
                325                 330                 335

Lys Met Ser Leu Val Lys Ser Val Val Tyr Glu Val Leu Arg Ile Glu
            340                 345                 350

Pro Pro Val Pro Phe Gln Tyr Gly Lys Ala Lys Glu Asp Ile Ile Ile
        355                 360                 365

Gln Ser His Asp Ser Thr Phe Leu Val Lys Lys Gly Glu Met Ile Phe
    370                 375                 380

Gly Tyr Gln Pro Phe Ala Thr Lys Asp Pro Lys Ile Phe Asp Lys Pro
385                 390                 395                 400

Glu Glu Phe Ile Pro Glu Arg Phe Met Ala Glu Gly Glu Lys Leu Leu
                405                 410                 415

Lys Tyr Val Tyr Trp Ser Asn Ala Arg Glu Thr Asp Asp Pro Thr Val
            420                 425                 430

Asp Asn Lys Gln Cys Pro Ala Lys Asn Leu Val Val Leu Leu Cys Arg
        435                 440                 445

Leu Met Leu Val Glu Val Phe Met Arg Tyr Asp Thr Phe Thr Val Glu
    450                 455                 460

Ser Thr Lys Leu Phe Leu Gly Ser Ser Val Thr Phe Thr Thr Leu Glu
465                 470                 475                 480

Lys Ala Thr

<210>  157
<211>  1593
<212>  DNA
<213>  Nicotiana tabacum

<400>  157
ccaaaaatgg agcttcaatc ttctcctttc aatttaattt ctttgttcct cttcttttct      60

tttcatttta ttctagtgaa gaaatggaat gccaaaatcc caaagttacc tccaggtccg     120

tggaggcttc cctttattgg aagcctccat cacttgaagg gaaaacttcc acaccataat     180

cttagagatc tagcgcgaaa atatgggcct ctcatgtact tacaactcgg agaaattcct     240

gtagttgtaa tatcttcgcc acgtgtagca aaagctgtac taaaaactca tgatctcgct     300

tttgcaacta gaccacgatt catgtcctca gacattgtgt tttacaaaag cagggacatc     360

tcttttgccc catttggtga ttactggaga cagatgcgta aaatattgac tcaggaactc     420

ctgagtaaca agatgctcaa gtcatatagc ttaatccgaa aggatgagct ctcgaagctc     480

ctctcatcga ttcgtttgga aacaggttct gcagtgaaca taaatgaaaa gcttctctgg     540

tttacgagct gcatgacctg tagattagcc tttggaaaaa tatgcaatga tcgggatgag     600

ttgatcatgc taattaggga gatattaaca ttatcaggag gatttgatgt gggtgatttg     660

ttcccttcct ggaaattact tcataatatg agcaacatga aagctaggtt gacgaatgta     720

caccacaagt atgatttagt tatggagaac atcatcaatg agcaccaaga gaatcatgca     780

gcagggataa agggtaacaa cgagtttggt ggcgaagata tgatcgatgc tctactgagg     840

gctaaggaga ataatgagct tcaatttcct atcgaaaatg acaacatgaa agcagtaatt     900

ctggacttgt ttattgctgg aactgaaact tcatatactg caattatatg ggcactatca     960

gaattgatga agcacccaag tgtgatggcc aaggcacaag ctgaagtgag aaaagtcttc    1020

aaagaaaatg aaaatttcga cgaaaatgat cttgacaagt tgccatactt aaaatcagtg    1080

attaaagaaa cactaaggat gcaccctcca gttcctttgt tagggcctag agaatgcagg    1140

gaccaaacag agatcgatgg ctacactgta cctattaaag ctagagttat ggttaatgct 1200

tgggcgatag gaagagatcc tgaaagttgg gaagatcctg aaagtttcaa accggagcga 1260

tttgaaaata cttctgttga tcttacagga aatcactatc agttcattcc tttcggttca 1320

ggaagaagaa tgtgtccagg aatgtcgttt ggtttagtta acacagggca tcctttagcc 1380

cagttgctct attgctttga ctggaaactc cctgacaagg ttaatgcaaa tgattttcgc 1440

actactgaaa caagtagagt ttttgcagca agcaaagatg acctctactt gattcccaca 1500

aatcacaggg agcaagaata gcttaattta atggagttct tggaagaatt aaagaagaag 1560

ggctatatag gtgagatttt ttgtatggtt gca 1593

<210> 158
<211> 504
<212> PRT
<213> Nicotiana tabacum

<400> 158

Met Glu Leu Gln Ser Ser Pro Phe Asn Leu Ile Ser Leu Phe Leu Phe
1               5                   10                  15

Phe Ser Phe His Phe Ile Leu Val Lys Lys Trp Asn Ala Lys Ile Pro
            20                  25                  30

Lys Leu Pro Pro Gly Pro Trp Arg Leu Pro Phe Ile Gly Ser Leu His
        35                  40                  45

His Leu Lys Gly Lys Leu Pro His His Asn Leu Arg Asp Leu Ala Arg
    50                  55                  60

Lys Tyr Gly Pro Leu Met Tyr Leu Gln Leu Gly Glu Ile Pro Val Val
65                  70                  75                  80

Val Ile Ser Ser Pro Arg Val Ala Lys Ala Val Leu Lys Thr His Asp
                85                  90                  95

Leu Ala Phe Ala Thr Arg Pro Arg Phe Met Ser Ser Asp Ile Val Phe
            100                 105                 110

Tyr Lys Ser Arg Asp Ile Ser Phe Ala Pro Phe Gly Asp Tyr Trp Arg
        115                 120                 125

Gln Met Arg Lys Ile Leu Thr Gln Glu Leu Leu Ser Asn Lys Met Leu
    130                 135                 140

Lys Ser Tyr Ser Leu Ile Arg Lys Asp Glu Leu Ser Lys Leu Leu Ser
145                 150                 155                 160

Ser Ile Arg Leu Glu Thr Gly Ser Ala Val Asn Ile Asn Glu Lys Leu
                165                 170                 175

Leu Trp Phe Thr Ser Cys Met Thr Cys Arg Leu Ala Phe Gly Lys Ile
            180                 185                 190

Cys Asn Asp Arg Asp Glu Leu Ile Met Leu Ile Arg Glu Ile Leu Thr
        195                 200                 205

Leu Ser Gly Gly Phe Asp Val Gly Asp Leu Phe Pro Ser Trp Lys Leu
    210                 215                 220

Leu His Asn Met Ser Asn Met Lys Ala Arg Leu Thr Asn Val His His
225                 230                 235                 240

Lys Tyr Asp Leu Val Met Glu Asn Ile Ile Asn Glu His Gln Glu Asn
                245                 250                 255

His Ala Ala Gly Ile Lys Gly Asn Asn Glu Phe Gly Gly Glu Asp Met
            260                 265                 270

Ile Asp Ala Leu Leu Arg Ala Lys Glu Asn Asn Glu Leu Gln Phe Pro
        275                 280                 285

Ile Glu Asn Asp Asn Met Lys Ala Val Ile Leu Asp Leu Phe Ile Ala
    290                 295                 300

Gly Thr Glu Thr Ser Tyr Thr Ala Ile Ile Trp Ala Leu Ser Glu Leu
305                 310                 315                 320

Met Lys His Pro Ser Val Met Ala Lys Ala Gln Ala Glu Val Arg Lys
                325                 330                 335

Val Phe Lys Glu Asn Glu Asn Phe Asp Glu Asn Asp Leu Asp Lys Leu
            340                 345                 350

Pro Tyr Leu Lys Ser Val Ile Lys Glu Thr Leu Arg Met His Pro Pro
        355                 360                 365

Val Pro Leu Leu Gly Pro Arg Glu Cys Arg Asp Gln Thr Glu Ile Asp
    370                 375                 380

Gly Tyr Thr Val Pro Ile Lys Ala Arg Val Met Val Asn Ala Trp Ala
385                 390                 395                 400

Ile Gly Arg Asp Pro Glu Ser Trp Glu Asp Pro Glu Ser Phe Lys Pro
                405                 410                 415

Glu Arg Phe Glu Asn Thr Ser Val Asp Leu Thr Gly Asn His Tyr Gln
            420                 425                 430

Phe Ile Pro Phe Gly Ser Gly Arg Arg Met Cys Pro Gly Met Ser Phe
        435                 440                 445

Gly Leu Val Asn Thr Gly His Pro Leu Ala Gln Leu Leu Tyr Cys Phe
    450                 455                 460

Asp Trp Lys Leu Pro Asp Lys Val Asn Ala Asn Asp Phe Arg Thr Thr
465                 470                 475                 480

Glu Thr Ser Arg Val Phe Ala Ala Ser Lys Asp Asp Leu Tyr Leu Ile
                485                 490                 495

Pro Thr Asn His Arg Glu Gln Glu
            500

<210> 159
<211> 1599
<212> DNA
<213> Nicotiana tabacum

<400> 159
cttcttccaa aaatggagct tcaatcttct cctttcaatt taatttcttt gttcctcttc 60

ttttcttttc tttttattct agtgaagaaa tggaatgcca aaatcccaaa gttacctcca 120

ggtccgtgga ggcttccctt tattggaagc ctccatcact tgaagggaaa acttccacac 180

cataatctta gagatctagc gcgaaaatat ggacctctca tgtacttaca actcggagaa 240

attcctgtag ttgtaatatc ttcgccacgt gtagcaaaag ctgtactaaa aactcatgat 300

ctcgcttttg caactagacc acgattcatg tcctcagaca ttgtgtttta caaaagcagg 360

gacatctctt ttgccccatt tggtgattac tggagacaga tgcgtaaaat attgactcag 420

gaactcctga gtaacaagat gctcaagtca tatagcttaa tccgaaagga tgagctctcg 480

aagctcctct catcgattcg tttggaaaca ggttctgcag tgaacataaa tgaaaagctt 540

ctctggttta cgagctgcat gacctgtaga ttagcctttg gaaaaatatg caatgatcgg 600

gatgagttga tcatgctaat tagggagata ttaacattat caggaggatt tgatgtgggt 660

gatttgttcc cttcctggaa attacttcat aatatgagca acatgaaagc taggttgacg 720

aatgtacacc acaagtatga tttagttatg gagaacatca tcaatgagca ccaagagaat 780

catgcagcag ggataaaggg taacaacgag tttggtggcg aagatatgat cgatgctcta 840

ctgagggcta aggagaataa tgagcttcaa tttcctatcg aaaatgacaa catgaaagca 900

gtaattctgg acttgtttat tgctggaact gaaacttcat atactgcaat tatatgggca 960

ctatcagaat tgatgaagca cccaagtgtg atggccaagg cacaagctga agtgagaaaa 1020

gtcttcaaag aaaatgaaaa tttcgacgaa aatgatcttg acaagttgcc atacttaaaa 1080

tcagtgatta aagaaacact aaggatgcac cctccagttc ctttgttagg gcctagagaa 1140

tgcagggacc aaacagagat cgatggctac actgtaccta ttaaagctag agttatggtt 1200

aatgcttggg cgataggaag agatcctgaa agttgggaag atcctgaaag tttcaaaccg 1260

gagcgatttg aaaatacttc tgttgatctt acaggaaatc actatcagtt cattcctttc 1320

ggttcaggaa gaagaatgtg tccaggaatg tcgtttggtt tagttaacac agggcatcct 1380

ttagcccagt tgctctattg ctttgactgg aaactccctg acaaggttaa tgcaaatgat 1440
tttcgcacta ctgaaacaag tagagttttt gcagcaagca aagatgacct ctacttgatt 1500
cccacaaatc acagggagca agaatagctt aatttaatgg agttcttgga agaattaaag 1560
aagaagggct atataggtga gatttttgt atggttgca 1599

<210> 160
<211> 504
<212> PRT
<213> Nicotiana tabacum

<400> 160

Met Glu Leu Gln Ser Ser Pro Phe Asn Leu Ile Ser Leu Phe Leu Phe
1               5                   10                  15

Phe Ser Phe Leu Phe Ile Leu Val Lys Lys Trp Asn Ala Lys Ile Pro
            20                  25                  30

Lys Leu Pro Pro Gly Pro Trp Arg Leu Pro Phe Ile Gly Ser Leu His
        35                  40                  45

His Leu Lys Gly Lys Leu Pro His His Asn Leu Arg Asp Leu Ala Arg
    50                  55                  60

Lys Tyr Gly Pro Leu Met Tyr Leu Gln Leu Gly Glu Ile Pro Val Val
65                  70                  75                  80

Val Ile Ser Ser Pro Arg Val Ala Lys Ala Val Leu Lys Thr His Asp
                85                  90                  95

Leu Ala Phe Ala Thr Arg Pro Arg Phe Met Ser Ser Asp Ile Val Phe
            100                 105                 110

Tyr Lys Ser Arg Asp Ile Ser Phe Ala Pro Phe Gly Asp Tyr Trp Arg
        115                 120                 125

Gln Met Arg Lys Ile Leu Thr Gln Glu Leu Leu Ser Asn Lys Met Leu
    130                 135                 140

Lys Ser Tyr Ser Leu Ile Arg Lys Asp Glu Leu Ser Lys Leu Leu Ser
145                 150                 155                 160

Ser Ile Arg Leu Glu Thr Gly Ser Ala Val Asn Ile Asn Glu Lys Leu
                165                 170                 175

Leu Trp Phe Thr Ser Cys Met Thr Cys Arg Leu Ala Phe Gly Lys Ile
            180                 185                 190

Cys Asn Asp Arg Asp Glu Leu Ile Met Leu Ile Arg Glu Ile Leu Thr
        195                 200                 205

Leu Ser Gly Gly Phe Asp Val Gly Asp Leu Phe Pro Ser Trp Lys Leu
    210                 215                 220

Leu His Asn Met Ser Asn Met Lys Ala Arg Leu Thr Asn Val His His
225                 230                 235                 240

Lys Tyr Asp Leu Val Met Glu Asn Ile Ile Asn Glu His Gln Glu Asn
                245                 250                 255

His Ala Ala Gly Ile Lys Gly Asn Asn Glu Phe Gly Gly Glu Asp Met
            260                 265                 270

Ile Asp Ala Leu Leu Arg Ala Lys Glu Asn Asn Glu Leu Gln Phe Pro
        275                 280                 285

Ile Glu Asn Asp Asn Met Lys Ala Val Ile Leu Asp Leu Phe Ile Ala
    290                 295                 300

Gly Thr Glu Thr Ser Tyr Thr Ala Ile Ile Trp Ala Leu Ser Glu Leu
305                 310                 315                 320

Met Lys His Pro Ser Val Met Ala Lys Ala Gln Ala Glu Val Arg Lys
                325                 330                 335

Val Phe Lys Glu Asn Glu Asn Phe Asp Glu Asn Asp Leu Asp Lys Leu
            340                 345                 350

Pro Tyr Leu Lys Ser Val Ile Lys Glu Thr Leu Arg Met His Pro Pro
        355                 360                 365

Val Pro Leu Leu Gly Pro Arg Glu Cys Arg Asp Gln Thr Glu Ile Asp
    370                 375                 380

Gly Tyr Thr Val Pro Ile Lys Ala Arg Val Met Val Asn Ala Trp Ala
385                 390                 395                 400

Ile Gly Arg Asp Pro Glu Ser Trp Glu Asp Pro Glu Ser Phe Lys Pro
                405                 410                 415

Glu Arg Phe Glu Asn Thr Ser Val Asp Leu Thr Gly Asn His Tyr Gln
            420                 425                 430

Phe Ile Pro Phe Gly Ser Gly Arg Arg Met Cys Pro Gly Met Ser Phe
        435                 440                 445

Gly Leu Val Asn Thr Gly His Pro Leu Ala Gln Leu Leu Tyr Cys Phe
    450                 455                 460

Asp Trp Lys Leu Pro Asp Lys Val Asn Ala Asn Asp Phe Arg Thr Thr
465                 470                 475                 480

Glu Thr Ser Arg Val Phe Ala Ala Ser Lys Asp Asp Leu Tyr Leu Ile
                485                 490                 495

Pro Thr Asn His Arg Glu Gln Glu
            500

<210> 161
<211> 1564
<212> DNA
<213> Nicotiana tabacum

<400> 161
ctttctttct tgtaccgaga tggagtttca acacttggtt tcgttcttgc tattcatctc 60

cttcatcttt cttctaattc aaaaatggag gaaatcgaaa aagctgccac ctggtccgtg 120

gaggctacct attattggaa gtgtgcatca cttgacaagt ggagtaccac atcgagttct 180

cagaaattta tcacaaaaat ttggcccgat catgtacttg cagctcgggg aagttcccac 240

agtagttgta tcctccccac acatggccaa acaaatttta aaaactcatg acctcgcttt 300

tgcatctagg ccagaaatca tgatgggaaa aattatttgc tacgattgta aggacattgc 360

cttttccccg tatggtgatt attggagaca tatgcgtaaa ttgagcacct tggaactact 420

tagtgccaag atggtcaagt ccttcagtcc aattcgtcaa gatgagctct caagtctcct 480

atcatccatt gaatcaatgg gaaatttgcc aatcaactta gtagaaaaac ttttatggtt 540

tatgaatgcc gcgacatgta ggtcagcatt tgggaaagtg tgtaaagatc aaaaagagtt 600

gataacattg attcaacgag cagaatcatt atctggtgga ttcgagctgg ctgatttgtt 660

cccttcgaag aagtttctac atggtattag tgggatgcga tctaaactaa tggaagctcg 720

taacaagata gacgcagtct tggacaacat tatcaatgtg cacagagaga atcgggcaaa 780

tggaaatagt tgtaatggtg agtctggaac tgtagatttc atcgatgttt ttctaagggt 840

catggagagt ggcgaattac catttccgat agaaaatgac aacatcaaag cagttattct 900

tgacatgttc gtagcaggat ctgacacatc atcttcaacc gttatttggg cattaacaga 960

aatgatgaag aatccaaaag tcatggctaa agcacaagct gaagtgagag aagcttttaa 1020

aggaaagaaa gcatgtgatg aggatactga tcttgaaaag cttcattacc taaatttagt 1080

gatcaaagag acactccgat tacaccctcc aactcctcta cttgtcccgc gagaatgcag 1140

ggaggaaaca gagatagaag gattcactat accattgaaa agcaaagtct tggttaacgt 1200

atgggcaatt ggaagagatc ccgagaattg gaaaaatcct gaatgtttta taccagagag 1260

attcgaaaat agttctattg agtttactgg aaatcatttt caacttcttc cgtttggcgc 1320

tggaagacga atttgtccag gaatgcaatt tggtttggct cttgttactc tgccattggc 1380

tcatttgctt cacaattttg attggaaact tcccgaagga attaatgcaa gggatttgga 1440

catgacagag gcaaatggga tatctgctag aagagaaaaa gatctttact tgattgctac 1500

tccttatgta tcacctcttg attaactctg aaattttgct ttaatgctgc ttgcttgctt 1560

cact 1564

<210>  162
<211>  501
<212>  PRT
<213>  Nicotiana tabacum

<400>  162

Met Glu Phe Gln His Leu Val Ser Phe Leu Leu Phe Ile Ser Phe Ile
1               5                   10                  15

Phe Leu Leu Ile Gln Lys Trp Arg Lys Ser Lys Lys Leu Pro Pro Gly
            20                  25                  30

Pro Trp Arg Leu Pro Ile Ile Gly Ser Val His His Leu Thr Ser Gly
        35                  40                  45

Val Pro His Arg Val Leu Arg Asn Leu Ser Gln Lys Phe Gly Pro Ile
    50                  55                  60

Met Tyr Leu Gln Leu Gly Glu Val Pro Thr Val Val Val Ser Ser Pro
65                  70                  75                  80

His Met Ala Lys Gln Ile Leu Lys Thr His Asp Leu Ala Phe Ala Ser
                85                  90                  95

Arg Pro Glu Ile Met Met Gly Lys Ile Ile Cys Tyr Asp Cys Lys Asp
            100                 105                 110

Ile Ala Phe Ser Pro Tyr Gly Asp Tyr Trp Arg His Met Arg Lys Leu
        115                 120                 125

Ser Thr Leu Glu Leu Leu Ser Ala Lys Met Val Lys Ser Phe Ser Pro
    130                 135                 140

Ile Arg Gln Asp Glu Leu Ser Ser Leu Leu Ser Ser Ile Glu Ser Met
145                 150                 155                 160

Gly Asn Leu Pro Ile Asn Leu Val Glu Lys Leu Leu Trp Phe Met Asn
                165                 170                 175

Ala Ala Thr Cys Arg Ser Ala Phe Gly Lys Val Cys Lys Asp Gln Lys
            180                 185                 190

Glu Leu Ile Thr Leu Ile Gln Arg Ala Glu Ser Leu Ser Gly Gly Phe
        195                 200                 205

Glu Leu Ala Asp Leu Phe Pro Ser Lys Lys Phe Leu His Gly Ile Ser
    210                 215                 220

Gly Met Arg Ser Lys Leu Met Glu Ala Arg Asn Lys Ile Asp Ala Val
225                 230                 235                 240

Leu Asp Asn Ile Ile Asn Val His Arg Glu Asn Arg Ala Asn Gly Asn
                245                 250                 255

Ser Cys Asn Gly Glu Ser Gly Thr Val Asp Phe Ile Asp Val Phe Leu
            260                 265                 270

Arg Val Met Glu Ser Gly Glu Leu Pro Phe Pro Ile Glu Asn Asp Asn
        275                 280                 285

Ile Lys Ala Val Ile Leu Asp Met Phe Val Ala Gly Ser Asp Thr Ser
    290                 295                 300

Ser Ser Thr Val Ile Trp Ala Leu Thr Glu Met Met Lys Asn Pro Lys
305                 310                 315                 320

Val Met Ala Lys Ala Gln Ala Glu Val Arg Glu Ala Phe Lys Gly Lys
                325                 330                 335

Lys Ala Cys Asp Glu Asp Thr Asp Leu Glu Lys Leu His Tyr Leu Asn
            340                 345                 350

Leu Val Ile Lys Glu Thr Leu Arg Leu His Pro Pro Thr Pro Leu Leu
        355                 360                 365

Val Pro Arg Glu Cys Arg Glu Glu Thr Glu Ile Glu Gly Phe Thr Ile
    370                 375                 380

Pro Leu Lys Ser Lys Val Leu Val Asn Val Trp Ala Ile Gly Arg Asp
385                 390                 395                 400

Pro Glu Asn Trp Lys Asn Pro Glu Cys Phe Ile Pro Glu Arg Phe Glu
                405                 410                 415

Asn Ser Ser Ile Glu Phe Thr Gly Asn His Phe Gln Leu Leu Pro Phe
            420                 425                 430

Gly Ala Gly Arg Arg Ile Cys Pro Gly Met Gln Phe Gly Leu Ala Leu
        435                 440                 445

Val Thr Leu Pro Leu Ala His Leu Leu His Asn Phe Asp Trp Lys Leu
    450                 455                 460

Pro Glu Gly Ile Asn Ala Arg Asp Leu Asp Met Thr Glu Ala Asn Gly
465                 470                 475                 480

Ile Ser Ala Arg Arg Glu Lys Asp Leu Tyr Leu Ile Ala Thr Pro Tyr
                485                 490                 495

Val Ser Pro Leu Asp
            500

<210> 163
<211> 1560
<212> DNA
<213> Nicotiana tabacum

<400> 163
ctttcttgta ccgagatgga gtttcaacac ttggtttcgt tcttgctatt catctccttc 60

atctttcttc taattcaaaa atggaggaaa tcgaaaaagc tgccacctgg tccgtggagg 120

ctacctatta ttggaagtgt gcatcacttg acaagtggag taccacatcg agttctcaga 180

aatttatcac aaaaatttgg cccgatcatg tacttgcagc tcggggaagt tcccacagta 240

gttgtatcct ccccacacat ggccaaacaa attttaaaaa ctcatgacct cgcttttgca 300

tctaggccag aaatcatgat gggaaaaatt atttgctacg attgtaagga cattgccttt 360

tccccgtatg gtgattattg gagacatatg cgtaaattga gcaccttgga actacttagt 420

gccaagatgg tcaagtcctt cagtccaatt cgtcaagatg agctctcaag tctcctatca 480

tccattgaat caatgggaaa tttgccaatc aacttagtag aaaaactttt atggtttatg 540

aatgccgcga catgtaggtc agcatttggg aaagtgtgta aagatcaaaa agagttgata 600

acattgattc aacgagcaga atcattatct ggtggattcg agctggctga tttgttccct 660

tcgaagaagt ttctacatgg tattagtggg atgcgatcta aactaatgga agctcgtaac 720

aagatagacg cagtcttgga caacattatc aatgtgcaca gagagaatcg ggcaaatgga 780

aatagttgta atggtgagtc tggaactgta gatttcatcg atgtttttct aagggtcatg 840

gagagtggcg aattaccatt tccgatagaa aatgacaaca tcaaagcagt tattcttgac 900

atgttcgtag caggatctga cacatcatct tcaaccgtta tttgggcatt aacagaaacg 960

atgaagaatc caaaagtcat ggctaaagca caagctgaag tgagagaagc ttttaaagga 1020

aagaaagcat gtgatgagga tactgatctt gaaaagcatc attacctaaa tttagtgatc 1080

aaagagacac tccgattaca ccctccaact cctctacttg tcccgcgaga atgcagggag 1140

gaaacagaga tagaaggatt cactatacca ttgaaaagca aagtcttggt taacgtatgg 1200

gcaattggaa gagatcccga gaattggaaa aatcctgaat gttttatacc agagagattc 1260

gaaaatagtt ctattgagtt tactggaaat cattttcaac ttcttccgtt tggcgctgga 1320

agacgaattt gtccaggaat gcaatttggt ttggctcttg ttactctgcc attggctcat 1380

ttgcttcaca attttgattg gaaacttccc gaaggaatta atgcaaggga tttggacatg 1440

acagaggcaa atgggatatc tgctagaaga gaaaaagatc tttacttgat tgctactcct 1500

tatgtatcac ctcttgatta actctgaaat tttgctttaa tgctgcttgc ttgcttcact 1560

<210> 164
<211> 501
<212> PRT
<213> Nicotiana tabacum

<400> 164

Met Glu Phe Gln His Leu Val Ser Phe Leu Leu Phe Ile Ser Phe Ile
1               5                   10                  15

Phe Leu Leu Ile Gln Lys Trp Arg Lys Ser Lys Lys Leu Pro Pro Gly
            20                  25                  30

Pro Trp Arg Leu Pro Ile Ile Gly Ser Val His His Leu Thr Ser Gly
        35                  40                  45

Val Pro His Arg Val Leu Arg Asn Leu Ser Gln Lys Phe Gly Pro Ile
    50                  55                  60

Met Tyr Leu Gln Leu Gly Glu Val Pro Thr Val Val Val Ser Ser Pro
65                  70                  75                  80

His Met Ala Lys Gln Ile Leu Lys Thr His Asp Leu Ala Phe Ala Ser
                85                  90                  95

Arg Pro Glu Ile Met Met Gly Lys Ile Ile Cys Tyr Asp Cys Lys Asp
            100                 105                 110

Ile Ala Phe Ser Pro Tyr Gly Asp Tyr Trp Arg His Met Arg Lys Leu
        115                 120                 125

Ser Thr Leu Glu Leu Leu Ser Ala Lys Met Val Lys Ser Phe Ser Pro
    130                 135                 140

Ile Arg Gln Asp Glu Leu Ser Ser Leu Leu Ser Ser Ile Glu Ser Met
145                 150                 155                 160

Gly Asn Leu Pro Ile Asn Leu Val Glu Lys Leu Leu Trp Phe Met Asn
                165                 170                 175

Ala Ala Thr Cys Arg Ser Ala Phe Gly Lys Val Cys Lys Asp Gln Lys
            180                 185                 190

Glu Leu Ile Thr Leu Ile Gln Arg Ala Glu Ser Leu Ser Gly Gly Phe
        195                 200                 205

Glu Leu Ala Asp Leu Phe Pro Ser Lys Lys Phe Leu His Gly Ile Ser
    210                 215                 220

Gly Met Arg Ser Lys Leu Met Glu Ala Arg Asn Lys Ile Asp Ala Val
225                 230                 235                 240

Leu Asp Asn Ile Ile Asn Val His Arg Glu Asn Arg Ala Asn Gly Asn
                245                 250                 255

Ser Cys Asn Gly Glu Ser Gly Thr Val Asp Phe Ile Asp Val Phe Leu
            260                 265                 270

Arg Val Met Glu Ser Gly Glu Leu Pro Phe Pro Ile Glu Asn Asp Asn
        275                 280                 285

Ile Lys Ala Val Ile Leu Asp Met Phe Val Ala Gly Ser Asp Thr Ser
    290                 295                 300

Ser Ser Thr Val Ile Trp Ala Leu Thr Glu Thr Met Lys Asn Pro Lys
305                 310                 315                 320

Val Met Ala Lys Ala Gln Ala Glu Val Arg Glu Ala Phe Lys Gly Lys
                325                 330                 335

Lys Ala Cys Asp Glu Asp Thr Asp Leu Glu Lys His His Tyr Leu Asn
            340                 345                 350

Leu Val Ile Lys Glu Thr Leu Arg Leu His Pro Pro Thr Pro Leu Leu
        355                 360                 365

Val Pro Arg Glu Cys Arg Glu Glu Thr Glu Ile Glu Gly Phe Thr Ile
    370                 375                 380

Pro Leu Lys Ser Lys Val Leu Val Asn Val Trp Ala Ile Gly Arg Asp
385                 390                 395                 400

Pro Glu Asn Trp Lys Asn Pro Glu Cys Phe Ile Pro Glu Arg Phe Glu
                405                 410                 415

Asn Ser Ser Ile Glu Phe Thr Gly Asn His Phe Gln Leu Leu Pro Phe
            420                 425                 430

Gly Ala Gly Arg Arg Ile Cys Pro Gly Met Gln Phe Gly Leu Ala Leu
        435                 440                 445

Val Thr Leu Pro Leu Ala His Leu Leu His Asn Phe Asp Trp Lys Leu
    450                 455                 460

Pro Glu Gly Ile Asn Ala Arg Asp Leu Asp Met Thr Glu Ala Asn Gly
465                 470                 475                 480

Ile Ser Ala Arg Arg Glu Lys Asp Leu Tyr Leu Ile Ala Thr Pro Tyr
                485                 490                 495

Val Ser Pro Leu Asp
            500

<210>  165
<211>  1552
<212>  DNA
<213>  Nicotiana tabacum

<400>  165
caaaaacaaa attccaatgg ttaacatgtt cactccaatt atatacgctc ctctcctttt      60

agctttttac attatcacaa aacatttctt acgcaaactc agaaataatc caccagctcc      120

atttcttact ttccccttta ttggccatct ttatctcttc aaaaaaccac ttcaacgtac 180
cttagccaaa atctccgaac gttatggctc tgttcttcta ctcgaattcg gttcacgaaa 240
agtacttttg gtttcttcac catctgcagc tgaagaatgc ttaacaaaaa acgatattat 300
tttcgcgaat cgtcctcttt tgatggctgg aaaacatctt ggatataatt ttacatcttt 360
ggcttggagt tcgtacggag atcattggag aaatctgcga aggattactt cagttgagat 420
gttttcgact catcgtcttc aaatgctaca tgggattcgt attgatgaag tgaaatctat 480
ggttaagagg ctcaattcct ctgccatagc tgaaaaatct gtggatatga agtctatgtt 540
ttttgagctg atgctcaatg ttatgatgag gacaattgct ggaaaaagat attacggtga 600
gaatgtggag gacattgagg aagctacgag attcaaaggt ttggtgcaag agactttcag 660
gattggcggg gcgacgaata ttggcgactt tttgccggcg ttgaagttat tggtgaggaa 720
attggagaaa agtttaattg tgttgcaaga gaacagagat gagtttatgc aggaattaat 780
taaagattgc agaaaaagaa tggagaaaga aggtactgtt actgattcag aaattgaagg 840
gaacaagaaa tgtttaattg aagttttgtt aacactacaa gaaaatgaac cggaatacta 900
caaagatgaa atcatcagaa gccttatgct tgttctatta tcagctggta cagatacttc 960
agttgggaca atggaatggg ctttatcatt aatgttaaac caccctgaaa ctctgaagaa 1020
agcacaagct gaaattgatg aacatatagg acatgaacgt ttagtggacg agtcggacat 1080
caacaaccta ccttacctac gttgtataat caacgagaca ttccgaatgt accctgcagg 1140
accactacta gtcccacacg agtcgtcaga ggaaaccacc gtaggaggct accgtgtacc 1200
cggaggaacc atgttacttg tgaatttgtg ggcaattcac aatgatccaa agctatggga 1260
tgaaccaaga aagtttaaac cagaaagatt tcaaggacta gatggtgtta gagatggtta 1320
caaaatgatg ccttttggtt ctggacgaag gagttgtcct ggagaaggat tggctgttcg 1380
aatggttgcc ttgtcattgg gatgtattat tcaatgtttt gattggcaac gaatcggcga 1440
agaattggtt gatatgactg aaggaactgg acttactttg cctaaagctc aacctttggt 1500
ggccaagtgt agcccacgac ctaaaatggc taatcttctc tctcagattt ga 1552

<210> 166
<211> 511
<212> PRT
<213> Nicotiana tabacum

<400> 166

Met Val Asn Met Phe Thr Pro Ile Ile Tyr Ala Pro Leu Leu Leu Ala
1               5                   10                  15

Phe Tyr Ile Ile Thr Lys His Phe Leu Arg Lys Leu Arg Asn Asn Pro
            20                  25                  30

Pro Ala Pro Phe Leu Thr Phe Pro Phe Ile Gly His Leu Tyr Leu Phe
        35                  40                  45

Lys Lys Pro Leu Gln Arg Thr Leu Ala Lys Ile Ser Glu Arg Tyr Gly
    50                  55                  60

Ser Val Leu Leu Leu Glu Phe Gly Ser Arg Lys Val Leu Leu Val Ser
65                  70                  75                  80

Ser Pro Ser Ala Ala Glu Glu Cys Leu Thr Lys Asn Asp Ile Ile Phe
                85                  90                  95

Ala Asn Arg Pro Leu Leu Met Ala Gly Lys His Leu Gly Tyr Asn Phe
            100                 105                 110

Thr Ser Leu Ala Trp Ser Ser Tyr Gly Asp His Trp Arg Asn Leu Arg
        115                 120                 125

Arg Ile Thr Ser Val Glu Met Phe Ser Thr His Arg Leu Gln Met Leu
    130                 135                 140

His Gly Ile Arg Ile Asp Glu Val Lys Ser Met Val Lys Arg Leu Asn
145                 150                 155                 160

Ser Ser Ala Ile Ala Glu Lys Ser Val Asp Met Lys Ser Met Phe Phe
                165                 170                 175

Glu Leu Met Leu Asn Val Met Met Arg Thr Ile Ala Gly Lys Arg Tyr
            180                 185                 190

Tyr Gly Glu Asn Val Glu Asp Ile Glu Glu Ala Thr Arg Phe Lys Gly
        195                 200                 205

Leu Val Gln Glu Thr Phe Arg Ile Gly Gly Ala Thr Asn Ile Gly Asp
    210                 215                 220

Phe Leu Pro Ala Leu Lys Leu Leu Val Arg Lys Leu Glu Lys Ser Leu
225                 230                 235                 240

Ile Val Leu Gln Glu Asn Arg Asp Glu Phe Met Gln Glu Leu Ile Lys
                245                 250                 255

Asp Cys Arg Lys Arg Met Glu Lys Glu Gly Thr Val Thr Asp Ser Glu
            260                 265                 270

Ile Glu Gly Asn Lys Lys Cys Leu Ile Glu Val Leu Leu Thr Leu Gln
        275                 280                 285

Glu Asn Glu Pro Glu Tyr Tyr Lys Asp Glu Ile Ile Arg Ser Leu Met
    290                 295                 300

Leu Val Leu Leu Ser Ala Gly Thr Asp Thr Ser Val Gly Thr Met Glu
305                 310                 315                 320

Trp Ala Leu Ser Leu Met Leu Asn His Pro Glu Thr Leu Lys Lys Ala
                325                 330                 335

Gln Ala Glu Ile Asp Glu His Ile Gly His Glu Arg Leu Val Asp Glu
            340                 345                 350

Ser Asp Ile Asn Asn Leu Pro Tyr Leu Arg Cys Ile Ile Asn Glu Thr
        355                 360                 365

Phe Arg Met Tyr Pro Ala Gly Pro Leu Leu Val Pro His Glu Ser Ser
    370                 375                 380

Glu Glu Thr Thr Val Gly Gly Tyr Arg Val Pro Gly Gly Thr Met Leu
385                 390                 395                 400

Leu Val Asn Leu Trp Ala Ile His Asn Asp Pro Lys Leu Trp Asp Glu
                405                 410                 415

Pro Arg Lys Phe Lys Pro Glu Arg Phe Gln Gly Leu Asp Gly Val Arg
            420                 425                 430

Asp Gly Tyr Lys Met Met Pro Phe Gly Ser Gly Arg Arg Ser Cys Pro
        435                 440                 445

Gly Glu Gly Leu Ala Val Arg Met Val Ala Leu Ser Leu Gly Cys Ile
    450                 455                 460

Ile Gln Cys Phe Asp Trp Gln Arg Ile Gly Glu Glu Leu Val Asp Met
465                 470                 475                 480

Thr Glu Gly Thr Gly Leu Thr Leu Pro Lys Ala Gln Pro Leu Val Ala
                485                 490                 495

Lys Cys Ser Pro Arg Pro Lys Met Ala Asn Leu Leu Ser Gln Ile
            500                 505                 510

<210> 167
<211> 1675
<212> DNA
<213> Nicotiana tabacum

<400> 167
caaaaacaaa attccaatgg ttaacatgtt cactccaatt atatacgctc ctctcctttt 60

agctttttac attatcacaa aacatttctt acgcaaactc agaaataacc caccagctcc 120

atttcttact ttccccttta ttggccatct ttatctcttc aaaaaaccac ttcaacgtac 180

cttagccaaa atctccgaac gttatggctc tgttcttcta ctcgaattcg gttcacgaaa 240

agtacttttg gtttcttcac catctgcagc tgaagaatgc ttaacaaaaa acgatattat 300

tttcgcgaat cgtcctcttt tgatggctgg aaaacatctt ggatataatt ttacttcttt 360

ggcttggagt tcgtacggag atcactggag aaatcttcgt aggattactt cagttgagat 420

gttttcgact catcgtcttc aaatgctaca tggaattcgt attgatgaag tgaaatctat 480

ggttaagagg ctcaattcct ctgccatagc tgaaaaatct gtggatatga agtctatgtt 540

ttttgagctg atgctcaatg ttatgatgag gacaattgct ggaaaaagat attacggtga 600

gaatgtggag gacattgagg aagctacgag attcaaaggt ttggtgcaag agactttcag 660

gattggcggg gcgacgaata ttggcgactt tttgccggcg ttgaagttat tggtgaggaa 720

attggagaaa agtttaattg tgttgcaaga gaacagagat gagtttatgc aggaattaat 780

taaagattgc agaaaaagaa tggagaaaga aggtactgtt actgattcag aaattgaagg 840

gaacaagaaa tgtttaattg aagttttgtt aacactacaa gaaaatgaac cggaatacta 900

caaagatgaa atcatcagaa gccttatgct tgttctatta tcagctggta cagatacttc 960

agttgggaca atggaatggg ctttatcatt aatgttaaac caccctgaaa ctctgaagaa 1020

agcacaagct gaaattgatg aacatatagg acatgaacgt ttagtggacg agtcggacat 1080

caacaaccta ccttacctac gttgtataat caacgagaca ttccgaatgt accctgcagg 1140

accactacta gtcccacacg agtcgtcaga ggaaaccacc gtaggaggct accgtgtacc 1200

cggaggaacc atgttacttg tgaatttgtg ggctattcac aatgatccaa agctatggga 1260

tgaaccaaga aagtttaagc cagaaagatt tgaaggacta gaaggtgtta gagacggtta 1320

caaaatgatg ccttttggtt ctggacgaag gagttgtcct ggagaaggat tggctattcg 1380

aatggttgca ttgtcattgg gatgtattat tcaatgcttt gattggcaac gacttgggga 1440

aggattggtt gataagactg aaggaactgg acttactttg cctaaagctc aacctttagt 1500

ggccaagtgt agcccacgac ctataatggc taatcttctt tctcagattt gaacataatt 1560

ggtttctacc aaacatcccc aaactagaat attattattg gttacatata caatgtaatc 1620

aattttgaac catattatat ctcaatgtat tcctttttaa aaaaaaaaaa aaaaa 1675

<210> 168
<211> 511
<212> PRT
<213> Nicotiana tabacum

<400> 168

Met Val Asn Met Phe Thr Pro Ile Ile Tyr Ala Pro Leu Leu Leu Ala
1               5                   10                  15

Phe Tyr Ile Ile Thr Lys His Phe Leu Arg Lys Leu Arg Asn Asn Pro
            20                  25                  30

Pro Ala Pro Phe Leu Thr Phe Pro Phe Ile Gly His Leu Tyr Leu Phe
        35                  40                  45

Lys Lys Pro Leu Gln Arg Thr Leu Ala Lys Ile Ser Glu Arg Tyr Gly
    50                  55                  60

Ser Val Leu Leu Leu Glu Phe Gly Ser Arg Lys Val Leu Leu Val Ser
65                  70                  75                  80

Ser Pro Ser Ala Ala Glu Glu Cys Leu Thr Lys Asn Asp Ile Ile Phe
                85                  90                  95

Ala Asn Arg Pro Leu Leu Met Ala Gly Lys His Leu Gly Tyr Asn Phe
            100                 105                 110

Thr Ser Leu Ala Trp Ser Ser Tyr Gly Asp His Trp Arg Asn Leu Arg
        115                 120                 125

Arg Ile Thr Ser Val Glu Met Phe Ser Thr His Arg Leu Gln Met Leu
    130                 135                 140

His Gly Ile Arg Ile Asp Glu Val Lys Ser Met Val Lys Arg Leu Asn
145                 150                 155                 160

Ser Ser Ala Ile Ala Glu Lys Ser Val Asp Met Lys Ser Met Phe Phe
                165                 170                 175

Glu Leu Met Leu Asn Val Met Met Arg Thr Ile Ala Gly Lys Arg Tyr
            180                 185                 190

Tyr Gly Glu Asn Val Glu Asp Ile Glu Glu Ala Thr Arg Phe Lys Gly
        195                 200                 205

Leu Val Gln Glu Thr Phe Arg Ile Gly Gly Ala Thr Asn Ile Gly Asp
    210                 215                 220

Phe Leu Pro Ala Leu Lys Leu Leu Val Arg Lys Leu Glu Lys Ser Leu
225                 230                 235                 240

Ile Val Leu Gln Glu Asn Arg Asp Glu Phe Met Gln Glu Leu Ile Lys
                245                 250                 255

Asp Cys Arg Lys Arg Met Glu Lys Glu Gly Thr Val Thr Asp Ser Glu
            260                 265                 270

Ile Glu Gly Asn Lys Lys Cys Leu Ile Glu Val Leu Leu Thr Leu Gln
        275                 280                 285

Glu Asn Glu Pro Glu Tyr Tyr Lys Asp Glu Ile Ile Arg Ser Leu Met
    290                 295                 300

Leu Val Leu Leu Ser Ala Gly Thr Asp Thr Ser Val Gly Thr Met Glu
305                 310                 315                 320

Trp Ala Leu Ser Leu Met Leu Asn His Pro Glu Thr Leu Lys Lys Ala
                325                 330                 335

Gln Ala Glu Ile Asp Glu His Ile Gly His Glu Arg Leu Val Asp Glu
            340                 345                 350

Ser Asp Ile Asn Asn Leu Pro Tyr Leu Arg Cys Ile Ile Asn Glu Thr
        355                 360                 365

Phe Arg Met Tyr Pro Ala Gly Pro Leu Leu Val Pro His Glu Ser Ser
    370                 375                 380

Glu Glu Thr Thr Val Gly Gly Tyr Arg Val Pro Gly Gly Thr Met Leu
385                 390                 395                 400

Leu Val Asn Leu Trp Ala Ile His Asn Asp Pro Lys Leu Trp Asp Glu
                405                 410                 415

Pro Arg Lys Phe Lys Pro Glu Arg Phe Glu Gly Leu Glu Gly Val Arg
            420                 425                 430

Asp Gly Tyr Lys Met Met Pro Phe Gly Ser Gly Arg Arg Ser Cys Pro
        435                 440                 445

Gly Glu Gly Leu Ala Ile Arg Met Val Ala Leu Ser Leu Gly Cys Ile
    450                 455                 460

Ile Gln Cys Phe Asp Trp Gln Arg Leu Gly Glu Gly Leu Val Asp Lys
465                 470                 475                 480

Thr Glu Gly Thr Gly Leu Thr Leu Pro Lys Ala Gln Pro Leu Val Ala
                485                 490                 495

Lys Cys Ser Pro Arg Pro Ile Met Ala Asn Leu Leu Ser Gln Ile
            500                 505                 510

<210>  169
<211>  1755
<212>  DNA
<213>  Nicotiana tabacum

<400>  169
caacacgctt actatctcct aaatctccac tcaaaaacaa agaagagaaa gatttaaaac      60

taataattat gaaagagatg gtgcaaaaca atatgagcac ttctcttctt gaaactttac     120

aagctacgcc catgatattc tacttcatcg tccctctctt ctgcttattc cttctctcca     180

aatctcgccg taaacgtttg cctccaggtc caactggctg gcctctcatt ggtaacatga     240

tgatgatgga ccagttaact caccgtggcc ttgccaaact agcccaaaaa tatggtggtg     300

tttttcacct taaaatgggt tatgttcaca aaattgtagt ctctggtcca gacgaagctc     360

gccaagtatt acaggaacac gacatcatat tttcgaaccg tccagcgacc gtagccataa     420

gttacctaac atatgacagg gcagacatgg cttttgctga ctatggactc ttctggcggc     480

agatgagaaa actatgtgta atgaaactct tcagccgcaa acgagctgag tcatgggact     540

cagttcgaga cgaagcggat tccatggtta gaattgtaac aaccaacaca ggcacagctg     600

ttaacttagg tgaacttgtt ttcagtctca ctcgtaatat tatctacaga gctgcttttg 660

gaacttgttc tgaagatgga caaggcgagt tcattaaaat tatgcaagag ttttcgaagc 720

tatttggtgc tttcaatata gctgatttta ttccatggct agggtgggtt ggtaagcaga 780

gtctaaatat tagacttgct aaggctagag cgtcgcttga tgggttcatt gattcgatta 840

ttgatgacca tattattaga aagaaagctt atgttaatgg caaaaatgat ggaggtgatc 900

gagaaactga tatggtggat gagcttttag ctttttacag tgaggaagca aaagtaactg 960

agtccgaaga tttgcagaat gctatcagac ttactaagga taatatcaaa gctatcatca 1020

tggatgtaat gtttggaggg acagaaacag tggcttctgc aatagaatgg gccatggcag 1080

agcttatgag gagtcctgaa gatcttaaaa aggtacaaca agagctggct aacgttgttg 1140

gactcaacag aaaagttgaa gaatctgact ttgaaaaatt aacatactta agatgttgtc 1200

taaaagaaac tctacgactt caccctccaa tccctctcct cctccatgag accgccgagg 1260

aatccaccgt ctccggctac catattccgg caaagtcaca tgttattata aattcatttg 1320

ccattgggcg tgacaaaaat tcatgggaag atcctgaaac ttataaacca tctaggtttc 1380

tcaaagaagg tgtaccagat tttaaaggag gtaattttga gtttatacca tttgggtcgg 1440

gtcggcggtc ttgccccggt atgcaacttg ggctttatgc attggaaatg gctgtggccc 1500

atcttcttca ttgttttact tgggaattgc cagatggtat gaaaccaagt gagcttaaaa 1560

tggatgatat ttttggactc actgctccaa gagctaatcg actcgtggct gtgcctactc 1620

cacgtttgtt gtgtcccctt tattaattga agaaaaaagg tggggctttt acttgcatca 1680

aagagtggtg cttgtgattt ttccaccttt tggttaaata tacgaattat tatgatatac 1740

gaattcttgg gcaca 1755

<210> 170
<211> 525
<212> PRT
<213> Nicotiana tabacum

<400> 170

Met Lys Glu Met Val Gln Asn Asn Met Ser Thr Ser Leu Leu Glu Thr
1               5                   10                  15

Leu Gln Ala Thr Pro Met Ile Phe Tyr Phe Ile Val Pro Leu Phe Cys
            20                  25                  30

Leu Phe Leu Leu Ser Lys Ser Arg Arg Lys Arg Leu Pro Pro Gly Pro
        35                  40                  45

Thr Gly Trp Pro Leu Ile Gly Asn Met Met Met Met Asp Gln Leu Thr
    50                  55                  60

His Arg Gly Leu Ala Lys Leu Ala Gln Lys Tyr Gly Gly Val Phe His
65                  70                  75                  80

Leu Lys Met Gly Tyr Val His Lys Ile Val Val Ser Gly Pro Asp Glu
                85                  90                  95

Ala Arg Gln Val Leu Gln Glu His Asp Ile Ile Phe Ser Asn Arg Pro
            100                 105                 110

Ala Thr Val Ala Ile Ser Tyr Leu Thr Tyr Asp Arg Ala Asp Met Ala
        115                 120                 125

Phe Ala Asp Tyr Gly Leu Phe Trp Arg Gln Met Arg Lys Leu Cys Val
    130                 135                 140

Met Lys Leu Phe Ser Arg Lys Arg Ala Glu Ser Trp Asp Ser Val Arg
145                 150                 155                 160

Asp Glu Ala Asp Ser Met Val Arg Ile Val Thr Thr Asn Thr Gly Thr
                165                 170                 175

Ala Val Asn Leu Gly Glu Leu Val Phe Ser Leu Thr Arg Asn Ile Ile
            180                 185                 190

Tyr Arg Ala Ala Phe Gly Thr Cys Ser Glu Asp Gly Gln Gly Glu Phe
        195                 200                 205

Ile Lys Ile Met Gln Glu Phe Ser Lys Leu Phe Gly Ala Phe Asn Ile
    210                 215                 220

Ala Asp Phe Ile Pro Trp Leu Gly Trp Val Gly Lys Gln Ser Leu Asn
225                 230                 235                 240

Ile Arg Leu Ala Lys Ala Arg Ala Ser Leu Asp Gly Phe Ile Asp Ser
                245                 250                 255

Ile Ile Asp Asp His Ile Ile Arg Lys Lys Ala Tyr Val Asn Gly Lys
            260                 265                 270

Asn Asp Gly Gly Asp Arg Glu Thr Asp Met Val Asp Glu Leu Leu Ala
        275                 280                 285

Phe Tyr Ser Glu Glu Ala Lys Val Thr Glu Ser Glu Asp Leu Gln Asn
    290                 295                 300

Ala Ile Arg Leu Thr Lys Asp Asn Ile Lys Ala Ile Ile Met Asp Val
305                 310                 315                 320

Met Phe Gly Gly Thr Glu Thr Val Ala Ser Ala Ile Glu Trp Ala Met
                325                 330                 335

Ala Glu Leu Met Arg Ser Pro Glu Asp Leu Lys Lys Val Gln Gln Glu
            340                 345                 350

Leu Ala Asn Val Val Gly Leu Asn Arg Lys Val Glu Glu Ser Asp Phe
        355                 360                 365

Glu Lys Leu Thr Tyr Leu Arg Cys Cys Leu Lys Glu Thr Leu Arg Leu
    370                 375                 380

His Pro Pro Ile Pro Leu Leu Leu His Glu Thr Ala Glu Glu Ser Thr
385                 390                 395                 400

Val Ser Gly Tyr His Ile Pro Ala Lys Ser His Val Ile Ile Asn Ser
                405                 410                 415

Phe Ala Ile Gly Arg Asp Lys Asn Ser Trp Glu Asp Pro Glu Thr Tyr
            420                 425                 430

Lys Pro Ser Arg Phe Leu Lys Glu Gly Val Pro Asp Phe Lys Gly Gly
        435                 440                 445

Asn Phe Glu Phe Ile Pro Phe Gly Ser Gly Arg Arg Ser Cys Pro Gly
    450                 455                 460

Met Gln Leu Gly Leu Tyr Ala Leu Glu Met Ala Val Ala His Leu Leu
465                 470                 475                 480

His Cys Phe Thr Trp Glu Leu Pro Asp Gly Met Lys Pro Ser Glu Leu
                485                 490                 495

Lys Met Asp Asp Ile Phe Gly Leu Thr Ala Pro Arg Ala Asn Arg Leu
            500                 505                 510

Val Ala Val Pro Thr Pro Arg Leu Leu Cys Pro Leu Tyr
        515                 520                 525

<210> 171
<211> 1684
<212> DNA
<213> Nicotiana tabacum

<400> 171
tgtgcttgtg agtgtgggag aaggccttca atatggagat accatattac agcttaaaaa 60

ttgcaatttc ttcatttgca attatctttg tactaagatg ggcatggaaa atcttgaatt 120

atgtgtggtt aaaaccaaaa gaattggaga aatacctcag acagcagggt ttcaaaggaa 180

actcttacaa attcttgttt ggggatatga aagagatgaa gaaaatgggt gaagaagcta 240

tgtctaagcc aatcaatttc tctcatgaca tgatttggcc tagagttatg ccattcatcc 300

acaaaaccat caccaattat ggtaagaatt gtattgtgtg gtttgggcca agaccagcag 360

tcctgatcac agacccggaa cttgtaaagg aggtgctaac gaagaatttc gtctatcaga 420

agccgcttgg caatccactc acaaagttgg cagcaactgg aattgcaggc tatgaaacag 480

ataaatgggc tacacataga aggcttctca atcctgcttt tcaccttgac aagttgaagc 540

atatgctacc tgcattccaa tttactgcta gtgagatgtt gagcaaattg gagaaagttg 600

tttcaccaaa cggaacagag atagatgtgt ggccatattt acaaactttg acaagtgatg 660

ccatttcaag aactgcgttt ggaagtagtt atgaagaagg aagaaagatt tttgaccttc 720

aaaaagaaca actttcacta attctagaag tttcacgcac aatatatatt ccaggatgga 780

ggtttttgcc aacgaaaagg aacaaaagga tgaagcaaat atttaatgaa gtacgagcac 840

tggtatttgg aattattaag aaaaggatga gtatgattga aaatggagaa gcacctgatg 900

atttattggg aatattattg gcatccaatt taaaagaaat ccaacaacat ggaaacaaca 960

agaaatttgg tatgagtatt gatgaggtga ttgaagagtg taaactcttc tattttgctg 1020

ggcaagagac tacttcatct ttacttgtat ggactatgat tttgttgtgc aaatatccta 1080

attggcaaga taaagctaga gaagaggttt tgcaagtgtt tgggagtagg gaagttgact 1140

atgacaagtt gaatcagcta aaaatagtaa ctatgatctt aaacgaggtc ttaaggttgt 1200

atccagcagg atatgtgatt aatcgaatgg taaacaaaga aacaaagtta gggaatttgt 1260

gtttaccagc cggcgtacag ctcgtgttac caacaatgtt gttgcaacat gatactgaaa 1320

tatggggaga tgatgcaatg gagttcaatc cagagagatt tagtgatgga atatccaaag 1380

caacaaaagg aaaacttgtg ttttttccat ttagttgggg tccaagaata tgtattgggc 1440

aaaattttgc tatgttagag gctaaaatgg caatggctat gattctgaaa acctatgcat 1500

ttgaactctc tccatcttat gctcatgctc ctcatccact actacttcaa cctcaatatg 1560

gtgctcaatt aattttgtac aagttgtaga tatggtcaat ttggaacttg ttatggaact 1620

tttatcattg taatcaacca tattgaggga acatggtttg aggttaaatc ctcgtgtgtg 1680

tgtc 1684

<210> 172
<211> 518
<212> PRT
<213> Nicotiana tabacum

<400> 172

Met Glu Ile Pro Tyr Tyr Ser Leu Lys Ile Ala Ile Ser Ser Phe Ala
1               5                   10                  15

Ile Ile Phe Val Leu Arg Trp Ala Trp Lys Ile Leu Asn Tyr Val Trp
            20                  25                  30

Leu Lys Pro Lys Glu Leu Glu Lys Tyr Leu Arg Gln Gln Gly Phe Lys
        35                  40                  45

Gly Asn Ser Tyr Lys Phe Leu Phe Gly Asp Met Lys Glu Met Lys Lys
    50                  55                  60

Met Gly Glu Glu Ala Met Ser Lys Pro Ile Asn Phe Ser His Asp Met
65                  70                  75                  80

Ile Trp Pro Arg Val Met Pro Phe Ile His Lys Thr Ile Thr Asn Tyr
                85                  90                  95

Gly Lys Asn Cys Ile Val Trp Phe Gly Pro Arg Pro Ala Val Leu Ile
            100                 105                 110

Thr Asp Pro Glu Leu Val Lys Glu Val Leu Thr Lys Asn Phe Val Tyr
        115                 120                 125

Gln Lys Pro Leu Gly Asn Pro Leu Thr Lys Leu Ala Ala Thr Gly Ile
    130                 135                 140

Ala Gly Tyr Glu Thr Asp Lys Trp Ala Thr His Arg Arg Leu Leu Asn
145                 150                 155                 160

Pro Ala Phe His Leu Asp Lys Leu Lys His Met Leu Pro Ala Phe Gln
                165                 170                 175

Phe Thr Ala Ser Glu Met Leu Ser Lys Leu Glu Lys Val Val Ser Pro
            180                 185                 190

Asn Gly Thr Glu Ile Asp Val Trp Pro Tyr Leu Gln Thr Leu Thr Ser
        195                 200                 205

Asp Ala Ile Ser Arg Thr Ala Phe Gly Ser Ser Tyr Glu Glu Gly Arg
    210                 215                 220

Lys Ile Phe Asp Leu Gln Lys Glu Gln Leu Ser Leu Ile Leu Glu Val
225                 230                 235                 240

Ser Arg Thr Ile Tyr Ile Pro Gly Trp Arg Phe Leu Pro Thr Lys Arg
                245                 250                 255

Asn Lys Arg Met Lys Gln Ile Phe Asn Glu Val Arg Ala Leu Val Phe
            260                 265                 270

Gly Ile Ile Lys Lys Arg Met Ser Met Ile Glu Asn Gly Glu Ala Pro
        275                 280                 285

Asp Asp Leu Leu Gly Ile Leu Leu Ala Ser Asn Leu Lys Glu Ile Gln
    290                 295                 300

Gln His Gly Asn Asn Lys Lys Phe Gly Met Ser Ile Asp Glu Val Ile
305                 310                 315                 320

Glu Glu Cys Lys Leu Phe Tyr Phe Ala Gly Gln Glu Thr Thr Ser Ser
                325                 330                 335

Leu Leu Val Trp Thr Met Ile Leu Leu Cys Lys Tyr Pro Asn Trp Gln
            340                 345                 350

Asp Lys Ala Arg Glu Glu Val Leu Gln Val Phe Gly Ser Arg Glu Val
        355                 360                 365

Asp Tyr Asp Lys Leu Asn Gln Leu Lys Ile Val Thr Met Ile Leu Asn
    370                 375                 380

Glu Val Leu Arg Leu Tyr Pro Ala Gly Tyr Val Ile Asn Arg Met Val
385                 390                 395                 400

Asn Lys Glu Thr Lys Leu Gly Asn Leu Cys Leu Pro Ala Gly Val Gln
                405                 410                 415

Leu Val Leu Pro Thr Met Leu Leu Gln His Asp Thr Glu Ile Trp Gly
            420                 425                 430

Asp Asp Ala Met Glu Phe Asn Pro Glu Arg Phe Ser Asp Gly Ile Ser
        435                 440                 445

Lys Ala Thr Lys Gly Lys Leu Val Phe Phe Pro Phe Ser Trp Gly Pro
    450                 455                 460

Arg Ile Cys Ile Gly Gln Asn Phe Ala Met Leu Glu Ala Lys Met Ala
465                 470                 475                 480

Met Ala Met Ile Leu Lys Thr Tyr Ala Phe Glu Leu Ser Pro Ser Tyr
                485                 490                 495

Ala His Ala Pro His Pro Leu Leu Leu Gln Pro Gln Tyr Gly Ala Gln
            500                 505                 510

Leu Ile Leu Tyr Lys Leu
        515

<210>  173
<211>  1697
<212>  DNA
<213>  Nicotiana tabacum

<400>  173
ccagcaccaa gacatggaga attcctgggt agttttagcc ttaacaggcc ttcttacatt      60

agtttttctc tcaaagtttc ttcatagtcc tcgtcgtaaa caaaatcttc caccaggtcc     120

aaaaccatgg cctattgttg gcaatataca tcttcttggt tccacccctc acagatccct     180

tcacgaactt gcaaaaagat acggagattt aatgctacta aagttcggtt cgcgcaatgt     240

ccttatttta tcctccccag atatggctag agaattcttg aaaacaaatg atgccatttg     300

ggcttctcgc cctgagcttg ccgctggtaa atatactgct tataattatt gcgacatgac     360

atgggcacgt tatggaccct tttggagaca agcaaggagg atctatctca acgagatttt     420

caatcctaaa cgtttggatt catttgagta cattcgcata gaggaaaggc ataatttgat     480

ttcacgtctt tttgttctct ctgggaagcc aattcttctt agagaccatt taactcggta     540

cactcttaca agtataagta gaacagtatt gagtggaaaa tattttagcg agtcacctgg 600
ccaaaattca atgataactt tgaaacaatt gcaggatatg cttgataagt ggtttttgct 660
taatggtgtg atcaatattg gggactggat accttggctt gctttcttgg atttgcaggg 720
ttatgtcaag caaatgaagg agttgcatag gaacttcgac aaatttcata actttgtgct 780
agatgatcac aaggctaata ggggagagaa gaactttgtg ccaagagaca tggtcgatgt 840
tttgctgcag caagctgagg atcctaatct tgaggtcaaa ctcaccaatg attgtgtcaa 900
gggtctaatg caggacttat tggctggcgg cacggacacc tcagcaacaa ccgttgaatg 960
ggctttttat gaacttctta gacaacctaa gattatgaag aaagcacaac aagagctaga 1020
ccttgtcatt tcacaggaca gatgggttca agaaaaagat tacactcaac tcccttacat 1080
tgagtcaatc atcaaggaaa cattgaggct tcacccagta agcaccatgc ttccaccgcg 1140
cattgccttg gaggattgtc atgtagcagg ctatgacata cctaaaggta caattttaat 1200
tgtgaacact tggagtattg gaagaaattc acagcattgg gagtcaccag aagaattcct 1260
tccggagagg tttgaaggga agaatattgg tgtcacagga caacattttg cgctcttgcc 1320
atttggcgcg ggccggagaa agtgcccagg atacagtctt gggattcgta taattagggc 1380
aactttagct aacttgttgc atggattcaa ctggagattg cctaatggta tgagtccaga 1440
agacattagc atggaagaga tttatgggct aattacacac cccaaagtcg cacttgacgt 1500
gatgatggag cctcgacttc ccaaccatct ttacaaatag tggataatta aaaccattaa 1560
aatcgttttg ttatatgcat gtctcatatt tgtagtggtc aaaatgtttg ttttctatca 1620
tggatgttca gtgcgaggtt gggaatttca agtcattaac gtgtgaaaat attttaaatt 1680
taaaaaaaaa aaaaaaa 1697

<210> 174
<211> 508
<212> PRT
<213> Nicotiana tabacum

<400> 174

Met Glu Asn Ser Trp Val Val Leu Ala Leu Thr Gly Leu Leu Thr Leu
1               5                   10                  15

Val Phe Leu Ser Lys Phe Leu His Ser Pro Arg Arg Lys Gln Asn Leu
            20                  25                  30

Pro Pro Gly Pro Lys Pro Trp Pro Ile Val Gly Asn Ile His Leu Leu
        35                  40                  45

Gly Ser Thr Pro His Arg Ser Leu His Glu Leu Ala Lys Arg Tyr Gly
    50                  55                  60

Asp Leu Met Leu Leu Lys Phe Gly Ser Arg Asn Val Leu Ile Leu Ser
65                  70                  75                  80

Ser Pro Asp Met Ala Arg Glu Phe Leu Lys Thr Asn Asp Ala Ile Trp
                85                  90                  95

Ala Ser Arg Pro Glu Leu Ala Ala Gly Lys Tyr Thr Ala Tyr Asn Tyr
            100                 105                 110

Cys Asp Met Thr Trp Ala Arg Tyr Gly Pro Phe Trp Arg Gln Ala Arg
        115                 120                 125

Arg Ile Tyr Leu Asn Glu Ile Phe Asn Pro Lys Arg Leu Asp Ser Phe
    130                 135                 140

Glu Tyr Ile Arg Ile Glu Glu Arg His Asn Leu Ile Ser Arg Leu Phe
145                 150                 155                 160

Val Leu Ser Gly Lys Pro Ile Leu Leu Arg Asp His Leu Thr Arg Tyr
                165                 170                 175

Thr Leu Thr Ser Ile Ser Arg Thr Val Leu Ser Gly Lys Tyr Phe Ser
            180                 185                 190

Glu Ser Pro Gly Gln Asn Ser Met Ile Thr Leu Lys Gln Leu Gln Asp
        195                 200                 205

Met Leu Asp Lys Trp Phe Leu Leu Asn Gly Val Ile Asn Ile Gly Asp
    210                 215                 220

Trp Ile Pro Trp Leu Ala Phe Leu Asp Leu Gln Gly Tyr Val Lys Gln
225                 230                 235                 240

Met Lys Glu Leu His Arg Asn Phe Asp Lys Phe His Asn Phe Val Leu
                245                 250                 255

Asp Asp His Lys Ala Asn Arg Gly Glu Lys Asn Phe Val Pro Arg Asp
            260                 265                 270

Met Val Asp Val Leu Leu Gln Gln Ala Glu Asp Pro Asn Leu Glu Val
        275                 280                 285

Lys Leu Thr Asn Asp Cys Val Lys Gly Leu Met Gln Asp Leu Leu Ala
    290                 295                 300

Gly Gly Thr Asp Thr Ser Ala Thr Thr Val Glu Trp Ala Phe Tyr Glu
305                 310                 315                 320

Leu Leu Arg Gln Pro Lys Ile Met Lys Lys Ala Gln Gln Glu Leu Asp
                325                 330                 335

Leu Val Ile Ser Gln Asp Arg Trp Val Gln Glu Lys Asp Tyr Thr Gln
            340                 345                 350

Leu Pro Tyr Ile Glu Ser Ile Ile Lys Glu Thr Leu Arg Leu His Pro
        355                 360                 365

Val Ser Thr Met Leu Pro Pro Arg Ile Ala Leu Glu Asp Cys His Val
    370                 375                 380

Ala Gly Tyr Asp Ile Pro Lys Gly Thr Ile Leu Ile Val Asn Thr Trp
385                 390                 395                 400

Ser Ile Gly Arg Asn Ser Gln His Trp Glu Ser Pro Glu Glu Phe Leu
                405                 410                 415

Pro Glu Arg Phe Glu Gly Lys Asn Ile Gly Val Thr Gly Gln His Phe
            420                 425                 430

Ala Leu Leu Pro Phe Gly Ala Gly Arg Arg Lys Cys Pro Gly Tyr Ser
        435                 440                 445

Leu Gly Ile Arg Ile Ile Arg Ala Thr Leu Ala Asn Leu Leu His Gly
    450                 455                 460

Phe Asn Trp Arg Leu Pro Asn Gly Met Ser Pro Glu Asp Ile Ser Met
465                 470                 475                 480

Glu Glu Ile Tyr Gly Leu Ile Thr His Pro Lys Val Ala Leu Asp Val
                485                 490                 495

Met Met Glu Pro Arg Leu Pro Asn His Leu Tyr Lys
            500                 505

<210>  175
<211>  1631
<212>  DNA
<213>  Nicotiana tabacum

<400>  175
actgttcaaa tcacagtaac agcatcttgt gctgccataa taattactct agtggtgtgt      60

atatggagag tgctgaattg ggtttggttc agaccaaaga agctggaaaa gctactgagg     120

aaacaaggtc tcaaaggcaa ttcctacagg attttgtatg gggatatgaa ggagctttct      180

ggtatgatta aggaagctaa ctccaaaccc atgaatcttt ctgatgatat tgccccaaga     240

ttggtccctt tctttcttga taccatcaag aaatatggga aaaaatcctt tgtatggttg     300

ggtccaaaac cgctggtttt tgtcatggac cccgagctta taaaggaagt attctccaaa     360

aactatctgt atcaaaagcc tcattcaaat ccattaacca agttactggc acaaggactt     420

gtaagccaag aggaagacaa atgggccaaa catagaaaaa tcgtcactcc tgccttccac     480

ctggagaagc taaagcatat gcttccagct ttttgtttga gctgtactga gatgctgagc      540

aaatgggaag acattgttgc agttgagggc tcacatgaga tagatatatg gcctggcctt     600

caacaattaa ctagtgatgt gatctctcgg acagcctttg gcagtagcta tgaagcaggt     660

agaaggatat ttgaacttca aaaggaacaa gctcaatttc ttatggaagc tatacgctcc 720
gtttatattc caggctggag gtttttgcca acaaagagga acagaagaat gaaggaaatt 780
gaaaaggatg ttcaagcctt agttagaggt attattgata aaagagtaaa gtcaatgaaa 840
gcaggagagg tgaataatga ggatctgctt ggtatattgc tggaatctaa ttttaaagaa 900
attgaacagc atggaaacaa ggattttgga atgagcattg aagaagtcat tcaagaatgc 960
aagttattct attttgctgg ccaagaaact acatcagtgt tgcttgtatg gactctaata 1020
ttgctgagca ggcatcagga ttggcaagca ctggccagag aagaggtgtt gcaagtcttt 1080
gggaatcaga aaccagattt tgatggatta aatcgtctaa aaattgttac aatgatcttg 1140
tacgagtctt taaggctcta tcccccagta gtgacactta cccgaaggcc taaggaagac 1200
actgtattag gagatgtatc tctaccagca ggtgtgttaa tctccttacc agtgatctta 1260
ttgcatcacg acgaagagat atggggtaaa gatgcaaaga agttcaagcc agagagattc 1320
agagatggag tctcaagtgc aacaaagggt caagtcactt ttttcccatt tacttggggt 1380
cccagaatat gcattggaca aaattttgcc atgttagaag caaagactac tttggctatg 1440
atcctacaac gcttctcctt tgaactgtct ccatcttatg cacatgctcc tcagtccata 1500
ataactttgc aaccccagta tggtgctcca cttattttgc ataaaatata gtttattact 1560
tgtaagtagt gtctcgtttt atgttaagca tgagtccaaa atgttaaggc ttgtagaact 1620
gcaaaatggg a 1631

<210> 176
<211> 461
<212> PRT
<213> Nicotiana tabacum

<400> 176

Met Lys Glu Leu Ser Gly Met Ile Lys Glu Ala Asn Ser Lys Pro Met
1               5                   10                  15

Asn Leu Ser Asp Asp Ile Ala Pro Arg Leu Val Pro Phe Phe Leu Asp
            20                  25                  30

Thr Ile Lys Lys Tyr Gly Lys Lys Ser Phe Val Trp Leu Gly Pro Lys
        35                  40                  45

Pro Leu Val Phe Val Met Asp Pro Glu Leu Ile Lys Glu Val Phe Ser
    50                  55                  60

Lys Asn Tyr Leu Tyr Gln Lys Pro His Ser Asn Pro Leu Thr Lys Leu
65                  70                  75                  80

Leu Ala Gln Gly Leu Val Ser Gln Glu Glu Asp Lys Trp Ala Lys His
                85                  90                  95

Arg Lys Ile Val Thr Pro Ala Phe His Leu Glu Lys Leu Lys His Met
            100                 105                 110

Leu Pro Ala Phe Cys Leu Ser Cys Thr Glu Met Leu Ser Lys Trp Glu
        115                 120                 125

Asp Ile Val Ala Val Glu Gly Ser His Glu Ile Asp Ile Trp Pro Gly
    130                 135                 140

Leu Gln Gln Leu Thr Ser Asp Val Ile Ser Arg Thr Ala Phe Gly Ser
145                 150                 155                 160

Ser Tyr Glu Ala Gly Arg Arg Ile Phe Glu Leu Gln Lys Glu Gln Ala
                165                 170                 175

Gln Phe Leu Met Glu Ala Ile Arg Ser Val Tyr Ile Pro Gly Trp Arg
            180                 185                 190

Phe Leu Pro Thr Lys Arg Asn Arg Arg Met Lys Glu Ile Glu Lys Asp
        195                 200                 205

Val Gln Ala Leu Val Arg Gly Ile Ile Asp Lys Arg Val Lys Ser Met
    210                 215                 220

Lys Ala Gly Glu Val Asn Asn Glu Asp Leu Leu Gly Ile Leu Leu Glu
225                 230                 235                 240

Ser Asn Phe Lys Glu Ile Glu Gln His Gly Asn Lys Asp Phe Gly Met
                245                 250                 255

Ser Ile Glu Glu Val Ile Gln Glu Cys Lys Leu Phe Tyr Phe Ala Gly
            260                 265                 270

Gln Glu Thr Thr Ser Val Leu Leu Val Trp Thr Leu Ile Leu Leu Ser
        275                 280                 285

Arg His Gln Asp Trp Gln Ala Leu Ala Arg Glu Glu Val Leu Gln Val
    290                 295                 300

Phe Gly Asn Gln Lys Pro Asp Phe Asp Gly Leu Asn Arg Leu Lys Ile
305                 310                 315                 320

Val Thr Met Ile Leu Tyr Glu Ser Leu Arg Leu Tyr Pro Pro Val Val
                325                 330                 335

Thr Leu Thr Arg Arg Pro Lys Glu Asp Thr Val Leu Gly Asp Val Ser
            340                 345                 350

Leu Pro Ala Gly Val Leu Ile Ser Leu Pro Val Ile Leu Leu His His
        355                 360                 365

Asp Glu Glu Ile Trp Gly Lys Asp Ala Lys Lys Phe Lys Pro Glu Arg
    370                 375                 380

Phe Arg Asp Gly Val Ser Ser Ala Thr Lys Gly Gln Val Thr Phe Phe
385                 390                 395                 400

Pro Phe Thr Trp Gly Pro Arg Ile Cys Ile Gly Gln Asn Phe Ala Met
                405                 410                 415

Leu Glu Ala Lys Thr Thr Leu Ala Met Ile Leu Gln Arg Phe Ser Phe
            420                 425                 430

Glu Leu Ser Pro Ser Tyr Ala His Ala Pro Gln Ser Ile Ile Thr Leu
        435                 440                 445

Gln Pro Gln Tyr Gly Ala Pro Leu Ile Leu His Lys Ile
    450                 455                 460

<210>  177
<211>  1572
<212>  DNA
<213>  Nicotiana tabacum

<400>  177
atttatctct gaaaatgcaa ttcttcagct tggtttccat tttcctattc ctatctttcc      60
tatttttgtt gaggaaatgg aagaactcca atagccaaag caaaaaattg ccaccaggtc     120
catggaaaat accaatacta ggaagtatgc ttcatatgat tggtggagaa ccgcaccatg     180
tccttagaga tttagccaaa aaagatggac cacttatgca ccttcagtta ggtgaaattt     240
ctgcagttgt ggttacttct agggacatgg caaaagaagt gctaaaaact catgacgtcg     300
tttttgcatc taggcctaaa attgtagcca tggacattat ctgttataac cagtccgaca     360
ttgcctttag cccttatggc gaccactgga gacaaatgcg taaaatttgt gtcatggaac     420
ttctcaatgc aaagaatgtt cggtctttca gctccatcag acgtgatgaa gtcgttcgtc     480
tcattgactc tatccggtca gattcttctt caggtgagct agttaatttt acgcagagga     540
tcatttggtt tgcaagctcc atgacgtgta gatcagcatt tgggcaagta ctcaaggggc     600
aagacatatt tgccaaaaag atcagagaag taataggatt agcagaaggc tttgatgtgg     660
tagacatctt ccctacatac aagtttcttc atgttctcag tgggatgaag cgtaaacttt     720
tgaatgccca ccttaaggta gacgccattg ttgaggatgt catcaacgag cacaagaaaa     780
atcttgcagc tggcaagagt aatggcgcat taggaggcga agatctaatt gatgtcctac     840
tgagacttat gaatgacaca agtcttcaat ttcccatcac caacgacaat atcaaagctg     900
ttgttgttga catgtttgct gccggaacag aaacttcatc aacaacaact gtatgggcca     960
tggctgaaat gatgaagaat ccaagtgtat tcgccaaagc tcaagcagaa gtgcgagaag    1020
cctttaggga caaagtatct tttgatgaaa atgatgtgga ggagctgaaa tacttaaagt    1080
tagtcattaa agaaactttg agacttcatc caccgtctcc acttttggtc ccaagagaat    1140
gcagggaaga tacggatata aacggctaca ctattcctgc aaagaccaaa gttatggtta    1200

atgtttgggc attgggaaga gatccaaaat attgggatga cgcggaaagc tttaagccag 1260

agagatttga gcaatgttct gtagatattt ttggtaataa ttttgagttt cttccctttg 1320

gcgggggacg gagaatttgt cctggaatgt catttggttt agctaatctt tacttaccat 1380

tggctcaatt actctatcac tttgactgga aactcccaac cggaatcaag ccaagagact 1440

tggacttgac cgaattatcg ggaataacta ttgctagaaa gggtgacctt tacttaaatg 1500

ctactcctta tcaaccttct cgagagtaat ttactattgg cataaacatt ttaaatttcc 1560

ttcatcaacc tc 1572

<210> 178
<211> 504
<212> PRT
<213> Nicotiana tabacum

<400> 178

Met Gln Phe Phe Ser Leu Val Ser Ile Phe Leu Phe Leu Ser Phe Leu
1               5                   10                  15

Phe Leu Leu Arg Lys Trp Lys Asn Ser Asn Ser Gln Ser Lys Lys Leu
            20                  25                  30

Pro Pro Gly Pro Trp Lys Ile Pro Ile Leu Gly Ser Met Leu His Met
        35                  40                  45

Ile Gly Gly Glu Pro His His Val Leu Arg Asp Leu Ala Lys Lys Asp
    50                  55                  60

Gly Pro Leu Met His Leu Gln Leu Gly Glu Ile Ser Ala Val Val Val
65                  70                  75                  80

Thr Ser Arg Asp Met Ala Lys Glu Val Leu Lys Thr His Asp Val Val
                85                  90                  95

Phe Ala Ser Arg Pro Lys Ile Val Ala Met Asp Ile Ile Cys Tyr Asn
            100                 105                 110

Gln Ser Asp Ile Ala Phe Ser Pro Tyr Gly Asp His Trp Arg Gln Met
        115                 120                 125

Arg Lys Ile Cys Val Met Glu Leu Leu Asn Ala Lys Asn Val Arg Ser
    130                 135                 140

Phe Ser Ser Ile Arg Arg Asp Glu Val Val Arg Leu Ile Asp Ser Ile
145                 150                 155                 160

Arg Ser Asp Ser Ser Ser Gly Glu Leu Val Asn Phe Thr Gln Arg Ile
                165                 170                 175

Ile Trp Phe Ala Ser Ser Met Thr Cys Arg Ser Ala Phe Gly Gln Val
            180                 185                 190

Leu Lys Gly Gln Asp Ile Phe Ala Lys Lys Ile Arg Glu Val Ile Gly
        195                 200                 205

Leu Ala Glu Gly Phe Asp Val Val Asp Ile Phe Pro Thr Tyr Lys Phe
    210                 215                 220

Leu His Val Leu Ser Gly Met Lys Arg Lys Leu Leu Asn Ala His Leu
225                 230                 235                 240

Lys Val Asp Ala Ile Val Glu Asp Val Ile Asn Glu His Lys Lys Asn
                245                 250                 255

Leu Ala Ala Gly Lys Ser Asn Gly Ala Leu Gly Gly Glu Asp Leu Ile
            260                 265                 270

Asp Val Leu Leu Arg Leu Met Asn Asp Thr Ser Leu Gln Phe Pro Ile
        275                 280                 285

Thr Asn Asp Asn Ile Lys Ala Val Val Val Asp Met Phe Ala Ala Gly
    290                 295                 300

Thr Glu Thr Ser Ser Thr Thr Thr Val Trp Ala Met Ala Glu Met Met
305                 310                 315                 320

Lys Asn Pro Ser Val Phe Ala Lys Ala Gln Ala Glu Val Arg Glu Ala
                325                 330                 335

Phe Arg Asp Lys Val Ser Phe Asp Glu Asn Asp Val Glu Glu Leu Lys
            340                 345                 350

Tyr Leu Lys Leu Val Ile Lys Glu Thr Leu Arg Leu His Pro Pro Ser
        355                 360                 365

Pro Leu Leu Val Pro Arg Glu Cys Arg Glu Asp Thr Asp Ile Asn Gly
    370                 375                 380

Tyr Thr Ile Pro Ala Lys Thr Lys Val Met Val Asn Val Trp Ala Leu
385                 390                 395                 400

Gly Arg Asp Pro Lys Tyr Trp Asp Asp Ala Glu Ser Phe Lys Pro Glu
                405                 410                 415

Arg Phe Glu Gln Cys Ser Val Asp Ile Phe Gly Asn Asn Phe Glu Phe
            420                 425                 430

Leu Pro Phe Gly Gly Gly Arg Arg Ile Cys Pro Gly Met Ser Phe Gly
        435                 440                 445

Leu Ala Asn Leu Tyr Leu Pro Leu Ala Gln Leu Leu Tyr His Phe Asp
    450                 455                 460

Trp Lys Leu Pro Thr Gly Ile Lys Pro Arg Asp Leu Asp Leu Thr Glu
465                 470                 475                 480

Leu Ser Gly Ile Thr Ile Ala Arg Lys Gly Asp Leu Tyr Leu Asn Ala
                485                 490                 495

Thr Pro Tyr Gln Pro Ser Arg Glu
            500

<210>  179
<211>  1633
<212>  DNA
<213>  Nicotiana tabacum

<400>  179
ataatgcttt ctcccataga agccattgta ggactagtaa ccttcacatt tctcttcttc      60

ttcctatgga caaaaaaatc tcaaaaacct tcaaaaccct taccaccgaa aatccccgga     120

ggatggccgg taatcggcca tcttttccac ttcaatgacg acggcgacga ccgtccatta     180

gctcgaaaac tcggagactt agctgacaaa tacggccccg ttttcacttt tcggctaggc     240

cttccccttg tcttagttgt aagcagttac gaagctgtaa aagactgttt ctctacaaat     300

gacgccattt tttccaatcg tccagctttt ctttacggcg attaccttgg ctacaataat     360

gccatgctat ttttggccaa ttacggacct tactggcgaa aaaatcgaaa attagttatt     420

caggaagttc tctccgctag tcgtctcgaa aaattcaaac acgtgagatt tgcaagaatt     480

caagcgagca ttaagaattt atatactcga attgatggaa attcgagtac gataaattta     540

actgattggt tagaagaatt gaattttggt ctgatcgtga agatgatcgc tggaaaaaat     600

tatgaatccg gtaaaggaga tgaacaagtg gagagattta agaaagcgtt taaggatttt     660

atgattttat caatggagtt tgtgttatgg gatgcatttc caattccatt atttaaatgg     720

gtggattttc aagggcatgt taaggctatg aaaaggactt ttaaagatat agattctgtt     780

tttcagaatt ggttagggga acatattaat aaaagagaaa aaatggaggt taatgcagaa     840

gggaatgaac aagatttcat tgatgtggtg ctttcaaaaa tgagtaatga atatcttggt     900

gaaggttact ctcgtgatac tgtcattaaa gcaacggtgt ttagtttggt cttggatgca     960

gcagacacag ttgctcttca cataaattgg ggaatggcat tattgataaa caatcaaaag    1020

gccttgacga aagcacaaga agagatagac acaaaagttg gtaaggacag atgggtagaa    1080

gagagtgata ttaaggattt ggtatacctc caagctattg ttaaagaagt gttacgatta    1140

tatccaccag gacctttgtt agtaccacac gaaaatgtag aagattgtgt tgttagtgga    1200

tatcacattc ctaaagggac aagattattc gcaaacgtca tgaaactgct acgtgatcct    1260

aaactctggc ctgatcctga tactttcgat ccagagagat tcattgctac tgatattgac    1320

tttcgtggtc agtactataa gtatatcccg tttggttctg gaagacgatc ttgtccaggg    1380

atgacttatg cattgcaagt ggaacactta acaatggcac atttgatcca aggtttcaat    1440

tacagaactc caaatgacga gcccttggat atgaaggaag gtgcaggcat aactatacgt 1500
aaggtaaatc ctgtggaact gataatagcg cctcgcctgg cacctgagct ttattaaaac 1560
ctaagatctt tcatcttggt tgatcattgt ataatactcc taaatggata ttcatttacc 1620
ttttatcaat taa 1633

<210> 180
<211> 517
<212> PRT
<213> Nicotiana tabacum

<400> 180

Met Leu Ser Pro Ile Glu Ala Ile Val Gly Leu Val Thr Phe Thr Phe
1               5                   10                  15

Leu Phe Phe Phe Leu Trp Thr Lys Lys Ser Gln Lys Pro Ser Lys Pro
            20                  25                  30

Leu Pro Pro Lys Ile Pro Gly Gly Trp Pro Val Ile Gly His Leu Phe
        35                  40                  45

His Phe Asn Asp Asp Gly Asp Asp Arg Pro Leu Ala Arg Lys Leu Gly
    50                  55                  60

Asp Leu Ala Asp Lys Tyr Gly Pro Val Phe Thr Phe Arg Leu Gly Leu
65                  70                  75                  80

Pro Leu Val Leu Val Val Ser Ser Tyr Glu Ala Val Lys Asp Cys Phe
                85                  90                  95

Ser Thr Asn Asp Ala Ile Phe Ser Asn Arg Pro Ala Phe Leu Tyr Gly
            100                 105                 110

Asp Tyr Leu Gly Tyr Asn Asn Ala Met Leu Phe Leu Ala Asn Tyr Gly
        115                 120                 125

Pro Tyr Trp Arg Lys Asn Arg Lys Leu Val Ile Gln Glu Val Leu Ser
    130                 135                 140

Ala Ser Arg Leu Glu Lys Phe Lys His Val Arg Phe Ala Arg Ile Gln
145                 150                 155                 160

Ala Ser Ile Lys Asn Leu Tyr Thr Arg Ile Asp Gly Asn Ser Ser Thr
                165                 170                 175

Ile Asn Leu Thr Asp Trp Leu Glu Glu Leu Asn Phe Gly Leu Ile Val
            180                 185                 190

Lys Met Ile Ala Gly Lys Asn Tyr Glu Ser Gly Lys Gly Asp Glu Gln
        195                 200                 205

Val Glu Arg Phe Lys Lys Ala Phe Lys Asp Phe Met Ile Leu Ser Met
    210                 215                 220

Glu Phe Val Leu Trp Asp Ala Phe Pro Ile Pro Leu Phe Lys Trp Val
225                 230                 235                 240

Asp Phe Gln Gly His Val Lys Ala Met Lys Arg Thr Phe Lys Asp Ile
                245                 250                 255

Asp Ser Val Phe Gln Asn Trp Leu Gly Glu His Ile Asn Lys Arg Glu
            260                 265                 270

Lys Met Glu Val Asn Ala Glu Gly Asn Glu Gln Asp Phe Ile Asp Val
        275                 280                 285

Val Leu Ser Lys Met Ser Asn Glu Tyr Leu Gly Glu Gly Tyr Ser Arg
    290                 295                 300

Asp Thr Val Ile Lys Ala Thr Val Phe Ser Leu Val Leu Asp Ala Ala
305                 310                 315                 320

Asp Thr Val Ala Leu His Ile Asn Trp Gly Met Ala Leu Leu Ile Asn
                325                 330                 335

Asn Gln Lys Ala Leu Thr Lys Ala Gln Glu Glu Ile Asp Thr Lys Val
            340                 345                 350

Gly Lys Asp Arg Trp Val Glu Glu Ser Asp Ile Lys Asp Leu Val Tyr
        355                 360                 365

Leu Gln Ala Ile Val Lys Glu Val Leu Arg Leu Tyr Pro Pro Gly Pro
    370                 375                 380

Leu Leu Val Pro His Glu Asn Val Glu Asp Cys Val Val Ser Gly Tyr
385                 390                 395                 400

His Ile Pro Lys Gly Thr Arg Leu Phe Ala Asn Val Met Lys Leu Leu
                405                 410                 415

Arg Asp Pro Lys Leu Trp Pro Asp Pro Asp Thr Phe Asp Pro Glu Arg
            420                 425                 430

Phe Ile Ala Thr Asp Ile Asp Phe Arg Gly Gln Tyr Tyr Lys Tyr Ile
        435                 440                 445

Pro Phe Gly Ser Gly Arg Arg Ser Cys Pro Gly Met Thr Tyr Ala Leu
    450                 455                 460

Gln Val Glu His Leu Thr Met Ala His Leu Ile Gln Gly Phe Asn Tyr
465                 470                 475                 480

Arg Thr Pro Asn Asp Glu Pro Leu Asp Met Lys Glu Gly Ala Gly Ile
                485                 490                 495

Thr Ile Arg Lys Val Asn Pro Val Glu Leu Ile Ile Ala Pro Arg Leu
            500                 505                 510

Ala Pro Glu Leu Tyr
        515

<210>  181
<211>  1585
<212>  DNA
<213>  Nicotiana tabacum

<400>  181
   aatccataat gctttctccc atagaagcca ttgtaggact agtaaccttc acatttctct      60
   tcttcttcct atggacaaaa aaatctcaaa aaccttcaaa acccttacca ccgaaaatcc     120
   ccggaggatg gccggtaatc ggccatcttt tccacttcaa tgacgacggc gacgaccgtc     180
   cattagctcg aaaactcgga gacttagctg acaaatacgg ccccgttttc acttttcggc     240
   taggccttcc ccttgtctta gttgtaagca gttacgaagc tgtaaaagac tgtttctcta     300
   caaatgacgc cattttttcc aatcgtccag cttttcttta cggcgattac cttggctaca     360
   ataatgccat gctatttttg gccaattacg gaccttactg gcgaaaaaat cgaaaattag     420
   ttattcagga agttctctcc gctagtcgtc tcgaaaaatt caaacacgtg agatttgcaa     480
   gaattcaagc gagcattaag aatttatata ctcgaattga tggaaattcg agtacgataa     540
   atttaactga ttggttagaa gaattgaatt ttggtctgat cgtgaagatg atcgctggaa     600
   aaaattatga atccggtaaa ggagatgaac aagtggagag atttaagaaa gcgtttaagg     660
   attttatgat tttatcaatg gagtttgtgt tatgggatgc atttccaatt ccattattta     720
   aatgggtgga ttttcaaggg catgttaagg ctatgaaaag gacttttaaa gatatagatt     780
   ctgtttttca gaattggtta gaggaacata ttaataaaag agaaaaaatg gaggttaatg     840
   cagaagggaa tgaacaagat ttcattgatg tggtgctttc aaaaatgagt aatgaatatc     900
   ttggtgaagg ttactctcgt gatactgtca ttaaagcaac ggtgtttagt ttggtcttgg     960
   atgcagcaga cacagttgct cttcacataa attggggaat ggcattattg ataaacaatc    1020
   aaaaggcctt gacgaaagca caagaagaga tagacacaaa agttggtaag gacagatggg    1080
   tagaagagag tgatattaag gatttggtat acctccaagc tattgttaaa gaagtgttac    1140
   gattatatcc accaggacct ttgttagtac cacacgaaaa tgtagaagat tgtgttgtta    1200
   gtggatatca cattcctaaa gggacaagat tattcgcaaa cgtcatgaaa ctgcaacgtg    1260
   atcctaaact ctggtctgat cctgatactt tcgatccaga gagattcatt gctactgata    1320
   ttgactttcg tggtcagtac tataagtata tcccgtttgg ttctggaaga cgatcttgtc    1380
   cagggatgac ttatgcattg caagtggaac acttaacaat ggcacatttg atccaaggtt    1440
   tcaattacag aactccaaat gacgagccct tggatatgaa ggaaggtgca ggcataacta    1500
   tacgtaaggt aaatcctgtg gaactgataa tagcgcctcg cctggcacct gagctttatt 1560
   aaaacctaag atcatcttgc ttgat 1585

<210> 182
<211> 517
<212> PRT
<213> Nicotiana tabacum

<400> 182

Met Leu Ser Pro Ile Glu Ala Ile Val Gly Leu Val Thr Phe Thr Phe
1               5                   10                  15

Leu Phe Phe Phe Leu Trp Thr Lys Lys Ser Gln Lys Pro Ser Lys Pro
            20                  25                  30

Leu Pro Pro Lys Ile Pro Gly Gly Trp Pro Val Ile Gly His Leu Phe
        35                  40                  45

His Phe Asn Asp Asp Gly Asp Asp Arg Pro Leu Ala Arg Lys Leu Gly
    50                  55                  60

Asp Leu Ala Asp Lys Tyr Gly Pro Val Phe Thr Phe Arg Leu Gly Leu
65                  70                  75                  80

Pro Leu Val Leu Val Val Ser Ser Tyr Glu Ala Val Lys Asp Cys Phe
                85                  90                  95

Ser Thr Asn Asp Ala Ile Phe Ser Asn Arg Pro Ala Phe Leu Tyr Gly
            100                 105                 110

Asp Tyr Leu Gly Tyr Asn Asn Ala Met Leu Phe Leu Ala Asn Tyr Gly
        115                 120                 125

Pro Tyr Trp Arg Lys Asn Arg Lys Leu Val Ile Gln Glu Val Leu Ser
    130                 135                 140

Ala Ser Arg Leu Glu Lys Phe Lys His Val Arg Phe Ala Arg Ile Gln
145                 150                 155                 160

Ala Ser Ile Lys Asn Leu Tyr Thr Arg Ile Asp Gly Asn Ser Ser Thr
                165                 170                 175

Ile Asn Leu Thr Asp Trp Leu Glu Glu Leu Asn Phe Gly Leu Ile Val
            180                 185                 190

Lys Met Ile Ala Gly Lys Asn Tyr Glu Ser Gly Lys Gly Asp Glu Gln
        195                 200                 205

Val Glu Arg Phe Lys Lys Ala Phe Lys Asp Phe Met Ile Leu Ser Met
    210                 215                 220

Glu Phe Val Leu Trp Asp Ala Phe Pro Ile Pro Leu Phe Lys Trp Val
225                 230                 235                 240

Asp Phe Gln Gly His Val Lys Ala Met Lys Arg Thr Phe Lys Asp Ile
                245                 250                 255

Asp Ser Val Phe Gln Asn Trp Leu Glu Glu His Ile Asn Lys Arg Glu
            260                 265                 270

Lys Met Glu Val Asn Ala Glu Gly Asn Glu Gln Asp Phe Ile Asp Val
        275                 280                 285

Val Leu Ser Lys Met Ser Asn Glu Tyr Leu Gly Glu Gly Tyr Ser Arg
    290                 295                 300

Asp Thr Val Ile Lys Ala Thr Val Phe Ser Leu Val Leu Asp Ala Ala
305                 310                 315                 320

Asp Thr Val Ala Leu His Ile Asn Trp Gly Met Ala Leu Leu Ile Asn
                325                 330                 335

Asn Gln Lys Ala Leu Thr Lys Ala Gln Glu Glu Ile Asp Thr Lys Val
            340                 345                 350

Gly Lys Asp Arg Trp Val Glu Glu Ser Asp Ile Lys Asp Leu Val Tyr
        355                 360                 365

Leu Gln Ala Ile Val Lys Glu Val Leu Arg Leu Tyr Pro Pro Gly Pro
    370                 375                 380

Leu Leu Val Pro His Glu Asn Val Glu Asp Cys Val Val Ser Gly Tyr
385                 390                 395                 400

His Ile Pro Lys Gly Thr Arg Leu Phe Ala Asn Val Met Lys Leu Gln
                405                 410                 415

Arg Asp Pro Lys Leu Trp Ser Asp Pro Asp Thr Phe Asp Pro Glu Arg
            420                 425                 430

Phe Ile Ala Thr Asp Ile Asp Phe Arg Gly Gln Tyr Tyr Lys Tyr Ile
        435                 440                 445

Pro Phe Gly Ser Gly Arg Arg Ser Cys Pro Gly Met Thr Tyr Ala Leu
    450                 455                 460

Gln Val Glu His Leu Thr Met Ala His Leu Ile Gln Gly Phe Asn Tyr
465                 470                 475                 480

Arg Thr Pro Asn Asp Glu Pro Leu Asp Met Lys Glu Gly Ala Gly Ile
                485                 490                 495

Thr Ile Arg Lys Val Asn Pro Val Glu Leu Ile Ile Ala Pro Arg Leu
            500                 505                 510

Ala Pro Glu Leu Tyr
        515

<210>  183
<211>  1620
<212>  DNA
<213>  Nicotiana tabacum

<400>  183
ctaaaactcc ataatggttt ctcccgtaga agccattgta ggactagtaa cccttacact      60

tctcttctac ttcctatggc ccaaaaaatt tcaaatacct tcaaaaccat taccaccgaa     120

aattcccgga gggtggccgg taatcggcca tcttttctac ttcgatgatg acggcgacga     180

ccgtccatta gctcgaaaac tcggagactt agctgacaaa tacggcccgg ttttcacttt     240

ccggctaggc cttccgcttg tgttaattgt aagcagttac gaagctgtaa aagactgctt     300

ctctacaaat gacgccattt tctccaatcg tccagctttt ctttacggtg aataccttgg     360

ctacaataat gccatgctat ttttgacaaa atacggacct tattggcgaa aaaatagaaa     420

attagtcatt caggaagttc tctctgctag tcgtctcgaa aaattgaagc acgtgagatt     480

tggtaaaatt caaacgagca ttaagagttt atacactcga attgatggaa attcgagtac     540

gataaatcta actgattggt tagaagaatt gaattttggt ctgatcgtga aaatgatcgc     600

tgggaaaaat tatgaatccg gtaaaggaga tgaacaagtg gagagattta ggaaagcgta     660

taaggatttt ataattttat caatggagtt tgtgttatgg gatgcttttc caattccatt     720

gttcaaatgg gtggattttc aaggctatgt taaggccatg aaaaggacat ttaaggatat     780

agattctgtt tttcagaatt ggttagagga acatgtcaag aaaagagaaa aaatggaggt     840

taatgcacaa gggaatgaac aagatttcat tgatgtggtg ctttcaaaaa tgagtaatga     900

atatcttgat gaaggttact ctcgtgatac tgtcataaaa gcaacagtgt ttagtttggt     960

cttggatgct gcggacacag ttgctcttca catgaattgg ggaatggcat tactgataaa    1020

caatcaacat gccttgaaga aagcacaaga agagatcgat aagaaagttg gtaaggaaag    1080

atgggtagaa gagagtgata ttaaggattt ggtctacctc caagctattg ttaaagaagt    1140

gttacgatta tatccaccag gacctttatt agtacctcat gaaaatgtag aggattgtgt    1200

tgttagtgga tatcacattc ctaaagggac tagactattc gcgaacgtta tgaaattgca    1260

gcgcgatcct aaactctggt caaatcctga taagtttgat ccagagagat tcttcgctga    1320

tgatattgac taccgtggtc agcactatga gtttatccca tttggttctg gaagacgatc    1380

ttgtccgggg atgacttatg cattacaagt ggaacaccta acaatagcac atttgatcca    1440

gggtttcaat tacaaaactc caaatgacga gcccttggat atgaaggaag gtgcaggatt    1500

aactatacgt aaagtaaatc ctgtagaagt gacaattacg gctcgcctgg cacctgagct    1560

ttattaaaac cttagatgtt ttatcttgat tgtactaata tatatatgca gaaaaaattg    1620

<210>  184
<211>  517
<212>  PRT
<213>  Nicotiana tabacum

<400>  184

Met Val Ser Pro Val Glu Ala Ile Val Gly Leu Val Thr Leu Thr Leu
1               5                   10                  15

Leu Phe Tyr Phe Leu Trp Pro Lys Lys Phe Gln Ile Pro Ser Lys Pro
            20                  25                  30

Leu Pro Pro Lys Ile Pro Gly Gly Trp Pro Val Ile Gly His Leu Phe
        35                  40                  45

Tyr Phe Asp Asp Asp Gly Asp Asp Arg Pro Leu Ala Arg Lys Leu Gly
    50                  55                  60

Asp Leu Ala Asp Lys Tyr Gly Pro Val Phe Thr Phe Arg Leu Gly Leu
65                  70                  75                  80

Pro Leu Val Leu Ile Val Ser Ser Tyr Glu Ala Val Lys Asp Cys Phe
                85                  90                  95

Ser Thr Asn Asp Ala Ile Phe Ser Asn Arg Pro Ala Phe Leu Tyr Gly
            100                 105                 110

Glu Tyr Leu Gly Tyr Asn Asn Ala Met Leu Phe Leu Thr Lys Tyr Gly
        115                 120                 125

Pro Tyr Trp Arg Lys Asn Arg Lys Leu Val Ile Gln Glu Val Leu Ser
    130                 135                 140

Ala Ser Arg Leu Glu Lys Leu Lys His Val Arg Phe Gly Lys Ile Gln
145                 150                 155                 160

Thr Ser Ile Lys Ser Leu Tyr Thr Arg Ile Asp Gly Asn Ser Ser Thr
                165                 170                 175

Ile Asn Leu Thr Asp Trp Leu Glu Glu Leu Asn Phe Gly Leu Ile Val
            180                 185                 190

Lys Met Ile Ala Gly Lys Asn Tyr Glu Ser Gly Lys Gly Asp Glu Gln
        195                 200                 205

Val Glu Arg Phe Arg Lys Ala Tyr Lys Asp Phe Ile Ile Leu Ser Met
    210                 215                 220

Glu Phe Val Leu Trp Asp Ala Phe Pro Ile Pro Leu Phe Lys Trp Val
225                 230                 235                 240

Asp Phe Gln Gly Tyr Val Lys Ala Met Lys Arg Thr Phe Lys Asp Ile
                245                 250                 255

Asp Ser Val Phe Gln Asn Trp Leu Glu Glu His Val Lys Lys Arg Glu
            260                 265                 270

Lys Met Glu Val Asn Ala Gln Gly Asn Glu Gln Asp Phe Ile Asp Val
        275                 280                 285

Val Leu Ser Lys Met Ser Asn Glu Tyr Leu Asp Glu Gly Tyr Ser Arg
    290                 295                 300

Asp Thr Val Ile Lys Ala Thr Val Phe Ser Leu Val Leu Asp Ala Ala
305                 310                 315                 320

Asp Thr Val Ala Leu His Met Asn Trp Gly Met Ala Leu Leu Ile Asn
                325                 330                 335

Asn Gln His Ala Leu Lys Lys Ala Gln Glu Glu Ile Asp Lys Lys Val
            340                 345                 350

Gly Lys Glu Arg Trp Val Glu Glu Ser Asp Ile Lys Asp Leu Val Tyr
        355                 360                 365

Leu Gln Ala Ile Val Lys Glu Val Leu Arg Leu Tyr Pro Pro Gly Pro
    370                 375                 380

Leu Leu Val Pro His Glu Asn Val Glu Asp Cys Val Val Ser Gly Tyr
385                 390                 395                 400

His Ile Pro Lys Gly Thr Arg Leu Phe Ala Asn Val Met Lys Leu Gln
                405                 410                 415

Arg Asp Pro Lys Leu Trp Ser Asn Pro Asp Lys Phe Asp Pro Glu Arg
            420                 425                 430

Phe Phe Ala Asp Asp Ile Asp Tyr Arg Gly Gln His Tyr Glu Phe Ile
        435                 440                 445

Pro Phe Gly Ser Gly Arg Arg Ser Cys Pro Gly Met Thr Tyr Ala Leu
    450                 455                 460

Gln Val Glu His Leu Thr Ile Ala His Leu Ile Gln Gly Phe Asn Tyr
465                 470                 475                 480

Lys Thr Pro Asn Asp Glu Pro Leu Asp Met Lys Glu Gly Ala Gly Leu
                485                 490                 495

Thr Ile Arg Lys Val Asn Pro Val Glu Val Thr Ile Thr Ala Arg Leu
            500                 505                 510

Ala Pro Glu Leu Tyr
        515

<210> 185
<211> 1623
<212> DNA
<213> Nicotiana tabacum

<400> 185
cgaggctccc caccaaaaaa tcatttctct cgtctaaaat ggatcttctc ttactagaga 60

agaccttaat tggtcttttc tttgccattt taatcgcttt aattgtctct aaacttcgtt 120

caaagcgttt taagcttcct ccaggaccaa ttccagtacc agtttttggt aattggcttc 180

aagttggtga tgatttaaac cacagaaatc ttactgatta tgccaaaaaa tttggcgatc 240

ttttcttgtt aagaatgggt caacgtaact tagttgttgt gtcatctcct gaattagcta 300

aagaagtttt acacacacaa ggtgttgaat ttggttcaag aacaagaaat gttgtgtttg 360

atatttttac tggaaaaggt caagatatgg tttttactgt atatggtgaa cattggagaa 420

aaatgaggag aattatgact gtaccatttt ttactaataa agttgtgcaa cagtatagag 480

gggggtggga gtttgaggtg gcaagtgtaa ttgaggatgt gaaaaaaaat cctgaatctg 540

ctactaatgg gatcgtatta aggaggagat tacaattaat gatgtataat aatatgttta 600

ggattatgtt tgataggaga tttgagagtg aagatgatcc tttgtttgtt aagcttaagg 660

ctttgaatgg tgaaaggagt agattggctc aaagttttga gtataattat ggtgatttta 720

ttccaatttt gaggcctttt ttgagaggtt atttgaagat ctgtaaagaa gttaaggaga 780

agaggctgca gcttttcaaa gattactttg ttgatgaaag aaagaagctt tcaaatacca 840

agagctcgga cagcaatgcc ctaaaatgtg cgattgatca cattcttgag gctcaacaga 900

agggagagat caatgaggac aacgttcttt acattgttga aaacatcaat gttgctgcaa 960

ttgaaacaac attatggtca attgagtggg gtatcgccga gctagtcaac caccctcaca 1020

tccaaaagaa actgcgcgac gagattgaca cagttcttgg accaggagtg caagtgactg 1080

aaccagacac ccacaagctt ccataccttc aggctgtgat caaggaggca cttcgtctcc 1140

gtatggcaat tcctctatta gtcccacaca tgaaccttca cgacgcaaag cttggcgggt 1200

ttgatattcc agcagagagc aaaatcttgg ttaacgcttg gtggttagct aacaacccgg 1260

ctcattggaa gaaacccgaa gagttcagac ccgagaggtt ctttgaagag gagaagcatg 1320

ttgaggccaa tggcaatgac ttcagatatc ttccgtttgg cgttggtagg aggagctgcc 1380

ctggaattat acttgcattg ccaattcttg gcatcacttt gggacgtttg gttcagaact 1440

ttgagctgtt gcctcctcca ggccagtcga agctcgacac cacagagaaa ggtggacagt 1500

tcagtctcca cattttgaag cattccacca ttgtgttgaa accaaggtct ttctgaactt 1560

tgtgatctta ttaattaagg ggttctgaag aaatttgata gtgttggata ttaagggcga 1620

att 1623

<210> 186

<211>  505
<212>  PRT
<213>  Nicotiana tabacum

<400>  186

Met Asp Leu Leu Leu Leu Glu Lys Thr Leu Ile Gly Leu Phe Phe Ala
1               5                   10                  15

Ile Leu Ile Ala Leu Ile Val Ser Lys Leu Arg Ser Lys Arg Phe Lys
            20                  25                  30

Leu Pro Pro Gly Pro Ile Pro Val Pro Val Phe Gly Asn Trp Leu Gln
        35                  40                  45

Val Gly Asp Asp Leu Asn His Arg Asn Leu Thr Asp Tyr Ala Lys Lys
    50                  55                  60

Phe Gly Asp Leu Phe Leu Leu Arg Met Gly Gln Arg Asn Leu Val Val
65                  70                  75                  80

Val Ser Ser Pro Glu Leu Ala Lys Glu Val Leu His Thr Gln Gly Val
                85                  90                  95

Glu Phe Gly Ser Arg Thr Arg Asn Val Val Phe Asp Ile Phe Thr Gly
            100                 105                 110

Lys Gly Gln Asp Met Val Phe Thr Val Tyr Gly Glu His Trp Arg Lys
        115                 120                 125

Met Arg Arg Ile Met Thr Val Pro Phe Phe Thr Asn Lys Val Val Gln
    130                 135                 140

Gln Tyr Arg Gly Gly Trp Glu Phe Glu Val Ala Ser Val Ile Glu Asp
145                 150                 155                 160

Val Lys Lys Asn Pro Glu Ser Ala Thr Asn Gly Ile Val Leu Arg Arg
                165                 170                 175

Arg Leu Gln Leu Met Met Tyr Asn Asn Met Phe Arg Ile Met Phe Asp
            180                 185                 190

Arg Arg Phe Glu Ser Glu Asp Asp Pro Leu Phe Val Lys Leu Lys Ala
        195                 200                 205

Leu Asn Gly Glu Arg Ser Arg Leu Ala Gln Ser Phe Glu Tyr Asn Tyr
    210                 215                 220

Gly Asp Phe Ile Pro Ile Leu Arg Pro Phe Leu Arg Gly Tyr Leu Lys
225                 230                 235                 240

Ile Cys Lys Glu Val Lys Glu Lys Arg Leu Gln Leu Phe Lys Asp Tyr
                245                 250                 255

Phe Val Asp Glu Arg Lys Lys Leu Ser Asn Thr Lys Ser Ser Asp Ser
            260                 265                 270

Asn Ala Leu Lys Cys Ala Ile Asp His Ile Leu Glu Ala Gln Gln Lys
        275                 280                 285

Gly Glu Ile Asn Glu Asp Asn Val Leu Tyr Ile Val Glu Asn Ile Asn
    290                 295                 300

Val Ala Ala Ile Glu Thr Thr Leu Trp Ser Ile Glu Trp Gly Ile Ala
305                 310                 315                 320

Glu Leu Val Asn His Pro His Ile Gln Lys Lys Leu Arg Asp Glu Ile
                325                 330                 335

Asp Thr Val Leu Gly Pro Gly Val Gln Val Thr Glu Pro Asp Thr His
            340                 345                 350

Lys Leu Pro Tyr Leu Gln Ala Val Ile Lys Glu Ala Leu Arg Leu Arg
        355                 360                 365

Met Ala Ile Pro Leu Leu Val Pro His Met Asn Leu His Asp Ala Lys
    370                 375                 380

Leu Gly Gly Phe Asp Ile Pro Ala Glu Ser Lys Ile Leu Val Asn Ala
385                 390                 395                 400

Trp Trp Leu Ala Asn Asn Pro Ala His Trp Lys Lys Pro Glu Glu Phe
                405                 410                 415

Arg Pro Glu Arg Phe Phe Glu Glu Glu Lys His Val Glu Ala Asn Gly
            420                 425                 430

Asn Asp Phe Arg Tyr Leu Pro Phe Gly Val Gly Arg Arg Ser Cys Pro
        435                 440                 445

Gly Ile Ile Leu Ala Leu Pro Ile Leu Gly Ile Thr Leu Gly Arg Leu
    450                 455                 460

Val Gln Asn Phe Glu Leu Leu Pro Pro Pro Gly Gln Ser Lys Leu Asp
465                 470                 475                 480

Thr Thr Glu Lys Gly Gly Gln Phe Ser Leu His Ile Leu Lys His Ser
                485                 490                 495

Thr Ile Val Leu Lys Pro Arg Ser Phe
            500                 505

<210> 187
<211> 1667

<212>  DNA
<213>  Nicotiana tabacum

<400>  187
caacacgctt actatctcct aaatctccac tcaaaaacaa agaagagaaa gatttaaaac      60

taataattat gaaagagatg gtgcaaaaca atatgagcac ttctcttctt gaaactttac     120

aagctacgcc catgatattc tacttcatcg tccctctctt ctgcttattc cttctctcca     180

aatctcgccg taaacgtttg cctccaggtc caactggctg gcctctcatt ggtaacatga     240

tgatgatgga ccagttaact caccgtggcc ttgccaaact agcccaaaaa tatggtggtg     300

tttttcacct taaaatgggt tatgttcaca aaattgtagt ctctggtcca gacgaagctc     360

gccaagtatt acaggaacac gacatcatat tttcgaaccg tccagcgacc gtagccataa     420

gttacctaac atatgacagg gcagacatgg cttttgctga ctatggactc ttctggcggc     480

agatgagaaa actatgtgta atgaaactct tcagccgcaa acgagctgag tcatgggact     540

cagttcgaga cgaagcggat tccatggtta gaattgtaac aaccaacaca ggcacagctg     600

ttaacttagg tgaacttgtt ttcagtctca ctcgtaatat tatctacaga gctgcttttg     660

gaacttgttc tgaagatgga caaggcgagt tcattgaaat tatgcaagag ttttcgaagc     720

tatttggcgc tttcaatata gctgatttta ttccatggct agggtgggtt ggtaagcaga     780

gtctaaatat tagacttgct aaggctagag cgtcgcttga tgggttcatt gattcgatta     840

ttgatgacca tattattaga aagaaagctt atgttaatgg caaaaatgat ggaggtgatc     900

gagaaactga tatggtggat gagcttttag ctttttacag tgaggaagca aaagtaactg     960

agtccgaaga tttgcagaat gctatcagac ttactaagga tagtatcaaa gctatcatca    1020

tggatgtaat gtttggaggg acagaaacag tggcttctgc aatagaatgg gccatggcag    1080

agcttatgag gagtcctgaa gatcttaaaa aagtacaaca agggctggct aacgttgttg    1140

gactcaacag aaaagttgaa gaatctgact ttgaaaaatt aacatactta agatgttgtc    1200

taaaagaaac tctacgactt caccctccaa tccctctcct cctccatgag accgccgagg    1260

aatccaccgt ctccggctac catattccgg caaagtcaca tgttattata aattcatttg    1320

ccattgggcg tgacaaaaat tcatgggaag atcctgaaac ttataaacca tctaggtttc    1380

tcaaagaagg tgtaccagat tttaaaggag gtaattttga gtttatacca tttgggtcgg    1440

gtcggcggtc ttgccccggt atgcaacttg ggctttatgc attggaaatg gctgtggccc    1500

atcttcttca ttgttttact tgggaattgc cagatggtat gaaaccaagt gagcttaaaa    1560

tggatgatat ttttggactc actgctccaa gagctaatcg actcgtggct gtgcctactc    1620

cacgcttgtt gtgtcccctt tattaattga agaaaaaagg tggggct                  1667

<210>  188
<211>  525
<212>  PRT
<213>  Nicotiana tabacum

<400>  188

Met Lys Glu Met Val Gln Asn Asn Met Ser Thr Ser Leu Leu Glu Thr
1               5                   10                  15

Leu Gln Ala Thr Pro Met Ile Phe Tyr Phe Ile Val Pro Leu Phe Cys
            20                  25                  30

Leu Phe Leu Leu Ser Lys Ser Arg Arg Lys Arg Leu Pro Pro Gly Pro
        35                  40                  45

Thr Gly Trp Pro Leu Ile Gly Asn Met Met Met Met Asp Gln Leu Thr
    50                  55                  60

His Arg Gly Leu Ala Lys Leu Ala Gln Lys Tyr Gly Gly Val Phe His
65                  70                  75                  80

Leu Lys Met Gly Tyr Val His Lys Ile Val Val Ser Gly Pro Asp Glu
                85                  90                  95

Ala Arg Gln Val Leu Gln Glu His Asp Ile Ile Phe Ser Asn Arg Pro
            100                 105                 110

Ala Thr Val Ala Ile Ser Tyr Leu Thr Tyr Asp Arg Ala Asp Met Ala
        115                 120                 125

Phe Ala Asp Tyr Gly Leu Phe Trp Arg Gln Met Arg Lys Leu Cys Val
    130                 135                 140

Met Lys Leu Phe Ser Arg Lys Arg Ala Glu Ser Trp Asp Ser Val Arg
145                 150                 155                 160

Asp Glu Ala Asp Ser Met Val Arg Ile Val Thr Thr Asn Thr Gly Thr
                165                 170                 175

Ala Val Asn Leu Gly Glu Leu Val Phe Ser Leu Thr Arg Asn Ile Ile
            180                 185                 190

Tyr Arg Ala Ala Phe Gly Thr Cys Ser Glu Asp Gly Gln Gly Glu Phe
        195                 200                 205

Ile Glu Ile Met Gln Glu Phe Ser Lys Leu Phe Gly Ala Phe Asn Ile
    210                 215                 220

Ala Asp Phe Ile Pro Trp Leu Gly Trp Val Gly Lys Gln Ser Leu Asn
225                 230                 235                 240

Ile Arg Leu Ala Lys Ala Arg Ala Ser Leu Asp Gly Phe Ile Asp Ser
                245                 250                 255

Ile Ile Asp Asp His Ile Ile Arg Lys Lys Ala Tyr Val Asn Gly Lys
            260                 265                 270

Asn Asp Gly Gly Asp Arg Glu Thr Asp Met Val Asp Glu Leu Leu Ala
        275                 280                 285

Phe Tyr Ser Glu Glu Ala Lys Val Thr Glu Ser Glu Asp Leu Gln Asn
    290                 295                 300

Ala Ile Arg Leu Thr Lys Asp Ser Ile Lys Ala Ile Ile Met Asp Val
305                 310                 315                 320

Met Phe Gly Gly Thr Glu Thr Val Ala Ser Ala Ile Glu Trp Ala Met
                325                 330                 335

Ala Glu Leu Met Arg Ser Pro Glu Asp Leu Lys Lys Val Gln Gln Gly
            340                 345                 350

Leu Ala Asn Val Val Gly Leu Asn Arg Lys Val Glu Glu Ser Asp Phe
        355                 360                 365

Glu Lys Leu Thr Tyr Leu Arg Cys Cys Leu Lys Glu Thr Leu Arg Leu
    370                 375                 380

His Pro Pro Ile Pro Leu Leu Leu His Glu Thr Ala Glu Glu Ser Thr
385                 390                 395                 400

Val Ser Gly Tyr His Ile Pro Ala Lys Ser His Val Ile Ile Asn Ser
                405                 410                 415

Phe Ala Ile Gly Arg Asp Lys Asn Ser Trp Glu Asp Pro Glu Thr Tyr
            420                 425                 430

Lys Pro Ser Arg Phe Leu Lys Glu Gly Val Pro Asp Phe Lys Gly Gly
        435                 440                 445

Asn Phe Glu Phe Ile Pro Phe Gly Ser Gly Arg Arg Ser Cys Pro Gly
    450                 455                 460

Met Gln Leu Gly Leu Tyr Ala Leu Glu Met Ala Val Ala His Leu Leu
465                 470                 475                 480

His Cys Phe Thr Trp Glu Leu Pro Asp Gly Met Lys Pro Ser Glu Leu
                485                 490                 495

Lys Met Asp Asp Ile Phe Gly Leu Thr Ala Pro Arg Ala Asn Arg Leu
            500                 505                 510

Val Ala Val Pro Thr Pro Arg Leu Leu Cys Pro Leu Tyr
        515                 520                 525

<210>  189
<211>  1620
<212>  DNA
<213>  Nicotiana tabacum

<400> 189
gggggataag aatatggaga taccatatta cagcttaaaa cttacaattt tttcatttgc 60
aattatcttt gtactaagat gggcatggaa aatcttgaat tatgtgtggt taaaaccaaa 120
agaattggag aaatgcatca gacagcaggg tttcaaagga aactcttaca aattcttgtt 180
tggggatatg aaagagataa agaaaatggg tgaagaagct atgtctaagc caatcaattt 240
ctctcatgac atgatttggc ctagagtcat gcccttcatc cacaaaacca tcaccaatta 300
tggtaagaat tgttttgtgt ggtttgggcc aagaccagca gtcctgatca cagacccgga 360
acttgtaaag gaggtgctaa cgaagaattt cgtttatcag aagccacctg gcactccact 420
cacaaaattg gcagcaactg gaattgcagg ctatgaaaca gataaatggg ctacacatag 480
aaggcttctc aatcctgctt ttcaccttga caagttgaag catatgctac ctgcattcca 540
atttactgct tgtgagatgt tgagcaaatt ggagaaagtt gtctcaccaa atggaacaga 600
gatagatgtg tggccatatc tacaaacttt aacaagtgat gccatttcaa gaactgcttt 660
tggcagtagt tatgaagaag gaagaaagct tttgaacttt caaaaggaac aactttcact 720
aattctagaa gtgtcccgca caatatacat cccaggatgg aggtttttgc caacaaaaag 780
gaacaaaagg atgaagcaaa tatttaatga agtacgagcg ctggtattgg gaattattaa 840
gaaaagattg agtatgattg aaaatggaga agctcctgat gatttattgg gtatattatt 900
ggcatccaat ttaaaagaaa tccaacaaca tggaaataac aagaaatttg gtatgagtat 960
tgatgaggtg attgaagagt gtaaactctt ctattttgcg gggcaagaga caacttcatc 1020
tttacttgta tggactatga ttttgttgtg caaacatcct agttggcaag ataaagctag 1080
agaagaggtt ttgcaagtgt ttggaagtag ggaagttgac tatgacaagt tgaatcagct 1140
aaaaatagta actatgatct taaacgaggt cttaaggttg tatccagcag gatatgcgat 1200
taatcgaatg gtaaccaaag aaacaaagtt agggaattta tgtttaccag ctggggtaca 1260
actcttgtta ccaacaattt tgttgcaaca tgatactgaa atatggggag atgatgcaat 1320
ggagttcaat ccagagagat ttagtgatgg aatatccaaa gcaacaaaag gaaaacttgt 1380
gttctttcca tttagttggg gtccaagaat atgtattggg caaaattttg ctatgttaga 1440
ggccaagatg gcaatggcta tgattctgaa aaactatgca tttgaactct ctccatctta 1500
tgctcatgct cctcatccac tactacttca acctcaatat ggtgctcaat taattttgta 1560
caagttgtag aaatggtcaa tttggaactt gttatggaac ttttatcatc gtaatcaacc 1620

<210> 190
<211> 518
<212> PRT
<213> Nicotiana tabacum

<400> 190

Met Glu Ile Pro Tyr Tyr Ser Leu Lys Leu Thr Ile Phe Ser Phe Ala
1               5                   10                  15

Ile Ile Phe Val Leu Arg Trp Ala Trp Lys Ile Leu Asn Tyr Val Trp
            20                  25                  30

Leu Lys Pro Lys Glu Leu Glu Lys Cys Ile Arg Gln Gln Gly Phe Lys
        35                  40                  45

Gly Asn Ser Tyr Lys Phe Leu Phe Gly Asp Met Lys Glu Ile Lys Lys
    50                  55                  60

Met Gly Glu Glu Ala Met Ser Lys Pro Ile Asn Phe Ser His Asp Met
65                  70                  75                  80

Ile Trp Pro Arg Val Met Pro Phe Ile His Lys Thr Ile Thr Asn Tyr
                85                  90                  95

Gly Lys Asn Cys Phe Val Trp Phe Gly Pro Arg Pro Ala Val Leu Ile
            100                 105                 110

Thr Asp Pro Glu Leu Val Lys Glu Val Leu Thr Lys Asn Phe Val Tyr
        115                 120                 125

Gln Lys Pro Pro Gly Thr Pro Leu Thr Lys Leu Ala Ala Thr Gly Ile
    130                 135                 140

Ala Gly Tyr Glu Thr Asp Lys Trp Ala Thr His Arg Arg Leu Leu Asn
145                 150                 155                 160

Pro Ala Phe His Leu Asp Lys Leu Lys His Met Leu Pro Ala Phe Gln
                165                 170                 175

Phe Thr Ala Cys Glu Met Leu Ser Lys Leu Glu Lys Val Val Ser Pro
            180                 185                 190

Asn Gly Thr Glu Ile Asp Val Trp Pro Tyr Leu Gln Thr Leu Thr Ser
        195                 200                 205

Asp Ala Ile Ser Arg Thr Ala Phe Gly Ser Ser Tyr Glu Glu Gly Arg
    210                 215                 220

Lys Leu Phe Glu Leu Gln Lys Glu Gln Leu Ser Leu Ile Leu Glu Val
225                 230                 235                 240

Ser Arg Thr Ile Tyr Ile Pro Gly Trp Arg Phe Leu Pro Thr Lys Arg
                245                 250                 255

Asn Lys Arg Met Lys Gln Ile Phe Asn Glu Val Arg Ala Leu Val Leu
            260                 265                 270

Gly Ile Ile Lys Lys Arg Leu Ser Met Ile Glu Asn Gly Glu Ala Pro
        275                 280                 285

Asp Asp Leu Leu Gly Ile Leu Leu Ala Ser Asn Leu Lys Glu Ile Gln
    290                 295                 300

Gln His Gly Asn Asn Lys Lys Phe Gly Met Ser Ile Asp Glu Val Ile
305                 310                 315                 320

Glu Glu Cys Lys Leu Phe Tyr Phe Ala Gly Gln Glu Thr Thr Ser Ser
                325                 330                 335

Leu Leu Val Trp Thr Met Ile Leu Leu Cys Lys His Pro Ser Trp Gln
            340                 345                 350

Asp Lys Ala Arg Glu Glu Val Leu Gln Val Phe Gly Ser Arg Glu Val
        355                 360                 365

Asp Tyr Asp Lys Leu Asn Gln Leu Lys Ile Val Thr Met Ile Leu Asn
    370                 375                 380

Glu Val Leu Arg Leu Tyr Pro Ala Gly Tyr Ala Ile Asn Arg Met Val
385                 390                 395                 400

Thr Lys Glu Thr Lys Leu Gly Asn Leu Cys Leu Pro Ala Gly Val Gln
                405                 410                 415

Leu Leu Leu Pro Thr Ile Leu Leu Gln His Asp Thr Glu Ile Trp Gly
            420                 425                 430

Asp Asp Ala Met Glu Phe Asn Pro Glu Arg Phe Ser Asp Gly Ile Ser
        435                 440                 445

Lys Ala Thr Lys Gly Lys Leu Val Phe Phe Pro Phe Ser Trp Gly Pro
    450                 455                 460

Arg Ile Cys Ile Gly Gln Asn Phe Ala Met Leu Glu Ala Lys Met Ala
465                 470                 475                 480

Met Ala Met Ile Leu Lys Asn Tyr Ala Phe Glu Leu Ser Pro Ser Tyr
                485                 490                 495

Ala His Ala Pro His Pro Leu Leu Leu Gln Pro Gln Tyr Gly Ala Gln
            500                 505                 510

Leu Ile Leu Tyr Lys Leu
        515

<210>  191
<211>  1566
<212>  DNA
<213>  Nicotiana tabacum

<400>  191
gtcctaacta aaaatggaga ttcagttttc taacttagtt gcattcttgc tctttctctc      60

cagcatcttt cttctattca aaaaatggaa aaccagaaaa ctaaatttgc ctcctggtcc 120
atggaaatta ccttttattg gaagtttaca ccatttggct gtggcaggtc cacttcctca 180
ccatggccta aaaaatttag ccaaacgcta tggtcctctt atgcatttac aacttggaca 240
aattcctaca ctcatcatat catcacctca aatggcaaaa gaagtactaa aaactcacga 300
cctcgctttt gccactagac caaagcttgt cgtggccgac atcattcact acgacagcac 360
ggacatagca ttttctccgt acggtgaata ctggagacaa attcgtaaaa tttgcatatt 420
ggaactcttg agtgccaaga tggtcaaatt ttttagctcg attcgccaag atgagctctc 480
gaagatgctc tcatctatac gaacgacacc caatcttaca gtcaatctta ctgacaaaat 540
tttttggttt acgagttcgg taacttgtag atcagcttta gggaagatat gtggtgacca 600
agacaaattg atcattttta tgagggaaat aatatcattg gcaggtggat ttagtattgc 660
tgattttttc cctacatgga aaatgattca tgatattgat ggttcgaaat ctaaactggt 720
gaaagcacat cgtaagattg atgaaatttt gggaaatgtt gttgatgagc acaaaaagaa 780
cagagcagat ggcaagaagg gtaatggtga atttggtggt gaagatttga ttgatgtatt 840
gttaagagtt agagaaagtg gagaagttca aattcctatc acaaatgaca atatcaaatc 900
aatattaatc gacatgttct ctgcgggatc tgaaacatca tcgacgacta taatttgggc 960
attagctgaa atgatgaaga aaccaagtgt tttagcaaag gcacaagctg aagtaaggca 1020
agctttgaag gagaaaaaag gttttcaaca gattgatctt gatgagctaa aatatctcaa 1080
gttagtaatc aaagaaacct taagaatgca ccctccaatt cctctattag ttcctagaga 1140
atgtatggag gatacaaaga ttgatggtta caatatacct ttcaaaacaa gagtcatagt 1200
taatgcatgg gcaatcggac gagatccaga aagttgggat gaccccgaaa gctttatgcc 1260
agagagattt gagaatagtt ctattgactt tcttggaaat catcatcagt ttataccatt 1320
tggtgcagga agaaggattt gtccgggaat gctatttggt ttagctaatg ttggacaacc 1380
tttagctcag ttactttatc acttcgattg gaaactccct aatggacaaa gtcatgagaa 1440
tttcgacatg actgagtcac ctggaatttc tgctacaaga aaggatgatc ttgttttgat 1500
tgccactcct tatgattctt attaagcagt agcagaaata aaaagccggg gcaaacagaa 1560
aaaagt 1566

<210> 192
<211> 503
<212> PRT
<213> Nicotiana tabacum

<400> 192

Met Glu Ile Gln Phe Ser Asn Leu Val Ala Phe Leu Leu Phe Leu Ser
1               5                   10                  15

Ser Ile Phe Leu Leu Phe Lys Lys Trp Lys Thr Arg Lys Leu Asn Leu
            20                  25                  30

Pro Pro Gly Pro Trp Lys Leu Pro Phe Ile Gly Ser Leu His His Leu
        35                  40                  45

Ala Val Ala Gly Pro Leu Pro His His Gly Leu Lys Asn Leu Ala Lys
    50                  55                  60

Arg Tyr Gly Pro Leu Met His Leu Gln Leu Gly Gln Ile Pro Thr Leu
65                  70                  75                  80

Ile Ile Ser Ser Pro Gln Met Ala Lys Glu Val Leu Lys Thr His Asp
                85                  90                  95

Leu Ala Phe Ala Thr Arg Pro Lys Leu Val Val Ala Asp Ile Ile His
            100                 105                 110

Tyr Asp Ser Thr Asp Ile Ala Phe Ser Pro Tyr Gly Glu Tyr Trp Arg
        115                 120                 125

Gln Ile Arg Lys Ile Cys Ile Leu Glu Leu Leu Ser Ala Lys Met Val
    130                 135                 140

Lys Phe Phe Ser Ser Ile Arg Gln Asp Glu Leu Ser Lys Met Leu Ser
145                 150                 155                 160

Ser Ile Arg Thr Thr Pro Asn Leu Thr Val Asn Leu Thr Asp Lys Ile
                165                 170                 175

Phe Trp Phe Thr Ser Ser Val Thr Cys Arg Ser Ala Leu Gly Lys Ile
            180                 185                 190

Cys Gly Asp Gln Asp Lys Leu Ile Ile Phe Met Arg Glu Ile Ile Ser
        195                 200                 205

Leu Ala Gly Gly Phe Ser Ile Ala Asp Phe Phe Pro Thr Trp Lys Met
    210                 215                 220

Ile His Asp Ile Asp Gly Ser Lys Ser Lys Leu Val Lys Ala His Arg
225                 230                 235                 240

Lys Ile Asp Glu Ile Leu Gly Asn Val Val Asp Glu His Lys Lys Asn
                245                 250                 255

Arg Ala Asp Gly Lys Lys Gly Asn Gly Glu Phe Gly Gly Glu Asp Leu
            260                 265                 270

Ile Asp Val Leu Leu Arg Val Arg Glu Ser Gly Glu Val Gln Ile Pro
        275                 280                 285

Ile Thr Asn Asp Asn Ile Lys Ser Ile Leu Ile Asp Met Phe Ser Ala
    290                 295                 300

Gly Ser Glu Thr Ser Ser Thr Thr Ile Ile Trp Ala Leu Ala Glu Met
305                 310                 315                 320

Met Lys Lys Pro Ser Val Leu Ala Lys Ala Gln Ala Glu Val Arg Gln
                325                 330                 335

Ala Leu Lys Glu Lys Lys Gly Phe Gln Gln Ile Asp Leu Asp Glu Leu
            340                 345                 350

Lys Tyr Leu Lys Leu Val Ile Lys Glu Thr Leu Arg Met His Pro Pro
        355                 360                 365

Ile Pro Leu Leu Val Pro Arg Glu Cys Met Glu Asp Thr Lys Ile Asp
    370                 375                 380

Gly Tyr Asn Ile Pro Phe Lys Thr Arg Val Ile Val Asn Ala Trp Ala
385                 390                 395                 400

Ile Gly Arg Asp Pro Glu Ser Trp Asp Asp Pro Glu Ser Phe Met Pro
                405                 410                 415

Glu Arg Phe Glu Asn Ser Ser Ile Asp Phe Leu Gly Asn His His Gln
            420                 425                 430

Phe Ile Pro Phe Gly Ala Gly Arg Arg Ile Cys Pro Gly Met Leu Phe
        435                 440                 445

Gly Leu Ala Asn Val Gly Gln Pro Leu Ala Gln Leu Leu Tyr His Phe
    450                 455                 460

Asp Trp Lys Leu Pro Asn Gly Gln Ser His Glu Asn Phe Asp Met Thr
465                 470                 475                 480

Glu Ser Pro Gly Ile Ser Ala Thr Arg Lys Asp Asp Leu Val Leu Ile
                485                 490                 495

Ala Thr Pro Tyr Asp Ser Tyr
            500

<210>  193
<211>  1652
<212>  DNA
<213>  Nicotiana tabacum

<400>  193
caactaacaa acacattgag tcctctccca aatcactgat tcaccaccaa aagtaccaac      60

aattcaatgg aaggtacaaa cttgactaca tatgcagcag tatttcttga tactctgttt     120

cttttgttcc tttccaaact tcttcgccag aggaaactca atttacctcc aggcccaaaa     180

ccatggccga tcatcggaaa cttaaacctt attggcaatc ttcctcatcg ctcaatccac     240

gaactctccc tcaagtacgg acccgttatg caactccaat tcgggtcttt ccccgttgta     300

gttggatcct ccgtcgaaat ggctaagatt ttcctcaaat ccatggatat taactttgta 360
ggcaggccta aaacggctgc cggaaaatac acaacgtaca attattccga tattacatgg 420
tctccttacg gaccatattg gcgccaggca cgtaggatgt gcctaacgga attattcagc 480
acgaaacgtc tcgattcata cgagtatatt cgggctgagg agttgcattc tcttctccat 540
aatttgaaca aaatatcagg gaaaccaatt gtgttgaaag attattcgac gacgttgagt 600
ttaaatgtta ttagcaggat ggtactgggg aaaaggtatt tggacgaatc cgagaactcg 660
ttcgtgaatc ctgaggaatt taagaagatg ttggacgaat tgtttttgct aaatggtgta 720
cttaatattg gagattcaat tccatggatt gatttcatgg atttgcaagg ttatgttaag 780
aggatgaaag tagtgagcaa gaaattcgac aagtttttag agcatgttat tgatgagcat 840
aacattagga gaaatggagt ggagaattat gttgctaagg atatggtgga tgttttgttg 900
cagctcgctg atgatccgaa gttggaagtt aagctggaga gacatggagt caaagcattc 960
actcaggata tgctggctgg tggaaccgag agttcagcag tgacagtgga gtgggcaatt 1020
tcagagctgc taaagaagcc ggagattttc aaaaaggcta cagaagaatt ggatcgagta 1080
attgggcaga atagatgggt acaagaaaag gacattccaa atcttcctta catagaggca 1140
atagtcaaag agactatgcg actgcacccc gtggcaccaa tgttggtgcc acgtgagtgt 1200
cgagaagata ttaaggtagc aggctacgac gttcagaaag gaactagggt tctcgtgagt 1260
gtatggacta ttggaagaga ccctacattg tgggacgagc ctgaggtgtt caagccggag 1320
agattccatg aaaggtccat agatgttaaa ggacatgatt atgagctttt gccatttgga 1380
gcggggagaa gaatgtgccc gggttatagc ttggggctca aggtgattca agctagctta 1440
gctaatcttc tacatggatt taactggtca ttgcctgata atatgactcc tgaggacctc 1500
aacatggatg agatttttgg gctctctaca cctaaaaaat ttccacttgc tactgtgatt 1560
gagccaagac tttcaccaaa actttactct gtttgattca gcagttctat ggttccgtca 1620
agatagactt tgttacgttt gaacctgtgc tc 1652

<210> 194
<211> 509
<212> PRT
<213> Nicotiana tabacum

<400> 194

Met Glu Gly Thr Asn Leu Thr Thr Tyr Ala Ala Val Phe Leu Asp Thr
1               5                   10                  15

Leu Phe Leu Leu Phe Leu Ser Lys Leu Leu Arg Gln Arg Lys Leu Asn
            20                  25                  30

Leu Pro Pro Gly Pro Lys Pro Trp Pro Ile Ile Gly Asn Leu Asn Leu
        35                  40                  45

Ile Gly Asn Leu Pro His Arg Ser Ile His Glu Leu Ser Leu Lys Tyr
    50                  55                  60

Gly Pro Val Met Gln Leu Gln Phe Gly Ser Phe Pro Val Val Val Gly
65                  70                  75                  80

Ser Ser Val Glu Met Ala Lys Ile Phe Leu Lys Ser Met Asp Ile Asn
                85                  90                  95

Phe Val Gly Arg Pro Lys Thr Ala Ala Gly Lys Tyr Thr Thr Tyr Asn
            100                 105                 110

Tyr Ser Asp Ile Thr Trp Ser Pro Tyr Gly Pro Tyr Trp Arg Gln Ala
        115                 120                 125

Arg Arg Met Cys Leu Thr Glu Leu Phe Ser Thr Lys Arg Leu Asp Ser
    130                 135                 140

Tyr Glu Tyr Ile Arg Ala Glu Glu Leu His Ser Leu Leu His Asn Leu
145                 150                 155                 160

Asn Lys Ile Ser Gly Lys Pro Ile Val Leu Lys Asp Tyr Ser Thr Thr
                165                 170                 175

Leu Ser Leu Asn Val Ile Ser Arg Met Val Leu Gly Lys Arg Tyr Leu
            180                 185                 190

Asp Glu Ser Glu Asn Ser Phe Val Asn Pro Glu Glu Phe Lys Lys Met
        195                 200                 205

Leu Asp Glu Leu Phe Leu Leu Asn Gly Val Leu Asn Ile Gly Asp Ser
    210                 215                 220

Ile Pro Trp Ile Asp Phe Met Asp Leu Gln Gly Tyr Val Lys Arg Met
225                 230                 235                 240

Lys Val Val Ser Lys Lys Phe Asp Lys Phe Leu Glu His Val Ile Asp
                245                 250                 255

Glu His Asn Ile Arg Arg Asn Gly Val Glu Asn Tyr Val Ala Lys Asp
            260                 265                 270

Met Val Asp Val Leu Leu Gln Leu Ala Asp Asp Pro Lys Leu Glu Val
        275                 280                 285

Lys Leu Glu Arg His Gly Val Lys Ala Phe Thr Gln Asp Met Leu Ala
    290                 295                 300

Gly Gly Thr Glu Ser Ser Ala Val Thr Val Glu Trp Ala Ile Ser Glu
305                 310                 315                 320

Leu Leu Lys Lys Pro Glu Ile Phe Lys Lys Ala Thr Glu Glu Leu Asp
                325                 330                 335

Arg Val Ile Gly Gln Asn Arg Trp Val Gln Glu Lys Asp Ile Pro Asn
            340                 345                 350

Leu Pro Tyr Ile Glu Ala Ile Val Lys Glu Thr Met Arg Leu His Pro
        355                 360                 365

Val Ala Pro Met Leu Val Pro Arg Glu Cys Arg Glu Asp Ile Lys Val
    370                 375                 380

Ala Gly Tyr Asp Val Gln Lys Gly Thr Arg Val Leu Val Ser Val Trp
385                 390                 395                 400

Thr Ile Gly Arg Asp Pro Thr Leu Trp Asp Glu Pro Glu Val Phe Lys
                405                 410                 415

Pro Glu Arg Phe His Glu Arg Ser Ile Asp Val Lys Gly His Asp Tyr
            420                 425                 430

Glu Leu Leu Pro Phe Gly Ala Gly Arg Arg Met Cys Pro Gly Tyr Ser
        435                 440                 445

Leu Gly Leu Lys Val Ile Gln Ala Ser Leu Ala Asn Leu Leu His Gly
    450                 455                 460

Phe Asn Trp Ser Leu Pro Asp Asn Met Thr Pro Glu Asp Leu Asn Met
465                 470                 475                 480

Asp Glu Ile Phe Gly Leu Ser Thr Pro Lys Lys Phe Pro Leu Ala Thr
                485                 490                 495

Val Ile Glu Pro Arg Leu Ser Pro Lys Leu Tyr Ser Val
            500                 505

<210> 195
<211> 1657
<212> DNA
<213> Nicotiana tabacum

<400> 195
   cttcttcctt cctaactaaa aatggagatt cagttttcta acttagttgc attcttgctc 60
   tttctctcca gcatctttct tgtattcaaa aaatggaaaa ccagaaaact aaatttgcct 120
   cctggtccat ggaaattacc ttttattgga agtttacacc atttggctgt ggcaggtcca 180
   cttcctcacc atggcctaaa aaatttagcc aaacgctatg gtcctcttat gcatttacaa 240
   cttggacaaa ttcctacact cgtcatatca tcacctcaaa tggcaaaaga agtactaaaa 300
   actcacgacc tcgcttttgc cactagacca aagcttgtcg tggccgacat cattcactac 360
   gacagcacgg acatagcatt ttcgccatac ggtgaatact ggagacaaat tcgtaaaatt 420
   tgcatattgg aactcttgag tgccaagatg gtcaagtttt ttagctcgat tcgccaagat 480
   gagctctcga agatggtttc atctatacga acgacgccca atcttccagt caatcttacc 540
   gacaagattt tttggtttac gagttcggta atttgtagat cagctttagg gaagatatgt 600
   ggtgaccaag acaaattgat catttttatg agggaaataa tatcattggc aggtggattt 660
   agtattgctg attttttccc tacatggaaa atgattcatg atattgatgg ttcaaaatct 720
   aaactggtga aggcacatcg taagattgat gaaattttgg aaaatgtggt aaatgagcac 780
   aaacagaatc gagcagatgg taaaaagggt aatggtgaat ttggtggaga agatctgatt 840
   gatgttttgt taagagttag agaaagtgga gaagttcaaa ttccaatcac agatgacaat 900
   atcaaatcaa tattaatcga catgttctct gccggatcgg aaacatcatc gacaactata 960
   atttgggcat tagctgaaat gatgaagaaa ccaagtgttt tagcaaaggc acaagctgaa 1020
   gtgaggcaag ctttgaaggg gaagaaaatt agttttcaag agattgatat tgataagcta 1080
   aagtatttga agttagtgat caaagaaact ttaagaatgc accctccaat tcctctgtta 1140
   gtccctagag aatgtatgga agatacaaag attgatggtt acaatatacc tttcaaaaca 1200
   agagtcattg ttaatgcatg ggcaattgga cgagatcctc aaagttggga tgatcctgaa 1260
   agctttacgc cagagagatt tgagaataat tctattgatt ttcttggaaa tcatcatcaa 1320
   tttattccat ttggtgcagg aagaaggatt tgtcctggaa tgctatttgg tttagctaat 1380
   gttggacaac ctttagctca gttactttat cacttcgatt ggaaactccc taatggacaa 1440
   agtcatgaga atttcgacat gactgagtca cctggaattt ctgctacaag aaaggatgat 1500
   cttgttttga ttgccactcc ttatgattct tattaagcag tagcagaaat aaaaagccgg 1560
   ggcaaacaga aaaaagtatt gctgcttcta ggtattttct attggataaa tttcaaaatt 1620
   catccacaat atttagtgtt tgctagagtt ggttagc 1657

<210> 196
<211> 504
<212> PRT
<213> Nicotiana tabacum

<400> 196

Met Glu Ile Gln Phe Ser Asn Leu Val Ala Phe Leu Leu Phe Leu Ser
1               5                   10                  15

Ser Ile Phe Leu Val Phe Lys Lys Trp Lys Thr Arg Lys Leu Asn Leu
            20                  25                  30

Pro Pro Gly Pro Trp Lys Leu Pro Phe Ile Gly Ser Leu His His Leu
        35                  40                  45

Ala Val Ala Gly Pro Leu Pro His His Gly Leu Lys Asn Leu Ala Lys
    50                  55                  60

Arg Tyr Gly Pro Leu Met His Leu Gln Leu Gly Gln Ile Pro Thr Leu
65                  70                  75                  80

Val Ile Ser Ser Pro Gln Met Ala Lys Glu Val Leu Lys Thr His Asp
                85                  90                  95

Leu Ala Phe Ala Thr Arg Pro Lys Leu Val Val Ala Asp Ile Ile His
            100                 105                 110

Tyr Asp Ser Thr Asp Ile Ala Phe Ser Pro Tyr Gly Glu Tyr Trp Arg
        115                 120                 125

Gln Ile Arg Lys Ile Cys Ile Leu Glu Leu Leu Ser Ala Lys Met Val
    130                 135                 140

Lys Phe Phe Ser Ser Ile Arg Gln Asp Glu Leu Ser Lys Met Val Ser
145                 150                 155                 160

Ser Ile Arg Thr Thr Pro Asn Leu Pro Val Asn Leu Thr Asp Lys Ile
                165                 170                 175

Phe Trp Phe Thr Ser Ser Val Ile Cys Arg Ser Ala Leu Gly Lys Ile
            180                 185                 190

Cys Gly Asp Gln Asp Lys Leu Ile Ile Phe Met Arg Glu Ile Ile Ser
        195                 200                 205

Leu Ala Gly Gly Phe Ser Ile Ala Asp Phe Phe Pro Thr Trp Lys Met
    210                 215                 220

Ile His Asp Ile Asp Gly Ser Lys Ser Lys Leu Val Lys Ala His Arg
225                 230                 235                 240

Lys Ile Asp Glu Ile Leu Glu Asn Val Val Asn Glu His Lys Gln Asn
                245                 250                 255

Arg Ala Asp Gly Lys Lys Gly Asn Gly Glu Phe Gly Gly Glu Asp Leu
            260                 265                 270

Ile Asp Val Leu Leu Arg Val Arg Glu Ser Gly Glu Val Gln Ile Pro
        275                 280                 285

Ile Thr Asp Asp Asn Ile Lys Ser Ile Leu Ile Asp Met Phe Ser Ala
    290                 295                 300

Gly Ser Glu Thr Ser Ser Thr Thr Ile Ile Trp Ala Leu Ala Glu Met
305                 310                 315                 320

Met Lys Lys Pro Ser Val Leu Ala Lys Ala Gln Ala Glu Val Arg Gln
                325                 330                 335

Ala Leu Lys Gly Lys Lys Ile Ser Phe Gln Glu Ile Asp Ile Asp Lys
            340                 345                 350

Leu Lys Tyr Leu Lys Leu Val Ile Lys Glu Thr Leu Arg Met His Pro
        355                 360                 365

Pro Ile Pro Leu Leu Val Pro Arg Glu Cys Met Glu Asp Thr Lys Ile
    370                 375                 380

Asp Gly Tyr Asn Ile Pro Phe Lys Thr Arg Val Ile Val Asn Ala Trp
385                 390                 395                 400

Ala Ile Gly Arg Asp Pro Gln Ser Trp Asp Asp Pro Glu Ser Phe Thr
                405                 410                 415

Pro Glu Arg Phe Glu Asn Asn Ser Ile Asp Phe Leu Gly Asn His His
            420                 425                 430

Gln Phe Ile Pro Phe Gly Ala Gly Arg Arg Ile Cys Pro Gly Met Leu
        435                 440                 445

Phe Gly Leu Ala Asn Val Gly Gln Pro Leu Ala Gln Leu Leu Tyr His
    450                 455                 460

Phe Asp Trp Lys Leu Pro Asn Gly Gln Ser His Glu Asn Phe Asp Met
465                 470                 475                 480

Thr Glu Ser Pro Gly Ile Ser Ala Thr Arg Lys Asp Asp Leu Val Leu
                485                 490                 495

Ile Ala Thr Pro Tyr Asp Ser Tyr
            500

<210>  197
<211>  1610
<212>  DNA
<213>  Nicotiana tabacum

<400>  197
cttcttcctt cctaactaaa aatggagatt cagttttcta acttagttgc attcttgctc      60

tttctctcca gcatctttct tgtattcaaa aaatggaaaa ccagaaaact aaatttgcct     120

cctggtccat ggaaattacc ttttattgga agtttacacc atttggctgt ggcaggtcca     180

cttcctcacc atggcctaaa aaatttagcc aaacgctatg gtcctcttat gcatttacaa     240

cttggacaaa ttcctacact cgtcatatca tcacctcaaa tggcaaaaga agtactaaaa     300

actcacgacc tcgcttttgc cactagacca aagcttgtcg tggccgacat cattcactac     360

gacagcacgg acatagcact ttcgccatac ggtgaatact ggagacaaat tcgtaaaatt     420

tgcatattgg aactcttgag tgccaagatg gtcaagtttt ttagctcgat tcgccaagat     480

gagctctcga agatggtttc atctatacga acgacgccca atcttccagt caatcttacc     540

gacaagattt tttggtttac gagttcggta atttgtagat cagctttagg gaagatatgt     600

ggtgaccaag acaaattgat catttttatg agggaaataa tatcattggc aggtggattt     660

agtattgctg attttttccc tacatggaaa atgattcatg atattgatgg ttcaaaatct 720
aaactggtga aggcacatcg taagattgat gaaattttgg aaaatgtggt aaatgagcac 780
aaacagaatc gagcagatgg taaaaagggt aatggtgaat ttggtggaga agatctgatt 840
gatgttttgt taagagttag agaaagtgga gaagttcaaa ttccaatcac agatgacaat 900
atcaaatcaa tattaatcga catgttctct gccggatcgg aaacatcatc gacaactata 960
atttgggcat tagctgaaat gatgaagaaa ccaagtgttt tagcaaaggc acaagctgaa 1020
gtgagccaag ctttgaaggg gaagaaaatt agttttcaag agattgatat tgataagcta 1080
aagtatttga agttagtgat caaagaaact ttaagaatgc accctccaat tcctctgtta 1140
gtccctagag aatgtatgga agatacaaag attgatggtt acaatatacc tttcaaaaca 1200
agagtcattg ttaatgcatg ggcaattgga cgagatcctc aaagttggga tgatcctgaa 1260
agctttacgc cagagagatt tgagaataat tctattgatt ttcttggaaa tcatcatcaa 1320
tttattccat ttggtgcagg aagaaggatt tgtcctggaa tgctatttgg tttagctaat 1380
gttggacaac ctttagctca gttactttat cacttcgatt ggaaactccc taatggacaa 1440
actcaccaaa atttcgacat gactgagtca cctggaattt ctgctacaag aaaggatgat 1500
cttattttga ttgccactcc tgctcattct tgattaagta ttgctgcttt tctattggag 1560
aattttcaaa attcatccac aatatatagt gtttgctaga gttggttagc 1610

<210> 198
<211> 503
<212> PRT
<213> Nicotiana tabacum

<400> 198

Met Glu Ile Gln Phe Ser Asn Leu Val Ala Phe Leu Leu Phe Leu Ser
1               5                   10                  15

Ser Ile Phe Leu Val Phe Lys Lys Trp Lys Thr Arg Lys Leu Asn Leu
            20                  25                  30

Pro Pro Gly Pro Trp Lys Leu Pro Phe Ile Gly Ser Leu His His Leu
        35                  40                  45

Ala Val Ala Gly Pro Leu Pro His His Gly Leu Lys Asn Leu Ala Lys
    50                  55                  60

Arg Tyr Gly Pro Leu Met His Leu Gln Leu Gly Gln Ile Pro Thr Leu
65                  70                  75                  80

Val Ile Ser Ser Pro Gln Met Ala Lys Glu Val Leu Lys Thr His Asp
                85                  90                  95

Leu Ala Phe Ala Thr Arg Pro Lys Leu Val Val Ala Asp Ile Ile His
            100                 105                 110

Tyr Asp Ser Thr Asp Ile Ala Leu Ser Pro Tyr Gly Glu Tyr Trp Arg
        115                 120                 125

Gln Ile Arg Lys Ile Cys Ile Leu Glu Leu Leu Ser Ala Lys Met Val
    130                 135                 140

Lys Phe Phe Ser Ser Ile Arg Gln Asp Glu Leu Ser Lys Met Val Ser
145                 150                 155                 160

Ser Ile Arg Thr Thr Pro Asn Leu Pro Val Asn Leu Thr Asp Lys Ile
                165                 170                 175

Phe Trp Phe Thr Ser Ser Val Ile Cys Arg Ser Ala Leu Gly Lys Ile
            180                 185                 190

Cys Gly Asp Gln Asp Lys Leu Ile Ile Phe Met Arg Glu Ile Ile Ser
        195                 200                 205

Leu Ala Gly Gly Phe Ser Ile Ala Asp Phe Phe Pro Thr Trp Lys Met
    210                 215                 220

Ile His Asp Ile Asp Gly Ser Lys Ser Lys Leu Val Lys Ala His Arg
225                 230                 235                 240

Lys Ile Asp Glu Ile Leu Glu Asn Val Val Asn Glu His Lys Gln Asn
                245                 250                 255

Arg Ala Asp Gly Lys Lys Gly Asn Gly Glu Phe Gly Gly Glu Asp Leu
            260                 265                 270

Ile Asp Val Leu Leu Arg Val Arg Glu Ser Gly Glu Val Gln Ile Pro
        275                 280                 285

Ile Thr Asp Asp Asn Ile Lys Ser Ile Leu Ile Asp Met Phe Ser Ala
    290                 295                 300

Gly Ser Glu Thr Ser Ser Thr Thr Ile Ile Trp Ala Leu Ala Glu Met
305                 310                 315                 320

Met Lys Lys Pro Ser Val Leu Ala Lys Ala Gln Ala Glu Val Ser Gln
                325                 330                 335

Ala Leu Lys Gly Lys Lys Ile Ser Phe Gln Glu Ile Asp Ile Asp Lys
            340                 345                 350

Leu Lys Tyr Leu Lys Leu Val Ile Lys Glu Thr Leu Arg Met His Pro
        355                 360                 365

Pro Ile Pro Leu Leu Val Pro Arg Glu Cys Met Glu Asp Thr Lys Ile
    370                 375                 380

Asp Gly Tyr Asn Ile Pro Phe Lys Thr Arg Val Ile Val Asn Ala Trp
385                 390                 395                 400

Ala Ile Gly Arg Asp Pro Gln Ser Trp Asp Asp Pro Glu Ser Phe Thr
                405                 410                 415

Pro Glu Arg Phe Glu Asn Asn Ser Ile Asp Phe Leu Gly Asn His His
            420                 425                 430

Gln Phe Ile Pro Phe Gly Ala Gly Arg Arg Ile Cys Pro Gly Met Leu
        435                 440                 445

Phe Gly Leu Ala Asn Val Gly Gln Pro Leu Ala Gln Leu Leu Tyr His
    450                 455                 460

Phe Asp Trp Lys Leu Pro Asn Gly Gln Thr His Gln Asn Phe Asp Met
465                 470                 475                 480

Thr Glu Ser Pro Gly Ile Ser Ala Thr Arg Lys Asp Asp Leu Ile Leu
                485                 490                 495

Ile Ala Thr Pro Ala His Ser
            500

<210>  199
<211>  1664
<212>  DNA
<213>  Nicotiana tabacum

<400>  199
atccttcttc cttcctaggt cctaactaaa aatggagatt cagttttcta acttagttgc      60

attcttgctc tttctctcca gcatctttct tctattcaaa aaatggaaaa ccagaaaact     120

aaatttgcct cctggtccat ggaaattacc ttttattgga agtttacacc atttggctgt     180

ggcaggtcca cttcctcacc atggcctaaa aaatttagcc aaacgctatg gtcctcttat     240

gcatttacaa cttggacaaa ttcctacact catcatatca tcacctcaaa tggcaaaaga     300

agtactaaaa actcacgacc tcgcttttgc cactagacca aagcttgtcg tggccgacat     360

cattcactac gacagcacgg acatagcatt ttctccgtac ggtgaatact ggagacaaat     420

tcgtaaaatt tgcatattgg aactcttgag tgccaagatg gtcaaatttt ttagctcgat     480

tcgccaagat gagctctcga agatgctctc atctatacga acgacaccca atcttacagt     540

caatcttact gacaaaattt tttggtttac gagttcggta acttgtagat cagctttagg     600

gaagatatgt ggtgaccaag acaaattgat catttttatg agggaaataa tatcattggc     660

aggtggattt agtattgctg atttttttcc tacatggaaa atgattcatg atattgatgg     720

ttcgaaatct aaactggtga aagcacatcg taagattgat gaaattttgg gaaatgttgt     780

tgatgagcac aaaaagaaca gagcagatgg caagaagggt aatggtgaat ttggtggtga     840

agatttgatt gatgtattgt taagagttag agaaagtgga gaagttcaaa ttcctatcac     900

aaatgacaat atcaaatcaa tattaatcga catgttctct gcgggatctg aaacatcatc 960

gacgactata atttgggcat tagctgaaat gatgaagaaa ccaagtgttt tagcaaaggc 1020

acaagctgaa gtaaggcaag ctttgaagga gaaaaaaggt tttcaacaga ttgatcttga 1080

tgagctaaaa tatctcaagt tagtaatcaa agaaacctta agaatgcacc ctccaattcc 1140

tctattagtt cctagagaat gtatggagga tacaaagatt gatggttaca atataccttt 1200

caaaacaaga gtcatagtta atgcatgggc aatcggacga gatccagaaa gttgggatga 1260

ccccgaaagc tttatgccag agagatttga gaatagttct attgactttc ttggaaatca 1320

tcatcagttt ataccatttg gtgcaggaag aaggatttgt ccgggaatgc tatttggttt 1380

agctaatgtt ggacaacctt tagctcagtt actttatcac ttcgattgga aactccctaa 1440

tggacaaagt catgagaatt tcgacatgac tgagtcacct ggaatttctg ctacaagaaa 1500

ggatgatctt gttttgattg ccactcctta tgattcttat taagcagtag cagaaataaa 1560

aagccggggc aaacagaaaa aagtattgct gcttctaggt attttctatt ggataaattt 1620

caaaattcat ccacaatatt tagtgtttgc tagagttggt tagc 1664

<210> 200
<211> 503
<212> PRT
<213> Nicotiana tabacum

<400> 200

Met Glu Ile Gln Phe Ser Asn Leu Val Ala Phe Leu Leu Phe Leu Ser
1               5                   10                  15

Ser Ile Phe Leu Leu Phe Lys Lys Trp Lys Thr Arg Lys Leu Asn Leu
            20                  25                  30

Pro Pro Gly Pro Trp Lys Leu Pro Phe Ile Gly Ser Leu His His Leu
        35                  40                  45

Ala Val Ala Gly Pro Leu Pro His His Gly Leu Lys Asn Leu Ala Lys
    50                  55                  60

Arg Tyr Gly Pro Leu Met His Leu Gln Leu Gly Gln Ile Pro Thr Leu
65                  70                  75                  80

Ile Ile Ser Ser Pro Gln Met Ala Lys Glu Val Leu Lys Thr His Asp
                85                  90                  95

Leu Ala Phe Ala Thr Arg Pro Lys Leu Val Val Ala Asp Ile Ile His
            100                 105                 110

Tyr Asp Ser Thr Asp Ile Ala Phe Ser Pro Tyr Gly Glu Tyr Trp Arg
        115                 120                 125

Gln Ile Arg Lys Ile Cys Ile Leu Glu Leu Leu Ser Ala Lys Met Val
    130                 135                 140

Lys Phe Phe Ser Ser Ile Arg Gln Asp Glu Leu Ser Lys Met Leu Ser
145                 150                 155                 160

Ser Ile Arg Thr Thr Pro Asn Leu Thr Val Asn Leu Thr Asp Lys Ile
                165                 170                 175

Phe Trp Phe Thr Ser Ser Val Thr Cys Arg Ser Ala Leu Gly Lys Ile
            180                 185                 190

Cys Gly Asp Gln Asp Lys Leu Ile Ile Phe Met Arg Glu Ile Ile Ser
        195                 200                 205

Leu Ala Gly Gly Phe Ser Ile Ala Asp Phe Phe Pro Thr Trp Lys Met
    210                 215                 220

Ile His Asp Ile Asp Gly Ser Lys Ser Lys Leu Val Lys Ala His Arg
225                 230                 235                 240

Lys Ile Asp Glu Ile Leu Gly Asn Val Val Asp Glu His Lys Lys Asn
                245                 250                 255

Arg Ala Asp Gly Lys Lys Gly Asn Gly Glu Phe Gly Gly Glu Asp Leu
            260                 265                 270

Ile Asp Val Leu Leu Arg Val Arg Glu Ser Gly Glu Val Gln Ile Pro
        275                 280                 285

Ile Thr Asn Asp Asn Ile Lys Ser Ile Leu Ile Asp Met Phe Ser Ala
    290                 295                 300

Gly Ser Glu Thr Ser Ser Thr Thr Ile Ile Trp Ala Leu Ala Glu Met
305                 310                 315                 320

Met Lys Lys Pro Ser Val Leu Ala Lys Ala Gln Ala Glu Val Arg Gln
                325                 330                 335

Ala Leu Lys Glu Lys Lys Gly Phe Gln Gln Ile Asp Leu Asp Glu Leu
            340                 345                 350

Lys Tyr Leu Lys Leu Val Ile Lys Glu Thr Leu Arg Met His Pro Pro
        355                 360                 365

Ile Pro Leu Leu Val Pro Arg Glu Cys Met Glu Asp Thr Lys Ile Asp
    370                 375                 380

Gly Tyr Asn Ile Pro Phe Lys Thr Arg Val Ile Val Asn Ala Trp Ala
385                 390                 395                 400

Ile Gly Arg Asp Pro Glu Ser Trp Asp Asp Pro Glu Ser Phe Met Pro
                405                 410                 415

Glu Arg Phe Glu Asn Ser Ser Ile Asp Phe Leu Gly Asn His His Gln
            420                 425                 430

Phe Ile Pro Phe Gly Ala Gly Arg Arg Ile Cys Pro Gly Met Leu Phe
        435                 440                 445

Gly Leu Ala Asn Val Gly Gln Pro Leu Ala Gln Leu Leu Tyr His Phe
    450                 455                 460

Asp Trp Lys Leu Pro Asn Gly Gln Ser His Glu Asn Phe Asp Met Thr
465                 470                 475                 480

Glu Ser Pro Gly Ile Ser Ala Thr Arg Lys Asp Asp Leu Val Leu Ile
                485                 490                 495

Ala Thr Pro Tyr Asp Ser Tyr
            500

<210>  201
<211>  1625
<212>  DNA
<213>  Nicotiana tabacum

<400>  201
ttcttatttt gattcaacca tggagaacca atactcctac tcattctctt cctacttcta      60
cttagctata gtactgtttc ttcttccaat tttggtcaaa tatttcttcc atcggagaag     120
aaatttacct ccaagtccat tttctcttcc aataattggt cacctttacc ttctcaagaa     180
aactctccat ctcactctaa catccttatc agctaaatat ggtcctgttt tatacctcaa     240
attgggctct atgcctgtga ttgttgtgtc ctcaccatct gctgttgaag aatgtttaac     300
caagaatgat atcatattcg caaataggcc caagaccgtg gctggtgaca agtttaccta     360
caattatact gtttatgttt gggcacccta tggccaactt tggagaattc ttcgccgatt     420
aactgtcgtt gaactcttct cttcacatag cctacagaaa acttctatcc ttagagatca     480
agaagttgca atatttatcc gttcgttata caaattctca aaggatagta gcaaaaaagt     540
cgatttgacc aactggtctt ttactttggt tttcaatctt atgaccaaaa ttattgctgg     600
gagacatatt gtgaaggagg aagatgctgg caaggaaaag ggcattgaaa ttattgaaaa     660
acttagaggg actttcttag taactacatc attcttgaat atgtgtgatt tcttgccagt     720
attcaggtgg gttggttaca aagggctgga gaagaagatg gcctcaattc acaatagaag     780
aaatgaattc ttgaacagct tgcttgatga atttcgacac aagaaaagta gtgcttcaca     840
atctaacaca actgttggaa acatggagaa gaaaaccaca ctgattgaaa agctcttgtc     900
tcttcaagaa tcagagcctg aattctacac tgatgatatc atcaaaagta ttatgctggt     960
agtttttgtt gcaggaacag agacctcatc aacaaccatc caatgggtaa tgaggcttct    1020
tgtagctcac cctgaggcat tgtataagct acgagctgac attgacagta aagttgggaa    1080

taagcgcttg ctgaatgaat cagacctcaa caagcttccg tatttgcatt gtgttgttaa 1140

tgagacaatg agattataca ctccgatacc acttttattg cctcattatt caactaaaga 1200

ttgtattgtg gaaggatatg atgtaccaaa acatacaatg ttgtttgtca acgcttgggc 1260

cattcacagg gatcccaagg tatgggagga gcctgacaag ttcaagccag agagatttga 1320

ggcaacagaa ggggaaacag aaaggttcaa ttacaagctt gtaccatttg gaatggggag 1380

aagagcgtgc cctggagctg atatggggtt gcgagcagtt tctttggcat taggtgcact 1440

tattcaatgc tttgactggc aaattgagga agcggaaagc ttggaggaaa gctataattc 1500

tagaatgact atgcagaaca agcctttgaa ggttgtctgc actccacgcg aagatcttgg 1560

ccagcttcta tcccaactct aaggcaattt atcaatgcca aacgtaatct tcatctacca 1620

ctatg 1625

<210> 202
<211> 520
<212> PRT
<213> Nicotiana tabacum

<400> 202

Met Glu Asn Gln Tyr Ser Tyr Ser Phe Ser Ser Tyr Phe Tyr Leu Ala
1               5                   10                  15

Ile Val Leu Phe Leu Leu Pro Ile Leu Val Lys Tyr Phe Phe His Arg
            20                  25                  30

Arg Arg Asn Leu Pro Pro Ser Pro Phe Ser Leu Pro Ile Ile Gly His
        35                  40                  45

Leu Tyr Leu Leu Lys Lys Thr Leu His Leu Thr Leu Thr Ser Leu Ser
    50                  55                  60

Ala Lys Tyr Gly Pro Val Leu Tyr Leu Lys Leu Gly Ser Met Pro Val
65                  70                  75                  80

Ile Val Val Ser Ser Pro Ser Ala Val Glu Glu Cys Leu Thr Lys Asn
                85                  90                  95

Asp Ile Ile Phe Ala Asn Arg Pro Lys Thr Val Ala Gly Asp Lys Phe
            100                 105                 110

Thr Tyr Asn Tyr Thr Val Tyr Val Trp Ala Pro Tyr Gly Gln Leu Trp
        115                 120                 125

Arg Ile Leu Arg Arg Leu Thr Val Val Glu Leu Phe Ser Ser His Ser
    130                 135                 140

Leu Gln Lys Thr Ser Ile Leu Arg Asp Gln Glu Val Ala Ile Phe Ile
145                 150                 155                 160

Arg Ser Leu Tyr Lys Phe Ser Lys Asp Ser Ser Lys Lys Val Asp Leu
                165                 170                 175

Thr Asn Trp Ser Phe Thr Leu Val Phe Asn Leu Met Thr Lys Ile Ile
            180                 185                 190

Ala Gly Arg His Ile Val Lys Glu Glu Asp Ala Gly Lys Glu Lys Gly
        195                 200                 205

Ile Glu Ile Ile Glu Lys Leu Arg Gly Thr Phe Leu Val Thr Thr Ser
    210                 215                 220

Phe Leu Asn Met Cys Asp Phe Leu Pro Val Phe Arg Trp Val Gly Tyr
225                 230                 235                 240

Lys Gly Leu Glu Lys Lys Met Ala Ser Ile His Asn Arg Arg Asn Glu
                245                 250                 255

Phe Leu Asn Ser Leu Leu Asp Glu Phe Arg His Lys Lys Ser Ser Ala
            260                 265                 270

Ser Gln Ser Asn Thr Thr Val Gly Asn Met Glu Lys Lys Thr Thr Leu
        275                 280                 285

Ile Glu Lys Leu Leu Ser Leu Gln Glu Ser Glu Pro Glu Phe Tyr Thr
    290                 295                 300

Asp Asp Ile Ile Lys Ser Ile Met Leu Val Val Phe Val Ala Gly Thr
305                 310                 315                 320

Glu Thr Ser Ser Thr Thr Ile Gln Trp Val Met Arg Leu Leu Val Ala
                325                 330                 335

His Pro Glu Ala Leu Tyr Lys Leu Arg Ala Asp Ile Asp Ser Lys Val
            340                 345                 350

Gly Asn Lys Arg Leu Leu Asn Glu Ser Asp Leu Asn Lys Leu Pro Tyr
        355                 360                 365

Leu His Cys Val Val Asn Glu Thr Met Arg Leu Tyr Thr Pro Ile Pro
    370                 375                 380

Leu Leu Leu Pro His Tyr Ser Thr Lys Asp Cys Ile Val Glu Gly Tyr
385                 390                 395                 400

Asp Val Pro Lys His Thr Met Leu Phe Val Asn Ala Trp Ala Ile His
                405                 410                 415

Arg Asp Pro Lys Val Trp Glu Glu Pro Asp Lys Phe Lys Pro Glu Arg
            420                 425                 430

Phe Glu Ala Thr Glu Gly Glu Thr Glu Arg Phe Asn Tyr Lys Leu Val
        435                 440                 445

Pro Phe Gly Met Gly Arg Arg Ala Cys Pro Gly Ala Asp Met Gly Leu
    450                 455                 460

Arg Ala Val Ser Leu Ala Leu Gly Ala Leu Ile Gln Cys Phe Asp Trp
465                 470                 475                 480

Gln Ile Glu Glu Ala Glu Ser Leu Glu Glu Ser Tyr Asn Ser Arg Met
                485                 490                 495

Thr Met Gln Asn Lys Pro Leu Lys Val Val Cys Thr Pro Arg Glu Asp
            500                 505                 510

Leu Gly Gln Leu Leu Ser Gln Leu
        515                 520

<210>  203
<211>  1767
<212>  DNA
<213>  Nicotiana tabacum

<400>  203
aaccaacctt ccttttctta cttagtaaaa tggatattca gtcttctcct ttcaacttaa      60

ttgctttgct actcttcatt tcatttcttt ttatcctatt gaaaaagtgg aataccaaaa     120

tcccaaagtt acctccaggt ccatggagac ttccccttat tggcagcctc catcacttga     180

aaggtaaact cccacaccat catcttagag atttagcccg aaaatatgga cctctcatgt     240

atttacaact tggagaagtt cctgtagttg taatatcttc gccacgtata gcaaaagctg     300

tactaaaaac tcatgatctt gcttttgcaa cgaggcctcg gttcatgtcc tcggacattg     360

tgttttacaa aagcagggac atatcattcg ccccatatgg cgattactgg agacaaatgc     420

gtaaaatatt aacacaagaa ctcttgagta acaagatgct caagtcattt agcacaatcc     480

gaaaggatga gctctcgaag ctcctctcgt cgattcgttt agcaacagct tcttctgcag     540

tgaacataaa cgaaaagctt ctctggttta caagttgcat gacttgtaga ttagcctttg     600

gaaaaatatg caacgatcgt gatgaattga ttatgttaat aagggagata ttagcattat     660

caggaggatt tgatgtgtgt gatttgttcc cttcatggaa attacttcac aatatgagca     720

acatgaaagc tagattgacg aatgttcacc ataagtataa tctaattatg gagaatatca     780

tcaatgagca caaagagaat catgcagcag ggataaaggg aaataacgag tttggtggcg     840

aagatatgat tgatgcttta ctgagggtta aggagaataa tgagcttcaa tttcctatcg     900

aaaatgacaa catgaaagca gtaattctgg acttgtttat tgctggaact gaaacttcat     960

atactgcaat tatatgggca ctatcagaat tgatgaagca cccaagtgtt atggccaagg    1020

cacaagctga agtgagaaaa gtcttcaaag aaaatgaaaa cttggacgaa aatgatcttg    1080

acaagttgcc atacttaaaa tcagtgatca aagaaacact aaggatgcat cctccagttc    1140

ctttattagg acctagagaa tgcagagaac aaactgagat tgatggatat actgtacctc 1200
ttaaagctag agtaatggtt aatgcatggg caattggaag agatcctgaa agttgggaag 1260
atcctgaaag tttcaaaccc gagcgatttg aaaatatttc tgttgatctt acgggaaatc 1320
actatcagtt catccctttc ggttcaggaa gaagaatgtg tccaggaatg tcgtttggtt 1380
tagttaacac tgggcatcct ttagctcagt tgctctattt ctttgactgg aaattccctc 1440
ataaggttaa tgcagctgat tttcacacta ctgaaacaag tagagttttt gcagcaagca 1500
aagatgacct ctacttgatt ccaacaaatc acatggagca agagtagctc taaattgaat 1560
tcttgtcttg gaacaataaa agaagaaact ccagcttggt ctacattatt tctttttgct 1620
ttatattagt atgggtgtgt tcagtttctt atttttaagg gtaccctgaa agataaaggg 1680
ctatataaac cagtgagact ttttattggt tgcaaggttt tagatcaagc cataagacag 1740
catattttat tcaaaaaaaa aaaaaaa 1767

<210> 204
<211> 505
<212> PRT
<213> Nicotiana tabacum

<400> 204

Met Asp Ile Gln Ser Ser Pro Phe Asn Leu Ile Ala Leu Leu Leu Phe
1               5                   10                  15

Ile Ser Phe Leu Phe Ile Leu Leu Lys Lys Trp Asn Thr Lys Ile Pro
            20                  25                  30

Lys Leu Pro Pro Gly Pro Trp Arg Leu Pro Leu Ile Gly Ser Leu His
        35                  40                  45

His Leu Lys Gly Lys Leu Pro His His His Leu Arg Asp Leu Ala Arg
    50                  55                  60

Lys Tyr Gly Pro Leu Met Tyr Leu Gln Leu Gly Glu Val Pro Val Val
65                  70                  75                  80

Val Ile Ser Ser Pro Arg Ile Ala Lys Ala Val Leu Lys Thr His Asp
                85                  90                  95

Leu Ala Phe Ala Thr Arg Pro Arg Phe Met Ser Ser Asp Ile Val Phe
            100                 105                 110

Tyr Lys Ser Arg Asp Ile Ser Phe Ala Pro Tyr Gly Asp Tyr Trp Arg
        115                 120                 125

Gln Met Arg Lys Ile Leu Thr Gln Glu Leu Leu Ser Asn Lys Met Leu
    130                 135                 140

Lys Ser Phe Ser Thr Ile Arg Lys Asp Glu Leu Ser Lys Leu Leu Ser
145                 150                 155                 160

Ser Ile Arg Leu Ala Thr Ala Ser Ser Ala Val Asn Ile Asn Glu Lys
                165                 170                 175

Leu Leu Trp Phe Thr Ser Cys Met Thr Cys Arg Leu Ala Phe Gly Lys
            180                 185                 190

Ile Cys Asn Asp Arg Asp Glu Leu Ile Met Leu Ile Arg Glu Ile Leu
        195                 200                 205

Ala Leu Ser Gly Gly Phe Asp Val Cys Asp Leu Phe Pro Ser Trp Lys
    210                 215                 220

Leu Leu His Asn Met Ser Asn Met Lys Ala Arg Leu Thr Asn Val His
225                 230                 235                 240

His Lys Tyr Asn Leu Ile Met Glu Asn Ile Ile Asn Glu His Lys Glu
                245                 250                 255

Asn His Ala Ala Gly Ile Lys Gly Asn Asn Glu Phe Gly Gly Glu Asp
            260                 265                 270

Met Ile Asp Ala Leu Leu Arg Val Lys Glu Asn Asn Glu Leu Gln Phe
        275                 280                 285

Pro Ile Glu Asn Asp Asn Met Lys Ala Val Ile Leu Asp Leu Phe Ile
    290                 295                 300

Ala Gly Thr Glu Thr Ser Tyr Thr Ala Ile Ile Trp Ala Leu Ser Glu
305                 310                 315                 320

Leu Met Lys His Pro Ser Val Met Ala Lys Ala Gln Ala Glu Val Arg
                325                 330                 335

Lys Val Phe Lys Glu Asn Glu Asn Leu Asp Glu Asn Asp Leu Asp Lys
            340                 345                 350

Leu Pro Tyr Leu Lys Ser Val Ile Lys Glu Thr Leu Arg Met His Pro
        355                 360                 365

Pro Val Pro Leu Leu Gly Pro Arg Glu Cys Arg Glu Gln Thr Glu Ile
    370                 375                 380

Asp Gly Tyr Thr Val Pro Leu Lys Ala Arg Val Met Val Asn Ala Trp
385                 390                 395                 400

Ala Ile Gly Arg Asp Pro Glu Ser Trp Glu Asp Pro Glu Ser Phe Lys
                405                 410                 415

Pro Glu Arg Phe Glu Asn Ile Ser Val Asp Leu Thr Gly Asn His Tyr
            420                 425                 430

Gln Phe Ile Pro Phe Gly Ser Gly Arg Arg Met Cys Pro Gly Met Ser
        435                 440                 445

Phe Gly Leu Val Asn Thr Gly His Pro Leu Ala Gln Leu Leu Tyr Phe
    450                 455                 460

Phe Asp Trp Lys Phe Pro His Lys Val Asn Ala Ala Asp Phe His Thr
465                 470                 475                 480

Thr Glu Thr Ser Arg Val Phe Ala Ala Ser Lys Asp Asp Leu Tyr Leu
                485                 490                 495

Ile Pro Thr Asn His Met Glu Gln Glu
            500                 505

<210>  205
<211>  1736
<212>  DNA
<213>  Nicotiana tabacum

<400>  205
aaccaacctt ccttttctta cttagtaaaa tggatattca gtcttctcct ttcaacttaa      60
ttgctttgct actcttcatt tcatttcttt ttatcctatt gaaaaagtgg aataccaaaa     120
tcccaaagtt acctccaggt ccatggagac ttccccttat tggcagcctc catcacttga     180
aaggtaaact cccacaccat catcttagag atttagcccg aaaatatgga cctctcatgt     240
atttacaact tggagaagtt cctgtagttg taatatcttc gccacgtata gcaaaagctg     300
tactaaaaac tcatgatctt gcttttgcaa cgaggcctcg gttcatgtcc tcggacattg     360
tgttttacaa aagcagggac atatcattcg ccccatatgg cgattactgg agacaaatgc     420
gtaaaatatt aacacaagaa ctcttgagta acaagatgct caagtcattt agcacaatcc     480
gaaaggatgg gctctcgaag ctcctctcgt cgattcgttt agcaacagct tcttctgcag     540
tgaacataaa cgaaaagctt ctctggttta caagttgcat gacttgtaga ttagcctttg     600
gaaaaatatg caacgatcgt gatgaattga ttatgttaat aagggagata ttagcattat     660
caggaggatt tgatgtgtgt gatttgttcc cttcatggaa attacttcac aatatgagca     720
acatgaaagc tagattgacg aatgttcacc ataagtataa tctaattatg gagaatatca     780
tcaatgagca caaagagaat catgcagcag ggataaaggg aaataacgag tttggtggcg     840
aagatatgat tgatgcttta ctgagggtta aggagaataa tgagcttcaa tttcctatcg     900
aaaatgacaa catgaaagca gtaattctgg acttgtttat tgctggaact gaaacttcat     960
atactgcaat tatatgggca ctatcagaat tgatgaagca cccaagtgtt atggccaagg    1020
cacaagctga agtgagaaaa gtcttcaaag aaaatgaaaa cttggacgaa aatgatcttg    1080
acaagttgcc atacttaaaa tcagtgatca aagaaacact aaggatgcat cctccagttc    1140
ctttattagg acctagagaa tgcagagaac aaactgagat tgatggatat actgtacctc    1200

ttaaagctag agtaatggtt aatgcatggg caattggaag agatcctgaa agttgggaag 1260

atcctgaaag tttcaaaccc gagcgatttg aaaatatttc tgttgatctt acgggaaatc 1320

actatcagtt cattcctttc ggttcaggaa gaagaatgtg tccaggaatg tcgtttggtt 1380

tagttaacac tgggcatcct ttagctcagt tgctctattt ctttgactgg aaattccctc 1440

ataaggttaa tgcagctgat tttcacacta ctgaaacaag tagagttttt gcagcaagca 1500

aagatgacct ctacttgatt ccaacaaatc acatggagca agagtagctc taaattgaat 1560

tcttgtcttg gaacgataaa agaagaaact ccagcttggt ctacattatt tctttttgct 1620

ttatattagt atgggtgtgt tcagtttctt gtttttaagg gtaccctgaa agataaaggg 1680

ctatataaac cagtgagact ttttattgaa aaaaaaaaaa aaaaaaaaaa aaaaaa 1736

<210> 206
<211> 505
<212> PRT
<213> Nicotiana tabacum

<400> 206

Met Asp Ile Gln Ser Ser Pro Phe Asn Leu Ile Ala Leu Leu Leu Phe
1               5                   10                  15

Ile Ser Phe Leu Phe Ile Leu Leu Lys Lys Trp Asn Thr Lys Ile Pro
            20                  25                  30

Lys Leu Pro Pro Gly Pro Trp Arg Leu Pro Leu Ile Gly Ser Leu His
        35                  40                  45

His Leu Lys Gly Lys Leu Pro His His His Leu Arg Asp Leu Ala Arg
    50                  55                  60

Lys Tyr Gly Pro Leu Met Tyr Leu Gln Leu Gly Glu Val Pro Val Val
65                  70                  75                  80

Val Ile Ser Ser Pro Arg Ile Ala Lys Ala Val Leu Lys Thr His Asp
                85                  90                  95

Leu Ala Phe Ala Thr Arg Pro Arg Phe Met Ser Ser Asp Ile Val Phe
            100                 105                 110

Tyr Lys Ser Arg Asp Ile Ser Phe Ala Pro Tyr Gly Asp Tyr Trp Arg
        115                 120                 125

Gln Met Arg Lys Ile Leu Thr Gln Glu Leu Leu Ser Asn Lys Met Leu
    130                 135                 140

Lys Ser Phe Ser Thr Ile Arg Lys Asp Glu Leu Ser Lys Leu Leu Ser
145                 150                 155                 160

Ser Ile Arg Leu Ala Thr Ala Ser Ser Ala Val Asn Ile Asn Glu Lys
                165                 170                 175

Leu Leu Trp Phe Thr Ser Cys Met Thr Cys Arg Leu Ala Phe Gly Lys
            180                 185                 190

Ile Cys Asn Asp Arg Asp Glu Leu Ile Met Leu Ile Arg Glu Ile Leu
        195                 200                 205

Ala Leu Ser Gly Gly Phe Asp Val Cys Asp Leu Phe Pro Ser Trp Lys
    210                 215                 220

Leu Leu His Asn Met Ser Asn Met Lys Ala Arg Leu Thr Asn Val His
225                 230                 235                 240

His Lys Tyr Asn Leu Ile Met Glu Asn Ile Ile Asn Glu His Lys Glu
                245                 250                 255

Asn His Ala Ala Gly Ile Lys Gly Asn Asn Glu Phe Gly Gly Glu Asp
            260                 265                 270

Met Ile Asp Ala Leu Leu Arg Val Lys Glu Asn Asn Glu Leu Gln Phe
        275                 280                 285

Pro Ile Glu Asn Asp Asn Met Lys Ala Val Ile Leu Asp Leu Phe Ile
    290                 295                 300

Ala Gly Thr Glu Thr Ser Tyr Thr Ala Ile Ile Trp Ala Leu Ser Glu
305                 310                 315                 320

Leu Met Lys His Pro Ser Val Met Ala Lys Ala Gln Ala Glu Val Arg
                325                 330                 335

Lys Val Phe Lys Glu Asn Glu Asn Leu Asp Glu Asn Asp Leu Asp Lys
            340                 345                 350

Leu Pro Tyr Leu Lys Ser Val Ile Lys Glu Thr Leu Arg Met His Pro
        355                 360                 365

Pro Val Pro Leu Leu Gly Pro Arg Glu Cys Arg Glu Gln Thr Glu Ile
    370                 375                 380

Asp Gly Tyr Thr Val Pro Leu Lys Ala Arg Val Met Val Asn Ala Trp
385                 390                 395                 400

Ala Ile Gly Arg Asp Pro Glu Ser Trp Glu Asp Pro Glu Ser Phe Lys
                405                 410                 415

Pro Glu Arg Phe Glu Asn Ile Ser Val Asp Leu Thr Gly Asn His Tyr
            420                 425                 430

Gln Phe Ile Pro Phe Gly Ser Gly Arg Arg Met Cys Pro Gly Met Ser
        435                 440                 445

Phe Gly Leu Val Asn Thr Gly His Pro Leu Ala Gln Leu Leu Tyr Leu
    450                 455                 460

Phe Asp Trp Lys Phe Pro His Lys Val Asn Ala Ala Asp Phe His Thr
465                 470                 475                 480

Thr Glu Thr Ser Arg Val Phe Ala Ala Ser Lys Asp Asp Leu Tyr Leu
                485                 490                 495

Ile Pro Thr Asn His Met Glu Gln Glu
            500                 505

<210>  207
<211>  1823
<212>  DNA
<213>  Nicotiana tabacum

<400>  207
aaccaacctt ccttttctta cttagtaaaa tggatattca gtcttctcct ttcaacttaa      60
ttgctttgct actcttcatt tcatttcttt ttatcctatt gaaaaagtgg aataccaaaa     120
tcccaaagtt acctccaggt ccatggagac ttccccttat tggcagcctc catcacttga     180
aaggtaaact cccacaccat catcttagag atttagcccg aaaatatgga cctctcatgt     240
atttacaact tggagaagtt cctgtagttg taatatcttc gccacgtata gcaaaagctg     300
tactaaaaac tcatgatctt gcttttgcaa cgaggcctcg gttcatgtcc tcggacattg     360
tgttttacaa aagcagggac atatcattcg ccccatatgg cgattactgg agacaaatgc     420
gtaaaatatt aacacaagaa ctcttgagta acaagatgct caagtcattt agcacaatcc     480
gaaaggatga gctctcgaag ctcctctcgt cgattcgttt agcaacagct tcttctgcag     540
tgaacataaa cgaaaagctt ctctggttta caagttgcat gacttgtaga ttagcctttg     600
gaaaaatatg caacgatcgt gatgaattga ttatgttaat aagggagata ttagcattat     660
caggaggatt tgatgtgtgt gatttgttcc cttcatggaa attacttcac aatatgagca     720
acatgaaagc tagattgacg aatgttcacc ataagtataa tctaattatg gagaatatca     780
tcaatgagca caaagagaat catgcagcag ggataaaggg aaataacgag tttggtggcg     840
aagatatgat tgatgcttta ctgagggtta aggagaataa tgagcttcaa tttcctatcg     900
aaaatgacaa catgaaagca gtaattctgg acttgtttat tgctggaact gaaacttcat     960
atactgcaat tatatgggca ctatcagaat tgatgaagca cccaagtgtt atggccaagg    1020
cacaagctga agtgagaaaa gtcttcaaag aaaatgaaaa cttggacgaa aatgatcttg    1080
acaagttgcc atacttaaaa tcagtgatca aagaaacact aaggatgcat cctccagttc    1140
ctttattagg acctagagaa tgcagagaac aaactgagat tgatggatat actgtacctc    1200
ttaaagctag agtaatggtt aatgcatggg caattggaag agatcctgaa agttgggaag    1260
atcctgaaag tttcaaaccc gagcgatttg aaaatatttc tgttgatctt acgggaaatc    1320

actatcagtt cattcctttc ggttcaggaa gaagaatgtg tccaggaatg tcgtttggtt 1380

tagttaacac tgggcatcct ttagctcagt tgctctatct ctttgactgg aaattccctc 1440

ataaggttaa tgcagctgat tttcacacta ctgaaacaag tagagttttt gcagcaagca 1500

aagatgacct ctacttgatt ccaacaaatc acatggagca agagtagctc taaattgaat 1560

tcttgtcttg gaacaataaa agaagaaact ccagcttggt ctacattatt tccttttgct 1620

ttatattagt atgggtgtgt tcagtctctt gtttttaagg gtaccctgaa agataaaggg 1680

ctatataaac cagtgagact ttttattggt tgcaaggttt tagatcaagc cataagacag 1740

catattttat tccaccattt tctatcatgt ttaataaagt tcctttcgtt tattgttaga 1800

aaaaaaaaaa aaaaaaaaaa aaa 1823

<210> 208
<211> 505
<212> PRT
<213> Nicotiana tabacum

<400> 208

Met Asp Ile Gln Ser Ser Pro Phe Asn Leu Ile Ala Leu Leu Leu Phe
1               5                   10                  15

Ile Ser Phe Leu Phe Ile Leu Leu Lys Lys Trp Asn Thr Lys Ile Pro
            20                  25                  30

Lys Leu Pro Pro Gly Pro Trp Arg Leu Pro Leu Ile Gly Ser Leu His
        35                  40                  45

His Leu Lys Gly Lys Leu Pro His His His Leu Arg Asp Leu Ala Arg
    50                  55                  60

Lys Tyr Gly Pro Leu Met Tyr Leu Gln Leu Gly Glu Val Pro Val Val
65                  70                  75                  80

Val Ile Ser Ser Pro Arg Ile Ala Lys Ala Val Leu Lys Thr His Asp
                85                  90                  95

Leu Ala Phe Ala Thr Arg Pro Arg Phe Met Ser Ser Asp Ile Val Phe
            100                 105                 110

Tyr Lys Ser Arg Asp Ile Ser Phe Ala Pro Tyr Gly Asp Tyr Trp Arg
        115                 120                 125

Gln Met Arg Lys Ile Leu Thr Gln Glu Leu Leu Ser Asn Lys Met Leu
    130                 135                 140

Lys Ser Phe Ser Thr Ile Arg Lys Asp Glu Leu Ser Lys Leu Leu Ser
145                 150                 155                 160

Ser Ile Arg Leu Ala Thr Ala Ser Ser Ala Val Asn Ile Asn Glu Lys
                165                 170                 175

Leu Leu Trp Phe Thr Ser Cys Met Thr Cys Arg Leu Ala Phe Gly Lys
            180                 185                 190

Ile Cys Asn Asp Arg Asp Glu Leu Ile Met Leu Ile Arg Glu Ile Leu
        195                 200                 205

Ala Leu Ser Gly Gly Phe Asp Val Cys Asp Leu Phe Pro Ser Trp Lys
    210                 215                 220

Leu Leu His Asn Met Ser Asn Met Lys Ala Arg Leu Thr Asn Val His
225                 230                 235                 240

His Lys Tyr Asn Leu Ile Met Glu Asn Ile Ile Asn Glu His Lys Glu
                245                 250                 255

Asn His Ala Ala Gly Ile Lys Gly Asn Asn Glu Phe Gly Gly Glu Asp
            260                 265                 270

Met Ile Asp Ala Leu Leu Arg Val Lys Glu Asn Asn Glu Leu Gln Phe
        275                 280                 285

Pro Ile Glu Asn Asp Asn Met Lys Ala Val Ile Leu Asp Leu Phe Ile
    290                 295                 300

Ala Gly Thr Glu Thr Ser Tyr Thr Ala Ile Ile Trp Ala Leu Ser Glu
305                 310                 315                 320

Leu Met Lys His Pro Ser Val Met Ala Lys Ala Gln Ala Glu Val Arg
                325                 330                 335

Lys Val Phe Lys Glu Asn Glu Asn Leu Asp Glu Asn Asp Leu Asp Lys
            340                 345                 350

Leu Pro Tyr Leu Lys Ser Val Ile Lys Glu Thr Leu Arg Met His Pro
        355                 360                 365

Pro Val Pro Leu Leu Gly Pro Arg Glu Cys Arg Glu Gln Thr Glu Ile
    370                 375                 380

Asp Gly Tyr Thr Val Pro Leu Lys Ala Arg Val Met Val Asn Ala Trp
385                 390                 395                 400

Ala Ile Gly Arg Asp Pro Glu Ser Trp Glu Asp Pro Glu Ser Phe Lys
                405                 410                 415

Pro Glu Arg Phe Glu Asn Ile Ser Val Asp Leu Thr Gly Asn His Tyr
            420                 425                 430

Gln Phe Ile Pro Phe Gly Ser Gly Arg Arg Met Cys Pro Gly Met Ser
        435                 440                 445

Phe Gly Leu Val Asn Thr Gly His Pro Leu Ala Gln Leu Leu Tyr Leu
    450                 455                 460

Phe Asp Trp Lys Phe Pro His Lys Val Asn Ala Ala Asp Phe His Thr
465                 470                 475                 480

Thr Glu Thr Ser Arg Val Phe Ala Ala Ser Lys Asp Asp Leu Tyr Leu
                485                 490                 495

Ile Pro Thr Asn His Met Glu Gln Glu
            500                 505

<210>  209
<211>  1673
<212>  DNA
<213>  Nicotiana tabacum

<400>  209
atatgcaact gagatttgaa gaataccaac taaccaaaat gcagttcttc agcctggttt      60
ccattttcct atttctatct ttcctctttt tgttaagggt atggaagaac tccaatagcc     120
aaagcaaaaa gttgccacca ggtccatgga aactaccaat actaggaagt atgcttcata     180
tggttggtgg actaccacac catgtcctta gagatttagc caaaaaatat ggaccactta     240
tgcaccttca attaggtgaa gtttctgcgg ttgtggttac ttctcctgat acggcaaaag     300
aagtattaaa aactcatgac atcgcttttg cgtctaggcc tagccttttg gccccggaga     360
ttgtctgtta caataggtct gatctagcct tttgccccta tggcgactat tggagacaaa     420
tgcgtaaaat atgtgtcttg gaagtgctca gtgccaagaa tgttcggaca tttagctcta     480
ttaggcggaa tgaagttctt cgtctcatta attttatccg gtcatcttct ggtgaaccta     540
ttaatgttac ggaaaggatc tttttgttca caagctccat gacatgtaga tcagcgtttg     600
ggcaagtgtt caaagagcaa gacaaattta tacaactaat taaagaagtg atactcttag     660
caggagggtt tgatgtggct gacatattcc cttcactgaa gtttcttcat gtgctcagtg     720
gaatgaaggg taagattatg aatgcacacc ataaggtaga tgccattgtt gagaatgtca     780
tcaatgagca caagaaaaat cttgcaattg ggaaaactaa tggagcgtta ggaggtgaag     840
atttaattga tgttcttcta agacttatga atgatggagg ccttcaattt cctatcacca     900
acgacaacat caaagctata atttttgaca tgtttgctgc cgggacagag acttcatcgt     960
caacaattgt gtgggctatg gtagaaatgg tgaaaaatcc agccgtattc gcgaaagctc    1020
aagcagaagt aagagaagca tttagaggaa aagaaacttt cgatgaaaat gatgtggagg    1080
agctaaacta cctaaagtta gtaataaaag aaactctaag acttcatcca ccggttccac    1140
ttttgctccc aagagaatgt agggaagaga caaatataaa cggctacact attcctgtaa    1200
agaccaaagt catggttaat gtttgggctt tgggaagaga tccaaaatat tggaatgacg    1260
cagaaacttt tatgccagag agatttgagc agtgctctaa ggattttgtt ggtaataatt    1320

ttgaatatct tccatttggt ggcggaagga ggatttgtcc tgggatttcg tttggcttag 1380

ctaatgctta tttgccattg gctcaattac tatatcactt cgattggaaa ctccctgctg 1440

gaatcgaacc aagcgacttg gacttgactg agttggttgg agtaactgcc gctagaaaaa 1500

gtgaccttta cttggttgcg actccttatc aacctcctca aaagtgattt aatggtttca 1560

agtttttatt tcctagcaaa ccccactatt gtcctatctt tcttttggtg ttttcggttt 1620

tatctactct aatacatgca tcttttacca tataggaatg taccatgttg tcg 1673

<210> 210
<211> 514
<212> PRT
<213> Nicotiana tabacum

<400> 210

Met Gln Leu Arg Phe Glu Glu Tyr Gln Leu Thr Lys Met Gln Phe Phe
1               5                   10                  15

Ser Leu Val Ser Ile Phe Leu Phe Leu Ser Phe Leu Phe Leu Leu Arg
            20                  25                  30

Val Trp Lys Asn Ser Asn Ser Gln Ser Lys Lys Leu Pro Pro Gly Pro
        35                  40                  45

Trp Lys Leu Pro Ile Leu Gly Ser Met Leu His Met Val Gly Gly Leu
    50                  55                  60

Pro His His Val Leu Arg Asp Leu Ala Lys Lys Tyr Gly Pro Leu Met
65                  70                  75                  80

His Leu Gln Leu Gly Glu Val Ser Ala Val Val Val Thr Ser Pro Asp
                85                  90                  95

Thr Ala Lys Glu Val Leu Lys Thr His Asp Ile Ala Phe Ala Ser Arg
            100                 105                 110

Pro Ser Leu Leu Ala Pro Glu Ile Val Cys Tyr Asn Arg Ser Asp Leu
        115                 120                 125

Ala Phe Cys Pro Tyr Gly Asp Tyr Trp Arg Gln Met Arg Lys Ile Cys
    130                 135                 140

Val Leu Glu Val Leu Ser Ala Lys Asn Val Arg Thr Phe Ser Ser Ile
145                 150                 155                 160

Arg Arg Asn Glu Val Leu Arg Leu Ile Asn Phe Ile Arg Ser Ser Ser
                165                 170                 175

Gly Glu Pro Ile Asn Val Thr Glu Arg Ile Phe Leu Phe Thr Ser Ser
            180                 185                 190

Met Thr Cys Arg Ser Ala Phe Gly Gln Val Phe Lys Glu Gln Asp Lys
        195                 200                 205

Phe Ile Gln Leu Ile Lys Glu Val Ile Leu Leu Ala Gly Gly Phe Asp
    210                 215                 220

Val Ala Asp Ile Phe Pro Ser Leu Lys Phe Leu His Val Leu Ser Gly
225                 230                 235                 240

Met Lys Gly Lys Ile Met Asn Ala His His Lys Val Asp Ala Ile Val
                245                 250                 255

Glu Asn Val Ile Asn Glu His Lys Lys Asn Leu Ala Ile Gly Lys Thr
            260                 265                 270

Asn Gly Ala Leu Gly Gly Glu Asp Leu Ile Asp Val Leu Leu Arg Leu
        275                 280                 285

Met Asn Asp Gly Gly Leu Gln Phe Pro Ile Thr Asn Asp Asn Ile Lys
    290                 295                 300

Ala Ile Ile Phe Asp Met Phe Ala Ala Gly Thr Glu Thr Ser Ser Ser
305                 310                 315                 320

Thr Ile Val Trp Ala Met Val Glu Met Val Lys Asn Pro Ala Val Phe
                325                 330                 335

Ala Lys Ala Gln Ala Glu Val Arg Glu Ala Phe Arg Gly Lys Glu Thr
            340                 345                 350

Phe Asp Glu Asn Asp Val Glu Glu Leu Asn Tyr Leu Lys Leu Val Ile
        355                 360                 365

Lys Glu Thr Leu Arg Leu His Pro Pro Val Pro Leu Leu Leu Pro Arg
    370                 375                 380

Glu Cys Arg Glu Glu Thr Asn Ile Asn Gly Tyr Thr Ile Pro Val Lys
385                 390                 395                 400

Thr Lys Val Met Val Asn Val Trp Ala Leu Gly Arg Asp Pro Lys Tyr
                405                 410                 415

Trp Asn Asp Ala Glu Thr Phe Met Pro Glu Arg Phe Glu Gln Cys Ser
            420                 425                 430

Lys Asp Phe Val Gly Asn Asn Phe Glu Tyr Leu Pro Phe Gly Gly Gly
        435                 440                 445

Arg Arg Ile Cys Pro Gly Ile Ser Phe Gly Leu Ala Asn Ala Tyr Leu
    450                 455                 460

Pro Leu Ala Gln Leu Leu Tyr His Phe Asp Trp Lys Leu Pro Ala Gly
465                 470                 475                 480

Ile Glu Pro Ser Asp Leu Asp Leu Thr Glu Leu Val Gly Val Thr Ala
                485                 490                 495

Ala Arg Lys Ser Asp Leu Tyr Leu Val Ala Thr Pro Tyr Gln Pro Pro
            500                 505                 510

Gln Lys

<210>  211
<211>  1673
<212>  DNA
<213>  Nicotiana tabacum

<400>  211
atatgcaact gagatttgaa gaataccaac taaccaaaat gcagttcttc agcttggttt      60
ccattttcct atttctatct ttcctctttt tgttaaggat atggaagaac tccaatagcc     120
aaagcaaaaa gttgccacca ggtccatgga aactaccaat actaggaagt atgcttcata     180
tggttggtgg actaccacac catgtcctta gagatttagc caaaaaatat ggaccactta     240
tgcaccttca attaggtgaa gtttctgcgg ttgtggttac ttctcctgat acggcaaaag     300
aagtattaaa aactcatgac atcgcttttg cgtctaggcc tagccttttg gccccggaga     360
ttgtctgtta caataggtct gatctagcct tttgccccta tggcgactat tggagacaaa     420
tgcgtaaaat atgtgtcttg gaagtgctca gtgccaagaa tgttcggaca tttagctcta     480
ttaggcggaa tgaagttctt cgtctcatta attttatccg gtcatcttct ggtgaaccta     540
ttaatgttac ggaaaggatc tttttgttca caagctccat gacatgtaga tcagcgtttg     600
ggcaagtgtt caaagagcaa gacaaattta tacaactaat taaagaagtg atactcttag     660
caggagggtt tgatgtggct gacatattcc cttcactgaa gtttcttcat gtgctcagtg     720
gaatgaaggg taagattatg aatgcacacc ataaggtaga tgccattgtt gagaatgtca     780
tcaatgagca caagaaaaat cttgcaattg ggaaaactaa tggagcgtta ggaggtgaag     840
atttaattga tgttcttcta agacttatga atgatggagg ccttcaattt cctatcacca     900
acgacaacat caaagccata atttttgaca tgtttgctgc cgggacagag acttcatcgt     960
caacaattgt gtgggctatg gtagaaatgg tgaaaaatcc agccgtattc gcgaaagctc    1020
aagcagaagt aagagaagca tttagaggaa aagaaacttt cgatgaaaat gatgtggagg    1080
agctaaacta cctaaagtta gtaataaaag aaactctaag acttcatcca ccggttccac    1140
ttttgctccc aagagaatgt agggaagaga caaatataaa cggctacact attcctgtaa    1200
agaccaaagt catggttaat gtttgggctt tgggaagaga tccaaaatat tggaatgacg    1260
cagaaacttt tatgccagag agatttgagc agtgctctaa ggattttgtt ggtaataatt    1320
ttgaatatct tccatttggt ggcggaagga ggatttgtcc tgggatttcg tttggcttag    1380

ctaatgctta tttgccattg gctcaattac tatatcactt cgattggaaa ctccctgctg 1440

gaatcgaacc aagcgacttg gacttgactg agttggttgg agtaactgcc gctagaaaaa 1500

gtgaccttta cttggttgcg actccttatc aacctcctca aaagtgattt aatggtttca 1560

agtttttatt tcctagcaaa ccccactatt gtcctatctt tcttttggtg ttttcggttt 1620

tatctactct aatacatgca tcttttacca tataggaatg taccatgttg tcg 1673

<210> 212
<211> 514
<212> PRT
<213> Nicotiana tabacum

<400> 212

Met Gln Leu Arg Phe Glu Glu Tyr Gln Leu Thr Lys Met Gln Phe Phe
1               5                   10                  15

Ser Leu Val Ser Ile Phe Leu Phe Leu Ser Phe Leu Phe Leu Leu Arg
            20                  25                  30

Ile Trp Lys Asn Ser Asn Ser Gln Ser Lys Lys Leu Pro Pro Gly Pro
        35                  40                  45

Trp Lys Leu Pro Ile Leu Gly Ser Met Leu His Met Val Gly Gly Leu
    50                  55                  60

Pro His His Val Leu Arg Asp Leu Ala Lys Lys Tyr Gly Pro Leu Met
65                  70                  75                  80

His Leu Gln Leu Gly Glu Val Ser Ala Val Val Val Thr Ser Pro Asp
                85                  90                  95

Thr Ala Lys Glu Val Leu Lys Thr His Asp Ile Ala Phe Ala Ser Arg
            100                 105                 110

Pro Ser Leu Leu Ala Pro Glu Ile Val Cys Tyr Asn Arg Ser Asp Leu
        115                 120                 125

Ala Phe Cys Pro Tyr Gly Asp Tyr Trp Arg Gln Met Arg Lys Ile Cys
    130                 135                 140

Val Leu Glu Val Leu Ser Ala Lys Asn Val Arg Thr Phe Ser Ser Ile
145                 150                 155                 160

Arg Arg Asn Glu Val Leu Arg Leu Ile Asn Phe Ile Arg Ser Ser Ser
                165                 170                 175

Gly Glu Pro Ile Asn Val Thr Glu Arg Ile Phe Leu Phe Thr Ser Ser
            180                 185                 190

Met Thr Cys Arg Ser Ala Phe Gly Gln Val Phe Lys Glu Gln Asp Lys
        195                 200                 205

Phe Ile Gln Leu Ile Lys Glu Val Ile Leu Leu Ala Gly Gly Phe Asp
    210                 215                 220

Val Ala Asp Ile Phe Pro Ser Leu Lys Phe Leu His Val Leu Ser Gly
225                 230                 235                 240

Met Lys Gly Lys Ile Met Asn Ala His His Lys Val Asp Ala Ile Val
                245                 250                 255

Glu Asn Val Ile Asn Glu His Lys Lys Asn Leu Ala Ile Gly Lys Thr
            260                 265                 270

Asn Gly Ala Leu Gly Gly Glu Asp Leu Ile Asp Val Leu Leu Arg Leu
        275                 280                 285

Met Asn Asp Gly Gly Leu Gln Phe Pro Ile Thr Asn Asp Asn Ile Lys
    290                 295                 300

Ala Ile Ile Phe Asp Met Phe Ala Ala Gly Thr Glu Thr Ser Ser Ser
305                 310                 315                 320

Thr Ile Val Trp Ala Met Val Glu Met Val Lys Asn Pro Ala Val Phe
                325                 330                 335

Ala Lys Ala Gln Ala Glu Val Arg Glu Ala Phe Arg Gly Lys Glu Thr
            340                 345                 350

Phe Asp Glu Asn Asp Val Glu Glu Leu Asn Tyr Leu Lys Leu Val Ile
        355                 360                 365

Lys Glu Thr Leu Arg Leu His Pro Pro Val Pro Leu Leu Leu Pro Arg
    370                 375                 380

Glu Cys Arg Glu Glu Thr Asn Ile Asn Gly Tyr Thr Ile Pro Val Lys
385                 390                 395                 400

Thr Lys Val Met Val Asn Val Trp Ala Leu Gly Arg Asp Pro Lys Tyr
                405                 410                 415

Trp Asn Asp Ala Glu Thr Phe Met Pro Glu Arg Phe Glu Gln Cys Ser
            420                 425                 430

Lys Asp Phe Val Gly Asn Asn Phe Glu Tyr Leu Pro Phe Gly Gly Gly
        435                 440                 445

Arg Arg Ile Cys Pro Gly Ile Ser Phe Gly Leu Ala Asn Ala Tyr Leu
    450                 455                 460

Pro Leu Ala Gln Leu Leu Tyr His Phe Asp Trp Lys Leu Pro Ala Gly
465                 470                 475                 480

Ile Glu Pro Ser Asp Leu Asp Leu Thr Glu Leu Val Gly Val Thr Ala
                485                 490                 495

Ala Arg Lys Ser Asp Leu Tyr Leu Val Ala Thr Pro Tyr Gln Pro Pro
            500                 505                 510

Gln Lys

<210>  213
<211>  1673
<212>  DNA
<213>  Nicotiana tabacum

<400>  213
atatgcaact gagatttgaa gaataccaac taaccaaagt gcagttcttc agcttggttt      60
ccattttcct atttctatct ttcctctttt tgttaaggat atggaagaac tccaatagcc     120
aaagcaaaaa gttgccacca ggtccatgga aactaccaat actaggaagt atgcttcata     180
tggttggtgg actaccacac catgtcctta gagatttagc caaaaaatat ggaccactta     240
tgcaccttca attaggtgaa gtttctgcgg ttgtggttac ttctcctgat acggcaaaag     300
aagtattaaa aactcatgac atcgcttttg cgtctaggcc tagccttttg gccccggaga     360
ttgtctgtta caataggtct gatctagcct tttgccccta tggcgactat tggagacaaa     420
tgcgtaaaat atgtgtcttg gaagtgctca gtgccaagaa tgttcggaca tttagctcta     480
ttaggcggaa tgaagttctt cgtctcatta attttatccg gtcatcttct ggtgaaccta     540
ttaatgttac ggaaaggatc tttttgttca caagctccat gacatgtaga tcagcgtttg     600
ggcaagtgtt caaagagcaa gacaaattta tacaactaat taaagaagtg atactcttag     660
caggagggtt tgatgtggct gacatattcc cttcactgaa gtttcttcat gtgctcagtg     720
gaatgaaggg taagattatg aatgcacacc ataaggtaga tgccattgtt gagaatgtca     780
tcaatgagca caagaaaaat cttgcaattg ggaaaactaa tggagcgtta ggaggtgaag     840
atttaattga tgttcctcta agacttatga atgatggagg ccttcaattt cctatcacca     900
acgacaacat caaagctata atttttgaca tgtttgctgc cgggacggag acttcatcgt     960
caacaattgt gtgggctatg gtagaaatgg tgaaaaatcc agccgtattc gcgaaagctc    1020
aagcagaagt aagagaagca tttagaggaa aagaaacttt cgatgaaaat gatgtggagg    1080
agctaaacta cctaaagtta gtaataaaag aaactctaag acttcatcca ccggttccac    1140
ttttgctccc aagagaatgt agggaagaga caaatataaa cggctacact attcctgtaa    1200
agaccaaagt catggttaat gtttgggctt tgggaagaga tccaaaatat tggaatgacg    1260
cagaaacttt tatgccagag agatttgagc agtgctctaa ggattttgtt ggtaataatt    1320
ttgaatatct tccatttggt ggcggaagga ggatttgtcc tgggatttcg tttggcttag    1380
ctaatgctta tttgccattg gctcaattac tatatcactt cgattggaaa ctccctgctg    1440

gaatcgaacc aagcgacttg gacttgactg agttggttgg agtaactgcc gctagaaaaa 1500
gtgaccttta cttggttgcg actccttatc aacctcctca aaagtgattt aatggtttca 1560
agtttttatt tcctagcaaa ccccactatt gtcctatctt tcttttggtg ttttcggttt 1620
tatctactct aatacatgca tcttttacca tataggaatg taccatgttg tcg 1673

<210> 214
<211> 514
<212> PRT
<213> Nicotiana tabacum

<400> 214

Met Gln Leu Arg Phe Glu Glu Tyr Gln Leu Thr Lys Val Gln Phe Phe
1               5                   10                  15

Ser Leu Val Ser Ile Phe Leu Phe Leu Ser Phe Leu Phe Leu Leu Arg
            20                  25                  30

Ile Trp Lys Asn Ser Asn Ser Gln Ser Lys Lys Leu Pro Pro Gly Pro
        35                  40                  45

Trp Lys Leu Pro Ile Leu Gly Ser Met Leu His Met Val Gly Gly Leu
    50                  55                  60

Pro His His Val Leu Arg Asp Leu Ala Lys Lys Tyr Gly Pro Leu Met
65                  70                  75                  80

His Leu Gln Leu Gly Glu Val Ser Ala Val Val Val Thr Ser Pro Asp
                85                  90                  95

Thr Ala Lys Glu Val Leu Lys Thr His Asp Ile Ala Phe Ala Ser Arg
            100                 105                 110

Pro Ser Leu Leu Ala Pro Glu Ile Val Cys Tyr Asn Arg Ser Asp Leu
        115                 120                 125

Ala Phe Cys Pro Tyr Gly Asp Tyr Trp Arg Gln Met Arg Lys Ile Cys
    130                 135                 140

Val Leu Glu Val Leu Ser Ala Lys Asn Val Arg Thr Phe Ser Ser Ile
145                 150                 155                 160

Arg Arg Asn Glu Val Leu Arg Leu Ile Asn Phe Ile Arg Ser Ser Ser
                165                 170                 175

Gly Glu Pro Ile Asn Val Thr Glu Arg Ile Phe Leu Phe Thr Ser Ser
            180                 185                 190

Met Thr Cys Arg Ser Ala Phe Gly Gln Val Phe Lys Glu Gln Asp Lys
        195                 200                 205

Phe Ile Gln Leu Ile Lys Glu Val Ile Leu Leu Ala Gly Gly Phe Asp
    210                 215                 220

Val Ala Asp Ile Phe Pro Ser Leu Lys Phe Leu His Val Leu Ser Gly
225                 230                 235                 240

Met Lys Gly Lys Ile Met Asn Ala His His Lys Val Asp Ala Ile Val
                245                 250                 255

Glu Asn Val Ile Asn Glu His Lys Lys Asn Leu Ala Ile Gly Lys Thr
            260                 265                 270

Asn Gly Ala Leu Gly Gly Glu Asp Leu Ile Asp Val Pro Leu Arg Leu
        275                 280                 285

Met Asn Asp Gly Gly Leu Gln Phe Pro Ile Thr Asn Asp Asn Ile Lys
    290                 295                 300

Ala Ile Ile Phe Asp Met Phe Ala Ala Gly Thr Glu Thr Ser Ser Ser
305                 310                 315                 320

Thr Ile Val Trp Ala Met Val Glu Met Val Lys Asn Pro Ala Val Phe
                325                 330                 335

Ala Lys Ala Gln Ala Glu Val Arg Glu Ala Phe Arg Gly Lys Glu Thr
            340                 345                 350

Phe Asp Glu Asn Asp Val Glu Glu Leu Asn Tyr Leu Lys Leu Val Ile
        355                 360                 365

Lys Glu Thr Leu Arg Leu His Pro Pro Val Pro Leu Leu Leu Pro Arg
    370                 375                 380

Glu Cys Arg Glu Glu Thr Asn Ile Asn Gly Tyr Thr Ile Pro Val Lys
385                 390                 395                 400

Thr Lys Val Met Val Asn Val Trp Ala Leu Gly Arg Asp Pro Lys Tyr
                405                 410                 415

Trp Asn Asp Ala Glu Thr Phe Met Pro Glu Arg Phe Glu Gln Cys Ser
            420                 425                 430

Lys Asp Phe Val Gly Asn Asn Phe Glu Tyr Leu Pro Phe Gly Gly Gly
        435                 440                 445

Arg Arg Ile Cys Pro Gly Ile Ser Phe Gly Leu Ala Asn Ala Tyr Leu
    450                 455                 460

Pro Leu Ala Gln Leu Leu Tyr His Phe Asp Trp Lys Leu Pro Ala Gly
465                 470                 475                 480

Ile Glu Pro Ser Asp Leu Asp Leu Thr Glu Leu Val Gly Val Thr Ala
                485                 490                 495

Ala Arg Lys Ser Asp Leu Tyr Leu Val Ala Thr Pro Tyr Gln Pro Pro
            500                 505                 510

Gln Lys

<210>  215
<211>  1580
<212>  DNA
<213>  Nicotiana tabacum

<400>  215
gaaatgggaa atgctcacaa cagcaaaatt gcagcaatct gtttgataat tttcttggta      60
tataaagcat gggaattgtt gaagtggata tggattaagc caaagaaact ggagagttgc     120
ctcagaaaac agggactcaa aggaaattcc tacaggctat tctatggaga tatgaaagaa     180
ttgtccaaaa gtctcaagga aatcaattca aagcccatca tcaatctatc aaatgaagta     240
gccccaagaa tcattcctta ttatcttgaa atcatccaaa aatatggtaa aagatgtttt     300
gtttggcaag gaccaacccc cgcaatatta ataacagagc cagaattaat aaaggagata     360
tttggtaaga actatgtttt tcagaagcct aataatccca acccactgac caagttattg     420
gctcgaggtg ttgtaagcta cgaggaagaa aaatgggcaa aacacagaaa gatcttaaac     480
cctgcctttc atatggagaa gttgaagcat atgctaccag cattttactt gagctgtagt     540
gagatgctga acaaatggga ggagattatc ccagtaaaag aatcaaatga gttggacatt     600
tggcctcatc ttcaaagaat gacaagtgat gtgatttctc gtgctgcctt tggtagtagc     660
tacgaagaag gaagaagaat atttgaactt caagaagaac aagctgagta tctaacgaag     720
acattcaatt cagtttatat cccaggttcc agattttttc ccaataaaat gaacaaaaga     780
atgaaagaat gtgaaaagga agtacgagaa acaattacgt gtctaattga caacagatta     840
aaggcaaaag aagaaggcaa tggcaaggcc ctcaatgatg acctactggg tatattatta     900
gagtcaaatt ctatagaaat tgaagaacat ggtaacaaga agtttggaat gagtatacct     960
gaagtaattg aagagtgcaa attattctat tttgctggcc aagagactac atcagtattg    1020
cttgtgtgga cactgatttt gttagggaga aatccagaat ggcaggaacg tgctagagag    1080
gaagtttttc aagcctttgg aagtgataaa ccaacttttg acgaattata tcgcttgaaa    1140
attgtgacga tgattttgta cgagtcttta aggttatatc caccaatagc aactcgtact    1200
cgaaggacta atgaagaaac aaaattaggg gaactagatt taccaaaggg tgcactgctc    1260
tttataccaa caatcttatt acatcttgac aaggaaattt ggggtgaaga tgcagatgag    1320
ttcaatccgg agagatttag cgaaggggtg gcaaaggcaa caaaggggaa aatgacatat    1380
tttccatttg gtgcaggacc gcgaaaatgc attgggcaaa acttcgcgat tttggaagca    1440
aaaatggcta tagctatgat tctacaacgc ttctccttcg agctctctcc atcttataca    1500

cactctccat acactgtggt cactttgaaa cccaaatatg gtgctcccct aataatgcac 1560
aggctgtagt cctgtgagaa 1580

<210> 216
<211> 521
<212> PRT
<213> Nicotiana tabacum

<400> 216

Met Gly Asn Ala His Asn Ser Lys Ile Ala Ala Ile Cys Leu Ile Ile
1               5                   10                  15

Phe Leu Val Tyr Lys Ala Trp Glu Leu Leu Lys Trp Ile Trp Ile Lys
            20                  25                  30

Pro Lys Lys Leu Glu Ser Cys Leu Arg Lys Gln Gly Leu Lys Gly Asn
        35                  40                  45

Ser Tyr Arg Leu Phe Tyr Gly Asp Met Lys Glu Leu Ser Lys Ser Leu
    50                  55                  60

Lys Glu Ile Asn Ser Lys Pro Ile Ile Asn Leu Ser Asn Glu Val Ala
65                  70                  75                  80

Pro Arg Ile Ile Pro Tyr Tyr Leu Glu Ile Ile Gln Lys Tyr Gly Lys
                85                  90                  95

Arg Cys Phe Val Trp Gln Gly Pro Thr Pro Ala Ile Leu Ile Thr Glu
            100                 105                 110

Pro Glu Leu Ile Lys Glu Ile Phe Gly Lys Asn Tyr Val Phe Gln Lys
        115                 120                 125

Pro Asn Asn Pro Asn Pro Leu Thr Lys Leu Leu Ala Arg Gly Val Val
    130                 135                 140

Ser Tyr Glu Glu Glu Lys Trp Ala Lys His Arg Lys Ile Leu Asn Pro
145                 150                 155                 160

Ala Phe His Met Glu Lys Leu Lys His Met Leu Pro Ala Phe Tyr Leu
                165                 170                 175

Ser Cys Ser Glu Met Leu Asn Lys Trp Glu Glu Ile Ile Pro Val Lys
            180                 185                 190

Glu Ser Asn Glu Leu Asp Ile Trp Pro His Leu Gln Arg Met Thr Ser
        195                 200                 205

Asp Val Ile Ser Arg Ala Ala Phe Gly Ser Ser Tyr Glu Glu Gly Arg
    210                 215                 220

Arg Ile Phe Glu Leu Gln Glu Glu Gln Ala Glu Tyr Leu Thr Lys Thr
225                 230                 235                 240

Phe Asn Ser Val Tyr Ile Pro Gly Ser Arg Phe Phe Pro Asn Lys Met
                245                 250                 255

Asn Lys Arg Met Lys Glu Cys Glu Lys Glu Val Arg Glu Thr Ile Thr
            260                 265                 270

Cys Leu Ile Asp Asn Arg Leu Lys Ala Lys Glu Glu Gly Asn Gly Lys
        275                 280                 285

Ala Leu Asn Asp Asp Leu Leu Gly Ile Leu Leu Glu Ser Asn Ser Ile
    290                 295                 300

Glu Ile Glu Glu His Gly Asn Lys Lys Phe Gly Met Ser Ile Pro Glu
305                 310                 315                 320

Val Ile Glu Glu Cys Lys Leu Phe Tyr Phe Ala Gly Gln Glu Thr Thr
                325                 330                 335

Ser Val Leu Leu Val Trp Thr Leu Ile Leu Leu Gly Arg Asn Pro Glu
            340                 345                 350

Trp Gln Glu Arg Ala Arg Glu Glu Val Phe Gln Ala Phe Gly Ser Asp
        355                 360                 365

Lys Pro Thr Phe Asp Glu Leu Tyr Arg Leu Lys Ile Val Thr Met Ile
    370                 375                 380

Leu Tyr Glu Ser Leu Arg Leu Tyr Pro Pro Ile Ala Thr Arg Thr Arg
385                 390                 395                 400

Arg Thr Asn Glu Glu Thr Lys Leu Gly Glu Leu Asp Leu Pro Lys Gly
                405                 410                 415

Ala Leu Leu Phe Ile Pro Thr Ile Leu Leu His Leu Asp Lys Glu Ile
            420                 425                 430

Trp Gly Glu Asp Ala Asp Glu Phe Asn Pro Glu Arg Phe Ser Glu Gly
        435                 440                 445

Val Ala Lys Ala Thr Lys Gly Lys Met Thr Tyr Phe Pro Phe Gly Ala
    450                 455                 460

Gly Pro Arg Lys Cys Ile Gly Gln Asn Phe Ala Ile Leu Glu Ala Lys
465                 470                 475                 480

Met Ala Ile Ala Met Ile Leu Gln Arg Phe Ser Phe Glu Leu Ser Pro
                485                 490                 495

Ser Tyr Thr His Ser Pro Tyr Thr Val Val Thr Leu Lys Pro Lys Tyr
            500                 505                 510

Gly Ala Pro Leu Ile Met His Arg Leu
        515                 520

<210>  217
<211>  1587
<212>  DNA
<213>  Nicotiana tabacum

<400>  217
atgctttctc ccatagaagc ctttgtagga ctagtaacct tcacatttct cttatacttc      60

ctatggacaa aaaaatctca aaaacttcca aaacccttac caccgaaaat ccccggagga     120

tggccggtaa tcggccatct ttttcacttc aataacgacg gcgacgaccg tccattagct     180

cgaaagctcg gagacttagc tgataaatac ggccccgttt tcacttttcg gctaggtctt     240

ccccttgtgc tagttgtaag cagttacgaa gctataaaag attgcttctc tacaaatgat     300

gccattttct ccaatcgtcc agctcttctt tacggcgaat accttggcta caataataca     360

atgctttttc tagcaaatta cggaccttac tggcgaaaaa atcgtaaatt agtcattcag     420

gaagttctct ctgctagtcg tctcgaaaaa ttcaaacaag tgagattcac cagaattcaa     480

acgagcatta agaatttata cactcgaatt aatggaaatt cgagtacgat aaatctaact     540

gattggttag aagaattgaa ttttggtctg atcgtgaaaa tgatcgctgg gaaaaattat     600

gaatccggta aaggagatga acaagtggaa agatttaaga atgcgtttaa ggattttatg     660

gttttatcaa tggaatttgt attatgggat gcatttccaa ttccattatt taaatgggtg     720

gattttcaag gtcatattaa ggcaatgaaa aggacattta aggatataga ttctgttttt     780

cagaactggt tagaggaaca tattaataaa agagaaaaaa tagaggttgg tgcagaaggg     840

aatgaacaag atttcattga tgtggtgctt tcaaaattga gtaaagaata tcttgatgaa     900

ggttactctc gtgatactgt cattaaagca acagttttta gtttggtctt ggatgcagca     960

gacacagttg ctcttcacat aaattgggga atgacattat tgataaacaa tcaaaatgcc    1020

ttgatgaaag cacaagaaga gatagacaca aaagttggta aggatagatg ggtagaagag    1080

agtgatatta aggatttagt atacctccaa gctattgtta aaaaggtgtt acgattatat    1140

ccaccaggac ctttgttagt accacatgaa aatgtaaagg attgtgttgt tagtggatat    1200

cacattccta aagggactag attattcgca aacgtcatga aactgcagcg cgatcctaaa    1260

ctcttgtcaa atcctgataa gttcgatcca gagagattca tcgctggtga tattgacttc    1320

cgtggtcacc actatgagtt tatcccattt ggttctggaa gacgatcttg tccggggatg    1380

acttatgcat tgcaagtgga acacctaaca atggcacatt taatccaggg tttcaattac    1440

aaaactccaa atgacgaggc cttggatatg aaggaaggtg caggcataac aatacgtaag    1500

gtaaatccag tggaattgat aataacgcct cgcttggcac ctgagcttta ctaaaaccta    1560

agatgtttca tcttggttga tcattgt                                        1587

<210>  218
<211>  517
<212>  PRT
<213>  Nicotiana tabacum

<400>  218

Met Leu Ser Pro Ile Glu Ala Phe Val Gly Leu Val Thr Phe Thr Phe
1               5                   10                  15

Leu Leu Tyr Phe Leu Trp Thr Lys Lys Ser Gln Lys Leu Pro Lys Pro
            20                  25                  30

Leu Pro Pro Lys Ile Pro Gly Gly Trp Pro Val Ile Gly His Leu Phe
        35                  40                  45

His Phe Asn Asn Asp Gly Asp Asp Arg Pro Leu Ala Arg Lys Leu Gly
    50                  55                  60

Asp Leu Ala Asp Lys Tyr Gly Pro Val Phe Thr Phe Arg Leu Gly Leu
65                  70                  75                  80

Pro Leu Val Leu Val Val Ser Ser Tyr Glu Ala Ile Lys Asp Cys Phe
                85                  90                  95

Ser Thr Asn Asp Ala Ile Phe Ser Asn Arg Pro Ala Leu Leu Tyr Gly
            100                 105                 110

Glu Tyr Leu Gly Tyr Asn Asn Thr Met Leu Phe Leu Ala Asn Tyr Gly
        115                 120                 125

Pro Tyr Trp Arg Lys Asn Arg Lys Leu Val Ile Gln Glu Val Leu Ser
    130                 135                 140

Ala Ser Arg Leu Glu Lys Phe Lys Gln Val Arg Phe Thr Arg Ile Gln
145                 150                 155                 160

Thr Ser Ile Lys Asn Leu Tyr Thr Arg Ile Asn Gly Asn Ser Ser Thr
                165                 170                 175

Ile Asn Leu Thr Asp Trp Leu Glu Glu Leu Asn Phe Gly Leu Ile Val
            180                 185                 190

Lys Met Ile Ala Gly Lys Asn Tyr Glu Ser Gly Lys Gly Asp Glu Gln
        195                 200                 205

Val Glu Arg Phe Lys Asn Ala Phe Lys Asp Phe Met Val Leu Ser Met
    210                 215                 220

Glu Phe Val Leu Trp Asp Ala Phe Pro Ile Pro Leu Phe Lys Trp Val
225                 230                 235                 240

Asp Phe Gln Gly His Ile Lys Ala Met Lys Arg Thr Phe Lys Asp Ile
                245                 250                 255

Asp Ser Val Phe Gln Asn Trp Leu Glu Glu His Ile Asn Lys Arg Glu
            260                 265                 270

Lys Ile Glu Val Gly Ala Glu Gly Asn Glu Gln Asp Phe Ile Asp Val
        275                 280                 285

Val Leu Ser Lys Leu Ser Lys Glu Tyr Leu Asp Glu Gly Tyr Ser Arg
    290                 295                 300

Asp Thr Val Ile Lys Ala Thr Val Phe Ser Leu Val Leu Asp Ala Ala
305                 310                 315                 320

Asp Thr Val Ala Leu His Ile Asn Trp Gly Met Thr Leu Leu Ile Asn
                325                 330                 335

Asn Gln Asn Ala Leu Met Lys Ala Gln Glu Glu Ile Asp Thr Lys Val
            340                 345                 350

Gly Lys Asp Arg Trp Val Glu Glu Ser Asp Ile Lys Asp Leu Val Tyr
        355                 360                 365

Leu Gln Ala Ile Val Lys Lys Val Leu Arg Leu Tyr Pro Pro Gly Pro
    370                 375                 380

Leu Leu Val Pro His Glu Asn Val Lys Asp Cys Val Val Ser Gly Tyr
385                 390                 395                 400

His Ile Pro Lys Gly Thr Arg Leu Phe Ala Asn Val Met Lys Leu Gln
                405                 410                 415

Arg Asp Pro Lys Leu Leu Ser Asn Pro Asp Lys Phe Asp Pro Glu Arg
            420                 425                 430

Phe Ile Ala Gly Asp Ile Asp Phe Arg Gly His His Tyr Glu Phe Ile
        435                 440                 445

Pro Phe Gly Ser Gly Arg Arg Ser Cys Pro Gly Met Thr Tyr Ala Leu
    450                 455                 460

Gln Val Glu His Leu Thr Met Ala His Leu Ile Gln Gly Phe Asn Tyr
465                 470                 475                 480

Lys Thr Pro Asn Asp Glu Ala Leu Asp Met Lys Glu Gly Ala Gly Ile
                485                 490                 495

Thr Ile Arg Lys Val Asn Pro Val Glu Leu Ile Ile Thr Pro Arg Leu
            500                 505                 510

Ala Pro Glu Leu Tyr
        515

<210>  219
<211>  1634
<212>  DNA
<213>  Nicotiana tabacum

<400>  219
gggagaaggc cttcaatatg gagataccat attacagctt aaaaattgca atttcttcat      60

ttgcaattat ctttgtacta agatgggcat ggaaaatctt gaattatgtg tggttaaaac     120

caaaagaatt ggagaaatac ctcagacagc agggtttcaa aggaaactct tacaaattct     180

tgtttgggga tatgaaagag acgaagaaaa tgggtgaaga agctatgtct aagccaatca     240

atttctctca tgacatgatt tggcctagag ttatgccatt catccacaaa accatcacca     300

attatggtaa gaattgtatt gtgtggtttg ggccaagacc agcagtcctg atcacagacc     360

cggaacttgt aaaggaggtg ctaacgaaga atttcgtcta tcagaagccg cttggcaatc     420

cactcacaaa gttggcagca actggaattg caggctatga aacagataaa tgggctacac     480

atagaaggct tctcaatcct gcttttcacc ttgacaagtt gaagcatatg ctacctgcat     540

tccaatttac tgctagtgag atgttgagca aattggagaa agttgtttca ccaaacggaa     600

cagagataga tgtgtggcca tatttacaaa ctttgacaag tgatgccatt tcaagaactg     660

cgtttggaag tagttatgaa gaaggaagaa agatttttga ccttcaaaaa gaacaacttt     720

cactaattct agaagtttca cgcacaatat atattccagg atggaggttt ttgccaacga     780

aaaggaacaa aaggatgaag caaatattta atgaagtacg agcactggta tttggaatta     840

ttaagaaaag gatgagtatg attgaaaatg gagaagcacc tgatgattta ttgggaatat     900

tattggcatc caatttaaaa gaaatccaac aacatggaaa caacaagaaa tttggtatga     960

gtattgatga ggtgattgaa gagtgtaaac tcttctattt tgctgggcaa gagactactt    1020

catctttact tgtatggact atgattttgt tgtgcaaata tcctaattgg caagataaag    1080

ctagagaaga ggttttgcaa gtgtttggga gtagggaagt tgactatgac aagttgaatc    1140

agctaaaaat agtaactatg atcttaaacg aggtcttaag gttgtatcca gcaggatatg    1200

tgattaatcg aatggtaaac aaagaaacaa agttagggaa tttgtgttta ccagccggcg    1260

tacagctcgt gttaccaaca atgttgttgc aacatgatac tgaaatatgg ggagatgatg    1320

caatggagtt caatccagag agatttagtg atggaatatc caaagcaaca aaaggaaaac    1380

ttgtgttttt tccatttagt tggggtccaa gaatatgtat tgggcaaaat tttgctatgt    1440

tagaggctaa aatggcaatg gctatgattc tgaaaaccta tgcatttgaa ctctctccat    1500

cttatgctca tgctcctcat ccactactac ttcaacctca atatggtgct caattaattt    1560

tgtacaagtt gtagatatgg tcaatctgga acttgttatg gaacttttat catcgtaatc    1620

aaccatattg aggg                                                      1634

<210>  220

<211>  518
<212>  PRT
<213>  Nicotiana tabacum

<400>  220

Met Glu Ile Pro Tyr Tyr Ser Leu Lys Ile Ala Ile Ser Ser Phe Ala
1               5                   10                  15

Ile Ile Phe Val Leu Arg Trp Ala Trp Lys Ile Leu Asn Tyr Val Trp
            20                  25                  30

Leu Lys Pro Lys Glu Leu Glu Lys Tyr Leu Arg Gln Gln Gly Phe Lys
        35                  40                  45

Gly Asn Ser Tyr Lys Phe Leu Phe Gly Asp Met Lys Glu Thr Lys Lys
    50                  55                  60

Met Gly Glu Glu Ala Met Ser Lys Pro Ile Asn Phe Ser His Asp Met
65                  70                  75                  80

Ile Trp Pro Arg Val Met Pro Phe Ile His Lys Thr Ile Thr Asn Tyr
                85                  90                  95

Gly Lys Asn Cys Ile Val Trp Phe Gly Pro Arg Pro Ala Val Leu Ile
            100                 105                 110

Thr Asp Pro Glu Leu Val Lys Glu Val Leu Thr Lys Asn Phe Val Tyr
        115                 120                 125

Gln Lys Pro Leu Gly Asn Pro Leu Thr Lys Leu Ala Ala Thr Gly Ile
    130                 135                 140

Ala Gly Tyr Glu Thr Asp Lys Trp Ala Thr His Arg Arg Leu Leu Asn
145                 150                 155                 160

Pro Ala Phe His Leu Asp Lys Leu Lys His Met Leu Pro Ala Phe Gln
                165                 170                 175

Phe Thr Ala Ser Glu Met Leu Ser Lys Leu Glu Lys Val Val Ser Pro
            180                 185                 190

Asn Gly Thr Glu Ile Asp Val Trp Pro Tyr Leu Gln Thr Leu Thr Ser
        195                 200                 205

Asp Ala Ile Ser Arg Thr Ala Phe Gly Ser Ser Tyr Glu Glu Gly Arg
    210                 215                 220

Lys Ile Phe Asp Leu Gln Lys Glu Gln Leu Ser Leu Ile Leu Glu Val
225                 230                 235                 240

Ser Arg Thr Ile Tyr Ile Pro Gly Trp Arg Phe Leu Pro Thr Lys Arg
                245                 250                 255

Asn Lys Arg Met Lys Gln Ile Phe Asn Glu Val Arg Ala Leu Val Phe
            260                 265                 270

Gly Ile Ile Lys Lys Arg Met Ser Met Ile Glu Asn Gly Glu Ala Pro
        275                 280                 285

Asp Asp Leu Leu Gly Ile Leu Leu Ala Ser Asn Leu Lys Glu Ile Gln
    290                 295                 300

Gln His Gly Asn Asn Lys Lys Phe Gly Met Ser Ile Asp Glu Val Ile
305                 310                 315                 320

Glu Glu Cys Lys Leu Phe Tyr Phe Ala Gly Gln Glu Thr Thr Ser Ser
                325                 330                 335

Leu Leu Val Trp Thr Met Ile Leu Leu Cys Lys Tyr Pro Asn Trp Gln
            340                 345                 350

Asp Lys Ala Arg Glu Glu Val Leu Gln Val Phe Gly Ser Arg Glu Val
        355                 360                 365

Asp Tyr Asp Lys Leu Asn Gln Leu Lys Ile Val Thr Met Ile Leu Asn
    370                 375                 380

Glu Val Leu Arg Leu Tyr Pro Ala Gly Tyr Val Ile Asn Arg Met Val
385                 390                 395                 400

Asn Lys Glu Thr Lys Leu Gly Asn Leu Cys Leu Pro Ala Gly Val Gln
                405                 410                 415

Leu Val Leu Pro Thr Met Leu Leu Gln His Asp Thr Glu Ile Trp Gly
            420                 425                 430

Asp Asp Ala Met Glu Phe Asn Pro Glu Arg Phe Ser Asp Gly Ile Ser
        435                 440                 445

Lys Ala Thr Lys Gly Lys Leu Val Phe Phe Pro Phe Ser Trp Gly Pro
    450                 455                 460

Arg Ile Cys Ile Gly Gln Asn Phe Ala Met Leu Glu Ala Lys Met Ala
465                 470                 475                 480

Met Ala Met Ile Leu Lys Thr Tyr Ala Phe Glu Leu Ser Pro Ser Tyr
                485                 490                 495

Ala His Ala Pro His Pro Leu Leu Leu Gln Pro Gln Tyr Gly Ala Gln
            500                 505                 510

Leu Ile Leu Tyr Lys Leu
        515

<210> 221
<211> 1593
<212> DNA
<213> Nicotiana tabacum

<400> 221
atggtttttc ccatagaagc ctttgtagga ctagtaacct tcacatttct cttatacttc 60

ctatggacaa aaaaatctca aaaacttcca aaacccttac caccgaaaat ccccggagga 120

tggccggtaa tcggccacct ttttcacttc aataacgacg gcgacgaccg tccattagct 180

cgaaaactcg gagacttagc tgataaatac ggccccgttt tcacttttcg gctaggtctt 240

ccccttgtgc tagttgtaag cagttacgaa gctacaaaag attgcttctc tacaaatgac 300

gccatttctt ccaatcgtcc agcttttctt tacggcgaat accttggcta caataataca 360

atgctttttc tagcaaatta cggaccttac tggcgaaaaa atcgtaaatt agtcattcag 420

gaagttctct ctgctagtcg tctcgaaaaa ttcaaacaag tgagattcac cagaattcaa 480

acgagcatta agaatttata cactcgaatt aatggaaatt cgagtacgat aaatctaact 540

gattggttag aagaattgaa ttttggtctg atcgtgaaaa tgatcgctgg gaaaaattat 600

gaatccggta aaggagatga acaagtggaa agatttaaga atgcgtttaa ggattttatg 660

gttttatcaa tggaatttgt attatgggat gcatttccaa ttccattatt taaatgggtg 720

gattttcaag gtcatattaa gacaatgaaa aggacattta aggatataga ttctgttttt 780

cagaactggt tagaggaaca tattaataaa agagaaaaaa tggaggttgg tgcagaaggg 840

aatgaacaag atttcattga tgtggtgctt tcaaaattga gtaaagaata tcttgatgaa 900

ggttactctc gtgatactgt cattaaagca acagttttta gtttggtctt ggatgcagca 960

gacacagttg ctcttcacat aaattgggga atgacattat tgataaacaa tcaaaatgcc 1020

ttgatgaaag cacaagaaga gatagacaca aaagttggta aggatagatg ggtagaagag 1080

agtgatatta aggatttagt atacctccaa gctattgtta aaaaggtgtt acgattatat 1140

ccaccaggac ctttgttagt accacatgaa aatgtaaagg attgtgttgt tagtggatat 1200

cacattccta aagggactag attattcgca aacgtcatga aactgcagcg cgatcctaaa 1260

ctcttgtcaa atcctgataa gttcgatcca gagagattca tcgctggtga tattgacttc 1320

cgtggtcacc actatgagtt tatcccatct ggttctggaa gacgatcttg tccggggatg 1380

acttatgcat tgcaagtgga acacctaaca atggcacatt taatccaggg tttcaattac 1440

aaaactccaa atgacgaggt cttggatatg aaggaaggtg caggcataac aatacgtaag 1500

gtaaatccag tggaattgat aataacgcct cgcttggcac ctgagcttta ctaaaaccta 1560

agatctttca tcttggttga tcattgttta ata 1593

<210> 222
<211> 517
<212> PRT
<213> Nicotiana tabacum

<400> 222

Met Val Phe Pro Ile Glu Ala Phe Val Gly Leu Val Thr Phe Thr Phe
1               5                   10                  15

Leu Leu Tyr Phe Leu Trp Thr Lys Lys Ser Gln Lys Leu Pro Lys Pro
            20                  25                  30

Leu Pro Pro Lys Ile Pro Gly Gly Trp Pro Val Ile Gly His Leu Phe
        35                  40                  45

His Phe Asn Asn Asp Gly Asp Asp Arg Pro Leu Ala Arg Lys Leu Gly
    50                  55                  60

Asp Leu Ala Asp Lys Tyr Gly Pro Val Phe Thr Phe Arg Leu Gly Leu
65                  70                  75                  80

Pro Leu Val Leu Val Val Ser Ser Tyr Glu Ala Thr Lys Asp Cys Phe
                85                  90                  95

Ser Thr Asn Asp Ala Ile Phe Ser Asn Arg Pro Ala Phe Leu Tyr Gly
            100                 105                 110

Glu Tyr Leu Gly Tyr Asn Asn Thr Met Leu Phe Leu Ala Asn Tyr Gly
        115                 120                 125

Pro Tyr Trp Arg Lys Asn Arg Lys Leu Val Ile Gln Glu Val Leu Ser
    130                 135                 140

Ala Ser Arg Leu Glu Lys Phe Lys Gln Val Arg Phe Thr Arg Ile Gln
145                 150                 155                 160

Thr Ser Ile Lys Asn Leu Tyr Thr Arg Ile Asn Gly Asn Ser Ser Thr
                165                 170                 175

Ile Asn Leu Thr Asp Trp Leu Glu Glu Leu Asn Phe Gly Leu Ile Val
            180                 185                 190

Lys Met Ile Ala Gly Lys Asn Tyr Glu Ser Gly Lys Gly Asp Glu Gln
        195                 200                 205

Val Glu Arg Phe Lys Asn Ala Phe Lys Asp Phe Met Val Leu Ser Met
    210                 215                 220

Glu Phe Val Leu Trp Asp Ala Phe Pro Ile Pro Leu Phe Lys Trp Val
225                 230                 235                 240

Asp Phe Gln Gly His Ile Lys Thr Met Lys Arg Thr Phe Lys Asp Ile
                245                 250                 255

Asp Ser Val Phe Gln Asn Trp Leu Glu Glu His Ile Asn Lys Arg Glu
            260                 265                 270

Lys Met Glu Val Gly Ala Glu Gly Asn Glu Gln Asp Phe Ile Asp Val
        275                 280                 285

Val Leu Ser Lys Leu Ser Lys Glu Tyr Leu Asp Glu Gly Tyr Ser Arg
    290                 295                 300

Asp Thr Val Ile Lys Ala Thr Val Phe Ser Leu Val Leu Asp Ala Ala
305                 310                 315                 320

Asp Thr Val Ala Leu His Ile Asn Trp Gly Met Thr Leu Leu Ile Asn
                325                 330                 335

Asn Gln Asn Ala Leu Met Lys Ala Gln Glu Glu Ile Asp Thr Lys Val
            340                 345                 350

Gly Lys Asp Arg Trp Val Glu Glu Ser Asp Ile Lys Asp Leu Val Tyr
        355                 360                 365

Leu Gln Ala Ile Val Lys Lys Val Leu Arg Leu Tyr Pro Pro Gly Pro
    370                 375                 380

Leu Leu Val Pro His Glu Asn Val Lys Asp Cys Val Val Ser Gly Tyr
385                 390                 395                 400

His Ile Pro Lys Gly Thr Arg Leu Phe Ala Asn Val Met Lys Leu Gln
                405                 410                 415

Arg Asp Pro Lys Leu Leu Ser Asn Pro Asp Lys Phe Asp Pro Glu Arg
            420                 425                 430

Phe Ile Ala Gly Asp Ile Asp Phe Arg Gly His His Tyr Glu Phe Ile
        435                 440                 445

Pro Ser Gly Ser Gly Arg Arg Ser Cys Pro Gly Met Thr Tyr Ala Leu
    450                 455                 460

Gln Val Glu His Leu Thr Met Ala His Leu Ile Gln Gly Phe Asn Tyr
465                 470                 475                 480

Lys Thr Pro Asn Asp Glu Val Leu Asp Met Lys Glu Gly Ala Gly Ile
                485                 490                 495

Thr Ile Arg Lys Val Asn Pro Val Glu Leu Ile Ile Thr Pro Arg Leu
            500                 505                 510

Ala Pro Glu Leu Tyr
        515

<210> 223
<211> 1580

<212>  DNA
<213>  Nicotiana tabacum

<400>  223
atgctttctc ccatagaagc cattgtagga ctagtaacct tcacatttct cttcttcttc      60

ctatggacaa aaaaatctca aaaaccttca aaacccttac caccgaaaat ccccggagga     120

tggccggtaa tcggccatct tttccacttc aatgacgacg gcgacgaccg tccattagct     180

cgaaaactcg gagacttagc tgacaaatac ggccccgttt tcacttttcg gctaggcctt     240

ccccttgtct tagttgtaag cagttacgaa gctgtaaaag actgtttctc cacaaatgac     300

gccatttttt ccaatcgtcc agcttttctt tacggcgatt accttggcta caataatgcc     360

atgctatttt tggccaatta cggaccttac tggcgaaaaa atcgaaaatt agttattcag     420

gaagttctct ccgctagtcg tctcgaaaaa ttcaaacacg tgagatttgc aagaattcaa     480

gcgagcatga agaatttata tactcgaatt gatggaaatt cgagtacgat aaatttaact     540

gattggttag aagaattgaa ttttggtctg atcgtgaaga tgatcgctgg aaaaaattat     600

gaatccggta aaggagatga acaagtggag agatttaaga aagcgtttaa ggattttatg     660

attttatcaa tggagtttgt gttatgggat gcatttccaa ttccattatt taaatgggtg     720

gattttcaag ggcatgttaa ggctatgaaa aggactttta aagatataga ttctgttttt     780

cagaattggt tagaggaaca tattaataaa agagaaaaaa tggaggttaa tgcagaaggg     840

aatgaacaag atttcattga tgtggtgctt tcaaaaatga gtaatgaata tcttggtgaa     900

ggttactctc gtgatactgt cattgaagca acggtgttta gtttggtctt ggatgcagca     960

gacacagttg ctcttcacat aaattgggga atggcattat tgataaacaa tcaaaaggcc    1020

ttgacgaaag cacaagaaga gatagacaca aaagtttgta aggacagatg ggtagaagag    1080

agtgatatta aggatttggt atacctccaa gctattgtta aagaagtgtt acgattatat    1140

ccaccaggac ctttgttagt accacacgaa aatgtagaag attgtgttgt tagtggatat    1200

cacattccta aagggacaag attattcgca aacgtcatga aactgcaacg tgatcctaaa    1260

ctctggtctg atcctgatac tttcgatcca gagagattca ttgctactga tattgacttt    1320

cgtggtcagt actataagta tatcccgttt ggtcctggaa gacgatcttg tccagggatg    1380

acttatgcat tgcaagtgga acacttaaca atggcacatt tgatccaagg tttcaattac    1440

agaactccaa atgacgagcc cttggatatg aaggaaggtg caggcataac tatacgtaag    1500

gtaaatcctg tggaactgat aatagcgcct cgcctggcac ctgagcttta ttaaaaccta    1560

agatgtttca tcttggttga                                                1580

<210>  224
<211>  517
<212>  PRT
<213>  Nicotiana tabacum

<400>  224

Met Leu Ser Pro Ile Glu Ala Ile Val Gly Leu Val Thr Phe Thr Phe
1               5                   10                  15

Leu Phe Phe Phe Leu Trp Thr Lys Lys Ser Gln Lys Pro Ser Lys Pro
            20                  25                  30

Leu Pro Pro Lys Ile Pro Gly Gly Trp Pro Val Ile Gly His Leu Phe
        35                  40                  45

His Phe Asn Asp Asp Gly Asp Asp Arg Pro Leu Ala Arg Lys Leu Gly
    50                  55                  60

Asp Leu Ala Asp Lys Tyr Gly Pro Val Phe Thr Phe Arg Leu Gly Leu
65                  70                  75                  80

Pro Leu Val Leu Val Val Ser Ser Tyr Glu Ala Val Lys Asp Cys Phe
                85                  90                  95

Ser Thr Asn Asp Ala Ile Phe Ser Asn Arg Pro Ala Phe Leu Tyr Gly
            100                 105                 110

Asp Tyr Leu Gly Tyr Asn Asn Ala Met Leu Phe Leu Ala Asn Tyr Gly
        115                 120                 125

Pro Tyr Trp Arg Lys Asn Arg Lys Leu Val Ile Gln Glu Val Leu Ser
    130                 135                 140

Ala Ser Arg Leu Glu Lys Phe Lys His Val Arg Phe Ala Arg Ile Gln
145                 150                 155                 160

Ala Ser Met Lys Asn Leu Tyr Thr Arg Ile Asp Gly Asn Ser Ser Thr
                165                 170                 175

Ile Asn Leu Thr Asp Trp Leu Glu Glu Leu Asn Phe Gly Leu Ile Val
            180                 185                 190

Lys Met Ile Ala Gly Lys Asn Tyr Glu Ser Gly Lys Gly Asp Glu Gln
        195                 200                 205

Val Glu Arg Phe Lys Lys Ala Phe Lys Asp Phe Met Ile Leu Ser Met
    210                 215                 220

Glu Phe Val Leu Trp Asp Ala Phe Pro Ile Pro Leu Phe Lys Trp Val
225                 230                 235                 240

Asp Phe Gln Gly His Val Lys Ala Met Lys Arg Thr Phe Lys Asp Ile
                245                 250                 255

Asp Ser Val Phe Gln Asn Trp Leu Glu Glu His Ile Asn Lys Arg Glu
            260                 265                 270

Lys Met Glu Val Asn Ala Glu Gly Asn Glu Gln Asp Phe Ile Asp Val
        275                 280                 285

Val Leu Ser Lys Met Ser Asn Glu Tyr Leu Gly Glu Gly Tyr Ser Arg
    290                 295                 300

Asp Thr Val Ile Glu Ala Thr Val Phe Ser Leu Val Leu Asp Ala Ala
305                 310                 315                 320

Asp Thr Val Ala Leu His Ile Asn Trp Gly Met Ala Leu Leu Ile Asn
                325                 330                 335

Asn Gln Lys Ala Leu Thr Lys Ala Gln Glu Glu Ile Asp Thr Lys Val
            340                 345                 350

Cys Lys Asp Arg Trp Val Glu Glu Ser Asp Ile Lys Asp Leu Val Tyr
        355                 360                 365

Leu Gln Ala Ile Val Lys Glu Val Leu Arg Leu Tyr Pro Pro Gly Pro
    370                 375                 380

Leu Leu Val Pro His Glu Asn Val Glu Asp Cys Val Val Ser Gly Tyr
385                 390                 395                 400

His Ile Pro Lys Gly Thr Arg Leu Phe Ala Asn Val Met Lys Leu Gln
                405                 410                 415

Arg Asp Pro Lys Leu Trp Ser Asp Pro Asp Thr Phe Asp Pro Glu Arg
            420                 425                 430

Phe Ile Ala Thr Asp Ile Asp Phe Arg Gly Gln Tyr Tyr Lys Tyr Ile
        435                 440                 445

Pro Phe Gly Pro Gly Arg Arg Ser Cys Pro Gly Met Thr Tyr Ala Leu
    450                 455                 460

Gln Val Glu His Leu Thr Met Ala His Leu Ile Gln Gly Phe Asn Tyr
465                 470                 475                 480

Arg Thr Pro Asn Asp Glu Pro Leu Asp Met Lys Glu Gly Ala Gly Ile
                485                 490                 495

Thr Ile Arg Lys Val Asn Pro Val Glu Leu Ile Ile Ala Pro Arg Leu
            500                 505                 510

Ala Pro Glu Leu Tyr
        515

<210>  225
<211>  1639
<212>  DNA
<213>  Nicotiana tabacum

<400>  225

tttcatatac ctttagtact cttgaaattt tcaaataatg gtttatcttc tttctcccat 60
agaagccatt gtaggatttg taaccttttc atttctattc tactttctat ggaccaaaaa 120
acaatcaaaa atcttaaacc cactacctcc aaaaatccca ggtggatggc cagtaatcgg 180
ccatctcttt tatttcaaga acaatggcga tgaagatcgc catttttctc aaaaactcgg 240
tgacttagct gacaaatatg gtcccgtctt cactttccgg ttagggtttc gccgtttctt 300
ggcggtgagt agttatgaag ctatgaaaga atgcttcact accaatgata tccatttcgc 360
cgatcggcca tctttactct acggagaata cctttgctat aataacgcca tgcttgctgt 420
tgccaaatat ggcccttact ggaaaaaaaa tcgaaagtta gtcaatcaag aagttctctc 480
cgttagtcgg ctcgaaaaat tcaaacatgt tagattttct ataattcaga aaaatattaa 540
acaattgtat aattgtgatt caccaatggt gaagataaac cttagtgatt ggatagataa 600
attgacattc gacatcattt tgaaaatggt tgttgggaag aactataata atggacatgg 660
agaaatactc aaagttgctt ttcagaaatt catggttcaa gctatggaga tggagctcta 720
tgatgttttt cacattccat ttttcaagtg gttggatctt acagggaata ttaaggctat 780
gaaacaaact ttcaaagaca ttgataatat tatccaaggt tggttagatg agcacattaa 840
gaagagagaa acaaaggatg ttggaggtga aaacgaacaa gattttatag atgtggtgct 900
ttccaagatg agcgacgaac atcttggcga gggttactct catgacacaa ccatcaaagc 960
aactgtattc actttggtct tggatgcaac agacacactt gcacttcata taaagtgggt 1020
aatggcgtta atgataaaca ataagcatgt catgaagaaa gcacaagaag agatggacac 1080
aattgttggt agagatagat gggtagaaga gagtgatatc aagaatttgg tgtatctcca 1140
agcaattgtc aaagaagtat tacgattaca tccacccgca cctttgtcag tgcaacacct 1200
atctgtagaa gattgtgttg tcaatgggta ccatattcct aaggggactg cactacttac 1260
caatattatg aaactacagc gagatcctca aacatggcca aatcctgata aattcgatcc 1320
agagagattc ctgacgactc atgctactat tgactaccgc gggcagcact atgagtcgat 1380
cccctttggt acggggagac gagcttgtcc cgcgatgaat tattcattgc aagtggaaca 1440
cctttcaatt gctcatatga tccaaggttt cagttttgca actacgacca atgagccttt 1500
ggatatgaaa caaggtgtgg gtttaacttt accaaagaag actgatgttg aagtgctaat 1560
tacacctcgc cttcctccta cgctttatca atattaagat gttttgttgt cgggattcgt 1620
tctgatcaat ccctcaatg 1639

<210> 226
<211> 519
<212> PRT
<213> Nicotiana tabacum

<400> 226

Met Val Tyr Leu Leu Ser Pro Ile Glu Ala Ile Val Gly Phe Val Thr
1               5                   10                  15

Phe Ser Phe Leu Phe Tyr Phe Leu Trp Thr Lys Lys Gln Ser Lys Ile
            20                  25                  30

Leu Asn Pro Leu Pro Pro Lys Ile Pro Gly Gly Trp Pro Val Ile Gly
        35                  40                  45

His Leu Phe Tyr Phe Lys Asn Asn Gly Asp Glu Asp Arg His Phe Ser
    50                  55                  60

Gln Lys Leu Gly Asp Leu Ala Asp Lys Tyr Gly Pro Val Phe Thr Phe
65                  70                  75                  80

Arg Leu Gly Phe Arg Arg Phe Leu Ala Val Ser Ser Tyr Glu Ala Met
                85                  90                  95

Lys Glu Cys Phe Thr Thr Asn Asp Ile His Phe Ala Asp Arg Pro Ser
            100                 105                 110

Leu Leu Tyr Gly Glu Tyr Leu Cys Tyr Asn Asn Ala Met Leu Ala Val
        115                 120                 125

Ala Lys Tyr Gly Pro Tyr Trp Lys Lys Asn Arg Lys Leu Val Asn Gln
    130                 135                 140

Glu Val Leu Ser Val Ser Arg Leu Glu Lys Phe Lys His Val Arg Phe
145                 150                 155                 160

Ser Ile Ile Gln Lys Asn Ile Lys Gln Leu Tyr Asn Cys Asp Ser Pro
                165                 170                 175

Met Val Lys Ile Asn Leu Ser Asp Trp Ile Asp Lys Leu Thr Phe Asp
            180                 185                 190

Ile Ile Leu Lys Met Val Val Gly Lys Asn Tyr Asn Asn Gly His Gly
        195                 200                 205

Glu Ile Leu Lys Val Ala Phe Gln Lys Phe Met Val Gln Ala Met Glu
    210                 215                 220

Met Glu Leu Tyr Asp Val Phe His Ile Pro Phe Phe Lys Trp Leu Asp
225                 230                 235                 240

Leu Thr Gly Asn Ile Lys Ala Met Lys Gln Thr Phe Lys Asp Ile Asp
                245                 250                 255

Asn Ile Ile Gln Gly Trp Leu Asp Glu His Ile Lys Lys Arg Glu Thr
            260                 265                 270

Lys Asp Val Gly Gly Glu Asn Glu Gln Asp Phe Ile Asp Val Val Leu
        275                 280                 285

Ser Lys Met Ser Asp Glu His Leu Gly Glu Gly Tyr Ser His Asp Thr
    290                 295                 300

Thr Ile Lys Ala Thr Val Phe Thr Leu Val Leu Asp Ala Thr Asp Thr
305                 310                 315                 320

Leu Ala Leu His Ile Lys Trp Val Met Ala Leu Met Ile Asn Asn Lys
                325                 330                 335

His Val Met Lys Lys Ala Gln Glu Glu Met Asp Thr Ile Val Gly Arg
            340                 345                 350

Asp Arg Trp Val Glu Glu Ser Asp Ile Lys Asn Leu Val Tyr Leu Gln
        355                 360                 365

Ala Ile Val Lys Glu Val Leu Arg Leu His Pro Pro Ala Pro Leu Ser
    370                 375                 380

Val Gln His Leu Ser Val Glu Asp Cys Val Val Asn Gly Tyr His Ile
385                 390                 395                 400

Pro Lys Gly Thr Ala Leu Leu Thr Asn Ile Met Lys Leu Gln Arg Asp
                405                 410                 415

Pro Gln Thr Trp Pro Asn Pro Asp Lys Phe Asp Pro Glu Arg Phe Leu
            420                 425                 430

Thr Thr His Ala Thr Ile Asp Tyr Arg Gly Gln His Tyr Glu Ser Ile
        435                 440                 445

Pro Phe Gly Thr Gly Arg Arg Ala Cys Pro Ala Met Asn Tyr Ser Leu
    450                 455                 460

Gln Val Glu His Leu Ser Ile Ala His Met Ile Gln Gly Phe Ser Phe
465                 470                 475                 480

Ala Thr Thr Thr Asn Glu Pro Leu Asp Met Lys Gln Gly Val Gly Leu
                485                 490                 495

Thr Leu Pro Lys Lys Thr Asp Val Glu Val Leu Ile Thr Pro Arg Leu
            500                 505                 510

Pro Pro Thr Leu Tyr Gln Tyr
        515

<210>  227
<211>  1610
<212>  DNA
<213>  Nicotiana tabacum

<400>  227
ctttttctcc ccaaaaaaga gctcatttcc cttgtcccca aaaatggatc ttctcttact      60

agagaagacc ttaattggtc tcttctttgc cattttaatc gctataattg tctctagact 120
tcgttcaaag cgttttaagc ttcccccagg accaatccca gtaccagttt ttggtaattg 180
gcttcaagtt ggtgatgatt taaaccacag aaatcttact gattttgcca aaaaatttgg 240
tgatcttttc ttgttaagaa tgggccagcg taatttagtt gttgtgtcat ctcctgaatt 300
agctaaagaa gttttacaca cacaaggtgt tgaatttggt tcaagaacaa gaaatgttgt 360
atttgatatt tttactggaa aaggtcaaga tatggttttt actgtatatg gtgaacactg 420
gagaaaaatg aggagaatta tgactgtacc attttttact aataaagttg tgcagcaata 480
tagagggggg tgggagtttg aagtggcaag tgtaattgag gatgtgaaga aaaatcctga 540
atctgctact aatgggattg tattaaggag gagattacaa ttgatgatgt ataataatat 600
gtttaggatt atgtttgata ggagatttga gagtgaagat gatcctttgt ttgttaagct 660
taaggctttg aatggtgaaa ggagtagatt ggctcagagt tttgagtata attatggtga 720
ttttattccc attttgaggc cttttttgag aggttatttg aagatctgta aagaagttaa 780
ggagaagagg ctgcagcttt tcaaagatta ctttgttgat gaaagaaaga agctttcaaa 840
taccaagagc ttggacagca atgctctgaa atgtgcgatt gatcacattc ttgaggctca 900
acagaagggg gagatcaatg aggacaacgt tctttacatt gttgaaaaca tcaatgttgc 960
tgctatagaa accacattat ggtcaattga gtggggtatc gccgagttag tcaaccaccc 1020
tcacatccaa aagaaactcc gcgacgagat tgacacagtt cttggcccag gagtgcaagt 1080
gactgaacca gacacccaca agcttccata ccttcaggct gtgatcaagg agacgcttcg 1140
tctccgtatg gcaattcctc tattagtccc acacatgaac cttcacgatg caaagcttgg 1200
cgggtttgat attccagcag agagcaaaat cttggttaac gcttggtggc tagctaacaa 1260
cccggctcat tggaagaaac ccgaagagtt cagacccgag aggttcttcg aagaggagaa 1320
gcacgttgag gccaatggca atgacttcag atatcttccg tttggcgttg gtaggaggag 1380
ttgccctgga attatacttg cattgccaat tcttggcatt actttgggac gtttggttca 1440
gaactttgag ctgttgcctc ctccaggcca gtcgaagctc gacaccacag agaaaggtgg 1500
acagttcagt ctccatattt tgaagcattc caccattgtg ttgaaaccaa ggtcttgctg 1560
aactttctga tcctaatcaa ttaaggggtt gaagaaattt tataattatg 1610

<210> 228
<211> 505
<212> PRT
<213> Nicotiana tabacum

<400> 228

Met Asp Leu Leu Leu Leu Glu Lys Thr Leu Ile Gly Leu Phe Phe Ala
1               5                   10                  15

Ile Leu Ile Ala Ile Ile Val Ser Arg Leu Arg Ser Lys Arg Phe Lys
            20                  25                  30

Leu Pro Pro Gly Pro Ile Pro Val Pro Val Phe Gly Asn Trp Leu Gln
        35                  40                  45

Val Gly Asp Asp Leu Asn His Arg Asn Leu Thr Asp Phe Ala Lys Lys
    50                  55                  60

Phe Gly Asp Leu Phe Leu Leu Arg Met Gly Gln Arg Asn Leu Val Val
65                  70                  75                  80

Val Ser Ser Pro Glu Leu Ala Lys Glu Val Leu His Thr Gln Gly Val
                85                  90                  95

Glu Phe Gly Ser Arg Thr Arg Asn Val Val Phe Asp Ile Phe Thr Gly
            100                 105                 110

Lys Gly Gln Asp Met Val Phe Thr Val Tyr Gly Glu His Trp Arg Lys
        115                 120                 125

Met Arg Arg Ile Met Thr Val Pro Phe Phe Thr Asn Lys Val Val Gln
    130                 135                 140

Gln Tyr Arg Gly Gly Trp Glu Phe Glu Val Ala Ser Val Ile Glu Asp
145                 150                 155                 160

Val Lys Lys Asn Pro Glu Ser Ala Thr Asn Gly Ile Val Leu Arg Arg
                165                 170                 175

Arg Leu Gln Leu Met Met Tyr Asn Asn Met Phe Arg Ile Met Phe Asp
            180                 185                 190

Arg Arg Phe Glu Ser Glu Asp Asp Pro Leu Phe Val Lys Leu Lys Ala
        195                 200                 205

Leu Asn Gly Glu Arg Ser Arg Leu Ala Gln Ser Phe Glu Tyr Asn Tyr
    210                 215                 220

Gly Asp Phe Ile Pro Ile Leu Arg Pro Phe Leu Arg Gly Tyr Leu Lys
225                 230                 235                 240

Ile Cys Lys Glu Val Lys Glu Lys Arg Leu Gln Leu Phe Lys Asp Tyr
                245                 250                 255

Phe Val Asp Glu Arg Lys Lys Leu Ser Asn Thr Lys Ser Leu Asp Ser
            260                 265                 270

Asn Ala Leu Lys Cys Ala Ile Asp His Ile Leu Glu Ala Gln Gln Lys
        275                 280                 285

Gly Glu Ile Asn Glu Asp Asn Val Leu Tyr Ile Val Glu Asn Ile Asn
    290                 295                 300

Val Ala Ala Ile Glu Thr Thr Leu Trp Ser Ile Glu Trp Gly Ile Ala
305                 310                 315                 320

Glu Leu Val Asn His Pro His Ile Gln Lys Lys Leu Arg Asp Glu Ile
                325                 330                 335

Asp Thr Val Leu Gly Pro Gly Val Gln Val Thr Glu Pro Asp Thr His
            340                 345                 350

Lys Leu Pro Tyr Leu Gln Ala Val Ile Lys Glu Thr Leu Arg Leu Arg
        355                 360                 365

Met Ala Ile Pro Leu Leu Val Pro His Met Asn Leu His Asp Ala Lys
    370                 375                 380

Leu Gly Gly Phe Asp Ile Pro Ala Glu Ser Lys Ile Leu Val Asn Ala
385                 390                 395                 400

Trp Trp Leu Ala Asn Asn Pro Ala His Trp Lys Lys Pro Glu Glu Phe
                405                 410                 415

Arg Pro Glu Arg Phe Phe Glu Glu Glu Lys His Val Glu Ala Asn Gly
            420                 425                 430

Asn Asp Phe Arg Tyr Leu Pro Phe Gly Val Gly Arg Arg Ser Cys Pro
        435                 440                 445

Gly Ile Ile Leu Ala Leu Pro Ile Leu Gly Ile Thr Leu Gly Arg Leu
    450                 455                 460

Val Gln Asn Phe Glu Leu Leu Pro Pro Pro Gly Gln Ser Lys Leu Asp
465                 470                 475                 480

Thr Thr Glu Lys Gly Gly Gln Phe Ser Leu His Ile Leu Lys His Ser
                485                 490                 495

Thr Ile Val Leu Lys Pro Arg Ser Cys
            500                 505

<210>  229
<211>  1595
<212>  DNA
<213>  Nicotiana tabacum

<400>  229
aacaataaaa atggagacat tatttaacat caaagttgca gtttcattag taattgtgat      60

aatttttctg agatgggtat ggaaattctt gaattgggtg tggattcaac caaagaaaat     120

ggaaaaaaga ctaaaaatgg aaggtttcaa aggaagctca tataagctat tatttggaga     180

tatgaaagaa ataaatacaa tggttgaaga agccaaaacc aagcctatga attttaccaa     240

tgattatgtg gctagagtct tgcctcactt cacaaagttg atgctccaat atggcaagaa     300

tagctttatg tggttagggc caaaaccaac aatgtttatc acagaccctg aactaataag 360
ggagatcttg tcaaaaagtt acatatacca ggagattcaa ggcaatccaa tcactaagtt 420
gctagcacaa ggactagtaa gttatgaagc agagaaatgg gctaagcata gaaaaattat 480
caatcctgca tttcaccttg acaagttgaa gcatatgcta ccatcattct acttgagttg 540
ttgtgacatg ctcagaaaat gggaaagtat agcttcatca gagggatcag aaatagacgt 600
gtggcctttt ctggaaacgt tgacaagcga tgctatttca agaacagctt ttggtagtaa 660
ctatgaagac gggagacaga tatttgagct tcaaaaagaa caagctgagt tgattttaca 720
agcagcgcga tggctttaca tccccggatg gaggtttgtg ccaacaaaga ggaacaagag 780
gatgaagcaa atcgctaaag aagtacgatc attagtgttg ggaataatca ataagagaat 840
aagggaaatg aaagcagggg aagctgcaaa agatgactta ctgggaatac tattggaatc 900
taatttcaaa gaaatccaaa tgcacggaaa caagaacttt ggcatgacta tcgacgaagt 960
gattgaagag tgcaagttat tttactttgc tgggcaagaa actacttcag ttttgcttgt 1020
ttggactttg attttactga gtaagcatgt cgattggcaa gaaagagcta gagaagaagt 1080
tcatcaagtc tttggaagta acaaacctga ttatgacgca ttgaatcagt tgaaagttgt 1140
aacgatgata ttcaacgagg ttttaaggtt gtacccaccg ggaattacca taagtcgaac 1200
tgtacacgag gataccaaat tagggaactt gtcattgcca gcagggatac agcttgtgtt 1260
acctgcaatt tggttgcatc atgacaatga aatatgggga gatgatgcaa aggagttcaa 1320
accagagagg tttagtgaag gagttaataa agcaacaaag ggtaaatttg catattttcc 1380
atttagttgg ggaccaagaa tatgtgttgg actgaatttt gcaatgttag aggcaaaaat 1440
ggcacttgca ttgattctac aacactatgc ttttgagctc tctccatctt atgcacatgc 1500
tcctcataca attatcactc tgcaacctca acatggtgct cctttgattt tgcgcaagct 1560
gtagcgcgga tatattgatt ggttatctac tgtag 1595

<210> 230
<211> 517
<212> PRT
<213> Nicotiana tabacum

<400> 230

Met Glu Thr Leu Phe Asn Ile Lys Val Ala Val Ser Leu Val Ile Val
1               5                   10                  15

Ile Ile Phe Leu Arg Trp Val Trp Lys Phe Leu Asn Trp Val Trp Ile
            20                  25                  30

Gln Pro Lys Lys Met Glu Lys Arg Leu Lys Met Glu Gly Phe Lys Gly
        35                  40                  45

Ser Ser Tyr Lys Leu Leu Phe Gly Asp Met Lys Glu Ile Asn Thr Met
    50                  55                  60

Val Glu Glu Ala Lys Thr Lys Pro Met Asn Phe Thr Asn Asp Tyr Val
65                  70                  75                  80

Ala Arg Val Leu Pro His Phe Thr Lys Leu Met Leu Gln Tyr Gly Lys
                85                  90                  95

Asn Ser Phe Met Trp Leu Gly Pro Lys Pro Thr Met Phe Ile Thr Asp
            100                 105                 110

Pro Glu Leu Ile Arg Glu Ile Leu Ser Lys Ser Tyr Ile Tyr Gln Glu
        115                 120                 125

Ile Gln Gly Asn Pro Ile Thr Lys Leu Leu Ala Gln Gly Leu Val Ser
    130                 135                 140

Tyr Glu Ala Glu Lys Trp Ala Lys His Arg Lys Ile Ile Asn Pro Ala
145                 150                 155                 160

Phe His Leu Asp Lys Leu Lys His Met Leu Pro Ser Phe Tyr Leu Ser
                165                 170                 175

Cys Cys Asp Met Leu Arg Lys Trp Glu Ser Ile Ala Ser Ser Glu Gly
            180                 185                 190

Ser Glu Ile Asp Val Trp Pro Phe Leu Glu Thr Leu Thr Ser Asp Ala
        195                 200                 205

Ile Ser Arg Thr Ala Phe Gly Ser Asn Tyr Glu Asp Gly Arg Gln Ile
    210                 215                 220

Phe Glu Leu Gln Lys Glu Gln Ala Glu Leu Ile Leu Gln Ala Ala Arg
225                 230                 235                 240

Trp Leu Tyr Ile Pro Gly Trp Arg Phe Val Pro Thr Lys Arg Asn Lys
                245                 250                 255

Arg Met Lys Gln Ile Ala Lys Glu Val Arg Ser Leu Val Leu Gly Ile
            260                 265                 270

Ile Asn Lys Arg Ile Arg Glu Met Lys Ala Gly Glu Ala Ala Lys Asp
        275                 280                 285

Asp Leu Leu Gly Ile Leu Leu Glu Ser Asn Phe Lys Glu Ile Gln Met
    290                 295                 300

His Gly Asn Lys Asn Phe Gly Met Thr Ile Asp Glu Val Ile Glu Glu
305                 310                 315                 320

Cys Lys Leu Phe Tyr Phe Ala Gly Gln Glu Thr Thr Ser Val Leu Leu
                325                 330                 335

Val Trp Thr Leu Ile Leu Leu Ser Lys His Val Asp Trp Gln Glu Arg
            340                 345                 350

Ala Arg Glu Glu Val His Gln Val Phe Gly Ser Asn Lys Pro Asp Tyr
        355                 360                 365

Asp Ala Leu Asn Gln Leu Lys Val Val Thr Met Ile Phe Asn Glu Val
    370                 375                 380

Leu Arg Leu Tyr Pro Pro Gly Ile Thr Ile Ser Arg Thr Val His Glu
385                 390                 395                 400

Asp Thr Lys Leu Gly Asn Leu Ser Leu Pro Ala Gly Ile Gln Leu Val
                405                 410                 415

Leu Pro Ala Ile Trp Leu His His Asp Asn Glu Ile Trp Gly Asp Asp
            420                 425                 430

Ala Lys Glu Phe Lys Pro Glu Arg Phe Ser Glu Gly Val Asn Lys Ala
        435                 440                 445

Thr Lys Gly Lys Phe Ala Tyr Phe Pro Phe Ser Trp Gly Pro Arg Ile
    450                 455                 460

Cys Val Gly Leu Asn Phe Ala Met Leu Glu Ala Lys Met Ala Leu Ala
465                 470                 475                 480

Leu Ile Leu Gln His Tyr Ala Phe Glu Leu Ser Pro Ser Tyr Ala His
                485                 490                 495

Ala Pro His Thr Ile Ile Thr Leu Gln Pro Gln His Gly Ala Pro Leu
            500                 505                 510

Ile Leu Arg Lys Leu
        515

<210>  231
<211>  1673
<212>  DNA
<213>  Nicotiana tabacum

<400>  231
atatgcaact gagatttgaa gaataccaac taaccaaaat gcagttcttc agcttggttt      60

ccattttcct atttctatct ttcctctttt tgttaaggat atggaagaac tccaatagcc     120

aaagcaaaaa gttgccacca ggtccatgga aactaccaat actaggaagt atgcttcata     180

tggttggtgg actaccacac catgtcctta gagatttagc caaaaaatat ggaccactta     240

tgcaccttca attaggtgaa gtttctgcgg ttgtggttac ttctcctgat acggcaaaag     300

aagtattaaa aactcatgac atcgcttttg cgtctaggcc tagccttttg gccccggaga     360

ttgtctgtta caataggtct gatctagcct tttgccccta tggcgactat tggagacaaa     420

tgcgtaaaat atgtgtcttg gaagtgctca gtgccaagaa tgttcggaca tttagctcta 480
ttaggcggaa tgaagttctt cgtctcatta attttatccg gtcatcttct ggtgaaccta 540
ttaatgttac ggaaaggatc tttttgttca caagctccat gacatgtaga tcagcgtttg 600
ggcaagtgtt caaagagcaa gacaaattta tacaactaat taaagaagtg atactcttag 660
caggagggtt tgatgtggct gacatattcc cttcactgaa gtttcttcat gtgctcagtg 720
gaatgaaggg taagattatg aatgcacacc ataaggtaga tgccattgtt gagaatgtca 780
tcaatgagca caagaaaaat cttgcaattg ggaaaactaa tggagcgtta ggaggtgaag 840
atttaattga tgttcttcta agacttatga atgatggagg ccttcaattt cctatcacca 900
acgacaacat caaagccata atttttgaca tgtttgctgc cgggacagag acttcatcgt 960
caacaattgt gtgggctatg gtagaaatgg tgaaaaatcc agccgtattc gcgaaagctc 1020
aagcagaagt aagagaagca tttagaggaa aagaaacttt cgatgaaaat gatgtggagg 1080
agctaaacta cctaaagtta gtaataaaag aaactctaag acttcatcca ccggttccac 1140
ttttgctccc aagagaatgt agggaagaga caaatataaa cggctacact attcctgtaa 1200
agaccaaagt catggttaat gtttgggctt tgggaagaga tccaaaatat tggaatgacg 1260
cagaaacttt tatgccagag agatttgagc agtgctctaa ggattttgtt ggtaataatt 1320
ttgaatatct tccatttggt ggcggaagga ggatttgtcc tgggatttcg tttggcttag 1380
ctaatgctta tttgccattg gctcaattac tatatcactt cgattggaaa ctccctgctg 1440
gaatcgaacc aagcgacttg gacttgactg agttggttgg agtaactgcc gctagaaaaa 1500
gtgaccttta cttggttgcg actccttatc aacctcctca aaagtgattt aatggtttca 1560
agtttttatt tcctagcaaa ccccactatt gtcctatctt tcttttggtg ttttcggttt 1620
tatctactct aatacatgca tcttttacca tataggaatg taccatgttg tcg 1673

<210> 232
<211> 514
<212> PRT
<213> Nicotiana tabacum

<400> 232

Met Gln Leu Arg Phe Glu Glu Tyr Gln Leu Thr Lys Met Gln Phe Phe
1               5                   10                  15

Ser Leu Val Ser Ile Phe Leu Phe Leu Ser Phe Leu Phe Leu Leu Arg
            20                  25                  30

Ile Trp Lys Asn Ser Asn Ser Gln Ser Lys Lys Leu Pro Pro Gly Pro
        35                  40                  45

Trp Lys Leu Pro Ile Leu Gly Ser Met Leu His Met Val Gly Gly Leu
    50                  55                  60

Pro His His Val Leu Arg Asp Leu Ala Lys Lys Tyr Gly Pro Leu Met
65                  70                  75                  80

His Leu Gln Leu Gly Glu Val Ser Ala Val Val Val Thr Ser Pro Asp
                85                  90                  95

Thr Ala Lys Glu Val Leu Lys Thr His Asp Ile Ala Phe Ala Ser Arg
            100                 105                 110

Pro Ser Leu Leu Ala Pro Glu Ile Val Cys Tyr Asn Arg Ser Asp Leu
        115                 120                 125

Ala Phe Cys Pro Tyr Gly Asp Tyr Trp Arg Gln Met Arg Lys Ile Cys
    130                 135                 140

Val Leu Glu Val Leu Ser Ala Lys Asn Val Arg Thr Phe Ser Ser Ile
145                 150                 155                 160

Arg Arg Asn Glu Val Leu Arg Leu Ile Asn Phe Ile Arg Ser Ser Ser
                165                 170                 175

Gly Glu Pro Ile Asn Val Thr Glu Arg Ile Phe Leu Phe Thr Ser Ser
            180                 185                 190

Met Thr Cys Arg Ser Ala Phe Gly Gln Val Phe Lys Glu Gln Asp Lys
        195                 200                 205

Phe Ile Gln Leu Ile Lys Glu Val Ile Leu Leu Ala Gly Gly Phe Asp
    210                 215                 220

Val Ala Asp Ile Phe Pro Ser Leu Lys Phe Leu His Val Leu Ser Gly
225                 230                 235                 240

Met Lys Gly Lys Ile Met Asn Ala His His Lys Val Asp Ala Ile Val
                245                 250                 255

Glu Asn Val Ile Asn Glu His Lys Lys Asn Leu Ala Ile Gly Lys Thr
            260                 265                 270

Asn Gly Ala Leu Gly Gly Glu Asp Leu Ile Asp Val Leu Leu Arg Leu
        275                 280                 285

Met Asn Asp Gly Gly Leu Gln Phe Pro Ile Thr Asn Asp Asn Ile Lys
    290                 295                 300

Ala Ile Ile Phe Asp Met Phe Ala Ala Gly Thr Glu Thr Ser Ser Ser
305                 310                 315                 320

Thr Ile Val Trp Ala Met Val Glu Met Val Lys Asn Pro Ala Val Phe
                325                 330                 335

Ala Lys Ala Gln Ala Glu Val Arg Glu Ala Phe Arg Gly Lys Glu Thr
            340                 345                 350

Phe Asp Glu Asn Asp Val Glu Glu Leu Asn Tyr Leu Lys Leu Val Ile
        355                 360                 365

Lys Glu Thr Leu Arg Leu His Pro Pro Val Pro Leu Leu Leu Pro Arg
    370                 375                 380

Glu Cys Arg Glu Glu Thr Asn Ile Asn Gly Tyr Thr Ile Pro Val Lys
385                 390                 395                 400

Thr Lys Val Met Val Asn Val Trp Ala Leu Gly Arg Asp Pro Lys Tyr
                405                 410                 415

Trp Asn Asp Ala Glu Thr Phe Met Pro Glu Arg Phe Glu Gln Cys Ser
            420                 425                 430

Lys Asp Phe Val Gly Asn Asn Phe Glu Tyr Leu Pro Phe Gly Gly Gly
        435                 440                 445

Arg Arg Ile Cys Pro Gly Ile Ser Phe Gly Leu Ala Asn Ala Tyr Leu
    450                 455                 460

Pro Leu Ala Gln Leu Leu Tyr His Phe Asp Trp Lys Leu Pro Ala Gly
465                 470                 475                 480

Ile Glu Pro Ser Asp Leu Asp Leu Thr Glu Leu Val Gly Val Thr Ala
                485                 490                 495

Ala Arg Lys Ser Asp Leu Tyr Leu Val Ala Thr Pro Tyr Gln Pro Pro
            500                 505                 510

Gln Lys

<210>  233
<211>  1601
<212>  DNA
<213>  Nicotiana tabacum

<400>  233
gaattatttc acgtgttgta ttccttgtct atgataggaa gctcgttacc tcagcgtaca      60

aaccccaaat aaaaaatgaa tttccttgtg gtgttagctt ctctctttct ctttgtgttc     120

ctaatgagga taagcaaagc aaaaaagctc cctccaggtc caaggaaact gcctataata     180

ggaaaccttc atcaaattgg aaaattacct catcgttcac ttcaaaaact ttctaatgaa     240

tatggggatt tcattttctt gcaattaggt tctgtaccga ctgtggttgt ctcctcagct     300

gacattgccc gagagatctt tagaactcac gaccttgttt tctcaggccg tcctgcttta     360

tatgctgcca gaaaactttc ctacaattgc tacaacgttt catttgcacc ctatggtaat     420

tactggagag aggctcggaa aattctagtg ttggagttgc taagtacaaa gagagtacaa     480

agtttcgagg caattcgaga cgaggaagta agtagcttgg ttcaaattat ctgtagttcc 540
ttgagctcac ctgttaacat aagcacatta gcactatcct tggcaaataa cgttgtttgt 600
cgagtggctt ttgggaaagg gagtgctgaa ggaggaaatg attatgagga taggaagttt 660
aatgaaattc tatatgagac acaagaatta ttgggtgagt ttaacgttgc tgattatttt 720
cctcggatgg catggattaa caaaataaat gggtttgatg aacgattgga aaataatttt 780
agggaattgg ataagtttta tgacaaagta atagaagatc atcttaattc atgtagctgg 840
atgaaacaaa gggatgatga agacgttatt gatgtattgc ttcgaattca aaaggatcca 900
agccaagaaa ttcctctcaa agatgatcac attaagggcc ttcttgcgga tatattcata 960
gctggaactg atacatcatc aacaaccata gaatgggcaa tgtcagaact cataaaaaat 1020
ccaagagtct tgagaaaagc tcaagaggaa gttagagaag tttctaaggg aaaacaaaag 1080
gtccaagaaa gtgatctttg caaactagat tacttgaaat tggtcatcaa agaaaccttt 1140
agactacacc caccagtccc attactagtc cctcgagtaa caacagccag ctgcaaaata 1200
atggaatacg aaattccagt aaatacaaga gtcttcatca acgcgacagc aaatgggaca 1260
aatccaaaat actgggaaaa tccattgaca ttcttgccag agagattctt ggataaggag 1320
attgattaca gaggcaaaaa ttttgagttg ttgccatttg gggcagggag aagagggtgt 1380
ccaggaatta atttttcaat accacttgtt gagcttgcac ttgctaatct attgtttcat 1440
tataattggt cacttcctga agggatgcta gctaaggatg ttgatatgga agaagctttg 1500
gggattacca tgcacaagaa atctcccctt tgcttagtag cttctcatta tacttgttga 1560
gattttaaaa gattttagca tagctatata tagcttgaag t 1601

<210> 234
<211> 494
<212> PRT
<213> Nicotiana tabacum

<400> 234

Met Asn Phe Leu Val Val Leu Ala Ser Leu Phe Leu Phe Val Phe Leu
1               5                   10                  15

Met Arg Ile Ser Lys Ala Lys Lys Leu Pro Pro Gly Pro Arg Lys Leu
            20                  25                  30

Pro Ile Ile Gly Asn Leu His Gln Ile Gly Lys Leu Pro His Arg Ser
        35                  40                  45

Leu Gln Lys Leu Ser Asn Glu Tyr Gly Asp Phe Ile Phe Leu Gln Leu
    50                  55                  60

Gly Ser Val Pro Thr Val Val Val Ser Ser Ala Asp Ile Ala Arg Glu
65                  70                  75                  80

Ile Phe Arg Thr His Asp Leu Val Phe Ser Gly Arg Pro Ala Leu Tyr
                85                  90                  95

Ala Ala Arg Lys Leu Ser Tyr Asn Cys Tyr Asn Val Ser Phe Ala Pro
            100                 105                 110

Tyr Gly Asn Tyr Trp Arg Glu Ala Arg Lys Ile Leu Val Leu Glu Leu
        115                 120                 125

Leu Ser Thr Lys Arg Val Gln Ser Phe Glu Ala Ile Arg Asp Glu Glu
    130                 135                 140

Val Ser Ser Leu Val Gln Ile Ile Cys Ser Ser Leu Ser Ser Pro Val
145                 150                 155                 160

Asn Ile Ser Thr Leu Ala Leu Ser Leu Ala Asn Asn Val Val Cys Arg
                165                 170                 175

Val Ala Phe Gly Lys Gly Ser Ala Glu Gly Gly Asn Asp Tyr Glu Asp
            180                 185                 190

Arg Lys Phe Asn Glu Ile Leu Tyr Glu Thr Gln Glu Leu Leu Gly Glu
        195                 200                 205

Phe Asn Val Ala Asp Tyr Phe Pro Arg Met Ala Trp Ile Asn Lys Ile
    210                 215                 220

Asn Gly Phe Asp Glu Arg Leu Glu Asn Asn Phe Arg Glu Leu Asp Lys
225                 230                 235                 240

Phe Tyr Asp Lys Val Ile Glu Asp His Leu Asn Ser Cys Ser Trp Met
                245                 250                 255

Lys Gln Arg Asp Asp Glu Asp Val Ile Asp Val Leu Leu Arg Ile Gln
            260                 265                 270

Lys Asp Pro Ser Gln Glu Ile Pro Leu Lys Asp Asp His Ile Lys Gly
        275                 280                 285

Leu Leu Ala Asp Ile Phe Ile Ala Gly Thr Asp Thr Ser Ser Thr Thr
    290                 295                 300

Ile Glu Trp Ala Met Ser Glu Leu Ile Lys Asn Pro Arg Val Leu Arg
305                 310                 315                 320

Lys Ala Gln Glu Glu Val Arg Glu Val Ser Lys Gly Lys Gln Lys Val
                325                 330                 335

Gln Glu Ser Asp Leu Cys Lys Leu Asp Tyr Leu Lys Leu Val Ile Lys
            340                 345                 350

Glu Thr Phe Arg Leu His Pro Pro Val Pro Leu Leu Val Pro Arg Val
        355                 360                 365

Thr Thr Ala Ser Cys Lys Ile Met Glu Tyr Glu Ile Pro Val Asn Thr
    370                 375                 380

Arg Val Phe Ile Asn Ala Thr Ala Asn Gly Thr Asn Pro Lys Tyr Trp
385                 390                 395                 400

Glu Asn Pro Leu Thr Phe Leu Pro Glu Arg Phe Leu Asp Lys Glu Ile
                405                 410                 415

Asp Tyr Arg Gly Lys Asn Phe Glu Leu Leu Pro Phe Gly Ala Gly Arg
            420                 425                 430

Arg Gly Cys Pro Gly Ile Asn Phe Ser Ile Pro Leu Val Glu Leu Ala
        435                 440                 445

Leu Ala Asn Leu Leu Phe His Tyr Asn Trp Ser Leu Pro Glu Gly Met
    450                 455                 460

Leu Ala Lys Asp Val Asp Met Glu Glu Ala Leu Gly Ile Thr Met His
465                 470                 475                 480

Lys Lys Ser Pro Leu Cys Leu Val Ala Ser His Tyr Thr Cys
                485                 490

<210>  235
<211>  1570
<212>  DNA
<213>  Nicotiana tabacum

<400>  235
   caaagactaa aagatgtcgg tctttgcggt tatttcattc tttctacttc tgtttttctt      60
   tttcaaatca tatttgccct catcgaaaac aaagaaaaat tctccaccat ctccttcaaa     120
   gcttccgtta atcggtcact tccacaaact aggcttacaa cctcaccgtt ctctacaaaa     180
   actatcaaat gaacatggtc ccatgatgat gcttcaattc ggtagcgtac ctgtgcttat     240
   cgcttcatca gctgaagctg cttccgaaat catgaaaacc caagatttgt cttttgcaaa     300
   caaacccatt tcaaccattc ctagcaagct tttcttcggc ccaaaggacg ttgccttcac     360
   cccatatggg gattactgga ggaatgccag aagcatttgc atgcttcagc ttttgaacaa     420
   caaaagagtc cagtcttttc gaaagataag ggaagaagag acttctcttc ttctccagag     480
   gattagggaa tcgccaaatt cagaagtcga tttaacggag ctgttcgttt ccatgactaa     540
   cgacatagtt tgcagggtgg ccttaggaag gaagtattgt gatggggaag aagggaggaa     600
   attcaagtct ttgctgttag agtttgtgga attgttggga gtttttaaca ttggagatta     660
   catgccgtgg cttgcatgga tgaatcgttt caatggtttg aatgccaaag tggataaagt     720
   ggcgaaagag tttgatgcat ttttggagga tgtgattgag gaacacggag gaaataagaa     780
   atcagacact gaagctgaag ggcagacttc gtggatatat ttattgcagg ttcacaaaga     840
   aaacaaggct ggttttcaag tcgaaatgga tgcaatcaaa gctattatca tggatatgtt 900
   tgctgcggga acagatacaa cttccacgct tctagagtgg acaatgaacg agctcttaag 960
   aaatccaaaa acattgaata agttgagaga tgaggtgaga caagtgactc aagggaagac 1020
   agaggtaaca gaggatgact tagagaaaat gccgtattta agagcagcag ttaaggagag 1080
   ttccaggcta cactctccag tgccacttct acctcgagaa gcaattaagg atgcaaaggt 1140
   tttgggctac gatatagctg cagggactca agtcctcgtt tgtccatggg caatctcaag 1200
   agatccaaac ctttgggaaa atccagagga gtttcaacct gaaagattct tggatacttc 1260
   catagattac aaaggcttac atttcgagtt aattccattc ggtgcaggtc ggaggggttg 1320
   ccctggcatc acatttgcta agtttgtgaa tgagctagca ttggcaagat taatgttcca 1380
   ttttgatttc tcgctaccaa aaggagttaa gcatgaggat ttggacgtgg aggaagctgc 1440
   tggaattact gttagaagga agttccccct tttagccgtc gccactccat gctcgtgatt 1500
   tttattttag agctcattct atgccttaaa aactactact agataactgc gtagtaaata 1560
   atgcttggta 1570

<210> 236
<211> 494
<212> PRT
<213> Nicotiana tabacum

<400> 236

Met Ser Val Phe Ala Val Ile Ser Phe Phe Leu Leu Leu Phe Phe Leu
1               5                   10                  15

Phe Lys Ser Tyr Leu Pro Ser Ser Lys Thr Lys Lys Asn Ser Pro Pro
            20                  25                  30

Ser Pro Ser Lys Leu Pro Leu Ile Gly His Phe His Lys Leu Gly Leu
        35                  40                  45

Gln Pro His Arg Ser Leu Gln Lys Leu Ser Asn Glu His Gly Pro Met
    50                  55                  60

Met Met Leu Gln Phe Gly Ser Val Pro Val Leu Ile Ala Ser Ser Ala
65                  70                  75                  80

Glu Ala Ala Ser Glu Ile Met Lys Thr Gln Asp Leu Ser Phe Ala Asn
                85                  90                  95

Lys Pro Ile Ser Thr Ile Pro Ser Lys Leu Phe Phe Gly Pro Lys Asp
            100                 105                 110

Val Ala Phe Thr Pro Tyr Gly Asp Tyr Trp Arg Asn Ala Arg Ser Ile
        115                 120                 125

Cys Met Leu Gln Leu Leu Asn Asn Lys Arg Val Gln Ser Phe Arg Lys
    130                 135                 140

Ile Arg Glu Glu Glu Thr Ser Leu Leu Leu Gln Arg Ile Arg Glu Ser
145                 150                 155                 160

Pro Asn Ser Glu Val Asp Leu Thr Glu Leu Phe Val Ser Met Thr Asn
                165                 170                 175

Asp Ile Val Cys Arg Val Ala Leu Gly Arg Lys Tyr Cys Asp Gly Glu
            180                 185                 190

Glu Gly Arg Lys Phe Lys Ser Leu Leu Leu Glu Phe Val Glu Leu Leu
        195                 200                 205

Gly Val Phe Asn Ile Gly Asp Tyr Met Pro Trp Leu Ala Trp Met Asn
    210                 215                 220

Arg Phe Asn Gly Leu Asn Ala Lys Val Asp Lys Val Ala Lys Glu Phe
225                 230                 235                 240

Asp Ala Phe Leu Glu Asp Val Ile Glu Glu His Gly Gly Asn Lys Lys
                245                 250                 255

Ser Asp Thr Glu Ala Glu Gly Ala Asp Phe Val Asp Ile Leu Leu Gln
            260                 265                 270

Val His Lys Glu Asn Lys Ala Gly Phe Gln Val Glu Met Asp Ala Ile
        275                 280                 285

Lys Ala Ile Ile Met Asp Met Phe Ala Ala Gly Thr Asp Thr Thr Ser
    290                 295                 300

Thr Leu Leu Glu Trp Thr Met Asn Glu Leu Leu Arg Asn Pro Lys Thr
305                 310                 315                 320

Leu Asn Lys Leu Arg Asp Glu Val Arg Gln Val Thr Gln Gly Lys Thr
                325                 330                 335

Glu Val Thr Glu Asp Asp Leu Glu Lys Met Pro Tyr Leu Arg Ala Ala
            340                 345                 350

Val Lys Glu Ser Ser Arg Leu His Ser Pro Val Pro Leu Leu Pro Arg
        355                 360                 365

Glu Ala Ile Lys Asp Ala Lys Val Leu Gly Tyr Asp Ile Ala Ala Gly
    370                 375                 380

Thr Gln Val Leu Val Cys Pro Trp Ala Ile Ser Arg Asp Pro Asn Leu
385                 390                 395                 400

Trp Glu Asn Pro Glu Glu Phe Gln Pro Glu Arg Phe Leu Asp Thr Ser
                405                 410                 415

Ile Asp Tyr Lys Gly Leu His Phe Glu Leu Ile Pro Phe Gly Ala Gly
            420                 425                 430

Arg Arg Gly Cys Pro Gly Ile Thr Phe Ala Lys Phe Val Asn Glu Leu
        435                 440                 445

Ala Leu Ala Arg Leu Met Phe His Phe Asp Phe Ser Leu Pro Lys Gly
    450                 455                 460

Val Lys His Glu Asp Leu Asp Val Glu Glu Ala Ala Gly Ile Thr Val
465                 470                 475                 480

Arg Arg Lys Phe Pro Leu Leu Ala Val Ala Thr Pro Cys Ser
                485                 490

<210>  237
<211>  1585
<212>  DNA
<213>  Nicotiana tabacum

<400>  237
attatccatc acctaaaatg gagaattctt gggtttttct agccttggca gggctatctg      60
cattagcttt tctctgtaaa ataatcacct gtcgaagacc ggttaaccgg aaaataccac     120
caggtccaaa accatggccc atcattggca atttgaacct acttggtcct ataccacatc     180
aatcttttga cttgctttcc aaaaaatatg gagagttgat gctgctgaaa tttggctcca     240
ggccagttct tgttgcttca tctgctgaaa tggcaaaaca gtttttaaaa gtacatgatg     300
ctaatttcgc ctcccgtcct atgctagctg gtggaaagta tacaagctat aactattgtg     360
acatgacatg ggcaccctat ggtccctatt ggcgccaagc acgacgaatt taccttaacc     420
agatatttac tccgaaaagg ctagactcgt tcgagtacat tcgtgttgaa gaaaggcagg     480
ccttgatttc ccagctgaat tcccttgctg gaaagccatt ttttctcaaa gaccatttgt     540
cgcgatttag cctctgcagc atgacaagga tggttttgag caacaagtac tttggtgaat     600
caacagttag agtagaagat ttgcagtacc tggtagatca atggttctta cttaatggtg     660
ctttcaacat tggagattgg attccatggc tcagcttctt ggacctacaa ggctatgtga     720
aacaaatgaa ggctttgaaa agaacttttg ataagttcca caacattgtg ctagatgatc     780
gcagggctaa gaagaatgca gagaagaact ttgtcccaaa agacatggtt gatgtcttgt     840
tgaagatggc tgaagatcct aatctggaag tcaaactcac taatgactgt gtcaaagggt     900
taatgcagga tttactaact ggaggaacag atagcttaac agcagcagtg caatgggcat     960
ttcaagaact tcttagacgg ccaagggtta ttgagaaggc aaccgaagag cttgaccgga    1020
ttgtcgggaa agagagatgg gtagaagaga aagattgctc gcagctatct tacgttgaag    1080
caatcctcaa ggaaacacta aggttacatc ctctaggaac tatgctagca ccgcattgtg    1140
ctatagaaga ttgtaacgtg gctggttatg acatacagaa aggaacgacc gttctggtga    1200

atgtttggac cattggaagg gacccaaaat actgggatag agcacaagag tttctccccg 1260

agagattctt agagaacgac attgatatgg acggacataa ctttgctttc ttgccatttg 1320

gctcggggcg aaggaggtgc cctggctata gccttggact taaggttatc cgagtaacat 1380

tagccaacat gttgcatgga ttcaactgga aattacctga aggtatgaag ccagaagata 1440

taagtgtgga agaacattat gggctcacta cacatcctaa gtttcctgtt cctgtgatct 1500

tggaatctag actttcttca gatctctatt cccccatcac ttaatcctaa gtgcttccta 1560

ttatagcatc atatcaatat ccctc 1585

<210> 238
<211> 508
<212> PRT
<213> Nicotiana tabacum

<400> 238

Met Glu Asn Ser Trp Val Phe Leu Ala Leu Ala Gly Leu Ser Ala Leu
1               5                   10                  15

Ala Phe Leu Cys Lys Ile Ile Thr Cys Arg Arg Pro Val Asn Arg Lys
            20                  25                  30

Ile Pro Pro Gly Pro Lys Pro Trp Pro Ile Ile Gly Asn Leu Asn Leu
        35                  40                  45

Leu Gly Pro Ile Pro His Gln Ser Phe Asp Leu Leu Ser Lys Lys Tyr
    50                  55                  60

Gly Glu Leu Met Leu Leu Lys Phe Gly Ser Arg Pro Val Leu Val Ala
65                  70                  75                  80

Ser Ser Ala Glu Met Ala Lys Gln Phe Leu Lys Val His Asp Ala Asn
                85                  90                  95

Phe Ala Ser Arg Pro Met Leu Ala Gly Gly Lys Tyr Thr Ser Tyr Asn
            100                 105                 110

Tyr Cys Asp Met Thr Trp Ala Pro Tyr Gly Pro Tyr Trp Arg Gln Ala
        115                 120                 125

Arg Arg Ile Tyr Leu Asn Gln Ile Phe Thr Pro Lys Arg Leu Asp Ser
    130                 135                 140

Phe Glu Tyr Ile Arg Val Glu Glu Arg Gln Ala Leu Ile Ser Gln Leu
145                 150                 155                 160

Asn Ser Leu Ala Gly Lys Pro Phe Phe Leu Lys Asp His Leu Ser Arg
                165                 170                 175

Phe Ser Leu Cys Ser Met Thr Arg Met Val Leu Ser Asn Lys Tyr Phe
            180                 185                 190

Gly Glu Ser Thr Val Arg Val Glu Asp Leu Gln Tyr Leu Val Asp Gln
        195                 200                 205

Trp Phe Leu Leu Asn Gly Ala Phe Asn Ile Gly Asp Trp Ile Pro Trp
    210                 215                 220

Leu Ser Phe Leu Asp Leu Gln Gly Tyr Val Lys Gln Met Lys Ala Leu
225                 230                 235                 240

Lys Arg Thr Phe Asp Lys Phe His Asn Ile Val Leu Asp Asp Arg Arg
                245                 250                 255

Ala Lys Lys Asn Ala Glu Lys Asn Phe Val Pro Lys Asp Met Val Asp
            260                 265                 270

Val Leu Leu Lys Met Ala Glu Asp Pro Asn Leu Glu Val Lys Leu Thr
        275                 280                 285

Asn Asp Cys Val Lys Gly Leu Met Gln Asp Leu Leu Thr Gly Gly Thr
    290                 295                 300

Asp Ser Leu Thr Ala Ala Val Gln Trp Ala Phe Gln Glu Leu Leu Arg
305                 310                 315                 320

Arg Pro Arg Val Ile Glu Lys Ala Thr Glu Glu Leu Asp Arg Ile Val
                325                 330                 335

Gly Lys Glu Arg Trp Val Glu Glu Lys Asp Cys Ser Gln Leu Ser Tyr
            340                 345                 350

Val Glu Ala Ile Leu Lys Glu Thr Leu Arg Leu His Pro Leu Gly Thr
        355                 360                 365

Met Leu Ala Pro His Cys Ala Ile Glu Asp Cys Asn Val Ala Gly Tyr
    370                 375                 380

Asp Ile Gln Lys Gly Thr Thr Val Leu Val Asn Val Trp Thr Ile Gly
385                 390                 395                 400

Arg Asp Pro Lys Tyr Trp Asp Arg Ala Gln Glu Phe Leu Pro Glu Arg
                405                 410                 415

Phe Leu Glu Asn Asp Ile Asp Met Asp Gly His Asn Phe Ala Phe Leu
            420                 425                 430

Pro Phe Gly Ser Gly Arg Arg Arg Cys Pro Gly Tyr Ser Leu Gly Leu
        435                 440                 445

Lys Val Ile Arg Val Thr Leu Ala Asn Met Leu His Gly Phe Asn Trp
    450                 455                 460

Lys Leu Pro Glu Gly Met Lys Pro Glu Asp Ile Ser Val Glu Glu His
465                 470                 475                 480

Tyr Gly Leu Thr Thr His Pro Lys Phe Pro Val Pro Val Ile Leu Glu
                485                 490                 495

Ser Arg Leu Ser Ser Asp Leu Tyr Ser Pro Ile Thr
            500                 505

<210>  239
<211>  1591
<212>  DNA
<213>  Nicotiana tabacum

<400>  239
ctcattatcc atcacctaaa atggagaatt cttgggtttt tctagccttg gcagggctat      60

ctgcattagc ttttctctgt aaaataatca cctgtcgaag accggttaac cggaaaatac     120

caccaggtcc aaaaccatgg cccatcattg gcaatttgaa cctacttggt cctataccac     180

atcaatcttt tgacttgctt tccaaaaaat atggagagtt gatgctgctg aaatttggct     240

ccaggccagt tcttgttgct tcatctgctg aaatggcaaa acagttttta aaagtacatg     300

atgctaattt cgcctcccgt cctatgctag ctggtggaaa gtatacaagc tataactatt     360

gtgacatgac atgggcaccc tatggtccct attggcgcca agcacgacga cgaatttacc     420

ttaaccagat atttactccg aaaaggctag actcgttcga gtacattcgt gttgaagaaa     480

ggcaggcctt gatttcccag ctgaattccc ttgctggaaa gccatttttt ctcaaagacc     540

atttgtcgcg atttagcctc tgcagcatga caaggatggt tttgagcaac aagtattttg     600

gtgaatcaac agttagagta gaagatttgc agtacctggt agatcaatgg ttcttactta     660

atggtgcttt caacattgga gattggattc catggctcag cttcttggac ctacaaggct     720

atgtgaaaca aatgaaggct ttgaaaagaa cttttgataa gttccacaac attgtgctag     780

atgatcacag ggctaagaag aatgcagaga agaactttgt cccaaaagac atggttgatg     840

tcttgttgaa gatggctgaa gatcctaatc tggaagtcaa actcactaat gactgtgtca     900

aagggttaat gcaggattta ctaactggag gaacagatag cttaacagca gcagtgcaat     960

gggcatttca agaacttctt agacagccaa gggttattga gaaggcaacc gaagagcttg    1020

accggattgt cgggaaagag agatgggtag aagagaaaga ttgctcgcag ctatcttacg    1080

ttgaagcaat cctcaaggaa acactaaggt tacatcctct aggaactatg ctagcaccgc    1140

attgtgctat agaagattgt aacgtggctg gttatgacat acagaaagga acgaccgttc    1200

tggtgaatgt ttggaccatt ggaagggacc caaaatactg ggatagagca caagagtttc    1260

tccccgagag attcttagag aacgacattg atatggacgg acataacttt gctttcttgc    1320

catttggctc ggggcgaagg aggtgccctg gctatagcct tggacttaag gttatccgag    1380

taacattagc caacatgttg catggattca actggaaatt acctgaaggt atgaagccag    1440

aagatataag tgtggaagaa cattatgggc tcactacaca tcctaagttt cctgttcctg 1500
tgatcttgga atctagactt tcttcagatc tctattcccc catcacttaa tcctaagtgc 1560
ttcctattat agcatcatat caatatccct c 1591

<210> 240
<211> 509
<212> PRT
<213> Nicotiana tabacum

<400> 240

Met Glu Asn Ser Trp Val Phe Leu Ala Leu Ala Gly Leu Ser Ala Leu
1               5                   10                  15

Ala Phe Leu Cys Lys Ile Ile Thr Cys Arg Arg Pro Val Asn Arg Lys
            20                  25                  30

Ile Pro Pro Gly Pro Lys Pro Trp Pro Ile Ile Gly Asn Leu Asn Leu
        35                  40                  45

Leu Gly Pro Ile Pro His Gln Ser Phe Asp Leu Leu Ser Lys Lys Tyr
    50                  55                  60

Gly Glu Leu Met Leu Leu Lys Phe Gly Ser Arg Pro Val Leu Val Ala
65                  70                  75                  80

Ser Ser Ala Glu Met Ala Lys Gln Phe Leu Lys Val His Asp Ala Asn
                85                  90                  95

Phe Ala Ser Arg Pro Met Leu Ala Gly Gly Lys Tyr Thr Ser Tyr Asn
            100                 105                 110

Tyr Cys Asp Met Thr Trp Ala Pro Tyr Gly Pro Tyr Trp Arg Gln Ala
        115                 120                 125

Arg Arg Arg Ile Tyr Leu Asn Gln Ile Phe Thr Pro Lys Arg Leu Asp
    130                 135                 140

Ser Phe Glu Tyr Ile Arg Val Glu Glu Arg Gln Ala Leu Ile Ser Gln
145                 150                 155                 160

Leu Asn Ser Leu Ala Gly Lys Pro Phe Phe Leu Lys Asp His Leu Ser
                165                 170                 175

Arg Phe Ser Leu Cys Ser Met Thr Arg Met Val Leu Ser Asn Lys Tyr
            180                 185                 190

Phe Gly Glu Ser Thr Val Arg Val Glu Asp Leu Gln Tyr Leu Val Asp
        195                 200                 205

Gln Trp Phe Leu Leu Asn Gly Ala Phe Asn Ile Gly Asp Trp Ile Pro
    210                 215                 220

Trp Leu Ser Phe Leu Asp Leu Gln Gly Tyr Val Lys Gln Met Lys Ala
225                 230                 235                 240

Leu Lys Arg Thr Phe Asp Lys Phe His Asn Ile Val Leu Asp Asp His
                245                 250                 255

Arg Ala Lys Lys Asn Ala Glu Lys Asn Phe Val Pro Lys Asp Met Val
            260                 265                 270

Asp Val Leu Leu Lys Met Ala Glu Asp Pro Asn Leu Glu Val Lys Leu
        275                 280                 285

Thr Asn Asp Cys Val Lys Gly Leu Met Gln Asp Leu Leu Thr Gly Gly
    290                 295                 300

Thr Asp Ser Leu Thr Ala Ala Val Gln Trp Ala Phe Gln Glu Leu Leu
305                 310                 315                 320

Arg Gln Pro Arg Val Ile Glu Lys Ala Thr Glu Glu Leu Asp Arg Ile
                325                 330                 335

Val Gly Lys Glu Arg Trp Val Glu Glu Lys Asp Cys Ser Gln Leu Ser
            340                 345                 350

Tyr Val Glu Ala Ile Leu Lys Glu Thr Leu Arg Leu His Pro Leu Gly
        355                 360                 365

Thr Met Leu Ala Pro His Cys Ala Ile Glu Asp Cys Asn Val Ala Gly
    370                 375                 380

Tyr Asp Ile Gln Lys Gly Thr Thr Val Leu Val Asn Val Trp Thr Ile
385                 390                 395                 400

Gly Arg Asp Pro Lys Tyr Trp Asp Arg Ala Gln Glu Phe Leu Pro Glu
                405                 410                 415

Arg Phe Leu Glu Asn Asp Ile Asp Met Asp Gly His Asn Phe Ala Phe
            420                 425                 430

Leu Pro Phe Gly Ser Gly Arg Arg Arg Cys Pro Gly Tyr Ser Leu Gly
        435                 440                 445

Leu Lys Val Ile Arg Val Thr Leu Ala Asn Met Leu His Gly Phe Asn
    450                 455                 460

Trp Lys Leu Pro Glu Gly Met Lys Pro Glu Asp Ile Ser Val Glu Glu
465                 470                 475                 480

His Tyr Gly Leu Thr Thr His Pro Lys Phe Pro Val Pro Val Ile Leu
                485                 490                 495

Glu Ser Arg Leu Ser Ser Asp Leu Tyr Ser Pro Ile Thr
            500                 505

<210>  241
<211>  1615
<212>  DNA
<213>  Nicotiana tabacum

<400>  241
tgataatgct ctttctactc tttgtagccc ttcctttcat tcttattttt cttcttccta      60
aattcaaaaa tggtggaaat aacagattgc caccaggtcc tataggttta ccattcattg     120
gaaatttgca tcaatacgat agtataactc ctcatatcta tttttggaaa ctttcaaaaa     180
aatatggcaa aatcttctca ttaaaacttg cttctactaa tgtggtagta gtttcttcag     240
caaaattagc aaaagaagta ttgaaaaaac aagatttaat attttgtagt agaccatcta     300
ttcttggcca acaaaaactg tcttattatg gtcgtgatat tgcttttaat gattattgga     360
gagaaatgag aaaaatttgt gttcttcatc tttttagttt aaaaaaagtt caattattta     420
gtccaattcg tgaagatgaa gtttttagaa tgattaagaa aatatcaaaa caagcttcta     480
cttcacaaat tattaatttg agtaatttaa tgatttcatt aacaagtaca attatttgta     540
gagttgcttt tggtgttagg attgaagaag aagcacatgc aaggaagaga tttgattttc     600
ttttggccga ggcacaagaa atgatggcta gtttctttgt atctgatttt tttccctttt     660
taagttggat tgataaatta agtggattga catatagact tgagaggaat ttcaaggatt     720
tggataattt ttatgaagaa ctcattgagc aacatcaaaa tcctaataag ccaaaatata     780
tggaaggaga tattgttgat cttttgctac aattgaagaa agagaaatta acaccacttg     840
atctcactat ggaagatata aaaggaattc tcatgaatgt gttagttgca ggatcagaca     900
ctagtgcagc tgctactgtt tgggcaatga cagccttgat aaagaatcct aaagccatgg     960
aaaaagttca attagaaatc agaaaatcag ttgggaagaa aggcattgta aatgaagaag    1020
atgtccaaaa catcccttat tttaaagcag tgataaagga aatatttaga ttgtatccac    1080
cagctccact tttagttcca agagaatcaa tggaaaaaac catattagaa ggttatgaaa    1140
ttcggccaag aaccatagtt catgttaacg cttgggctat agcaagggat cctgaaatat    1200
gggaaaatcc agatgaattt atacctgaga gatttttgaa tagcagtatc gattacaagg    1260
gtcaagattt tgagttactt ccatttggtg caggcagaag aggttgccca ggtattgcac    1320
ttggggttgc atccatggaa cttgctttgt caaatcttct ttatgcattt gattgggagt    1380
tgccttatgg agtaaaaaaa gaagacatcg acacaaacgt taggcctgga attgccatgc    1440
acaagaaaaa cgaactttgc cttgtcccaa aaaattattt ataaattata ttgggacgtg    1500
gatctcatgc tagttctgtg cggtcagcta agcttattat ttttggctca aattatgtat    1560
acataattag tacatgttta aaatgtataa atatagtaga accattctca tggtt         1615

<210>  242

<211>  492
<212>  PRT
<213>  Nicotiana tabacum

<400>  242

Met Leu Phe Leu Leu Phe Val Ala Leu Pro Phe Ile Leu Ile Phe Leu
1               5                   10                  15

Leu Pro Lys Phe Lys Asn Gly Gly Asn Asn Arg Leu Pro Pro Gly Pro
            20                  25                  30

Ile Gly Leu Pro Phe Ile Gly Asn Leu His Gln Tyr Asp Ser Ile Thr
        35                  40                  45

Pro His Ile Tyr Phe Trp Lys Leu Ser Lys Lys Tyr Gly Lys Ile Phe
    50                  55                  60

Ser Leu Lys Leu Ala Ser Thr Asn Val Val Val Val Ser Ser Ala Lys
65                  70                  75                  80

Leu Ala Lys Glu Val Leu Lys Lys Gln Asp Leu Ile Phe Cys Ser Arg
                85                  90                  95

Pro Ser Ile Leu Gly Gln Gln Lys Leu Ser Tyr Tyr Gly Arg Asp Ile
            100                 105                 110

Ala Phe Asn Asp Tyr Trp Arg Glu Met Arg Lys Ile Cys Val Leu His
        115                 120                 125

Leu Phe Ser Leu Lys Lys Val Gln Leu Phe Ser Pro Ile Arg Glu Asp
    130                 135                 140

Glu Val Phe Arg Met Ile Lys Lys Ile Ser Lys Gln Ala Ser Thr Ser
145                 150                 155                 160

Gln Ile Ile Asn Leu Ser Asn Leu Met Ile Ser Leu Thr Ser Thr Ile
                165                 170                 175

Ile Cys Arg Val Ala Phe Gly Val Arg Ile Glu Glu Glu Ala His Ala
            180                 185                 190

Arg Lys Arg Phe Asp Phe Leu Leu Ala Glu Ala Gln Glu Met Met Ala
        195                 200                 205

Ser Phe Phe Val Ser Asp Phe Phe Pro Phe Leu Ser Trp Ile Asp Lys
    210                 215                 220

Leu Ser Gly Leu Thr Tyr Arg Leu Glu Arg Asn Phe Lys Asp Leu Asp
225                 230                 235                 240

Asn Phe Tyr Glu Glu Leu Ile Glu Gln His Gln Asn Pro Asn Lys Pro
                245                 250                 255

Lys Tyr Met Glu Gly Asp Ile Val Asp Leu Leu Leu Gln Leu Lys Lys
            260                 265                 270

Glu Lys Leu Thr Pro Leu Asp Leu Thr Met Glu Asp Ile Lys Gly Ile
        275                 280                 285

Leu Met Asn Val Leu Val Ala Gly Ser Asp Thr Ser Ala Ala Ala Thr
    290                 295                 300

Val Trp Ala Met Thr Ala Leu Ile Lys Asn Pro Lys Ala Met Glu Lys
305                 310                 315                 320

Val Gln Leu Glu Ile Arg Lys Ser Val Gly Lys Lys Gly Ile Val Asn
                325                 330                 335

Glu Glu Asp Val Gln Asn Ile Pro Tyr Phe Lys Ala Val Ile Lys Glu
            340                 345                 350

Ile Phe Arg Leu Tyr Pro Pro Ala Pro Leu Leu Val Pro Arg Glu Ser
        355                 360                 365

Met Glu Lys Thr Ile Leu Glu Gly Tyr Glu Ile Arg Pro Arg Thr Ile
    370                 375                 380

Val His Val Asn Ala Trp Ala Ile Ala Arg Asp Pro Glu Ile Trp Glu
385                 390                 395                 400

Asn Pro Asp Glu Phe Ile Pro Glu Arg Phe Leu Asn Ser Ser Ile Asp
                405                 410                 415

Tyr Lys Gly Gln Asp Phe Glu Leu Leu Pro Phe Gly Ala Gly Arg Arg
            420                 425                 430

Gly Cys Pro Gly Ile Ala Leu Gly Val Ala Ser Met Glu Leu Ala Leu
        435                 440                 445

Ser Asn Leu Leu Tyr Ala Phe Asp Trp Glu Leu Pro Tyr Gly Val Lys
    450                 455                 460

Lys Glu Asp Ile Asp Thr Asn Val Arg Pro Gly Ile Ala Met His Lys
465                 470                 475                 480

Lys Asn Glu Leu Cys Leu Val Pro Lys Asn Tyr Leu
                485                 490

<210>  243
<211>  1624
<212>  DNA
<213>  Nicotiana tabacum

<400>  243

tgataatgct ctttctactc tttgtagccc ttcctttcat tcttattttt cttcttccta 60
aattcaaaaa tggtggaaat aacagattgc caccaggtcc tataggttta ccattcattg 120
gaaatttgca tcaatatgat agtataactc ctcatatcta tttttggaaa ctttccaaaa 180
aatatggcaa aatcttctca ttaaaacttg cttctactaa tgtggtagta gtttcttcag 240
caaaattagc aaaagaagta ttgaaaaaac aagatttaat attttgtagt agaccatcta 300
ttcttggcca acaaaaactg tcttattatg gtcgtgatat tgcttttgca ccttataatg 360
attattggag agaaatgaga aaaatttgtg ttcttcatct tttagtttta aaaaaagttc 420
aattatttag tccaattcgt gaagatgaag tttttagaat gattaagaaa atatcaaaac 480
aagcttctac ttcacaaatt attaatttga gtaatttaat gatttcatta acaagtacaa 540
ttatttgtag agttgctttt ggtgttaggt ttgaagaaga agcacatgca aggaagagat 600
ttgattttct tttggccgag gcacaagaaa tgatggctag tttctttgta tctgattttt 660
ttcccttttt aagttggatt gataaattaa gtggattgac atatagactt gagaggaatt 720
tcaaggattt ggataatttt tatgaagaac tcattgagca acatcaaaat cctaataagc 780
caaaatatat ggaaggagat attgttgatc ttttgctaca attgaagaaa gagaaattaa 840
caccacttga tctcactatg gaagatataa aaggaattct catgaatgtg ttagttgcag 900
gatcagacac tagtgcagct gctactgttt gggcaatgac agccttgata aagaatccta 960
aagccatgga aaaagttcaa ttagaaatca gaaaatcagt tgggaagaaa ggcattgtaa 1020
atgaagaaga tgtccaaaac atcccttatt ttaaagcagt gataaaggaa atatttagat 1080
tgtatccacc agctccactt ttagttccaa gagaatcaat ggaaaaaacc atattagaag 1140
gttatgaaat tcggccaaga accatagttc atgttaacgc ttgggctata gcaagggatc 1200
ctgaaatatg ggaaaatcca gatgaattta tacctgagag atttttgaat agcagtatcg 1260
attacaaggg tcaagatttt gagttacttc catttggtgc aggcagaaga ggttgcccag 1320
gtattgcact tggggttgca tccatggaac ttgctttgtc aaatcttctt tatgcatttg 1380
attgggagtt gccttatgga gtgaaaaaag aagacatcga cacaaacgtt aggcctggaa 1440
ttgccatgca caagaaaaac gaactttgcc ttgtcccaaa aaattattta taaattatat 1500
tgggacgtgg atctcatgct agttctgtgc ggtcagctaa gcttattatt tttggctcaa 1560
attatgtata cataattagt acatgtttaa aatgtataaa tatagtagaa ccattctcat 1620
ggtt 1624

<210> 244
<211> 495
<212> PRT
<213> Nicotiana tabacum

<400> 244

Met Leu Phe Leu Leu Phe Val Ala Leu Pro Phe Ile Leu Ile Phe Leu
1               5                   10                  15

Leu Pro Lys Phe Lys Asn Gly Gly Asn Asn Arg Leu Pro Pro Gly Pro
            20                  25                  30

Ile Gly Leu Pro Phe Ile Gly Asn Leu His Gln Tyr Asp Ser Ile Thr
        35                  40                  45

Pro His Ile Tyr Phe Trp Lys Leu Ser Lys Lys Tyr Gly Lys Ile Phe
    50                  55                  60

Ser Leu Lys Leu Ala Ser Thr Asn Val Val Val Val Ser Ser Ala Lys
65                  70                  75                  80

Leu Ala Lys Glu Val Leu Lys Lys Gln Asp Leu Ile Phe Cys Ser Arg
                85                  90                  95

Pro Ser Ile Leu Gly Gln Gln Lys Leu Ser Tyr Tyr Gly Arg Asp Ile
            100                 105                 110

Ala Phe Ala Pro Tyr Asn Asp Tyr Trp Arg Glu Met Arg Lys Ile Cys
        115                 120                 125

Val Leu His Leu Phe Ser Leu Lys Lys Val Gln Leu Phe Ser Pro Ile
    130                 135                 140

Arg Glu Asp Glu Val Phe Arg Met Ile Lys Lys Ile Ser Lys Gln Ala
145                 150                 155                 160

Ser Thr Ser Gln Ile Ile Asn Leu Ser Asn Leu Met Ile Ser Leu Thr
                165                 170                 175

Ser Thr Ile Ile Cys Arg Val Ala Phe Gly Val Arg Phe Glu Glu Glu
            180                 185                 190

Ala His Ala Arg Lys Arg Phe Asp Phe Leu Leu Ala Glu Ala Gln Glu
        195                 200                 205

Met Met Ala Ser Phe Phe Val Ser Asp Phe Phe Pro Phe Leu Ser Trp
    210                 215                 220

Ile Asp Lys Leu Ser Gly Leu Thr Tyr Arg Leu Glu Arg Asn Phe Lys
225                 230                 235                 240

Asp Leu Asp Asn Phe Tyr Glu Glu Leu Ile Glu Gln His Gln Asn Pro
                245                 250                 255

Asn Lys Pro Lys Tyr Met Glu Gly Asp Ile Val Asp Leu Leu Leu Gln
            260                 265                 270

Leu Lys Lys Glu Lys Leu Thr Pro Leu Asp Leu Thr Met Glu Asp Ile
        275                 280                 285

Lys Gly Ile Leu Met Asn Val Leu Val Ala Gly Ser Asp Thr Ser Ala
    290                 295                 300

Ala Ala Thr Val Trp Ala Met Thr Ala Leu Ile Lys Asn Pro Lys Ala
305                 310                 315                 320

Met Glu Lys Val Gln Leu Glu Ile Arg Lys Ser Val Gly Lys Lys Gly
                325                 330                 335

Ile Val Asn Glu Glu Asp Val Gln Asn Ile Pro Tyr Phe Lys Ala Val
            340                 345                 350

Ile Lys Glu Ile Phe Arg Leu Tyr Pro Pro Ala Pro Leu Leu Val Pro
        355                 360                 365

Arg Glu Ser Met Glu Lys Thr Ile Leu Glu Gly Tyr Glu Ile Arg Pro
    370                 375                 380

Arg Thr Ile Val His Val Asn Ala Trp Ala Ile Ala Arg Asp Pro Glu
385                 390                 395                 400

Ile Trp Glu Asn Pro Asp Glu Phe Ile Pro Glu Arg Phe Leu Asn Ser
                405                 410                 415

Ser Ile Asp Tyr Lys Gly Gln Asp Phe Glu Leu Leu Pro Phe Gly Ala
            420                 425                 430

Gly Arg Arg Gly Cys Pro Gly Ile Ala Leu Gly Val Ala Ser Met Glu
        435                 440                 445

Leu Ala Leu Ser Asn Leu Leu Tyr Ala Phe Asp Trp Glu Leu Pro Tyr
    450                 455                 460

Gly Val Lys Lys Glu Asp Ile Asp Thr Asn Val Arg Pro Gly Ile Ala
465                 470                 475                 480

Met His Lys Lys Asn Glu Leu Cys Leu Val Pro Lys Asn Tyr Leu
                485                 490                 495

<210>  245
<211>  1627
<212>  DNA
<213>  Nicotiana tabacum

<400>  245
aaaattcata atggtttttc ccatagaagc ctttgtagga ctagtaacct tcacatttct      60

cttatacttc ctatggacaa aaaaatctca aaaacttcca aaacccttac taccgaaaat     120

ccccggagga tggccggtaa tcggccatct ttttcacttc aataacgacg gcgacgaccg     180

tccattagct cgaaaactcg gagacttagc tgataaatac ggccccgttt tcacttttcg     240

gctaggtctt ccccttgtgc tagttgtaag cagttacgaa gctataaaag attgcttctc     300

tacaaatgac gccattttct ccaatcgtcc agcttttctt tacggcgaat accttggcta 360
caataataca atgctttttc tagcaaatta cggaccttac tggcgaaaaa atcgtaaatt 420
agtcattcag gaagttctct ctgctagtcg tctcgaaaaa ttcaaacaag tgagattcac 480
cagaattcaa acgagcatta agaatttata cactcgaatt aatggaaatt cgagtacgat 540
aaatctaact gattggttag aagaattgga ttttggtctg atcgtgaaaa tgatcgctgg 600
gaaaaattat gaatccggta aaggagatga acaagtggaa agatttaaga atgcgtttaa 660
ggattttatg gttttatcaa tggaatttgt attatgggat gcatttccaa ttccattatt 720
taaatgggtg gattttcaag gtcatattaa ggcaatgaaa aggacattta aggatataga 780
ttctgttttt cagaactggt tagaggaaca tattaataaa agagaaaaaa tggaggttgg 840
tgcagaaggg aatgaacaag atttcattga tgtggtgctt tcaaaattga gtaaagaata 900
tcttgatgaa ggttactctc gtgatactgt cattaaagca acagttttta gtttggtctt 960
ggatgcagca gacacagttg ctcttcacat aaattgggga atgacattat tgataaacaa 1020
tcaaaatgcc ttgatgaaag cacaagaaga gatagacaca aaagttggta agtatagatg 1080
ggtagaagag agtgatatta aggatttagt atacctccaa gctattgtta aaaaggtgtt 1140
acgattatat ccaccaggac ctttgttagt accacatgaa tatgtaaagg attgtgttgt 1200
tagtggatat cacattccta aagggactag attattcgca aacgtcatga aactgcagcg 1260
cgatcctaaa ctcttgtcaa atcctgataa gttcgatcca gagagattca tcgctggtga 1320
tatcgacttc cgtggtcacc actatgagtt tatcccattt ggttctggaa gacgatcttg 1380
tccggggatg acttatgcat tgcaagtgga acacctaaca atggcacatt taatccaggg 1440
tttcaattac aaaactccaa atgacgaggc cttggatatg aaggaaggtg caggcataac 1500
aatacgtaag gtaaatccgg tggaattgat aataacgcct cgcttggcac ctgagcttta 1560
ctaaaaccta agatctttca tcttggttga tcattgttta atactcctag atagatgggt 1620
attcatc 1627

<210> 246
<211> 517
<212> PRT
<213> Nicotiana tabacum

<400> 246

Met Val Phe Pro Ile Glu Ala Phe Val Gly Leu Val Thr Phe Thr Phe
1               5                   10                  15

Leu Leu Tyr Phe Leu Trp Thr Lys Lys Ser Gln Lys Leu Pro Lys Pro
            20                  25                  30

Leu Leu Pro Lys Ile Pro Gly Gly Trp Pro Val Ile Gly His Leu Phe
        35                  40                  45

His Phe Asn Asn Asp Gly Asp Asp Arg Pro Leu Ala Arg Lys Leu Gly
    50                  55                  60

Asp Leu Ala Asp Lys Tyr Gly Pro Val Phe Thr Phe Arg Leu Gly Leu
65                  70                  75                  80

Pro Leu Val Leu Val Val Ser Ser Tyr Glu Ala Ile Lys Asp Cys Phe
                85                  90                  95

Ser Thr Asn Asp Ala Ile Phe Ser Asn Arg Pro Ala Phe Leu Tyr Gly
            100                 105                 110

Glu Tyr Leu Gly Tyr Asn Asn Thr Met Leu Phe Leu Ala Asn Tyr Gly
        115                 120                 125

Pro Tyr Trp Arg Lys Asn Arg Lys Leu Val Ile Gln Glu Val Leu Ser
    130                 135                 140

Ala Ser Arg Leu Glu Lys Phe Lys Gln Val Arg Phe Thr Arg Ile Gln
145                 150                 155                 160

Thr Ser Ile Lys Asn Leu Tyr Thr Arg Ile Asn Gly Asn Ser Ser Thr
                165                 170                 175

Ile Asn Leu Thr Asp Trp Leu Glu Glu Leu Asp Phe Gly Leu Ile Val
            180                 185                 190

Lys Met Ile Ala Gly Lys Asn Tyr Glu Ser Gly Lys Gly Asp Glu Gln
        195                 200                 205

Val Glu Arg Phe Lys Asn Ala Phe Lys Asp Phe Met Val Leu Ser Met
    210                 215                 220

Glu Phe Val Leu Trp Asp Ala Phe Pro Ile Pro Leu Phe Lys Trp Val
225                 230                 235                 240

Asp Phe Gln Gly His Ile Lys Ala Met Lys Arg Thr Phe Lys Asp Ile
                245                 250                 255

Asp Ser Val Phe Gln Asn Trp Leu Glu Glu His Ile Asn Lys Arg Glu
            260                 265                 270

Lys Met Glu Val Gly Ala Glu Gly Asn Glu Gln Asp Phe Ile Asp Val
        275                 280                 285

Val Leu Ser Lys Leu Ser Lys Glu Tyr Leu Asp Glu Gly Tyr Ser Arg
    290                 295                 300

Asp Thr Val Ile Lys Ala Thr Val Phe Ser Leu Val Leu Asp Ala Ala
305                 310                 315                 320

Asp Thr Val Ala Leu His Ile Asn Trp Gly Met Thr Leu Leu Ile Asn
                325                 330                 335

Asn Gln Asn Ala Leu Met Lys Ala Gln Glu Glu Ile Asp Thr Lys Val
            340                 345                 350

Gly Lys Tyr Arg Trp Val Glu Glu Ser Asp Ile Lys Asp Leu Val Tyr
        355                 360                 365

Leu Gln Ala Ile Val Lys Lys Val Leu Arg Leu Tyr Pro Pro Gly Pro
    370                 375                 380

Leu Leu Val Pro His Glu Tyr Val Lys Asp Cys Val Val Ser Gly Tyr
385                 390                 395                 400

His Ile Pro Lys Gly Thr Arg Leu Phe Ala Asn Val Met Lys Leu Gln
                405                 410                 415

Arg Asp Pro Lys Leu Leu Ser Asn Pro Asp Lys Phe Asp Pro Glu Arg
            420                 425                 430

Phe Ile Ala Gly Asp Ile Asp Phe Arg Gly His His Tyr Glu Phe Ile
        435                 440                 445

Pro Phe Gly Ser Gly Arg Arg Ser Cys Pro Gly Met Thr Tyr Ala Leu
    450                 455                 460

Gln Val Glu His Leu Thr Met Ala His Leu Ile Gln Gly Phe Asn Tyr
465                 470                 475                 480

Lys Thr Pro Asn Asp Glu Ala Leu Asp Met Lys Glu Gly Ala Gly Ile
                485                 490                 495

Thr Ile Arg Lys Val Asn Pro Val Glu Leu Ile Ile Thr Pro Arg Leu
            500                 505                 510

Ala Pro Glu Leu Tyr
        515

<210>  247
<211>  1594
<212>  DNA
<213>  Nicotiana tabacum

<400>  247
caaaaaatca tttctctcgt ctaaaatgga tcttctctta ctagagaaga ccttaattgg      60

tcttttcttt gccattttaa tcgctttaat tgtctctaaa cttcgttcaa agcgttttaa     120

gcttcctcca ggaccaattc cagtaccagt ttttggtaat tggcttcaag ttggtgatga     180

tttaaaccac agaaatctta ctgattatgc caaaaaattt ggcgatcttt tcttgttaag     240

aatgggtcaa cgtaacttag ttgttgtgtc atctcctgaa ttagctaaag aagttttaca      300

cacacaaggt gttgaatttg gttcaagaac aagaaatgtt gtgtttgata tttttactgg     360

aaaaggtcaa gatatggttt ttactgtata tggtgaacat tggagaaaaa tgaggagaat 420
tatgactgta ccatttttta ctaataaagt tgtgcaacag tatagagggg ggtgggagtt 480
tgaggtggca agtgtaattg aggatgtgaa aaaaaatcct gaatctgcta ctaatgggat 540
cgtattaagg aggagattac aattaatgat gtataataat atgtttagga ttatgtttga 600
taggagattt gagagtgaag atgatccttt gtttgttaag cttaaggctt tgaatggtga 660
aaggagtaga ttggctcaaa gttttgagta taattatggt gattttattc caattttgag 720
gcctcttttg agaggttatt tgaagatctg taaagaagtt aaggagaaga ggctgcagct 780
tttcaaagat tactttgttg atgaaagaaa gaagctttca aataccaaga gctcggacag 840
caatgcccta aaatgtgcga ttgatcacat tcttgaggct caacagaagg gagagatcaa 900
tgaggacaac gttctttaca ttgttgaaaa catcaatgtt gctgcaattg aaacaacatt 960
atggtcaatt gagtggggta tcgccgagct agtcaaccac cctcacatcc aaaagaaact 1020
gcgcgacgag attgacacag ttcttggacc aggagtgcaa gtgactgaac cagacaccca 1080
caagcttcca taccttcagg ctgtgatcaa ggaggcactt cgtctccgta tggcaattcc 1140
tctattagtc ccacacatga accttcacga cgcaaagctt ggcgggcttg atattccagc 1200
agagagcaaa atcttggtta acgcttggtg gttagctaac aacccggctc attggaagaa 1260
acccgaagag ttcagacccg agaggttctt tgaagaggag aagcatgttg aggccaatgg 1320
caatgacttc agatatcttc cgtttggcgt tggtaggagg agctgccctg gaattatact 1380
tgcattgcca attcttggca tcactttggg acgtttggtt cagaactttg agctgttgcc 1440
tcctccaggc cagtcgaagc tcgacaccac agagaaaggt ggacagttca gtctccacat 1500
tttgaagcat tccaccattg tgttgaaacc aaggtctttc tgaactttgt gatcttatta 1560
attaaggggt tctgaagaaa tttgatagtg ttgg 1594

<210> 248
<211> 505
<212> PRT
<213> Nicotiana tabacum

<400> 248

Met Asp Leu Leu Leu Leu Glu Lys Thr Leu Ile Gly Leu Phe Phe Ala
1               5                   10                  15

Ile Leu Ile Ala Leu Ile Val Ser Lys Leu Arg Ser Lys Arg Phe Lys
            20                  25                  30

Leu Pro Pro Gly Pro Ile Pro Val Pro Val Phe Gly Asn Trp Leu Gln
        35                  40                  45

Val Gly Asp Asp Leu Asn His Arg Asn Leu Thr Asp Tyr Ala Lys Lys
    50                  55                  60

Phe Gly Asp Leu Phe Leu Leu Arg Met Gly Gln Arg Asn Leu Val Val
65                  70                  75                  80

Val Ser Ser Pro Glu Leu Ala Lys Glu Val Leu His Thr Gln Gly Val
                85                  90                  95

Glu Phe Gly Ser Arg Thr Arg Asn Val Val Phe Asp Ile Phe Thr Gly
            100                 105                 110

Lys Gly Gln Asp Met Val Phe Thr Val Tyr Gly Glu His Trp Arg Lys
        115                 120                 125

Met Arg Arg Ile Met Thr Val Pro Phe Phe Thr Asn Lys Val Val Gln
    130                 135                 140

Gln Tyr Arg Gly Gly Trp Glu Phe Glu Val Ala Ser Val Ile Glu Asp
145                 150                 155                 160

Val Lys Lys Asn Pro Glu Ser Ala Thr Asn Gly Ile Val Leu Arg Arg
                165                 170                 175

Arg Leu Gln Leu Met Met Tyr Asn Asn Met Phe Arg Ile Met Phe Asp
            180                 185                 190

Arg Arg Phe Glu Ser Glu Asp Asp Pro Leu Phe Val Lys Leu Lys Ala
        195                 200                 205

Leu Asn Gly Glu Arg Ser Arg Leu Ala Gln Ser Phe Glu Tyr Asn Tyr
    210                 215                 220

Gly Asp Phe Ile Pro Ile Leu Arg Pro Leu Leu Arg Gly Tyr Leu Lys
225                 230                 235                 240

Ile Cys Lys Glu Val Lys Glu Lys Arg Leu Gln Leu Phe Lys Asp Tyr
                245                 250                 255

Phe Val Asp Glu Arg Lys Lys Leu Ser Asn Thr Lys Ser Ser Asp Ser
            260                 265                 270

Asn Ala Leu Lys Cys Ala Ile Asp His Ile Leu Glu Ala Gln Gln Lys
        275                 280                 285

Gly Glu Ile Asn Glu Asp Asn Val Leu Tyr Ile Val Glu Asn Ile Asn
    290                 295                 300

Val Ala Ala Ile Glu Thr Thr Leu Trp Ser Ile Glu Trp Gly Ile Ala
305                 310                 315                 320

Glu Leu Val Asn His Pro His Ile Gln Lys Lys Leu Arg Asp Glu Ile
                325                 330                 335

Asp Thr Val Leu Gly Pro Gly Val Gln Val Thr Glu Pro Asp Thr His
            340                 345                 350

Lys Leu Pro Tyr Leu Gln Ala Val Ile Lys Glu Ala Leu Arg Leu Arg
        355                 360                 365

Met Ala Ile Pro Leu Leu Val Pro His Met Asn Leu His Asp Ala Lys
    370                 375                 380

Leu Gly Gly Leu Asp Ile Pro Ala Glu Ser Lys Ile Leu Val Asn Ala
385                 390                 395                 400

Trp Trp Leu Ala Asn Asn Pro Ala His Trp Lys Lys Pro Glu Glu Phe
                405                 410                 415

Arg Pro Glu Arg Phe Phe Glu Glu Glu Lys His Val Glu Ala Asn Gly
            420                 425                 430

Asn Asp Phe Arg Tyr Leu Pro Phe Gly Val Gly Arg Arg Ser Cys Pro
        435                 440                 445

Gly Ile Ile Leu Ala Leu Pro Ile Leu Gly Ile Thr Leu Gly Arg Leu
    450                 455                 460

Val Gln Asn Phe Glu Leu Leu Pro Pro Pro Gly Gln Ser Lys Leu Asp
465                 470                 475                 480

Thr Thr Glu Lys Gly Gly Gln Phe Ser Leu His Ile Leu Lys His Ser
                485                 490                 495

Thr Ile Val Leu Lys Pro Arg Ser Phe
            500                 505

<210>  249
<211>  1678
<212>  DNA
<213>  Nicotiana tabacum

<400>  249
aacattttgc aatatagttt tcctagtcag ttctagcctc cttttcctta gaaataatgg      60

attatcatat ttctttccat tttcaagctc ttttagggct tttagccttt gtgttcttgt     120

ctattatctt atggagaaga acactcactt caagaaaatt agcccctgaa atcccagggg     180

catggcctat tataggccat cttcgtcagc tgagtggtac tgataagaat atcccatttc     240

cccgaatatt gggcgctttg gcagataaat atggacctgt cttcacactg agaataggga     300

tgtaccccta tttgattgtc aacaattggg aagcagctaa ggattgtctc acaacgcatg     360

ataaggactt cgctgcccga ccaacttcta tggctggtga aagcatcggg tacaagtatg     420

cgaggtttac ttatgctaat tttggtcctt attataacca agtgcgcaaa ctagccctac     480

aacatgtacc ctcgagtact aaactcgaga aaatgaaaca catacgtgtt tctgaattgg     540

aaactagcat caaagaatta tattctttga cgctgggcaa aaacaacatg caaaaagtga     600

atataagtaa atggtttgaa caattgactt taaacataat cgtgaagaca atttgtggca 660
agagatatag caacatagag gaggatgaag aggcacaacg tttcagaaag gcatttaagg 720
gcatcatgtt tgttgtaggg caaattgttt tatatgacgc aattccattc ccattgttca 780
aatactttga tttccaaggt catatacaat tgatgaacaa aatttataaa gacttagatt 840
ctattcttca aggatggttg gatgatcata tgatgaacaa ggatgtaaac aataaggatc 900
aagatgccat agatgccatg cttaaggtaa cacaacttaa tgaattcaaa gcctatggtt 960
tttctcaggc cactgtgatc aagtcgacag tcttgagttt gatcttagat ggaaatgaca 1020
caaccgctgt tcatttgata tgggtaatgt ccttattact gaacaatcca catgttatga 1080
aacaaggcca agaagagata gacatgaaag tgggtaaaga gaggtggatt gaagatactg 1140
acataaaaaa tttagtgtac cttcaggcta tcgttaaaga gacattgcgc ttgtatccac 1200
ctgttccttt tcttttacca cacgaagcag tgcaagattg taaagtgact ggttaccaca 1260
ttcctaaagg tactcgtcta tatatcaatg cgtggaaagt acatcgcgat cctgaaattt 1320
ggtcagagcc cgaaaagttt atgcccaata gattcttgac tagcaaagca aatatagatg 1380
ctcgcggtca aaattttgaa tttataccgt ttggttctgg gagacggtca tgtccaggga 1440
taggttttgc gactttagtg acacatctga cttttggtcg cttgcttcaa ggttttgatt 1500
ttagtaagcc atcaaacacg ccaattgaca tgacagaagg cgtaggcgtt actttgccta 1560
aggttaatca agttgaagtt ctaattaccc ctcgtttacc ttctaagctt tatttatttt 1620
gaaagtgcaa atcatcaatc atggcttgag taattagtta tactttaata tgtttctc 1678

<210> 250
<211> 521
<212> PRT
<213> Nicotiana tabacum

<400> 250

Met Asp Tyr His Ile Ser Phe His Phe Gln Ala Leu Leu Gly Leu Leu
1               5                   10                  15

Ala Phe Val Phe Leu Ser Ile Ile Leu Trp Arg Arg Thr Leu Thr Ser
            20                  25                  30

Arg Lys Leu Ala Pro Glu Ile Pro Gly Ala Trp Pro Ile Ile Gly His
        35                  40                  45

Leu Arg Gln Leu Ser Gly Thr Asp Lys Asn Ile Pro Phe Pro Arg Ile
    50                  55                  60

Leu Gly Ala Leu Ala Asp Lys Tyr Gly Pro Val Phe Thr Leu Arg Ile
65                  70                  75                  80

Gly Met Tyr Pro Tyr Leu Ile Val Asn Asn Trp Glu Ala Ala Lys Asp
                85                  90                  95

Cys Leu Thr Thr His Asp Lys Asp Phe Ala Ala Arg Pro Thr Ser Met
            100                 105                 110

Ala Gly Glu Ser Ile Gly Tyr Lys Tyr Ala Arg Phe Thr Tyr Ala Asn
        115                 120                 125

Phe Gly Pro Tyr Tyr Asn Gln Val Arg Lys Leu Ala Leu Gln His Val
    130                 135                 140

Pro Ser Ser Thr Lys Leu Glu Lys Met Lys His Ile Arg Val Ser Glu
145                 150                 155                 160

Leu Glu Thr Ser Ile Lys Glu Leu Tyr Ser Leu Thr Leu Gly Lys Asn
                165                 170                 175

Asn Met Gln Lys Val Asn Ile Ser Lys Trp Phe Glu Gln Leu Thr Leu
            180                 185                 190

Asn Ile Ile Val Lys Thr Ile Cys Gly Lys Arg Tyr Ser Asn Ile Glu
        195                 200                 205

Glu Asp Glu Glu Ala Gln Arg Phe Arg Lys Ala Phe Lys Gly Ile Met
    210                 215                 220

Phe Val Val Gly Gln Ile Val Leu Tyr Asp Ala Ile Pro Phe Pro Leu
225                 230                 235                 240

Phe Lys Tyr Phe Asp Phe Gln Gly His Ile Gln Leu Met Asn Lys Ile
                245                 250                 255

Tyr Lys Asp Leu Asp Ser Ile Leu Gln Gly Trp Leu Asp Asp His Met
            260                 265                 270

Met Asn Lys Asp Val Asn Asn Lys Asp Gln Asp Ala Ile Asp Ala Met
        275                 280                 285

Leu Lys Val Thr Gln Leu Asn Glu Phe Lys Ala Tyr Gly Phe Ser Gln
    290                 295                 300

Ala Thr Val Ile Lys Ser Thr Val Leu Ser Leu Ile Leu Asp Gly Asn
305                 310                 315                 320

Asp Thr Thr Ala Val His Leu Ile Trp Val Met Ser Leu Leu Leu Asn
                325                 330                 335

Asn Pro His Val Met Lys Gln Gly Gln Glu Glu Ile Asp Met Lys Val
            340                 345                 350

Gly Lys Glu Arg Trp Ile Glu Asp Thr Asp Ile Lys Asn Leu Val Tyr
        355                 360                 365

Leu Gln Ala Ile Val Lys Glu Thr Leu Arg Leu Tyr Pro Pro Val Pro
    370                 375                 380

Phe Leu Leu Pro His Glu Ala Val Gln Asp Cys Lys Val Thr Gly Tyr
385                 390                 395                 400

His Ile Pro Lys Gly Thr Arg Leu Tyr Ile Asn Ala Trp Lys Val His
                405                 410                 415

Arg Asp Pro Glu Ile Trp Ser Glu Pro Glu Lys Phe Met Pro Asn Arg
            420                 425                 430

Phe Leu Thr Ser Lys Ala Asn Ile Asp Ala Arg Gly Gln Asn Phe Glu
        435                 440                 445

Phe Ile Pro Phe Gly Ser Gly Arg Arg Ser Cys Pro Gly Ile Gly Phe
    450                 455                 460

Ala Thr Leu Val Thr His Leu Thr Phe Gly Arg Leu Leu Gln Gly Phe
465                 470                 475                 480

Asp Phe Ser Lys Pro Ser Asn Thr Pro Ile Asp Met Thr Glu Gly Val
                485                 490                 495

Gly Val Thr Leu Pro Lys Val Asn Gln Val Glu Val Leu Ile Thr Pro
            500                 505                 510

Arg Leu Pro Ser Lys Leu Tyr Leu Phe
        515                 520

<210>  251
<211>  1537
<212>  DNA
<213>  Nicotiana tabacum

<400>  251
tgataatgct ctttctactc tttgtagccc ttcctttcat tcttattttt cttcttccta      60

aattcaaaaa tggtggaaat aacagattgc caccaggtcc tataggttta ccattcattg     120

gaaatttgca tcaatatgat agtataactc ctcatatcta tttttggaaa ctttccaaaa     180

aatatggcaa aatcttctca ttaaaacttg cttctactaa tgtggtagta gtttcttcag     240

caaaattagc aaaagaagta ttgaaaaaac aagatttaat attttgtagt agaccatcta     300

ttcttggcca acaaaaactg tcttattatg gtcgtgatat tgcttttgca ccttataatg     360

attattggag agaaatgaga aaaatttgtg ttcttcatct tttagtttta aaaaaagttc     420

aattatttag tccaattcgt gaagatgaag tttttagaat gattaagaaa atatcaaaac     480

aagcttctac ttcacaaatt attaatttga gtaatttaat gatttcatta acaagtacaa     540

ttatttgtag agttgctttt ggtgttaggt ttgaagaaga agcacatgca aggaagagat     600

ttgattttct tttggccgag gcacaagaaa tgatggctag tttctttgta tctgattttt     660

ttcccttttt aagttggatt gataaattaa gtggattgac atatagactt gagaggaatt 720
tcaaggattt ggataatttt tatgaagaac tcattgagca acatcaaaat cctaataagc 780
caaaatatat ggaaggagat attgttgatc ttttgctaca attgaagaaa gagaaattaa 840
caccacttga tctcactatg gaagatataa aaggaattct catgaatgtg ttagttgcag 900
gatcagacac tagtgcagct gctactgttt gggcaatgac agccttgata aagaatccta 960
aagccatgga aaaagttcaa ttagaaatca gaaaatcagt tgggaagaaa ggcattgtaa 1020
atgaagaaga tgtccaaaac atcccttatt ttaaagcagt gataaaggaa atatttagat 1080
tgtatccacc agctccactt ttagttccaa gagaatcaat ggaaaaaacc atattagaag 1140
gttatgaaat tcggccaaga accatagttc atgttaacgc ttgggctata gcaagggatc 1200
ctgaaatatg ggaaaatcca gatgaattta tacctgagag atttttgaat agcagtaccg 1260
attacaaggg tcaagatttt gagttacttc catttggtgc aggcagaaga ggttgcccag 1320
gtattgcact tggggttgca tccatggaac ttgctttgtc aaatcttctt tatgcatttg 1380
attgggagtt gccttatgga gtgaaaaaag aagacatcga cacaaacgtt aggcctggaa 1440
ttgccatgca caagaaaaac gaactttgcc ttgtcccaaa aaattattta taaattatat 1500
tgggacgtgg atctcaattt agttctgtga ggtcagc 1537

<210> 252
<211> 495
<212> PRT
<213> Nicotiana tabacum

<400> 252

Met Leu Phe Leu Leu Phe Val Ala Leu Pro Phe Ile Leu Ile Phe Leu
1               5                   10                  15

Leu Pro Lys Phe Lys Asn Gly Gly Asn Asn Arg Leu Pro Pro Gly Pro
            20                  25                  30

Ile Gly Leu Pro Phe Ile Gly Asn Leu His Gln Tyr Asp Ser Ile Thr
        35                  40                  45

Pro His Ile Tyr Phe Trp Lys Leu Ser Lys Lys Tyr Gly Lys Ile Phe
    50                  55                  60

Ser Leu Lys Leu Ala Ser Thr Asn Val Val Val Val Ser Ser Ala Lys
65                  70                  75                  80

Leu Ala Lys Glu Val Leu Lys Lys Gln Asp Leu Ile Phe Cys Ser Arg
                85                  90                  95

Pro Ser Ile Leu Gly Gln Gln Lys Leu Ser Tyr Tyr Gly Arg Asp Ile
            100                 105                 110

Ala Phe Ala Pro Tyr Asn Asp Tyr Trp Arg Glu Met Arg Lys Ile Cys
        115                 120                 125

Val Leu His Leu Phe Ser Leu Lys Lys Val Gln Leu Phe Ser Pro Ile
    130                 135                 140

Arg Glu Asp Glu Val Phe Arg Met Ile Lys Lys Ile Ser Lys Gln Ala
145                 150                 155                 160

Ser Thr Ser Gln Ile Ile Asn Leu Ser Asn Leu Met Ile Ser Leu Thr
                165                 170                 175

Ser Thr Ile Ile Cys Arg Val Ala Phe Gly Val Arg Phe Glu Glu Glu
            180                 185                 190

Ala His Ala Arg Lys Arg Phe Asp Phe Leu Leu Ala Glu Ala Gln Glu
        195                 200                 205

Met Met Ala Ser Phe Phe Val Ser Asp Phe Phe Pro Phe Leu Ser Trp
    210                 215                 220

Ile Asp Lys Leu Ser Gly Leu Thr Tyr Arg Leu Glu Arg Asn Phe Lys
225                 230                 235                 240

Asp Leu Asp Asn Phe Tyr Glu Glu Leu Ile Glu Gln His Gln Asn Pro
                245                 250                 255

Asn Lys Pro Lys Tyr Met Glu Gly Asp Ile Val Asp Leu Leu Leu Gln
            260                 265                 270

Leu Lys Lys Glu Lys Leu Thr Pro Leu Asp Leu Thr Met Glu Asp Ile
        275                 280                 285

Lys Gly Ile Leu Met Asn Val Leu Val Ala Gly Ser Asp Thr Ser Ala
    290                 295                 300

Ala Ala Thr Val Trp Ala Met Thr Ala Leu Ile Lys Asn Pro Lys Ala
305                 310                 315                 320

Met Glu Lys Val Gln Leu Glu Ile Arg Lys Ser Val Gly Lys Lys Gly
                325                 330                 335

Ile Val Asn Glu Glu Asp Val Gln Asn Ile Pro Tyr Phe Lys Ala Val
            340                 345                 350

Ile Lys Glu Ile Phe Arg Leu Tyr Pro Pro Ala Pro Leu Leu Val Pro
        355                 360                 365

Arg Glu Ser Met Glu Lys Thr Ile Leu Glu Gly Tyr Glu Ile Arg Pro
    370                 375                 380

Arg Thr Ile Val His Val Asn Ala Trp Ala Ile Ala Arg Asp Pro Glu
385                 390                 395                 400

Ile Trp Glu Asn Pro Asp Glu Phe Ile Pro Glu Arg Phe Leu Asn Ser
                405                 410                 415

Ser Thr Asp Tyr Lys Gly Gln Asp Phe Glu Leu Leu Pro Phe Gly Ala
            420                 425                 430

Gly Arg Arg Gly Cys Pro Gly Ile Ala Leu Gly Val Ala Ser Met Glu
        435                 440                 445

Leu Ala Leu Ser Asn Leu Leu Tyr Ala Phe Asp Trp Glu Leu Pro Tyr
    450                 455                 460

Gly Val Lys Lys Glu Asp Ile Asp Thr Asn Val Arg Pro Gly Ile Ala
465                 470                 475                 480

Met His Lys Lys Asn Glu Leu Cys Leu Val Pro Lys Asn Tyr Leu
                485                 490                 495

<210>  253
<211>  1572
<212>  DNA
<213>  Nicotiana tabacum

<400>  253
ccaaaatcat ggctctatct ttcatattca tatccataac cctaattttt ctagttcata      60

aactctacca ccgtcttaga ttcaaactac caccaggtcc gcggccgtta ccggtggtcg     120

gaaacctcta cgacataaaa ccggtgagat tccggtgctt tgccgattgg gccaaaactt     180

acggtccgat tttctcagta tactttgggt cacagttaaa tgttgtggta acaacagctg     240

aattagctaa agaagtattg aaagaaaatg accagaattt agcagataga tttaggacta     300

gacctgcaaa taatttgagc agaaatggga tggatttgat ttgggctgat tatgggcctc     360

attatgtgaa agtaaggaag ctctgtaatc ttgagctttt tactcctaaa agacttgaag     420

ctcttagacc tattagagaa gatgaagtta ctgctatggt tgaaaacatt ttcaaggatt     480

gtactaagcc tgataacaca ggtaaaagct tgttgataag agagtactta ggatcagtag     540

cattcaacaa cattacaagg ttaacatttg ggaaaaggtt catgaactca aaaggtgaga     600

ttgatgagca aggtcaagaa ttcaagggta ttgtctctaa tggcatcaaa attggcggaa     660

aacttccctt ggcagagtat gttccatggc tccgttggtt tttcacaatg gaaaacgagg     720

cactcgtgaa gcactctgca cgtagagacc ggttaacaag aatgatcatg gatgaacaca     780

cactggctcg caagaaaact ggtgatacta agcagcattt tgtcgatgca ttgcttactc     840

ttcagaagca gtatgatctt agtgatgaca ctgttattgg cctcctctgg gatatgatta     900

cagcaggaat ggacacaaca accataacag tggaatgggc aatggcagaa ctagttaaga     960

acccaagagt gcaactaaaa gctcaagagg agcttgacag ggtaatcgga acggatcgaa    1020

tcatgtcaga aaccgatttc tctaacttcc cttacctaca atgtgtagcc aaagaggctc    1080

taaggttgca ccctccaact cctctaatgc ttcctcataa ggccagtgcc agtgtcaaaa 1140
ttggtggtta tgacattcct aaggggtcca tcgtgcacgt gaacgtttgg gctgtcgctc 1200
gtgacccagc cgtgtggaag aacccgttgg agttcagacc agagcgcttc cttgaggaag 1260
acgttgacat gaagggtcac gactatcggt tattgccctt tggtgcagga aggcgtgttt 1320
gccccggtgc acaacttgct atcaacttgg tcacatctat gttgggtcat ttgttgcatc 1380
attttacatg ggctccggcc ccgggggtta acccggagga tattgacttg gaggagagcc 1440
ctggaacagt aacttacatg aaaaatccaa tacaagctat tccaactcca agattgcctg 1500
cacacttgta tggacgtgtg ccagtggata tgtaaaacat tttgttcttt ccctttttgg 1560
ttatatgatg ag 1572

<210> 254
<211> 508
<212> PRT
<213> Nicotiana tabacum

<400> 254

Met Ala Leu Ser Phe Ile Phe Ile Ser Ile Thr Leu Ile Phe Leu Val
1               5                   10                  15

His Lys Leu Tyr His Arg Leu Arg Phe Lys Leu Pro Pro Gly Pro Arg
            20                  25                  30

Pro Leu Pro Val Val Gly Asn Leu Tyr Asp Ile Lys Pro Val Arg Phe
        35                  40                  45

Arg Cys Phe Ala Asp Trp Ala Lys Thr Tyr Gly Pro Ile Phe Ser Val
    50                  55                  60

Tyr Phe Gly Ser Gln Leu Asn Val Val Val Thr Thr Ala Glu Leu Ala
65                  70                  75                  80

Lys Glu Val Leu Lys Glu Asn Asp Gln Asn Leu Ala Asp Arg Phe Arg
                85                  90                  95

Thr Arg Pro Ala Asn Asn Leu Ser Arg Asn Gly Met Asp Leu Ile Trp
            100                 105                 110

Ala Asp Tyr Gly Pro His Tyr Val Lys Val Arg Lys Leu Cys Asn Leu
        115                 120                 125

Glu Leu Phe Thr Pro Lys Arg Leu Glu Ala Leu Arg Pro Ile Arg Glu
    130                 135                 140

Asp Glu Val Thr Ala Met Val Glu Asn Ile Phe Lys Asp Cys Thr Lys
145                 150                 155                 160

Pro Asp Asn Thr Gly Lys Ser Leu Leu Ile Arg Glu Tyr Leu Gly Ser
                165                 170                 175

Val Ala Phe Asn Asn Ile Thr Arg Leu Thr Phe Gly Lys Arg Phe Met
            180                 185                 190

Asn Ser Lys Gly Glu Ile Asp Glu Gln Gly Gln Glu Phe Lys Gly Ile
        195                 200                 205

Val Ser Asn Gly Ile Lys Ile Gly Gly Lys Leu Pro Leu Ala Glu Tyr
    210                 215                 220

Val Pro Trp Leu Arg Trp Phe Phe Thr Met Glu Asn Glu Ala Leu Val
225                 230                 235                 240

Lys His Ser Ala Arg Arg Asp Arg Leu Thr Arg Met Ile Met Asp Glu
                245                 250                 255

His Thr Leu Ala Arg Lys Lys Thr Gly Asp Thr Lys Gln His Phe Val
            260                 265                 270

Asp Ala Leu Leu Thr Leu Gln Lys Gln Tyr Asp Leu Ser Asp Asp Thr
        275                 280                 285

Val Ile Gly Leu Leu Trp Asp Met Ile Thr Ala Gly Met Asp Thr Thr
    290                 295                 300

Thr Ile Thr Val Glu Trp Ala Met Ala Glu Leu Val Lys Asn Pro Arg
305                 310                 315                 320

Val Gln Leu Lys Ala Gln Glu Glu Leu Asp Arg Val Ile Gly Thr Asp
                325                 330                 335

Arg Ile Met Ser Glu Thr Asp Phe Ser Lys Leu Pro Tyr Leu Gln Cys
            340                 345                 350

Val Ala Lys Glu Ala Leu Arg Leu His Pro Pro Thr Pro Leu Met Leu
        355                 360                 365

Pro His Lys Ala Ser Ala Ser Val Lys Ile Gly Gly Tyr Asp Ile Pro
    370                 375                 380

Lys Gly Ser Ile Val His Val Asn Val Trp Ala Val Ala Arg Asp Pro
385                 390                 395                 400

Ala Val Trp Lys Asn Pro Leu Glu Phe Arg Pro Glu Arg Phe Leu Glu
                405                 410                 415

Glu Asp Val Asp Met Lys Gly His Asp Tyr Arg Leu Leu Pro Phe Gly
            420                 425                 430

Ala Gly Arg Arg Val Cys Pro Gly Ala Gln Leu Ala Ile Asn Leu Val
        435                 440                 445

Thr Ser Met Leu Gly His Leu Leu His His Phe Thr Trp Ala Pro Ala
    450                 455                 460

Pro Gly Val Asn Pro Glu Asp Ile Asp Leu Glu Glu Ser Pro Gly Thr
465                 470                 475                 480

Val Thr Tyr Met Lys Asn Pro Ile Gln Ala Ile Pro Thr Pro Arg Leu
                485                 490                 495

Pro Ala His Leu Tyr Gly Arg Val Pro Val Asp Met
            500                 505

<210>  255
<211>  1580
<212>  DNA
<213>  Nicotiana tabacum

<400>  255
aatcactaat tttcatgtac tctcataggt caaaagtttc aaccaaaatc atggctctat      60
ccttcatatt catatccata accctaattt ttctagttca taaactctac caccgtctta     120
gattcaaact accaccaggt ccgcggccgt taccggtggt cggaaacctc tacgacatag     180
aaccggtgag attccggtgc tttgccgatt gggccaaaac ttacggtccg attttctcag      240
tatactttgg gtcacagtta aatgttgtgg taacaacagc tgaattagct aaagaagtat     300
tgaaagaaaa tgaccagaat ttagcagata gatttaggac tagacctgca aataatttga     360
gcagaaatgg gatggatttg atttgggctg attatgggcc tcattatgtg aaagtaagga     420
agctctgtaa tcttgagctt tttactccta aaagacttga agctcttaga cctattagag     480
aagatgaagt tactgctatg gttgaaaaca ttttcaagga ttgtactaag cctgataaca     540
caggtaaaag cttgttgata agagagtact taggatcagt agcattcaac aacattacaa     600
ggttaacatt tgggaaaagg ttcatgaact caaaaggtga gattgatgag caaggtcaag     660
aattcaaggg tattgtctct aatggcatca aaattggcgg aaaacttccc ttggcagagt     720
atgttccatg gctccgttgg tttttcacaa tggaaaacga ggcactcgtg aagcactctg     780
cacgtagaga ccggttaaca agaatgatca tggatgaaca cacactggct cgcaagaaaa     840
ctggtgatac taagcagcat tttgtcgatg cattgcttac tcttcagaag cagtatgatc     900
ttagtgatga cactgttatt ggcctcctct gggatatgat tacagcagga atggacacaa     960
caaccataac agtggaatgg gcaatggcag aactagttaa gaacccaaga gtgcaactaa    1020
aagctcaaga ggagcttgac agggtaatcg gaacggatcg aatcatgtca gaaaccgatt    1080
tctctaaact tccttaccta caatgtgtag ccaaagaggc tctaaggttg caccctccaa    1140
ctcctctaat gcttcctcat agggccagtg ccagtgtcaa aattggtggt tatgacattc    1200
ctaaggggtc catcgtgcac gtgaacgttt gggctgtcgc tcgtgaccca gccgtgtgga    1260
agaacccgtt ggagttcaga ccagagcgct tccttgagga agacgttgac atgaagggtc    1320

acgactatcg gttattgccc tttggtgcag gaaggcgtgt ttgccccggt gcacaacttg 1380

ctatcaactt ggtcacatct atgttgggtc atttgttgca tcattttaca tgggctccgg 1440

ccccgggggt taacccggag gatattgact tggaggagag ccctggaaca gtaacttaca 1500

tgaaaaatcc aatacaagct attccaactc caagattgcc tgcacacttg tatggacgtg 1560

tgccagtgga tatgtaaaac 1580

<210> 256
<211> 520
<212> PRT
<213> Nicotiana tabacum

<400> 256

Met Tyr Ser His Arg Ser Lys Val Ser Thr Lys Ile Met Ala Leu Ser
1               5                   10                  15

Phe Ile Phe Ile Ser Ile Thr Leu Ile Phe Leu Val His Lys Leu Tyr
            20                  25                  30

His Arg Leu Arg Phe Lys Leu Pro Pro Gly Pro Arg Pro Leu Pro Val
        35                  40                  45

Val Gly Asn Leu Tyr Asp Ile Glu Pro Val Arg Phe Arg Cys Phe Ala
    50                  55                  60

Asp Trp Ala Lys Thr Tyr Gly Pro Ile Phe Ser Val Tyr Phe Gly Ser
65                  70                  75                  80

Gln Leu Asn Val Val Val Thr Thr Ala Glu Leu Ala Lys Glu Val Leu
                85                  90                  95

Lys Glu Asn Asp Gln Asn Leu Ala Asp Arg Phe Arg Thr Arg Pro Ala
            100                 105                 110

Asn Asn Leu Ser Arg Asn Gly Met Asp Leu Ile Trp Ala Asp Tyr Gly
        115                 120                 125

Pro His Tyr Val Lys Val Arg Lys Leu Cys Asn Leu Glu Leu Phe Thr
    130                 135                 140

Pro Lys Arg Leu Glu Ala Leu Arg Pro Ile Arg Glu Asp Glu Val Thr
145                 150                 155                 160

Ala Met Val Glu Asn Ile Phe Lys Asp Cys Thr Lys Pro Asp Asn Thr
                165                 170                 175

Gly Lys Ser Leu Leu Ile Arg Glu Tyr Leu Gly Ser Val Ala Phe Asn
            180                 185                 190

Asn Ile Thr Arg Leu Thr Phe Gly Lys Arg Phe Met Asn Ser Lys Gly
        195                 200                 205

Glu Ile Asp Glu Gln Gly Gln Glu Phe Lys Gly Ile Val Ser Asn Gly
    210                 215                 220

Ile Lys Ile Gly Gly Lys Leu Pro Leu Ala Glu Tyr Val Pro Trp Leu
225                 230                 235                 240

Arg Trp Phe Phe Thr Met Glu Asn Glu Ala Leu Val Lys His Ser Ala
                245                 250                 255

Arg Arg Asp Arg Leu Thr Arg Met Ile Met Asp Glu His Thr Leu Ala
            260                 265                 270

Arg Lys Lys Thr Gly Asp Thr Lys Gln His Phe Val Asp Ala Leu Leu
        275                 280                 285

Thr Leu Gln Lys Gln Tyr Asp Leu Ser Asp Asp Thr Val Ile Gly Leu
    290                 295                 300

Leu Trp Asp Met Ile Thr Ala Gly Met Asp Thr Thr Thr Ile Thr Val
305                 310                 315                 320

Glu Trp Ala Met Ala Glu Leu Val Lys Asn Pro Arg Val Gln Leu Lys
                325                 330                 335

Ala Gln Glu Glu Leu Asp Arg Val Ile Gly Thr Asp Arg Ile Met Ser
            340                 345                 350

Glu Thr Asp Phe Ser Lys Leu Pro Tyr Leu Gln Cys Val Ala Lys Glu
        355                 360                 365

Ala Leu Arg Leu His Pro Pro Thr Pro Leu Met Leu Pro His Arg Ala
    370                 375                 380

Ser Ala Ser Val Lys Ile Gly Gly Tyr Asp Ile Pro Lys Gly Ser Ile
385                 390                 395                 400

Val His Val Asn Val Trp Ala Val Ala Arg Asp Pro Ala Val Trp Lys
                405                 410                 415

Asn Pro Leu Glu Phe Arg Pro Glu Arg Phe Leu Glu Glu Asp Val Asp
            420                 425                 430

Met Lys Gly His Asp Tyr Arg Leu Leu Pro Phe Gly Ala Gly Arg Arg
        435                 440                 445

Val Cys Pro Gly Ala Gln Leu Ala Ile Asn Leu Val Thr Ser Met Leu
    450                 455                 460

Gly His Leu Leu His His Phe Thr Trp Ala Pro Ala Pro Gly Val Asn
465                 470                 475                 480

Pro Glu Asp Ile Asp Leu Glu Glu Ser Pro Gly Thr Val Thr Tyr Met
                485                 490                 495

Lys Asn Pro Ile Gln Ala Ile Pro Thr Pro Arg Leu Pro Ala His Leu
            500                 505                 510

Tyr Gly Arg Val Pro Val Asp Met
        515                 520

<210> 257
<211> 1544
<212> DNA
<213> Nicotiana tabacum

<400> 257
ataatgctct ttctactctt tgtagccctt cctttcattc ttatttttct tcttcctaaa 60

ttcaaaaatg gtggaaataa cagattgcca ccaggtccta taggtttacc attcattgga 120

aatttgcatc aatatgatag tataactcct catatctatt tttggaaact ttccaaaaaa 180

tatggcaaaa tcttctcatt aaaacttgct tctactaatg tggtagtagt ttcttcagca 240

aaattagcaa aagaagtatt gaaaaaacaa gatttaatat tttgtagtag accatctatt 300

cttggccaac aaaaactgtc ttattatggt cgtgatattg cttttgcacc ttataatgat 360

tattggagag aaatgagaaa aatttgtgtt cttcatcttt ttagtttaaa aaaagttcaa 420

ttatttagtc caattcgtga agatgaagtt tttagaatga ttaagaaaat atcaaaacaa 480

gcttctactt cacaaattat taatttgagt aatttaatga tttcattaac aagtacaatt 540

atttgtagag ttgcttttgg tgttaggttt gaagaagaag cacatgcaag gaagagattt 600

gattttcttt tggccgaggc acaagaaatg atggctagtt tctttgtatc tgattttttt 660

ccctttttaa gttagattga caaattaagt ggattgacat atagacttga gaggaatttc 720

aaggatttgg ataattttta tgaagaactc attgagcaac atcaaaatcc taataagcca 780

aaatatatgg aaggagatat tgttgatctt ttgctacaat tgaagaaaga gaaattaaca 840

ccacttgatc tcactatgga agatataaaa ggaattctca tgaatgtgtt agttgcagga 900

tcagacacta gtgcagctgc tactgtttgg gcaatgacag ccttgataaa gaatcctaaa 960

gccatggaaa aagttcaatt agaaatcaga aaatcagttg ggaagaaagg cattgtaaat 1020

gaagaagatg tccaaaacat cccttatttt aaagcagtga taaaggaaat atttagattg 1080

tatccaccag ctccactttt agttccaaga gaatcaatgg aaaaaaccat attagaaggt 1140

tatgaaattc ggccaagaac catagttcat gttaacgctt gggctatagc aagggatcct 1200

gaaatatggg aaaatccaga tgaatttata cctgagagat ttttgaatag cagtatcgat 1260

tacaagggtc aagattttga gttacttcca tttggtgcag gcagaagagg ttgcccaggt 1320

attgcacttg ggttgcatcc catggaactt gctttgtcaa atcttcttta tgcatttgat 1380

tgggagttgc cttatggagt gaaaaaagaa gacatcgaca caaacgttag gcctggaatt 1440

gccatgcaca agaaaaacga actttgcctt gtcccaaaaa aattatttat aaattatatt    1500

gggacgtgga tctcatgcta gttctgtgcg gtcagctaag ctta                    1544

<210>  258
<211>  223
<212>  PRT
<213>  Nicotiana tabacum

<400>  258

Met Leu Phe Leu Leu Phe Val Ala Leu Pro Phe Ile Leu Ile Phe Leu
1               5                   10                  15

Leu Pro Lys Phe Lys Asn Gly Gly Asn Asn Arg Leu Pro Pro Gly Pro
            20                  25                  30

Ile Gly Leu Pro Phe Ile Gly Asn Leu His Gln Tyr Asp Ser Ile Thr
        35                  40                  45

Pro His Ile Tyr Phe Trp Lys Leu Ser Lys Lys Tyr Gly Lys Ile Phe
    50                  55                  60

Ser Leu Lys Leu Ala Ser Thr Asn Val Val Val Val Ser Ser Ala Lys
65                  70                  75                  80

Leu Ala Lys Glu Val Leu Lys Lys Gln Asp Leu Ile Phe Cys Ser Arg
                85                  90                  95

Pro Ser Ile Leu Gly Gln Gln Lys Leu Ser Tyr Tyr Gly Arg Asp Ile
            100                 105                 110

Ala Phe Ala Pro Tyr Asn Asp Tyr Trp Arg Glu Met Arg Lys Ile Cys
        115                 120                 125

Val Leu His Leu Phe Ser Leu Lys Lys Val Gln Leu Phe Ser Pro Ile
    130                 135                 140

Arg Glu Asp Glu Val Phe Arg Met Ile Lys Lys Ile Ser Lys Gln Ala
145                 150                 155                 160

Ser Thr Ser Gln Ile Ile Asn Leu Ser Asn Leu Met Ile Ser Leu Thr
                165                 170                 175

Ser Thr Ile Ile Cys Arg Val Ala Phe Gly Val Arg Phe Glu Glu Glu
            180                 185                 190

Ala His Ala Arg Lys Arg Phe Asp Phe Leu Leu Ala Glu Ala Gln Glu
        195                 200                 205

Met Met Ala Ser Phe Phe Val Ser Asp Phe Phe Pro Phe Leu Ser
    210                 215                 220

<210>  259
<211>  1615
<212>  DNA
<213>  Nicotiana tabacum

<400>  259
cacattgagt cctctcccaa atcactgatt caccaccaaa agtaccaaca attcaatgga      60

aggtacaaac ttgactacat atgcagcagt atttcttgat actctgtttc ttttgttcct     120

ttccaaactt cttcgccaga ggaaactcaa tttacctcca ggcccaaaac catggccgat     180

catcggaaac ttaaacctta ttggcaatct tcctcatcgc tcaatccacg aactctccct     240

caagtacgga cccgttatgc aactccaatt cgggtctttc cccgttgtag ttggatcctc     300

cgtcgaaatg gctaagattt tcctcaaatc catggatatt aactttgtag gcaggcctaa     360

aacggctgcc ggaaaataca caacgtacaa ttattccgat attacatggt ctccttacgg     420

accatattgg cgccaggcac gtaggatgtg cctaacggaa ttattcagca cgaaacgtct     480

cgattcatac gagtatattc gggctgagga gttgcattct cttctccata atttgaacaa     540

aatatcaggg aaaccaattg tgttgaaaga ttatttgacg acgttgagtt taaatgttat     600

tagcaggatg gtactgggga aaaggtattt ggacgaatcc gagaactcgt tcgtgaatcc     660

tgaggaattt aagaagatgt tggacgaatt gtttttgcta aatggtgtac ttaatattgg     720

agattcaatt ccatggattg atttcatgga tttgcaaggt tatgttaaga ggatgaaagt     780

agtgagcaag aaattcgaca agtttttaga gcatgttatt gatgagcata acattaggag     840

aaatggagtg gagaattatg ttgctaagga tatggtggat gttttgttgc agcttgctga     900

tgatccgaag ttggaagtta agctggagag acatggagtc aaagcattca ctcaggatat     960

gctggctggt ggaaccgaga gttcagcagt gacagtggag tgggcaattt cagagctgct    1020

aaagaagccg gagattttca aaaaggctac agaagaattg gatcgagtaa ttgggcagaa    1080

tagatgggta caagaaaagg acattccaaa tcttccttac atagaggcaa tagtcaaaga    1140

gactatgcga ctgcaccccg tggcaccaat gttggtgcca cgtgagtgtc gagaagatat    1200

taaggtagca ggctacgacg ttcagaaagg aactagggtt ctcgtgagtg tatggactat    1260

tggaagagac cctacattgt gggacgagcc tgaggtgttc aagccggaga gattccatga    1320

aaagtccata gatgttaaag gacatgatta tgagcttttg ccatttggag cggggagaag    1380

aatgtgcccg ggttatagct tggggctcaa ggtgattcaa gctagcttag ctaatcttct    1440

acatggattt aactggtcat tgcctgataa tatgactcct gaggacctca acatggatga    1500

gatttttggg ctctctacac ctaaaaaatt tccacttgct actgtgattg agccaagact    1560

ttcaccaaaa ctttactctg tttgattcag cagttctatg gttccgtcaa gatag         1615

<210>  260
<211>  509
<212>  PRT
<213>  Nicotiana tabacum

<400>  260

Met Glu Gly Thr Asn Leu Thr Thr Tyr Ala Ala Val Phe Leu Asp Thr
1               5                   10                  15

Leu Phe Leu Leu Phe Leu Ser Lys Leu Leu Arg Gln Arg Lys Leu Asn
            20                  25                  30

Leu Pro Pro Gly Pro Lys Pro Trp Pro Ile Ile Gly Asn Leu Asn Leu
        35                  40                  45

Ile Gly Asn Leu Pro His Arg Ser Ile His Glu Leu Ser Leu Lys Tyr
    50                  55                  60

Gly Pro Val Met Gln Leu Gln Phe Gly Ser Phe Pro Val Val Val Gly
65                  70                  75                  80

Ser Ser Val Glu Met Ala Lys Ile Phe Leu Lys Ser Met Asp Ile Asn
                85                  90                  95

Phe Val Gly Arg Pro Lys Thr Ala Ala Gly Lys Tyr Thr Thr Tyr Asn
            100                 105                 110

Tyr Ser Asp Ile Thr Trp Ser Pro Tyr Gly Pro Tyr Trp Arg Gln Ala
        115                 120                 125

Arg Arg Met Cys Leu Thr Glu Leu Phe Ser Thr Lys Arg Leu Asp Ser
    130                 135                 140

Tyr Glu Tyr Ile Arg Ala Glu Glu Leu His Ser Leu Leu His Asn Leu
145                 150                 155                 160

Asn Lys Ile Ser Gly Lys Pro Ile Val Leu Lys Asp Tyr Leu Thr Thr
                165                 170                 175

Leu Ser Leu Asn Val Ile Ser Arg Met Val Leu Gly Lys Arg Tyr Leu
            180                 185                 190

Asp Glu Ser Glu Asn Ser Phe Val Asn Pro Glu Glu Phe Lys Lys Met
        195                 200                 205

Leu Asp Glu Leu Phe Leu Leu Asn Gly Val Leu Asn Ile Gly Asp Ser
    210                 215                 220

Ile Pro Trp Ile Asp Phe Met Asp Leu Gln Gly Tyr Val Lys Arg Met
225                 230                 235                 240

Lys Val Val Ser Lys Lys Phe Asp Lys Phe Leu Glu His Val Ile Asp
                245                 250                 255

Glu His Asn Ile Arg Arg Asn Gly Val Glu Asn Tyr Val Ala Lys Asp
            260                 265                 270

Met Val Asp Val Leu Leu Gln Leu Ala Asp Asp Pro Lys Leu Glu Val
        275                 280                 285

Lys Leu Glu Arg His Gly Val Lys Ala Phe Thr Gln Asp Met Leu Ala
    290                 295                 300

Gly Gly Thr Glu Ser Ser Ala Val Thr Val Glu Trp Ala Ile Ser Glu
305                 310                 315                 320

Leu Leu Lys Lys Pro Glu Ile Phe Lys Lys Ala Thr Glu Glu Leu Asp
                325                 330                 335

Arg Val Ile Gly Gln Asn Arg Trp Val Gln Glu Lys Asp Ile Pro Asn
            340                 345                 350

Leu Pro Tyr Ile Glu Ala Ile Val Lys Glu Thr Met Arg Leu His Pro
        355                 360                 365

Val Ala Pro Met Leu Val Pro Arg Glu Cys Arg Glu Asp Ile Lys Val
    370                 375                 380

Ala Gly Tyr Asp Val Gln Lys Gly Thr Arg Val Leu Val Ser Val Trp
385                 390                 395                 400

Thr Ile Gly Arg Asp Pro Thr Leu Trp Asp Glu Pro Glu Val Phe Lys
                405                 410                 415

Pro Glu Arg Phe His Glu Lys Ser Ile Asp Val Lys Gly His Asp Tyr
            420                 425                 430

Glu Leu Leu Pro Phe Gly Ala Gly Arg Arg Met Cys Pro Gly Tyr Ser
        435                 440                 445

Leu Gly Leu Lys Val Ile Gln Ala Ser Leu Ala Asn Leu Leu His Gly
    450                 455                 460

Phe Asn Trp Ser Leu Pro Asp Asn Met Thr Pro Glu Asp Leu Asn Met
465                 470                 475                 480

Asp Glu Ile Phe Gly Leu Ser Thr Pro Lys Lys Phe Pro Leu Ala Thr
                485                 490                 495

Val Ile Glu Pro Arg Leu Ser Pro Lys Leu Tyr Ser Val
            500                 505

<210>  261
<211>  1610
<212>  DNA
<213>  Nicotiana tabacum

<400>  261
cttcttcctt cctaactaaa aatggagatt cagttttcta acttagttgc attcttgctc      60

tttctctcca gcatctttct tgtattcaaa aaatggaaaa ccagaaaact aaatttgcct 120
cctggtccat ggaaattacc ttttattgga agtttacacc atttggctgt ggcaggtcca 180
cttcctcacc atggcctaaa aaatttagcc aaacgctatg gtcctcttat gcatttacaa 240
cttggacaaa ttcctacact cgtcatatca tcacctcaaa tggcaaaaga agtactaaaa 300
actcacgacc tcgcttttgc cactagacca aagcttgtcg tggccgacat cattcactac 360
gacagcacgg acatagcact ttcgccatac ggtgaatact ggagacaaat tcgtaaaatt 420
tgcatattgg aactcttgag tgccaagatg gtcaagtttt ttagctcgat tcgccaagat 480
gagctctcga agatggtttc atctatacga acgacgccca atcttccagt caatcttacc 540
gacaagattt tttggtttac gagttcggta atttgtagat cagctttagg gaagatatgt 600
ggtgaccaag acaaattgat catttttatg agggaaataa tatcattggc aggtggattt 660
agtattgctg attttttccc tacatggaaa atgattcatg atattgatgg ttcaaaatct 720
aaactggtga aggcacatcg taagattgat gaaattttgg aaaatgtggt aaatgagcac 780
aaacagaatc gagcagatgg taaaaagggt aatggtgaat ttggtggaga agatctgatt 840
gatgttttgt taagagttag agaaagtgga gaagttcaaa ttccaatcac agatgacaat 900
atcaaatcaa tattaatcga catgttctct gccggatcgg aaacatcatc gacaactata 960
atttgggcat tagctgaaat gatgaagaaa ccaagtgttt tagcaaaggc acaagctgaa 1020
gtgagccaag ctttgaaggg gaagaaaatt agttttcaag agattgatat tgataagcta 1080
aagtatttga agttagtgat caaagaaact ttaagaatgc accctccaat tcctctgtta 1140
gtccctagag aatgtatgga agatacaaag attgatggtt acaatatacc tttcaaaaca 1200
agagtcattg ttaatgcatg ggcaattgga cgagatcctc aaagttggga tgatcctgaa 1260
agctttacgc cagagagatt tgagaataat tctattgatt ttcttggaaa tcatcatcaa 1320
tttattccat ttggtgcagg aagaaggatt tgtcctggaa tgctatttgg tttagctaat 1380
gttggacaac ctttagctca gttactttat cacttcgatt ggaaactccc taatggacaa 1440
actcaccaaa atttcgacat gactgagtca cctggaattt ctgctacaag aaaggatgat 1500
cttattttga ttgccactcc tgctcattct tgattaagta ttgctgcttt tctattggag 1560
aattttcaaa attcatccac aatatatagt gtttgctaga gttggttagc 1610

<210> 262
<211> 503
<212> PRT
<213> Nicotiana tabacum

<400> 262

Met Glu Ile Gln Phe Ser Asn Leu Val Ala Phe Leu Leu Phe Leu Ser
1               5                   10                  15

Ser Ile Phe Leu Val Phe Lys Lys Trp Lys Thr Arg Lys Leu Asn Leu
            20                  25                  30

Pro Pro Gly Pro Trp Lys Leu Pro Phe Ile Gly Ser Leu His His Leu
        35                  40                  45

Ala Val Ala Gly Pro Leu Pro His His Gly Leu Lys Asn Leu Ala Lys
    50                  55                  60

Arg Tyr Gly Pro Leu Met His Leu Gln Leu Gly Gln Ile Pro Thr Leu
65                  70                  75                  80

Val Ile Ser Ser Pro Gln Met Ala Lys Glu Val Leu Lys Thr His Asp
                85                  90                  95

Leu Ala Phe Ala Thr Arg Pro Lys Leu Val Val Ala Asp Ile Ile His
            100                 105                 110

Tyr Asp Ser Thr Asp Ile Ala Leu Ser Pro Tyr Gly Glu Tyr Trp Arg
        115                 120                 125

Gln Ile Arg Lys Ile Cys Ile Leu Glu Leu Leu Ser Ala Lys Met Val
    130                 135                 140

Lys Phe Phe Ser Ser Ile Arg Gln Asp Glu Leu Ser Lys Met Val Ser
145                 150                 155                 160

Ser Ile Arg Thr Thr Pro Asn Leu Pro Val Asn Leu Thr Asp Lys Ile
                165                 170                 175

Phe Trp Phe Thr Ser Ser Val Ile Cys Arg Ser Ala Leu Gly Lys Ile
            180                 185                 190

Cys Gly Asp Gln Asp Lys Leu Ile Ile Phe Met Arg Glu Ile Ile Ser
        195                 200                 205

Leu Ala Gly Gly Phe Ser Ile Ala Asp Phe Phe Pro Thr Trp Lys Met
    210                 215                 220

Ile His Asp Ile Asp Gly Ser Lys Ser Lys Leu Val Lys Ala His Arg
225                 230                 235                 240

Lys Ile Asp Glu Ile Leu Glu Asn Val Val Asn Glu His Lys Gln Asn
                245                 250                 255

Arg Ala Asp Gly Lys Lys Gly Asn Gly Glu Phe Gly Gly Glu Asp Leu
            260                 265                 270

Ile Asp Val Leu Leu Arg Val Arg Glu Ser Gly Glu Val Gln Ile Pro
        275                 280                 285

Ile Thr Asp Asp Asn Ile Lys Ser Ile Leu Ile Asp Met Phe Ser Ala
    290                 295                 300

Gly Ser Glu Thr Ser Ser Thr Thr Ile Ile Trp Ala Leu Ala Glu Met
305                 310                 315                 320

Met Lys Lys Pro Ser Val Leu Ala Lys Ala Gln Ala Glu Val Ser Gln
                325                 330                 335

Ala Leu Lys Gly Lys Lys Ile Ser Phe Gln Glu Ile Asp Ile Asp Lys
            340                 345                 350

Leu Lys Tyr Leu Lys Leu Val Ile Lys Glu Thr Leu Arg Met His Pro
        355                 360                 365

Pro Ile Pro Leu Leu Val Pro Arg Glu Cys Met Glu Asp Thr Lys Ile
    370                 375                 380

Asp Gly Tyr Asn Ile Pro Phe Lys Thr Arg Val Ile Val Asn Ala Trp
385                 390                 395                 400

Ala Ile Gly Arg Asp Pro Gln Ser Trp Asp Asp Pro Glu Ser Phe Thr
                405                 410                 415

Pro Glu Arg Phe Glu Asn Asn Ser Ile Asp Phe Leu Gly Asn His His
            420                 425                 430

Gln Phe Ile Pro Phe Gly Ala Gly Arg Arg Ile Cys Pro Gly Met Leu
        435                 440                 445

Phe Gly Leu Ala Asn Val Gly Gln Pro Leu Ala Gln Leu Leu Tyr His
    450                 455                 460

Phe Asp Trp Lys Leu Pro Asn Gly Gln Thr His Gln Asn Phe Asp Met
465                 470                 475                 480

Thr Glu Ser Pro Gly Ile Ser Ala Thr Arg Lys Asp Asp Leu Ile Leu
                485                 490                 495

Ile Ala Thr Pro Ala His Ser
            500

<210>  263
<211>  1611
<212>  DNA
<213>  Nicotiana tabacum

<400>  263
ctttcatcat atggcatgaa atgggaaatg ctcacaacag caaaattgca gcaatctgtt      60

tgataatttt cttggtatat aaagcatggg aattgttgaa gtggatatgg attaagccaa     120

agaaactgga gagttgcctc agaaaacagg gactcaaagg aaattcctac gggctattct     180

atggagatat gaaagaattg tccaaaagtc tcaaggaaat caattcaaag cccatcatca     240

atctatcaaa tgaagtagcc ccaagaatca ttccttatta tcttgaaatc atccaaaaat     300

atggtaaaag atgttttgtt tggcaaggac caacccccgc aatattaata acagagccag 360
aattaataaa ggagatattt ggtaagaact atgtttttca gaagcctaat aatcccaacc 420
cactgaccaa gttattggct cgaggtgttg taagctacga ggaagaaaaa tgggcaaaac 480
acagaaagat cttaaatcct gcctttcata tggagaagtt gaagcatatg ctaccagcat 540
tttacttgag ctgtagtgag atgctgaaca aatgggagga gattatccca gtaaaagaat 600
caaatgagtt ggacatttgg cctcatcttc aaagaatgac aagtgatgtg atttctcgtg 660
ctgcctttgg tagtagctac gaagaaggaa gaagaatatt tgaacttcaa gaagaacaag 720
ctgagtatct aacgaagaca ttcaattcag tttatatccc aggttccaga ttttttccca 780
ataaaatgaa caaaagaatg aaagaatgtg aaaaggaagt acgagaaaca attacgtgtc 840
taattgacaa cagattaaag gcaaaagaag aaggcaatgg caaggccctc aatgatgacc 900
tattgggtat attattagag tcaaattcta tagaaattga agaacatggt aacaagaagt 960
ttggaatgag tatacctgaa gtaattgaag agtgcaaatt attctatttt gctggccaag 1020
agactacatc agtattgctt gtgtggacac tgattttgtt agggagaaat ccagaatggc 1080
aggaacgtgc tagagaggaa gtttttcaag cctttggaag tgataaacca acttttgacg 1140
aattatatcg cttgaaaatt gtgacgatga ttttgtacga gtctttaagg ttatatccac 1200
caatagcaac tcgtactcga aggactaatg aagaaacaaa attaggggaa ctagatttac 1260
caaagggtgc actgctcttt ataccaacaa tcttattaca tcttgacagg gaaatttggg 1320
gtgaagatgc agatgagttc aatccggaga gatttagcga aggggtggca aaggcaacaa 1380
aggggaaaat gacatatttt ccatttggtg caggaccgcg aaaatgcatt gggcaaaact 1440
tcgcgatttt ggaagcaaaa atggctatag ctatgattct acaacgcttc tccttcgagc 1500
tctctccatc ttatacacac tctccataca ctgtggtcac tttgaaaccc aaatatggtg 1560
ctcccctaat aatgcacagg ctgtagtcct gtgagaatat gctatccgag g 1611

<210> 264
<211> 521
<212> PRT
<213> Nicotiana tabacum

<400> 264

Met Gly Asn Ala His Asn Ser Lys Ile Ala Ala Ile Cys Leu Ile Ile
1               5                   10                  15

Phe Leu Val Tyr Lys Ala Trp Glu Leu Leu Lys Trp Ile Trp Ile Lys
            20                  25                  30

Pro Lys Lys Leu Glu Ser Cys Leu Arg Lys Gln Gly Leu Lys Gly Asn
        35                  40                  45

Ser Tyr Gly Leu Phe Tyr Gly Asp Met Lys Glu Leu Ser Lys Ser Leu
    50                  55                  60

Lys Glu Ile Asn Ser Lys Pro Ile Ile Asn Leu Ser Asn Glu Val Ala
65                  70                  75                  80

Pro Arg Ile Ile Pro Tyr Tyr Leu Glu Ile Ile Gln Lys Tyr Gly Lys
                85                  90                  95

Arg Cys Phe Val Trp Gln Gly Pro Thr Pro Ala Ile Leu Ile Thr Glu
            100                 105                 110

Pro Glu Leu Ile Lys Glu Ile Phe Gly Lys Asn Tyr Val Phe Gln Lys
        115                 120                 125

Pro Asn Asn Pro Asn Pro Leu Thr Lys Leu Leu Ala Arg Gly Val Val
    130                 135                 140

Ser Tyr Glu Glu Glu Lys Trp Ala Lys His Arg Lys Ile Leu Asn Pro
145                 150                 155                 160

Ala Phe His Met Glu Lys Leu Lys His Met Leu Pro Ala Phe Tyr Leu
                165                 170                 175

Ser Cys Ser Glu Met Leu Asn Lys Trp Glu Glu Ile Ile Pro Val Lys
            180                 185                 190

Glu Ser Asn Glu Leu Asp Ile Trp Pro His Leu Gln Arg Met Thr Ser
        195                 200                 205

Asp Val Ile Ser Arg Ala Ala Phe Gly Ser Ser Tyr Glu Glu Gly Arg
    210                 215                 220

Arg Ile Phe Glu Leu Gln Glu Glu Gln Ala Glu Tyr Leu Thr Lys Thr
225                 230                 235                 240

Phe Asn Ser Val Tyr Ile Pro Gly Ser Arg Phe Phe Pro Asn Lys Met
                245                 250                 255

Asn Lys Arg Met Lys Glu Cys Glu Lys Glu Val Arg Glu Thr Ile Thr
            260                 265                 270

Cys Leu Ile Asp Asn Arg Leu Lys Ala Lys Glu Glu Gly Asn Gly Lys
        275                 280                 285

Ala Leu Asn Asp Asp Leu Leu Gly Ile Leu Leu Glu Ser Asn Ser Ile
    290                 295                 300

Glu Ile Glu Glu His Gly Asn Lys Lys Phe Gly Met Ser Ile Pro Glu
305                 310                 315                 320

Val Ile Glu Glu Cys Lys Leu Phe Tyr Phe Ala Gly Gln Glu Thr Thr
                325                 330                 335

Ser Val Leu Leu Val Trp Thr Leu Ile Leu Leu Gly Arg Asn Pro Glu
            340                 345                 350

Trp Gln Glu Arg Ala Arg Glu Glu Val Phe Gln Ala Phe Gly Ser Asp
        355                 360                 365

Lys Pro Thr Phe Asp Glu Leu Tyr Arg Leu Lys Ile Val Thr Met Ile
    370                 375                 380

Leu Tyr Glu Ser Leu Arg Leu Tyr Pro Pro Ile Ala Thr Arg Thr Arg
385                 390                 395                 400

Arg Thr Asn Glu Glu Thr Lys Leu Gly Glu Leu Asp Leu Pro Lys Gly
                405                 410                 415

Ala Leu Leu Phe Ile Pro Thr Ile Leu Leu His Leu Asp Arg Glu Ile
            420                 425                 430

Trp Gly Glu Asp Ala Asp Glu Phe Asn Pro Glu Arg Phe Ser Glu Gly
        435                 440                 445

Val Ala Lys Ala Thr Lys Gly Lys Met Thr Tyr Phe Pro Phe Gly Ala
    450                 455                 460

Gly Pro Arg Lys Cys Ile Gly Gln Asn Phe Ala Ile Leu Glu Ala Lys
465                 470                 475                 480

Met Ala Ile Ala Met Ile Leu Gln Arg Phe Ser Phe Glu Leu Ser Pro
                485                 490                 495

Ser Tyr Thr His Ser Pro Tyr Thr Val Val Thr Leu Lys Pro Lys Tyr
            500                 505                 510

Gly Ala Pro Leu Ile Met His Arg Leu
        515                 520

<210>  265
<211>  1570
<212>  DNA
<213>  Nicotiana tabacum

<400>  265
ctcattatcc atcacctaaa atggagaatt cttgggtttt tctagccttg gcagggctat      60

ctgcattagc ttttctctgt aaaataatca cctgtcgaag accggttaac cggaaaatac     120

caccaggtcc aaaaccatgg cccatcattg gcaatttgaa cctacttggt cctataccac     180

atcaatcttt tgacttgctt tccaaaaaat atggagagtt gatgctgctg aaatttggct     240

ccaggccagt tcttgttgct tcatctgctg aaatggcaaa acagttttta aaagtacatg     300

atgctaattt cgcctcccgt cctatgctag ctggtggaaa gtatacaagc tataactatt     360

gtgacatgac atgggcaccc tatggtccct attggcgcca agcacgacga atttacctta     420

accagatatt tactccgaaa aggctagact cgttcgagta cattcgtgtt gaagaaaggc 480
aggccttgat ttcccagctg aattcccttg ctggaaagcc attttttctc aaagaccatt 540
tgtcgcgatt tagcctctgc agcatgacaa ggatggtttt gagcaacaag tattttggtg 600
aatcaacagt tagagtagaa gatttgcagt acctggtaga tcaatggttc ttacttaatg 660
gtgctttcaa cattggagat tggattccat ggctcagctt cttggaccta caaggctatg 720
tgaaacaaat gaaggctttg aaaagaactt ttgataagtt ccacaacatt gtgctagatg 780
atcacagggc taagaagaat gcagagaaga actttgtccc aaaagacatg gttgatgtct 840
tgttgaagat ggctgaagat cctaatctgg aagtcaaact cactaatgac tgtgtcaaag 900
ggttaatgca ggatttacta actggaggaa cagatagctt aacagcagca gtgcaatggg 960
catttcaaga acttcttaga cagccaaggg ttattgagaa ggcaaccgaa gagcttgacc 1020
ggattgtcgg gaaagagaga tgggtagaag agaaagattg ctcgcagcta tcttacgttg 1080
aagcaatcct caaggaaaca ctaaggttac atcctctagg aactatgcta gcaccgcatt 1140
gtgctataga agattgtaac gtggctggtt atgacataca gaaaggaacg acctttctgg 1200
tgaatgtttg gaccattgga agggacccaa aatactggga tagagcacaa gagtttctcc 1260
ccgagagatt tttagagaac gacattgata tggacggaca taactttgct ttcttgccat 1320
ttggctcggg gcgaaggagg tgccctggct atagccttgg acttaaggtt atccgagtaa 1380
cattagccaa catgttgcat ggattcaact ggaaattacc tgaaggtatg aagccagaag 1440
atataagtgt ggaagaacat tatgggctca ctacacatcc taagtttcct gttcctgtga 1500
tcttggaatc tagactttct tcagatctct attcccccat cacttaatcc taagtgcttc 1560
ctattatagc 1570

<210> 266
<211> 508
<212> PRT
<213> Nicotiana tabacum

<400> 266

Met Glu Asn Ser Trp Val Phe Leu Ala Leu Ala Gly Leu Ser Ala Leu
1               5                   10                  15

Ala Phe Leu Cys Lys Ile Ile Thr Cys Arg Arg Pro Val Asn Arg Lys
            20                  25                  30

Ile Pro Pro Gly Pro Lys Pro Trp Pro Ile Ile Gly Asn Leu Asn Leu
        35                  40                  45

Leu Gly Pro Ile Pro His Gln Ser Phe Asp Leu Leu Ser Lys Lys Tyr
    50                  55                  60

Gly Glu Leu Met Leu Leu Lys Phe Gly Ser Arg Pro Val Leu Val Ala
65                  70                  75                  80

Ser Ser Ala Glu Met Ala Lys Gln Phe Leu Lys Val His Asp Ala Asn
                85                  90                  95

Phe Ala Ser Arg Pro Met Leu Ala Gly Gly Lys Tyr Thr Ser Tyr Asn
            100                 105                 110

Tyr Cys Asp Met Thr Trp Ala Pro Tyr Gly Pro Tyr Trp Arg Gln Ala
        115                 120                 125

Arg Arg Ile Tyr Leu Asn Gln Ile Phe Thr Pro Lys Arg Leu Asp Ser
    130                 135                 140

Phe Glu Tyr Ile Arg Val Glu Glu Arg Gln Ala Leu Ile Ser Gln Leu
145                 150                 155                 160

Asn Ser Leu Ala Gly Lys Pro Phe Phe Leu Lys Asp His Leu Ser Arg
                165                 170                 175

Phe Ser Leu Cys Ser Met Thr Arg Met Val Leu Ser Asn Lys Tyr Phe
            180                 185                 190

Gly Glu Ser Thr Val Arg Val Glu Asp Leu Gln Tyr Leu Val Asp Gln
        195                 200                 205

Trp Phe Leu Leu Asn Gly Ala Phe Asn Ile Gly Asp Trp Ile Pro Trp
    210                 215                 220

Leu Ser Phe Leu Asp Leu Gln Gly Tyr Val Lys Gln Met Lys Ala Leu
225                 230                 235                 240

Lys Arg Thr Phe Asp Lys Phe His Asn Ile Val Leu Asp Asp His Arg
                245                 250                 255

Ala Lys Lys Asn Ala Glu Lys Asn Phe Val Pro Lys Asp Met Val Asp
            260                 265                 270

Val Leu Leu Lys Met Ala Glu Asp Pro Asn Leu Glu Val Lys Leu Thr
        275                 280                 285

Asn Asp Cys Val Lys Gly Leu Met Gln Asp Leu Leu Thr Gly Gly Thr
    290                 295                 300

Asp Ser Leu Thr Ala Ala Val Gln Trp Ala Phe Gln Glu Leu Leu Arg
305                 310                 315                 320

Gln Pro Arg Val Ile Glu Lys Ala Thr Glu Glu Leu Asp Arg Ile Val
                325                 330                 335

Gly Lys Glu Arg Trp Val Glu Glu Lys Asp Cys Ser Gln Leu Ser Tyr
            340                 345                 350

Val Glu Ala Ile Leu Lys Glu Thr Leu Arg Leu His Pro Leu Gly Thr
        355                 360                 365

Met Leu Ala Pro His Cys Ala Ile Glu Asp Cys Asn Val Ala Gly Tyr
    370                 375                 380

Asp Ile Gln Lys Gly Thr Thr Phe Leu Val Asn Val Trp Thr Ile Gly
385                 390                 395                 400

Arg Asp Pro Lys Tyr Trp Asp Arg Ala Gln Glu Phe Leu Pro Glu Arg
                405                 410                 415

Phe Leu Glu Asn Asp Ile Asp Met Asp Gly His Asn Phe Ala Phe Leu
            420                 425                 430

Pro Phe Gly Ser Gly Arg Arg Arg Cys Pro Gly Tyr Ser Leu Gly Leu
        435                 440                 445

Lys Val Ile Arg Val Thr Leu Ala Asn Met Leu His Gly Phe Asn Trp
    450                 455                 460

Lys Leu Pro Glu Gly Met Lys Pro Glu Asp Ile Ser Val Glu Glu His
465                 470                 475                 480

Tyr Gly Leu Thr Thr His Pro Lys Phe Pro Val Pro Val Ile Leu Glu
                485                 490                 495

Ser Arg Leu Ser Ser Asp Leu Tyr Ser Pro Ile Thr
            500                 505

<210>  267
<211>  1617
<212>  DNA
<213>  Nicotiana tabacum

<400>  267
cacattgagt cctctcccaa atcactgatt caccaccaaa agtaccaaca attcaatgga      60

aggtacaaac ttgactacat atgcagcagt atttcttgat actctgtttc ttttgttcct     120

ttccaaactt cttcgccaga ggaaactcaa tttacctcca ggcccaaaac catggccgat     180

catcggaaac ttaaacctta ttggcaatct tcctcatcgc tcaatccacg aactctccct     240

caagtacgga cccgttatgc aactccaatt cgggtctttc cccgttgtag ttggatcctc     300

cgtcgaaatg gctaagattt tcctcaaatc catggatatt aactttgtag gcaggcctaa     360

aacggctgcc ggaaaataca caacgtacaa ttattccgat attacatggt ctccttacgg     420

accatattgg cgccaggcac gtaggatgtg cctaacggaa ttattcagca cgaaacgtct     480

cgattcatac gagtatattc gggctgagga gttgcattct cttctccata atttgaacaa     540

aatatcaggg aaaccaattg tgttgaaaga ttatttgacg acgttgagtt taaatgttat     600

tagcaggatg gtactgggga aaaggtattt ggacgaatcc gagaactcgt tcgtgaatcc     660

tgaggaattt aagaagatgt tggacgaatt gtttttgcta aatggtgtac ttaatattgg 720
agattcaatt ccatggattg atttcatgga tttgcaaggt tatgttaaga ggatgaaagt 780
agtgagcaag aaattcgaca agtttttaga gcatgttatt gatgagcata acattaggag 840
aaatggagtg gagaattatg ttgctaagga tatggtggat gttttgttgc agcttgctga 900
tgatccgaag ttggaagtta agctggagag acatggagtc aaagcattca ctcaggatat 960
gctggctggt ggaaccgaga gttcagcagt gacagtggag tgggcaattt cagagctgct 1020
aaagaagccg gagattttca aaaaggctac agaagaattg gatcgagtaa ttgggcagaa 1080
tagatgggta caagaaaagg acattccaaa tcatccttac atagaggcaa tagtcaaaga 1140
gactatgcga ctgcaccccg tggcaccaat gttggtgcca cgtgagtgtc gagaagatat 1200
taaggtagca ggctacgacg ttcagaaagg aactagggtt ctcgtgagtg tatggactat 1260
tggaagagac cctacattgt gggacgagcc tgaggtgttc aagccggaga gattccatga 1320
aaagtccata gatgttaaag gacatgatta tgagcttttg ccatttggag cggggagaag 1380
aatgtgcccg ggttatagct tggggctcaa ggtgattcaa gctagcttag ctaatcttct 1440
acatggattt aactggtcat tgcctgataa tatgactcct gaggacctca acatggatga 1500
gatttttggg ctctctacac ctaaaaaatt tccacttgct actgtgattg agccaagact 1560
ttcaccaaaa ctttactctg tttgattcag cagttctatg gatccgtcaa gatagac 1617

<210> 268
<211> 509
<212> PRT
<213> Nicotiana tabacum

<400> 268

Met Glu Gly Thr Asn Leu Thr Thr Tyr Ala Ala Val Phe Leu Asp Thr
1               5                   10                  15

Leu Phe Leu Leu Phe Leu Ser Lys Leu Leu Arg Gln Arg Lys Leu Asn
            20                  25                  30

Leu Pro Pro Gly Pro Lys Pro Trp Pro Ile Ile Gly Asn Leu Asn Leu
        35                  40                  45

Ile Gly Asn Leu Pro His Arg Ser Ile His Glu Leu Ser Leu Lys Tyr
    50                  55                  60

Gly Pro Val Met Gln Leu Gln Phe Gly Ser Phe Pro Val Val Val Gly
65                  70                  75                  80

Ser Ser Val Glu Met Ala Lys Ile Phe Leu Lys Ser Met Asp Ile Asn
                85                  90                  95

Phe Val Gly Arg Pro Lys Thr Ala Ala Gly Lys Tyr Thr Thr Tyr Asn
            100                 105                 110

Tyr Ser Asp Ile Thr Trp Ser Pro Tyr Gly Pro Tyr Trp Arg Gln Ala
        115                 120                 125

Arg Arg Met Cys Leu Thr Glu Leu Phe Ser Thr Lys Arg Leu Asp Ser
    130                 135                 140

Tyr Glu Tyr Ile Arg Ala Glu Glu Leu His Ser Leu Leu His Asn Leu
145                 150                 155                 160

Asn Lys Ile Ser Gly Lys Pro Ile Val Leu Lys Asp Tyr Leu Thr Thr
                165                 170                 175

Leu Ser Leu Asn Val Ile Ser Arg Met Val Leu Gly Lys Arg Tyr Leu
            180                 185                 190

Asp Glu Ser Glu Asn Ser Phe Val Asn Pro Glu Glu Phe Lys Lys Met
        195                 200                 205

Leu Asp Glu Leu Phe Leu Leu Asn Gly Val Leu Asn Ile Gly Asp Ser
    210                 215                 220

Ile Pro Trp Ile Asp Phe Met Asp Leu Gln Gly Tyr Val Lys Arg Met
225                 230                 235                 240

Lys Val Val Ser Lys Lys Phe Asp Lys Phe Leu Glu His Val Ile Asp
                245                 250                 255

Glu His Asn Ile Arg Arg Asn Gly Val Glu Asn Tyr Val Ala Lys Asp
            260                 265                 270

Met Val Asp Val Leu Leu Gln Leu Ala Asp Asp Pro Lys Leu Glu Val
        275                 280                 285

Lys Leu Glu Arg His Gly Val Lys Ala Phe Thr Gln Asp Met Leu Ala
    290                 295                 300

Gly Gly Thr Glu Ser Ser Ala Val Thr Val Glu Trp Ala Ile Ser Glu
305                 310                 315                 320

Leu Leu Lys Lys Pro Glu Ile Phe Lys Lys Ala Thr Glu Glu Leu Asp
                325                 330                 335

Arg Val Ile Gly Gln Asn Arg Trp Val Gln Glu Lys Asp Ile Pro Asn
            340                 345                 350

His Pro Tyr Ile Glu Ala Ile Val Lys Glu Thr Met Arg Leu His Pro
        355                 360                 365

Val Ala Pro Met Leu Val Pro Arg Glu Cys Arg Glu Asp Ile Lys Val
    370                 375                 380

Ala Gly Tyr Asp Val Gln Lys Gly Thr Arg Val Leu Val Ser Val Trp
385                 390                 395                 400

Thr Ile Gly Arg Asp Pro Thr Leu Trp Asp Glu Pro Glu Val Phe Lys
                405                 410                 415

Pro Glu Arg Phe His Glu Lys Ser Ile Asp Val Lys Gly His Asp Tyr
            420                 425                 430

Glu Leu Leu Pro Phe Gly Ala Gly Arg Arg Met Cys Pro Gly Tyr Ser
        435                 440                 445

Leu Gly Leu Lys Val Ile Gln Ala Ser Leu Ala Asn Leu Leu His Gly
    450                 455                 460

Phe Asn Trp Ser Leu Pro Asp Asn Met Thr Pro Glu Asp Leu Asn Met
465                 470                 475                 480

Asp Glu Ile Phe Gly Leu Ser Thr Pro Lys Lys Phe Pro Leu Ala Thr
                485                 490                 495

Val Ile Glu Pro Arg Leu Ser Pro Lys Leu Tyr Ser Val
            500                 505

<210>  269
<211>  1657
<212>  DNA
<213>  Nicotiana tabacum

<400>  269
tgcaatatag ttttcctagt cagttctagc ctccttttcc ttagaaataa tggattatca      60

tatttctttc cattttcaag ctcttttagg gcttttagcc tttgtgttct tgtctattat     120

cttatggaga agaacactca cttcaagaaa attagcccct gaaatcccag gggcatggcc     180

tattataggc catcttcgtc agctgagtgg tactgataag aatatcccat ttccccgaat     240

attgggcgct ttggcagata aatatggacc tgtcttcaca ctgagaatag ggatgtaccc     300

ctatttgatt gtcaacaatt gggaagcagc taaggattgt ctcacaacgc atgataagga     360

cttcgctgcc cgaccaactt ctatggctgg tgaaagcatc gggtacaagt atgcgaggtt     420

tacttatgct aattttggtc cttattataa ccaagtgcgc aaactagccc tacaacatgt     480

actctcgagt actaaactcg agaaaatgaa acacatacgt gtttctgaat tggaaactag     540

catcaaagaa ttatattctt tgacgctggg caaaaacaac atgcaaaaag tgaatataag     600

taaatggttt gaacaattga ctttaaacat aatcgtgaag acaatttgtg gcaagagata     660

tagcaacata gaggaggatg aagaggcaca acgtttcaga aaggcattta agggcatcat     720

gtttgttgta gggcaaattg ttttatatga cgcaattcca ttcccattgt tcaaatactt     780

tgatttccaa ggtcatatac aattgatgaa caaaatttat aaagacttag attctattct     840

tcaaggatgg ttggatgatc atatgatgaa caaggatgta aacaataagg atcaagatgc     900

catagatgcc atgcttaagg taacacaact taatgaattc aaagcctatg gtttttctca 960
ggccactgtg atcaagtcga cagtcttgag tttgatctta gatggaaatg acacaaccgc 1020
tgttcatttg atatgggtaa tgtccttatt actgaacaat ccacatgtta tgaaacaagg 1080
ccaagaagag atagacatga aagtgggtaa agagaggtgg attgaagata ctgacataaa 1140
aaatttagtg taccttcagg ctatcgttaa agagacattg cgcttgtatc cacctgttcc 1200
ttttctttta ccacacgaag cagtgcaaga ttgtaaagtg actggttacc acattcctaa 1260
aggtactcgt ctatatatca atgcgtggaa agtacatcgc gattctgaaa tttggtcaga 1320
gcccgaaaag tttatgccca atagattctt gactagcaaa gcaaatatag atgctcgcgg 1380
tcaaaatttt gaatttatac cgtttggttc tgggagacgg tcatgtccag ggttaggttt 1440
tgcgacttta gtgacacatc tgacttttgg tcgcttgctt caaggttttg attttagtaa 1500
gccatcaaac acgccaattg acatgacaga aggcgtaggc gttactttgc ctaaggttaa 1560
tcaagttgaa gttctaatta cccctcgttt accttctaag ctttatttat tttgaaagtg 1620
caaatcatca atcatgggtt gagtaattag tgatact 1657

<210> 270
<211> 521
<212> PRT
<213> Nicotiana tabacum

<400> 270

Met Asp Tyr His Ile Ser Phe His Phe Gln Ala Leu Leu Gly Leu Leu
1               5                   10                  15

Ala Phe Val Phe Leu Ser Ile Ile Leu Trp Arg Arg Thr Leu Thr Ser
            20                  25                  30

Arg Lys Leu Ala Pro Glu Ile Pro Gly Ala Trp Pro Ile Ile Gly His
        35                  40                  45

Leu Arg Gln Leu Ser Gly Thr Asp Lys Asn Ile Pro Phe Pro Arg Ile
    50                  55                  60

Leu Gly Ala Leu Ala Asp Lys Tyr Gly Pro Val Phe Thr Leu Arg Ile
65                  70                  75                  80

Gly Met Tyr Pro Tyr Leu Ile Val Asn Asn Trp Glu Ala Ala Lys Asp
                85                  90                  95

Cys Leu Thr Thr His Asp Lys Asp Phe Ala Ala Arg Pro Thr Ser Met
            100                 105                 110

Ala Gly Glu Ser Ile Gly Tyr Lys Tyr Ala Arg Phe Thr Tyr Ala Asn
        115                 120                 125

Phe Gly Pro Tyr Tyr Asn Gln Val Arg Lys Leu Ala Leu Gln His Val
    130                 135                 140

Leu Ser Ser Thr Lys Leu Glu Lys Met Lys His Ile Arg Val Ser Glu
145                 150                 155                 160

Leu Glu Thr Ser Ile Lys Glu Leu Tyr Ser Leu Thr Leu Gly Lys Asn
                165                 170                 175

Asn Met Gln Lys Val Asn Ile Ser Lys Trp Phe Glu Gln Leu Thr Leu
            180                 185                 190

Asn Ile Ile Val Lys Thr Ile Cys Gly Lys Arg Tyr Ser Asn Ile Glu
        195                 200                 205

Glu Asp Glu Glu Ala Gln Arg Phe Arg Lys Ala Phe Lys Gly Ile Met
    210                 215                 220

Phe Val Val Gly Gln Ile Val Leu Tyr Asp Ala Ile Pro Phe Pro Leu
225                 230                 235                 240

Phe Lys Tyr Phe Asp Phe Gln Gly His Ile Gln Leu Met Asn Lys Ile
                245                 250                 255

Tyr Lys Asp Leu Asp Ser Ile Leu Gln Gly Trp Leu Asp Asp His Met
            260                 265                 270

Met Asn Lys Asp Val Asn Asn Lys Asp Gln Asp Ala Ile Asp Ala Met
        275                 280                 285

Leu Lys Val Thr Gln Leu Asn Glu Phe Lys Ala Tyr Gly Phe Ser Gln
    290                 295                 300

Ala Thr Val Ile Lys Ser Thr Val Leu Ser Leu Ile Leu Asp Gly Asn
305                 310                 315                 320

Asp Thr Thr Ala Val His Leu Ile Trp Val Met Ser Leu Leu Leu Asn
                325                 330                 335

Asn Pro His Val Met Lys Gln Gly Gln Glu Glu Ile Asp Met Lys Val
            340                 345                 350

Gly Lys Glu Arg Trp Ile Glu Asp Thr Asp Ile Lys Asn Leu Val Tyr
        355                 360                 365

Leu Gln Ala Ile Val Lys Glu Thr Leu Arg Leu Tyr Pro Pro Val Pro
    370                 375                 380

Phe Leu Leu Pro His Glu Ala Val Gln Asp Cys Lys Val Thr Gly Tyr
385                 390                 395                 400

His Ile Pro Lys Gly Thr Arg Leu Tyr Ile Asn Ala Trp Lys Val His
                405                 410                 415

Arg Asp Ser Glu Ile Trp Ser Glu Pro Glu Lys Phe Met Pro Asn Arg
            420                 425                 430

Phe Leu Thr Ser Lys Ala Asn Ile Asp Ala Arg Gly Gln Asn Phe Glu
        435                 440                 445

Phe Ile Pro Phe Gly Ser Gly Arg Arg Ser Cys Pro Gly Leu Gly Phe
    450                 455                 460

Ala Thr Leu Val Thr His Leu Thr Phe Gly Arg Leu Leu Gln Gly Phe
465                 470                 475                 480

Asp Phe Ser Lys Pro Ser Asn Thr Pro Ile Asp Met Thr Glu Gly Val
                485                 490                 495

Gly Val Thr Leu Pro Lys Val Asn Gln Val Glu Val Leu Ile Thr Pro
            500                 505                 510

Arg Leu Pro Ser Lys Leu Tyr Leu Phe
        515                 520

<210> 271
<211> 1620
<212> DNA
<213> Nicotiana tabacum

<400> 271
agagagtgaa aatggacgca ctacttcaaa tgacagtaac agcatcttgt gctgccatag 60

taattactct gctggtgtgt atatggagag tgctgaactg gatttggttc agaccaaaga 120

aattggagtt gttgttgaga aaacaaggtt tggaaggaaa ttcttacaag gttttgtatg 180

gggacatgaa agagttttct gggatgatta aggaagcata ctcaaagcct atgagtctat 240

ctgatgatgt agcaccaaga ctgatgcctt tctttcttga aaccatcaaa aaatatggaa 300

aaagatcctt tatatggttt ggtccaagac cactagtatt gattatggat cctgagctta 360

taaaggaagt actctcaaaa atccatctgt atcaaaagcc tggtggaaat ccattagcaa 420

cactattggt acaaggaata gcaacctatg aggaagacaa atgggccaaa catagaaaaa 480

tcatcaatcc cgctttccat ctagagaagc taaagcttat gcttccagca tttcgcttaa 540

gctgtagtga gatgctgagc aaatgggaag acattgtttc agctgatagc tcacatgaga 600

tagatgtatg gtctcacctt gagcaattga cttgcgatgt gatctctcgg acagcttttg 660

gcagtagtta tgaagaaggt agaaagattt ttgaacttca aaaggaacaa gctcagtatc 720

ttgtggaagt tttccgctcc gtttatatcc caggaaggag atttttgcca acaaagagga 780

atagaagaat gaaggaaata aaaaaggatg tccgggcatc aattaaaggt attattgata 840

aaagattgaa ggcaatgaaa gcaggggaca ccaataatga ggatctattg ggtatattac 900

tggaatcgaa tattaaagaa attgaacagc acggaaacaa ggattttgga atgagcattg 960

aagaagtcat tgaagaatgc aagttattct attttgctgg ccaagaaact acatcagtgt 1020
tactcctatg gtctctagtg ttgttgagca ggtatcaaga ttggcaggca cgggccagag 1080
aagaaatctt gcaagtcttt ggcagtcgaa aaccagattt tgacggatta aatcatctaa 1140
aaattgtgac aatgatcttg tacgagtctt taaggctgta tccctcacta ataacactta 1200
cccgccggtg taatgaagac attgtattag gagaactatc tctaccagct ggtgttctag 1260
tctctttgcc attgattttg ttgcatcatg atgaagagat atggggtgaa gatgcaaagg 1320
agttcaaacc agagagattt agagaaggaa tatcaagtgc aacaaagggt caactcacat 1380
attttccatt tagctggggt cctagaatat gtattggaca aaattttgcc atgttagaag 1440
caaagatggc tctgtctatg atcctgcaac gcttctcttt tgaactgtct ccgtcttatg 1500
cacatgcccc tcggtccata ataaccgttc agcctcagta tggtgctcca cttattttcc 1560
acaaactata attttggtac ttctactaat attttagggt ttattcagac tcaaaaaaaa 1620

<210> 272
<211> 513
<212> PRT
<213> Nicotiana tabacum

<400> 272

Met Thr Val Thr Ala Ser Cys Ala Ala Ile Val Ile Thr Leu Leu Val
1               5                   10                  15

Cys Ile Trp Arg Val Leu Asn Trp Ile Trp Phe Arg Pro Lys Lys Leu
            20                  25                  30

Glu Leu Leu Leu Arg Lys Gln Gly Leu Glu Gly Asn Ser Tyr Lys Val
        35                  40                  45

Leu Tyr Gly Asp Met Lys Glu Phe Ser Gly Met Ile Lys Glu Ala Tyr
    50                  55                  60

Ser Lys Pro Met Ser Leu Ser Asp Asp Val Ala Pro Arg Leu Met Pro
65                  70                  75                  80

Phe Phe Leu Glu Thr Ile Lys Lys Tyr Gly Lys Arg Ser Phe Ile Trp
                85                  90                  95

Phe Gly Pro Arg Pro Leu Val Leu Ile Met Asp Pro Glu Leu Ile Lys
            100                 105                 110

Glu Val Leu Ser Lys Ile His Leu Tyr Gln Lys Pro Gly Gly Asn Pro
        115                 120                 125

Leu Ala Thr Leu Leu Val Gln Gly Ile Ala Thr Tyr Glu Glu Asp Lys
    130                 135                 140

Trp Ala Lys His Arg Lys Ile Ile Asn Pro Ala Phe His Leu Glu Lys
145                 150                 155                 160

Leu Lys Leu Met Leu Pro Ala Phe Arg Leu Ser Cys Ser Glu Met Leu
                165                 170                 175

Ser Lys Trp Glu Asp Ile Val Ser Ala Asp Ser Ser His Glu Ile Asp
            180                 185                 190

Val Trp Ser His Leu Glu Gln Leu Thr Cys Asp Val Ile Ser Arg Thr
        195                 200                 205

Ala Phe Gly Ser Ser Tyr Glu Glu Gly Arg Lys Ile Phe Glu Leu Gln
    210                 215                 220

Lys Glu Gln Ala Gln Tyr Leu Val Glu Val Phe Arg Ser Val Tyr Ile
225                 230                 235                 240

Pro Gly Arg Arg Phe Leu Pro Thr Lys Arg Asn Arg Arg Met Lys Glu
                245                 250                 255

Ile Lys Lys Asp Val Arg Ala Ser Ile Lys Gly Ile Ile Asp Lys Arg
            260                 265                 270

Leu Lys Ala Met Lys Ala Gly Asp Thr Asn Asn Glu Asp Leu Leu Gly
        275                 280                 285

Ile Leu Leu Glu Ser Asn Ile Lys Glu Ile Glu Gln His Gly Asn Lys
    290                 295                 300

Asp Phe Gly Met Ser Ile Glu Glu Val Ile Glu Glu Cys Lys Leu Phe
305                 310                 315                 320

Tyr Phe Ala Gly Gln Glu Thr Thr Ser Val Leu Leu Leu Trp Ser Leu
                325                 330                 335

Val Leu Leu Ser Arg Tyr Gln Asp Trp Gln Ala Arg Ala Arg Glu Glu
            340                 345                 350

Ile Leu Gln Val Phe Gly Ser Arg Lys Pro Asp Phe Asp Gly Leu Asn
        355                 360                 365

His Leu Lys Ile Val Thr Met Ile Leu Tyr Glu Ser Leu Arg Leu Tyr
    370                 375                 380

Pro Ser Leu Ile Thr Leu Thr Arg Arg Cys Asn Glu Asp Ile Val Leu
385                 390                 395                 400

Gly Glu Leu Ser Leu Pro Ala Gly Val Leu Val Ser Leu Pro Leu Ile
                405                 410                 415

Leu Leu His His Asp Glu Glu Ile Trp Gly Glu Asp Ala Lys Glu Phe
            420                 425                 430

Lys Pro Glu Arg Phe Arg Glu Gly Ile Ser Ser Ala Thr Lys Gly Gln
        435                 440                 445

Leu Thr Tyr Phe Pro Phe Ser Trp Gly Pro Arg Ile Cys Ile Gly Gln
    450                 455                 460

Asn Phe Ala Met Leu Glu Ala Lys Met Ala Leu Ser Met Ile Leu Gln
465                 470                 475                 480

Arg Phe Ser Phe Glu Leu Ser Pro Ser Tyr Ala His Ala Pro Arg Ser
                485                 490                 495

Ile Ile Thr Val Gln Pro Gln Tyr Gly Ala Pro Leu Ile Phe His Lys
            500                 505                 510

Leu

<210>  273
<211>  1671
<212>  DNA
<213>  Nicotiana tabacum

<400>  273
tgcaatatag ttttcctagt cagttctagc ctccttttcc ttagaaataa tggattatca      60

tatttctttc cattttcaag ctcttttagg gcttttagcc tttgtgttct tgtctattat     120

cttatggaga agaacactca cttcaagaaa attagcccct gaaatcccag gggcatggcc     180

tattataggc catcttcgtc agctgagtgg tactgataag aatatcccat ttccccgaat     240

attgggcgct ttggcagata aatatggacc tgtcttcaca ctgagaatag ggatgtaccc     300

ctatttgatt gtcaacaatt gggaagcagc taaggattgt ctcacaacgc atgataagga     360

cttggctgcc cgaccaactt ctatggctgg tgaaagcatc gggtacaagt atgcgaggtt     420

tacttatgct aattttggtc cttattataa ccaagtgcgc aaactagccc tacaacatgt     480

actctcgagt actaaactcg agaaaatgaa acacatacgt gtttctgaat tggaaactag     540

catcaaagaa ttatattctt tgacgctggg caaaaacaac atgcaaaaag tgaatataag     600

taaatggttt gaacaattga ctttaaacat aatcgtgaag acaatttgtg gcaagagata     660

tagcaacata gaggaggatg aagaggcaca acgtttcaga aaggcattta agggcatcat     720

gtttgttgta gggcaaattg ttttatatga cgcaattcca ttcccattgt tcaaatactt     780

tgatttccaa ggtcatatac aattgatgaa caaaatttat aaagacttag attctattct     840

tcaaggatgg ttggatgatc atatgatgaa caaggatgta aacaataagg atcaagatgc     900

catagatgcc atgcttaagg taacacaact taatgaattc aaagcctatg gtttttctca      960

ggccactgtg atcaagtcga cagtcttgag tttgatctta gatggaaatg acacaaccgc    1020

tgttcatttg atatgggtaa tgtccttatt actgaacaat ccacatgtta tgaaacaagg    1080

ccaagaagag atagacatga aagtgggtaa agagaggtgg attgaagata ctgacataaa 1140

aaatttagtg taccttcagg ctatcgttaa agagacattg cgcttgtatc cacctgttcc 1200

ttttctttta ccacacgaag cagtgcaaga ttgtaaagtg actggttacc acattcctaa 1260

aggtactcgt ctatatatca atgcgtggaa agtacatcgc gatcctgaaa tttggtcaga 1320

gcccgaaaag tttatgccca atagattctt gactagcaaa gcaaatatag atgctcgcgg 1380

tcaaaatttt gaatttatac cgtttggttc tgggagacgg tcatgtccag ggataggttt 1440

tgcgacttta gtgacacatc tgacttttgg tcgcttgctt caaggttttg attttagtaa 1500

gccatcaaac acgccaattg acatgacaga aggcgtaggc gttactttgc ctaaggttaa 1560

tcaagttgaa gttctaatta cccctcgttt accttctaag ctttatttat tttgaaggtg 1620

caaatcatca atcatggctt gagtaattag ttatacttta atatgtttct c 1671

<210> 274
<211> 521
<212> PRT
<213> Nicotiana tabacum

<400> 274

Met Asp Tyr His Ile Ser Phe His Phe Gln Ala Leu Leu Gly Leu Leu
1               5                   10                  15

Ala Phe Val Phe Leu Ser Ile Ile Leu Trp Arg Arg Thr Leu Thr Ser
            20                  25                  30

Arg Lys Leu Ala Pro Glu Ile Pro Gly Ala Trp Pro Ile Ile Gly His
        35                  40                  45

Leu Arg Gln Leu Ser Gly Thr Asp Lys Asn Ile Pro Phe Pro Arg Ile
    50                  55                  60

Leu Gly Ala Leu Ala Asp Lys Tyr Gly Pro Val Phe Thr Leu Arg Ile
65                  70                  75                  80

Gly Met Tyr Pro Tyr Leu Ile Val Asn Asn Trp Glu Ala Ala Lys Asp
                85                  90                  95

Cys Leu Thr Thr His Asp Lys Asp Leu Ala Ala Arg Pro Thr Ser Met
            100                 105                 110

Ala Gly Glu Ser Ile Gly Tyr Lys Tyr Ala Arg Phe Thr Tyr Ala Asn
        115                 120                 125

Phe Gly Pro Tyr Tyr Asn Gln Val Arg Lys Leu Ala Leu Gln His Val
    130                 135                 140

Leu Ser Ser Thr Lys Leu Glu Lys Met Lys His Ile Arg Val Ser Glu
145                 150                 155                 160

Leu Glu Thr Ser Ile Lys Glu Leu Tyr Ser Leu Thr Leu Gly Lys Asn
                165                 170                 175

Asn Met Gln Lys Val Asn Ile Ser Lys Trp Phe Glu Gln Leu Thr Leu
            180                 185                 190

Asn Ile Ile Val Lys Thr Ile Cys Gly Lys Arg Tyr Ser Asn Ile Glu
        195                 200                 205

Glu Asp Glu Glu Ala Gln Arg Phe Arg Lys Ala Phe Lys Gly Ile Met
    210                 215                 220

Phe Val Val Gly Gln Ile Val Leu Tyr Asp Ala Ile Pro Phe Pro Leu
225                 230                 235                 240

Phe Lys Tyr Phe Asp Phe Gln Gly His Ile Gln Leu Met Asn Lys Ile
                245                 250                 255

Tyr Lys Asp Leu Asp Ser Ile Leu Gln Gly Trp Leu Asp Asp His Met
            260                 265                 270

Met Asn Lys Asp Val Asn Asn Lys Asp Gln Asp Ala Ile Asp Ala Met
        275                 280                 285

Leu Lys Val Thr Gln Leu Asn Glu Phe Lys Ala Tyr Gly Phe Ser Gln
    290                 295                 300

Ala Thr Val Ile Lys Ser Thr Val Leu Ser Leu Ile Leu Asp Gly Asn
305                 310                 315                 320

Asp Thr Thr Ala Val His Leu Ile Trp Val Met Ser Leu Leu Leu Asn
                325                 330                 335

Asn Pro His Val Met Lys Gln Gly Gln Glu Glu Ile Asp Met Lys Val
            340                 345                 350

Gly Lys Glu Arg Trp Ile Glu Asp Thr Asp Ile Lys Asn Leu Val Tyr
        355                 360                 365

Leu Gln Ala Ile Val Lys Glu Thr Leu Arg Leu Tyr Pro Pro Val Pro
    370                 375                 380

Phe Leu Leu Pro His Glu Ala Val Gln Asp Cys Lys Val Thr Gly Tyr
385                 390                 395                 400

His Ile Pro Lys Gly Thr Arg Leu Tyr Ile Asn Ala Trp Lys Val His
                405                 410                 415

Arg Asp Pro Glu Ile Trp Ser Glu Pro Glu Lys Phe Met Pro Asn Arg
            420                 425                 430

Phe Leu Thr Ser Lys Ala Asn Ile Asp Ala Arg Gly Gln Asn Phe Glu
        435                 440                 445

Phe Ile Pro Phe Gly Ser Gly Arg Arg Ser Cys Pro Gly Ile Gly Phe
    450                 455                 460

Ala Thr Leu Val Thr His Leu Thr Phe Gly Arg Leu Leu Gln Gly Phe
465                 470                 475                 480

Asp Phe Ser Lys Pro Ser Asn Thr Pro Ile Asp Met Thr Glu Gly Val
                485                 490                 495

Gly Val Thr Leu Pro Lys Val Asn Gln Val Glu Val Leu Ile Thr Pro
            500                 505                 510

Arg Leu Pro Ser Lys Leu Tyr Leu Phe
        515                 520

<210>  275
<211>  1618
<212>  DNA
<213>  Nicotiana tabacum

<400>  275
tttgattcaa ccatggagaa ccaatactcc tactcattct cttcctactt ctacttagct      60
atagtactgt ttcttcttcc aattttggtc aaatatttct tccatcggag aagaaattta     120
cctccaagtc cattttctct tccaataatt ggtcaccttt accttctcaa gaaaactctc     180
catctcactc taacatcctt atcagctaaa tatggtcctg ttttatacct caaattgggc     240
tctatgcctg tgattgttgt gtcctcacca tctgctgttg aagaatgttt aaccaagaat     300
gatatcatat tcgcaaatag gcccaagacc gtggctggtg acaagtttac ctacaattat     360
actgtttatg tttgggcacc ctatggccaa ctttggagaa ttcttcgccg attaactgtc     420
gttgaactct tctcttcaca tagcctacag aaaacttcta tccttagaga tcaagaagtt     480
gcaatattta tccgttcgtt atacaaattc tcaaaggata gtagcaaaaa agtcgatttg     540
accaactggt cttttacttt ggttttcaat cttatgacca aaattattgc tgggagacat     600
attgtgaagg aggaagatgc tggcaaggaa aagggcattg aaattattga aaaacttaga     660
gggactttct tagtaactac atcattcttg aatatgtgtg atttcttgcc agtattcagg     720
tgggttggtt acaaagggca ggagaagaag atggcctcaa ttcacaatag aagaaatgaa     780
ttcttgaaca gcttgcttga tgaatttcga cacaagaaaa gtagtgcttc acaatctaac     840
acaactgttg gaaacatgga gaagaaaacc acactgattg aaaagctctt gtctcttcaa     900
gaatcagagc ctgaattcta cactgatgat atcatcaaaa gtattatgct ggtagttttt     960
gttgcaggaa cagagacctc atcaacaacc atccaatggg taatgaggct tcttgtagct    1020
caccctgagg cattgtataa gctacgagct gacattgaca gtaaagttgg gaataagcgc    1080
ttgctgaatg aatcagacct caacaagctt ccgtatttgc attgtgttgt taatgagaca    1140

atgagattat acactccgat accactttta ttgcctcatt attcaactaa agattgtatt 1200
gtggaaggat atgatgtacc aaaacataca atgttgtttg tcaacgcttg ggccattcac 1260
agggatccca aggtatggga ggagcctgac aagttcaagc cagagagatt tgaggcaaca 1320
gaaggggaaa cagaaaggtt caattacaag cttgtaccat ttggaatggg gagaagagcg 1380
tgccctggag ctgatatggg gttgcgagca gtttctttgg cattaggtgc acttattcaa 1440
tgctttgact ggcaaattga ggaagcggaa agcttggagg aaagctataa ttctagaatg 1500
actatgcaga acaagccttt gaaggttgtc tgcactccac gcgaagatct tggccagctt 1560
ctatcccaac tctaaggcaa tttatcaatg ccaaacgtaa tcttcatcta ccactatg 1618

<210> 276
<211> 520
<212> PRT
<213> Nicotiana tabacum

<400> 276

Met Glu Asn Gln Tyr Ser Tyr Ser Phe Ser Ser Tyr Phe Tyr Leu Ala
1               5                   10                  15

Ile Val Leu Phe Leu Leu Pro Ile Leu Val Lys Tyr Phe Phe His Arg
            20                  25                  30

Arg Arg Asn Leu Pro Pro Ser Pro Phe Ser Leu Pro Ile Ile Gly His
        35                  40                  45

Leu Tyr Leu Leu Lys Lys Thr Leu His Leu Thr Leu Thr Ser Leu Ser
    50                  55                  60

Ala Lys Tyr Gly Pro Val Leu Tyr Leu Lys Leu Gly Ser Met Pro Val
65                  70                  75                  80

Ile Val Val Ser Ser Pro Ser Ala Val Glu Glu Cys Leu Thr Lys Asn
                85                  90                  95

Asp Ile Ile Phe Ala Asn Arg Pro Lys Thr Val Ala Gly Asp Lys Phe
            100                 105                 110

Thr Tyr Asn Tyr Thr Val Tyr Val Trp Ala Pro Tyr Gly Gln Leu Trp
        115                 120                 125

Arg Ile Leu Arg Arg Leu Thr Val Val Glu Leu Phe Ser Ser His Ser
    130                 135                 140

Leu Gln Lys Thr Ser Ile Leu Arg Asp Gln Glu Val Ala Ile Phe Ile
145                 150                 155                 160

Arg Ser Leu Tyr Lys Phe Ser Lys Asp Ser Ser Lys Lys Val Asp Leu
                165                 170                 175

Thr Asn Trp Ser Phe Thr Leu Val Phe Asn Leu Met Thr Lys Ile Ile
            180                 185                 190

Ala Gly Arg His Ile Val Lys Glu Glu Asp Ala Gly Lys Glu Lys Gly
        195                 200                 205

Ile Glu Ile Ile Glu Lys Leu Arg Gly Thr Phe Leu Val Thr Thr Ser
    210                 215                 220

Phe Leu Asn Met Cys Asp Phe Leu Pro Val Phe Arg Trp Val Gly Tyr
225                 230                 235                 240

Lys Gly Gln Glu Lys Lys Met Ala Ser Ile His Asn Arg Arg Asn Glu
                245                 250                 255

Phe Leu Asn Ser Leu Leu Asp Glu Phe Arg His Lys Lys Ser Ser Ala
            260                 265                 270

Ser Gln Ser Asn Thr Thr Val Gly Asn Met Glu Lys Lys Thr Thr Leu
        275                 280                 285

Ile Glu Lys Leu Leu Ser Leu Gln Glu Ser Glu Pro Glu Phe Tyr Thr
    290                 295                 300

Asp Asp Ile Ile Lys Ser Ile Met Leu Val Val Phe Val Ala Gly Thr
305                 310                 315                 320

Glu Thr Ser Ser Thr Thr Ile Gln Trp Val Met Arg Leu Leu Val Ala
                325                 330                 335

His Pro Glu Ala Leu Tyr Lys Leu Arg Ala Asp Ile Asp Ser Lys Val
            340                 345                 350

Gly Asn Lys Arg Leu Leu Asn Glu Ser Asp Leu Asn Lys Leu Pro Tyr
        355                 360                 365

Leu His Cys Val Val Asn Glu Thr Met Arg Leu Tyr Thr Pro Ile Pro
    370                 375                 380

Leu Leu Leu Pro His Tyr Ser Thr Lys Asp Cys Ile Val Glu Gly Tyr
385                 390                 395                 400

Asp Val Pro Lys His Thr Met Leu Phe Val Asn Ala Trp Ala Ile His
                405                 410                 415

Arg Asp Pro Lys Val Trp Glu Glu Pro Asp Lys Phe Lys Pro Glu Arg
            420                 425                 430

Phe Glu Ala Thr Glu Gly Glu Thr Glu Arg Phe Asn Tyr Lys Leu Val
        435                 440                 445

Pro Phe Gly Met Gly Arg Arg Ala Cys Pro Gly Ala Asp Met Gly Leu
    450                 455                 460

Arg Ala Val Ser Leu Ala Leu Gly Ala Leu Ile Gln Cys Phe Asp Trp
465                 470                 475                 480

Gln Ile Glu Glu Ala Glu Ser Leu Glu Glu Ser Tyr Asn Ser Arg Met
                485                 490                 495

Thr Met Gln Asn Lys Pro Leu Lys Val Val Cys Thr Pro Arg Glu Asp
            500                 505                 510

Leu Gly Gln Leu Leu Ser Gln Leu
        515                 520

<210>  277
<211>  1676
<212>  DNA
<213>  Nicotiana tabacum

<400>  277
cctttttaag atgtatttaa gatttaagat ttaagatgaa gcaactgagg taagtccttt      60
caaggagtag ttgtcacttc tgagaatgga gatgatgtac agcataatag cagcagccag     120
tattgcaatt atcttggtat atacatggaa agtgttgaat tgggcttggt ttgggccgaa     180
gaaaatggag aaatgcttaa gacagagggg tctcaaggga aatccttata agctactcta     240
tggagatcta aacgaactga caaaaagcat aatagaagcc aagtctaagc ccatcaattt     300
ctctgatgat attgctcaaa ggctcatccc tttttttctt gacgccatca acaaaaatgg     360
taaaaactcc ttcgtctggc ttggaccgta tccaatagtg ttgatcacgg atcctgagca     420
tttaaaggag attttcacaa agaattatgt gtatcaaaag caaactcatc ccaatccata     480
cgccaagcta ttagctcacg gtcttgtcag ccttgaggaa gacaaatggg ccaaacacag     540
aaaaatcatt agtcctgcct tccatgtcga gaagctaaag catatgctgc ctgcatttta     600
tctgagttgt agtgaaatga taagcaaatg ggaggaggtt gttccaaaag aaacatcatt     660
cgagctcgat gtatggccag accttcaaat aatgaccagt gaagtcattt ctcgcactgc     720
atttgggagt agctatgaag aaggaagaat agtatttgaa cttcagaaag aacaagctga     780
gtatgtaatg gacataggac gttcaattta tataccagga tcaaggttct tgcctactaa     840
aaggaacaaa agaatgctgg aaattgaaaa gcaagtgcaa acaacaatta ggcgtatcat     900
cgacaaaaga ttgaaggcaa tggaagaagg ggagactagt aaagatgact tattaggcat     960
attacttgaa tccaatttga aagaaattga acttcatgga agaaatgact tgggaataac    1020
aacgtcagaa gtgattgaag agtgcaagtt attctatttt gccggccaag agaccacttc    1080
agtgttgctt gtttggacaa tgattttgtt gtgcttacat ccagagtggc aagtacgtgc    1140
cagaaaggaa gtgttgcaga tctttggaaa tgataaacca gatttggaag gactaagtcg    1200
cttgaaaatt gtaacaatga tcttgtacga gacgttacgc ctattccccc cattaccagc    1260

atttggtaga aggaacaaag aagaagtcaa attaggggag ctacatctac cggctggagt 1320
gttactcgtt ataccagcaa tcttagtaca ttatgataag gaaatatggg gtgaagatgc 1380
aaaggaattc aaaccagaaa gattcagtga aggagtgtca aaggcaacaa atggacaagt 1440
ctcatttata ccatttagct ggggacctcg tgtttgcatt ggacaaaact tcgcaatgat 1500
ggaagcaaaa atggcagtaa ctatgatact acaaaaattc tcctttgaac tatccccttc 1560
ttatacacat gctccatttg caattgtgac tattcatccc cagtatggtg ctcctctgct 1620
tatgcgcaga ctttaaaaca tatgttgctg atatttaaga tcagtggcgt tttatt 1676

<210> 278
<211> 516
<212> PRT
<213> Nicotiana tabacum

<400> 278

Met Glu Met Met Tyr Ser Ile Ile Ala Ala Ala Ser Ile Ala Ile Ile
1               5                   10                  15

Leu Val Tyr Thr Trp Lys Val Leu Asn Trp Ala Trp Phe Gly Pro Lys
            20                  25                  30

Lys Met Glu Lys Cys Leu Arg Gln Arg Gly Leu Lys Gly Asn Pro Tyr
        35                  40                  45

Lys Leu Leu Tyr Gly Asp Leu Asn Glu Leu Thr Lys Ser Ile Ile Glu
    50                  55                  60

Ala Lys Ser Lys Pro Ile Asn Phe Ser Asp Asp Ile Ala Gln Arg Leu
65                  70                  75                  80

Ile Pro Phe Phe Leu Asp Ala Ile Asn Lys Asn Gly Lys Asn Ser Phe
                85                  90                  95

Val Trp Leu Gly Pro Tyr Pro Ile Val Leu Ile Thr Asp Pro Glu His
            100                 105                 110

Leu Lys Glu Ile Phe Thr Lys Asn Tyr Val Tyr Gln Lys Gln Thr His
        115                 120                 125

Pro Asn Pro Tyr Ala Lys Leu Leu Ala His Gly Leu Val Ser Leu Glu
    130                 135                 140

Glu Asp Lys Trp Ala Lys His Arg Lys Ile Ile Ser Pro Ala Phe His
145                 150                 155                 160

Val Glu Lys Leu Lys His Met Leu Pro Ala Phe Tyr Leu Ser Cys Ser
                165                 170                 175

Glu Met Ile Ser Lys Trp Glu Glu Val Val Pro Lys Glu Thr Ser Phe
            180                 185                 190

Glu Leu Asp Val Trp Pro Asp Leu Gln Ile Met Thr Ser Glu Val Ile
        195                 200                 205

Ser Arg Thr Ala Phe Gly Ser Ser Tyr Glu Glu Gly Arg Ile Val Phe
    210                 215                 220

Glu Leu Gln Lys Glu Gln Ala Glu Tyr Val Met Asp Ile Gly Arg Ser
225                 230                 235                 240

Ile Tyr Ile Pro Gly Ser Arg Phe Leu Pro Thr Lys Arg Asn Lys Arg
                245                 250                 255

Met Leu Glu Ile Glu Lys Gln Val Gln Thr Thr Ile Arg Arg Ile Ile
            260                 265                 270

Asp Lys Arg Leu Lys Ala Met Glu Glu Gly Glu Thr Ser Lys Asp Asp
        275                 280                 285

Leu Leu Gly Ile Leu Leu Glu Ser Asn Leu Lys Glu Ile Glu Leu His
    290                 295                 300

Gly Arg Asn Asp Leu Gly Ile Thr Thr Ser Glu Val Ile Glu Glu Cys
305                 310                 315                 320

Lys Leu Phe Tyr Phe Ala Gly Gln Glu Thr Thr Ser Val Leu Leu Val
                325                 330                 335

Trp Thr Met Ile Leu Leu Cys Leu His Pro Glu Trp Gln Val Arg Ala
            340                 345                 350

Arg Lys Glu Val Leu Gln Ile Phe Gly Asn Asp Lys Pro Asp Leu Glu
        355                 360                 365

Gly Leu Ser Arg Leu Lys Ile Val Thr Met Ile Leu Tyr Glu Thr Leu
    370                 375                 380

Arg Leu Phe Pro Pro Leu Pro Ala Phe Gly Arg Arg Asn Lys Glu Glu
385                 390                 395                 400

Val Lys Leu Gly Glu Leu His Leu Pro Ala Gly Val Leu Leu Val Ile
                405                 410                 415

Pro Ala Ile Leu Val His Tyr Asp Lys Glu Ile Trp Gly Glu Asp Ala
            420                 425                 430

Lys Glu Phe Lys Pro Glu Arg Phe Ser Glu Gly Val Ser Lys Ala Thr
        435                 440                 445

Asn Gly Gln Val Ser Phe Ile Pro Phe Ser Trp Gly Pro Arg Val Cys
    450                 455                 460

Ile Gly Gln Asn Phe Ala Met Met Glu Ala Lys Met Ala Val Thr Met
465                 470                 475                 480

Ile Leu Gln Lys Phe Ser Phe Glu Leu Ser Pro Ser Tyr Thr His Ala
                485                 490                 495

Pro Phe Ala Ile Val Thr Ile His Pro Gln Tyr Gly Ala Pro Leu Leu
            500                 505                 510

Met Arg Arg Leu
        515

<210>  279
<211>  1577
<212>  DNA
<213>  Nicotiana tabacum

<400>  279
ctaaatttca tataccttta gtactcttga aattttcaaa taatggttta tcttctttct      60

cccatagaag ccattgtagg atttgtaacc ttttcatttc tattctactt tctatggacc     120

aaaaaacaat caaaaatctt aaacccacta cctccaaaaa tcccaggtgg atggccagta     180

atcggccatc tcttttattt caagaacaat ggcgatgaag atcgccattt ttctcaaaaa     240

ctcggtgact tagctgacaa atatggtccc gtcttcactt tccggttagg gtttcgccgt     300

ttcttggcgg tgagtagtta tgaagctatg aaagaatgct tcactaccaa tgatatccat     360

ttcgccgatc ggccatcttt actctacgga gaataccttt gctataataa tgccatgctt     420

gctgttgcca aatatggccc ttactggaaa aaaaatcgaa agttagtcaa tcaagaagtt     480

ctctccgtta gtcggctcga aaaattcaaa catgttagat tttctataat tcagaaaaat     540

attaaacaat tgtataattg tgattcacca atggtgaaga taaaccttag tgattggata     600

gataaattga cattcgacat cattttgaaa atggttgttg ggaagaacta taataatgga     660

catggagaaa tactcaaagt tgcttttcag aaattcatgg ttcaagctat ggagatggag     720

ctctatgatg tttttcacat tccatttttc aagtggttgg atcttacagg gaatattaag     780

gctatgaaac aaactttcaa agacattgat aatattatcc aaggttggtt agatgagcac     840

attaagaaga gagaaacaaa ggatgttgga ggtgaaaacg aacaagattt tatagatgtg     900

gtgctttcca agatgagcga cgaacatctt ggcgagggtt actctcatga cacaaccatc     960

aaagcaactg tattcacttt ggtcttggat gcaacagaca cacttgcact tcatataaag    1020

tgggtaatgg cgttaatgat aaacaataag catgtcatga agaaagcaca agaagagatg    1080

gacacaattg ttggtagaga tagatgggta gaagagagtg atatcaagaa tttggtgtat    1140

ctccaagcaa ttgttaaaga agtattacga ttacatccac ctgcaccttt gtcagtgcaa    1200

cacctatctg tggaagattg tgttgtcaat gggtaccata ttcctaaggg gactgcacta    1260

cttaccaata ttatgaaact acagcgagat cctcaaacat ggccaaatcc tgataaattc    1320

gatccagaga gattcctgac gactcatgct actattgact accgcgggca gcactatgag 1380
ttgatcccct ttggtacggg gagacgagct tgtcccgcga tgaattattc attgcaagtg 1440
gaacaccttt caattgctca tatgatccaa ggtttcagtt ttgcaactac gaccaatgag 1500
cctttggata tgaaacaagg tgtgggttta actttaccaa agaagactga tgttgaagtt 1560
ctaattaccc ctcgttt 1577

<210> 280
<211> 511
<212> PRT
<213> Nicotiana tabacum

<400> 280

Met Val Tyr Leu Leu Ser Pro Ile Glu Ala Ile Val Gly Phe Val Thr
1               5                   10                  15

Phe Ser Phe Leu Phe Tyr Phe Leu Trp Thr Lys Lys Gln Ser Lys Ile
            20                  25                  30

Leu Asn Pro Leu Pro Pro Lys Ile Pro Gly Gly Trp Pro Val Ile Gly
        35                  40                  45

His Leu Phe Tyr Phe Lys Asn Asn Gly Asp Glu Asp Arg His Phe Ser
    50                  55                  60

Gln Lys Leu Gly Asp Leu Ala Asp Lys Tyr Gly Pro Val Phe Thr Phe
65                  70                  75                  80

Arg Leu Gly Phe Arg Arg Phe Leu Ala Val Ser Ser Tyr Glu Ala Met
                85                  90                  95

Lys Glu Cys Phe Thr Thr Asn Asp Ile His Phe Ala Asp Arg Pro Ser
            100                 105                 110

Leu Leu Tyr Gly Glu Tyr Leu Cys Tyr Asn Asn Ala Met Leu Ala Val
        115                 120                 125

Ala Lys Tyr Gly Pro Tyr Trp Lys Lys Asn Arg Lys Leu Val Asn Gln
    130                 135                 140

Glu Val Leu Ser Val Ser Arg Leu Glu Lys Phe Lys His Val Arg Phe
145                 150                 155                 160

Ser Ile Ile Gln Lys Asn Ile Lys Gln Leu Tyr Asn Cys Asp Ser Pro
                165                 170                 175

Met Val Lys Ile Asn Leu Ser Asp Trp Ile Asp Lys Leu Thr Phe Asp
            180                 185                 190

Ile Ile Leu Lys Met Val Val Gly Lys Asn Tyr Asn Asn Gly His Gly
        195                 200                 205

Glu Ile Leu Lys Val Ala Phe Gln Lys Phe Met Val Gln Ala Met Glu
    210                 215                 220

Met Glu Leu Tyr Asp Val Phe His Ile Pro Phe Phe Lys Trp Leu Asp
225                 230                 235                 240

Leu Thr Gly Asn Ile Lys Ala Met Lys Gln Thr Phe Lys Asp Ile Asp
                245                 250                 255

Asn Ile Ile Gln Gly Trp Leu Asp Glu His Ile Lys Lys Arg Glu Thr
            260                 265                 270

Lys Asp Val Gly Gly Glu Asn Glu Gln Asp Phe Ile Asp Val Val Leu
        275                 280                 285

Ser Lys Met Ser Asp Glu His Leu Gly Glu Gly Tyr Ser His Asp Thr
    290                 295                 300

Thr Ile Lys Ala Thr Val Phe Thr Leu Val Leu Asp Ala Thr Asp Thr
305                 310                 315                 320

Leu Ala Leu His Ile Lys Trp Val Met Ala Leu Met Ile Asn Asn Lys
                325                 330                 335

His Val Met Lys Lys Ala Gln Glu Glu Met Asp Thr Ile Val Gly Arg
            340                 345                 350

Asp Arg Trp Val Glu Glu Ser Asp Ile Lys Asn Leu Val Tyr Leu Gln
        355                 360                 365

Ala Ile Val Lys Glu Val Leu Arg Leu His Pro Pro Ala Pro Leu Ser
    370                 375                 380

Val Gln His Leu Ser Val Glu Asp Cys Val Val Asn Gly Tyr His Ile
385                 390                 395                 400

Pro Lys Gly Thr Ala Leu Leu Thr Asn Ile Met Lys Leu Gln Arg Asp
                405                 410                 415

Pro Gln Thr Trp Pro Asn Pro Asp Lys Phe Asp Pro Glu Arg Phe Leu
            420                 425                 430

Thr Thr His Ala Thr Ile Asp Tyr Arg Gly Gln His Tyr Glu Leu Ile
        435                 440                 445

Pro Phe Gly Thr Gly Arg Arg Ala Cys Pro Ala Met Asn Tyr Ser Leu
    450                 455                 460

Gln Val Glu His Leu Ser Ile Ala His Met Ile Gln Gly Phe Ser Phe
465                 470                 475                 480

Ala Thr Thr Thr Asn Glu Pro Leu Asp Met Lys Gln Gly Val Gly Leu
                485                 490                 495

Thr Leu Pro Lys Lys Thr Asp Val Glu Val Leu Ile Thr Pro Arg
            500                 505                 510

<210> 281
<211> 1434
<212> DNA
<213> Nicotiana tabacum

<220>
<221> misc_feature
<222> (564)..(564)
<223> n is a, c, g, or t

<400> 281
ctttttctcc ccaaaaaaga gctcatttcc cttgtcccca aaaatggatc ttctcttact 60

agagaagacc ttaattggtc tcttctttgc cattttaatc gctgtaattg tctctagact 120

tcgttcaaag cgttttaagc ttcccccagg accaatccca gtaccagttt ttggtaattg 180

gcttcaagtt ggtgatgatt taaaccacag aaatcttact gattttgcca aaaaatttgg 240

tgatcttttc ttgttaagaa tgggccagcg taatttagtt gttgtgtcat ctcctgaatt 300

agctaaagaa gttttacaca cacaaggtgt tgaatttggt tcaagaacaa gaaatgttgt 360

atttgatatt tttactggaa aaggtcaaga tatggttttt actgtatatg gtgaacactg 420

gagaaaaatg aggagaatta tgactgtacc attttttact aataaagttg tgcagcaata 480

tagagggggg tgggagtttg aagtggcaag tgtaattgag gatgtgaaga aaaatcctga 540

atctgctact aatgggattg tatnaaggag gagattacaa ttgatgatgt ataataatat 600

gtttaggatt atgtttgata ggagatttga gagtgaagat gatcctttgt ttgttaagct 660

taaggctttg aatggtgaaa ggagtagatt ggctcagagt tttgagtata attatggtga 720

ttttattccc attttgaggc cttttttgag aggttatttg aagatctgta aagaagttaa 780

ggagaagagg ctgcagcttt tcaaagatta ctttgttgat gaaagaaaga agctttcaaa 840

taccaagagc ttggacagca atgctctgaa atgtgcgatt gatcacattc ttgaggctca 900

acagaagggg gagatcaatg aggacaacgt tctttacatt gttgaaaaca tcaatgttgc 960

tgctatagaa accacattat ggtcaattga gtggggtatc gccgagttag tcaaccaccc 1020

tcacatccaa aagaaactcc gcgacgagat tgacacagtt cttggcccag gagtgcaagt 1080

gactgaacca gacacccaca agcttccata ccttcaggct gtgatcaagg agacgcttcg 1140

tctccgtatg gcaattcctc tattagtccc acacatgaac cttcacgatg caaagcttgg 1200

cgggtttgat attccagcag agagcaaaat cttggttaac gcttggtggc tagctaacaa 1260

cccggctcat tggaagaaac ccgaagagtt cagacccgag aggttcttcg aagaggagaa 1320

gcacgttgag gccaatggca atgacttcag atatcttccg tttggcgttg gtaggaggag 1380

ttgccctgga actatacttg cattgccaat tcttggcatt actttgggac gttt 1434

<210> 282
<211> 463
<212> PRT
<213> Nicotiana tabacum

<400> 282

Met Asp Leu Leu Leu Leu Glu Lys Thr Leu Ile Gly Leu Phe Phe Ala
1               5                   10                  15

Ile Leu Ile Ala Val Ile Val Ser Arg Leu Arg Ser Lys Arg Phe Lys
            20                  25                  30

Leu Pro Pro Gly Pro Ile Pro Val Pro Val Phe Gly Asn Trp Leu Gln
        35                  40                  45

Val Gly Asp Asp Leu Asn His Arg Asn Leu Thr Asp Phe Ala Lys Lys
    50                  55                  60

Phe Gly Asp Leu Phe Leu Leu Arg Met Gly Gln Arg Asn Leu Val Val
65                  70                  75                  80

Val Ser Ser Pro Glu Leu Ala Lys Glu Val Leu His Thr Gln Gly Val
                85                  90                  95

Glu Phe Gly Ser Arg Thr Arg Asn Val Val Phe Asp Ile Phe Thr Gly
            100                 105                 110

Lys Gly Gln Asp Met Val Phe Thr Val Tyr Gly Glu His Trp Arg Lys
        115                 120                 125

Met Arg Arg Ile Met Thr Val Pro Phe Phe Thr Asn Lys Val Val Gln
    130                 135                 140

Gln Tyr Arg Gly Gly Trp Glu Phe Glu Val Ala Ser Val Ile Glu Asp
145                 150                 155                 160

Val Lys Lys Asn Pro Glu Ser Ala Thr Asn Gly Ile Val Leu Arg Arg
                165                 170                 175

Arg Leu Gln Leu Met Met Tyr Asn Asn Met Phe Arg Ile Met Phe Asp
            180                 185                 190

Arg Arg Phe Glu Ser Glu Asp Asp Pro Leu Phe Val Lys Leu Lys Ala
        195                 200                 205

Leu Asn Gly Glu Arg Ser Arg Leu Ala Gln Ser Phe Glu Tyr Asn Tyr
    210                 215                 220

Gly Asp Phe Ile Pro Ile Leu Arg Pro Phe Leu Arg Gly Tyr Leu Lys
225                 230                 235                 240

Ile Cys Lys Glu Val Lys Glu Lys Arg Leu Gln Leu Phe Lys Asp Tyr
                245                 250                 255

Phe Val Asp Glu Arg Lys Lys Leu Ser Asn Thr Lys Ser Leu Asp Ser
            260                 265                 270

Asn Ala Leu Lys Cys Ala Ile Asp His Ile Leu Glu Ala Gln Gln Lys
        275                 280                 285

Gly Glu Ile Asn Glu Asp Asn Val Leu Tyr Ile Val Glu Asn Ile Asn
    290                 295                 300

Val Ala Ala Ile Glu Thr Thr Leu Trp Ser Ile Glu Trp Gly Ile Ala
305                 310                 315                 320

Glu Leu Val Asn His Pro His Ile Gln Lys Lys Leu Arg Asp Glu Ile
                325                 330                 335

Asp Thr Val Leu Gly Pro Gly Val Gln Val Thr Glu Pro Asp Thr His
            340                 345                 350

Lys Leu Pro Tyr Leu Gln Ala Val Ile Lys Glu Thr Leu Arg Leu Arg
        355                 360                 365

Met Ala Ile Pro Leu Leu Val Pro His Met Asn Leu His Asp Ala Lys
    370                 375                 380

Leu Gly Gly Phe Asp Ile Pro Ala Glu Ser Lys Ile Leu Val Asn Ala
385                 390                 395                 400

Trp Trp Leu Ala Asn Asn Pro Ala His Trp Lys Lys Pro Glu Glu Phe
                405                 410                 415

Arg Pro Glu Arg Phe Phe Glu Glu Glu Lys His Val Glu Ala Asn Gly
            420                 425                 430

Asn Asp Phe Arg Tyr Leu Pro Phe Gly Val Gly Arg Arg Ser Cys Pro
        435                 440                 445

Gly Thr Ile Leu Ala Leu Pro Ile Leu Gly Ile Thr Leu Gly Arg
    450                 455                 460

<210>  283
<211>  1723
<212>  DNA
<213>  Nicotiana tabacum

<400>  283
ccaaattaga gcaagaaatt aacaagtcta gttaccttct ccctttttaa gagtatttaa      60

gatttaagat ttaagatgaa gcaactgagg taagtccttt caaggagtag ttgtcacttc     120

tgagaatgga gatgatgtac agcataatag cagcagccag tattgcaatt atcttggtat 180
atacatggaa agtgttgaat tgggcttggt ttgggccaaa gaaaatggag aaatgcttaa 240
gacagagggg tctcaaggga aatccttata agctactcta tggagatcta aacgaactga 300
caaaaagcat aatagaagcc aagtctaagc ccatcaattt ctctgatgat attgctcaaa 360
ggctcatccc tttttttctt gacgccatca acaaaaatgg taaaaactcc ttcgtctggc 420
ttggaccgta tccaatagtg ttgatcacgg atcctgagca tttaaaggag attttcacaa 480
agaattatgt gtatcaaaag caaactcatc ccaatccata cgccaagcta ttagctcacg 540
gtcttgtcag ccttgaggaa gacaaatggg ccaaacacag aaaaatcatt agtcctgcct 600
tccatgtcga gaagctaaag catatgctgc ctgcatttta tctgagttgt agtgaaatga 660
taagcaaatg ggaggaggtt gttccaaaag aaacatcatt cgagctcgat gtatggccag 720
accttcaaat aatgaccagt gaagtcattt ctcgcactgc atttgggagt agctatgaag 780
aaggaagaat agtatttgaa cttcagaaag aacaagctga gtatgtaatg gacataggac 840
gttcaattta tataccagga tcaaggttct tgcctactaa aaggaacaaa agaatgctgg 900
aaattgaaaa gcaagtgcaa acaacaatta ggcgtatcat cgacaaaaga ttgaaggcaa 960
tggaagaagg ggagactagt aaagatgact tattaggcat attacttgaa tccaatttga 1020
aagaaattga acttcatgga agaaatgact tgggaataac aacatcagaa gtgattgaag 1080
agtgcaagtt aatctatttt gccggccaag agaccacttc agtgttgctt gtttggacaa 1140
tgattttgtt gtgcttacat ccagagtggc aagtacgtgc cagaaaggaa gtgttgcaga 1200
cctttggaaa tgataaacca gatttggaag gactaagtcg cttgaaaatt gtaacaatga 1260
tcttgtacga gacgttacgc ctattccccc cattaccagc atttggtaga aggaacaaag 1320
aagaagtcaa attaggggag ctacatctac cggctggagt gttactcgtt ataccagcaa 1380
tcttagtaca ttatgataag gaaatatggg gtgaagatgc aaaggaattc aaaccagaaa 1440
gattcagtga aggagtgtca aaggcaacaa atggacaagt ctcatttata ccatttagct 1500
agggacctcg tgtttgcatt ggacaaaact tcgcaatgat ggaagcaaaa atggcagtaa 1560
ctatgatact acaaaaattc tcctttgaac tatccccttc ttatacacat gctccatttg 1620
caattgtgac tattcatccc cagtatggtg ctcctctgct tatgcgcaga ctttaaaaca 1680
tatgttgctg atatttaaga tcagtggcgt tttattctcc atg 1723

<210> 284
<211> 458
<212> PRT
<213> Nicotiana tabacum

<400> 284

Met Glu Met Met Tyr Ser Ile Ile Ala Ala Ala Ser Ile Ala Ile Ile
1               5                   10                  15

Leu Val Tyr Thr Trp Lys Val Leu Asn Trp Ala Trp Phe Gly Pro Lys
            20                  25                  30

Lys Met Glu Lys Cys Leu Arg Gln Arg Gly Leu Lys Gly Asn Pro Tyr
        35                      40                      45

Lys Leu Leu Tyr Gly Asp Leu Asn Glu Leu Thr Lys Ser Ile Ile Glu
    50                      55                      60

Ala Lys Ser Lys Pro Ile Asn Phe Ser Asp Asp Ile Ala Gln Arg Leu
65                      70                      75                      80

Ile Pro Phe Phe Leu Asp Ala Ile Asn Lys Asn Gly Lys Asn Ser Phe
                85                      90                      95

Val Trp Leu Gly Pro Tyr Pro Ile Val Leu Ile Thr Asp Pro Glu His
            100                     105                     110

Leu Lys Glu Ile Phe Thr Lys Asn Tyr Val Tyr Gln Lys Gln Thr His
        115                     120                     125

Pro Asn Pro Tyr Ala Lys Leu Leu Ala His Gly Leu Val Ser Leu Glu
    130                     135                     140

Glu Asp Lys Trp Ala Lys His Arg Lys Ile Ile Ser Pro Ala Phe His
145                     150                     155                     160

Val Glu Lys Leu Lys His Met Leu Pro Ala Phe Tyr Leu Ser Cys Ser
                165                     170                     175

Glu Met Ile Ser Lys Trp Glu Glu Val Val Pro Lys Glu Thr Ser Phe
            180                     185                     190

Glu Leu Asp Val Trp Pro Asp Leu Gln Ile Met Thr Ser Glu Val Ile
        195                     200                     205

Ser Arg Thr Ala Phe Gly Ser Ser Tyr Glu Glu Gly Arg Ile Val Phe
    210                     215                     220

Glu Leu Gln Lys Glu Gln Ala Glu Tyr Val Met Asp Ile Gly Arg Ser
225                     230                     235                     240

Ile Tyr Ile Pro Gly Ser Arg Phe Leu Pro Thr Lys Arg Asn Lys Arg
                245                     250                     255

Met Leu Glu Ile Glu Lys Gln Val Gln Thr Thr Ile Arg Arg Ile Ile
            260                     265                     270

Asp Lys Arg Leu Lys Ala Met Glu Glu Gly Glu Thr Ser Lys Asp Asp
        275                     280                     285

Leu Leu Gly Ile Leu Leu Glu Ser Asn Leu Lys Glu Ile Glu Leu His
    290                     295                     300

Gly Arg Asn Asp Leu Gly Ile Thr Thr Ser Glu Val Ile Glu Glu Cys
305                 310                 315                 320

Lys Leu Ile Tyr Phe Ala Gly Gln Glu Thr Thr Ser Val Leu Leu Val
                325                 330                 335

Trp Thr Met Ile Leu Leu Cys Leu His Pro Glu Trp Gln Val Arg Ala
            340                 345                 350

Arg Lys Glu Val Leu Gln Thr Phe Gly Asn Asp Lys Pro Asp Leu Glu
        355                 360                 365

Gly Leu Ser Arg Leu Lys Ile Val Thr Met Ile Leu Tyr Glu Thr Leu
    370                 375                 380

Arg Leu Phe Pro Pro Leu Pro Ala Phe Gly Arg Arg Asn Lys Glu Glu
385                 390                 395                 400

Val Lys Leu Gly Glu Leu His Leu Pro Ala Gly Val Leu Leu Val Ile
                405                 410                 415

Pro Ala Ile Leu Val His Tyr Asp Lys Glu Ile Trp Gly Glu Asp Ala
            420                 425                 430

Lys Glu Phe Lys Pro Glu Arg Phe Ser Glu Gly Val Ser Lys Ala Thr
        435                 440                 445

Asn Gly Gln Val Ser Phe Ile Pro Phe Ser
    450                 455

<210>  285
<211>  1717
<212>  DNA
<213>  Nicotiana tabacum

<400>  285
tttgcctttg ctcgtcattg atgacgactt cattttgttt tcttccccac gaaaatggta      60
gatatgatat ggagggacgt agggaagaat tactgggaca aacctagtga gtgaaaatgg     120
aaacagttga aatgatagta aaagtatctt gtgctgccat agtaattact ctgttggtgt     180
gtctatggag agtgctgaat tgggtttggt tcagaccaaa gaaattagag aagttgttga     240
gaaaacaggt tttgtatggg gacatgaaag agttttctgg gatgattaag gaagcatact     300
caaagcctat gagtctgtct gatgatgtag caccacgaat gatgcctttc tttcttgaaa     360
ccatcaagaa atatggaaaa agatccttta tatggttcgg tccaagacca ctagtattga     420
tcatggatcc tgagcttata aaggaagtac tctccaaaat ctatctttat caaaagcccg     480
gtggaaatcc attagcaaca ctattggtac aaggattagc aacctatgag gaagacaaat     540
gggccaaaca tagaaaaatc atcaatcccg ctttccatct agagaagcta aagcatatgc     600

ttccagcttt tcgcttgagc tgtagtgaga tgctgagcaa atgggaagac attgtttcag 660
ctgaaggctc acatgagata gatgtatggc ctaaccttga gcaattgagt tgcgatgtga 720
tctctcggac agcttttggc aatagttatg aagaaggtag aaagattttt gaacttcaaa 780
aggaacaaac tcagcatctt gtggaagctt tccgctctgt ttatatccca ggaaggagat 840
ttttgccaac aaagaggaat agaagaatga aggaaataaa aaaggaggtt cgagcgtcaa 900
ttaaaggtat tattgataaa agattgaagg caatgaaagc aggggacacc aataatgagg 960
atctattggg atattgctgg aatcaaattt taaagaaatt gaacagcgcg gaaacaagga 1020
ttttggaatg agcattgaag atgtcattga agaatgcaag ttattctatt ttgctggcca 1080
agaaactaca tcagtgttgc tcctatggtc tctagtgtcg ttgagcaggt atcaagattg 1140
gcagacacgg gccagagaag aagtcttgca tgtctttggg agtcggaaac cagattttga 1200
tgaattaaat catctaaaag ttgtgacaat gatcatgtac gagtctttaa ggctatatcc 1260
ctcactaata acacttaccc gccggtgtaa tgaagacatt gtattaggag aactatctct 1320
accagctggt gtcctagtct ctttgccaat gattttgttg catcatgatg aagagatatg 1380
gggtgaagat gcaaaggagt tcaaaccaga gagatttaga gaaggattgt caagtgcaac 1440
aaagggtcaa cttacatatt ttccatttgg ctggggtcct agaatatgta ttggacaaaa 1500
ttttgccatg ttagaagcaa agatggctct gtctatgatc ctgcaacgct tctcttttga 1560
actgtctccg tcttatgcac atgcccctca gtccatatta accgttcagc ctcaatatgg 1620
tgctccactt attttccaca agctataatt tggtacttgt gaaaggtgtc ttgtacaata 1680
tgttagtaga gtttattcag acttagatac atgcttc 1717

<210> 286
<211> 304
<212> PRT
<213> Nicotiana tabacum

<400> 286

Met Glu Thr Val Glu Met Ile Val Lys Val Ser Cys Ala Ala Ile Val
1               5                   10                  15

Ile Thr Leu Leu Val Cys Leu Trp Arg Val Leu Asn Trp Val Trp Phe
            20                  25                  30

Arg Pro Lys Lys Leu Glu Lys Leu Leu Arg Lys Gln Val Leu Tyr Gly
        35                  40                  45

Asp Met Lys Glu Phe Ser Gly Met Ile Lys Glu Ala Tyr Ser Lys Pro
    50                  55                  60

Met Ser Leu Ser Asp Asp Val Ala Pro Arg Met Met Pro Phe Phe Leu
65                  70                  75                  80

Glu Thr Ile Lys Lys Tyr Gly Lys Arg Ser Phe Ile Trp Phe Gly Pro
                85                  90                  95

Arg Pro Leu Val Leu Ile Met Asp Pro Glu Leu Ile Lys Glu Val Leu
            100                 105                 110

Ser Lys Ile Tyr Leu Tyr Gln Lys Pro Gly Gly Asn Pro Leu Ala Thr
        115                 120                 125

Leu Leu Val Gln Gly Leu Ala Thr Tyr Glu Glu Asp Lys Trp Ala Lys
    130                 135                 140

His Arg Lys Ile Ile Asn Pro Ala Phe His Leu Glu Lys Leu Lys His
145                 150                 155                 160

Met Leu Pro Ala Phe Arg Leu Ser Cys Ser Glu Met Leu Ser Lys Trp
                165                 170                 175

Glu Asp Ile Val Ser Ala Glu Gly Ser His Glu Ile Asp Val Trp Pro
            180                 185                 190

Asn Leu Glu Gln Leu Ser Cys Asp Val Ile Ser Arg Thr Ala Phe Gly
        195                 200                 205

Asn Ser Tyr Glu Glu Gly Arg Lys Ile Phe Glu Leu Gln Lys Glu Gln
    210                 215                 220

Thr Gln His Leu Val Glu Ala Phe Arg Ser Val Tyr Ile Pro Gly Arg
225                 230                 235                 240

Arg Phe Leu Pro Thr Lys Arg Asn Arg Arg Met Lys Glu Ile Lys Lys
                245                 250                 255

Glu Val Arg Ala Ser Ile Lys Gly Ile Ile Asp Lys Arg Leu Lys Ala
            260                 265                 270

Met Lys Ala Gly Asp Thr Asn Asn Glu Asp Leu Leu Gly Tyr Cys Trp
        275                 280                 285

Asn Gln Ile Leu Lys Lys Leu Asn Ser Ala Glu Thr Arg Ile Leu Glu
    290                 295                 300

<210> 287
<211> 1559
<212> DNA
<213> Nicotiana tabacum

<400> 287
ccaaaatgca gttcttcaac ttcatttcct ttgtcttttt tgtgtctttc ctctttttat 60

taaggaaatg gaagaactcc aatagccaaa ccaaaagatt gcctccaggt ccatggaaat 120

tacctgtact tggaagcatg tttcatttgc taggtggacc tccacatcat gtccttggag 180

atttagccaa aaaatatggt ccacttatgc accttcaact aggtgaagtt tctgtagttt 240

ctgttacttc tcctgagatg gcaaaagaag tactaaaaac tcatgacctc gcttttgcat 300

ctaggccgtt acttttggca gccaaaattg tctgctataa tgggacagac attgtctttt 360

ccccctatgg cgattattgg agacaaacgc gtaaaatttg tctcttggaa ttgctcagtg 420

ccaaaaatgt taggtcattc agctcagtca gacgagatga agttttccat atgattgaat 480

ttttttcgat catcttctgg taagccagtt aatgtatcaa aaaggatttc tctattcaca 540

acctctatga catgtagatc agcctttgga caagaataca aggagcaaga cgaattcgca 600

caactagtaa aaaaagtgtc aagcttaatg gaagggtttg atgttgctga tatattccct 660

tcattgaagt ttcttcatgt gctcagtgga atgaaggcta aagttatgga tgcacaccat 720

gagttagatg ccattcttga aaaaattatc aatgagcaca agaaaattgc aactggaaag 780

aataataatg aattaggagg tgaaggatta attgacgtac tgctaagact tatgaaagag 840

ggaggccttc aattcccgat caccaacgac aacatcaaag ctattatttt tgacatgttt 900

ggtgcgggaa cggaaacttc atcaaccaca attgactggg ccatggtcga aatgataaag 960

aatccaagtg tattcgctaa agctcaagca gaggtaagag aagccttcag agagaaagaa 1020

acttttgatg aaaatgatgt cgaggagttg aaatacttaa aattggttat caaagaaact 1080

ttcagactcc atcctccatt tccccttttg ctcccaagag aatctagaga agaaacagat 1140

ataaacggct acactattcc ttttaaaaca aaacttatgg ttaacgttcg ggctattgga 1200

agagatccaa aatattggga tgacgtggaa agttttaagc cagagagatt tgagcacaac 1260

tctatggatt ttattggtaa taattttgaa tatcttccct ttggtagtgg aaggagaatg 1320

tgccctggga tatcatttgg tttggctaat gtttatttgc cactagctca attgttatat 1380

cattttgatt ggaaactccc tactggaatc aattcaagtg acttggacat gactgagtcg 1440

tcaggagtaa cttgtgctag aaagagtgat ttatacttga ctgctactcc atatcaactt 1500

tctcaagagt gatgcaatga tatcaacctt ttgaatttcg gtcaacccca ccaatagtg 1559

<210> 288
<211> 165
<212> PRT
<213> Nicotiana tabacum

<400> 288

Met Gln Phe Phe Asn Phe Ile Ser Phe Val Phe Phe Val Ser Phe Leu
1               5                   10                  15

Phe Leu Leu Arg Lys Trp Lys Asn Ser Asn Ser Gln Thr Lys Arg Leu
            20                  25                  30

Pro Pro Gly Pro Trp Lys Leu Pro Val Leu Gly Ser Met Phe His Leu
        35                  40                  45

Leu Gly Gly Pro Pro His His Val Leu Gly Asp Leu Ala Lys Lys Tyr
    50                  55                  60

Gly Pro Leu Met His Leu Gln Leu Gly Glu Val Ser Val Val Ser Val
65                  70                  75                  80

Thr Ser Pro Glu Met Ala Lys Glu Val Leu Lys Thr His Asp Leu Ala
                85                  90                  95

Phe Ala Ser Arg Pro Leu Leu Leu Ala Ala Lys Ile Val Cys Tyr Asn
            100                 105                 110

Gly Thr Asp Ile Val Phe Ser Pro Tyr Gly Asp Tyr Trp Arg Gln Thr
        115                 120                 125

Arg Lys Ile Cys Leu Leu Glu Leu Leu Ser Ala Lys Asn Val Arg Ser
    130                 135                 140

Phe Ser Ser Val Arg Arg Asp Glu Val Phe His Met Ile Glu Phe Phe
145                 150                 155                 160

Ser Ile Ile Phe Trp
                165

<210>  289
<211>  1601
<212>  DNA
<213>  Nicotiana tabacum

<400>  289
ccccaccaaa aaatcatttc tctcgtctaa aatggatctt ctcttactag agaagacctt      60

aattggtctt ttctttgcca ttttaatcgc tttaattgtc tctaaacttc gttcaaagcg     120

ttttaagctt cctccaggac caattccagt accagttttt ggtaattggc ttcaagttgg     180

tgatgattta aaccacagaa atcttactga ttatgccaag aaatttggag atcttttctt     240

gttaagaatg ggtcaacgta acttagttgt tgtgtcatct cctgaattag ctaaagaagt     300

tttacacaca caaggtgttg aatttggttc aagaacaaga aatgttgtgt ttgatatttt     360

tactggaaaa ggtcaagata tggtttttac tgtatatggt gaacattgga gaaaaatgag     420

gagaattatg actgtaccat tttttactaa taaagttgtg caacagtata gaggggggtg     480

ggagtttgag gtggcaagtg taattgagga tgtgaaaaaa aatcctgaat ctgctactaa     540

tgggatcgta ttaaggagga gattacaatt aatgatgtat aataatatgt ttaggattat     600

gtttgatagg agatttgaga gtgaagatga tcctttgttt gttaagctta aggctttgaa     660

tggtgaaagg agtagattgg ctcaaagttt tgagtataat tatggtgatt ttattccaat     720

tttgaggcct ttttttgaga ggttatttga agatctgtaa agaagttaag gagaagaggc     780

tgcagctttt caaagattac tttgttgatg aaagaaagaa gctttcgaat accaagagct     840

cggacagcaa tgccctaaaa tgtgcgattg atcacattct tgaggctcaa cagaagggag     900

agatcaatga ggacaacgtt ctttacattg ttgaaaacat caatgttgct gcaattgaaa     960

caacattatg gtcaattgag tggggtatcg ccgagctagt caaccaccct cacatccaaa    1020

agaaactgcg cgacgagatt gacacagttc ttggaccagg agtgcaagtg actgaaccag 1080

acacccacaa gcttccatac cttcaggctg tgatcaagga ggcacttcgt ctccgtatgg 1140

caattcctct attagtccca cacatgaacc ttcacgacgc aaagcttggc gggtttgata 1200

ttccagcaga gagcaaaatc ttggttaacg cttggtggtt agctaacaac ccggctcatt 1260

ggaagaaacc cgaagagttc agacccgaga ggttctttga agaggagaag catgttgagg 1320

ccaatggcaa tgacttcaga tatcttccgt ttggcgttgg taggaggagc tgccctggaa 1380

ttatacttgc attgccaact cttggcatca ctttgggacg tttggttcag aactttgagc 1440

tgttgcctcc tccaggccag tcgaagctcg acaccacaga gaaaggtgga cagttcagtc 1500

tccacatttt gaagcattcc accattgtgt tgaaaccaag gtctttctga actttgtgat 1560

cttattaatt aaggggttct gaagaaattt gatagtgttg g 1601

<210> 290
<211> 242
<212> PRT
<213> Nicotiana tabacum

<400> 290

Met Asp Leu Leu Leu Leu Glu Lys Thr Leu Ile Gly Leu Phe Phe Ala
1               5                   10                  15

Ile Leu Ile Ala Leu Ile Val Ser Lys Leu Arg Ser Lys Arg Phe Lys
            20                  25                  30

Leu Pro Pro Gly Pro Ile Pro Val Pro Val Phe Gly Asn Trp Leu Gln
        35                  40                  45

Val Gly Asp Asp Leu Asn His Arg Asn Leu Thr Asp Tyr Ala Lys Lys
    50                  55                  60

Phe Gly Asp Leu Phe Leu Leu Arg Met Gly Gln Arg Asn Leu Val Val
65                  70                  75                  80

Val Ser Ser Pro Glu Leu Ala Lys Glu Val Leu His Thr Gln Gly Val
                85                  90                  95

Glu Phe Gly Ser Arg Thr Arg Asn Val Val Phe Asp Ile Phe Thr Gly
            100                 105                 110

Lys Gly Gln Asp Met Val Phe Thr Val Tyr Gly Glu His Trp Arg Lys
        115                 120                 125

Met Arg Arg Ile Met Thr Val Pro Phe Phe Thr Asn Lys Val Val Gln
    130                 135                 140

Gln Tyr Arg Gly Gly Trp Glu Phe Glu Val Ala Ser Val Ile Glu Asp
145                 150                 155                 160

Val Lys Lys Asn Pro Glu Ser Ala Thr Asn Gly Ile Val Leu Arg Arg
                165                 170                 175

Arg Leu Gln Leu Met Met Tyr Asn Asn Met Phe Arg Ile Met Phe Asp
            180                 185                 190

Arg Arg Phe Glu Ser Glu Asp Asp Pro Leu Phe Val Lys Leu Lys Ala
        195                 200                 205

Leu Asn Gly Glu Arg Ser Arg Leu Ala Gln Ser Phe Glu Tyr Asn Tyr
    210                 215                 220

Gly Asp Phe Ile Pro Ile Leu Arg Pro Phe Phe Glu Arg Leu Phe Glu
225                 230                 235                 240

Asp Leu

<210>  291
<211>  1544
<212>  DNA
<213>  Nicotiana tabacum

<400>  291
ataatgctct ttctactctt tgtagccctt cctttcattc ttatttttct tcttcctaaa      60

ttcaaaaatg gtggaaataa cagattgcca ccaggtccta taggtttacc attcattgga     120

aatttgcatc aatatgatag tataactcct catatctatt tttggaaact ttccaaaaaa     180

tatggcaaaa tcttctcatt aaaacttgct tctactaatg tggtagtagt ttcttcagca     240

aaattagcaa aagaagtatt gaaaaaacaa gatttaatat tttgtagtag accatctatt     300

cttggccaac aaaaactgtc ttattatggt cgtgatattg cttttgcacc ttataatgat     360

tattggagag aaatgagaaa aatttgtgtt cttcatcttt ttagtttaaa aaaagttcaa     420

ttatttagtc caattcgtga agatgaagtt tttagaatga ttaagaaaat atcaaaacaa     480

gcttctactt cacaaattat taatttgagt aatttaatga tttcattaac aagtacaatt     540

atttgtagag ttgcttttgg tgttaggttt gaagaagaag cacatgcaag gaagagattt     600

gatttctttt tggccgaggc acaagaaatg atggctagtt tctttgtatc tgattttttt     660

ccctttttaa gttagattga caaattaagt ggattgacat atagacttga gaggaatttc     720

aaggatttgg ataattttta tgaagaactc attgagcaac atcaaaatcc taataagcca     780

aaatatatgg aaggagatat tgttgatctt ttgctacaat tgaagaaaga gaaattaaca     840

ccacttgatc tcactatgga agatataaaa ggaattctca tgaatgtgtt agttgcagga     900

tcagacacta gtgcagctgc tactgtttgg gcaatgacag ccttgataaa gaatcctaaa     960

gccatggaaa aagttcaatt agaaatcaga aaatcagttg ggaagaaagg cattgtaaat    1020

gaagaagatg tccaaaacat cccttatttt aaagcagtga taaaggaaat atttagattg    1080

tatccaccag ctccactttt agttccaaga gaatcaatgg aaaaaaccat attagaaggt    1140

tatgaaattc ggccaagaac catagttcat gttaacgctt gggctatagc aagggatcct 1200

gaaatatggg aaaatccaga tgaatttata cctgagagat ttttgaatag cagtatcgat 1260

tacaagggtc aagattttga gttacttcca tttggtgcag gcagaagagg ttgcccaggt 1320

attgcacttg gggttgcatc catggaactt gctttgtcaa atcttcttta tgcatttgat 1380

tgggagttgc cttatggagt gaaaaaagaa gacatcgaca caaacgttag gcctggaatt 1440

gccatgcaca agaaaaacga actttgcctt gtcccaaaaa aattatttat aaattatatt 1500

gggacgtgga tctcatgcta gttctgtgcg gtcagctaag ctta 1544

<210> 292
<211> 223
<212> PRT
<213> Nicotiana tabacum

<400> 292

Met Leu Phe Leu Leu Phe Val Ala Leu Pro Phe Ile Leu Ile Phe Leu
1               5                   10                  15

Leu Pro Lys Phe Lys Asn Gly Gly Asn Asn Arg Leu Pro Pro Gly Pro
            20                  25                  30

Ile Gly Leu Pro Phe Ile Gly Asn Leu His Gln Tyr Asp Ser Ile Thr
        35                  40                  45

Pro His Ile Tyr Phe Trp Lys Leu Ser Lys Lys Tyr Gly Lys Ile Phe
    50                  55                  60

Ser Leu Lys Leu Ala Ser Thr Asn Val Val Val Val Ser Ser Ala Lys
65                  70                  75                  80

Leu Ala Lys Glu Val Leu Lys Lys Gln Asp Leu Ile Phe Cys Ser Arg
                85                  90                  95

Pro Ser Ile Leu Gly Gln Gln Lys Leu Ser Tyr Tyr Gly Arg Asp Ile
            100                 105                 110

Ala Phe Ala Pro Tyr Asn Asp Tyr Trp Arg Glu Met Arg Lys Ile Cys
        115                 120                 125

Val Leu His Leu Phe Ser Leu Lys Lys Val Gln Leu Phe Ser Pro Ile
    130                 135                 140

Arg Glu Asp Glu Val Phe Arg Met Ile Lys Lys Ile Ser Lys Gln Ala
145                 150                 155                 160

Ser Thr Ser Gln Ile Ile Asn Leu Ser Asn Leu Met Ile Ser Leu Thr
                165                 170                 175

Ser Thr Ile Ile Cys Arg Val Ala Phe Gly Val Arg Phe Glu Glu Glu
            180                 185                 190

Ala His Ala Arg Lys Arg Phe Asp Phe Leu Leu Ala Glu Ala Gln Glu
        195                 200                 205

Met Met Ala Ser Phe Phe Val Ser Asp Phe Phe Pro Phe Leu Ser
    210                 215                 220

<210>  293
<211>  1765
<212>  DNA
<213>  Nicotiana tabacum

<400>  293
gtgaggtttg aatcctctgc ctcaatgaaa ctcaccaaat tggttttcta atttccatct      60

aaaatattgt ccaaagctaa agattctttc tccttaaata gtcaacttta gtggttcctc     120

ttcatttcat agctcaatct ttcttatttt gattcaacca tggagaacca atactcctac     180

tcattctctt cctacttcta cttagctata gtactgtttc ttcttccaat tttggtcaaa     240

tatttcttcc atcggagaag aaatttacct ccaagtccat tttctcttcc aataattggt     300

cacctttacc ttctcaagaa aactctccat ctcactctaa catccttatc agctaaatat     360

ggtcctgttt tatacctcaa attgggctct atgcctgtga ttgttgtgtc ctcaccatct     420

gctgttgaag aatgtttaac caagaatgat atcatattcg caaataggcc caagaccgtg     480

gctggtgaca agtttaccta caattatact gtttatgttt gggcacccta tggccaactt     540

tggagaattc ttcgccgatt aactgtcgtt gaactcttct cttcacatag cctacagaaa     600

acttctatcc ttagagatca agaagttgca atatttatcc gttcgttata caaattctca     660

aaggatagta gcaaaaaagt cgatttgacc aactggtctt ttactttggt tttcaatctt     720

atgaccaaaa ttattgctgg gagacatatt gtgaaggagg aagatgctgg caaggaaaag     780

ggcattgaaa ttattgaaaa acttagaggg actttcttag taactacatc attcttgaat     840

atgtgtgatt tcttgccagt attcaggtgg gttggttaca aagggctgga gaagaagatg     900

gcctcaattc acaatagaag aaatgaattc ttgaacagct tgcttgatga atttcgacac     960

aagaaaagta gtgcttcaca atctaacaca actgttggaa acatggagaa gaaaaccaca    1020

ctgattgaaa agctcttgtc tcttcaagaa tcagagcctg aattctacac tgatgatatc    1080

atcaaaagta ttatgctggt agtttttgtt gcaggaacag agacctcatc aacaaccatc    1140

caatgggtaa tgaggcttct tgtagctcac cctgaggcat tgtataagct acgagctgac    1200

attgacagta aagttgggaa taagcgcttg ctgaatgaat cagacctcaa caagcttccg    1260

tatttgcatt gtgttgttaa tgagacaatg agattataca ctccgatacc acttttattg    1320

cctcattatt caactaaaga ttgtattgtg gaaggatatg atgtaccaaa acatacaatg    1380

ttgtttgtca acgcttgggc cattcacagg gatcccaagg tatgggagga gcctgacaag    1440

ttcaagccag agagatttga ggcaacagaa ggggaaacag aaaggttcaa ttacaagctt    1500

gtaccatttg gaatggggag aagagcgtgc cctggagctg atatggggtt gcgagcagtt    1560

tctttggcat taggtgcact tattcaatgc tttgactggc aaattgagga agcggaaagc 1620
ttggaggaaa gctataattc tagaatgact atgcagaaca agcctttgaa ggttgtctgc 1680
actccacgcg aagatcttgg ccagcttcta tcccaactct aaggcaattt atcaatgcca 1740
aacgtaatct tcatctacca ctatg 1765

<210> 294
<211> 520
<212> PRT
<213> Nicotiana tabacum

<400> 294

Met Glu Asn Gln Tyr Ser Tyr Ser Phe Ser Ser Tyr Phe Tyr Leu Ala
1               5                   10                  15

Ile Val Leu Phe Leu Leu Pro Ile Leu Val Lys Tyr Phe Phe His Arg
            20                  25                  30

Arg Arg Asn Leu Pro Pro Ser Pro Phe Ser Leu Pro Ile Ile Gly His
        35                  40                  45

Leu Tyr Leu Leu Lys Lys Thr Leu His Leu Thr Leu Thr Ser Leu Ser
    50                  55                  60

Ala Lys Tyr Gly Pro Val Leu Tyr Leu Lys Leu Gly Ser Met Pro Val
65                  70                  75                  80

Ile Val Val Ser Ser Pro Ser Ala Val Glu Glu Cys Leu Thr Lys Asn
                85                  90                  95

Asp Ile Ile Phe Ala Asn Arg Pro Lys Thr Val Ala Gly Asp Lys Phe
            100                 105                 110

Thr Tyr Asn Tyr Thr Val Tyr Val Trp Ala Pro Tyr Gly Gln Leu Trp
        115                 120                 125

Arg Ile Leu Arg Arg Leu Thr Val Val Glu Leu Phe Ser Ser His Ser
    130                 135                 140

Leu Gln Lys Thr Ser Ile Leu Arg Asp Gln Glu Val Ala Ile Phe Ile
145                 150                 155                 160

Arg Ser Leu Tyr Lys Phe Ser Lys Asp Ser Ser Lys Lys Val Asp Leu
                165                 170                 175

Thr Asn Trp Ser Phe Thr Leu Val Phe Asn Leu Met Thr Lys Ile Ile
            180                 185                 190

Ala Gly Arg His Ile Val Lys Glu Glu Asp Ala Gly Lys Glu Lys Gly
        195                 200                 205

Ile Glu Ile Ile Glu Lys Leu Arg Gly Thr Phe Leu Val Thr Thr Ser
    210                 215                 220

Phe Leu Asn Met Cys Asp Phe Leu Pro Val Phe Arg Trp Val Gly Tyr
225                 230                 235                 240

Lys Gly Leu Glu Lys Lys Met Ala Ser Ile His Asn Arg Arg Asn Glu
                245                 250                 255

Phe Leu Asn Ser Leu Leu Asp Glu Phe Arg His Lys Lys Ser Ser Ala
            260                 265                 270

Ser Gln Ser Asn Thr Thr Val Gly Asn Met Glu Lys Lys Thr Thr Leu
        275                 280                 285

Ile Glu Lys Leu Leu Ser Leu Gln Glu Ser Glu Pro Glu Phe Tyr Thr
    290                 295                 300

Asp Asp Ile Ile Lys Ser Ile Met Leu Val Val Phe Val Ala Gly Thr
305                 310                 315                 320

Glu Thr Ser Ser Thr Thr Ile Gln Trp Val Met Arg Leu Leu Val Ala
                325                 330                 335

His Pro Glu Ala Leu Tyr Lys Leu Arg Ala Asp Ile Asp Ser Lys Val
            340                 345                 350

Gly Asn Lys Arg Leu Leu Asn Glu Ser Asp Leu Asn Lys Leu Pro Tyr
        355                 360                 365

Leu His Cys Val Val Asn Glu Thr Met Arg Leu Tyr Thr Pro Ile Pro
    370                 375                 380

Leu Leu Leu Pro His Tyr Ser Thr Lys Asp Cys Ile Val Glu Gly Tyr
385                 390                 395                 400

Asp Val Pro Lys His Thr Met Leu Phe Val Asn Ala Trp Ala Ile His
                405                 410                 415

Arg Asp Pro Lys Val Trp Glu Glu Pro Asp Lys Phe Lys Pro Glu Arg
            420                 425                 430

Phe Glu Ala Thr Glu Gly Glu Thr Glu Arg Phe Asn Tyr Lys Leu Val
        435                 440                 445

Pro Phe Gly Met Gly Arg Arg Ala Cys Pro Gly Ala Asp Met Gly Leu
    450                 455                 460

Arg Ala Val Ser Leu Ala Leu Gly Ala Leu Ile Gln Cys Phe Asp Trp
465                 470                 475                 480

Gln Ile Glu Glu Ala Glu Ser Leu Glu Glu Ser Tyr Asn Ser Arg Met
                485                 490                 495

Thr Met Gln Asn Lys Pro Leu Lys Val Val Cys Thr Pro Arg Glu Asp
            500                 505                 510

Leu Gly Gln Leu Leu Ser Gln Leu
        515                 520

<210> 295
<211> 1694
<212> DNA
<213> Nicotiana tabacum

<400> 295
aacatccttt ccttcttcca aaaatggagc ttcaatcttc tcctttcaat ttaatttctt 60

tgttcctctt cttttctttt ctttttattc tagtgaagaa atggaatgcc aaaatcccaa 120

agttacctcc aggtccgtgg aggcttccct ttattggaag cctccatcac ttgaagggaa 180

aacttccaca ccataatctt agagatctag cgcgaaaata tggacctctc atgtacttac 240

aactcggaga aattcctgta gttgtaatat cttcgccacg tgtagcaaaa gctgtactaa 300

aaactcatga tctcgctttt gcaactagac cacgattcat gtcctcagac attgtgtttt 360

acaaaagcag ggacatctct tttgccccat ttggtgatta ctggagacag atgcgtaaaa 420

tattgactca ggaactcctg agcaacaaga tgctcaagtc atatagctta atccgaaagg 480

atgagctctc gaagctcctc tcatcgattc gtttggaaac aggttctgca gtgaacataa 540

atgaaaagct tctctggttt acgagctgca tgacctgtag attagccttt ggaaaaatat 600

gcaatgatcg ggatgagttg atcatgctaa ttagggagat attaacatta tcaggaggat 660

ttgatgtggg tgatttgttc ccttcctgga aattacttca taatatgagc aacatgaaag 720

ctaggttgac gaatgtacac cacaagtatg atttagttat ggagaacatc atcaatgagc 780

accaagagaa tcatgcagca gggataaagg gtaacaacga gtttggtggc gaagatatga 840

tcgatgctct actgagggct aaggagaata atgagcttca atttcctatc gaaaatgaca 900

acatgaaagc agtaattctg gacttgttta ttgctggaac tgaaacttca tatactgcaa 960

ttatatgggc actatcagaa ttgatgaagc acccaagtgt gatggccaag gcacaagctg 1020

aagtgagaaa agtcttcaaa gaaaatgaaa atttcgacga aaatgatctt gacaagttgc 1080

catacctaaa atcagtgatt aaagaaacac taaggatgca ccctccagtt cctttgttag 1140

ggcctagaga atgcagggac caaacagaga tcgatggcta cactgtacct attaaagcta 1200

gagttatggt taatgcttgg gcgataggaa gagatcctga aagttgggaa gatcctgaaa 1260

gtttcaaacc ggagcgattt gaaaatactt ctgttgatct tacaggaaat cactatcagt 1320

tcattccttt cggttcagga agaagaatgt gtccaggaat gtcgtttggt ttagttaaca 1380

cagggcatcc tttagcccag ttgctctatt gctttgactg gaaactccct gacaaggtta 1440

atgcaaatga ttttcgcact actgaaacaa gtagagtttt tgcagcaagc aaagatgacc 1500

tctacttgat tcccacaaat cacagggagc aagaatagct taatttaatg gagttcttgg 1560
aagaattaaa gaagaagggc tatataggtg agattttttg tatggttgca aggtttttag 1620
ttcatacaat aagacaatac attatattcc agtattgtgt atcatgtata ataaggttcc 1680
ttttgtttaa aaaa 1694

<210> 296
<211> 504
<212> PRT
<213> Nicotiana tabacum

<400> 296

Met Glu Leu Gln Ser Ser Pro Phe Asn Leu Ile Ser Leu Phe Leu Phe
1               5                   10                  15

Phe Ser Phe Leu Phe Ile Leu Val Lys Lys Trp Asn Ala Lys Ile Pro
            20                  25                  30

Lys Leu Pro Pro Gly Pro Trp Arg Leu Pro Phe Ile Gly Ser Leu His
        35                  40                  45

His Leu Lys Gly Lys Leu Pro His His Asn Leu Arg Asp Leu Ala Arg
    50                  55                  60

Lys Tyr Gly Pro Leu Met Tyr Leu Gln Leu Gly Glu Ile Pro Val Val
65                  70                  75                  80

Val Ile Ser Ser Pro Arg Val Ala Lys Ala Val Leu Lys Thr His Asp
                85                  90                  95

Leu Ala Phe Ala Thr Arg Pro Arg Phe Met Ser Ser Asp Ile Val Phe
            100                 105                 110

Tyr Lys Ser Arg Asp Ile Ser Phe Ala Pro Phe Gly Asp Tyr Trp Arg
        115                 120                 125

Gln Met Arg Lys Ile Leu Thr Gln Glu Leu Leu Ser Asn Lys Met Leu
    130                 135                 140

Lys Ser Tyr Ser Leu Ile Arg Lys Asp Glu Leu Ser Lys Leu Leu Ser
145                 150                 155                 160

Ser Ile Arg Leu Glu Thr Gly Ser Ala Val Asn Ile Asn Glu Lys Leu
                165                 170                 175

Leu Trp Phe Thr Ser Cys Met Thr Cys Arg Leu Ala Phe Gly Lys Ile
            180                 185                 190

Cys Asn Asp Arg Asp Glu Leu Ile Met Leu Ile Arg Glu Ile Leu Thr
        195                 200                 205

Leu Ser Gly Gly Phe Asp Val Gly Asp Leu Phe Pro Ser Trp Lys Leu
    210                 215                 220

Leu His Asn Met Ser Asn Met Lys Ala Arg Leu Thr Asn Val His His
225                 230                 235                 240

Lys Tyr Asp Leu Val Met Glu Asn Ile Ile Asn Glu His Gln Glu Asn
                245                 250                 255

His Ala Ala Gly Ile Lys Gly Asn Asn Glu Phe Gly Gly Glu Asp Met
            260                 265                 270

Ile Asp Ala Leu Leu Arg Ala Lys Glu Asn Asn Glu Leu Gln Phe Pro
        275                 280                 285

Ile Glu Asn Asp Asn Met Lys Ala Val Ile Leu Asp Leu Phe Ile Ala
    290                 295                 300

Gly Thr Glu Thr Ser Tyr Thr Ala Ile Ile Trp Ala Leu Ser Glu Leu
305                 310                 315                 320

Met Lys His Pro Ser Val Met Ala Lys Ala Gln Ala Glu Val Arg Lys
                325                 330                 335

Val Phe Lys Glu Asn Glu Asn Phe Asp Glu Asn Asp Leu Asp Lys Leu
            340                 345                 350

Pro Tyr Leu Lys Ser Val Ile Lys Glu Thr Leu Arg Met His Pro Pro
        355                 360                 365

Val Pro Leu Leu Gly Pro Arg Glu Cys Arg Asp Gln Thr Glu Ile Asp
    370                 375                 380

Gly Tyr Thr Val Pro Ile Lys Ala Arg Val Met Val Asn Ala Trp Ala
385                 390                 395                 400

Ile Gly Arg Asp Pro Glu Ser Trp Glu Asp Pro Glu Ser Phe Lys Pro
                405                 410                 415

Glu Arg Phe Glu Asn Thr Ser Val Asp Leu Thr Gly Asn His Tyr Gln
            420                 425                 430

Phe Ile Pro Phe Gly Ser Gly Arg Arg Met Cys Pro Gly Met Ser Phe
        435                 440                 445

Gly Leu Val Asn Thr Gly His Pro Leu Ala Gln Leu Leu Tyr Cys Phe
    450                 455                 460

Asp Trp Lys Leu Pro Asp Lys Val Asn Ala Asn Asp Phe Arg Thr Thr
465                 470                 475                 480

Glu Thr Ser Arg Val Phe Ala Ala Ser Lys Asp Asp Leu Tyr Leu Ile
                485                 490                 495

Pro Thr Asn His Arg Glu Gln Glu
            500

<210> 297
<211> 1819
<212> DNA
<213> Nicotiana tabacum

<400> 297
   caatcagtgg atgcgggagt aatatataat atgcaagttg tagaaagaga aaaaaaaaat 60
   caagtagcta ttctatactg gggcacaaat agtgagtgaa aatggagact gttcaaatca 120
   taataacagc atcttgtgct gccataataa ttactctagt ggtgtgtatt tggagagtac 180
   tgaattgggt ttggttcaga ccaaagaagc tggaaaaact attgaggaaa caaggtctca 240
   aaggcaactc ctacaagatt ttgtatgggg atatgaagga gctttctggt atgattaagg 300
   aagctaattc caaacccatg aatctttctg atgatattgc accaagattg gtgcctttct 360
   ttcttgacac catcaagaaa tatggtaaaa aatcctttgt atggttaggt ccgaaaccac 420
   tggttcttat catggaccct gagcttataa aggaaatatt ttccaaatac tatctgtatc 480
   aaaagcctca tggaaatcca gttaccaagc tattagtaca aggactagta agcctagagg 540
   aagacaaatg ggccaaacat agaaaaatca tcaatccagc tttccatcta gagaagctaa 600
   agcatatgct tccagctttt tgcttgagct gcactgagat gctgtgcaaa tgggaagata 660
   ttgtttcaat taagggctca catgagatag atgtatggcc tcaccttgaa caattaagta 720
   gcgatgtgat ctctcggaca gcttttggca gtaactttga agaaggtaaa aggatatttg 780
   aacttcagaa ggaacaagct cagtattttg tagaagctat acgctcggtt tatataccag 840
   gctggaggtt tttgccaaca aagaggaaca gaagaatgaa ggaagttgaa aaggatgttc 900
   gggcctcgat aagaggcatt attgataaaa gagtgaaggc aatgaaagca ggagaggcga 960
   gtaatgagga tctacttggt atattgttgg aatctaattt tacagaagct gaacagcata 1020
   gacacaagga ttctgcgatg agcattgaag aagtcattca agaatgcaag ttattctatg 1080
   ttgctggcca agaaactaca tcagtgttgc ttgtgtggac tctaatattg ttgagtaggc 1140
   atcaagattg gcagagccga gccagagaag aggtgtttca agtctttggt aatcagaaac 1200
   cagattttga cggattgaat cgtctaaaag ttgtgacaat gatcttgtat gagtctttaa 1260
   ggctatactc cccagtagtg tcactaatcc ggcggcctaa tgaggatgct atattaggaa 1320
   atgtatctct gccagaaggt gtgctactct cattaccagt gatcttatta caccacgatg 1380
   aagagatatg gggtaaagat gcaaagaagt tcaatccaga aagatttaga gatggagtct 1440
   caagtgcaac aaagggtcaa gtcacttttt ttccatttac ttggggtccc agaatatgca 1500
   tcggacaaaa ttttgccatg ttagaagcaa agactgcttt ggctatgatc ctacaacgct 1560
   tctcattcga actgtctcca tcttatgcac atgctcctca gtccatatta actatgcaac 1620
   cccaacatgg tgctccacta attctgcaca aaatatagtt tgttacttta agcagtgtct 1680
   tgttatatgt cagagagtcc aaaatgttta attaaggctt gtagaactgc caaatggaac 1740
   ttcatttgca ttcgtgggtt gtagattgtt gtaattggac aagtatactg tttattttag 1800
   agttttaaga aaaaaaaaa 1819

<210> 298
<211> 518
<212> PRT
<213> Nicotiana tabacum

<400> 298

Met Glu Thr Val Gln Ile Ile Ile Thr Ala Ser Cys Ala Ala Ile Ile
1               5                   10                  15

Ile Thr Leu Val Val Cys Ile Trp Arg Val Leu Asn Trp Val Trp Phe
            20                  25                  30

Arg Pro Lys Lys Leu Glu Lys Leu Leu Arg Lys Gln Gly Leu Lys Gly
        35                  40                  45

Asn Ser Tyr Lys Ile Leu Tyr Gly Asp Met Lys Glu Leu Ser Gly Met
    50                  55                  60

Ile Lys Glu Ala Asn Ser Lys Pro Met Asn Leu Ser Asp Asp Ile Ala
65                  70                  75                  80

Pro Arg Leu Val Pro Phe Phe Leu Asp Thr Ile Lys Lys Tyr Gly Lys
                85                  90                  95

Lys Ser Phe Val Trp Leu Gly Pro Lys Pro Leu Val Leu Ile Met Asp
            100                 105                 110

Pro Glu Leu Ile Lys Glu Ile Phe Ser Lys Tyr Tyr Leu Tyr Gln Lys
        115                 120                 125

Pro His Gly Asn Pro Val Thr Lys Leu Leu Val Gln Gly Leu Val Ser
    130                 135                 140

Leu Glu Glu Asp Lys Trp Ala Lys His Arg Lys Ile Ile Asn Pro Ala
145                 150                 155                 160

Phe His Leu Glu Lys Leu Lys His Met Leu Pro Ala Phe Cys Leu Ser
                165                 170                 175

Cys Thr Glu Met Leu Cys Lys Trp Glu Asp Ile Val Ser Ile Lys Gly
            180                 185                 190

Ser His Glu Ile Asp Val Trp Pro His Leu Glu Gln Leu Ser Ser Asp
        195                 200                 205

Val Ile Ser Arg Thr Ala Phe Gly Ser Asn Phe Glu Glu Gly Lys Arg
    210                 215                 220

Ile Phe Glu Leu Gln Lys Glu Gln Ala Gln Tyr Phe Val Glu Ala Ile
225                 230                 235                 240

Arg Ser Val Tyr Ile Pro Gly Trp Arg Phe Leu Pro Thr Lys Arg Asn
                245                 250                 255

Arg Arg Met Lys Glu Val Glu Lys Asp Val Arg Ala Ser Ile Arg Gly
            260                 265                 270

Ile Ile Asp Lys Arg Val Lys Ala Met Lys Ala Gly Glu Ala Ser Asn
        275                 280                 285

Glu Asp Leu Leu Gly Ile Leu Leu Glu Ser Asn Phe Thr Glu Ala Glu
    290                 295                 300

Gln His Arg His Lys Asp Ser Ala Met Ser Ile Glu Glu Val Ile Gln
305                 310                 315                 320

Glu Cys Lys Leu Phe Tyr Val Ala Gly Gln Glu Thr Thr Ser Val Leu
                325                 330                 335

Leu Val Trp Thr Leu Ile Leu Leu Ser Arg His Gln Asp Trp Gln Ser
            340                 345                 350

Arg Ala Arg Glu Glu Val Phe Gln Val Phe Gly Asn Gln Lys Pro Asp
        355                 360                 365

Phe Asp Gly Leu Asn Arg Leu Lys Val Val Thr Met Ile Leu Tyr Glu
    370                 375                 380

Ser Leu Arg Leu Tyr Ser Pro Val Val Ser Leu Ile Arg Arg Pro Asn
385                 390                 395                 400

Glu Asp Ala Ile Leu Gly Asn Val Ser Leu Pro Glu Gly Val Leu Leu
                405                 410                 415

Ser Leu Pro Val Ile Leu Leu His His Asp Glu Glu Ile Trp Gly Lys
            420                 425                 430

Asp Ala Lys Lys Phe Asn Pro Glu Arg Phe Arg Asp Gly Val Ser Ser
        435                 440                 445

Ala Thr Lys Gly Gln Val Thr Phe Phe Pro Phe Thr Trp Gly Pro Arg
    450                 455                 460

Ile Cys Ile Gly Gln Asn Phe Ala Met Leu Glu Ala Lys Thr Ala Leu
465                 470                 475                 480

Ala Met Ile Leu Gln Arg Phe Ser Phe Glu Leu Ser Pro Ser Tyr Ala
                485                 490                 495

His Ala Pro Gln Ser Ile Leu Thr Met Gln Pro Gln His Gly Ala Pro
            500                 505                 510

Leu Ile Leu His Lys Ile
        515

<210>  299
<211>  83
<212>  PRT
<213>  Nicotiana tabacum

<400>  299

Glu Val Leu Arg Leu Tyr Pro Pro Gly Pro Leu Leu Val Pro His Glu
1               5                   10                  15

Asn Val Glu Asp Cys Val Val Ser Gly Tyr His Ile Pro Lys Gly Thr
            20                  25                  30

Arg Leu Phe Ala Asn Val Met Lys Leu Gln Arg Asp Pro Lys Leu Trp
        35                  40                  45

Ser Asp Pro Asp Thr Phe Asp Pro Glu Arg Phe Ile Ala Thr Asp Ile
    50                  55                  60

Asp Phe Arg Gly Gln Tyr Tyr Lys Tyr Ile Pro Phe Gly Pro Gly Arg
65                  70                  75                  80

Arg Ser Cys

<210>  300
<211>  83
<212>  PRT
<213>  Nicotiana tabacum

<400>  300

Glu Val Leu Arg Leu Tyr Pro Pro Gly Pro Leu Leu Val Pro His Glu
1               5                   10                  15

Asn Val Glu Asp Cys Val Val Ser Gly Tyr His Ile Pro Lys Gly Thr
            20                  25                  30

Arg Leu Phe Ala Asn Val Met Lys Leu Leu Arg Asp Pro Lys Leu Trp
        35                  40                  45

Pro Asp Pro Asp Thr Phe Asp Pro Glu Arg Phe Ile Ala Thr Asp Ile
    50                  55                  60

Asp Phe Arg Gly Gln Tyr Tyr Lys Tyr Ile Pro Phe Gly Ser Gly Arg
65                  70                  75                  80

Arg Ser Cys

<210> 301
<211> 83
<212> PRT
<213> Nicotiana tabacum

<400> 301

Glu Val Leu Arg Leu Tyr Pro Pro Gly Pro Leu Leu Val Pro His Glu
1               5                   10                  15

Asn Val Glu Asp Cys Val Val Ser Gly Tyr His Ile Pro Lys Gly Thr
            20                  25                  30

Arg Leu Phe Ala Asn Val Met Lys Leu Gln Arg Asp Pro Lys Leu Trp
        35                  40                  45

Ser Asp Pro Asp Thr Phe Asp Pro Glu Arg Phe Ile Ala Thr Asp Ile
    50                  55                  60

Asp Phe Arg Gly Gln Tyr Tyr Lys Tyr Ile Pro Phe Gly Ser Gly Arg
65                  70                  75                  80

Arg Ser Cys

<210> 302
<211> 83
<212> PRT
<213> Nicotiana tabacum

<400> 302

Glu Val Leu Arg Leu Tyr Pro Pro Gly Pro Leu Leu Val Pro His Glu
1               5                   10                  15

Asn Val Glu Asp Cys Val Val Ser Gly Tyr His Ile Pro Lys Gly Thr
            20                  25                  30

Arg Leu Phe Ala Asn Val Met Lys Leu Gln Arg Asp Pro Lys Leu Trp
        35                  40                  45

Ser Asn Pro Asp Lys Phe Asp Pro Glu Arg Phe Phe Ala Asp Asp Ile
    50                  55                  60

Asp Tyr Arg Gly Gln His Tyr Glu Phe Ile Pro Phe Gly Ser Gly Arg
65                  70                  75                  80

Arg Ser Cys

<210> 303
<211> 83
<212> PRT
<213> Nicotiana tabacum

<400> 303

Lys Val Leu Arg Leu Tyr Pro Pro Gly Pro Leu Leu Val Pro His Glu
1               5                   10                  15

Asn Val Lys Asp Cys Val Val Ser Gly Tyr His Ile Pro Lys Gly Thr
            20                  25                  30

Arg Leu Phe Ala Asn Val Met Lys Leu Gln Arg Asp Pro Lys Leu Leu
        35                  40                  45

Ser Asn Pro Asp Lys Phe Asp Pro Glu Arg Phe Ile Ala Gly Asp Ile
    50                  55                  60

Asp Phe Arg Gly His His Tyr Glu Phe Ile Pro Ser Gly Ser Gly Arg
65                  70                  75                  80

Arg Ser Cys


<210> 304
<211> 83
<212> PRT
<213> Nicotiana tabacum

<400> 304

Lys Val Leu Arg Leu Tyr Pro Pro Gly Pro Leu Leu Val Pro His Glu
1               5                   10                  15

Asn Val Lys Asp Cys Val Val Ser Gly Tyr His Ile Pro Lys Gly Thr
            20                  25                  30

Arg Leu Phe Ala Asn Val Met Lys Leu Gln Arg Asp Pro Lys Leu Leu
        35                  40                  45

Ser Asn Pro Asp Lys Phe Asp Pro Glu Arg Phe Ile Ala Gly Asp Ile
    50                  55                  60

Asp Phe Arg Gly His His Tyr Glu Phe Ile Pro Phe Gly Ser Gly Arg
65                  70                  75                  80

Arg Ser Cys


<210> 305
<211> 83
<212> PRT
<213> Nicotiana tabacum

<400> 305

Lys Val Leu Arg Leu Tyr Pro Pro Gly Pro Leu Leu Val Pro His Glu
1               5                   10                  15

Tyr Val Lys Asp Cys Val Val Ser Gly Tyr His Ile Pro Lys Gly Thr
            20                  25                  30

Arg Leu Phe Ala Asn Val Met Lys Leu Gln Arg Asp Pro Lys Leu Leu
        35                  40                  45

Ser Asn Pro Asp Lys Phe Asp Pro Glu Arg Phe Ile Ala Gly Asp Ile
    50                  55                  60

Asp Phe Arg Gly His His Tyr Glu Phe Ile Pro Phe Gly Ser Gly Arg
65                  70                  75                  80

Arg Ser Cys

<210>  306
<211>  85
<212>  PRT
<213>  Nicotiana tabacum

<400>  306

Glu Thr Leu Arg Leu Tyr Pro Pro Val Pro Phe Leu Leu Pro His Glu
1               5                   10                  15

Ala Val Gln Asp Cys Lys Val Thr Gly Tyr His Ile Pro Lys Gly Thr
            20                  25                  30

Arg Leu Tyr Ile Asn Ala Trp Lys Val His Arg Asp Pro Glu Ile Trp
        35                  40                  45

Ser Glu Pro Glu Lys Phe Met Pro Asn Arg Phe Leu Thr Ser Lys Ala
    50                  55                  60

Asn Ile Asp Ala Arg Gly Gln Asn Phe Glu Phe Ile Pro Phe Gly Ser
65                  70                  75                  80

Gly Arg Arg Ser Cys
                85

<210>  307
<211>  85
<212>  PRT
<213>  Nicotiana tabacum

<400>  307

Glu Thr Leu Arg Leu Tyr Pro Pro Val Pro Phe Leu Leu Pro His Glu
1               5                   10                  15

Ala Val Gln Asp Cys Lys Val Thr Gly Tyr His Ile Pro Lys Gly Thr

20                  25                  30

Arg Leu Tyr Ile Asn Ala Trp Lys Val His Arg Asp Pro Glu Ile Trp
        35                  40                  45

Ser Glu Pro Glu Lys Phe Met Pro Asn Arg Phe Leu Thr Ser Lys Ala
    50                  55                  60

Asn Ile Asp Ala Arg Gly Gln Asn Phe Glu Phe Ile Pro Phe Gly Ser
65                  70                  75                  80

Gly Arg Arg Ser Cys
                85

<210>  308
<211>  85
<212>  PRT
<213>  Nicotiana tabacum

<400>  308

Glu Thr Leu Arg Leu Phe Pro Pro Val Pro Phe Leu Leu Pro His Glu
1               5                   10                  15

Ala Val Gln Asp Cys Lys Val Thr Gly Tyr His Ile Pro Lys Gly Thr
            20                  25                  30

Arg Leu Tyr Ile Asn Ala Trp Lys Val His Arg Asp Pro Glu Ile Trp
        35                  40                  45

Ser Glu Pro Glu Lys Phe Met Pro Asn Arg Phe Leu Thr Ser Lys Ala
    50                  55                  60

Asn Ile Asp Ala Arg Gly Gln Asn Phe Glu Phe Ile Pro Phe Gly Ser
65                  70                  75                  80

Gly Arg Arg Ser Cys
                85

<210>  309
<211>  85
<212>  PRT
<213>  Nicotiana tabacum

<400>  309

Glu Thr Leu Arg Leu Phe Pro Pro Val Pro Phe Leu Leu Pro His Glu
1               5                   10                  15

Ala Val Gln Asp Cys Lys Val Thr Gly Tyr His Ile Pro Lys Gly Thr
            20                  25                  30

Arg Leu Tyr Ile Asn Ala Trp Lys Val His Arg Asp Pro Glu Ile Trp
        35                  40                  45

Ser Glu Pro Glu Lys Phe Met Pro Asn Arg Phe Leu Thr Ser Lys Ala
    50                  55                  60

Asn Ile Asp Ala Arg Gly Gln Asn Phe Glu Phe Ile Pro Phe Gly Ser
65                  70                  75                  80

Gly Arg Arg Ser Cys
                85

<210>  310
<211>  85
<212>  PRT
<213>  Nicotiana tabacum

<400>  310

Glu Thr Leu Arg Leu Tyr Pro Pro Val Pro Phe Leu Leu Pro His Glu
1               5                   10                  15

Ala Val Gln Asp Cys Lys Val Thr Gly Tyr His Ile Pro Lys Gly Thr
            20                  25                  30

Arg Leu Tyr Ile Asn Ala Trp Lys Val His Arg Asp Ser Glu Ile Trp
        35                  40                  45

Ser Glu Pro Glu Lys Phe Met Pro Asn Arg Phe Leu Thr Ser Lys Ala
    50                  55                  60

Asn Ile Asp Ala Arg Gly Gln Asn Phe Glu Phe Ile Pro Phe Gly Ser
65                  70                  75                  80

Gly Arg Arg Ser Cys
                85

<210>  311
<211>  81
<212>  PRT
<213>  Nicotiana tabacum

<400>  311

Glu Thr Phe Arg Met Tyr Pro Ala Gly Pro Leu Leu Val Pro His Glu
1               5                   10                  15

Ser Ser Glu Glu Thr Thr Val Gly Gly Tyr Arg Val Pro Gly Gly Thr
            20                  25                  30

Met Leu Leu Val Asn Leu Trp Ala Ile His Asn Asp Pro Lys Leu Trp
        35                  40                  45

Asp Glu Pro Arg Lys Phe Lys Pro Glu Arg Phe Glu Gly Leu Glu Gly
    50                  55                  60

Val Arg Asp Gly Tyr Lys Met Met Pro Phe Gly Ser Gly Arg Arg Ser

65                  70                  75                  80

Cys

<210> 312
<211> 81
<212> PRT
<213> Nicotiana tabacum

<400> 312

Glu Thr Phe Arg Met Tyr Pro Ala Gly Pro Leu Leu Val Pro His Glu
1               5                   10                  15

Ser Ser Glu Glu Thr Thr Val Gly Gly Tyr Arg Val Pro Gly Gly Thr
            20                  25                  30

Met Leu Leu Val Asn Leu Trp Ala Ile His Asn Asp Pro Lys Leu Trp
        35                  40                  45

Asp Glu Pro Arg Lys Phe Lys Pro Glu Arg Phe Gln Gly Leu Asp Gly
    50                  55                  60

Val Arg Asp Gly Tyr Lys Met Met Pro Phe Gly Ser Gly Arg Arg Ser
65                  70                  75                  80

Cys

<210> 313
<211> 83
<212> PRT
<213> Nicotiana tabacum

<400> 313

Glu Thr Met Arg Leu Tyr Thr Pro Ile Pro Leu Leu Leu Pro His Tyr
1               5                   10                  15

Ser Thr Lys Asp Cys Ile Val Glu Gly Tyr Asp Val Pro Lys His Thr
            20                  25                  30

Met Leu Phe Val Asn Ala Trp Ala Ile His Arg Asp Pro Lys Val Trp
        35                  40                  45

Glu Glu Pro Asp Lys Phe Lys Pro Glu Arg Phe Glu Ala Thr Glu Gly
    50                  55                  60

Glu Thr Glu Arg Phe Asn Tyr Lys Leu Val Pro Phe Gly Met Gly Arg
65                  70                  75                  80

Arg Ala Cys

<210>  314
<211>  83
<212>  PRT
<213>  Nicotiana tabacum

<400>  314

Glu Thr Met Arg Leu Tyr Thr Pro Ile Pro Leu Leu Leu Pro His Tyr
1               5                   10                  15

Ser Thr Lys Asp Cys Ile Val Glu Gly Tyr Asp Val Pro Lys His Thr
            20                  25                  30

Met Leu Phe Val Asn Ala Trp Ala Ile His Arg Asp Pro Lys Val Trp
        35                  40                  45

Glu Glu Pro Asp Lys Phe Lys Pro Glu Arg Phe Glu Ala Thr Glu Gly
    50                  55                  60

Glu Thr Glu Arg Phe Asn Tyr Lys Leu Val Pro Phe Gly Met Gly Arg
65                  70                  75                  80

Arg Ala Cys


<210>  315
<211>  83
<212>  PRT
<213>  Nicotiana tabacum

<400>  315

Glu Thr Met Arg Leu Tyr Thr Pro Ile Pro Leu Leu Leu Pro His Tyr
1               5                   10                  15

Ser Thr Lys Asp Cys Ile Val Glu Gly Tyr Asp Val Pro Lys His Thr
            20                  25                  30

Met Leu Phe Val Asn Ala Trp Ala Ile His Arg Asp Pro Lys Val Trp
        35                  40                  45

Glu Glu Pro Asp Lys Phe Lys Pro Glu Arg Phe Glu Ala Thr Glu Gly
    50                  55                  60

Glu Thr Glu Arg Phe Asn Tyr Lys Leu Val Pro Phe Gly Met Gly Arg
65                  70                  75                  80

Arg Ala Cys


<210>  316
<211>  83
<212>  PRT
<213>  Nicotiana tabacum

<400>  316

Glu Thr Met Arg Leu His Pro Val Ala Pro Met Leu Val Pro Arg Glu
1               5                   10                  15

Cys Arg Glu Asp Ile Lys Val Ala Gly Tyr Asp Val Gln Lys Gly Thr
            20                  25                  30

Arg Val Leu Val Ser Val Trp Thr Ile Gly Arg Asp Pro Thr Leu Trp
        35                  40                  45

Asp Glu Pro Glu Val Phe Lys Pro Glu Arg Phe His Glu Lys Ser Ile
    50                  55                  60

Asp Val Lys Gly His Asp Tyr Glu Leu Leu Pro Phe Gly Ala Gly Arg
65                  70                  75                  80

Arg Met Cys


<210>  317
<211>  83
<212>  PRT
<213>  Nicotiana tabacum

<400>  317

Glu Thr Met Arg Leu His Pro Val Ala Pro Met Leu Val Pro Arg Glu
1               5                   10                  15

Cys Arg Glu Asp Ile Lys Val Ala Gly Tyr Asp Val Gln Lys Gly Thr
            20                  25                  30

Arg Val Leu Val Ser Val Trp Thr Ile Gly Arg Asp Pro Thr Leu Trp
        35                  40                  45

Asp Glu Pro Glu Val Phe Lys Pro Glu Arg Phe His Glu Lys Ser Ile
    50                  55                  60

Asp Val Lys Gly His Asp Tyr Glu Leu Leu Pro Phe Gly Ala Gly Arg
65                  70                  75                  80

Arg Met Cys


<210>  318
<211>  83
<212>  PRT
<213>  Nicotiana tabacum

<400>  318

Glu Thr Met Arg Leu His Pro Val Ala Pro Met Leu Val Pro Arg Glu
1               5                   10                  15

Cys Arg Glu Asp Ile Lys Val Ala Gly Tyr Asp Val Gln Lys Gly Thr
            20                  25                  30

Arg Val Leu Val Ser Val Trp Thr Ile Gly Arg Asp Pro Thr Leu Trp
        35                  40                  45

Asp Glu Pro Glu Val Phe Lys Pro Glu Arg Phe His Glu Arg Ser Ile
    50                  55                  60

Asp Val Lys Gly His Asp Tyr Glu Leu Leu Pro Phe Gly Ala Gly Arg
65                  70                  75                  80

Arg Met Cys

<210>  319
<211>  83
<212>  PRT
<213>  Nicotiana tabacum

<400>  319

Glu Ala Leu Arg Leu His Pro Pro Thr Pro Leu Met Leu Pro His Arg
1               5                   10                  15

Ala Ser Ala Ser Val Lys Ile Gly Gly Tyr Asp Ile Pro Lys Gly Ser
            20                  25                  30

Ile Val His Val Asn Val Trp Ala Val Ala Arg Asp Pro Ala Val Trp
        35                  40                  45

Lys Asn Pro Leu Glu Phe Arg Pro Glu Arg Phe Leu Glu Glu Asp Val
    50                  55                  60

Asp Met Lys Gly His Asp Tyr Arg Leu Leu Pro Phe Gly Ala Gly Arg
65                  70                  75                  80

Arg Val Cys

<210>  320
<211>  83
<212>  PRT
<213>  Nicotiana tabacum

<400>  320

Glu Ala Leu Arg Leu His Pro Pro Thr Pro Leu Met Leu Pro His Lys
1               5                   10                  15

Ala Ser Ala Ser Val Lys Ile Gly Gly Tyr Asp Ile Pro Lys Gly Ser
            20                  25                  30

Ile Val His Val Asn Val Trp Ala Val Ala Arg Asp Pro Ala Val Trp
        35                  40                  45

Lys Asn Pro Leu Glu Phe Arg Pro Glu Arg Phe Leu Glu Glu Asp Val
    50                  55                  60

Asp Met Lys Gly His Asp Tyr Arg Leu Leu Pro Phe Gly Ala Gly Arg
65                  70                  75                  80

Arg Val Cys

<210>  321
<211>  83
<212>  PRT
<213>  Nicotiana tabacum

<400>  321

Glu Thr Leu Arg Leu His Pro Leu Gly Thr Met Leu Ala Pro His Cys
1               5                   10                  15

Ala Ile Glu Asp Cys Asn Val Ala Gly Tyr Asp Ile Gln Lys Gly Thr
            20                  25                  30

Thr Phe Leu Val Asn Val Trp Thr Ile Gly Arg Asp Pro Lys Tyr Trp
        35                  40                  45

Asp Arg Ala Gln Glu Phe Leu Pro Glu Arg Phe Leu Glu Asn Asp Ile
    50                  55                  60

Asp Met Asp Gly His Asn Phe Ala Phe Leu Pro Phe Gly Ser Gly Arg
65                  70                  75                  80

Arg Arg Cys

<210>  322
<211>  83
<212>  PRT
<213>  Nicotiana tabacum

<400>  322

Glu Thr Leu Arg Leu His Pro Leu Gly Thr Met Leu Ala Pro His Cys
1               5                   10                  15

Ala Ile Glu Asp Cys Asn Val Ala Gly Tyr Asp Ile Gln Lys Gly Thr
            20                  25                  30

Thr Val Leu Val Asn Val Trp Thr Ile Gly Arg Asp Pro Lys Tyr Trp
        35                  40                  45

Asp Arg Ala Gln Glu Phe Leu Pro Glu Arg Phe Leu Glu Asn Asp Ile
    50                  55                  60

Asp Met Asp Gly His Asn Phe Ala Phe Leu Pro Phe Gly Ser Gly Arg
65                  70                  75                  80

Arg Arg Cys

<210>  323
<211>  83
<212>  PRT
<213>  Nicotiana tabacum

<400>  323

Glu Thr Leu Arg Leu His Pro Leu Gly Thr Met Leu Ala Pro His Cys
1               5                   10                  15

Ala Ile Glu Asp Cys Asn Val Ala Gly Tyr Asp Ile Gln Lys Gly Thr
            20                  25                  30

Thr Val Leu Val Asn Val Trp Thr Ile Gly Arg Asp Pro Lys Tyr Trp
        35                  40                  45

Asp Arg Ala Gln Glu Phe Leu Pro Glu Arg Phe Leu Glu Asn Asp Ile
    50                  55                  60

Asp Met Asp Gly His Asn Phe Ala Phe Leu Pro Phe Gly Ser Gly Arg
65                  70                  75                  80

Arg Arg Cys

<210>  324
<211>  83
<212>  PRT
<213>  Nicotiana tabacum

<400>  324

Glu Val Leu Arg Leu Tyr Pro Ala Gly Tyr Val Ile Asn Arg Met Val
1               5                   10                  15

Asn Lys Glu Thr Lys Leu Gly Asn Leu Cys Leu Pro Ala Gly Val Gln
            20                  25                  30

Leu Val Leu Pro Thr Met Leu Leu Gln His Asp Thr Glu Ile Trp Gly
        35                  40                  45

Asp Asp Ala Met Glu Phe Asn Pro Glu Arg Phe Ser Asp Gly Ile Ser
    50                  55                  60

Lys Ala Thr Lys Gly Lys Leu Val Phe Phe Pro Phe Ser Trp Gly Pro
65                  70                  75                  80

Arg Ile Cys

<210> 325
<211> 83
<212> PRT
<213> Nicotiana tabacum

<400> 325

Glu Val Leu Arg Leu Tyr Pro Ala Gly Tyr Val Ile Asn Arg Met Val
1               5                   10                  15

Asn Lys Glu Thr Lys Leu Gly Asn Leu Cys Leu Pro Ala Gly Val Gln
            20                  25                  30

Leu Val Leu Pro Thr Met Leu Leu Gln His Asp Thr Glu Ile Trp Gly
        35                  40                  45

Asp Asp Ala Met Glu Phe Asn Pro Glu Arg Phe Ser Asp Gly Ile Ser
    50                  55                  60

Lys Ala Thr Lys Gly Lys Leu Val Phe Phe Pro Phe Ser Trp Gly Pro
65                  70                  75                  80

Arg Ile Cys

<210> 326
<211> 83
<212> PRT
<213> Nicotiana tabacum

<400> 326

Glu Val Leu Arg Leu Tyr Pro Ala Gly Tyr Ala Ile Asn Arg Met Val
1               5                   10                  15

Thr Lys Glu Thr Lys Leu Gly Asn Leu Cys Leu Pro Ala Gly Val Gln
            20                  25                  30

Leu Leu Leu Pro Thr Ile Leu Leu Gln His Asp Thr Glu Ile Trp Gly
        35                  40                  45

Asp Asp Ala Met Glu Phe Asn Pro Glu Arg Phe Ser Asp Gly Ile Ser
    50                  55                  60

Lys Ala Thr Lys Gly Lys Leu Val Phe Phe Pro Phe Ser Trp Gly Pro
65                  70                  75                  80

Arg Ile Cys

<210> 327
<211> 83
<212> PRT
<213> Nicotiana tabacum

<400>  327

Glu Ser Leu Arg Leu Tyr Pro Pro Ile Ala Thr Arg Thr Arg Arg Thr
1               5                   10                  15

Asn Glu Glu Thr Lys Leu Gly Glu Leu Asp Leu Pro Lys Gly Ala Leu
            20                  25                  30

Leu Phe Ile Pro Thr Ile Leu Leu His Leu Asp Lys Glu Ile Trp Gly
        35                  40                  45

Glu Asp Ala Asp Glu Phe Asn Pro Glu Arg Phe Ser Glu Gly Val Ala
    50                  55                  60

Lys Ala Thr Lys Gly Lys Met Thr Tyr Phe Pro Phe Gly Ala Gly Pro
65                  70                  75                  80

Arg Lys Cys


<210>  328
<211>  83
<212>  PRT
<213>  Nicotiana tabacum

<400>  328

Glu Ser Leu Arg Leu Tyr Pro Pro Ile Ala Thr Arg Thr Arg Arg Thr
1               5                   10                  15

Asn Glu Glu Thr Lys Leu Gly Glu Leu Asp Leu Pro Lys Gly Ala Leu
            20                  25                  30

Leu Phe Ile Pro Thr Ile Leu Leu His Leu Asp Arg Glu Ile Trp Gly
        35                  40                  45

Glu Asp Ala Asp Glu Phe Asn Pro Glu Arg Phe Ser Glu Gly Val Ala
    50                  55                  60

Lys Ala Thr Lys Gly Lys Met Thr Tyr Phe Pro Phe Gly Ala Gly Pro
65                  70                  75                  80

Arg Lys Cys


<210>  329
<211>  83
<212>  PRT
<213>  Nicotiana tabacum

<400>  329

Glu Thr Leu Arg Met His Pro Pro Ile Pro Leu Leu Val Pro Arg Glu
1               5                   10                  15

Cys Met Glu Asp Thr Lys Ile Asp Gly Tyr Asn Ile Pro Phe Lys Thr
            20                  25                  30

Arg Val Ile Val Asn Ala Trp Ala Ile Gly Arg Asp Pro Glu Ser Trp
        35                  40                  45

Asp Asp Pro Glu Ser Phe Met Pro Glu Arg Phe Glu Asn Ser Ser Ile
    50                  55                  60

Asp Phe Leu Gly Asn His His Gln Phe Ile Pro Phe Gly Ala Gly Arg
65                  70                  75                  80

Arg Ile Cys


<210>  330
<211>  83
<212>  PRT
<213>  Nicotiana tabacum

<400>  330

Glu Thr Leu Arg Met His Pro Pro Ile Pro Leu Leu Val Pro Arg Glu
1               5                   10                  15

Cys Met Glu Asp Thr Lys Ile Asp Gly Tyr Asn Ile Pro Phe Lys Thr
            20                  25                  30

Arg Val Ile Val Asn Ala Trp Ala Ile Gly Arg Asp Pro Glu Ser Trp
        35                  40                  45

Asp Asp Pro Glu Ser Phe Met Pro Glu Arg Phe Glu Asn Ser Ser Ile
    50                  55                  60

Asp Phe Leu Gly Asn His His Gln Phe Ile Pro Phe Gly Ala Gly Arg
65                  70                  75                  80

Arg Ile Cys


<210>  331
<211>  83
<212>  PRT
<213>  Nicotiana tabacum

<400>  331

Glu Thr Leu Arg Met His Pro Pro Ile Pro Leu Leu Val Pro Arg Glu
1               5                   10                  15

Cys Met Glu Asp Thr Lys Ile Asp Gly Tyr Asn Ile Pro Phe Lys Thr
            20                  25                  30

Arg Val Ile Val Asn Ala Trp Ala Ile Gly Arg Asp Pro Glu Ser Trp

35                  40                  45

Asp Asp Pro Glu Ser Phe Met Pro Glu Arg Phe Glu Asn Ser Ser Ile
    50                  55                  60

Asp Phe Leu Gly Asn His His Gln Phe Ile Pro Phe Gly Ala Gly Arg
65                  70                  75                  80

Arg Ile Cys

<210>  332
<211>  83
<212>  PRT
<213>  Nicotiana tabacum

<400>  332

Glu Thr Leu Arg Met His Pro Pro Ile Pro Leu Leu Val Pro Arg Glu
1               5                   10                  15

Cys Met Glu Asp Thr Lys Ile Asp Gly Tyr Asn Ile Pro Phe Lys Thr
            20                  25                  30

Arg Val Ile Val Asn Ala Trp Ala Ile Gly Arg Asp Pro Gln Ser Trp
        35                  40                  45

Asp Asp Pro Glu Ser Phe Thr Pro Glu Arg Phe Glu Asn Asn Ser Ile
    50                  55                  60

Asp Phe Leu Gly Asn His His Gln Phe Ile Pro Phe Gly Ala Gly Arg
65                  70                  75                  80

Arg Ile Cys

<210>  333
<211>  83
<212>  PRT
<213>  Nicotiana tabacum

<400>  333

Glu Thr Leu Arg Met His Pro Pro Ile Pro Leu Leu Val Pro Arg Glu
1               5                   10                  15

Cys Met Glu Asp Thr Lys Ile Asp Gly Tyr Asn Ile Pro Phe Lys Thr
            20                  25                  30

Arg Val Ile Val Asn Ala Trp Ala Ile Gly Arg Asp Pro Gln Ser Trp
        35                  40                  45

Asp Asp Pro Glu Ser Phe Thr Pro Glu Arg Phe Glu Asn Asn Ser Ile
    50                  55                  60

Asp Phe Leu Gly Asn His His Gln Phe Ile Pro Phe Gly Ala Gly Arg
65                  70                  75                  80

Arg Ile Cys

<210>  334
<211>  83
<212>  PRT
<213>  Nicotiana tabacum

<400>  334

Glu Thr Leu Arg Met His Pro Pro Val Pro Leu Leu Gly Pro Arg Glu
1               5                   10                  15

Cys Arg Asp Gln Thr Glu Ile Asp Gly Tyr Thr Val Pro Ile Lys Ala
            20                  25                  30

Arg Val Met Val Asn Ala Trp Ala Ile Gly Arg Asp Pro Glu Ser Trp
        35                  40                  45

Glu Asp Pro Glu Ser Phe Lys Pro Glu Arg Phe Glu Asn Thr Ser Val
    50                  55                  60

Asp Leu Thr Gly Asn His Tyr Gln Phe Ile Pro Phe Gly Ser Gly Arg
65                  70                  75                  80

Arg Met Cys

<210>  335
<211>  83
<212>  PRT
<213>  Nicotiana tabacum

<400>  335

Glu Thr Leu Arg Met His Pro Pro Val Pro Leu Leu Gly Pro Arg Glu
1               5                   10                  15

Cys Arg Asp Gln Thr Glu Ile Asp Gly Tyr Thr Val Pro Ile Lys Ala
            20                  25                  30

Arg Val Met Val Asn Ala Trp Ala Ile Gly Arg Asp Pro Glu Ser Trp
        35                  40                  45

Glu Asp Pro Glu Ser Phe Lys Pro Glu Arg Phe Glu Asn Thr Ser Val
    50                  55                  60

Asp Leu Thr Gly Asn His Tyr Gln Phe Ile Pro Phe Gly Ser Gly Arg
65                  70                  75                  80

Arg Met Cys

<210>  336
<211>  83
<212>  PRT
<213>  Nicotiana tabacum

<400>  336

Glu Thr Leu Arg Met His Pro Pro Val Pro Leu Leu Gly Pro Arg Glu
1               5                   10                  15

Cys Arg Asp Gln Thr Glu Ile Asp Gly Tyr Thr Val Pro Ile Lys Ala
            20                  25                  30

Arg Val Met Val Asn Ala Trp Ala Ile Gly Arg Asp Pro Glu Ser Trp
        35                  40                  45

Glu Asp Pro Glu Ser Phe Lys Pro Glu Arg Phe Glu Asn Thr Ser Val
    50                  55                  60

Asp Leu Thr Gly Asn His Tyr Gln Phe Ile Pro Phe Gly Ser Gly Arg
65                  70                  75                  80

Arg Met Cys


<210>  337
<211>  83
<212>  PRT
<213>  Nicotiana tabacum

<400>  337

Glu Thr Leu Arg Met His Pro Pro Val Pro Leu Leu Gly Pro Arg Glu
1               5                   10                  15

Cys Arg Glu Gln Thr Glu Ile Asp Gly Tyr Thr Val Pro Leu Lys Ala
            20                  25                  30

Arg Val Met Val Asn Ala Trp Ala Ile Gly Arg Asp Pro Glu Ser Trp
        35                  40                  45

Glu Asp Pro Glu Ser Phe Lys Pro Glu Arg Phe Glu Asn Ile Ser Val
    50                  55                  60

Asp Leu Thr Gly Asn His Tyr Gln Phe Ile Pro Phe Gly Ser Gly Arg
65                  70                  75                  80

Arg Met Cys


<210>  338
<211>  83
<212>  PRT

<213>  Nicotiana tabacum

<400>  338

Glu Thr Leu Arg Met His Pro Pro Val Pro Leu Leu Gly Pro Arg Glu
1               5                   10                  15

Cys Arg Glu Gln Thr Glu Ile Asp Gly Tyr Thr Val Pro Leu Lys Ala
            20                  25                  30

Arg Val Met Val Asn Ala Trp Ala Ile Gly Arg Asp Pro Glu Ser Trp
        35                  40                  45

Glu Asp Pro Glu Ser Phe Lys Pro Glu Arg Phe Glu Asn Ile Ser Val
    50                  55                  60

Asp Leu Thr Gly Asn His Tyr Gln Phe Ile Pro Phe Gly Ser Gly Arg
65                  70                  75                  80

Arg Met Cys


<210>  339
<211>  83
<212>  PRT
<213>  Nicotiana tabacum

<400>  339

Glu Thr Leu Arg Met His Pro Pro Val Pro Leu Leu Gly Pro Arg Glu
1               5                   10                  15

Cys Arg Glu Gln Thr Glu Ile Asp Gly Tyr Thr Val Pro Leu Lys Ala
            20                  25                  30

Arg Val Met Val Asn Ala Trp Ala Ile Gly Arg Asp Pro Glu Ser Trp
        35                  40                  45

Glu Asp Pro Glu Ser Phe Lys Pro Glu Arg Phe Glu Asn Ile Ser Val
    50                  55                  60

Asp Leu Thr Gly Asn His Tyr Gln Phe Ile Pro Phe Gly Ser Gly Arg
65                  70                  75                  80

Arg Met Cys


<210>  340
<211>  83
<212>  PRT
<213>  Nicotiana tabacum

<400>  340

Glu Thr Leu Arg Leu His Pro Pro Thr Pro Leu Leu Val Pro Arg Glu
1               5                   10                  15

Cys Arg Glu Glu Thr Glu Ile Glu Gly Phe Thr Ile Pro Leu Lys Ser
            20                  25                  30

Lys Val Leu Val Asn Val Trp Ala Ile Gly Arg Asp Pro Glu Asn Trp
        35                  40                  45

Lys Asn Pro Glu Cys Phe Ile Pro Glu Arg Phe Glu Asn Ser Ser Ile
    50                  55                  60

Glu Phe Thr Gly Asn His Phe Gln Leu Leu Pro Phe Gly Ala Gly Arg
65                  70                  75                  80

Arg Ile Cys

<210> 341
<211> 83
<212> PRT
<213> Nicotiana tabacum

<400> 341

Glu Thr Leu Arg Leu His Pro Pro Thr Pro Leu Leu Val Pro Arg Glu
1               5                   10                  15

Cys Arg Glu Glu Thr Glu Ile Glu Gly Phe Thr Ile Pro Leu Lys Ser
            20                  25                  30

Lys Val Leu Val Asn Val Trp Ala Ile Gly Arg Asp Pro Glu Asn Trp
        35                  40                  45

Lys Asn Pro Glu Cys Phe Ile Pro Glu Arg Phe Glu Asn Ser Ser Ile
    50                  55                  60

Glu Phe Thr Gly Asn His Phe Gln Leu Leu Pro Phe Gly Ala Gly Arg
65                  70                  75                  80

Arg Ile Cys

<210> 342
<211> 83
<212> PRT
<213> Nicotiana tabacum

<400> 342

Glu Thr Leu Arg Leu His Pro Pro Val Pro Leu Leu Leu Pro Arg Glu
1               5                   10                  15

Cys Arg Glu Glu Thr Asn Ile Asn Gly Tyr Thr Ile Pro Val Lys Thr
            20                  25                  30

Lys Val Met Val Asn Val Trp Ala Leu Gly Arg Asp Pro Lys Tyr Trp
        35                  40                  45

Asn Asp Ala Glu Thr Phe Met Pro Glu Arg Phe Glu Gln Cys Ser Lys
    50                  55                  60

Asp Phe Val Gly Asn Asn Phe Glu Tyr Leu Pro Phe Gly Gly Gly Arg
65                  70                  75                  80

Arg Ile Cys

<210>  343
<211>  83
<212>  PRT
<213>  Nicotiana tabacum

<400>  343

Glu Thr Leu Arg Leu His Pro Pro Val Pro Leu Leu Leu Pro Arg Glu
1               5                   10                  15

Cys Arg Glu Glu Thr Asn Ile Asn Gly Tyr Thr Ile Pro Val Lys Thr
            20                  25                  30

Lys Val Met Val Asn Val Trp Ala Leu Gly Arg Asp Pro Lys Tyr Trp
        35                  40                  45

Asn Asp Ala Glu Thr Phe Met Pro Glu Arg Phe Glu Gln Cys Ser Lys
    50                  55                  60

Asp Phe Val Gly Asn Asn Phe Glu Tyr Leu Pro Phe Gly Gly Gly Arg
65                  70                  75                  80

Arg Ile Cys

<210>  344
<211>  83
<212>  PRT
<213>  Nicotiana tabacum

<400>  344

Glu Thr Leu Arg Leu His Pro Pro Val Pro Leu Leu Leu Pro Arg Glu
1               5                   10                  15

Cys Arg Glu Glu Thr Asn Ile Asn Gly Tyr Thr Ile Pro Val Lys Thr
            20                  25                  30

Lys Val Met Val Asn Val Trp Ala Leu Gly Arg Asp Pro Lys Tyr Trp
        35                  40                  45

Asn Asp Ala Glu Thr Phe Met Pro Glu Arg Phe Glu Gln Cys Ser Lys
    50                  55                  60

Asp Phe Val Gly Asn Asn Phe Glu Tyr Leu Pro Phe Gly Gly Gly Arg
65                  70                  75                  80

Arg Ile Cys

<210>  345
<211>  83
<212>  PRT
<213>  Nicotiana tabacum

<400>  345

Glu Thr Leu Arg Leu His Pro Pro Val Pro Leu Leu Leu Pro Arg Glu
1               5                   10                  15

Cys Arg Glu Glu Thr Asn Ile Asn Gly Tyr Thr Ile Pro Val Lys Thr
            20                  25                  30

Lys Val Met Val Asn Val Trp Ala Leu Gly Arg Asp Pro Lys Tyr Trp
        35                  40                  45

Asn Asp Ala Glu Thr Phe Met Pro Glu Arg Phe Glu Gln Cys Ser Lys
    50                  55                  60

Asp Phe Val Gly Asn Asn Phe Glu Tyr Leu Pro Phe Gly Gly Gly Arg
65                  70                  75                  80

Arg Ile Cys

<210>  346
<211>  83
<212>  PRT
<213>  Nicotiana tabacum

<400>  346

Glu Thr Leu Arg Leu His Pro Pro Val Pro Leu Leu Leu Pro Arg Glu
1               5                   10                  15

Cys Arg Glu Glu Thr Asn Ile Asn Gly Tyr Thr Ile Pro Val Lys Thr
            20                  25                  30

Lys Val Met Val Asn Val Trp Ala Leu Gly Arg Asp Pro Lys Tyr Trp
        35                  40                  45

Asp Asp Ala Glu Thr Phe Lys Pro Glu Arg Phe Glu Gln Cys Ser Lys
    50                  55                  60

Asp Phe Val Gly Asn Asn Phe Glu Tyr Leu Pro Phe Gly Gly Gly Arg
65                  70                  75                  80

Arg Ile Cys

<210> 347
<211> 83
<212> PRT
<213> Nicotiana tabacum

<400> 347

Glu Thr Leu Arg Leu His Pro Pro Ile Pro Leu Leu Leu His Glu Thr
1               5                   10                  15

Ala Glu Glu Ser Thr Val Ser Gly Tyr His Ile Pro Ala Lys Ser His
            20                  25                  30

Val Ile Ile Asn Ser Phe Ala Ile Gly Arg Asp Lys Asn Ser Trp Glu
        35                  40                  45

Asp Pro Glu Thr Tyr Lys Pro Ser Arg Phe Leu Lys Glu Gly Val Pro
    50                  55                  60

Asp Phe Lys Gly Gly Asn Phe Glu Phe Ile Pro Phe Gly Ser Gly Arg
65                  70                  75                  80

Arg Ser Cys

<210> 348
<211> 83
<212> PRT
<213> Nicotiana tabacum

<400> 348

Glu Thr Leu Arg Leu His Pro Pro Ile Pro Leu Leu Leu His Glu Thr
1               5                   10                  15

Ala Glu Glu Ser Thr Val Ser Gly Tyr His Ile Pro Ala Lys Ser His
            20                  25                  30

Val Ile Ile Asn Ser Phe Ala Ile Gly Arg Asp Lys Asn Ser Trp Glu
        35                  40                  45

Asp Pro Glu Thr Tyr Lys Pro Ser Arg Phe Leu Lys Glu Gly Val Pro
    50                  55                  60

Asp Phe Lys Gly Gly Asn Phe Glu Phe Ile Pro Phe Gly Ser Gly Arg
65                  70                  75                  80

Arg Ser Cys

<210> 349
<211> 83

<212>  PRT
<213>  Nicotiana tabacum

<400>  349

Glu Ile Phe Arg Leu Tyr Pro Pro Ala Pro Leu Leu Val Pro Arg Glu
1               5                   10                  15

Ser Met Glu Lys Thr Ile Leu Glu Gly Tyr Glu Ile Arg Pro Arg Thr
            20                  25                  30

Ile Val His Val Asn Ala Trp Ala Ile Ala Arg Asp Pro Glu Ile Trp
        35                  40                  45

Glu Asn Pro Asp Glu Phe Ile Pro Glu Arg Phe Leu Asn Ser Ser Ile
    50                  55                  60

Asp Tyr Lys Gly Gln Asp Phe Glu Leu Leu Pro Phe Gly Ala Gly Arg
65                  70                  75                  80

Arg Gly Cys


<210>  350
<211>  83
<212>  PRT
<213>  Nicotiana tabacum

<400>  350

Glu Ile Phe Arg Leu Tyr Pro Pro Ala Pro Leu Leu Val Pro Arg Glu
1               5                   10                  15

Ser Met Glu Lys Thr Ile Leu Glu Gly Tyr Glu Ile Arg Pro Arg Thr
            20                  25                  30

Ile Val His Val Asn Ala Trp Ala Ile Ala Arg Asp Pro Glu Ile Trp
        35                  40                  45

Glu Asn Pro Asp Glu Phe Ile Pro Glu Arg Phe Leu Asn Ser Ser Ile
    50                  55                  60

Asp Tyr Lys Gly Gln Asp Phe Glu Leu Leu Pro Phe Gly Ala Gly Arg
65                  70                  75                  80

Arg Gly Cys


<210>  351
<211>  83
<212>  PRT
<213>  Nicotiana tabacum

<400>  351

Glu Ile Phe Arg Leu Tyr Pro Pro Ala Pro Leu Leu Val Pro Arg Glu

1               5                   10                  15

Ser Met Glu Lys Thr Ile Leu Glu Gly Tyr Glu Ile Arg Pro Arg Thr
            20                  25                  30

Ile Val His Val Asn Ala Trp Ala Ile Ala Arg Asp Pro Glu Ile Trp
        35                  40                  45

Glu Asn Pro Asp Glu Phe Ile Pro Glu Arg Phe Leu Asn Ser Ser Ile
    50                  55                  60

Asp Tyr Lys Gly Gln Asp Phe Glu Leu Leu Pro Phe Gly Ala Gly Arg
65                  70                  75                  80

Arg Gly Cys

<210>  352
<211>  83
<212>  PRT
<213>  Nicotiana tabacum

<400>  352

Glu Ile Phe Arg Leu Tyr Pro Pro Ala Pro Leu Leu Val Pro Arg Glu
1               5                   10                  15

Ser Met Glu Lys Thr Ile Leu Glu Gly Tyr Glu Ile Arg Pro Arg Thr
            20                  25                  30

Ile Val His Val Asn Ala Trp Ala Ile Ala Arg Asp Pro Glu Ile Trp
        35                  40                  45

Glu Asn Pro Asp Glu Phe Ile Pro Glu Arg Phe Leu Asn Ser Ser Thr
    50                  55                  60

Asp Tyr Lys Gly Gln Asp Phe Glu Leu Leu Pro Phe Gly Ala Gly Arg
65                  70                  75                  80

Arg Gly Cys

<210>  353
<211>  85
<212>  PRT
<213>  Nicotiana tabacum

<400>  353

Glu Ala Leu Arg Leu Arg Met Ala Ile Pro Leu Leu Val Pro His Met
1               5                   10                  15

Asn Leu His Asp Ala Lys Leu Gly Gly Phe Asp Ile Pro Ala Glu Ser
            20                  25                  30

Lys Ile Leu Val Asn Ala Trp Trp Leu Ala Asn Asn Pro Ala His Trp
        35                  40                  45

Lys Lys Pro Glu Glu Phe Arg Pro Glu Arg Phe Phe Glu Glu Glu Lys
    50                  55                  60

His Val Glu Ala Asn Gly Asn Asp Phe Arg Tyr Leu Pro Phe Gly Val
65                  70                  75                  80

Gly Arg Arg Ser Cys
                85

<210>  354
<211>  85
<212>  PRT
<213>  Nicotiana tabacum

<400>  354

Glu Ala Leu Arg Leu Arg Met Ala Ile Pro Leu Leu Val Pro His Met
1               5                   10                  15

Asn Leu His Asp Ala Lys Leu Gly Gly Leu Asp Ile Pro Ala Glu Ser
            20                  25                  30

Lys Ile Leu Val Asn Ala Trp Trp Leu Ala Asn Asn Pro Ala His Trp
        35                  40                  45

Lys Lys Pro Glu Glu Phe Arg Pro Glu Arg Phe Phe Glu Glu Glu Lys
    50                  55                  60

His Val Glu Ala Asn Gly Asn Asp Phe Arg Tyr Leu Pro Phe Gly Val
65                  70                  75                  80

Gly Arg Arg Ser Cys
                85

<210>  355
<211>  85
<212>  PRT
<213>  Nicotiana tabacum

<400>  355

Glu Thr Leu Arg Leu Arg Met Ala Ile Pro Leu Leu Val Pro His Met
1               5                   10                  15

Asn Leu His Asp Ala Lys Leu Gly Gly Phe Asp Ile Pro Ala Glu Ser
            20                  25                  30

Lys Ile Leu Val Asn Ala Trp Trp Leu Ala Asn Asn Pro Ala His Trp
        35                  40                  45

Lys Lys Pro Glu Glu Phe Arg Pro Glu Arg Phe Phe Glu Glu Glu Lys

50                  55                  60

His Val Glu Ala Asn Gly Asn Asp Phe Arg Tyr Leu Pro Phe Gly Val
65                  70                  75                  80

Gly Arg Arg Ser Cys
                85

<210>  356
<211>  82
<212>  PRT
<213>  Nicotiana tabacum

<400>  356

Glu Ser Leu Arg Leu Tyr Ser Pro Val Val Ser Leu Ile Arg Arg Pro
1               5                   10                  15

Asn Glu Asp Ala Ile Leu Gly Asn Val Ser Leu Pro Glu Gly Val Leu
            20                  25                  30

Leu Ser Leu Pro Val Ile Leu Leu His His Asp Glu Glu Ile Trp Gly
        35                  40                  45

Lys Asp Lys Lys Phe Asn Pro Glu Arg Phe Arg Asp Gly Val Ser Ser
    50                  55                  60

Ala Thr Lys Gly Gln Val Thr Phe Phe Pro Phe Thr Trp Gly Pro Arg
65                  70                  75                  80

Ile Cys

<210>  357
<211>  83
<212>  PRT
<213>  Nicotiana tabacum

<400>  357

Glu Ser Leu Arg Leu Tyr Pro Pro Val Val Thr Leu Thr Arg Arg Pro
1               5                   10                  15

Lys Glu Asp Thr Val Leu Gly Asp Val Ser Leu Pro Ala Gly Val Leu
            20                  25                  30

Ile Ser Leu Pro Val Ile Leu Leu His His Asp Glu Glu Ile Trp Gly
        35                  40                  45

Lys Asp Ala Lys Lys Phe Lys Pro Glu Arg Phe Arg Asp Gly Val Ser
    50                  55                  60

Ser Ala Thr Lys Gly Gln Val Thr Phe Phe Pro Phe Thr Trp Gly Pro
65                  70                  75                  80

Arg Ile Cys

<210> 358
<211> 18
<212> DNA
<213> Nicotiana tabacum

<220>
<221> misc_feature
<222> (4)..(6)
<223> n = deoxyinosine

<220>
<221> misc_feature
<222> (9)..(9)
<223> n = deoxyinosine

<220>
<221> misc_feature
<222> (15)..(15)
<223> n = deoxyinosine

<400> 358
ttynnnccng armgntty 18

<210> 359
<211> 20
<212> DNA
<213> Nicotiana tabacum

<220>
<221> misc_feature
<222> (3)..(3)
<223> n = deoxyinosine

<220>
<221> misc_feature
<222> (6)..(6)
<223> n = deoxyinosine

<220>
<221> misc_feature
<222> (9)..(9)
<223> n = deoxyinosine

<220>
<221> misc_feature
<222> (10)..(12)
<223> n = deoxyinosine

<220>
<221> misc_feature
<222> (18)..(18)
<223> n = deoxyinosine

<400> 359
ggnmgnmgnn nntgyccngs 20

<210> 360
<211> 17
<212> DNA

<213>  Nicotiana tabacum

<400>  360
ttyaarcctg agagatt                                                    17


<210>  361
<211>  15
<212>  DNA
<213>  Nicotiana tabacum

<400>  361
ccagaragat tcttg                                                      15


<210>  362
<211>  17
<212>  DNA
<213>  Nicotiana tabacum

<400>  362
ggrmgrmgra tgtgycc                                                    17


<210>  363
<211>  19
<212>  DNA
<213>  Nicotiana tabacum


<220>
<221>  misc_feature
<222>  (19)..(19)
<223>  n = a, t, c, or g

<400>  363
tttttttttt ttttttttn                                                  19


<210>  364
<211>  20
<212>  DNA
<213>  Nicotiana tabacum

<400>  364
attatgctga gtgatatccc                                                 20


<210>  365
<211>  18
<212>  DNA
<213>  Nicotiana tabacum

<400>  365
atttaggtga cactatag                                                   18


<210>  366
<211>  7
<212>  PRT
<213>  Artificial Sequence

<220>
<223>  conserved p450 motif


<220>
<221>  MISC_FEATURE

<222>  (2)..(2)
<223>  Xaa = any amino acid

<220>
<221>  MISC_FEATURE
<222>  (4)..(4)
<223>  Xaa = any amino acid

<220>
<221>  MISC_FEATURE
<222>  (6)..(6)
<223>  Xaa = any amino acid

<220>
<221>  MISC_FEATURE
<222>  (7)..(7)
<223>  Xaa = gly or ala

<400>  366

Gly Xaa Arg Xaa Cys Xaa Xaa
1               5


<210>  367
<211>  7
<212>  PRT
<213>  Artificial Sequence

<220>
<223>  conserved p450 motif


<220>
<221>  MISC_FEATURE
<222>  (1)..(1)
<223>  Xaa =  Glu or Lys

<220>
<221>  MISC_FEATURE
<222>  (2)..(2)
<223>  Xaa =  any amino acid

<220>
<221>  MISC_FEATURE
<222>  (3)..(3)
<223>  Xaa =  any amino acid

<220>
<221>  MISC_FEATURE
<222>  (5)..(5)
<223>  Xaa =  any amino acid

<220>
<221>  MISC_FEATURE
<222>  (6)..(6)
<223>  Xaa =  any amino acid

<220>
<221>  MISC_FEATURE
<222>  (7)..(7)
<223>  Xaa =  Arg, Thr, Ser or Met

<400>  367

Xaa Xaa Xaa Arg Xaa Xaa Xaa
1               5

<210>  368
<211>  4
<212>  PRT
<213>  Artificial Sequence

<220>
<223>  Artificially synthesized peptide


<220>
<221>  misc_feature
<222>  (2)..(2)
<223>  Xaa can be any naturally occurring amino acid

<220>
<221>  misc_feature
<222>  (4)..(4)
<223>  Xaa can be any naturally occurring amino acid

<400>  368

Gly Xaa Arg Xaa
1


<210>  369
<211>  21
<212>  PRT
<213>  Artificial Sequence

<220>
<223>  artificially synthesized peptide

<400>  369

Asp Ile Asp Gly Ser Lys Ser Lys Leu Val Lys Ala His Arg Lys Ile
1               5                   10                  15


Asp Glu Ile Leu Gly
            20


<210>  370
<211>  20
<212>  PRT
<213>  Artificial Sequence

<220>
<223>  Artificially synthesized peptide

<400>  370

Arg Asp Ala Phe Arg Glu Lys Glu Thr Phe Asp Glu Asn Asp Val Glu
1               5                   10                  15


Glu Leu Asn Tyr
            20


<210>  371
<211>  22
<212>  PRT
<213>  Artificial Sequence

<220>

<223>  Artificially synthesized peptide

<400>  371

Phe Lys Asn Asn Gly Asp Glu Asp Arg His Phe Ser Gln Lys Leu Gly
1               5                   10                  15

Asp Leu Ala Asp Lys Tyr
            20

<210>  372
<211>  6
<212>  PRT
<213>  Artificial Sequence

<220>
<223>  conserved p450 motif

<220>
<221>  MISC_FEATURE
<222>  (2)..(2)
<223>  Xaa = any amino acid

<400>  372

Phe Xaa Pro Glu Arg Phe
1               5

<210>  373
<211>  7
<212>  PRT
<213>  Artificial Sequence

<220>
<223>  conserved p450 motif

<220>
<221>  MISC_FEATURE
<222>  (4)..(4)
<223>  Xaa = any amino acid

<220>
<221>  MISC_FEATURE
<222>  (7)..(7)
<223>  Xaa = ala or gly

<400>  373

Gly Arg Arg Xaa Cys Pro Xaa
1               5

<210>  374
<211>  7
<212>  PRT
<213>  Artificial Sequence

<220>
<223>  conserved p450 motif

<220>
<221>  MISC_FEATURE

<222>  (2)..(2)
<223>  Xaa = any amino acid

<220>
<221>  MISC_FEATURE
<222>  (4)..(4)
<223>  Xaa = any amino acid

<220>
<221>  MISC_FEATURE
<222>  (7)..(7)
<223>  Xaa = ala or gly

<400>  374

Gly Xaa Arg Xaa Cys Pro Xaa
1               5


<210>  375
<211>  6
<212>  PRT
<213>  Artificial Sequence

<220>
<223>  conserved p450 motif


<220>
<221>  MISC_FEATURE
<222>  (2)..(2)
<223>  Xaa =  any amino acid

<220>
<221>  MISC_FEATURE
<222>  (5)..(5)
<223>  Xaa =  any amino acid

<400>  375

Pro Xaa Arg Phe Xaa Phe
1               5


<210>  376
<211>  5
<212>  PRT
<213>  Artificial Sequence

<220>
<223>  conserved p450 motif


<220>
<221>  MISC_FEATURE
<222>  (2)..(2)
<223>  Xaa =  any amino acid

<220>
<221>  MISC_FEATURE
<222>  (4)..(4)
<223>  Xaa =  any amino acid

<400>  376

Gly Xaa Arg Xaa Cys
1               5

<210>  377
<211>  509
<212>  PRT
<213>  Nicotiana tabacum

<400>  377

Met Glu Gly Thr Asn Leu Thr Thr Tyr Ala Ala Val Phe Leu Gly Thr
1               5                   10                  15

Leu Phe Leu Leu Phe Leu Ser Lys Leu Leu Arg Gln Arg Lys Leu Asn
            20                  25                  30

Leu Pro Pro Gly Pro Lys Pro Trp Pro Ile Ile Gly Asn Leu Asn Leu
        35                  40                  45

Ile Gly Asn Leu Pro Tyr Arg Ser Ile His Glu Leu Ser Leu Lys Tyr
    50                  55                  60

Gly Pro Val Met Gln Leu Gln Phe Gly Ser Phe Pro Val Val Val Gly
65                  70                  75                  80

Ser Ser Val Glu Met Ala Lys Ile Phe Leu Lys Ser Met Asp Ile Asn
                85                  90                  95

Phe Val Gly Arg Pro Lys Thr Ala Ala Gly Lys Tyr Thr Thr Tyr Asn
            100                 105                 110

Tyr Ser Asp Ile Thr Trp Ser Pro Tyr Gly Pro Tyr Trp Arg Gln Ala
        115                 120                 125

Arg Arg Met Cys Leu Thr Glu Leu Phe Ser Thr Lys Cys Leu Asp Ser
    130                 135                 140

Tyr Glu Tyr Ile Arg Ala Glu Glu Leu His Ser Leu Leu His Asn Leu
145                 150                 155                 160

Asn Lys Ile Ser Gly Lys Pro Ile Val Leu Lys Asp Tyr Leu Thr Thr
                165                 170                 175

Leu Ser Leu Asn Val Ile Ser Arg Met Val Leu Gly Lys Arg Tyr Leu
            180                 185                 190

Asp Glu Ser Glu Asn Ser Phe Val Asn Pro Glu Glu Phe Lys Lys Met
        195                 200                 205

Leu Asp Glu Leu Phe Leu Leu Asn Gly Val Leu Asn Ile Gly Asp Ser
    210                 215                 220

Ile Pro Trp Ile Asp Phe Met Asp Leu Gln Gly Tyr Val Lys Arg Met
225                 230                 235                 240

Lys Val Val Ser Lys Lys Phe Asp Lys Phe Leu Glu His Val Ile Asp
                245                 250                 255

Glu His Asn Ile Arg Arg Asn Gly Val Glu Asn Tyr Val Ala Lys Asp
            260                 265                 270

Met Glu Asp Val Leu Leu Gln Leu Ala Asp Asp Pro Lys Leu Glu Val
        275                 280                 285

Lys Leu Glu Arg His Gly Val Lys Ala Phe Thr Gln Asp Met Leu Ala
    290                 295                 300

Gly Gly Thr Glu Ser Ser Ala Val Thr Val Glu Trp Ala Ile Ser Glu
305                 310                 315                 320

Leu Ser Lys Lys Pro Glu Ile Phe Lys Lys Ala Thr Glu Glu Leu Asp
                325                 330                 335

Arg Val Ile Gly Gln Asn Arg Trp Val Gln Glu Lys Asp Ile Pro Asn
            340                 345                 350

Leu Pro Tyr Ile Glu Ala Ile Val Lys Glu Thr Met Arg Leu His Pro
        355                 360                 365

Val Ala Pro Met Lys Ala Thr Glu Cys Arg Glu Asn Ser Lys Val Ala
    370                 375                 380

Gly Tyr Asp Val Gln Lys Gly Thr Arg Val Leu Val Ser Val Trp Thr
385                 390                 395                 400

Ile Gly Arg Asp Pro Thr Leu Trp Asp Glu Pro Glu Val Phe Lys Pro
                405                 410                 415

Glu Arg Phe His Glu Lys Ala Ser Ile Asp Val Lys Gly His Asp Tyr
            420                 425                 430

Glu Leu Leu Pro Phe Gly Val Gly Arg Arg Met Cys Pro Gly Tyr Ser
        435                 440                 445

Leu Gly Leu Lys Val Ile Gln Ala Ser Leu Ala Asn Leu Leu His Gly
    450                 455                 460

Phe Asn Trp Ser Leu Pro Asp Asn Met Thr Pro Glu Asp Leu Asn Met
465                 470                 475                 480

Asp Glu Ile Phe Gly Leu Ser Thr Pro Lys Lys Phe Pro Leu Ala Thr
                485                 490                 495

Val Ile Glu Pro Arg Leu Ser Pro Lys Leu Tyr Ser Val
            500                 505

<210>   378
<211>   509
<212>   PRT
<213>   Nicotiana tabacum

<400>   378

Met Glu Gly Thr Asn Leu Thr Thr Tyr Ala Ala Val Phe Leu Gly Thr
1               5                   10                  15

Leu Phe Leu Leu Leu Leu Ser Lys Phe Leu Arg Gln Arg Lys Leu Asn
            20                  25                  30

Leu Pro Pro Gly Pro Lys Pro Trp Pro Ile Ile Gly Asn Leu Asn Leu
        35                  40                  45

Ile Gly Asn Leu Pro His Arg Ser Ile His Glu Leu Ser Leu Lys Tyr
    50                  55                  60

Gly Pro Ile Met Gln Leu Gln Phe Gly Thr Phe Pro Val Val Val Gly
65                  70                  75                  80

Ser Ser Val Glu Met Ala Lys Val Phe Leu Lys Ser Met Asp Ile Asn
                85                  90                  95

Phe Val Gly Arg Pro Lys Thr Ala Ala Gly Lys Tyr Thr Thr Tyr Asn
            100                 105                 110

Tyr Ser Asp Ile Thr Trp Ser Pro Tyr Gly Pro Tyr Trp Arg Gln Ala
        115                 120                 125

Arg Arg Met Cys Leu Met Glu Leu Phe Ser Thr Lys Arg Leu Asp Ser
    130                 135                 140

Tyr Glu Tyr Ile Arg Ala Glu Glu Leu His Ser Leu Leu His Asn Leu
145                 150                 155                 160

Asn Lys Ile Ser Gly Lys Pro Ile Val Leu Lys Asp Tyr Leu Thr Thr
                165                 170                 175

Leu Ser Leu Asn Val Ile Ser Arg Met Val Leu Gly Lys Arg Tyr Leu
            180                 185                 190

Asp Glu Ser Glu Asn Ser Ile Val Thr Pro Glu Glu Phe Lys Lys Met
        195                 200                 205

Leu Asp Glu Leu Phe Leu Leu Asn Gly Val Leu Asn Ile Gly Asp Ser
    210                 215                 220

Ile Pro Trp Ile Asp Phe Met Asp Leu Gln Gly Tyr Val Lys Arg Met
225                 230                 235                 240

Lys Phe Val Ser Lys Lys Phe Asp Lys Phe Leu Glu His Val Ile Asp

245                 250                 255

Glu His Asn Val Arg Arg Asn Gly Val Glu Asn Tyr Ile Ala Lys Asp
            260                 265                 270

Met Val Asp Val Leu Leu Gln Leu Ala Asp Asp Pro Thr Leu Glu Val
        275                 280                 285

Lys Leu Glu Arg His Gly Val Lys Ala Phe Thr Gln Asp Met Leu Ala
    290                 295                 300

Gly Gly Thr Glu Ser Ser Ala Val Thr Val Glu Trp Ala Ile Ser Glu
305                 310                 315                 320

Leu Leu Lys Lys Pro Glu Ile Phe Lys Lys Ala Thr Glu Glu Leu Asp
                325                 330                 335

Arg Val Ile Gly Gln Asn Arg Trp Val Gln Glu Lys Asp Ile Pro Asn
            340                 345                 350

Leu Pro Tyr Ile Glu Ala Ile Val Lys Glu Thr Met Arg Leu His Pro
        355                 360                 365

Val Ala Pro Met Leu Val Pro Arg Glu Cys Arg Glu Asp Cys Lys Val
    370                 375                 380

Ala Gly Tyr Asp Val Lys Lys Gly Thr Arg Val Leu Val Ser Val Trp
385                 390                 395                 400

Thr Ile Gly Arg Asp Pro Thr Leu Trp Asp Glu Pro Glu Ala Phe Lys
                405                 410                 415

Pro Glu Arg Phe His Glu Lys Ser Ile Asp Val Lys Gly His Asp Phe
            420                 425                 430

Glu Leu Leu Pro Phe Gly Ala Gly Arg Arg Met Cys Pro Gly Tyr Asn
        435                 440                 445

Leu Gly Leu Lys Val Ile Gln Ala Ser Leu Ala Asn Leu Ile His Gly
    450                 455                 460

Phe Asn Trp Ser Leu Pro Asp Asn Met Thr Pro Glu Asp Leu Asp Met
465                 470                 475                 480

Asp Glu Ile Phe Gly Leu Ser Thr Pro Lys Lys Phe Pro Leu Ala Thr
                485                 490                 495

Val Ile Glu Pro Arg Leu Ser Pro Lys Leu Tyr Ser Val
            500                 505

<210>  379

<211>  473
<212>  PRT
<213>  Nicotiana tabacum

<400>  379

Met Gln Phe Phe Ser Leu Val Ser Ile Phe Leu Phe Leu Ala Phe Leu
1               5                   10                  15

Phe Leu Leu Arg Lys Trp Lys Asn Ser Asn Ser Gln Ser Lys Lys Leu
            20                  25                  30

Pro Pro Gly Pro Trp Lys Ile Pro Ile Leu Gly Ser Met Leu His Met
        35                  40                  45

Ile Gly Gly Glu Pro His His Val Leu Arg Asp Leu Ala Lys Lys Tyr
    50                  55                  60

Gly Pro Leu Met His Leu Gln Leu Gly Glu Ile Ser Ala Val Val Val
65                  70                  75                  80

Thr Ser Arg Asp Met Ala Lys Glu Val Leu Lys Thr His Asp Val Val
                85                  90                  95

Phe Ala Ser Arg Pro Lys Ile Val Ala Met Asp Ile Ile Cys Tyr Asn
            100                 105                 110

Gln Ser Asp Ile Ala Phe Ser Pro Tyr Gly Asp His Trp Arg Gln Met
        115                 120                 125

Arg Lys Ile Cys Val Met Glu Leu Leu Asn Ala Lys Asn Val Arg Ser
    130                 135                 140

Phe Ser Ser Ile Arg Arg Asp Glu Val Val Arg Leu Ile Asp Ser Ile
145                 150                 155                 160

Arg Ser Asp Ser Ser Ser Gly Glu Leu Val Asn Phe Thr Gln Arg Ile
                165                 170                 175

Ile Trp Phe Ala Ser Ser Met Thr Cys Arg Ser Ala Phe Gly Gln Val
            180                 185                 190

Leu Lys Gly Gln Asp Ile Phe Ala Lys Lys Ile Arg Glu Val Ile Gly
        195                 200                 205

Leu Ala Glu Gly Phe Asp Val Val Asp Ile Phe Pro Thr Tyr Lys Phe
    210                 215                 220

Leu His Val Leu Ser Gly Met Lys Arg Lys Leu Leu Asn Ala His Leu
225                 230                 235                 240

Lys Val Asp Ala Ile Val Glu Asp Val Ile Asn Glu His Lys Lys Asn
                245                 250                 255

Leu Ala Ala Gly Lys Ser Asn Gly Ala Leu Glu Asp Met Phe Ala Ala
            260                 265                 270

Gly Thr Glu Thr Ser Ser Thr Thr Thr Val Trp Ala Met Ala Glu Met
        275                 280                 285

Met Lys Asn Pro Ser Val Phe Thr Lys Ala Gln Ala Glu Val Arg Glu
    290                 295                 300

Ala Phe Arg Asp Lys Val Ser Phe Asp Glu Asn Asp Val Glu Glu Leu
305                 310                 315                 320

Lys Tyr Leu Lys Leu Val Ile Lys Glu Thr Leu Arg Leu His Pro Pro
                325                 330                 335

Ser Pro Leu Leu Val Pro Arg Glu Cys Arg Glu Asp Thr Asp Ile Asn
            340                 345                 350

Gly Tyr Thr Ile Pro Ala Lys Thr Lys Val Met Val Asn Val Trp Ala
        355                 360                 365

Leu Gly Arg Asp Pro Lys Tyr Trp Asp Asp Ala Glu Ser Phe Lys Pro
    370                 375                 380

Glu Arg Phe Glu Gln Cys Ser Val Asp Phe Phe Gly Asn Asn Phe Glu
385                 390                 395                 400

Phe Leu Pro Phe Gly Gly Gly Arg Arg Ile Cys Pro Gly Met Ser Phe
                405                 410                 415

Gly Leu Ala Asn Leu Tyr Leu Pro Leu Ala Gln Leu Leu Tyr His Phe
            420                 425                 430

Asp Trp Lys Leu Pro Thr Gly Ile Met Pro Arg Asp Leu Asp Leu Thr
        435                 440                 445

Glu Leu Ser Gly Ile Thr Ile Ala Arg Lys Gly Asp Leu Tyr Leu Asn
    450                 455                 460

Ala Thr Pro Tyr Gln Pro Ser Arg Glu
465                 470

<210>  380
<211>  519
<212>  PRT
<213>  Nicotiana tabacum

<400>  380

Met Tyr His Leu Leu Ser Pro Ile Glu Ala Ile Val Gly Leu Val Thr
1               5                   10                  15

Phe Ala Phe Leu Leu Tyr Leu Leu Trp Thr Lys Lys Gln Ser Lys Ile
            20                  25                  30

Leu Asn Pro Leu Pro Pro Lys Ile Pro Gly Gly Trp Pro Val Ile Gly
        35                  40                  45

His Leu Phe Tyr Phe Asn Asn Asn Gly Asp Asp Asp Arg His Phe Ser
    50                  55                  60

Gln Lys Leu Gly Asp Leu Ala Asp Lys Tyr Gly Pro Val Phe Thr Phe
65                  70                  75                  80

Arg Leu Gly Phe Arg Arg Phe Leu Ala Val Ser Ser Tyr Glu Ala Met
                85                  90                  95

Lys Glu Cys Phe Ser Thr Asn Asp Ile His Phe Ala Asp Arg Pro Ala
            100                 105                 110

Leu Leu Tyr Gly Glu Tyr Leu Cys Tyr Asn Asn Ala Met Leu Ala Val
        115                 120                 125

Ala Lys Tyr Gly Pro Tyr Trp Lys Lys Asn Arg Lys Leu Val Asn Gln
    130                 135                 140

Glu Leu Leu Ser Val Ser Arg Leu Glu Lys Phe Lys His Val Arg Phe
145                 150                 155                 160

Ser Ile Val Gln Lys Asn Ile Lys Gln Leu Tyr Asn Cys Asp Ser Pro
                165                 170                 175

Met Val Lys Ile Asn Leu Ser Asp Trp Ile Asp Lys Leu Thr Phe Asp
            180                 185                 190

Ile Ile Leu Lys Met Val Val Gly Lys Thr Tyr Asn Asn Gly His Gly
        195                 200                 205

Glu Ile Leu Lys Ala Ala Phe Gln Lys Phe Met Val Gln Ala Met Glu
    210                 215                 220

Ile Glu Leu Tyr Asp Val Phe His Ile Pro Phe Phe Lys Trp Leu Asp
225                 230                 235                 240

Leu Thr Gly Asn Ile Lys Ala Met Lys Gln Thr Phe Lys Asp Ile Asp
                245                 250                 255

Asn Ile Ile Gln Gly Trp Leu Asp Glu His Ile Lys Lys Arg Glu Thr
            260                 265                 270

Lys Asp Val Gly Gly Glu Asn Glu Gln Asp Phe Ile Asp Val Leu Leu
        275                 280                 285

Ser Lys Arg Ser Asn Glu His Leu Gly Asp Gly Tyr Ser His Asp Thr
    290                 295                 300

Thr Ile Lys Ala Thr Val Phe Thr Leu Val Leu Asp Ala Thr Asp Thr
305                 310                 315                 320

Leu Ala Leu His Ile Lys Trp Val Met Ala Leu Met Ile Asn Asn Lys
                325                 330                 335

Asn Val Met Lys Lys Ala Gln Glu Glu Met Asp Thr Ile Val Gly Arg
            340                 345                 350

Asp Arg Trp Val Glu Glu Asn Asp Ile Lys Asn Leu Val Tyr Leu Gln
        355                 360                 365

Ala Ile Val Lys Glu Val Leu Arg Leu His Pro Pro Ala Pro Leu Ser
    370                 375                 380

Val Gln His Leu Ser Val Lys Asp Cys Val Val Asn Gly Tyr His Ile
385                 390                 395                 400

Pro Lys Gly Thr Ala Leu Leu Thr Asn Ile Met Lys Leu Gln Arg Asp
                405                 410                 415

Pro Gln Ile Trp Val Asp Pro Asp Thr Phe Asp Pro Glu Arg Phe Leu
            420                 425                 430

Thr Thr Asn Ala Ala Ile Asp Tyr Arg Gly Gln His Tyr Glu Leu Ile
        435                 440                 445

Pro Phe Gly Ser Gly Arg Arg Ala Cys Pro Ala Met Asn Tyr Ser Leu
    450                 455                 460

Gln Val Glu His Leu Ser Ile Ala His Leu Ile Gln Gly Phe Asn Phe
465                 470                 475                 480

Ala Thr Thr Thr Asn Glu Pro Leu Asp Met Lys Gln Gly Val Gly Leu
                485                 490                 495

Thr Leu Pro Lys Lys Thr Asp Val Glu Val Leu Ile Thr Pro Arg Leu
            500                 505                 510

Pro Pro Thr Leu Tyr Gln Tyr
        515

<210>  381
<211>  473
<212>  PRT
<213>  Nicotiana tabacum

<400>  381

Met Glu Phe Phe Ser Leu Val Ser Ile Phe Leu Phe Leu Ser Phe Leu
1               5                   10                  15

Phe Leu Leu Arg Lys Cys Lys Asn Ser Asn Ser Gln Thr Lys Gln Leu
            20                  25                  30

Pro Pro Gly Pro Trp Lys Ile Pro Ile Leu Gly Ser Met Leu His Met
        35                  40                  45

Leu Gly Gly Glu Pro His His Ile Leu Arg Asp Leu Ala Lys Lys Tyr
    50                  55                  60

Gly Pro Ile Met His Leu Gln Phe Gly Glu Ile Ser Ala Val Val Val
65                  70                  75                  80

Thr Ser Arg Glu Met Ala Lys Glu Val Leu Lys Thr His Asp Val Val
                85                  90                  95

Phe Ala Ser Arg Pro Lys Ile Val Ala Met Asp Ile Ile Cys Tyr Asn
            100                 105                 110

Gln Ser Asp Ile Ala Phe Ser Pro Tyr Gly Asp His Trp Arg Gln Met
        115                 120                 125

Arg Lys Ile Cys Val Met Glu Leu Leu Asn Ala Lys Asn Val Arg Ser
    130                 135                 140

Phe Ser Ser Ile Arg Arg Asp Glu Val Val Arg Leu Ile Asp Ser Ile
145                 150                 155                 160

Arg Ser Asp Ser Ser Ser Gly Glu Leu Val Asn Phe Thr Gln Arg Ile
                165                 170                 175

Ile Trp Phe Ala Ser Ser Met Thr Cys Arg Ser Ala Phe Gly Gln Val
            180                 185                 190

Leu Lys Gly Gln Asp Val Phe Ala Lys Lys Ile Arg Glu Val Ile Gly
        195                 200                 205

Leu Ala Glu Gly Phe Asp Val Ala Asp Ile Phe Pro Ser Tyr Lys Phe
    210                 215                 220

Leu His Val Leu Ser Gly Met Lys Arg Lys Leu Leu Asn Ala His Leu
225                 230                 235                 240

Lys Val Asp Ala Ile Val Glu Asp Val Ile Asn Glu His Lys Lys Asn
                245                 250                 255

Leu Ala Thr Gly Lys Thr Asn Gly Ala Leu Gly Asp Met Phe Ala Ala
            260                 265                 270

Gly Thr Glu Thr Ser Ser Thr Thr Thr Val Trp Ala Met Ala Glu Met
        275                 280                 285

Met Lys Asn Pro Asn Val Phe Asn Lys Ala Gln Ala Glu Val Arg Glu
    290                 295                 300

Thr Phe Lys Asp Lys Val Thr Phe Asp Glu Ile Asp Ala Glu Glu Leu
305                 310                 315                 320

Glu Tyr Leu Lys Leu Val Ile Lys Glu Thr Leu Arg Leu His Pro Pro
                325                 330                 335

Ser Pro Leu Leu Val Pro Arg Glu Cys Arg Glu Asp Thr Asp Ile Asn
            340                 345                 350

Gly Tyr Thr Ile Pro Ala Lys Thr Lys Val Met Val Asn Val Trp Ala
        355                 360                 365

Leu Gly Arg Asp Pro Lys Tyr Trp Asp Asp Ala Glu Ser Phe Lys Pro
    370                 375                 380

Glu Arg Phe Glu Gln Cys Ser Val Asp Phe Phe Gly Asn Asn Phe Glu
385                 390                 395                 400

Phe Leu Pro Phe Gly Gly Gly Arg Arg Ile Cys Pro Gly Met Ser Phe
                405                 410                 415

Gly Leu Ala Asn Leu Tyr Leu Pro Leu Ala Gln Leu Leu Tyr His Phe
            420                 425                 430

Asp Trp Lys Leu Pro Ser Gly Met Met Pro Gly Asp Leu Asp Leu Thr
        435                 440                 445

Glu Leu Ala Gly Ile Thr Ile Ala Arg Lys Gly Asp Leu Tyr Leu Met
    450                 455                 460

Ala Thr Pro Tyr Gln Pro Ser Arg Glu
465                 470

<210>  382
<211>  7
<212>  PRT
<213>  Artificial Sequence

<220>
<223>  conserved p450 motif


<220>
<221>  MISC_FEATURE
<222>  (1)..(1)
<223>  Xaa = Glu or Lys

<220>
<221>  MISC_FEATURE

<222>  (2)..(3)
<223>  Xaa = any amino acid

<220>
<221>  MISC_FEATURE
<222>  (5)..(6)
<223>  Xaa = any amino acid

<220>
<221>  MISC_FEATURE
<222>  (7)..(7)
<223>  Xaa = Pro, Thr, Ser or Met

<400>  382

Xaa Xaa Xaa Arg Xaa Xaa Xaa
1               5


<210>  383
<211>  281
<212>  PRT
<213>  Nicotiana tabacum

<400>  383

Ile Asp Lys Leu Ser Gly Leu Thr Tyr Arg Leu Glu Arg Asn Phe Lys
1               5                   10                  15

Asp Leu Asp Asn Phe Tyr Glu Glu Leu Ile Glu Gln His Gln Asn Pro
            20                  25                  30

Asn Lys Pro Lys Tyr Met Glu Gly Asp Ile Val Asp Leu Leu Leu Gln
        35                  40                  45

Leu Lys Lys Glu Lys Leu Thr Pro Leu Asp Leu Thr Met Glu Asp Ile
    50                  55                  60

Lys Gly Ile Leu Met Asn Val Leu Val Ala Gly Ser Asp Thr Ser Ala
65                  70                  75                  80

Ala Ala Thr Val Trp Ala Met Thr Ala Leu Ile Lys Asn Pro Lys Ala
                85                  90                  95

Met Glu Lys Val Gln Leu Glu Ile Arg Lys Ser Val Gly Lys Lys Gly
            100                 105                 110

Ile Val Asn Glu Glu Asp Val Gln Asn Ile Pro Tyr Phe Lys Ala Val
        115                 120                 125

Ile Lys Glu Ile Phe Arg Leu Tyr Pro Pro Ala Pro Leu Leu Val Pro
    130                 135                 140

Arg Glu Ser Met Glu Lys Thr Ile Leu Glu Gly Tyr Glu Ile Arg Pro
145                 150                 155                 160

Arg Thr Ile Val His Val Asn Ala Trp Ala Ile Ala Arg Asp Pro Glu
                165                 170                 175

Ile Trp Glu Asn Pro Asp Glu Phe Ile Pro Glu Arg Phe Leu Asn Ser
            180                 185                 190

Ser Ile Asp Tyr Lys Gly Gln Asp Phe Glu Leu Leu Pro Phe Gly Ala
        195                 200                 205

Gly Arg Arg Gly Cys Pro Gly Ile Ala Leu Gly Val Ala Ser Met Glu
    210                 215                 220

Leu Ala Leu Ser Asn Leu Leu Tyr Ala Phe Asp Trp Glu Leu Pro Tyr
225                 230                 235                 240

Gly Val Lys Lys Glu Asp Ile Asp Thr Asn Val Arg Pro Gly Ile Ala
                245                 250                 255

Met His Lys Lys Asn Glu Leu Cys Leu Val Pro Lys Lys Leu Phe Ile
            260                 265                 270

Asn Tyr Ile Gly Thr Trp Ile Ser Cys
        275                 280